(19) Europäisches Patentamt
European Patent Office
Office européen des brevets

(11) EP 2 711 424 A2

(12) EUROPEAN PATENT APPLICATION

(43) Date of publication:
26.03.2014 Bulletin 2014/13

(21) Application number: 13195780.5

(22) Date of filing: 10.06.2009

(51) Int Cl.:
*C12N 15/82* (2006.01) *C12N 9/10* (2006.01)
*A01H 5/00* (2006.01)

(84) Designated Contracting States:
AT BE BG CH CY CZ DE DK EE ES FI FR GB GR HR HU IE IS IT LI LT LU LV MC MK MT NL NO PL PT RO SE SI SK TR

(30) Priority: 20.06.2008 EP 08158684
23.06.2008 US 74686 P
23.06.2008 EP 08158760
23.06.2008 US 74712 P
26.06.2008 US 75784 P
26.06.2008 EP 08159081
26.06.2008 EP 08159085
26.06.2008 US 75850 P

(62) Document number(s) of the earlier application(s) in accordance with Art. 76 EPC:
09765788.6 / 2 297 327

(71) Applicant: **BASF Plant Science GmbH**
**67056 Ludwigshafen (DE)**

(72) Inventors:
- **Sanz Molinero, Ana Isabel**
**28035 Madrid (ES)**
- **Hatzfeld, Yves**
**59000 Lille (FR)**
- **Frankard, Valerie**
**1410 Waterloo (BE)**
- **Reuzeau, Christophe**
**24350 La Chapelle Gonaguet (FR)**

(74) Representative: **Saelens, Claire**
**BASF SE**
**Global Intellectual Property**
**Carl-Bosch-Strasse 38**
**67056 Ludwigshafen (DE)**

Remarks:
This application was filed on 05-12-2013 as a divisional application to the application mentioned under INID code 62.

# (54) Plants having enhanced yield-related traits and a method for making the same

(57) The present invention relates generally to the field of molecular biology and concerns a method for improving various plant growth characteristics by modulating expression in a plant of a nucleic acid sequence encoding a GS1 (Glutamine Synthase 1). The present invention also concerns plants having modulated expression of a nucleic acid sequence encoding a GS1, which plants have improved growth characteristics relative to corresponding wild type plants or other control plants. The invention also provides constructs useful in the methods of the invention.

Furthermore, the present invention relates generally to the field of molecular biology and concerns a method for enhancing various plant yield-related traits by modulating expression in a plant of a nucleic acid sequence encoding a PEAMT (Phosphoethanolamine N-methyltransferase) polypeptide. The present invention also concerns plants having modulated expression of a nucleic acid sequence encoding a PEAMT, which plants have enhanced yield-related traits relative to corresponding wild type plants or other control plants. The invention also provides hitherto unknown PEAMT-encoding nucleic acid sequences, and constructs comprising the same, useful in performing the methods of the invention.

Yet furthermore, the present invention relates generally to the field of molecular biology and concerns a method for increasing various plant seed yield-related traits by increasing expression in a plant of a nucleic acid sequence encoding a fatty acyl-acyl carrier protein (ACP) thioesterase B (FATB) polypeptide. The present invention also concerns plants having increased expression of a nucleic acid sequence encoding a FATB polypeptide, which plants have increased seed yield-related traits relative to control plants. The invention additionally relates to nucleic acid sequences, nucleic acid sequence constructs, vectors and plants containing said nucleic acid sequences.

Even furthermore, the present invention relates generally to the field of molecular biology and concerns a method for improving various plant growth characteristics by modulating expression in a plant of a nucleic acid sequence encoding a LFY-like (LEAFY-like). The present invention also concerns plants having modulated expression of a nucleic acid sequence encoding a LFY-like, which plants have improved growth characteristics relative to corresponding wild type plants or other control plants. The invention also provides constructs useful in the methods of the invention.



Printed by Jouve, 75001 PARIS (FR)

## Description

The present invention relates generally to the field of molecular biology and concerns a method for improving various plant growth characteristics by modulating expression in a plant of a nucleic acid sequence encoding a GS1 (Glutamine Synthase 1). The present invention also concerns plants having modulated expression of a nucleic acid sequence encoding a GS1, which plants have improved growth characteristics relative to corresponding wild type plants or other control plants. The invention also provides constructs useful in the methods of the invention.

Furthermore, the present invention relates generally to the field of molecular biology and concerns a method for enhancing various plant yield-related traits by modulating expression in a plant of a nucleic acid sequence encoding a PEAMT (Phosphoethanolamine N-methyltransferase) polypeptide. The present invention also concerns plants having modulated expression of a nucleic acid sequence encoding a PEAMT, which plants have enhanced yield-related traits relative to corresponding wild type plants or other control plants. The invention also provides hitherto unknown PEAMT-encoding nucleic acid sequences, and constructs comprising the same, useful in performing the methods of the invention.

Yet furthermore, the present invention relates generally to the field of molecular biology and concerns a method for increasing various plant seed yield-related traits by increasing expression in a plant of a nucleic acid sequence encoding a fatty acyl-acyl carrier protein (ACP) thioesterase B (FATB) polypeptide. The present invention also concerns plants having increased expression of a nucleic acid sequence encoding a FATB polypeptide, which plants have increased seed yield-related traits relative to control plants. The invention additionally relates to nucleic acid sequences, nucleic acid sequence constructs, vectors and plants containing said nucleic acid sequences.

Even furthermore, the present invention relates generally to the field of molecular biology and concerns a method for improving various plant growth characteristics by modulating expression in a plant of a nucleic acid sequence encoding a LFY-like (LEAFY-like). The present invention also concerns plants having modulated expression of a nucleic acid sequence encoding a LFY-like, which plants have improved growth characteristics relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al 2005 Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar 2005 Maydica 50:39). This is in addition to the potential continuation of the micro-environmental or genetic advantage that the plant had to achieve the larger size initially. There is a strong genetic component to plant size and growth rate (e.g. ter Steege et al 2005 Plant Physiology 139:1078), and so for a range of diverse genotypes plant size under one environmental condition is likely to correlate with size under another (Hittalmani et al 2003 Theoretical Applied Genetics 107:679). In this way a standard environment is used as a proxy for the diverse and dynamic environments encountered at different locations and times by crops in the field.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

Harvest index, the ratio of seed yield to aboveground dry weight, is relatively stable under many environmental conditions and so a robust correlation between plant size and grain yield can often be obtained (e.g. Rebetzke et al 2002 Crop Science 42:739). These processes are intrinsically linked because the majority of grain biomass is dependent on current or stored photosynthetic productivity by the leaves and stem of the plant (Gardener et al 1985 Physiology of Crop Plants. Iowa State University Press, pp68-73). Therefore, selecting for plant size, even at early stages of development, has been used as an indicator for future potential yield (e.g. Tittonell et al 2005 Agric Ecosys & Environ 105: 213). When testing for the impact of genetic differences on stress tolerance, the ability to standardize soil properties, temperature, water and nutrient availability and light intensity is an intrinsic advantage of greenhouse or plant growth chamber environments compared to the field. However, artificial limitations on yield due to poor pollination due to the absence of wind or insects, or insufficient space for mature root or canopy growth, can restrict the use of these controlled environments for testing yield differences. Therefore, measurements of plant size in early development, under standardized conditions in a growth chamber or greenhouse, are standard practices to provide indication of potential genetic yield advantages.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

Concerning GS1 polypeptides, it has now been found that various growth characteristics may be improved in plants by modulating expression in a plant of a nucleic acid encoding a GS1 (Glutamine Synthase 1) in a plant.

Concerning PEAMT poilypeptides, it has now been found that various yield-related traits may be improved in plants by modulating expression in a plant of a nucleic acid sequence encoding a PEAMT (Phosphoethanolamine N-methyltransferase) in a plant.

Concerning FATB polypeptides, it has now been found that various seed yield-related traits may be increased in plants relative to control plants, by increasing expression in a plant of a nucleic acid sequence encoding a fatty acyl-acyl carrier protein (ACP) thioesterase B (FATB) polypeptide. The increased seed yield-related traits comprise one or more of: increased total seed yield per plant, increased total number of seeds, increased number of filled seeds, increased seed fill rate, and increased harvest index.

Concerning LFY-like polypeptides, it has now been found that various growth characteristics may be improved in plants by modulating expression in a plant of a nucleic acid sequence encoding a LFY-like (LEAFY-like) in a plant.

**Background**

Glutamine synthase (GS1)

Glutamine synthase catalyses the formation of glutamine from glutamate and $NH_3$, it is the last step of the nitrate assimilation pathway. Based on sequence comparison, glutamine synthases are grouped in two families, cytosolic (GS1) and chloroplastic (GS2) isoforms. GS1 glutamine synthases form a small gene family, where GS2 seems to occur as a single copy gene and both GS1 and GS2 occur in plants and algae. Many reports describe that glutamine synthases from higher plants have a direct impact on plant growth under conditions of nitrogen limitation (Oliveira et al. Plant Physiol. 129, 1170-1180, 2002; Fuentes et al. J. Exp. Bot. 52, 1071-1081, 2001; Migge et al. Planta 210, 252-260, 2000; Martin et al. Plant Cell 18, 3252-3274). However, so far no data are available on the effect of algal-type glutamine synthases on plant growth, in particular under conditions of reduced nitrogen availability.

Phosphoethanolamine N-methyltransferase (PEAMT)

Phosphoethanolamine N-methyltransferase (PEAMT), also called S-adenosyl-L-methionine:ethanolamine-phosphate N-methyltransferase is involved in choline biosynthesis in plants. PEAMT functions in the methylation steps required to convert phosphoethanolamine to phosphocholine (Nuccio et al. 2000. J Biol Chem. 275(19):14095-101). Accordingly a PEAMT enzyme catalyzes one or more of the following reactions :

1) N-dimethylethanolamine phosphate + S-adenosyl-L-methionine <=> phosphoryl-choline + S-adenosyl-homocysteine
2) N-methylethanolamine phosphate + S-adenosyl-L-methionine <=> N-dimethylethanolamine phosphate + S-adenosyl-homocysteine
3) phosphoryl-ethanolamine + S-adenosyl-L-methionine <=> S-adenosyl-homocysteine + N-methylethanolamine phosphate.

The Enzyme Commission numbers assigned by IUPAC-IUBMB (International Union of Biochemistry and Molecular Biology) to PEAMT is EC2.1.1.103. The PEAMT enzyme belongs a class of metyltransferases (Mtases) which are dependent on S-adenosyl-L-methionine (SAM). Methyl transfer from the ubiquitous SAM to nitrogen, oxygen or carbon atoms is frequently employed in diverse organisms ranging from bacteria to plants and mammals. Structural analysis shows that PEAMT proteins belongs to a class of Mtases comprising metyltransferase domains that form the Rossman-like alpha-beta fold (Yang et al. 2004 J. Mol. Biol. 340, 695-706). In addition Phosphatidylethanolamine transferases typically comprise a ubiE/COQ5 methyltransferase domain (Pfam reference PF01209). This domain is also present in a number of methyltransferases involved in ubiquinone/menaquinone, biotin and sterol biosynthesis.

Phospholipids are important structural components of cellular membranes and in addition they play a relevant role in metabolism of essential compounds such as fatty acids. In humans Choline, a B vitamin-like molecule, is an essential nutrient naturally produced and participates in building cell membranes and move fats and nutrients between cells.

Phosphocholine is the major phospholipid in almost every plant tissue. In non-photosynthetic tissue, phosphoethanolamine is the second most prevalent phospholipid, whereas in green tissue the levels of phosphocholine are similar to those of phosphatidylglycerol (Dykes et al. 1976. Biochem J. 158(3): 575-581).

Tobacco plants overexpressing a gene encoding a PEAMT enzyme had reportedly increased the levels of phosphocholine and free Choline without affecting phosphatidylcholine content or growth (McNeil et al. 2001. PNAS. 2001, vol. 98, no. 17 10001-10005).

Fatty acyl-acyl carrier protein (ACP) thioesterase B (FATB)

Plants contain a considerable variety of membrane and storage lipids, and in each lipid, a number of different fatty acids is found. Fatty acids differ by their chain length and the number of double bonds. All plant cells synthesize de novo fatty acids from acetyl-CoA by a common pathway localized in plastids, unlike in other organisms. Fatty acids are either utilized in this organelle or transported to supply diverse cytoplasmic biosynthetic pathways and cellular processes. Production of fatty acids for transport depends on the activity of fatty acyl-acyl carrier protein (ACP) thioesterases (FATs; also called acyl-ACP TE) that release free fatty acids and ACP. Their activity represents the terminal step in the plastidial fatty acid biosynthesis pathway. The resulting free fatty acids can enter the cytosol where they are esterified to coenzyme A and further metabolized into membrane lipids and/or storage triacylglycerols.

FATs play an essential role in determining the amount and composition of fatty acids entering the storage lipid pool. Two classes of FATs have been described in plants, based on amino acid sequence comparisons and substrate specificity: the FATA class and the FATB class (Voelker et al. (1997) Plant Physiol 114:669-677). Substrate specificity of these isoforms determines the chain length and level of saturated fatty acids in plants. The highest activity of FATA is with oleoly-ACP, an unsaturated acyl-ACP, with very low activities towards other acyl-ACPs. FATB has highest activity with saturated acyl-ACPs.

FATA and FATB are nuclear-encoded, plastid-targeted golubular proteins that are functional as dimers. In addition, FATB polypeptides comprise a helical transmembrane anchor. FATB acitivity is encoded by at least two genes in Arabidopsis (Bonaventure et al. (2003) Plant Cell 15: 1020-1033), and by at least four genes in Oryza sativa.

Transgenic Arabidopsis plants (Doermann et al. (2000) Plant Physiol 123: 637-643) and transgenic canola plants (Jones et al. (1995) Plant Cell 7: 359-371) expressing a gene encoding a FATB under the control of a seed-specific promoter, displayed modified seed oil composition.

International patent application WO 2008/006171 describes methods for genetically modifying rice plants such that rice oil, rice bran and rice seeds produced therefrom have altered levels of oleic oil, palmitic acid and/or linoleic acid, by modulation of FAD2 and/or FATB gene expression.

Leafy-like (LFY-like)

Leafy is a transcription factor necessary for floral induction and flower development, and is involved in the specification of floral meristem identity: LFY expression is regulated and restricted to small groups of cells flanking the shoot apical meristem wherein its high level expression marks the alteration of fate from a leaf primordium to a floral primordium (Weigel et al., Cell 69, 843-859, 1992). The protein sequence is highly conserved and in many plant species the protein is encoded by a single gene, in a few species also paralogues are present. In corn, 2 copies of the gene are present (zfl1 and zfl2). Double mutants show a normal development during vegetative growth, but floral development is disturbed (Bomblies et al., Development 130, 2385-2395, 2003). Also in *Arabidopsis,* loss-of-function mutants of LFY show deficiencies in floral development with a partial transformation of flowers into inflorescence shoots (Weigel et al., 1992). Leafy is also reported to play a role in the timing of flowering.

**Summary**

Glutamine synthase (GS1)

Surprisingly, it has now been found that modulating expression of a nucleic acid sequence encoding an algal-type GS1 polypeptide gives plants having enhanced yield-related traits, in particular increased seed yield relative to control plants.

According one embodiment, there is provided a method for improving yield related traits of a plant relative to control plants, comprising modulating expression of a nucleic acid sequence encoding a GS1 polypeptide in a plant.

Phosphoethanolamine N-methyltransferase (PEAMT)

Surprisingly, it has now been found that modulating expression of a nucleic acid sequence encoding a PEAMT polypeptide gives plants having enhanced yield-related traits, relative to control plants.

According to one embodiment, there is provided a method for enhancing yield-related traits of a plant relative to control plants, comprising modulating expression of a nucleic acid sequence encoding a PEAMT polypeptide in a plant.

Fatty acyl-acyl carrier protein (ACP) thioesterase B (FATB)

Surprisingly, it has now been found that increasing expression in a plant of a nucleic acid sequence encoding a FATB polypeptide as defined herein, gives plants having increased seed yield-related traits relative to control plants.

According to one embodiment, there is provided a method for increasing seed yield-related traits in plants relative to control plants, comprising increasing expression in a plant of a nucleic acid sequence encoding a FATB polypeptide as defined herein. The increased seed yield-related traits comprise one or more of: increased total seed yield per plant, increased total number of seeds, increased number of filled seeds, increased seed fill rate, and increased harvest index.

Leafy-like (LFY-like)

Surprisingly, it has now been found that modulating expression of a nucleic acid sequence encoding a LFY-like polypeptide gives plants having enhanced yield-related traits, in particular increased seed yield relative to control plants.

According one embodiment, there is provided a method for improving yield related traits of a plant relative to control plants, comprising modulating expression of a nucleic acid sequence encoding a LFY-like polypeptide in a plant. The improved yield related traits comprised increased seed yield and were obtained without change of flowering time compared to control plants.

## Definitions

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid sequence(s)/Nucleic acid sequence(s)/nucleotide sequence(s)

The terms "polynucleotide(s)", "nucleic acid sequence(s)", "nucleotide sequence(s)", "nucleic acid sequence (s)", "nucleic acid sequence molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.
A deletion refers to removal of one or more amino acids from a protein.
An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.
A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |

(continued)

| Residue | Conservative Substitutions | Residue | Conservative Substitutions |
|---|---|---|---|
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif' or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acid sequences are in solution. The hybridisation process can also occur with one of the complementary nucleic acid sequences immobilised to a matrix such as magnetic beads, Sepharose beads or any other resin. The hybridisation process can furthermore occur with one of the complementary nucleic acid sequences immobilised to a solid support such as a nitro-cellulose or nylon membrane or immobilised by e.g. photolithography to, for example, a siliceous glass support (the latter known as nucleic acid sequence arrays or microarrays or as nucleic acid sequence chips). In order to allow hybridisation to occur, the nucleic acid sequence molecules are generally thermally or chemically denatured to melt a double strand into two single strands and/or to remove hairpins or other secondary structures from single stranded nucleic acid sequences.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acid sequences may deviate in sequence and still encode a substantially identical polypeptide, due to the degeneracy of the genetic code. Therefore medium stringency hybridisation conditions may sometimes be needed to identify such nucleic acid sequence molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid sequence strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x logio[Na^+]^a + 0.41 x \%[G/C^b] - 500 x [L^c]^{-1} - 0.61\ x\%\ formamide$$

2) DNA-RNA or RNA-RNA hybrids:

$$Tm = 79.8 + 18.5 (log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (I_n)$
For 20-35 nucleotides: $T_m = 22 + 1.46 (I_n)$

[a] or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid sequence hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid sequence. When nucleic acid sequences of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acid sequences or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid sequence control sequence located upstream from the transcriptional start of a gene and which is involved in recognising and binding of RNA polymerase and other proteins, thereby directing transcription of an operably linked nucleic acid sequence. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid sequence molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein.

This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid sequence molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid sequence used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

<u>Operably linked</u>

The term "operably linked" as used herein refers to a functional linkage between the promoter sequence and the gene of interest, such that the promoter sequence is able to initiate transcription of the gene of interest.

<u>Constitutive promoter</u>

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |

(continued)

| Gene Source | Reference |
|---|---|
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic acid sequences Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b:** Examples of root-specific promoters

| Gene Source | Reference |
|---|---|
| RCc3 | Plant Mol Biol. 1995 Jan;27(2):237-48 |
| Arabidopsis PHT1 | Kovama et al., 2005; Mudge et al. (2002, Plant J. 31: 341) |
| Medicago phosphate transporter | Xiao et al., 2006 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |

(continued)

| Gene Source | Reference |
|---|---|
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004), which disclosure is incorporated by reference herein as if fully set forth.

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, $\gamma$-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |

(continued)

| Gene source | Reference |
|---|---|
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WS118 | WO 2004/070039 |
| PRO017 rice RAB21 | WO 2004/070039 |
| PRO005 | WO 2004/070039 |
| PRO00095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |

(continued)

| Gene source | Reference |
|---|---|
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88: 7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| Gene source | Expression pattern | Reference |
|---|---|---|
| rice OSH1 | Shoot apical meristem, from embryo globular stage to seedling stage | Sato *et al.* (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acid sequences which serve as promoter or enhancer elements may be introduced in an appropriate position (typically upstream) of a non-heterologous form of a polynucleotide so as to upregulate expression of a nucleic acid sequence encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid sequence/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants. Methods for decreasing expression are known in the art and the skilled person would readily be able to adapt the known methods for silencing so as to achieve reduction of expression of an endogenous gene in a whole plant or in parts thereof through the use of an appropriate promoter, for example.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid sequence encoding the protein of interest (target gene), or from any nucleic acid sequence capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

Examples of various methods for the reduction or substantial elimination of expression in a plant of an endogenous gene, or for lowering levels and/or activity of a protein, are known to the skilled in the art. A skilled person would readily be able to adapt the known methods for silencing, so as to achieve reduction of expression of an endogenous gene in a whole plant or in parts thereof through the use of an appropriate promoter, for example.

This reduction or substantial elimination of expression may be achieved using routine tools and techniques. A preferred method for the reduction or substantial elimination of endogenous gene expression is by introducing and expressing in a plant a genetic construct into which the nucleic acid sequence (in this case a stretch of substantially contiguous nucleotides derived from the gene of interest, or from any nucleic acid sequence capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid sequence or a part thereof (in this case a stretch of substantially contiguous nucleotides derived from the gene of interest, or from any nucleic acid sequence capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acid sequences forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid sequence is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid sequence capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid sequence capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid sequence will be of an antisense orientation to a target nucleic acid sequence of interest). Preferably, production of antisense nucleic acid sequences in plants occurs by means of a stably integrated nucleic acid sequence construct comprising a promoter, an operably linked antisense oligonucleotide, and a terminator.

The nucleic acid sequence molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site.

Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid sequence subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acid sequences, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid sequence to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid sequence construct of the invention. These marker genes enable the identification of a successful transfer of the nucleic acid sequence molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acid sequences into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid sequence molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid sequence can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

Since the marker genes, particularly genes for resistance to antibiotics and herbicides, are no longer required or are undesired in the transgenic host cell once the nucleic acid sequences have been introduced successfully, the process according to the invention for introducing the nucleic acid sequences advantageously employs techniques which enable the removal or excision of these marker genes. One such a method is what is known as co-transformation. The co-transformation method employs two vectors simultaneously for the transformation, one vector bearing the nucleic acid sequence according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid sequence (known as the Ac/Ds technology). The transformants can be crossed with a transposase source or the transformants are transformed with a nucleic acid sequence construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the IoxP sequences. If the marker gene is integrated between the IoxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acid sequences used in the method of the invention are not at their natural locus in the genome of said plant, it being possible for the nucleic acid sequences to be expressed homologously or heterologously. However, as mentioned, transgenic also means that, while the nucleic acid sequences according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acid sequences according to the invention at an unnatural locus in the genome, i.e. homologous or, preferably, heterologous expression of the nucleic acid sequences takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acid sequences or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

<u>T-DNA activation tagging</u>

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acid sequences encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid sequence at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), and g) increased number of primary panicles, which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased seed yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid sequence of interest. The term "plant" also encompasses plant cells, suspension cultures, callus tissue, embryos, meristematic regions, gametophytes, sporophytes, pollen and microspores, again wherein each of the aforementioned comprises the gene/nucleic acid sequence of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp.,

*Tagetes* spp., *Tamarindus indica, Theobroma cacao, Trifolium* spp., *Tripsacum dactyloides, Triticale sp., Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

**Detailed description of the invention**

Surprisingly, it has now been found that modulating expression in a plant of a nucleic acid sequence encoding a GS1 polypeptide gives plants having enhanced yield-related traits relative to control plants. According to a first embodiment, the present invention provides a method for enhancing yield-related traits in plants relative to control plants, comprising modulating expression in a plant of a nucleic acid sequence encoding a GS1 polypeptide.

Furthermore, surprisingly, it has now been found that modulating expression in a plant of a nucleic acid sequence encoding a PEAMT polypeptide gives plants having enhanced yield-related traits relative to control plants. According to a first embodiment, the present invention provides a method for enhancing yield-related traits in plants relative to control plants, comprising modulating expression in a plant of a nucleic acid sequence encoding a PEAMT polypeptide.

Furthermore, surprisingly, it has now been found that increasing expression in a plant of a nucleic acid sequence encoding a FATB polypeptide as defined herein, gives plants having increased seed yield-related traits relative to control plants. According to a first embodiment, the present invention provides a method for increasing seed yield-related traits in plants relative to control plants, comprising increasing expression in a plant of a nucleic acid sequence encoding a FATB polypeptide.

Furthermore, surprisingly, it has now been found that modulating expression in a plant of a nucleic acid sequence encoding a LFY-like polypeptide gives plants having enhanced yield-related traits relative to control plants. According to a first embodiment, the present invention provides a method for enhancing yield-related traits in plants relative to control plants, comprising modulating expression in a plant of a nucleic acid sequence encoding a LFY-like polypeptide.

A preferred method for modulating (preferably, increasing) expression of a nucleic acid sequence encoding a GS1 polypeptide, or a PEAMT polypeptide, or a FATB polypeptide, or a LFY-like polypeptide is by introducing and expressing in a plant a nucleic acid sequence encoding a GS1 polypeptide, or a PEAMT polypeptide, or a FATB polypeptide, or a LFY-like polypeptide.

Concerning GS1 polypeptides, any reference hereinafter to a "protein useful in the methods of the invention" is taken to mean a GS1 polypeptide as defined herein. Any reference hereinafter to a "nucleic acid sequence useful in the methods of the invention" is taken to mean a nucleic acid sequence capable of encoding such a GS1 polypeptide. The nucleic acid sequence to be introduced into a plant (and therefore useful in performing the methods of the invention) is any nucleic acid sequence encoding the type of protein which will now be described, hereafter also named "*GS1* nucleic acid sequence" or "*GS1* gene".

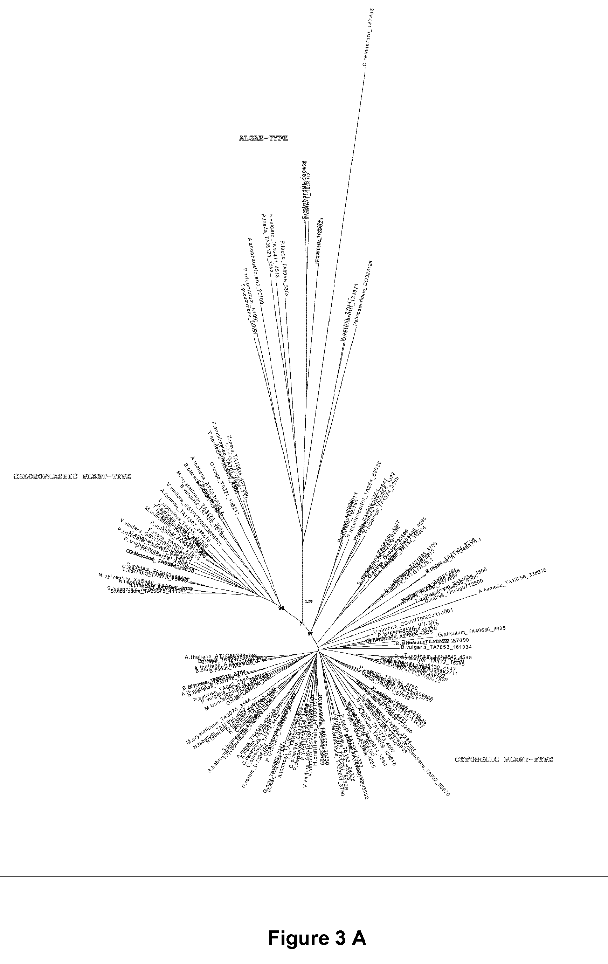

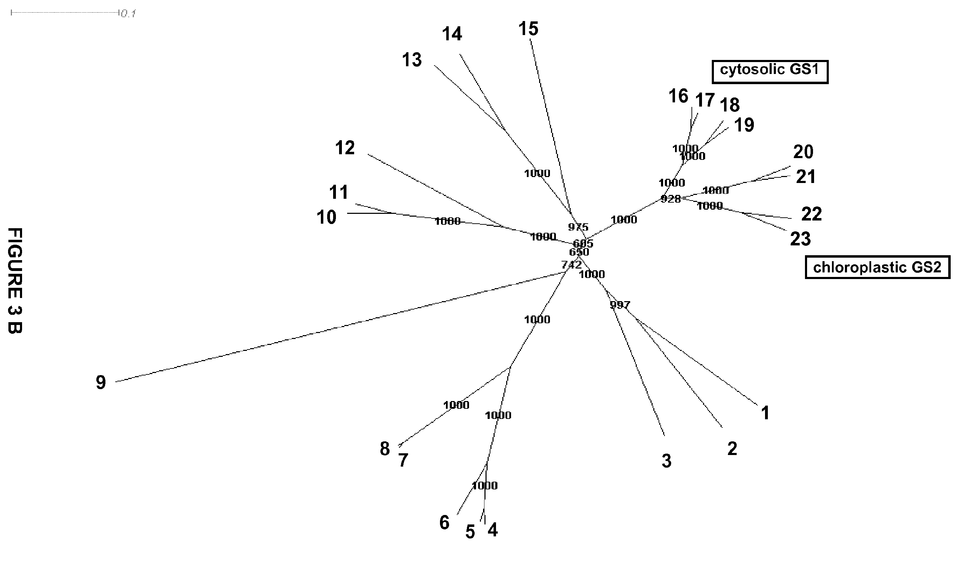

A "GS1 polypeptide" as defined herein for the purpose of the present invention refers to any Glutamine Synthase 1 (GS1) that clusters together with GS1 proteins of algal origin (to form an algal-type clade) in a phylogenetic tree such as the one displayed in Figure 3. Preferably the GS1 is of algal origin. Glutamine synthase (Enzyme Catalogue number EC 6.3.1.2) catalyses the following reaction:

$$ATP + L\text{-Glutamate} + NH_3 \leftrightarrows L\text{-Glutamine} + ADP + Phosphate$$

Preferably, the GS1 protein comprises Gln-synt_C domain (Pfam accession PF00120) and a Gln-synt_N domain (Pfam accession PF03951). Further preferably, the GS1 protein useful in the methods of the present invention comprises at least one, preferably at least two, more preferably all three of the following conserved sequences in which maximally 4, preferably 3 or less, more preferably 2 or less, most preferably 1 or no mismatches are present:

Motif 1 (SEQ ID NO: 3): GY(Y/L/F)(E/T)DRRP(A/S/P)(A/S)(N/D)(V/L/A/M)D (P/A)Y
Preferably Motif 1 is GY(Y/L/F)(E/T)DRRP(A/P)(A/S)(N/D)(V/L/A)D(P/A)Y
Motif 2 (SEQ ID NO: 4): DP(I/F)RG(A/E/D/S/G/L/V)(P/N/D)(H/N)(V/I)(L/I)V (L/I/M)(C/T/A)
Preferably, motif 2 is DP(I/F)RG(A/E/G)(P/N/D)(H/N)(V/I)LV(L/M)(C/A)
Motif 3 (SEQ ID NO: 5): G(A/L/M/G/C)H(T/S/I/V/F)(N/K)(F/Y/V)S(T/S/N)
Preferably Motif 3 is G(A/M/G/C)H(T/I/V/F)(N/K)(F/Y)S(T/N)

Alternatively, the homologue of a GS1 protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 2, provided that the homologous protein comprises the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

Preferably, the polypeptide sequence which when used in the construction of a phylogenetic tree, such as the one depicted in Figure 3a and 3b, clusters with the algal-type clade (the group of algal GS1 polypeptides comprising the amino acid sequence represented by SEQ ID NO: 2) rather than with the plant chloroplastic or plant cytosolic glutamine synthase group.

Concerning PEAMT polypeptides, any reference hereinafter to a "protein (or polypepetide) useful in the methods of the invention" is taken to mean a PEAMT polypeptide as defined herein. Any reference hereinafter to a "nucleic acid sequence useful in the methods of the invention" is taken to mean a nucleic acid sequence capable of encoding such a PEAMT polypeptide. The nucleic acid sequence to be introduced into a plant (and therefore useful in performing the methods of the invention) is any nucleic acid sequence encoding the type of protein which will now be described, hereafter also named "*PEAMT* nucleic acid sequence" or "*PEAMT* gene".

A "PEAMT polypeptide" as defined herein refers to any polypeptide having phosphoethanolamine N-methyltransferase activity.

Tools and techniques for measuring Phosphoethanolamine N-methyltransferase activity are well known in the art. For example in vivo activity of PEAMT polynucleotide and the polypeptide encoded thereof can be analyzed by complementation in Schizosaccharommyces pombe (Nuccio et al; 2000). PEAMT activity may also be determined in vitro as described by (Nuccio et al; 2000).

A "PEAMT polypeptide comprises two IPR013216, Methyltransferase type 11 domains (Interpro accession number: IPR013216; pfam accession number: PF08241) and optionally a ubiE/COQ5 methyltransferase domain (Ubie_ methyltran (pfam accession number: PF01209).

A Methyltransferase type 11 domain and method to identify the presence of such domain in a polypeptide are well known in the art. Examples of proteins comprising two Methyltransferase type 11 domains are set forth in Table A2. The Methyltransferase type 11 domains as present in SEQ ID NO: 58 are given in SEQ ID NO: 86 and 87. The Example section teaches methods to identify the presence of Methyltransferase type 11 and ubiE/COQ5 methyltransferase in the PEAMT polypeptide represented by SEQ ID NO: 58.

SEQ ID NO: 58 comprises two Methyltransferase type 11 domains represented by SEQ ID NO: 86 (PPYEGKSVLELGAGIGRFTGELAQKAGEVIALDIIESAIQKNESVNGHYKNIKFMC ADVTSPDLKIKDGSIDLIFSNWLLMYLSDKEVELMAERMIGWVKPGGYIFFRES) and SEQ ID NO: 87 (DLKPGQKVLDVGCGIGGGDFYMAENFDVHVVGIDLSVNMISFALERAIGLKCS VEFEVADCTTKTYPDNSFDVIYSRDTILHIQDKPALFRTFFKWLKPGGKVLITDY).

Additionally, SEQ ID NO: 58 comprises a ubiE/COQ5 methyltransferase domain represented by SEQ ID NO: 88 (ERVFGEGYVSTGGFETTKEFVAKMDLKPGQKVL DVGCGIGGGDFYMAENFDVHVVGIDLSVNMISFALE-RAIGLKCSVEFEVADCTTKTYPDNS FDVIYSRDTILHIQDKPALFRTFFKWLKPGGKVLITDYCRSAETPSPEFAE-YIKQRGYDLHDV QAYGQMLKDAGFDDVIAEDRTDQ)

A "PEAMT polypeptide" useful in the methods of the invention may additionally comprise one or more of the following motifs:

(i) Motif 4: IFFRESCFHQSGD (SEQ ID NO: 89);
(ii) Motif 5: EYIKQR ( SEQ ID NO: 90);
(iii) Motif 6: WGLFIA (SEQ ID NO: 91);

Motifs 4 to 6 are located in the C-terminal half of the PEAMT polypeptide represented by SEQ ID NO: 58 at amino acid positions 138-150, 383-388 and 467-472 respectively.

Preferably, the PEAMT protein useful in the methods of the invention comprises a motif having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to any one of Motifs 1 to 3.

More preferably, the PEAMT protein useful in the methods of the invention comprises a a conserved domain having in increasing order of preference at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any of SEQ ID NO: 86 to 88 or to any of the amino acid domains set forth in Table C2 of the Example section.

A "PEAMT or a homologue thereof" as defined herein refers to any polypeptide having in increasing order of preference at least 50%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 58.

Alternatively, the homologue of a PEAMT protein comprises a conserved amino acid domain having in increasing order of preference at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid motifs set forth in Table C2.

The sequence identity is determined using an alignment algorithms, such as the Needleman Wunsch algorithm in the program GAP (GCG Wisconsin Package, Accelrys), preferably with default parameters or BLAST. Compared to overall sequence identity, the sequence identity will generally be higher when only conserved domains or motifs are considered.

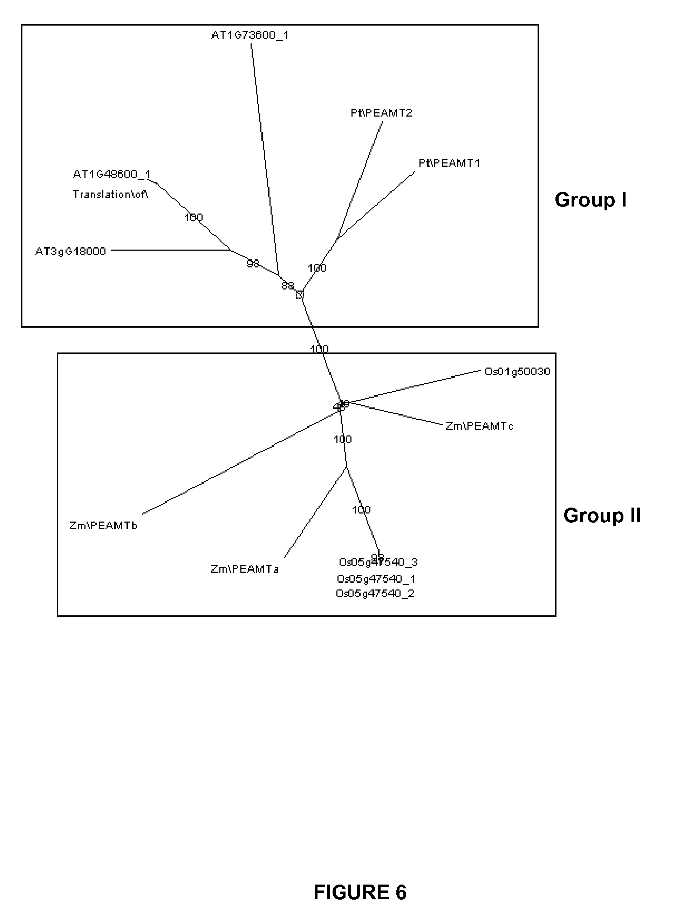

Preferably, the polypeptide sequence which when used in the construction of a phylogenetic tree, such as the one depicted in Figure 6, clusters with the group I of PEAMT polypeptides comprising the amino acid sequence represented by SEQ ID NO: 58 rather than with any other group.

Furthermore, the invention also provides hitherto unknown a nucleic acid sequence encoding a FATB polypeptide and a FATB polypeptide.

According to one embodiment of the present invention, there is therefore provided an isolated nucleic acid sequence comprising:

(i) a nucleic acid sequence as represented by SEQ ID NO: 130;
(ii) the complement of a nucleic acid sequence as represented by SEQ ID NO: 130;
(iii) a nucleic acid sequence encoding FATB polypeptide having, in increasing order of preference, at least 70%, 75%, 80%, 85%, 90%, 95%, 96%, 97%, 98%, 99% or more amino acid sequence identity to the polypeptide sequence as represented by SEQ ID NO: 131.

According to a further embodiment of the present invention, there is also provided an isolated polypeptide comprising:

(i) a polypeptide sequence represented by SEQ ID NO: 131;
(ii) a polypeptide sequence having, in increasing order of preference, at least 70%, 75%, 80%, 85%, 90%, 95%, 96%, 97%, 98%, 99% or more sequence identity to the polypeptide sequence as represented by SEQ ID NO: 131;
(iii) derivatives of any of the polypeptide sequences given in (i) or (ii) above.

A preferred method for increasing expression in a plant of a nucleic acid sequence encoding a FATB polypeptide is by introducing and expressing in a plant a nucleic acid sequence encoding a FATB polypeptide.

Concerning FATB polypeptides, any reference hereinafter to a "protein useful in the methods of the invention" is taken to mean a FATB polypeptide as defined herein. Any reference hereinafter to a "nucleic acid sequence useful in the methods of the invention" is taken to mean a nucleic acid sequence capable of encoding such a FATB polypeptide. The nucleic acid sequence to be introduced into a plant (and therefore useful in performing the methods of the invention) is any nucleic acid sequence encoding the type of polypeptide, which will now be described, hereafter also named "*FATB* nucleic acid sequence" or "*FATB* gene".

A "FATB polypeptide" as defined herein refers to any polypeptide comprising (i) a plastidic transit peptide; (ii) at least one transmembrane helix; (iii) and an acyl-ACP thioesterase family
domain with an InterPro accession IPR002864;

Alternatively or additionally, a "FATB polypeptide" as defined herein refers to any polypeptide sequence having (i) a plastidic transit peptide; (ii) in increasing order of preference at least 50%, 55%, 60%, 65%, 70%, 75%, 80%, 85%, 90%, 95%, 98%, 99% or more amino acid sequence identity to a transmembrane helix as represented by SEQ ID NO: 141; and having in increasing order of preference at least 50%, 55%, 60%, 65%, 70%, 75%, 80%, 85%, 90%, 95%, 98%, 99% or more amino acid sequence identity to an acyl-ACP thioesterase family domain as represented by SEQ ID NO: 140.

Alternatively or additionally, a "FATB polypeptide" as defined herein refers to any polypeptide having in increasing order of preference at least 50%, 55%, 60%, 65%, 70%, 75%, 80%, 85%, 90%, 95%, 98%, 99% or more amino acid sequence identity to a FATB polypeptide as represented by SEQ ID NO: 93 or to any of the polypeptide sequences given in Table A3 herein.

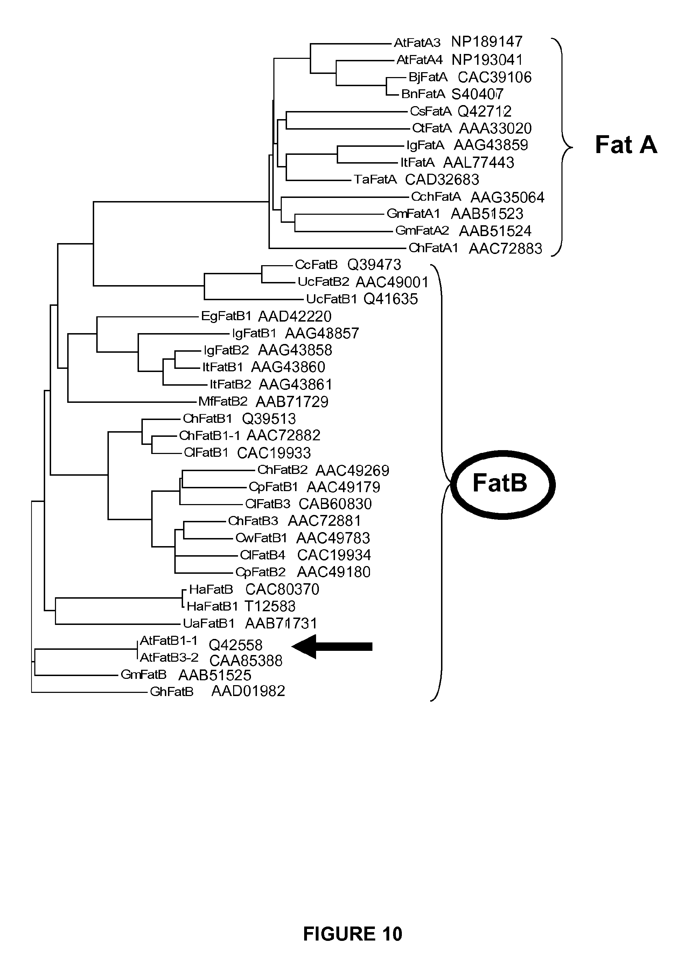

Alternatively or additionally, a "FATB polypeptide" as defined herein refers to any polypeptide sequence which when used in the construction of a FATs (FATA and FATB together) phylogenetic tree, such as the one depicted in Figure 10, clusters with the clade of FATB polypeptides comprising the polypeptide sequence as represented by SEQ ID NO: 93 (shown by an arrow in Figure 10) rather than with the clade of FATA polypeptides.

Alternatively or additionally, an "FATB polypeptide" is a polypeptide with enzymatic activity consisting in hydrolyzing acyl-ACP thioester bonds, preferentially from saturated acyl-ACPs (with chain lengths that vary between 8 and 18 carbons), releasing free fatty acids and acyl carrier protein (ACP).

Concerning LFY-like polypeptides, any reference hereinafter to a "protein useful in the methods of the invention" is taken to mean a LFY-like polypeptide as defined herein. Any reference hereinafter to a "nucleic acid sequence useful in the methods of the invention" is taken to mean a nucleic acid sequence capable of encoding such a LFY-like polypeptide. The nucleic acid sequence to be introduced into a plant (and therefore useful in performing the methods of the invention) is any nucleic acid sequence encoding the type of protein which will now be described, hereafter also named "*LFY-like* nucleic acid sequence" or "*LFY-like* gene".

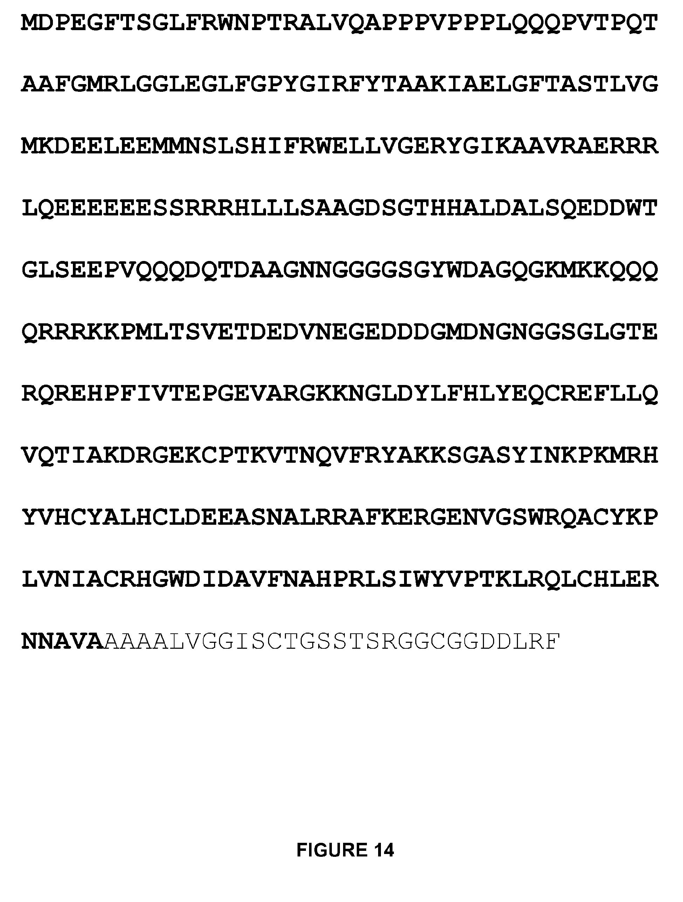

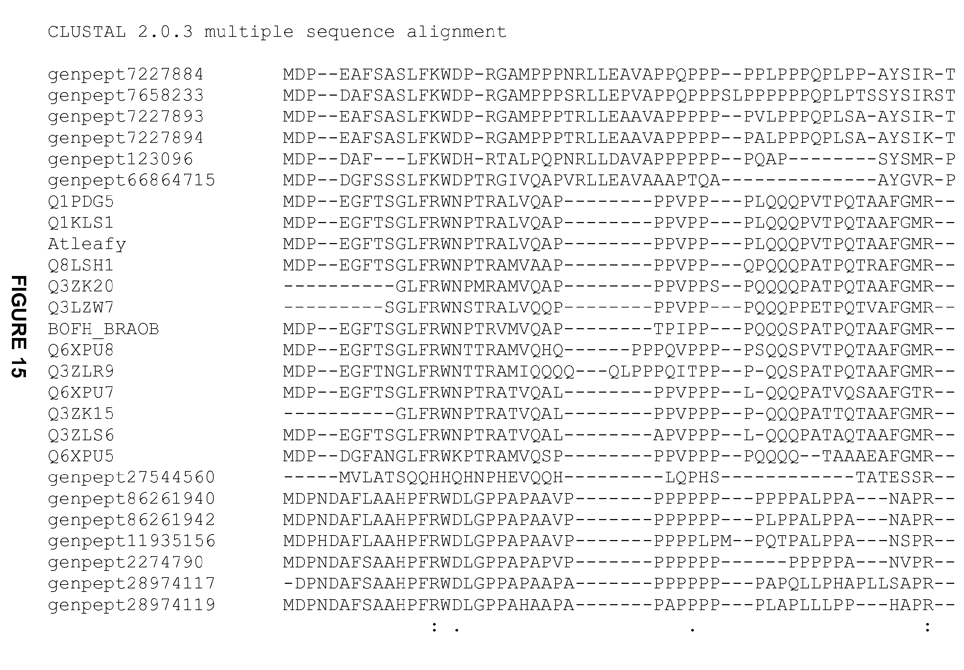

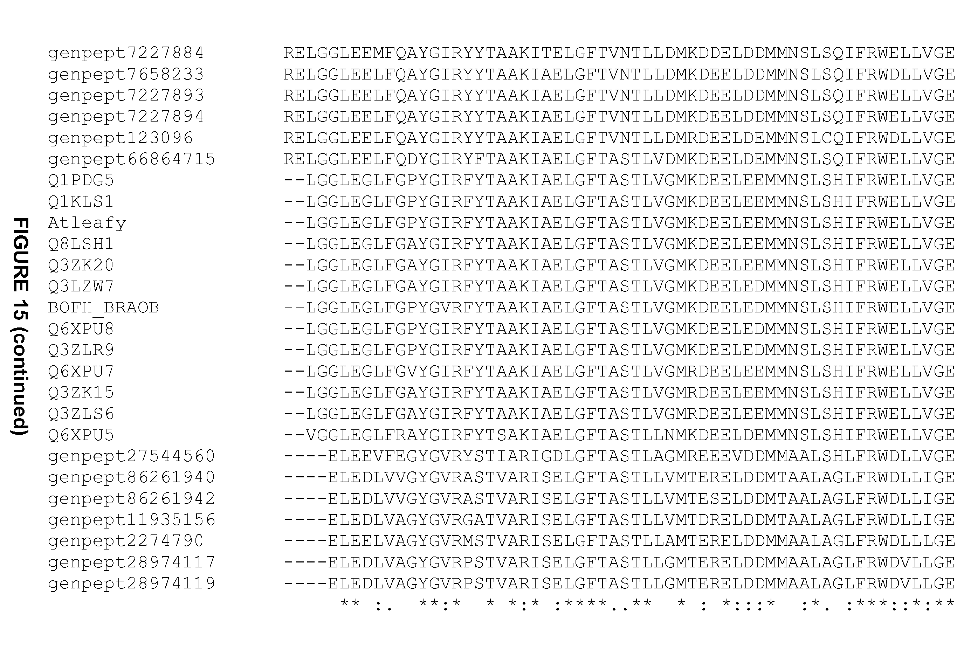

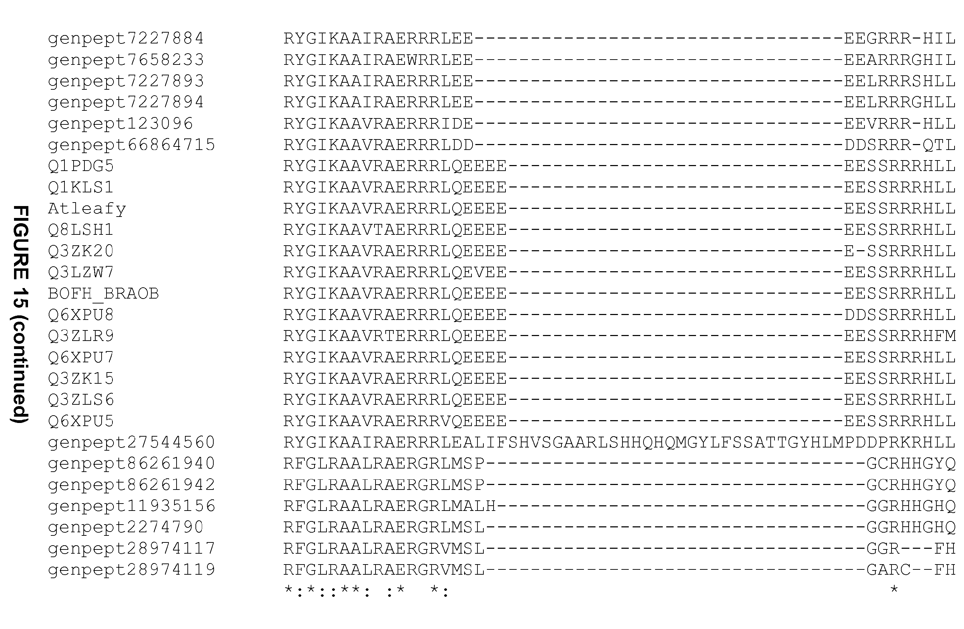

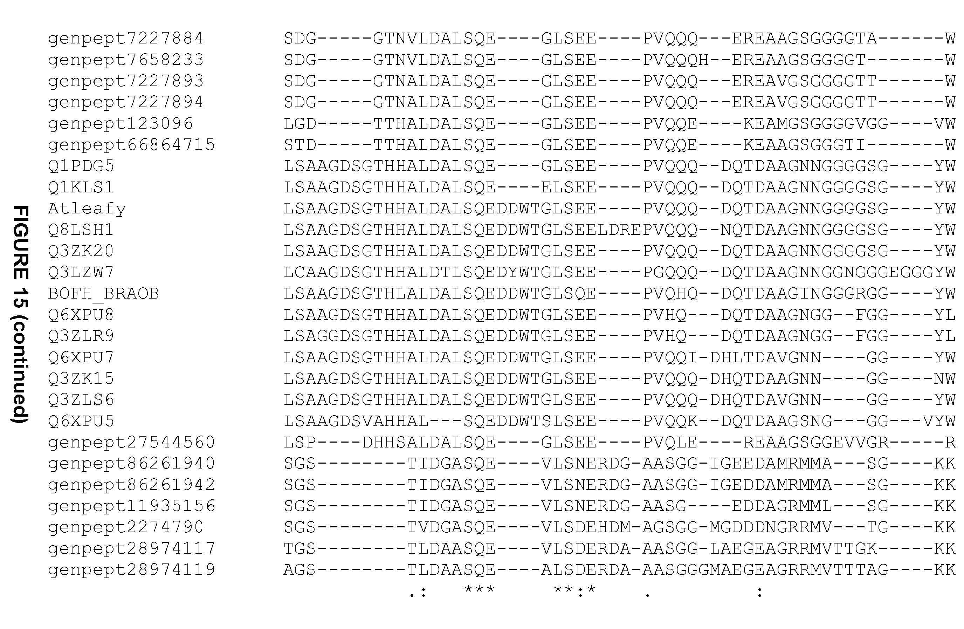

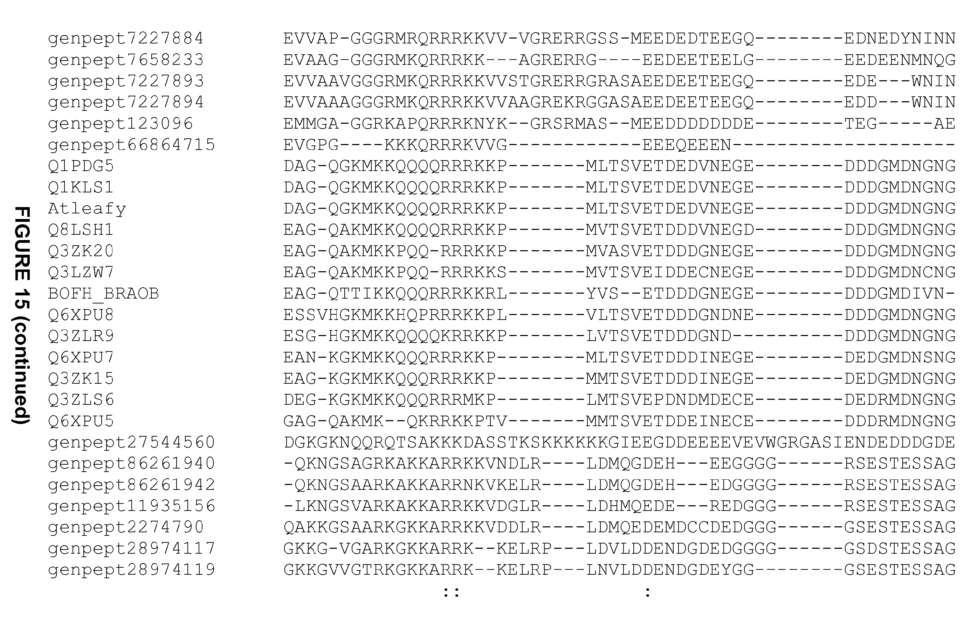

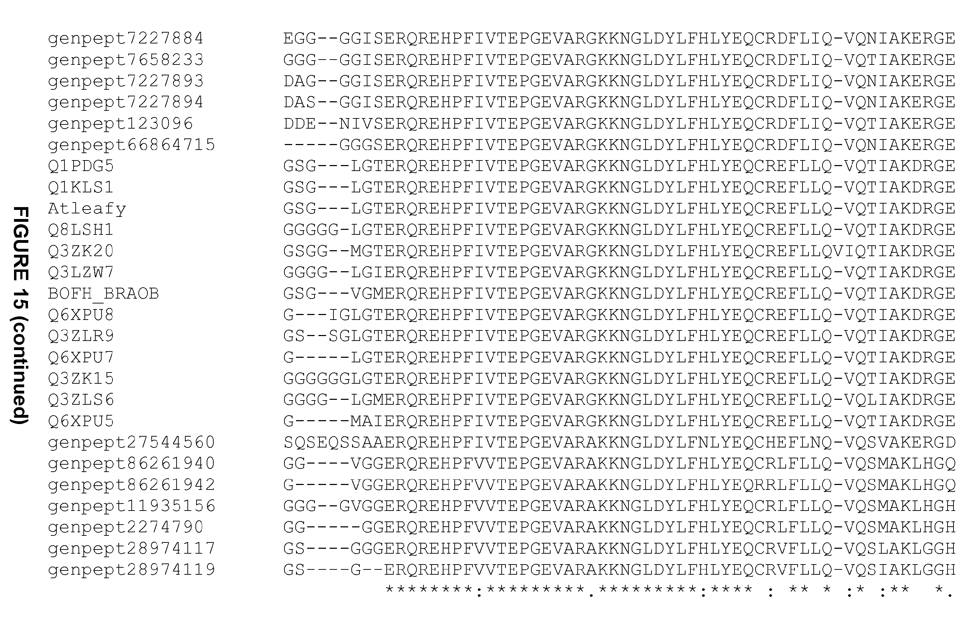

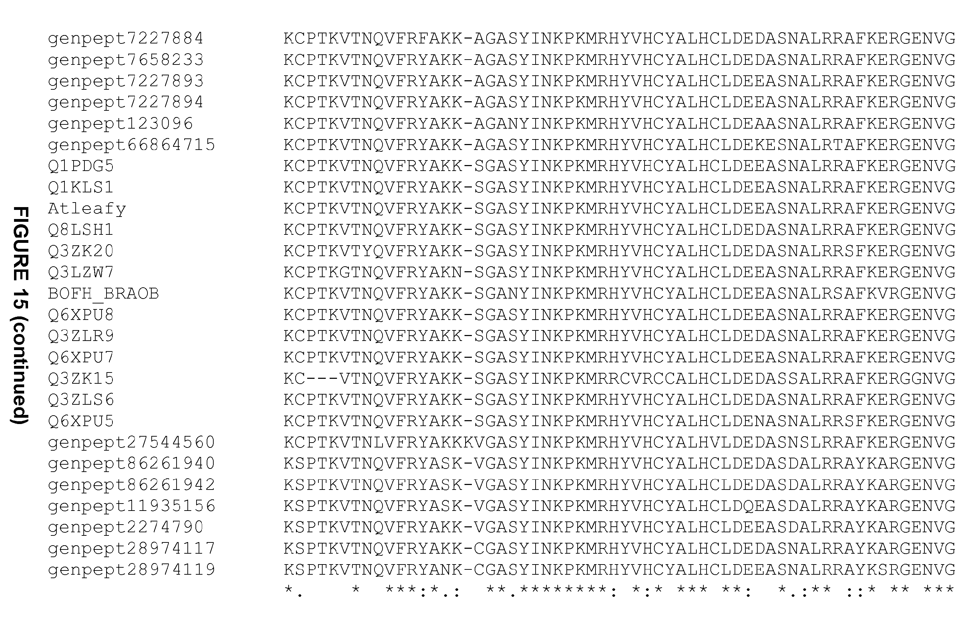

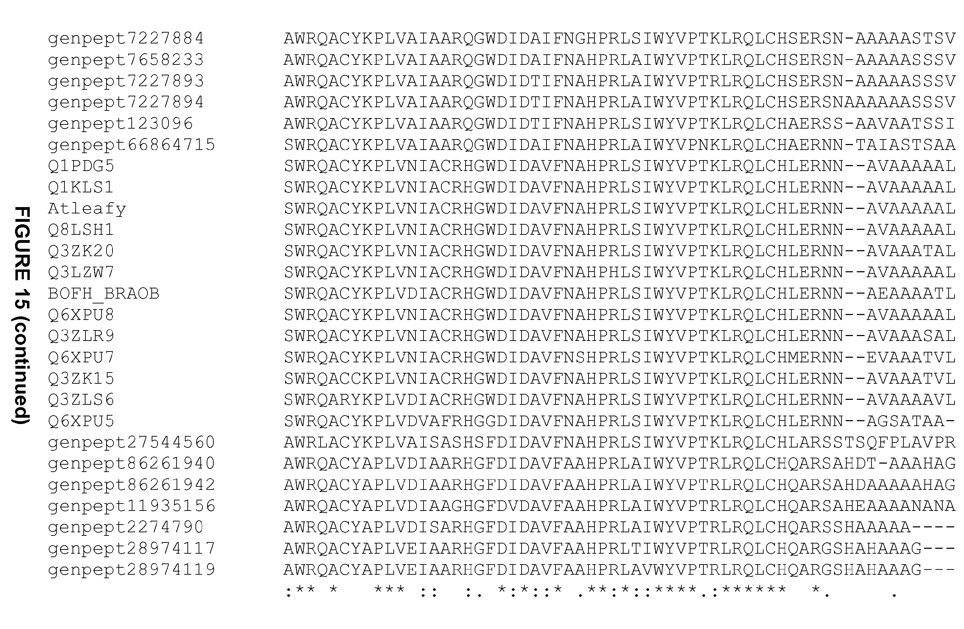

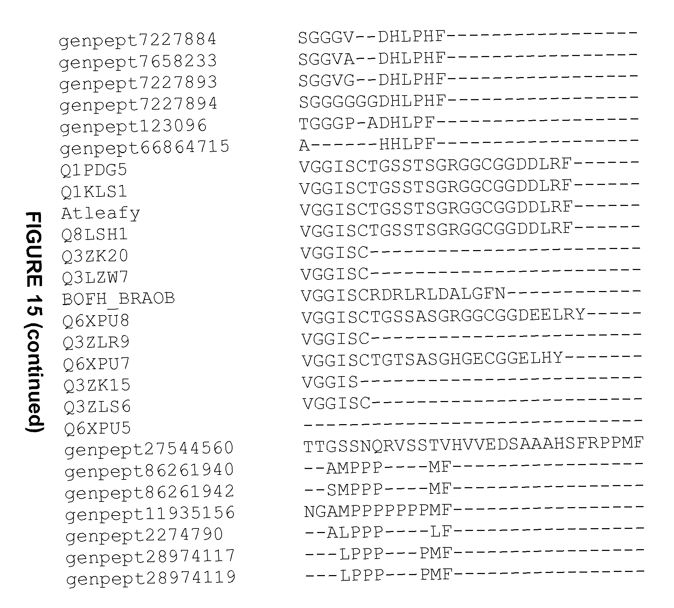

A "LFY-like polypeptide" as defined herein refers to any transcription factor comprising a FLO_LFY domain (InterPro accession IPR002910; Pfam accession PF01698). The FLO_LFY domain represents the major part of the protein sequence (see Figure 14) and is highly conserved (Figure 15).

Preferably, the LFY-like protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 146, provided that the homologous protein comprises the conserved FLO_LFY motif as outlined above. The overall sequence identity is determined using a global alignment algorithm, such as the Needleman Wunsch algorithm in the program GAP (GCG Wisconsin Package, Accelrys), preferably with default parameters. Compared to overall sequence identity, the sequence identity will generally be higher when only conserved domains or motifs (such as the FLO_LFY domain) are considered.

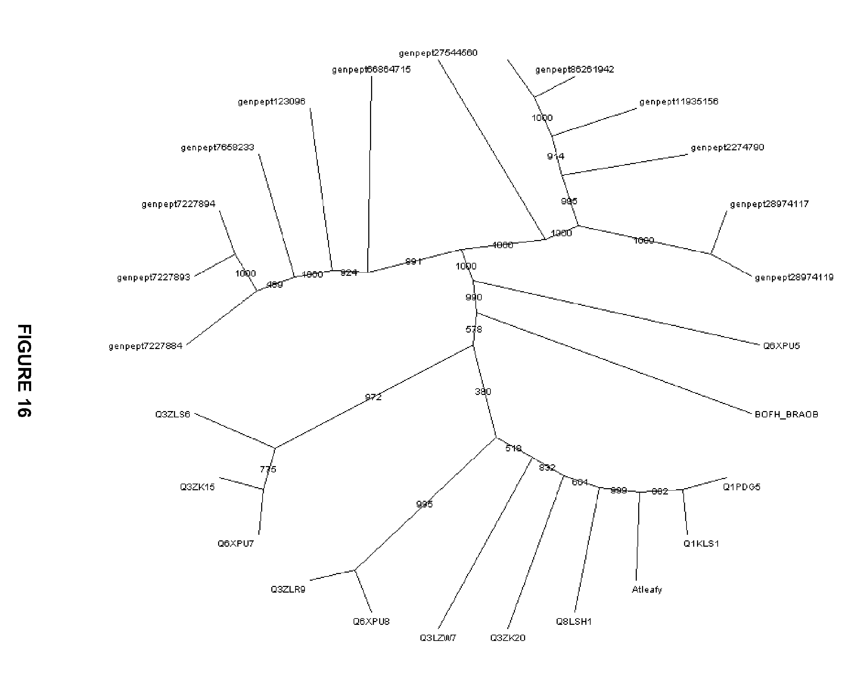

Preferably, the polypeptide sequence which when used in the construction of a phylogenetic tree, such as the one depicted in Figure 16, clusters with the group of LFY-like polypeptides.

The terms "domain", "signature" and "motif" are defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic acid sequences Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic acid sequences Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic acid sequences Res. 31:3784-3788(2003)). Domains or motifs may also be identified using routine techniques, such as by sequence alignment.

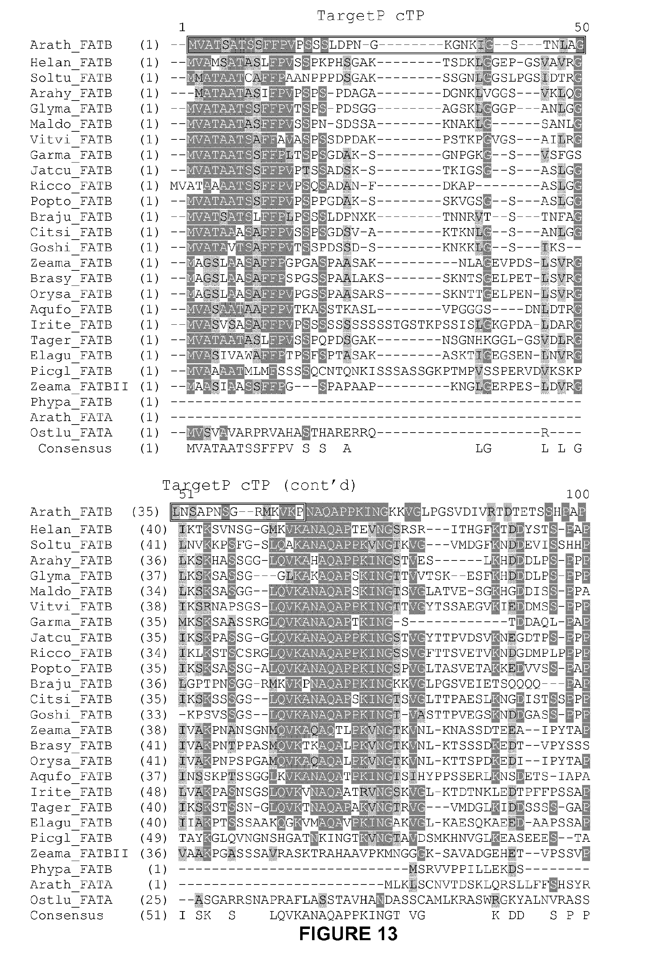

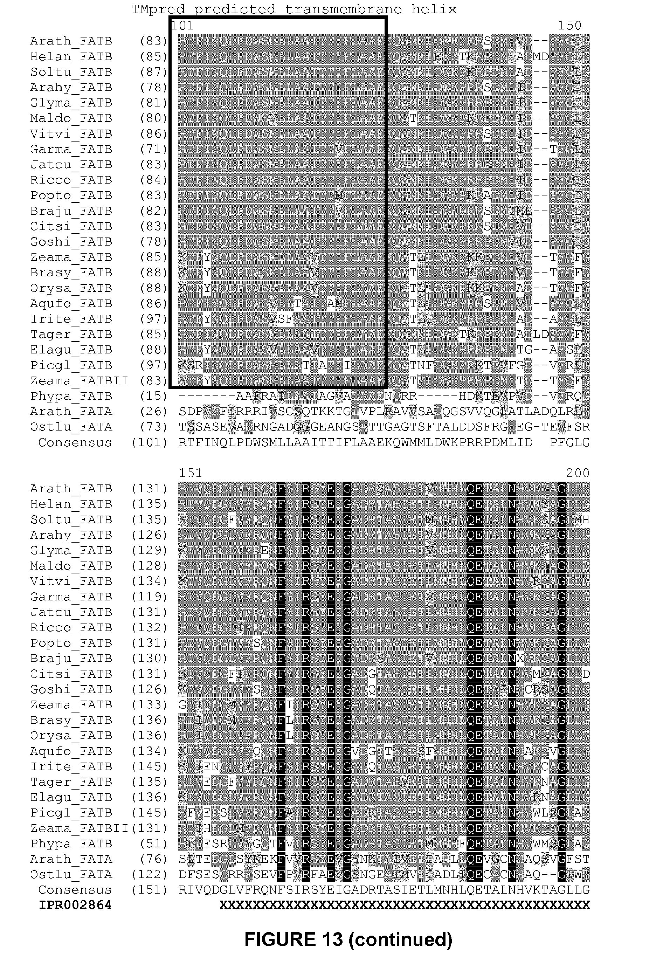

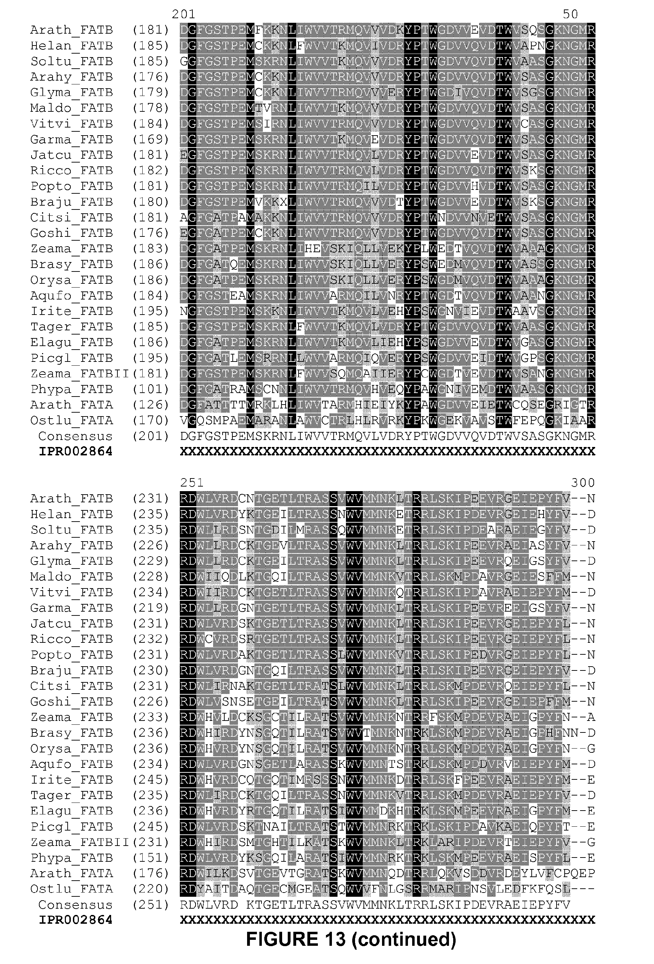

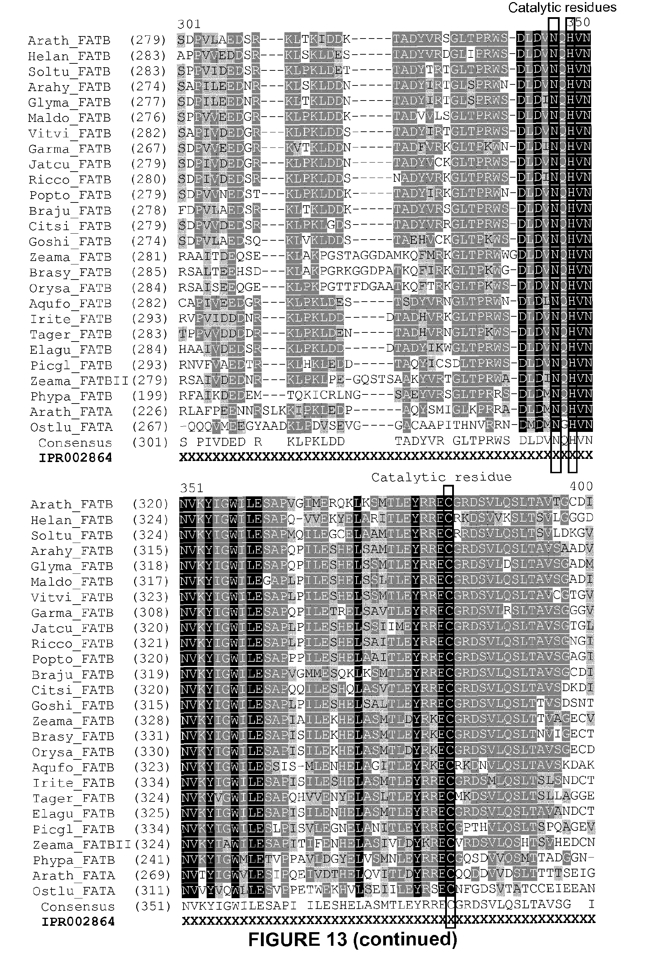

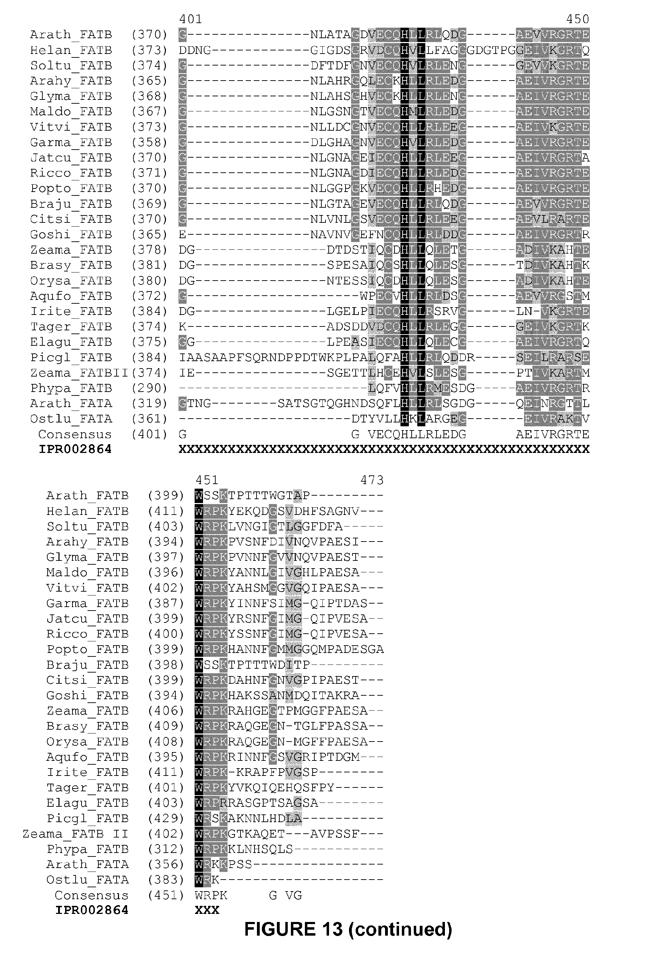

Concerning FATB polypeptides, analysis of the polypeptide sequence of SEQ ID NO: 93 is presented below in Example 4 herein. For example, a FATB polypeptide as represented by SEQ ID NO: 93 comprises an acyl-ACP thioesterase family domain with an InterPro accession IPR002864. An alignment of the polypeptides of Table A3 herein, is shown in Figure 13. Such alignments are useful for identifying the most conserved domains or motifs between the FATB polypeptides, such as the TMpred predicted transmembrane helix (see Example 5 herein) as represented by SEQ ID NO: 141 (comprised in SEQ ID NO: 93).

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid sequence or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Concerning FATB polypeptides, example 3 herein describes in Table B3 the percentage identity between the FATB polypeptide as represented by SEQ ID NO: 93 and the FATB polypeptides listed in Table A2, which can be as low as 53% amino acid sequence identity.

The task of protein subcellular localisation prediction is important and well studied. Knowing a protein's localisation helps elucidate its function. Experimental methods for protein localization range from immunolocalization to tagging of proteins using green fluorescent protein (GFP) or beta-glucuronidase (GUS). Such methods are accurate although labor-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP, TMHMM, and others. The identification of subcellular localisation of the polypeptide of the invention is shown in Example 5. In particular SEQ ID NO: 2 of the present invention is assigned to the plastidic (chloroplastic) compartment of plant cells. In addition to a transit peptide, FATB polypeptides further comprise a predicted transmembrane helix (see Example 5 herein) for anchoring to a chloroplast membrane.

Methods for targeting to plastids are well known in the art and include the use of transit peptides. Table 3 below shows examples of transit peptides which can be used to target any FATB polypeptide to a plastid, which FATB polypeptide is not, in its natural form, normally targeted to a plastid, or which FATB polypeptide in its natural form is targeted to a plastid by virtue of a different transit peptide (for example, its natural transit peptide). Cloning a nucleic acid sequence encoding a transit peptide upstream and in-frame of a nucleic acid sequence encoding a polypeptide (for example, a FATB polypeptide lacking its own transit peptide), involves standard molecular techniques that are well-known in the art.

**Table 3:** Examples of transit peptide sequences useful in targeting polypeptides to plastids

| NCBI Accession Number /SEQ ID NO | Source Organism | Protein Function | Transit Peptide Sequence |
|---|---|---|---|
| SEQ ID NO: P07839 | *Chlamydomonas* | Ferredoxin | MAMAMRSTFAARVGAKPAVRGARP ASRMSCMA |
| SEO ID NO: AAR23425 | *Chlamydomonas* | Rubisco activase | MQVTMKSSAVSGQRVGGARVATRS VRRAQLQV |
| SEO ID NO: CAA56932 | *Arabidopsis thaliana* | Aspartate amino transferase | MASLMLSLGSTSLLPREINKDKLKLG TSASNPFLKAKSFSRVTMTVAVKPSR |
| SEQ ID NO: CAA31991 | *Arabidopsis thaliana* | Acyl carrier protein 1 | MATQFSASVSLQTSCLATTRISFQKP ALISNHGKTNLSFNLRRSIPSRRLSVS C |
| SEQ ID NO: CAB63798 | *Arabidopsis thaliana* | Acyl carrier protein2 | MASIAASASISLQARPRQLAIAASQVK SFSNGRRSSLSFNLRQLPTRLTVSCA AKPETVDKVCAVVRKQL |
| SEQ ID NO: CAB63799 | *Arabidopsis thaliana* | Acyl carrier protein3 | MASIATSASTSLQARPRQLVIGAKQV KSFSYGSRSNLSFNLRQLPTRLTVYC AAKPETVDKVCAVVRKQLSLKE |

The FATB polypeptide is targeted and active in the chloroplast, i.e., the FATB polypeptide is capable of hydrolyzing acyl-ACP thioester bonds, preferentially from saturated acyl-ACPs (with chain lengths that vary between 8 and 18 carbons), releasing free fatty acids and acyl carrier protein (ACP). Assays for testing these activities are well known in the art. Further details are provided in Example 6.

Furthermore, GS1 polypeptides (at least in their native form) typically have glutamine synthase activity. Tools and techniques for measuring glutamine synthase activity are well known in the art (see for example Martin et al. Anal. Biochem. 125, 24-29, 1982 and Example 6).

In addition, PEAMT polypeptides, when expressed in rice according to the methods of the present invention as outlined in the Example section, give plants having increased yield related traits, in particular one or more of increased green biomass, early vigour, total seed weight, number of flowers per panicle, seed filing rate, thousand kernel weight and harvest index.

Furthermore, LFY-like polypeptides (at least in their native form) typically have DNA-binding activity. Tools and techniques for measuring DNA-binding activity are well known in the art. An example of characterisation of DNA binding properties of a protein is provided by Xue (Plant J. 41, 638-649, 2005).

In addition, LFY-like polypeptides, when expressed in rice according to the methods of the present invention as outlined in Examples 7 and 8, give plants having increased yield related traits, in particular increased seed yield.

Concerning GS1 polypeptides, the present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any GS1-encoding nucleic acid sequence or GS1 polypeptide as defined herein.

Examples of nucleic acid sequences encoding GS1 polypeptides are given in Table A1 of Example 1 herein. Such nucleic acid sequences are useful in performing the methods of the invention. The amino acid sequences given in Table A1 of Example 1 are example sequences of orthologues and paralogues of the GS1 polypeptide represented by SEQ ID NO: 2, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A1 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against *Chlamydomonas* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning PEAMT polypeptides, the present invention is illustrated by transforming plants with the nucleic acid sequence represented by SEQ ID NO: 57, encoding the polypeptide sequence of SEQ ID NO: 58. However, performance of the invention is not restricted to these sequences; the methods of the invention may advantageously be performed using any PEAMT-encoding nucleic acid sequence or PEAMT polypeptide as defined herein.

Examples of nucleic acid sequences encoding PEAMT polypeptides are given in Table A2 of the Examples section herein. Such nucleic acid sequences are useful in performing the methods of the invention. The amino acid sequences given in Table A of the Examples section are example sequences of orthologues and paralogues of the PEAMT polypeptide represented by SEQ ID NO: 58, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A2 of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 57 or SEQ ID NO: 58, the second BLAST would therefore be against Arabidopsis thaliana sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning FATB polypeptides, the present invention is illustrated by transforming plants with the nucleic acid sequence represented by SEQ ID NO: 92, encoding the FATB polypeptide sequence of SEQ ID NO: 93. However, performance of the invention is not restricted to these sequences; the methods of the invention may advantageously be performed using any nucleic acid sequence encoding a FATB polypeptide as defined herein.

Examples of nucleic acid sequences encoding FATB polypeptides are given in Table A3 of Example 1 herein. Such nucleic acid sequences are useful in performing the methods of the invention. The polypeptide sequences given in Table A3 of Example 1 are example sequences of orthologues and paralogues of the FATB polypeptide represented by SEQ ID NO: 93, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A3 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 92 or SEQ ID NO: 93, the second BLAST would therefore be against *Arabidopsis thaliana* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning LFY-like polypeptides, the present invention is illustrated by transforming plants with the nucleic acid sequence represented by SEQ ID NO: 145, encoding the polypeptide sequence of SEQ ID NO: 146. However, performance of the invention is not restricted to these sequences; the methods of the invention may advantageously be performed using any LFY-like-encoding nucleic acid sequence or LFY-like polypeptide as defined herein.

Examples of nucleic acid sequences encoding LFY-like polypeptides are given in Table A4 of Example 1 herein. Such nucleic acid sequences are useful in performing the methods of the invention. The amino acid sequences given in Table A4 of Example 1 are example sequences of orthologues and paralogues of the LFY-like polypeptide represented by SEQ ID NO: 146, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A4 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 145 or SEQ ID NO: 146, the second BLAST would therefore be against *Arabidopsis* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid sequence (or polypeptide) sequences over a particular length. In the case of large families, ClustalW may be used, followed by a neighbour joining tree, to help visualize clustering of related genes and to identify orthologues and paralogues.

Furthermore, the invention also provides hitherto unknown GS1-encoding nucleic acid sequences and GS1 polypeptides.

According to a further embodiment of the present invention, there is also provided an isolated polypeptide selected from:

(i) an amino acid sequence represented by SEQ ID NO: 53 or SEQ ID NO: 54;
(ii) an amino acid sequence having, in increasing order of preference, at least 50%, 55%, 60%, 65%, 70%, 75%, 80%, 85%, 90%, 95%, 96%, 97%, 98%, 99% or more sequence identity to the amino acid sequence represented by SEQ ID NO: 53 or SEQ ID NO: 54,
(iii) derivatives of any of the amino acid sequences given in (i) or (ii) above.

The inventions also provides nucleic acid sequences encoding the unknown GS1 polypeptides as disclosed above and nucleic acid sequences hybridising thereto, preferably under stringent conditions.

Nucleic acid sequence variants may also be useful in practising the methods of the invention. Examples of such variants include nucleic acid sequences encoding homologues and derivatives of any one of the amino acid sequences given in Table A1 to A4 of the Examples section, the terms "homologue" and "derivative" being as defined herein. Also useful in the methods of the invention are nucleic acid sequences encoding homologues and derivatives of orthologues or paralogues of any one of the amino acid sequences given in Table A1 to A4 of the Examples section. Homologues and derivatives useful in the methods of the present invention have substantially the same biological and functional activity as the unmodified protein from which they are derived.

Further nucleic acid sequence variants useful in practising the methods of the invention include portions of nucleic acid sequences encoding GS1 polypeptides, or PEAMT polypeptides, or FATB polypeptides, or LFY-like polypeptides, nucleic acid sequences hybridising to nucleic acid sequences encoding GS1 polypeptides, or PEAMT polypeptides, or FATB polypeptides, or LFY-like polypeptides, splice variants of nucleic acid sequences encoding GS1 polypeptides, or PEAMT polypeptides, or FATB polypeptides, or LFY-like polypeptides, allelic variants of nucleic acid sequences encoding GS1 polypeptides, or PEAMT polypeptides, or FATB polypeptides, or LFY-like polypeptides, and variants of nucleic acid sequences encoding GS1 polypeptides, or PEAMT polypeptides, or FATB polypeptides, or LFY-like polypeptides, obtained by gene shuffling. The terms hybridising sequence, splice variant, allelic variant and gene shuffling are as described herein.

Nucleic acid sequences encoding GS1 polypeptides, or PEAMT polypeptides, or FATB polypeptides, or LFY-like polypeptides, need not be full-length nucleic acid sequences, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A1 to A4 of the Examples section, or a portion of a nucleic acid sequence encoding an orthologue, paralogue or homologue of any of the amino acid sequences given in Table A1 to A4 of the Examples section.

A portion of a nucleic acid sequence may be prepared, for example, by making one or more deletions to the nucleic acid sequence. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Concerning GS1 polypeptices, portions useful in the methods of the invention, encode a GS1 polypeptide as defined herein, and have substantially the same biological activity as the amino acid sequences given in Table A1 of Example 1. Preferably, the portion is a portion of any one of the nucleic acid sequences given in Table A1 of Example 1, or is a portion of a nucleic acid sequence encoding an orthologue or paralogue of any one of the amino acid sequences given in Table A1 of Example 1. Preferably the portion is at least 500, 550, 600, 650, 700, 750, 800, 850, 900, 950, 1000, 1050, 1100, 1150 consecutive nucleotides in length, the consecutive nucleotides being of any one of the nucleic acid sequences given in Table A1 of Example 1, or of a nucleic acid sequence encoding an orthologue or paralogue of any one of the amino acid sequences given in Table A1 of Example 1. Most preferably the portion is a portion of the nucleic acid sequence of SEQ ID NO: 1. Preferably, the portion encodes a fragment of an amino acid sequence which, when used in the construction of a phylogenetic tree, such as the one depicted in Figure 3a and 3b, clusters with the algal-type clade (the group of algal GS1 polypeptides comprising the amino acid sequence represented by SEQ ID NO: 2) rather than with the plant chloroplastic or plant cytosolic glutamine synthase group.

Concerning PEAMT polypeptides, portions useful in the methods of the invention, encode a PEAMT polypeptide as defined herein, and have substantially the same biological activity as the amino acid sequences given in Table A2 of the Examples section. Preferably, the portion is a portion of any one of the nucleic acid sequences given in Table A2 of the Examples section, or is a portion of a nucleic acid sequence encoding an orthologue or paralogue of any one of the amino acid sequences given in Table A2 of the Examples section. Preferably the portion is at least 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, consecutive nucleotides in length, the consecutive nucleotides being of any one of the nucleic acid sequences given in Table A2 of the Examples section, or of a nucleic acid sequence encoding an orthologue or paralogue of any one of the amino acid sequences given in Table A2 of the Examples section. Most preferably the portion is a portion of the nucleic acid sequence of SEQ ID NO: 57. Preferably, the portion encodes a fragment of an amino acid sequence which, when used in the construction of a phylogenetic tree, such as the one depicted in Figure 6, clusters with the group I of PEAMT polypeptides comprising the amino acid sequence represented by SEQ ID NO: 58 rather than with any other group.

Concerning FATB polypeptides, portions useful in the methods of the invention, encode a FATB polypeptide as defined herein, and have substantially the same biological activity as the polypeptide sequences given in Table A3 of Example 1. Preferably, the portion is a portion of any one of the nucleic acid sequences given in Table A3 of Example 1, or is a portion of a nucleic acid sequence encoding an orthologue or paralogue of any one of the polypeptide sequences given in Table A3 of Example 1. Preferably the portion is, in increasing order of preference at least 600, 650, 700, 750, 800, 850, 900, 950, 1000,1050, 1100, 1150, 1200 or more consecutive nucleotides in length, the consecutive nucleotides being of any one of the nucleic acid sequences given in Table A3 of Example 1, or of a nucleic acid sequence encoding an orthologue or paralogue of any one of the polypeptide sequences given in Table A3 of Example 1. Preferably, the portion is a portion of a nucleic sequence encoding a polypeptide sequence having in increasing order of preference at least 50%, 55%, 60%, 65%, 70%, 75%, 80%, 85%, 90%, 95%, 98%, 99% or more amino acid sequence identity to the FATB polypeptide as represented by SEQ ID NO: 93 or to any of the polypeptide sequences given in Table A herein. Most preferably, the portion is a portion of the nucleic acid sequence of SEQ ID NO: 92.

Concerning LFY-like polypeptide, portions useful in the methods of the invention, encode a LFY-like polypeptide as defined herein, and have substantially the same biological activity as the amino acid sequences given in Table A4 of Example 1. Preferably, the portion is a portion of any one of the nucleic acid sequences given in Table A4 of Example 1, or is a portion of a nucleic acid sequence encoding an orthologue or paralogue of any one of the amino acid sequences given in Table A4 of Example 1. Preferably the portion is at least 600, 650, 700, 750, 800, 850, 900, 950, 1000, 1050, 1100, 1150, 1200, 1250, 1300, 1350 consecutive nucleotides in length, the consecutive nucleotides being of any one of the nucleic acid sequences given in Table A4 of Example 1, or of a nucleic acid sequence encoding an orthologue or paralogue of any one of the amino acid sequences given in Table A4 of Example 1. Most preferably the portion is a portion of the nucleic acid sequence of SEQ ID NO: 145. Preferably, the portion encodes a fragment of an amino acid sequence which, when used in the construction of a phylogenetic tree, such as the one depicted in Figure 16, clusters with the group of LFY-like polypeptides.

Another nucleic acid sequence variant useful in the methods of the invention is a nucleic acid sequence capable of hybridising, under reduced stringency conditions, preferably under stringent conditions, with a nucleic acid sequence encoding a GS1 polypeptide, or a PEAMT polypeptide, or a FATB polypeptide, a LFY-like polypeptide, as defined herein, or with a portion as defined herein.

According to the present invention, there is provided a method for enhancing yield-related traits in plants, comprising introducing and expressing in a plant a nucleic acid sequence capable of hybridizing to any one of the nucleic acid sequences given in Table A1 to A4 of Example 1, or comprising introducing and expressing in a plant a nucleic acid sequence capable of hybridising to a nucleic acid sequence encoding an orthologue, paralogue or homologue of any of the nucleic acid sequences given in Table A1 to A4 of Example 1.

Concerning GS1 polypeptides, hybridising sequences useful in the methods of the invention encode a GS1 polypeptide as defined herein, having substantially the same biological activity as the amino acid sequences given in Table A1 of Example 1. Preferably, the hybridising sequence is capable of hybridising to the complement of any one of the nucleic acid sequences given in Table A1 of Example 1, or to a portion of any of these sequences, a portion being as defined above, or the hybridising sequence is capable of hybridising to the complement of a nucleic acid sequence encoding an orthologue or paralogue of any one of the amino acid sequences given in Table A1 of Example 1. Most preferably, the hybridising sequence is capable of hybridising to the complement of a nucleic acid sequence as represented by SEQ ID NO: 1 or to a portion thereof.

Concerning GS1 polypeptides, preferably, the hybridising sequence encodes a polypeptide with an amino acid sequence which, when full-length and used in the construction of a phylogenetic tree, such as the one depicted in Figure 3a and 3b, clusters with the algal-type clade (the group of algal GS1 polypeptides comprising the amino acid sequence represented by SEQ ID NO: 2) rather than with the plant chloroplastic or plant cytosolic glutamine synthase group.

Concerning PEAMT polypeptides, hybridising sequences useful in the methods of the invention encode a PEAMT polypeptide as defined herein, having substantially the same biological activity as the amino acid sequences given in Table A2 of the Examples section. Preferably, the hybridising sequence is capable of hybridising to the complement of any one of the nucleic acid sequences given in Table A2 of the Examples section, or to a portion of any of these sequences, a portion being as defined above, or the hybridising sequence is capable of hybridising to the complement of a nucleic acid sequence encoding an orthologue or paralogue of any one of the amino acid sequences given in Table A2 of the Examples section. Most preferably, the hybridising sequence is capable of hybridising to the complement of a nucleic acid sequence as represented by SEQ ID NO: 57 or to a portion thereof.

Concerning PEAMT polypeptides, preferably, the hybridising sequence encodes a polypeptide with an amino acid sequence which, when full-length and used in the construction of a phylogenetic tree, such as the one depicted in Figure 6, clusters with the group I of PEAMT polypeptides comprising the amino acid sequence represented by SEQ ID NO: 58 rather than with any other group.

Concerning FATB polypeptides, hybridising sequences useful in the methods of the invention encode a FATB polypeptide as defined herein, and have substantially the same biological activity as the polypeptide sequences given in Table A3 of Example 1. Preferably, the hybridising sequence is capable of hybridising to any one of the nucleic acid sequences given in Table A3 of Example 1, or to a complement thereof, or to a portion of any of these sequences, a portion being as defined above, or wherein the hybridising sequence is capable of hybridising to a nucleic acid sequence encoding an orthologue or paralogue of any one of the polypeptide sequences given in Table A3 of Example 1, or to a complement thereof.

Concerning FATB polypeptides, preferably, the hybridising sequence is capable of hybridising to a nucleic acid sequence encoding a polypeptide sequence having in increasing order of preference at least 50%, 55%, 60%, 65%, 70%, 75%, 80%, 85%, 90%, 95%, 98%, 99% or more amino acid sequence identity to the FATB polypeptide as represented by SEQ ID NO: 93 or to any of the polypeptide sequences given in Table A3 of Example 1 herein. Most preferably, the hybridising sequence is capable of hybridising to a nucleic acid sequence as represented by SEQ ID NO: 92 or to a portion thereof.

Concerning LFY-like polypeptides, hybridising sequences useful in the methods of the invention encode a LFY-like polypeptide as defined herein, having substantially the same biological activity as the amino acid sequences given in Table A4 of Example 1. Preferably, the hybridising sequence is capable of hybridising to the complement of any one of the nucleic acid sequences given in Table A4 of Example 1, or to a portion of any of these sequences, a portion being as defined above, or the hybridising sequence is capable of hybridising to the complement of a nucleic acid sequence encoding an orthologue or paralogue of any one of the amino acid sequences given in Table A4 of Example 1. Most preferably, the hybridising sequence is capable of hybridising to the complement of a nucleic acid sequence as represented by SEQ ID NO: 145 or to a portion thereof.

Concerning LFY-like polypeptides, preferably, the hybridising sequence encodes a polypeptide with an amino acid sequence which, when full-length and used in the construction of a phylogenetic tree, such as the one depicted in Figure 16, clusters with the group of LFY-like polypeptides.

Another nucleic acid sequence variant useful in the methods of the invention is a splice variant encoding a GS1 polypeptide, or a PEAMT polypeptide, or a FATB polypeptide, or a LFY-like polypeptide, as defined hereinabove, a splice variant being as defined herein.

Concerning GS1 polypeptides, or PEAMT polypeptides, or LFY-like polypeptides, according to the present invention, there is provided a method for enhancing yield-related traits in plants, comprising introducing and expressing in a plant a splice variant of any one of the nucleic acid sequences given in Table A1, or A2, or A4 of Example 1, or a splice variant of a nucleic acid sequence encoding an orthologue, paralogue or homologue of any of the amino acid sequences given in Table A1, or A2, or A4 of Example 1.

Concerning FATB polypeptides, according to the present invention, there is provided a method for increasing seed yield-related traits, comprising introducing and expressing in a plant, a splice variant of any one of the nucleic acid sequences given in Table A3 of Example 1, or a splice variant of a nucleic acid sequence encoding an orthologue, paralogue or homologue of any of the polypeptide sequences given in Table A3 of Example 1, having substantially the same biological activity as the polypeptide sequence as represented by SEQ ID NO: 93 and any of the polypeptide sequences depicted in Table A3 of Example 1.

Concerning GS1 polypeptides, preferred splice variants are splice variants of a nucleic acid sequence represented by SEQ ID NO: 1, or a splice variant of a nucleic acid sequence encoding an orthologue or paralogue of SEQ ID NO: 2. Preferably, the amino acid sequence encoded by the splice variant, when used in the construction of a phylogenetic tree, such as the one depicted in Figure 3a and 3b, clusters with the algal-type clade (the group of algal GS1 polypeptides comprising the amino acid sequence represented by SEQ ID NO: 2) rather than with the plant chloroplastic or plant cytosolic glutamine synthase group.

Concerning PEAMT polypeptides, preferred splice variants are splice variants of a nucleic acid sequence represented by SEQ ID NO: 57, or a splice variant of a nucleic acid sequence encoding an orthologue or paralogue of SEQ ID NO: 58. Preferably, the amino acid sequence encoded by the splice variant, when used in the construction of a phylogenetic tree, such as the one depicted in Figure 6, clusters with the group I of PEAMT polypeptides comprising the amino acid sequence represented by SEQ ID NO: 58 rather than with any other group.

Concerning FATB polypeptides; preferred splice variants are splice variants of a nucleic acid sequence represented by SEQ ID NO: 92, or a splice variant of a nucleic acid sequence encoding an orthologue or paralogue of SEQ ID NO: 93. Preferably, the splice variant is a splice variant of a nucleic acid sequence encoding a polypeptide sequence having in increasing order of preference at least 50%, 55%, 60%, 65%, 70%, 75%, 80%, 85%, 90%, 95%, 98%, 99% or more amino acid sequence identity to the FATB polypeptide as represented by SEQ ID NO: 93 or to any of the polypeptide sequences given in Table A3 herein.

Concerning LFY-like polypeptides, preferred splice variants are splice variants of a nucleic acid sequence represented by SEQ ID NO: 145, or a splice variant of a nucleic acid sequence encoding an orthologue or paralogue of SEQ ID NO: 146. Preferably, the amino acid sequence encoded by the splice variant, when used in the construction of a phylogenetic tree, such as the one depicted in Figure 16, clusters with the group of LFY-like polypeptides.

Another nucleic acid sequence variant useful in performing the methods of the invention is an allelic variant of a nucleic acid sequence encoding a GS1 polypeptide, or a PEAMT polypeptide, or a FATB polypeptide, or a LFY-like polypeptide, as defined hereinabove, an allelic variant being as defined herein.

According to the present invention, there is provided a method for enhancing yield-related traits in plants, comprising introducing and expressing in a plant an allelic variant of any one of the nucleic acid sequences given in Table A1 to A4 of Example 1, or comprising introducing and expressing in a plant an allelic variant of a nucleic acid sequence encoding an orthologue, paralogue or homologue of any of the amino acid sequences given in Table A1 to A4 of Example 1.

Concerning GS1 polypeptides, the polypeptides encoded by allelic variants useful in the methods of the present invention have substantially the same biological activity as the GS1 polypeptide of SEQ ID NO: 2 and any of the amino acids depicted in Table A1 of Example 1. Allelic variants exist in nature, and encompassed within the methods of the present invention is the use of these natural alleles. Preferably, the allelic variant is an allelic variant of SEQ ID NO: 1 or an allelic variant of a nucleic acid sequence encoding an orthologue or paralogue of SEQ ID NO: 2. Preferably, the amino acid sequence encoded by the allelic variant, when used in the construction of a phylogenetic tree, such as the one depicted in Figure 3a and 3b, clusters with the algal-type clade (the group of algal GS1 polypeptides comprising the amino acid sequence represented by SEQ ID NO: 2) rather than with the plant chloroplastic or plant cytosolic glutamine synthase group.

Concerning PEAMT polypeptides, the polypeptides encoded by allelic variants useful in the methods of the present invention have substantially the same biological activity as the PEAMT polypeptide of SEQ ID NO: 58 and any of the amino acids depicted in Table A2 of the Examples section. Allelic variants exist in nature, and encompassed within the methods of the present invention is the use of these natural alleles. Preferably, the allelic variant is an allelic variant of SEQ ID NO: 57 or an allelic variant of a nucleic acid sequence encoding an orthologue or paralogue of SEQ ID NO: 58. Preferably, the amino acid sequence encoded by the allelic variant, when used in the construction of a phylogenetic tree, such as the one depicted in Figure 6, clusters with the group I of PEAMT polypeptides comprising the amino acid sequence represented by SEQ ID NO: 58 rather than with any other group.

Concerning FATB polypeptides, the allelic variants useful in the methods of the present invention have substantially the same biological activity as the FATB polypeptide of SEQ ID NO: 93 and any of the polypeptide sequences depicted in Table A3 of Example 1. Allelic variants exist in nature, and encompassed within the methods of the present invention is the use of these natural alleles. Preferably, the allelic variant is an allelic variant of SEQ ID NO: 92 or an allelic variant of a nucleic acid sequence encoding an orthologue or paralogue of SEQ ID NO: 93. Preferably, the allelic variant is an allelic variant of a polypeptide sequence having in increasing order of preference at least 50%, 55%, 60%, 65%, 70%, 75%, 80%, 85%, 90%, 95%, 98%, 99% or more amino acid sequence identity to the FATB polypeptide as represented by SEQ ID NO: 93 or to any of the polypeptide sequences given in Table A3 of Example 1 herein.

Concerning LFY-like polypeptides, the polypeptides encoded by allelic variants useful in the methods of the present invention have substantially the same biological activity as the LFY-like polypeptide of SEQ ID NO: 146 and any of the amino acids depicted in Table A4 of Example 1. Allelic variants exist in nature, and encompassed within the methods of the present invention is the use of these natural alleles. Preferably, the allelic variant is an allelic variant of SEQ ID NO: 145 or an allelic variant of a nucleic acid sequence encoding an orthologue or paralogue of SEQ ID NO: 146. Preferably, the amino acid sequence encoded by the allelic variant, when used in the construction of a phylogenetic tree, such as the one depicted in Figure 16, clusters with the group of LFY-like polypeptides.

Gene shuffling or directed evolution may also be used to generate variants of nucleic acid sequences encoding GS1 polypeptides, or PEAMT polypeptides, or FATB polypeptides, or LFY-like polypeptides, as defined above; the term "gene shuffling" being as defined herein.

According to the present invention, there is provided a method for enhancing yield-related traits in plants, comprising introducing and expressing in a plant a variant of any one of the nucleic acid sequences given in Table A1 to A4 of Example 1, or comprising introducing and expressing in a plant a variant of a nucleic acid sequence encoding an orthologue, paralogue or homologue of any of the amino acid sequences given in Table A1 to A4 of Example 1, which variant nucleic acid sequence is obtained by gene shuffling.

Concerning GS1 polypeptides, preferably, the amino acid sequence encoded by the variant nucleic acid sequence obtained by gene shuffling, when used in the construction of a phylogenetic tree such as the one depicted in Figure 3a and 3b, clusters with the algal-type clade (the group of algal GS1 polypeptides comprising the amino acid sequence represented by SEQ ID NO: 2) rather than with the plant chloroplastic or plant cytosolic glutamine synthase group.

Concerning PEAMT polypeptides, preferably, the amino acid sequence encoded by the variant nucleic acid sequence obtained by gene shuffling, when used in the construction of a phylogenetic tree such as the one depicted in Figure 6, clusters with the group I of PEAMT polypeptides comprising the amino acid sequence represented by SEQ ID NO: 58 rather than with any other group.

Concerning FATB polypeptides, preferably, the variant nucleic acid sequence obtained by gene shuffling encodes a polypeptide sequence having in increasing order of preference at least 50%, 55%, 60%, 65%, 70%, 75%, 80%, 85%, 90%, 95%, 98%, 99% or more amino acid sequence identity to the FATB polypeptide as represented by SEQ ID NO: 93 or to any of the polypeptide sequences given in Table A3 herein.

Concerning LFY-like polypeptides, preferably, the amino acid sequence encoded by the variant nucleic acid sequence obtained by gene shuffling, when used in the construction of a phylogenetic tree, such as the one depicted in Figure 16, clusters with the group of LFY-like polypeptides.

Furthermore, nucleic acid sequence variants may also be obtained by site-directed mutagenesis. Several methods are available to achieve site-directed mutagenesis, the most common being PCR based methods (Current Protocols in Molecular Biology. Wiley Eds.).

Nucleic acid sequences encoding GS1 polypeptides may be derived from any natural or artificial source. The nucleic acid sequence may be modified from its native form in composition and/or genomic environment through deliberate human manipulation. Preferably the GS1 polypeptide-encoding nucleic acid sequence is from the division of the Chlorophyta, further preferably from the class of the Chlorophyceae, more preferably from the family Chlamydomonadaceae, most preferably the nucleic acid sequence is from *Chlamydomonas reinhardtii.*

Nucleic acid sequences encoding PEAMT polypeptides may be derived from any natural or artificial source. The nucleic acid sequence may be modified from its native form in composition and/or genomic environment through deliberate human manipulation. Preferably the PEAMT polypeptide-encoding nucleic acid sequence is from a plant, further preferably from a dicotyledonous plant, more preferably from the family Brasicaceae, most preferably the nucleic acid sequence is from Arabidopsis thaliana.

Advantageously, the present invention provides hitherto unknown PEAMT nucleic acid sequence and polypeptide sequences.

According to a further embodiment of the present invention, there is provided an isolated PEAMT nucleic acid sequence molecule comprising at least 98 % sequence identity to SEQ ID NO: 57.

Additionally an isolated polypeptide comprising at least 99 % sequence identity to SEQ ID NO: 58, is provided.

Nucleic acid sequences encoding FATB polypeptides, or LFY-like polypeptides may be derived from any natural or artificial source. The nucleic acid sequence may be modified from its native form in composition and/or genomic environment through deliberate human manipulation. The nucleic acid sequence encoding a FATB polypeptide or a LFY-like polypeptide, is from a plant, further preferably from a dicotyledonous plant, more preferably from the family Brassicaceae, most preferably the nucleic acid sequence is from *Arabidopsis thaliana.*

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing yield, especially seed yield of plants, relative to control plants, which method comprises modulating expression in a plant of a nucleic acid sequence encoding a GS1 polypeptide, or a PEAMT polypeptide, or a FATB polypeptide, or a LFY-like polypeptide, as defined herein.

The present invention provides a method for increasing yield, especially seed yield of plants, relative to control plants, which method comprises modulating expression in a plant of a nucleic acid sequence encoding a GS1 polypeptide, or a PEAMT polypeptide, or a LFY-like polypeptide, as defined herein.

The present invention also provides a method for increasing seed yield-related traits of plants relative to control plants, which method comprises increasing expression in a plant of a nucleic acid sequence encoding a FATB polypeptide as defined herein.

Since the transgenic plants according to the present invention have increased yield and/or increased seed yield-related traits, it is likely that these plants exhibit an increased growth rate (during at least part of their life cycle), relative to the growth rate of control plants at a corresponding stage in their life cycle. However, concerning LFY-like polypeptides, no earlier induction of flowering time was observed.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating and/or increasing expression in a plant of a nucleic acid sequence encoding a GS1 polypeptide, or a PEAMT polypeptide, or a FATB polypeptide, or a LFY-like polypeptide, as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects.

Increased seed yield-related traits occur whether the plant is under non-stress conditions or whether the plant is exposed to various stresses compared to control plants grown under comparable conditions. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes, and insects. The term "non-stress" conditions as used herein are those environmental conditions that allow optimal growth of plants. Persons skilled in the art are aware of normal soil conditions and climatic conditions for a given location.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 90%, 87%, 85%, 83%, 80%, 77% or 75% of the average production of such plant in a given environment. Average production may be calculated on harvest and/or season basis. Persons skilled in the art are aware of average yield productions of a crop.

Concerning GS1 polypeptides performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid sequence encoding a GS1 polypeptide.

Concerning PEAMT polypeptides, performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid sequence encoding a PEAMT polypeptide.

Concerning FATB polypeptides, performance of the methods of the invention gives plants grown under non-stress conditions or under mild stress conditions having increased seed yield-related traits, relative to control plants grown under comparable conditions. Therefore, according to the present invention, there is provided a method for increasing seed yield-related traits in plants grown under non-stress conditions or under mild stress conditions, which method comprises increasing expression in a plant of a nucleic acid sequence encoding a FATB polypeptide.

Concerning LFY-like polypeptides, performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid sequence encoding a LFY-like polypeptide.

Concerning GS1 polypeptides performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid sequence encoding a GS1 polypeptide. Nutrient deficiency may result from a lack of nutrients such as nitrogen, phosphates and other phosphorous-containing compounds, potassium, calcium, cadmium, magnesium, manganese, iron and boron, amongst others. In a particular embodiment of the present invention, there is provided a method for increasing yield in plants grown under conditions of nitrogen deficiency, which method comprises modulating expression in a plant of a nucleic acid sequence encoding a GS1 polypeptide.

Concerning GS1 polypeptides performance of the methods of the invention gives plants grown under conditions of salt stress, increased yield relative to control plants grown under comparable conditions. Therefore, according to the present invention, there is provided a method for increasing yield in plants grown under conditions of salt stress, which method comprises modulating expression in a plant of a nucleic acid sequence encoding a GS1 polypeptide. The term salt stress is not restricted to common salt (NaCl), but may be any one or more of: NaCl, KCl, LiCl, $MgCl_2$, $CaCl_2$, amongst others.

Concerning PEAMT polypeptides, performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid sequence encoding a PEAMT polypeptide. Nutrient deficiency may result from a lack of nutrients such as nitrogen, phosphates and other phosphorous-containing compounds, potassium, calcium, cadmium, magnesium, manganese, iron and boron, amongst others.

Concerning FATB polypeptides, performance of the methods according to the present invention results in plants grown under abiotic stress conditions having increased seed yield-related traits relative to control plants grown under comparable stress conditions. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. Since diverse environmental stresses activate similar pathways, the exemplification of the present invention with drought stress should not be seen as a limitation to drought stress, but more as a screen to indicate the involvement of FATB polypeptides as defined above, in increasing seed yield-related traits relative to control plants grown in comparable stress conditions, in abiotic stresses in general.

The term "abiotic stress" as defined herein is taken to mean any one or more of: water stress (due to drought or excess water), anaerobic stress, salt stress, temperature stress (due to hot, cold or freezing temperatures), chemical toxicity stress and oxidative stress. According to one aspect of the invention, the abiotic stress is an osmotic stress, selected from water stress, salt stress, oxidative stress and ionic stress. Preferably, the water stress is drought stress. The term salt stress is not restricted to common salt (NaCl), but may be any stress caused by one or more of: NaCl, KCl, LiCl, $MgCl_2$, $CaCl_2$, amongst others.

Concerning FATB polypeptides, performance of the methods of the invention gives plants having increased seed yield-related traits, under abiotic stress conditions relative to control plants grown in comparable stress conditions. Therefore, according to the present invention, there is provided a method for increasing seed yield-related traits, in plants grown under abiotic stress conditions, which method comprises increasing expression in a plant of a nucleic acid sequence encoding a FATB polypeptide. According to one aspect of the invention, the abiotic stress is an osmotic stress, selected from one or more of the following: water stress, salt stress, oxidative stress and ionic stress.

Another example of abiotic environmental stress is the reduced availability of one or more nutrients that need to be assimilated by the plants for growth and development. Because of the strong influence of nutrition utilization efficiency on plant yield and product quality, a huge amount of fertilizer is poured onto fields to optimize plant growth and quality. Productivity of plants ordinarily is limited by three primary nutrients, phosphorous, potassium and nitrogen, which is usually the rate-limiting element in plant growth of these three. Therefore the major nutritional element required for plant growth is nitrogen (N). It is a constituent of numerous important compounds found in living cells, including amino acids, proteins (enzymes), nucleic acid sequences, and chlorophyll. 1.5% to 2% of plant dry matter is nitrogen and approximately 16% of total plant protein. Thus, nitrogen availability is a major limiting factor for crop plant growth and production (Frink et al. (1999) Proc Natl Acad Sci USA 96(4): 1175-1180), and has as well a major impact on protein accumulation and amino acid composition. Therefore, of great interest are crop plants with increased seed yield-related traits, when grown under nitrogen-limiting conditions.

Concerning FATB polypeptides, performance of the methods of the invention gives plants grown under conditions of reduced nutrient availability, particularly under conditions of reduced nitrogen availablity, having increased seed yield-related traits relative to control plants grown under comparable conditions. Therefore, according to the present invention, there is provided a method for increasing seed yield-related traits in plants grown under conditions of reduced nutrient availablity, preferably reduced nitrogen availability, which method comprises increasing expression in a plant of a nucleic acid sequence encoding a FATB polypeptide. Reduced nutrient availability may result from a deficiency or excess of nutrients such as nitrogen, phosphates and other phosphorous-containing compounds, potassium, calcium, cadmium, magnesium, manganese, iron and boron, amongst others. Preferably, reduced nutrient availablity is reduced nitrogen availability.

Concerning LFY-like polypeptides, performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid sequence encoding a LFY-like polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants or parts thereof (including seeds) or cells obtainable by the methods according to the present invention. The plants or parts or cells thereof comprise a nucleic acid sequence transgene encoding a GS1 polypeptide, or a PEAMT polypeptide, or a FATB polypeptide, or a LFY-like polypeptide, as defined above.

The invention also provides genetic constructs and vectors to facilitate introduction and/or expression in plants of nucleic acid sequences encoding GS1 polypeptides, or PEAMT polypeptides, or FATB polypeptides, or LFY-like polypeptides, as defined herein. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid sequence encoding a GS1 polypeptide, or a PEAMT polypeptide, or a FATB polypeptide, or a LFY-like polypeptide, as defined above;
(b) one or more control sequences capable of driving expression of the nucleic acid sequence of (a); and optionally
(c) a transcription termination sequence.

Preferably, the nucleic acid sequence encoding a GS1 polypeptide, or a PEAMT polypeptide, or a FATB polypeptide, or a LFY-like polypeptide, is as defined above. The term "control sequence" and "termination sequence" are as defined herein.

Plants are transformed with a vector comprising any of the nucleic acid sequences described above. The skilled artisan is well aware of the genetic elements that must be present on the vector in order to successfully transform, select and propagate host cells containing the sequence of interest. The sequence of interest is operably linked to one or more control sequences (at least to a promoter).

Concerning FATB, preferably, one of the control sequences of a construct is a constitutive promoter isolated from a plant genome. An example of a plant constitutive promoter is a GOS2 promoter, preferably a rice GOS2 promoter, more preferably a GOS2 promoter as represented by SEQ ID NO: 144.

Concerning GS1, advantageously, any type of promoter, whether natural or synthetic, may be used to drive expression of the nucleic acid sequence, but preferably the promoter is of plant origin. A promoter capable of driving expression in shoots, and in particular in green tissue, is particularly useful in the methods. See the "Definitions" section herein for definitions of the various promoter types.

Concerning PEAMT, advantageously, any type of promoter, whether natural or synthetic, may be used to drive expression of the nucleic acid sequence, but preferably the promoter is of plant origin. A constitutive promoter is particularly useful in the methods. Preferably the constitutive promoter is also a ubiquitous promoter of medium strength. See the "Definitions" section herein for definitions of the various promoter types.

Concerning FATB, advantageously, any type of promoter, whether natural or synthetic, may be used to increase expression of the nucleic acid sequence. A constitutive promoter is particularly useful in the methods, preferably a constitutive promoter isolated from a plant genome. The plant constitutive promoter drives expression of a coding sequence at a level that is in all instances below that obtained under the control of a 35S CaMV viral promoter.

Also concerning FATB, organ-specific promoters, for example for preferred expression in leaves, stems, tubers, meristems, are useful in performing the methods of the invention. Developmentally-regulated promoters are also useful in performing the methods of the invention See the "Definitions" section herein for definitions of the various promoter types.

Concerning LFY-like, advantageously, any type of promoter, whether natural or synthetic, may be used to drive expression of the nucleic acid sequence, but preferably the promoter is of plant origin. A constitutive promoter is particularly useful in the methods. Preferably the constitutive promoter is also a ubiquitous promoter of medium strength. See the "Definitions" section herein for definitions of the various promoter types. Also useful in the methods of the invention is a shoot-specific (or green-tissue specific) promoter.

Concerning GS1 polypeptides, It should be clear that the applicability of the present invention is not restricted to the GS1 polypeptide-encoding nucleic acid sequence represented by SEQ ID NO: 1, nor is the applicability of the invention restricted to expression of a GS1 polypeptide-encoding nucleic acid sequence when driven by a shoot-specific promoter.

The shoot-specific promoter preferntially, drives expression in green tissue, further preferably the shoot-specific promoter is isolated from a plant, such as a protochlorophyllide reductase promoter (pPCR), more preferably the protochlorophyllide reductase promoter is from rice. Further preferably the protochlorophyllide reductase promoter is represented by a nucleic acid sequence substantially similar to SEQ ID NO: 6, most preferably the constitutive promoter is as represented by SEQ ID NO: 6. See the "Definitions" section herein for further examples of green-tissue specific promoters.

Concerning GS1 polypeptides, optionally, one or more terminator sequences may be used in the construct introduced into a plant. Preferably, the construct comprises an expression cassette comprising a protochlorophyllide reductase promoter, substantially similar to SEQ ID NO: 6, and the nucleic acid encoding the GS1 polypeptide.

Concerning PEAMT polypeptides, it should be clear that the applicability of the present invention is not restricted to the PEAMT polypeptide-encoding nucleic acid sequence represented by SEQ ID NO: 57, nor is the applicability of the invention restricted to expression of a PEAMT polypeptide-encoding nucleic acid sequence when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 85, most preferably the constitutive promoter is as represented by SEQ ID NO: 85. See the "Definitions" section herein for further examples of constitutive promoters.

Concerning PEAMT polypeptides, optionally, one or more terminator sequences may be used in the construct introduced into a plant. Preferably, the construct comprises an expression cassette comprising a GOS2 promoter, substantially similar to SEQ ID NO: 85, and the nucleic acid encoding the PEAMT polypeptide.

Concerning FATB polypeptides, it should be clear that the applicability of the present invention is not restricted to a nucleic acid sequence encoding the FATB polypeptide, as represented by SEQ ID NO: 92, nor is the applicability of the invention restricted to expression of a FATB polypeptide-encoding nucleic acid sequence when driven by a constitutive promoter.

Optionally, one or more terminator sequences may be used in the construct introduced into a plant. Additional regulatory elements may include transcriptional as well as translational increasers. Those skilled in the art will be aware of terminator and increaser sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, increaser, silencer, intron sequences, 3'UTR and/or 5'UTR regions) may be protein and/or RNA stabilizing elements. Such sequences would be known or may readily be obtained by a person skilled in the art.

Concerning LFY-like polypeptides, it should be clear that the applicability of the present invention is not restricted to the LFY-like polypeptide-encoding nucleic acid represented by SEQ ID NO: 145, nor is the applicability of the invention restricted to expression of a LFY-like polypeptide-encoding nucleic acid when driven by a constitutive promoter, or when driven by a shoot-specific promoter.

The constitutive promoter is preferably a medium strength promoter, such as a GOS2 promoter, preferably the promoter is a GOS2 promoter from rice. Further preferably the constitutive promoter is represented by a nucleic acid sequence substantially similar to SEQ ID NO: 149, most preferably the constitutive promoter is as represented by SEQ ID NO: 149. See Table 2a in the "Definitions" section herein for further examples of constitutive promoters.

Concerning LFY-like polypeptides, according to another preferred feature of the invention, the nucleic acid encoding a LFY-like polypeptide is operably linked to a shoot-specific (or green-tissue specific) promoter. The shoot-specific promoter is preferably a protochlorophyllid reductase promoter, more preferably the protochlorophyllid reductase promoter is from rice, further preferably the protochlorophyllid reductase promoter is represented by a nucleic acid sequence substantially similar to SEQ ID NO: 150, most preferably the promoter is as represented by SEQ ID NO: 150. Examples of other shoot-specific promoters which may also be used to perform the methods of the invention are shown in Table 2b in the "Definitions" section above.

Concerning LFY-like polypeptides, optionally, one or more terminator sequences may be used in the construct introduced into a plant. Preferably, the construct comprises an expression cassette comprising the GOS2 promoter, or the protochlorophyllid reductase promoter, operably linked to the nucleic acid encoding the LFY-like polypeptide.

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

It is known that upon stable or transient integration of nucleic acid sequences into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid sequence molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid sequence can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

The invention also provides a method for the production of transgenic plants having enhanced yield-related traits relative to control plants, comprising introduction and expression in a plant of any nucleic acid encoding a GS1 polypeptide, or a PEAMT polypeptide, or a LFY-like polypeptide, as defined hereinabove.

More specifically, the present invention provides a method for the production of transgenic plants having enhanced yield-related traits, particularly increased (seed) yield, which method comprises:

(i) introducing and expressing in a plant or plant cell a GS1 polypeptide-encoding, or a PEAMT polypeptide-encoding, or a LFY-like polypeptide-encoding nucleic acid sequence; and
(ii) cultivating the plant cell under conditions promoting plant growth and development.

The nucleic acid of (i) may be any of the nucleic acids capable of encoding a GS1 polypeptide, or a PEAMT polypeptide, or a LFY-like polypeptide, as defined herein.

The invention also provides a method for the production of transgenic plants having increased seed yield-related traits relative to control plants, comprising introduction and expression in a plant of any nucleic acid sequence encoding a FATB polypeptide as defined hereinabove.

More specifically, the present invention provides a method for the production of transgenic plants having increased seed yield-related traits relative to control plants, which method comprises:

(i) introducing and expressing in a plant, plant part, or plant cell a nucleic acid sequence encoding a FATB polypeptide; and

(ii) cultivating the plant cell, plant part or plant under conditions promoting plant growth and development.

The nucleic acid sequence of (i) may be any of the nucleic acid sequences capable of encoding a FATB polypeptide as defined herein.

The nucleic acid sequence may be introduced directly into a plant cell or into the plant itself (including introduction into a tissue, organ or any other part of a plant). According to a preferred feature of the present invention, the nucleic acid sequence is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing an isolated nucleic acid sequence encoding a GS1 polypeptide, or a PEAMT polypeptide, or a LFY-like polypeptide, as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

Furthermore, the invention also includes host cells containing an isolated nucleic acid sequence encoding a FATB polypeptide as defined hereinabove, opereably linked to a constitutive promoter. Preferred host cells according to the invention are plant cells. Host plants for the nucleic acid sequences or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid sequence encoding a GS1 polypeptide, or a PEAMT polypeptide, or a LFY-like polypeptide. The invention furthermore relates to products derived, preferably directly derived, from a harvestable part of such a plant, such as dry pellets or powders, oil, fat and fatty acids, starch or proteins.

Furthermore, the invention also extends to harvestable parts of a plant comprising an isolated nucleic acid sequence encoding a FATB (as defined hereinabove) operably linked to a constitutive promoter, such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids sequences or genes, or gene products, are well documented in the art and examples are provided in the definitions section.

As mentioned above, a preferred method for modulating expression of a nucleic acid sequence encoding a GS1 polypeptide, or a PEAMT polypeptide, or a FATB polypeptide, or a LFY-like polypeptide, is by introducing and expressing in a plant a nucleic acid encoding a GS1 polypeptide, or a PEAMT polypeptide, or a FATB polypeptide, or a LFY-like polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The present invention also encompasses use of nucleic acid sequences encoding GS1 polypeptides, or PEAMT polypeptides, or LFY-like polypeptides, as described herein and use of these GS1 polypeptides, or PEAMT polypeptides, or LFY-like polypeptides, in enhancing any of the aforementioned yield-related traits in plants.

Furthermore, the present invention also encompasses use of nucleic acid sequences encoding FATB polypeptides as described herein and use of these FATB polypeptides in increasing any of the aforementioned seed yield-related traits in plants, under normal growth conditions, under abiotic stress growth (preferably osmotic stress growth conditions) conditions, and under growth conditions of reduced nutrient availability, preferably under conditions of reduced nitrogen availability.

Concerning GS1 polypeptides, nucleic acid sequences encoding GS1 polypeptides, or PEAMT polypeptides, or LFY-like polypetides, described herein, or the GS1 polypeptides themselves, may find use in breeding programmes in which a DNA marker is identified which may be genetically linked to gene encoding a GS1 polypeptide, or a PEAMT polypeptide, or a LFY-like polypeptide. The nucleic acids/genes, or the GS1 polypeptides themselves, or the PEAMT polypeptides themselves, or the LFY-like polypeptides, may be used to define a molecular marker. This DNA or protein marker may then be used in breeding programmes to select plants having enhanced yield-related traits as defined hereinabove in the methods of the invention.

Concerning FATB polypeptides, nucleic acid sequences encoding FATB polypeptides described herein, or the FATB polypeptides themselves, may find use in breeding programmes in which a DNA marker is identified that may be genetically linked to a FATB polypeptide-encoding gene. The genes/ nucleic acid sequences, or the FATB polypeptides themselves may be used to define a molecular marker. This DNA or protein marker may then be used in breeding programmes to select plants having increased seed yield-related traits, as defined hereinabove in the methods of the invention.

Allelic variants of a gene/nucleic acid seqeunce encoding a GS1 polypeptide, or a PEAMT polypeptide, or a FATB polypeptide, or a LFY-like polypeptide, may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acid sequences encoding GS1 polypeptides, or PEAMT polypeptides, or FATB polypeptides, or LFY-like polypeptides, may also be used as probes for genetically and physically mapping the genes that they are a part of, and as markers for traits linked to those genes. Such information may be useful in plant breeding in order to develop lines with desired phenotypes. Such use of nucleic acid sequences encoding GS1 polypeptides, or PEAMT polypeptides, or FATB polypeptides, or LFY-like polypeptides, requires only a nucleic acid sequence of at least 15 nucleotides in length. The nucleic acid sequences encoding GS1 polypeptides, or PEAMT polypeptides, or FATB polypeptides, or LFY-like polypeptides, may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the GS1-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid sequence encoding GS1 polypeptides, or PEAMT polypeptides, or FATB polypeptides, or LFY-like polypeptides, in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid sequence probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid sequence probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid sequence amplification-based methods for genetic and physical mapping may be carried out using the nucleic acid sequences. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic acid sequence Res. 18:3671), Radiation Hybrid Mapping (Walter et al. (1997) Nat. Genet. 7:22-28) and Happy Mapping (Dear and Cook (1989) Nucleic acid sequence Res. 17:6795-6807). For these methods, the sequence of a nucleic acid sequence is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related or enhanced seed-yield related traits, as described hereinbefore. These traits may also be combined with other economically advantageous traits, such as further yield-enhancing traits, tolerance to other abiotic and biotic stresses, traits modifying various architectural features and/or biochemical and/or physiological features.

**Items**

1. A method for enhancing yield-related traits in plants relative to control plants, comprising modulating expression in a plant of a nucleic acid encoding an algal-type cytoplasmic glutamine synthase (GS1) polypeptide, wherein said algal-type GS1 polypeptide comprises a Gln-synt_C domain (Pfam accession PF00120) and a Gln-synt_N domain (Pfam accession PF03951).

2. Method according to item 1, wherein said GS1 polypeptide comprises one or more of the following motifs:

(a) Motif 1, SEQ ID NO: 3;
(b) Motif 2, SEQ ID NO: 4;
(c) Motif 3, SEQ ID NO: 5,

in which motifs maximally 2 mismatches are allowed.

3. Method according to item 1 or 2, wherein said modulated expression is effected by introducing and expressing in a plant a nucleic acid encoding an algal-type GS1 polypeptide.

4. Method according to any of items 1 to 3, wherein said nucleic acid encoding a GS1 polypeptide encodes any one of the proteins listed in Table A1 or is a portion of such a nucleic acid, or a nucleic acid capable of hybridising with such a nucleic acid.
5. Method according to any of items 1 to 4, wherein said nucleic acid sequence encodes an orthologue or paralogue of any of the proteins given in Table A1.
6. Method according to any of items 1 to 5, wherein said enhanced yield-related traits comprise increased yield, preferably increased biomass and/or increased seed yield relative to control plants.
7. Method according to any one of items 1 to 6, wherein said enhanced yield-related traits are obtained under conditions of nutrient deficiency.
8. Method according to any one of items 3 to 7, wherein said nucleic acid is operably linked to a shoot-specific promoter, preferably to a protochlorophyllide reductase promoter, most preferably to a protochlorophyllide reductase promoter from rice.
9. Method according to any of items 1 to 8, wherein said nucleic acid encoding a GS1 polypeptide is of plant origin, preferably from a alga, further preferably from the class of Chlorophyceae, more preferably from the family Chlamydomonadaceae, most preferably from *Chlamydomonas reinhardtii.*
10. Plant or part thereof, including seeds, obtainable by a method according to any of items 1 to 9, wherein said plant or part thereof comprises a recombinant nucleic acid encoding a GS1 polypeptide.
11. Construct comprising:

(i) nucleic acid encoding a GS1 polypeptide as defined in items 1 or 2;
(ii) one or more control sequences capable of driving expression of the nucleic acid sequence of (a); and optionally
(iii) a transcription termination sequence.

12. Construct according to item 11, wherein one of said control sequences is a shoot-specific promoter, preferably a protochlorophyllide reductase promoter, most preferably a protochlorophyllide reductase promoter from rice.
13. Use of a construct according to item 11 or 12 in a method for making plants having increased yield, particularly increased biomass and/or increased seed yield relative to control plants.
14. Plant, plant part or plant cell transformed with a construct according to item 11 or 12.
15. Method for the production of a transgenic plant having increased yield, particularly increased biomass and/or increased seed yield relative to control plants, comprising:

(i) introducing and expressing in a plant a nucleic acid encoding a GS1 polypeptide as defined in item 1 or 2; and
(ii) cultivating the plant cell under conditions promoting plant growth and development.

16. Transgenic plant having increased yield, particularly increased biomass and/or increased seed yield, relative to control plants, resulting from modulated expression of a nucleic acid encoding a GS1 polypeptide as defined in item 1 or 2, or a transgenic plant cell derived from said transgenic plant.
17. Transgenic plant according to item 10, 14 or 16, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.
18. Harvestable parts of a plant according to item 17, wherein said harvestable parts are preferably shoot biomass and/or seeds.
19. Products derived from a plant according to item 17 and/or from harvestable parts of a plant according to item 18.
20. Use of a nucleic acid encoding a GS1 polypeptide in increasing yield, particularly in increasing seed yield and/or shoot biomass in plants, relative to control plants.
21. An isolated polypeptide selected from:

(i) an amino acid sequence represented by SEQ ID NO: 53 or 54;
(ii) an amino acid sequence having, in increasing order of preference, at least 50%, 55%, 60%, 65%, 70%, 75%, 80%, 85%, 90%, 95%, 96%, 97%, 98%, 99% or more sequence identity to the amino acid sequence represented by SEQ ID NO: 53 or 54,
(iii) derivatives of any of the amino acid sequences given in (i) or (ii) above.

22. An isolated nucleic acid encoding a polypeptide as defined in item 22, or a nucleic acid hybridising thereto.
23. A method for enhancing yield-related traits in plants relative to that of control plants, comprising modulating expression in a plant of a nucleic acid encoding a PEAMT polypeptide or a homologue thereof comprising a protein domain having in increasing order of preference at least 60%, 61 %, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to anyone of the protein domains set forth in Table C2.

24. Method according to item 23, wherein the nucleic acid encodes a PEAMT polypeptide or a homologue thereof having in increasing order of preference at least 50%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid sequence represented by SEQ ID NO: 58.

25. Method according to item 23 or 24, wherein said nucleic acid encoding a PEAMT polypeptide or a homologue thereof is a portion of the nucleic acid represented by SEQ ID NO: 57, or is a portion of a nucleic acid encoding an orthologue or paralogue of the amino acid sequence of SEQ ID NO: 58, wherein the portion is at least 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, consecutive nucleotides in length, the consecutive nucleotides being of SEQ ID NO: 57, or of a nucleic acid encoding an orthologue or paralogue of the amino acid sequence of SEQ ID NO: 58.

26. Method according to any one of items 23 to 25, wherein the nucleic acid encoding a PEAMT polypeptide or a homologue thereof is capable of hybridising to the nucleic acid represented by SEQ ID NO: 1 or is capable of hybridising to a nucleic acid encoding an orthologue, paralogue or homologue of SEQ ID NO: 58.

27. Method according to any one of items 23 to 26, wherein said nucleic acid encoding a PEAMT polypeptide or a homologue thereof encodes an orthologue or paralogue of the sequence represented by SEQ ID NO: 58.

28. Method according to any one of items 23 to 27, wherein said modulated expression is effected by introducing and expressing in a plant a nucleic acid encoding a PEAMT polypeptide or a homologue thereof.

29. Method according to any one of items 23 to 28, wherein said enhanced yield-related traits comprising increased yield, preferably increased biomass and/or increased seed yield relative to control plants is obtained under non-stress conditions.

30. Method according to any one of items 23 to 29, wherein said enhanced yield-related traits comprising increased yield, preferably increased biomass and/or increased seed yield relative to control plants is obtained under conditions of drought stress.

31. Method according to item 28, 29 or 30 wherein said nucleic acid is operably linked to a constitutive promoter, preferably to a GOS2 promoter, most preferably to a GOS2 promoter from rice.

32. Method according to any one of items 23 to 31, wherein said nucleic acid encoding a PEAMT polypeptide is of plant origin, preferably from a dicotyledonous plant, further preferably from the family Brassicaceae, more preferably from the genus Arabidopsis, most preferably from *Arabidopsis thaliana.*

33. Plant or part thereof, including seeds, obtainable by a method according to any preceding item, wherein said plant or part thereof comprises a recombinant nucleic acid encoding a PEAMT polypeptide or a homologue thereof.

34. An isolated nucleic acid molecule comprising at least 98 % sequence identity to SEQ ID NO: 57.

35. An isolated polypeptide comprising at least 99 % sequence identity to SEQ ID NO: 58.

36. Construct comprising:

(i) A nucleic acid encoding a PEAMT polypeptide or a homologue thereof as defined in any of items 23 to 27 and items 34 and 35;
(ii) one or more control sequences capable of driving expression of the nucleic acid sequence of (a); and optionally
(iii) a transcription termination sequence.

37. Construct according to item 36, wherein one of said control sequences is a constitutive promoter, preferably a GOS2 promoter, most preferably a GOS2 promoter from rice.

38. Use of a construct according to item 36 or 37 in a method for making plants having an altered yield-related traits relative to control plants.

39. Plant, plant part or plant cell transformed with a construct according to item 36 or 37.

40. Method for the production of a transgenic plant having an enhanced yield-related traits relative to control plants, comprising:

(i) introducing and expressing in a plant a nucleic acid encoding a PEAMT polypeptide or a homologue thereof as defined in any one of items 23 to 27 and items 34 and 35; and
(ii) cultivating the plant cell under conditions promoting plant growth and development.

41. Transgenic plant having enhanced yield-related traits relative to control plants, resulting from modulated expression of a nucleic acid encoding a PEAMT polypeptide or a homologue thereof as defined in any one of items 23 to 27 and items 34 and 35.

42. Transgenic plant according to item 33, 39 or 41, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

43. Products derived from a plant according to item 42.

44. Use of a nucleic acid encoding a PEAMT polypeptide or a homologue thereof in altering yield-related traits of plants relative to control plants.

45. A method for increasing seed yield-related traits in plants relative to control plants, comprising increasing expression in a plant of a nucleic acid sequence encoding a fatty acyl-acyl carrier protein (ACP) thioesterase B (FATB) polypeptide, which FATB polypeptide comprises (i) a plastidic transit peptide; (ii) at least one transmembrane helix; (iii) and an acyl-ACP thioesterase family domain with an InterPro accession IPR002864, and optionally selecting for plants having increased seed yield-related traits.

46. Method according to item 45, wherein said FATB polypeptide has (i) a plastidic transit peptide; (ii) in increasing order of preference at least 50%, 55%, 60%, 65%, 70%, 75%, 80%, 85%, 90%, 95%, 98%, 99% or more amino acid sequence identity to a transmembrane helix as represented by SEQ ID NO: 141; and having in increasing order of preference at least 50%, 55%, 60%, 65%, 70%, 75%, 80%, 85%, 90%, 95%, 98%, 99% or more amino acid sequence identity to an acyl-ACP thioesterase family domain as represented by SEQ ID NO: 140.

47. Method according to item 45 or 46, wherein said FATB polypeptide has in increasing order of preference at least 50%, 55%, 60%, 65%, 70%, 75%, 80%, 85%, 90%, 95%, 98%, 99% or more amino acid sequence identity to the FATB polypeptide as represented by SEQ ID NO: 93 or to any of the polypeptide sequences given in Table A3 herein.

48. Method according to any of item 45 to 47, wherein said FATB polypeptide is any polypeptide sequence which when used in the construction of a FATs phylogenetic tree, such as the one depicted in Figure 10, clusters with the clade of FATB polypeptides comprising the polypeptide sequence as represented by SEQ ID NO: 93 rather than with the clade of FATA polypeptides.

49. Method according to any of item 45 to 48, wherein said FATB polypeptide is a polypeptide with enzymatic activity consisting in hydrolyzing acyl-ACP thioester bonds, preferentially from saturated acyl-ACPs (with chain lengths that vary between 8 and 18 carbons), releasing free fatty acids and acyl carrier protein (ACP).

50. Method according to any of item 45 to 49, wherein said nucleic acid sequence encoding a FATB polypeptide is represented by any one of the nucleic acid sequence SEQ ID NOs given in Table A3 or a portion thereof, or a sequence capable of hybridising with any one of the nucleic acid sequences SEQ ID NOs given in Table A3, or to a complement thereof.

51. Method according to any preceding item, wherein said nucleic acid sequence encodes an orthologue or paralogue of any of the polypeptide sequence SEQ ID NOs given in Table A3.

52. Method according to any preceding item, wherein said increased expression is effected by any one or more of: T-DNA activation tagging, TILLING, or homologous recombination.

53. Method according to any preceding item, wherein said increased expression is effected by introducing and expressing in a plant a nucleic acid sequence encoding a FATB polypeptide.

54. Method according to any preceding item, wherein said increased yield-related trait is one or more of: increased total seed yield per plant, increased total number of seeds, increased number of filled seeds, increased seed fill rate, and increased harvest index.

55. Method according to any preceding item, wherein said nucleic acid sequence is operably linked to a constitutive promoter.

56. Method according to item 55, wherein said constitutive promoter is a GOS2 promoter, preferably a rice GOS2 promoter, more preferably a GOS2 promoter as represented by SEQ ID NO: 144.

57. Method according to any preceding item, wherein said nucleic acid sequence encoding a FATB polypeptide is from a plant, further preferably from a dicotyledonous plant, more preferably from the family Brassicaceae, most preferably the nucleic acid sequence is from *Arabidopsis thaliana.*

58. Plants, parts thereof (including seeds), or plant cells obtainable by a method according to any preceding item, wherein said plant, part or cell thereof comprises an isolated nucleic acid transgene encoding a FATB polypeptide, operably linked to a constitutive promoter.

59. An isolated nucleic acid sequence comprising:

(i) a nucleic acid sequence as represented by SEQ ID NO: 130;
(ii) the complement of a nucleic acid sequence as represented by SEQ ID NO: 130;
(iii) a nucleic acid sequence encoding FATB polypeptide having, in increasing order of preference, at least 70%, 75%, 80%, 85%, 90%, 95%, 96%, 97%, 98%, 99% or more amino acid sequence identity to the polypeptide sequence as represented by SEQ ID NO: 131.

60. An isolated polypeptide comprising:

(i) a polypeptide sequence represented by SEQ ID NO: 131;
(ii) a polypeptide sequence having, in increasing order of preference, at least 70%, 75%, 80%, 85%, 90%, 95%, 96%, 97%, 98%, 99% or more sequence identity to the polypeptide sequence as represented by SEQ ID NO: 131;
(iii) derivatives of any of the polypeptide sequences given in (i) or (ii) above.

61. Construct comprising:

(a) a nucleic acid sequence encoding a FATB polypeptide as defined in any one of items 45 to 51;
(b) one or more control sequences capable of driving expression of the nucleic acid sequence of (a); and optionally
(c) a transcription termination sequence.

62. Construct according to item 61, wherein said control sequence is a constitutive promoter.
63. Construct according to item 60, wherein said constitutive promoter is a GOS2 promoter, preferably a rice GOS2 promoter, more preferably a GOS2 promoter as represented by SEQ ID NO: 144.
64. Use of a construct according to any one of items 61 to 63, in a method for making plants having increased seed yield-related traits relative to control plants, which increased seed yield-related traits are one or more of: increased total seed yield per plant, increased total number of seeds, increased number of filled seeds, increased seed fill rate, and increased harvest index.
65. Plant, plant part or plant cell transformed with a construct according to any one of items 61 to 63.
66. Method for the production of transgenic plants having increased seed yield-related traits relative to control plants, comprising:

(i) introducing and expressing in a plant, plant part, or plant cell, a nucleic acid sequence encoding a FATB polypeptide as defined in any one of items 45 to 51; and
(ii) cultivating the plant cell, plant part, or plant under conditions promoting plant growth and development.

67. Transgenic plant having increased seed yield-related traits relative to control plants, resulting from increased expression of a nucleic acid sequence encoding a FATB polypeptide as defined in any one of items 45 to 51, operably linked to a constitutive promoter, or a transgenic plant cell or transgenic plant part derived from said transgenic plant.
68. Transgenic plant according to item 58, 65 or 67, wherein said plant is a crop plant or a monocot or a cereal, such as rice, maize, wheat, barley, millet, rye, triticale, sorghum and oats, or a transgenic plant cell derived from said transgenic plant.
69. Harvestable parts comprising an isolated nucleic acid sequence encoding a FATB polypeptide of a plant according to item 68, wherein said harvestable parts are preferably seeds.
70. Products derived from a plant according to item 68 and/or from harvestable parts of a plant according to item 69.
71. Use of a nucleic acid sequence encoding a FATB polypeptide as defined in any one of items 45 to 51 in increasing seed yield-related traits, comprising one or more of increased increased total seed yield per plant, increased total number of seeds, increased number of filled seeds, increased seed fill rate, and increased harvest index.
72. A method for enhancing yield-related traits in plants relative to control plants, comprising modulating expression in a plant of a nucleic acid encoding a LFY-like polypeptide, wherein said LFY-like polypeptide comprises a FLO_LFY domain.
73. Method according to item 72, wherein said LFY-like polypeptide has at least 50% sequence identity to SEQ ID NO: 146.
74. Method according to item 72 or 73, wherein said modulated expression is effected by introducing and expressing in a plant a nucleic acid encoding a LFY-like polypeptide.
75. Method according to any one of items 72 to 74, wherein said nucleic acid encoding a LFY-like polypeptide encodes any one of the proteins listed in Table A4 or is a portion of such a nucleic acid, or a nucleic acid capable of hybridising with such a nucleic acid.
76. Method according to any one of items 72 to 75, wherein said nucleic acid sequence encodes an orthologue or paralogue of any of the proteins given in Table A4.
77. Method according to any one of items 72 to 76, wherein said enhanced yield-related traits comprise increased yield, preferably increased seed yield relative to control plants.
78. Method according to any one of items 72 to 77, wherein said enhanced yield-related traits are obtained under non-stress conditions.
79. Method according to any one of items 74 to 78, wherein said nucleic acid is operably linked to a constitutive promoter, preferably to a GOS2 promoter, most preferably to a GOS2 promoter from rice.

80. Method according to any one of items 72 to 79, wherein said nucleic acid encoding a LFY-like polypeptide is of plant origin, preferably from a dicotyledonous plant, further preferably from the family Brassicaceae, more preferably from the genus Arabidopsis, most preferably from Arabidopsis thaliana.
81. Plant or part thereof, including seeds, obtainable by a method according to any preceding item, wherein said plant or part thereof comprises a recombinant nucleic acid encoding a LFY-like polypeptide.
82. Construct comprising:

(i) nucleic acid encoding a LFY-like polypeptide as defined in items 72 or 73;
(ii) one or more control sequences capable of driving expression of the nucleic acid sequence of (a); and optionally
(iii) a transcription termination sequence.

83. Construct according to item 82, wherein one of said control sequences is a constitutive promoter, preferably a GOS2 promoter, most preferably a GOS2 promoter from rice.
84. Use of a construct according to item 82 or 83 in a method for making plants having increased yield, particularly increased seed yield relative to control plants.
85. Plant, plant part or plant cell transformed with a construct according to item 82 or 83.
86. Method for the production of a transgenic plant having increased yield, particularly increased seed yield relative to control plants, comprising:

(i) introducing and expressing in a plant a nucleic acid encoding a LFY-like polypeptide as defined in item 72 or 73; and
(ii) cultivating the plant cell under conditions promoting plant growth and development.

87. Transgenic plant having increased yield, particularly increased seed yield, relative to control plants, resulting from modulated expression of a nucleic acid encoding a LFY-like polypeptide as defined in item 72 or 73, or a transgenic plant cell derived from said transgenic plant.
88. Transgenic plant according to item 81, 85 or 87, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.
89. Harvestable parts of a plant according to item 88, wherein said harvestable parts are preferably seeds.
90. Products derived from a plant according to item 88 and/or from harvestable parts of a plant according to item 89.
91. Use of a nucleic acid encoding a LFY-like polypeptide in increasing yield, particularly in increasing seed yield in plants, relative to control plants.

## Description of figures

The present invention will now be described with reference to the following figures in which:

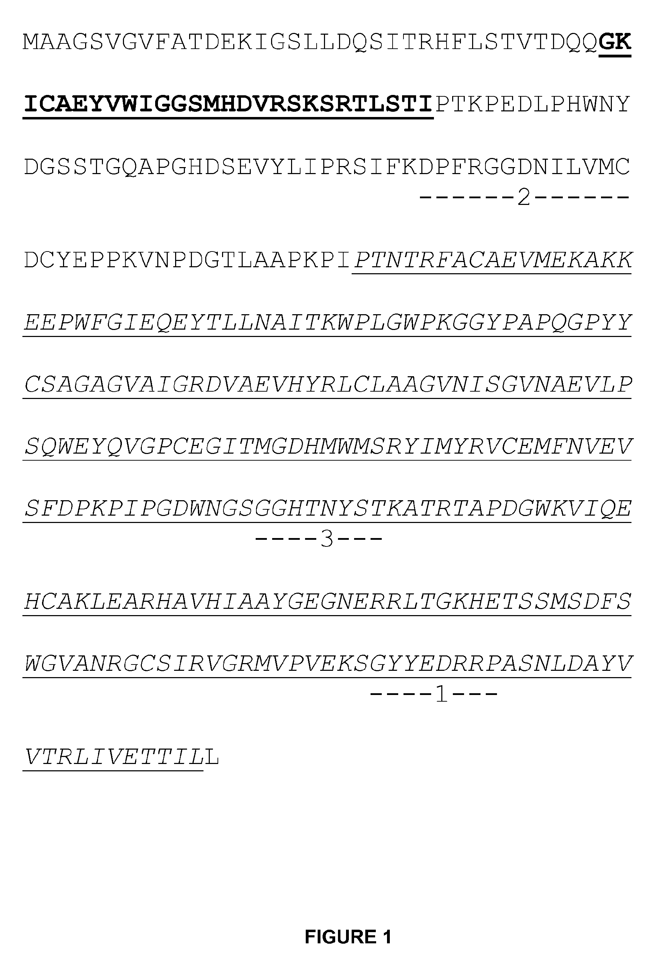

**Figure 1** represents the domain structure of SEQ ID NO: 2 with the Gln-synt_N domain (PF03951) shown in bold underlined, the Gln-synt_C domain (PF00120) shown in italics uncerlined and the conserved motifs 1 to 3 by the dashed line.
**Figure 2** represents a multiple alignment of algal GS1 protein sequences.
**Figure 3** shows phylogenetic trees of GS1 proteins. Panel a gives an overview of GS1 (cytosolic) and GS2 (chloroplastic) proteins in a circular phylogram. Panel b shows the sequences grouping in the algal group, with a few sequences of the cytosolic and cytoplasmic outgroups. The numbers in the tree of panel b correspond to the following SEQ ID NOs: (1) SEQ ID NO: 21, (2) SEQ ID NO: 26, (3) SEQ ID NO: 27, (4) SEQ ID NO: 10, (5) SEQ ID NO: 11, (6) SEQ ID NO: 15, (7) SEQ ID NO: 24, (8) SEQ ID NO: 25, (9) SEQ ID NO: 12, (10) SEQ ID NO: 2, (11) SEQ ID NO: 16, (12) SEQ ID NO: 13, (13) SEQ ID NO: 28, (14) SEQ ID NO: 14, (15) SEQ ID NO: 9, (16) SEQ ID NO: 17, (17) SEQ ID NO: 19, (18) SEQ ID NO: 22, (19) SEQ ID NO: 30, (20) SEQ ID NO: 18, (21) SEQ ID NO: 20, (22) SEQ ID NO: 23, (23) SEQ ID NO: 29.

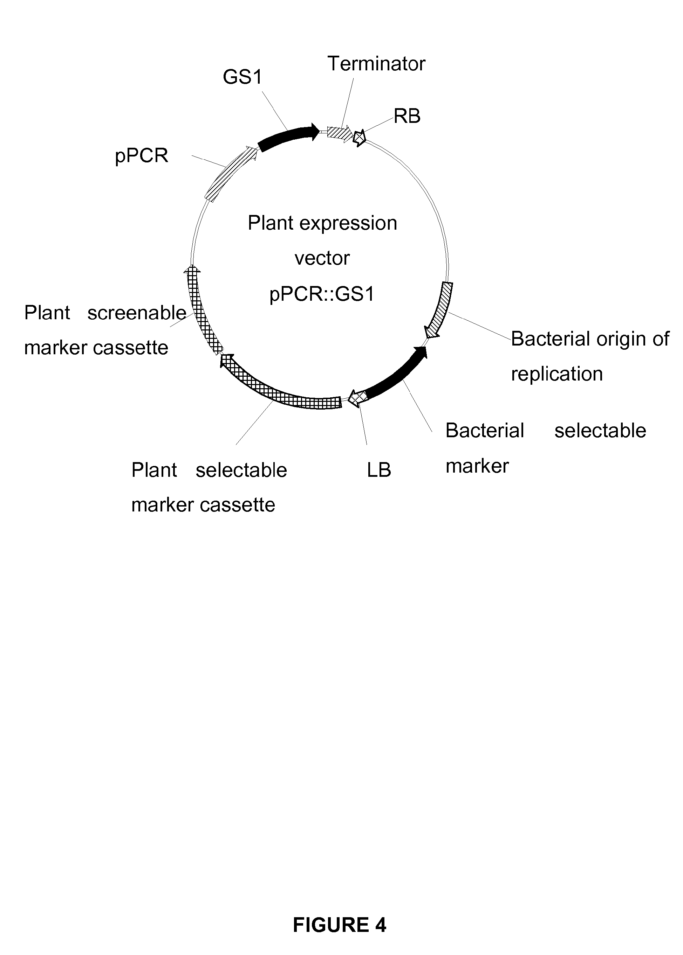

**Figure 4** represents the binary vector for increased expression in Oryza sativa of a GS1-encoding nucleic acid under the control of a rice protochlorophyllide reductase promoter (pPCR).
**Figure 5** represents a multiple alignment of the amino acid sequences of the PEAMT polypeptides of Table A2.
**Figure 6** represents a phylogenetic tree of the amino acid sequences of the PEAMT polypeptides of Table A2.
**Figure 7** represents the binary vector for increased expression in Oryza sativa of the Arath_PEAMT_1 encoding nucleic acid under the control of a rice GOS2 promoter (pGOS2)

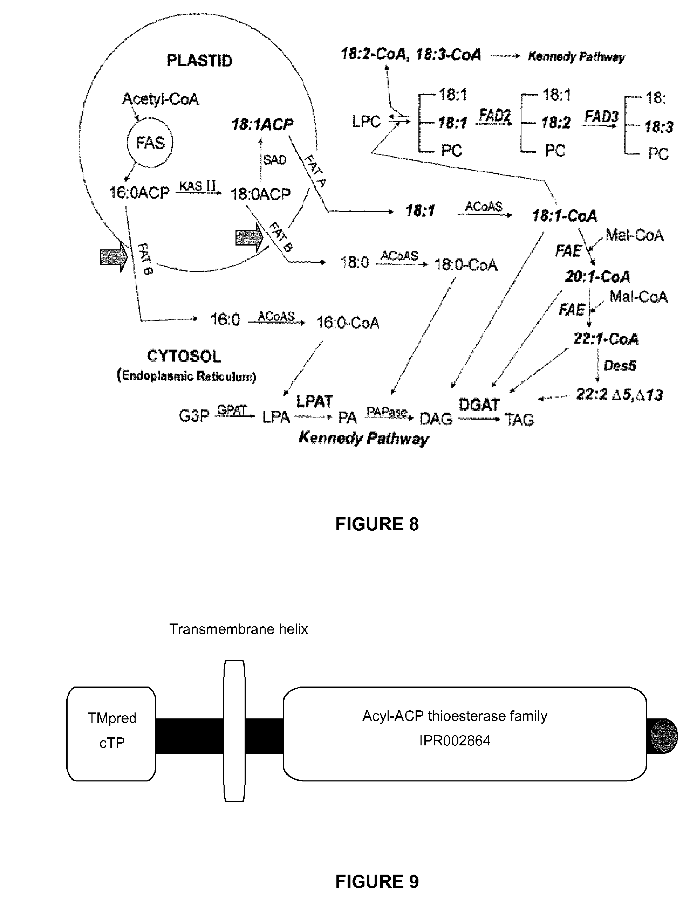

**Figure 8** schematically represents the general pathway for synthesis of various fatty acids (triacylglycerols; TAGs, synthesized via the Kennedy pathway) and steps normally involved for the production of seed storage lipids. The FATB polypeptides useful in performing the methods of the invention are shown with an arrow. According to Marillia et al. (2000) Developments in Plant Genetics and Breeding. Volume 5, 2000, Pages 182-188.

**Figure 9** represents a cartoon of a FATB polypeptide as represented by SEQ ID NO: 93, which comprises the following features: (i) a plastidic transit peptide; (ii) at least one transmembrane helix; (iii) and an acyl-ACP thioesterase family domain with an InterPro accession IPR002864.

**Figure 10** shows a phylogenetic tree of FATs polypeptides from various source organisms, according to Mayer et al. (2007) BMC Plant Biology 2007. FATA polypeptides and FATBA polypeptides belong to very clearly distinct clades. The FATB clade of polypeptides useful in performing the methods of the invention has been circled, the arrow points to the *Arabidopsis thaliana* FATB polypeptide as represented by SEQ ID NO: 93.

**Figure 11** represents the graphical output of the algorithm TMpred for SEQ ID NO: 93. From the algorithm prediction using SEQ ID NO: 93, a transmembrane helix is predicted between the transit peptide (located at the N-terminus of the polypeptide) and the acyl-ACP thioesterase family domain with an InterPro accession IPR002864 (located at the C-terminus of the polypeptide).

**Figure 12** shows the binary vector for increased expression in *Oryza sativa* plants of a nucleic acid sequence encoding a FATB polypeptide under the control of a constitutive promoter from rice.

**Figure 13** shows an AlignX (from Vector NTI 10.3, Invitrogen Corporation) multiple sequence alignment of the FATB polypeptides from Table A3. The N-terminal plastidic transit peptide as predicted by TargetP has been boxed in SEQ ID NO: 93 (Arath_FATB), and the predicted transmembrane helix (typical of FATB polypeptides only) as predicted by TMpred has been boxed across FATB polypeptides useful for performing the methods of the invention. The conserved IPR002864 of the acyl-ACP thioesterase family is marked by X under the consensus sequence. The three highly conserved catalytic residues have been boxed across the alignment.

**Figure 14** represents the LFY-like protein sequence of SEQ ID NO: 146, with the FLO_LFY domain shown in bold.

**Figure 15** represents a ClustalW 2.0.3 multiple alignment of various LFY-like proteins. The asterisks indicate absolutely conserved amino acids, the colons show highly conserved amino acid residues and the dots indicate conserved amino acids.

**Figure 16** shows a phylogenetic tree created from the alignment of Figure 15 with the Neighbour Joining algorithm and 1000 bootstrap repetitions. The bootstrap values are shown.

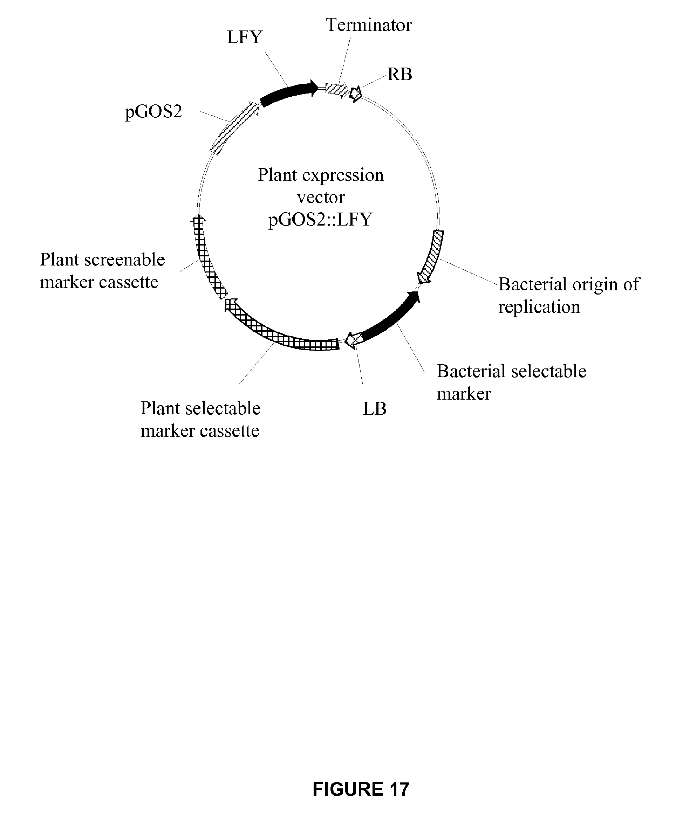

**Figure 17** represents the binary vector for increased expression in Oryza sativa of a LFY-like-encoding nucleic acid under the control of a rice GOS2 promoter (pGOS2)

## Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### *Example 1: Identification of sequences useful in the invention*

1.1 Glutamine synthase (GS1)

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

**Table A1** provides a list of nucleic acid sequences related to the nucleic acid sequence used in the methods of the present invention.

**Table A1:** Examples of algal-type GS1 polypeptides:

| Plant Source | Nucleic acid SEQ ID NO: | Protein SEQ ID NO: |
|---|---|---|
| Chlamydomonas reinhardtii 133971 | 1 | 2 |
| Aureococcus anophagefferens_20700 | 31 | 9 |
| Chlamydomonas reinhardtii_129468 | 32 | 10 |
| Chlamydomonas reinhardtii_136895 | 33 | 11 |
| Chlamydomonas reinhardtii_147468 | 34 | 12 |
| Helicosporidum sp.DQ323125 | 35 | 13 |
| Thalassiosira pseudonana_26051 | 36 | 14 |
| Volvox carterii_103492 | 37 | 15 |
| Volvox carterii_77041 | 38 | 16 |
| Hordeum vulgare_TA45411_4513 | 43 | 21 |
| Physcomitrella patens_122526 | 46 | 24 |
| Physcomitrella patens_146278 | 47 | 25 |
| Pinus taeda_TA26121_3352 | 48 | 26 |
| Pinus taeda_TA8958_3352 | 49 | 27 |
| Phaedactylum tricornutum_51092 | 50 | 28 |
| Hordeum vulgare_7728 | 53 | 55 |
| Hordeum vulgare_7958 | 54 | 56 |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest. Preferably the algal-type GS1 polypeptide is of algal origin (such as the proteins exemplified by SEQ ID NO: 2, and SEQ ID NO: 9 to 16).

1.2. Phosphoethanolamine N-methyltransferase (PEAMT)

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters were adjusted to modify the stringency of the search, for example the cut-off threshold for the E-value was increased to show less stringent matches. This way, short nearly exact matches may be identified.

Table A2 provides a list of nucleic acid sequences and threreof encoded polypeptides related to the nucleic acid sequence used in the methods of the present invention.

**Table A2:** Examples of PEAMT polypeptides:

| Name | Plant Source | Nucleic acid SEQ ID NO: | Protein SEQ ID NO: |
|---|---|---|---|
| Arath_PEAMT_1 | *Arabidopsis thaliana* | 57 | 58 |
| AT1G48600_1 | *Arabidopsis thaliana* | 59 | 60 |
| AT1G73600_1 | *Arabidopsis thaliana* | 61 | 62 |
| AT3gG18000 | *Arabidopsis thaliana* | 63 | 64 |
| Os01g50030 | *Oryza sativa* | 65 | 66 |
| Os05g47540_1 | *Oryza sativa* | 67 | 68 |
| Os05g47540_2 | *Oryza sativa* | 69 | 70 |
| Os05g47540_3 | *Oryza sativa* | 71 | 72 |
| PtPEAMT1 | *Populus trichocarpa* | 73 | 74 |
| PtPEAMT2 | *Populus trichocarpa* | 75 | 76 |
| ZmPEAMTa | *Zea Mays* | 77 | 78 |
| ZmPEAMTb | *Zea Mays* | 79 | 80 |
| ZmPEAMTc | *Zea Mays* | 81 | 82 |

1.3. Fatty acyl-acyl carrier protein (ACP) thioesterase B (FATB)

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid sequence or polypeptide sequences to sequence databases and by calculating the statistical significance of matches. For example, the polypeptide encoded by the nucleic acid sequence of the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid sequence (or polypeptide) sequences over a particular length. In some instances, the default parameters may be adjusted to modify the stringency of the search. For example the E-value may be increased to show less stringent matches. This way, short nearly exact matches may be identified.

Table A3 provides a list of nucleic acid sequences related to the nucleic acid sequence used in the methods of the present invention.

**Table A3:** Examples of FATB polypeptide sequences, and encoding nucleic acid sequences:

| Name | Source organism | Public database accession number | Nucleic acid SEQ ID NO: | Polypeptide SEQ ID NO: |
|---|---|---|---|---|
| Arath_FATB | *Arabidopsis thaliana* | N_100724.2 | 92 | 93 |
| Aqufo_FATB | *Aquilegia formosa x Aquilegia pubescens* | TA8354_338618 | 94 | 95 |
| Arahy_FATB | *Arachis hypogaea* | EF117305.1 | 96 | 97 |
| Braju_FATB | *Brassica juncea* | DQ856315.1 | 98 | 99 |
| Brasy_FATB | *Brachypodium sylvaticum* | EF059989 | 100 | 101 |
| Citsi_FA TB | *Citrus sinensis* | TA12334_2711 | 102 | 103 |

(continued)

| Name | Source organism | Public database accession number | Nucleic acid SEQ ID NO: | Polypeptide SEQ ID NO: |
|---|---|---|---|---|
| Elagu_FATB | *Elaeis guineensis* | AF147879 | 104 | 105 |
| Garma_FATB | *Garcinia mangostana* | U92878 | 106 | 107 |
| Glyma_FATB | *Glycine max* | BE211486.1 CX703472.1 | 108 | 109 |
| Goshi_FATB | *Gossypium hirsutum* | AF034266 | 110 | 111 |
| Helan_FATB | *Helianthus annuus* | AF036565 | 112 | 113 |
| Irite_FATB | *Iris tectorum* | AF213480 | 114 | 115 |
| Jatcu_FATB | *Jatropha curcas* | EU106891.1 | 116 | 117 |
| Maldo_FATB | *Madus domestica* | TA26272_3750 | 118 | 119 |
| Orysa_FATB | *Oryza sativa* | NM_001063311 | 120 | 121 |
| Picgl_FATB | *Picea glauca* | TA16055_3330 | 122 | 123 |
| Popto_FATB | *Populus tomentosa* | DQ321500.1 | 124 | 125 |
| Ricco_FATB | *Ricinus communis* | EU000562.1 | 126 | 127 |
| Soltu_FATB | *Solanum tuberosum* | A28470_4113 | 128 | 129 |
| Tager_FATB | *Tagetes erecta* | Proprietary | 130 | 131 |
| Vitvi_FATB | *Vitis vinifera* | GSVIVT0001680700 1 (Genoscope) | 132 | 133 |
| Zeama_FATB | *Zea mays* | EE033552.2, BQ577487.1, AW066432.1 | 134 | 135 |
| Zeama_FATB II | *Zea mays* | DV029251.1, CF010081.1 | 136 | 137 |
| Poptr_FATB | *Populus trichocarpa* | Poptr_FATB | 138 | 139 |

In some instances, related sequences have tentatively been assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute.

1.4. Leafy-like (LFY-like)

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A4 provides a list of nucleic acid sequences related to the nucleic acid sequence used in the methods of the present invention.

**Table A4:** Examples of LFY-like polypeptides:

| Plant Source | Nucleic acid SEQ ID NO: | Protein SEQ ID NO: |
|---|---|---|
| *Arabidopsis thaliana* | 145 | 146 |
| *Arabidopsis thaliana* | 176 | 151 |
| *Brassica juncea* | 177 | 152 |
| *Ionopsidium acaule* | 178 | 153 |
| *Leavenworthia crassa* | 179 | 154 |
| *Selenia aurea* | 180 | 155 |
| *Arabidopsis lyrata* | 181 | 156 |
| *Streptanthus glandulosus* | 182 | 157 |
| *Cochlearia officinalis* | 183 | 158 |
| *Brassica oleracea* var. botrytis | 184 | 159 |
| *Idahoa scapigera* | 185 | 160 |
| *Capsella bursa-pastoris* | 186 | 161 |
| *Barbarea vulgaris* | 187 | 162 |
| *Petunia hybrida* | 188 | 163 |
| *Antirhinum majus* | 189 | 164 |
| *Nicotiana tabacum* | 190 | 165 |
| *Nicotiana tabacum* | 191 | 166 |
| *Triticum aestivum* | 192 | 167 |
| *Triticum aestivum* | 193 | 168 |
| *Lolium temulentum* | 194 | 169 |
| *Oryza sativa* | 195 | 170 |
| *Zea mays* | 196 | 171 |
| *Zea mays* | 197 | 172 |
| *Ophrys tenthredinifera* | 198 | 173 |
| *Lycopersicon esculentum* | 199 | 174 |
| *Carica papaya* | 200 | 175 |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest.

***Example 2*: *Alignment of sequences useful in the invention***

2.1 Glutamine synthase (GS1)

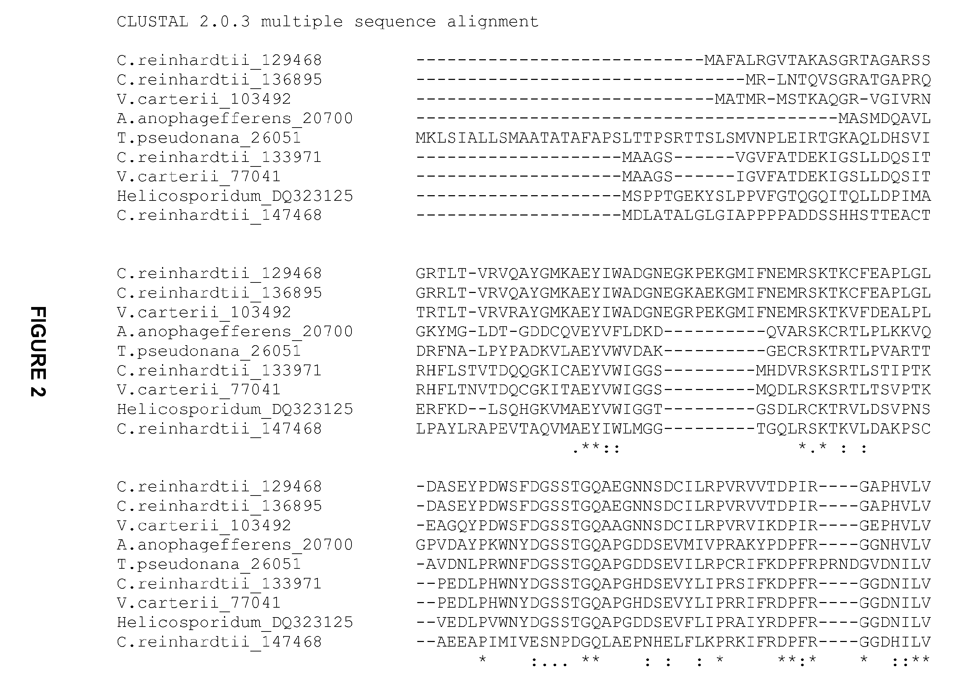

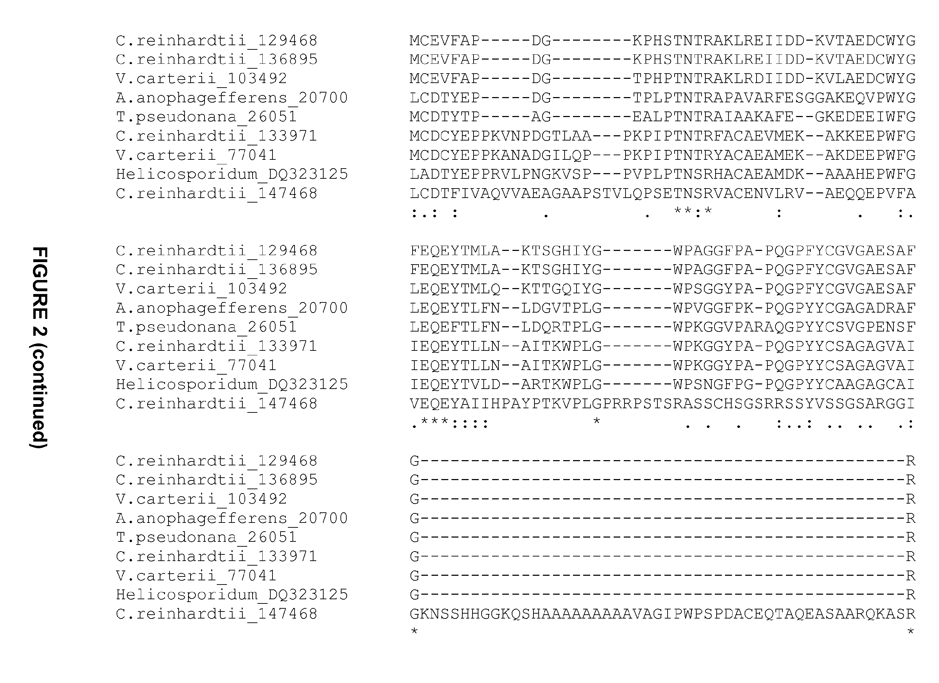

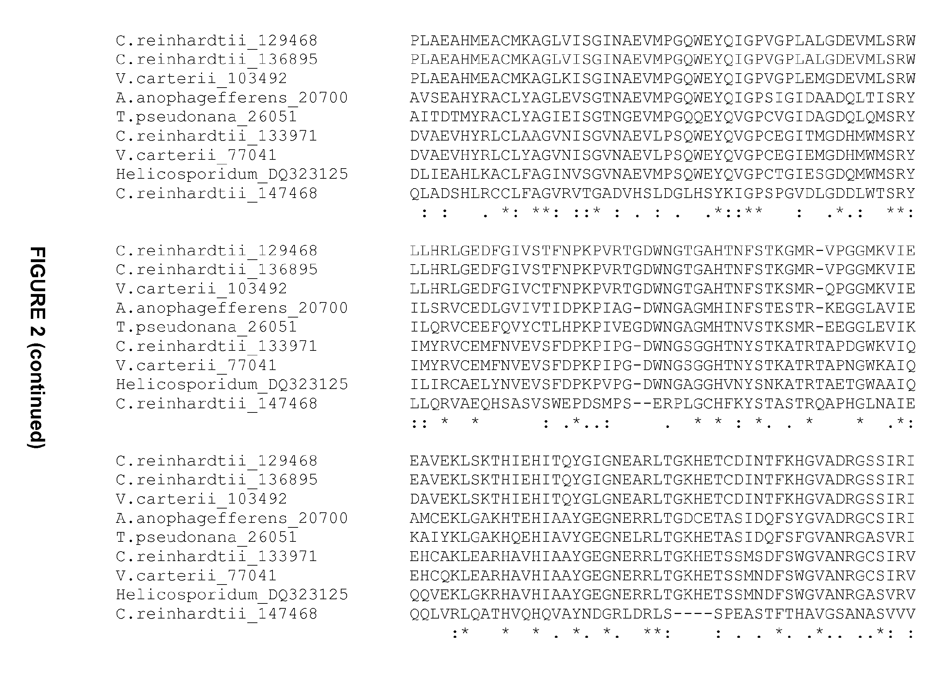

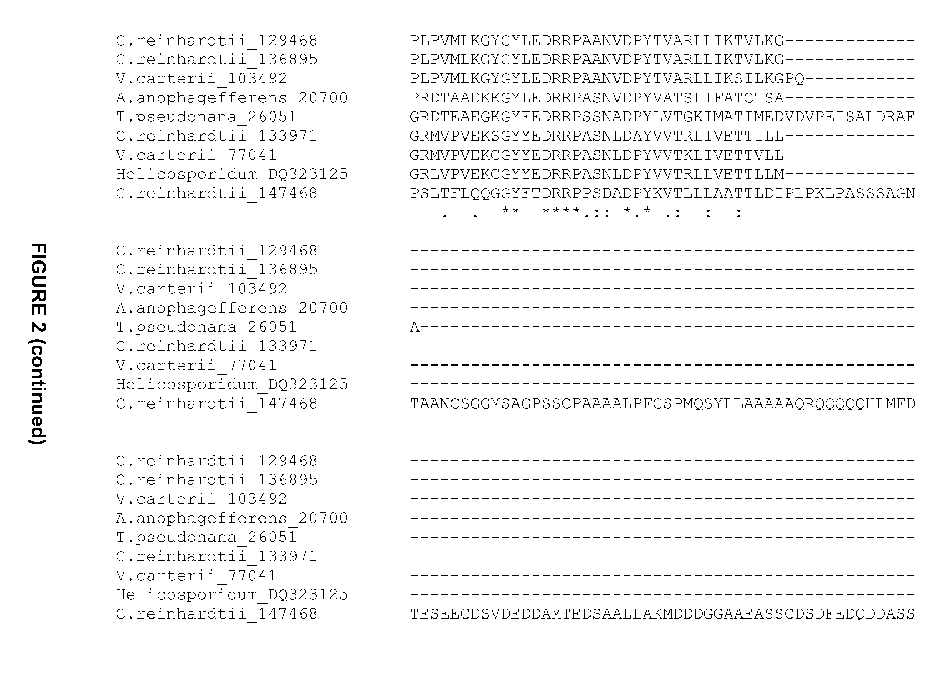

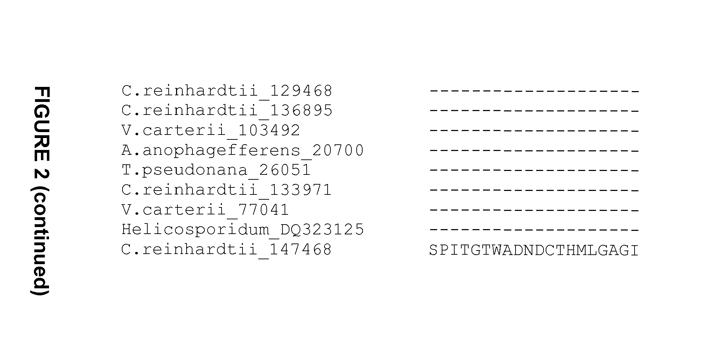

Alignment of polypeptide sequences was performed using the ClustalW 2 algorithm of progressive alignment (Larkin et al., Bioinformatics 23, 2947-2948, 2007). Default values are for the gap open penalty of 10, for the gap extension penalty of 0,2 and the selected weight matrix is Gonnet (if polypeptides are aligned). Minor manual editing may be done to further optimise the alignment. Sequence conservation among GS1 polypeptides is essentially throughout the complete sequence and corresponds to the fact that the Gln-synt_C domain and the Gln-synt_N domain largely span the complete protein sequence. The GS1 polypeptides are aligned in Figure 2.

A phylogenetic tree of GS1 polypeptides (Figure 3) was constructed from alignment using a large number of plant glutamine synthase protein sequences (panel a). From this tree, it can clearly be seen that the algal glutamine synthase proteins form a distinct group (the algal-type clade) compared to other glutamine synthase proteins of plant origin. Panel b shows the same algal-type clade of glutamine synthase proteins but with a limited set of outgroup proteins.

The proteins shown in panel a were aligned using MUSCLE (Edgar (2004), Nucleic Acids Research 32(5): 1792-97). A Neighbour-Joining tree was calculated using QuickTree (Howe et al. (2002), Bioinformatics 18(11): 1546-7). Support of the major branching is indicated for 100 bootstrap repetitions. A circular phylogram was drawn using Dendroscope (Huson et al. (2007), BMC Bioinformatics 8(1):460). The tree clearly shows that the algal GS1 proteins form a distinct group. The sequences shown in panel b were aligned using ClustalW 2 (protein weight matrix: Gonnet series, Gap opening penalty 10, Gap extension penalty 0.2) and a tree was calculated using the Neighbour Joining algorithm with 1000 bootstrap repetitions. Dendroscope was used for drawing the circular phylogram.

2.2. Phosphoethanolamine N-methyltransferase (PEAMT)

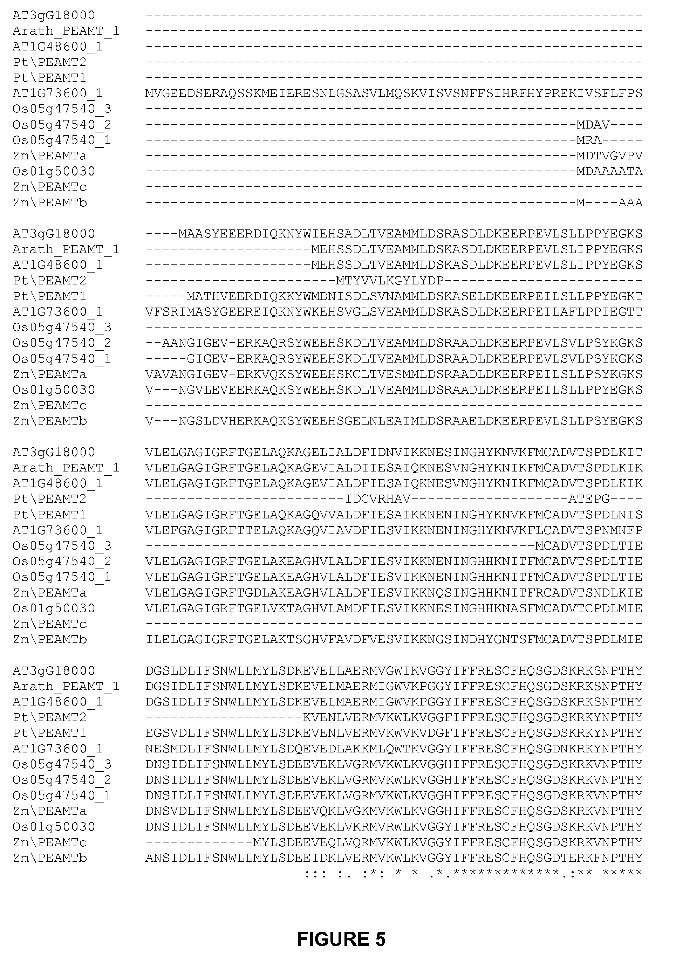

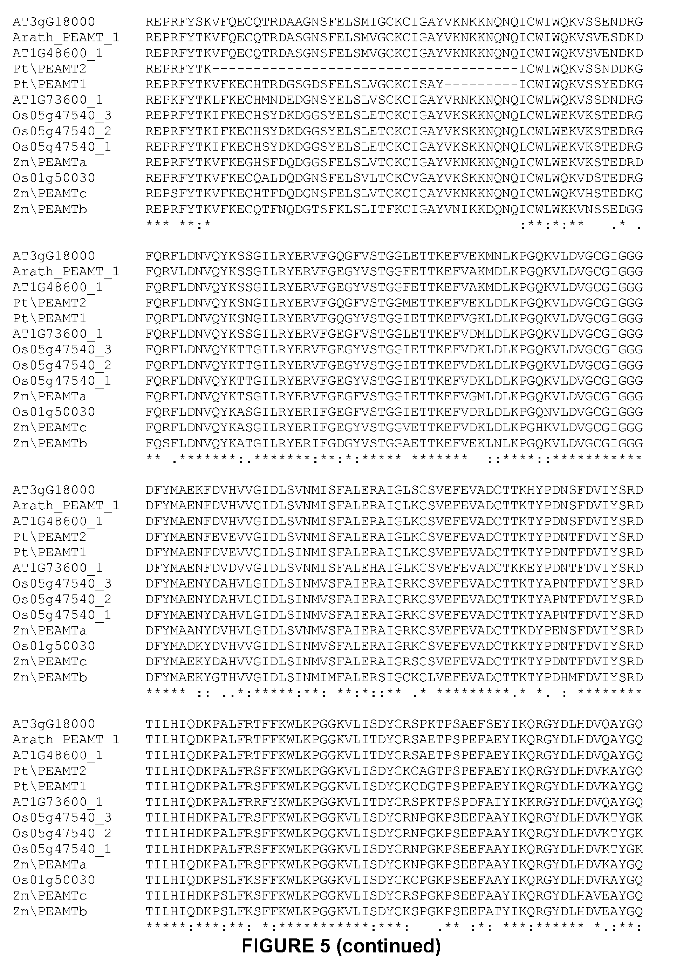

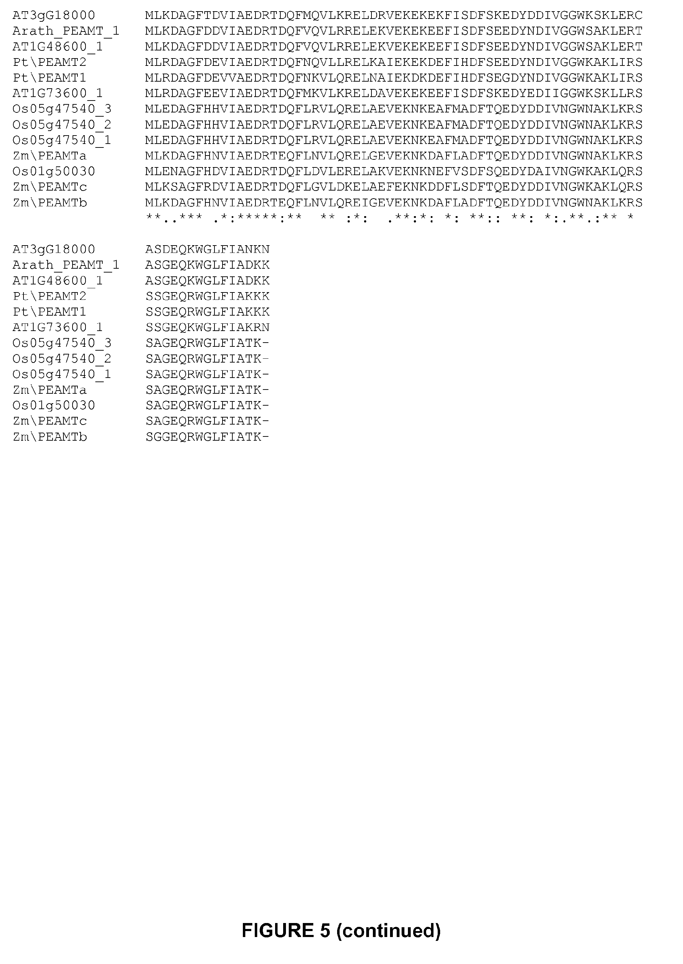

Alignment of polypeptide sequences was performed Clustal W algorithm of progressive alignment (Thompson et al. (1997) Nucleic Acids Res 25:4876-4882; Chenna et al. (2003). Nucleic Acids Res 31:3497-3500). Default values are for the gap open penalty of 10, for the gap extension penalty of 0,1 and the selected weight matrix is Blosum 62 (if polypeptides are aligned). Sequence conservation among PEAMT polypeptides is essentially in the C-terminal halt of the polypeptides, the N-terminal domain usually being more variable in sequence length and composition. The PEAMT polypeptides are aligned in Figure 5. Amino acid residues at positions labelled with * or : are highly conserved in PEAMT proteins.

A phylogenetic tree of PEAMT polypeptides (Figure 6) was constructed using a neighbour-joining clustering algorithm as provided in the Clustal W programme.

2.3. Fatty acyl-acyl carrier protein (ACP) thioesterase B (FATB)

Mutliple sequence alignment of all the FATB polypeptide sequences in Table A was performed using the AlignX algorithm (from Vector NTI 10.3, Invitrogen Corporation). Results of the alignment are shown in Figure 10 of the present application. The N-terminal plastidic transit peptide as predicted by TargetP (Example 5 herein) has been boxed in SEQ ID NO: 93 (Arath_FATB), and the predicted transmembrane helix (typical of FATB polypeptides only) as predicted by TMpred (Example 5 herein) has been boxed across FATB polypeptides useful for performing the methods of the invention. The conserved IPR002864 of the acyl-ACP thioesterase family is marked by X under the consensus sequence. The three highly conserved catalytic residues have been boxed across the alignment.

2.4. Leafy-like (LFY-like)

Alignment of polypeptide sequences was performed using ClustalW 2.0.3 (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Sequence conservation among LFY-like polypeptides is essentially over the whole length of the polypeptides, the N-terminus and the C-terminus usually being more variable in sequence length and composition. The LFY-like polypeptides are aligned in Figure 15.
A phylogenetic tree of LFY-like polypeptides (Figure 16) was constructed using a neighbour-joining clustering algorithm as provided in ClustalW 2.0.3, with 1000 bootstrap repetitions.

***Example 3: Calculation of global percentage identity between polypeptide sequences useful in the invention***

3.1 Glutamine synthase (GS1)

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

Results of the software analysis are shown in Table B1 for the global similarity and identity over the full length of the polypeptide sequences. Percentage identity is given above the diagonal in bold and percentage similarity is given below the diagonal (normal face).

The percentage identity between the algal GS1 polypeptide sequences useful in performing the methods of the invention can be as low as 23 % amino acid identity compared to SEQ ID NO: 2 (C.reinhardtii_133971). It should be noted that the algal-type GS1 polypeptides from higher plants (such as SEQ ID NO: 21, 24, 25, 26, 27, and 28) have at least 41% sequence identity when analysed with MatGAT as described above.

**Table B1:** MatGAT results for global similarity and identity over the full length of the GS1 polypeptide sequences.

| | 1 | 2 | 3 | 4 | 5 | 6 | 7 | 8 | 9 |
|---|---|---|---|---|---|---|---|---|---|
| **1. C.reinhardtii_129468** | | **43.7** | **95.3** | **20.5** | **86.6** | **43.9** | **45.6** | **41.7** | **40.0** |
| **2. C.reinhardtii_133971** | 62.3 | | **42.1** | **23.0** | **43.7** | **92.1** | **52.1** | **68.3** | **48.5** |
| **3. C.reinhardtii_136895** | 95.8 | 61.3 | | **20.1** | **86.3** | **42.9** | **46.2** | **42.2** | **39.8** |
| **4. C.reinhardtii_147468** | 31.5 | 36.6 | 31.2 | | **21.0** | **23.0** | **20.7** | **26.1** | **22.1** |
| **5. V.carterii_103492** | 92.4 | 63.9 | 91.3 | 33.6 | | **43.4** | **46.3** | **42.3** | **41.5** |
| **6. V.carterii_77041** | 62.3 | 95.3 | 61.3 | 37.1 | 63.9 | | **52.2** | **70.4** | **49.0** |
| **7. A.anophagefferens_20700** | 57.4 | 64.9 | 58.4 | 30.8 | 59.9 | 65.4 | | **49.6** | **52.3** |
| **8. Helicosporidum_DQ323125** | 60.1 | 79.8 | 59.6 | 37.1 | 60.1 | 81.1 | 62.7 | | **46.3** |
| **9. T.pseudonana_26051** | 56.0 | 60.1 | 55.0 | 34.8 | 57.2 | 61.1 | 63.5 | 59.9 | |

3.2. Phosphoethanolamine N-methyltransferase (PEAMT)

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

Results of the software analysis are shown in Table B2 for the global similarity and identity over the full length of the polypeptide sequences. Percentage identity is given below the diagonal in bold and percentage similarity is given above the diagonal (normal face).

The percentage identity between the PEAMT polypeptide sequences useful in performing the methods of the invention can be as low as 60.2 % amino acid identity compared to SEQ ID NO: 58.

**Table B2:** MatGAT results for global similarity and identity over the full length of the PEAMT polypeptide sequences.

| Polypeptide name | 1 | 2 | 3 | 4 | 5 | 6 | 7 | 8 | 9 | 10 | 11 | 12 | 13 |
|---|---|---|---|---|---|---|---|---|---|---|---|---|---|
| **1. AT3gG18000** | | 86.2 | 60.9 | 76.0 | 76.6 | 77.6 | 72.3 | 86.8 | 58.7 | 74.0 | 75.5 | 56.6 | 79.6 |
| **2. Arath_ PEAMT_1** | 93.1 | | 63.2 | 74.4 | 75.0 | 75.0 | 68.5 | 99.4 | 60.2 | 70.8 | 73.5 | 59.2 | 80.0 |
| **3. Os05g47540_3** | 70.7 | 73.3 | | 78.2 | 78.8 | 66.9 | 53.5 | 63.4 | 80.9 | 64.6 | 70.1 | 68.0 | 62.2 |
| **4. Os05g47540_2** | 88.7 | 86.7 | 78.2 | | 99.0 | 85.8 | 66.3 | 74.8 | 63.2 | 80.6 | 89.2 | 53.8 | 75.8 |
| **5. Os05g47540_1** | 89.4 | 87.4 | 78.8 | 99.0 | | 85.0 | 66.2 | 75.4 | 63.7 | 80.2 | 88.4 | 54.1 | 76.2 |
| **6. Os01g50030** | 88.6 | 85.2 | 73.1 | 93.6 | 92.8 | | 67.6 | 75.4 | 64.3 | 81.4 | 84.1 | 54.8 | 76.0 |
| **7. AT1G73600_ 1** | 81.8 | 78.6 | 62.2 | 79.1 | 78.6 | 80.0 | | 69.0 | 50.8 | 62.0 | 66.9 | 49.9 | 69.5 |
| **8. AT1G48600_1** | 93.5 | 99.6 | 73.5 | 87.1 | 87.8 | 85.6 | 78.9 | | 60.4 | 71.2 | 73.9 | 59.4 | 80.4 |
| **9. Zm\PEAMTc** | 66.8 | 68.0 | 88.1 | 68.9 | 69.5 | 69.5 | 58.6 | 68.2 | | 61.4 | 62.1 | 68.6 | 58.4 |
| **10. Zm\PEAMTb** | 86.3 | 84.0 | 72.5 | 91.9 | 91.5 | 91.6 | 76.9 | 84.4 | 67.5 | | 80.3 | 52.8 | 73.0 |
| **11. Zm\PEAMTa** | 87.6 | 85.6 | 74.3 | 94.8 | 94.0 | 92.4 | 80.7 | 86.0 | 67.5 | 89.6 | | 54.2 | 74.5 |
| **12. Pt\PEAMT2** | 63.1 | 65.7 | 76.0 | 60.2 | 61.3 | 61.5 | 57.1 | 66.1 | 81.2 | 60.0 | 60.1 | | 65.1 |
| **13. Pt\PEAMT1** | 91.0 | 90.2 | 69.6 | 85.7 | 86.2 | 86.2 | 79.3 | 90.6 | 65.7 | 83.6 | 84.4 | 68.0 | |

3.3. Fatty acyl-acyl carrier protein (ACP) thioesterase B (FATB)

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

Scoring matrix: Blosum62
First Gap: 12
Extending gap: 2

Results of the software analysis are shown in Table B3 for the global similarity and identity over the full length of the polypeptide sequences (excluding the partial polypeptide sequences).

The percentage identity between the full length polypeptide sequences useful in performing the methods of the invention can be as low as 53 % amino acid identity compared to SEQ ID NO: 93.

**Table B3:** MatGAT results for global similarity and identity over the full length of the FATB polypeptide sequences of Table A3.

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|---|
| **1. Aqufo_FATB** | | **64** | **63** | 61 | 57 | 67 | 64 | 65 | 65 | 62 | 59 | 58 | 68 | 66 | 59 | 51 | 68 | 66 | 63 | 63 | 69 | 56 | 56 | 34 |
| **2. Arahy_FATB** | 80 | | **75** | 72 | 60 | 75 | 67 | 80 | 88 | 74 | 68 | 63 | 80 | 79 | 63 | 53 | 78 | 79 | 71 | 71 | 79 | 60 | 61 | 35 |
| **3. Arath_FATB** | 78 | 86 | | **89** | **59** | **73** | **66** | **72** | **75** | **71** | **65** | **63** | **76** | **74** | **60** | **53** | **75** | **76** | **69** | **67** | **74** | **59** | **58** | **36** |
| **4. Braju_FATB** | 76 | 83 | 93 | | 56 | 70 | 64 | 72 | 71 | 68 | 64 | 62 | 73 | 71 | 59 | 53 | 72 | 73 | 66 | 64 | 71 | 56 | 57 | 35 |
| **5. Brasy_FATB** | 72 | 74 | 73 | 72 | | 60 | 69 | 60 | 60 | 58 | 56 | 62 | 62 | 61 | 86 | 50 | 63 | 61 | 61 | 60 | 62 | 81 | 64 | 31 |
| **6. Citsi_FATB** | 79 | 86 | 81 | 80 | 74 | | 67 | 71 | 76 | 76 | 65 | 64 | 79 | 79 | 62 | 52 | 78 | 78 | 70 | 69 | 79 | 60 | 58 | 34 |
| **7. Elagu_FATB** | 76 | 80 | 78 | 76 | 81 | 79 | | 64 | 66 | 64 | 60 | 71 | 71 | 67 | 71 | 54 | 68 | 68 | 64 | 64 | 70 | 67 | 65 | 35 |
| **8. Garma_FATB** | 79 | 88 | 83 | 82 | 73 | 85 | 78 | | 78 | 71 | 68 | 62 | 80 | 76 | 62 | 52 | 79 | 79 | 71 | 70 | 76 | 59 | 60 | 37 |
| **9. Glyma-FATB** | 78 | 93 | 85 | 80 | 72 | 87 | 79 | 89 | | 74 | 69 | 63 | 80 | 79 | 63 | 52 | 77 | 78 | 71 | 70 | 79 | 59 | 59 | 37 |
| **10. Goshi_FATB** | 77 | 86 | 81 | 80 | 72 | 84 | 77 | 82 | 86 | | 65 | 61 | 79 | 74 | 59 | 52 | 76 | 77 | 67 | 66 | 75 | 56 | 56 | 34 |
| **11. Helan_FATB** | 73 | 81 | 77 | 75 | 73 | 79 | 76 | 80 | 82 | 80 | | 59 | 67 | 69 | 58 | 51 | 67 | 67 | 70 | 75 | 68 | 56 | 58 | 34 |
| **12. Irite_FATB** | 74 | 77 | 76 | 75 | 78 | 78 | 85 | 77 | 77 | 75 | 76 | | 68 | 64 | 64 | 52 | 64 | 64 | 64 | 64 | 65 | 61 | 59 | 33 |
| **13. Jatcu_FATB** | 81 | 89 | 85 | 83 | 74 | 89 | 82 | 88 | 89 | 88 | 80 | 80 | | 80 | 65 | 56 | 84 | 89 | 71 | 70 | 84 | 62 | 63 | 36 |
| **14. Maldo_FATB** | 81 | 88 | 84 | 82 | 73 | 87 | 78 | 87 | 89 | 84 | 81 | 77 | 90 | | 64 | 55 | 80 | 79 | 71 | 72 | 81 | 60 | 59 | 35 |
| **15. Orysa_FATB** | 73 | 76 | 74 | 73 | 92 | 77 | 82 | 75 | 75 | 75 | 74 | 79 | 77 | 76 | | 50 | 65 | 63 | 63 | 61 | 64 | 85 | 65 | 33 |
| **16. Picgl_FATB** | 66 | 67 | 67 | 68 | 66 | 67 | 67 | 66 | 66 | 68 | 65 | 69 | 69 | 69 | 66 | | 55 | 54 | 52 | 53 | 55 | 48 | 49 | 34 |
| **17. Popto_FATB** | 78 | 87 | 84 | 81 | 76 | 88 | 80 | 86 | 87 | 86 | 80 | 78 | 91 | 88 | 78 | 67 | | 83 | 70 | 70 | 80 | 62 | 61 | 35 |
| **18. Ricco_FATB** | 79 | 87 | 84 | 82 | 74 | 87 | 79 | 88 | 89 | 85 | 79 | 79 | 94 | 88 | 76 | 69 | 90 | | 69 | 69 | 80 | 61 | 63 | 37 |
| **19. Soltu_FATB** | 77 | 82 | 80 | 77 | 74 | 82 | 79 | 80 | 82 | 79 | 81 | 76 | 81 | 82 | 76 | 67 | 82 | 83 | | 75 | 74 | 61 | 59 | 33 |
| **20. Taper_FATB** | 77 | 84 | 82 | 78 | 73 | 82 | 79 | 82 | 84 | 83 | 84 | 80 | 83 | 84 | 74 | 68 | 82 | 82 | 84 | | 72 | 58 | 60 | 33 |
| **21. Vitvi_FATB** | 80 | 87 | 84 | 80 | 75 | 88 | 80 | 85 | 87 | 85 | 80 | 79 | 90 | 90 | 78 | 68 | 90 | 88 | 83 | 83 | | 63 | 60 | 34 |
| **22. Zeama_FATB** | 70 | 74 | 73 | 70 | 89 | 74 | 79 | 73 | 74 | 72 | 71 | 77 | 74 | 73 | 90 | 64 | 75 | 75 | 74 | 71 | 75 | | 64 | 31 |
| **23. Zeama_FATBII** | 72 | 75 | 73 | 70 | 78 | 73 | 78 | 73 | 73 | 74 | 71 | 76 | 75 | 73 | 78 | 62 | 73 | 76 | 73 | 75 | 74 | 77 | | 33 |
| **24. Arath_FATA** | 51 | 51 | 53 | 52 | 49 | 52 | 52 | 56 | 54 | 53 | 50 | 50 | 53 | 54 | 50 | 49 | 51 | 53 | 52 | 51 | 51 | 47 | 49 | |

3.4. Leafy-like (LFY-like)

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.
Parameters used in the comparison were:

| | |
|---|---|
| Scoring matrix: | Blosum 62 |
| First Gap: | 12 |
| Extending gap: | 2 |

Results of the software analysis are shown in Table B4 for the global similarity and identity over the full length of the polypeptide sequences. Percentage identity is given above the diagonal and percentage similarity is given below the diagonal.
The percentage identity between the LFY-like polypeptide sequences useful in performing the methods of the invention can be as low as 50 % amino acid identity compared to SEQ ID NO: 146.

Table B4: MatGAT results for global similarity and identity over the full length of the LFY-like polypeptide sequences.

| | 1 | 2 | 3 | 4 | 5 | 6 | 7 | 8 | 9 | 10 | 11 | 12 | 13 |
|---|---|---|---|---|---|---|---|---|---|---|---|---|---|
| **1. Atleafy** | | 99.1 | 98.8 | 90.4 | 90.8 | 87.8 | 94.9 | 85.8 | 86.0 | 87.5 | 79.8 | 88.8 | 85.0 |
| **2. Q1PDG5** | 99.1 | | 99.8 | 89.5 | 89.9 | 86.9 | 94.0 | 85.1 | 85.1 | 86.6 | 78.7 | 87.8 | 84.1 |
| **3. Q1KLS1** | 99.1 | 99.8 | | 89.3 | 89.6 | 86.6 | 93.5 | 84.8 | 84.9 | 86.4 | 78.7 | 87.6 | 83.9 |
| **4. Q6XPU8** | 94.1 | 93.2 | 93.2 | | 87.1 | 83.2 | 87.8 | 82.3 | 87.9 | 83.9 | 76.1 | 84.2 | 81.0 |
| **5. Q6XPU7** | 93.9 | 93.8 | 93.8 | 90.6 | | 88.3 | 87.9 | 81.8 | 82.9 | 83.4 | 78.1 | 84.7 | 86.5 |
| **6. Q3ZLS6** | 90.3 | 90.2 | 90.2 | 86.6 | 91.6 | | 85.8 | 85.2 | 86.1 | 84.2 | 81.0 | 87.8 | 89.9 |
| **7. Q8LSH1** | 96.5 | 95.6 | 95.1 | 92.1 | 91.4 | 88.8 | | 84.8 | 85.1 | 84.7 | 78.7 | 88.4 | 84.7 |
| **8. Q3LZW7** | 88.0 | 88.1 | 88.1 | 85.7 | 86.1 | 90.1 | 87.0 | | 83.9 | 82.7 | 79.3 | 90.6 | 86.2 |
| **9. Q3ZLR9** | 90.8 | 90.7 | 90.7 | 91.8 | 89.2 | 90.7 | 88.4 | 88.9 | | 83.5 | 78.9 | 87.2 | 84.3 |
| **10. BOFH_BRAOB** | 90.6 | 90.5 | 90.5 | 87.3 | 88.0 | 88.4 | 88.4 | 86.7 | 89.9 | | 76.1 | 85.0 | 80.8 |
| **11. Q6XPU5** | 85.1 | 84.3 | 84.3 | 82.2 | 84.7 | 88.3 | 84.2 | 85.9 | 85.0 | 82.9 | | 82.2 | 78.9 |
| **12. Q3ZK20** | 91.0 | 91.0 | 91.0 | 87.1 | 89.0 | 91.8 | 89.8 | 92.6 | 90.9 | 88.7 | 88.5 | | 90.1 |
| **13. Q3ZK15** | 88.0 | 87.9 | 87.9 | 84.5 | 89.0 | 92.1 | 87.0 | 88.8 | 88.7 | 85.3 | 87.3 | 92.7 | |
| **14. genpept7227884** | 78.8 | 79.5 | 79.3 | 76.3 | 76.0 | 77.4 | 77.4 | 76.2 | 78.4 | 77.3 | 76.7 | 77.2 | 74.0 |
| **15. genpept123096** | 73.8 | 74.5 | 74.3 | 73.5 | 74.6 | 77.4 | 74.4 | 76.7 | 75.9 | 74.0 | 77.8 | 78.4 | 75.0 |

| | | | | | | | | | | | | | |
|---|---|---|---|---|---|---|---|---|---|---|---|---|---|
| **16. genpept7227893** | 77.8 | 78.6 | 78.3 | 76.1 | 76.7 | 77.2 | 76.3 | 76.3 | 78.2 | 78.6 | 75.3 | 78.0 | 74.3 |
| **17. genpept7227894** | 80.2 | 81.0 | 80.7 | 77.2 | 77.2 | 77.4 | 77.2 | 75.2 | 77.6 | 79.1 | 74.8 | 77.6 | 74.5 |
| **18. genpept86261940** | 62.5 | 61.9 | 61.7 | 62.2 | 63.8 | 65.5 | 61.9 | 62.8 | 65.4 | 63.4 | 65.8 | 65.2 | 64.9 |
| **19. genpept86261942** | 63.2 | 61.9 | 61.7 | 62.9 | 64.0 | 64.0 | 62.1 | 64.3 | 65.1 | 63.4 | 65.1 | 65.7 | 63.9 |
| **20. genpept11935156** | 63.7 | 64.0 | 64.0 | 63.6 | 62.8 | 63.8 | 62.8 | 64.5 | 67.1 | 64.6 | 63.3 | 66.3 | 62.3 |
| **21. genpept2274790** | 63.9 | 64.5 | 64.5 | 63.6 | 64.5 | 65.5 | 62.1 | 66.7 | 67.3 | 63.1 | 66.8 | 64.9 | 63.6 |
| **22. genpept28974117** | 65.8 | 66.4 | 66.4 | 64.1 | 65.0 | 64.3 | 63.7 | 64.5 | 66.3 | 63.6 | 64.6 | 64.9 | 65.1 |
| **23. genpept28974119** | 62.5 | 63.1 | 63.8 | 62.2 | 62.4 | 65.0 | 61.9 | 65.5 | 65.4 | 62.9 | 66.2 | 64.2 | 63.9 |
| **24. genpept27544560** | 62.9 | 62.9 | 62.7 | 61.6 | 62.9 | 61.6 | 60.7 | 61.0 | 61.8 | 63.2 | 58.8 | 60.7 | 60.1 |
| **25. genpept7658233** | 77.6 | 78.3 | 78.1 | 76.8 | 76.5 | 77.4 | 76.0 | 77.4 | 79.1 | 78.3 | 77.2 | 77.9 | 75.7 |
| **26. genpept66864715** | 73.6 | 74.3 | 74.0 | 73.2 | 74.6 | 76.7 | 71.9 | 73.7 | 76.4 | 74.2 | 76.1 | 75.9 | 73.8 |

| | **14** | **15** | **16** | **17** | **18** | **19** | **20** | **21** | **22** | **23** | **24** | **25** | **26** |
|---|---|---|---|---|---|---|---|---|---|---|---|---|---|
| **1. Atleafy** | 65.5 | 65.0 | 65.8 | 67.3 | 50.3 | 50.7 | 51.3 | 51.5 | 51.9 | 51.3 | 49.5 | 64.8 | 65.8 |
| **2. Q1PDG5** | 66.1 | 65.6 | 66.4 | 67.9 | 49.8 | 49.5 | 51.7 | 52.5 | 52.4 | 52.0 | 49.9 | 65.4 | 66.4 |
| **3. Q1KLS1** | 65.8 | 65.3 | 66.2 | 67.7 | 49.5 | 49.3 | 51.7 | 52.5 | 52.4 | 51.5 | 49.7 | 65.1 | 66.2 |
| **4. Q6XPU8** | 63.9 | 63.7 | 64.0 | 64.7 | 50.2 | 50.1 | 51.4 | 51.2 | 50.5 | 51.2 | 49.5 | 63.9 | 63.6 |
| **5. Q6XPU7** | 62.8 | 65.0 | 64.5 | 64.3 | 50.6 | 50.5 | 50.8 | 52.0 | 50.0 | 50.2 | 50.0 | 63.5 | 66.7 |
| **6. Q3ZLS6** | 64.5 | 66.0 | 66.0 | 65.0 | 51.8 | 52.2 | 51.4 | 52.4 | 52.6 | 53.2 | 49.0 | 65.4 | 67.0 |
| **7. Q8LSH1** | 65.8 | 64.1 | 64.7 | 65.7 | 50.7 | 51.2 | 50.5 | 50.8 | 51.5 | 50.9 | 48.6 | 64.2 | 64.4 |
| **8. Q3LZW7** | 63.7 | 64.0 | 64.5 | 64.3 | 50.1 | 50.4 | 50.2 | 52.4 | 52.0 | 52.5 | 49.5 | 64.8 | 64.0 |
| **9. Q3ZLR9** | 65.4 | 64.6 | 64.6 | 64.6 | 51.4 | 51.9 | 51.2 | 52.6 | 53.4 | 51.8 | 49.8 | 65.3 | 66.2 |
| **10. BOFH_BRAOB** | 64.1 | 64.1 | 64.3 | 64.8 | 51.3 | 51.2 | 51.9 | 51.6 | 50.9 | 52.0 | 49.3 | 64.1 | 64.4 |
| **11. Q6XPU5** | 63.1 | 64.6 | 64.1 | 64.3 | 52.8 | 52.4 | 51.8 | 53.1 | 52.4 | 53.1 | 47.6 | 65.4 | 65.6 |
| **12. Q3ZK20** | 65.0 | 65.5 | 64.5 | 64.5 | 51.2 | 51.6 | 50.5 | 51.8 | 51.6 | 51.2 | 49.5 | 65.6 | 65.7 |
| **13. Q3ZK15** | 62.1 | 63.2 | 63.2 | 61.7 | 50.7 | 50.0 | 48.2 | 49.4 | 50.5 | 49.5 | 48.3 | 63.6 | 64.4 |
| **14. genpept7227884** | | 76.2 | 89.9 | 89.3 | 55.4 | 55.4 | 55.0 | 55.5 | 55.7 | 55.2 | 49.5 | 89.3 | 72.6 |
| **15. genpept123096** | 84.7 | | 76.2 | 76.3 | 54.5 | 55.4 | 55.4 | 56.0 | 54.2 | 56.3 | 50.2 | 76.0 | 73.8 |
| **16. genpept7227893** | 93.9 | 84.5 | | 96.4 | 55.7 | 56.1 | 55.1 | 56.9 | 54.6 | 54.7 | 50.5 | 89.1 | 73.2 |
| **17. genpept7227894** | 93.3 | 83.4 | 97.6 | | 55.9 | 55.2 | 54.1 | 56.5 | 54.2 | 54.4 | 50.3 | 88.0 | 72.4 |
| **18. genpept86261940** | 68.4 | 66.2 | 67.1 | 67.5 | | 96.7 | 87.3 | 86.4 | 80.0 | 78.7 | 48.9 | 56.5 | 53.4 |
| **19. genpept86261942** | 68.0 | 66.9 | 67.6 | 67.1 | 98.0 | | 88.1 | 85.9 | 79.9 | 78.2 | 48.6 | 55.0 | 52.9 |
| **20. genpept11935156** | 69.2 | 67.5 | 67.8 | 66.6 | 91.8 | 92.0 | | 83.1 | 76.4 | 74.6 | 47.1 | 55.8 | 52.3 |
| **21. genpept2274790** | 69.2 | 67.7 | 68.8 | 68.0 | 91.3 | 90.6 | 88.8 | | 82.5 | 80.4 | 50.0 | 56.8 | 54.9 |
| **22. genpept28974117** | 69.2 | 65.4 | 66.8 | 66.3 | 87.0 | 86.5 | 85.3 | 89.6 | | 91.2 | 48.2 | 55.5 | 52.5 |
| **23. genpept28974119** | 68.4 | 68.4 | 67.6 | 66.8 | 85.7 | 85.7 | 84.0 | 87.5 | 94.4 | | 48.5 | 55.6 | 54.2 |
| **24. genpept27544560** | 62.5 | 63.6 | 64.0 | 63.6 | 58.8 | 60.3 | 58.8 | 61.2 | 58.8 | 59.2 | | 50.1 | 50.5 |
| **25. genpept7658233** | 93.9 | 84.5 | 93.7 | 93.3 | 67.7 | 67.2 | 68.4 | 69.4 | 68.2 | 69.2 | 62.9 | | 73.4 |
| **26. genpept66864715** | 80.1 | 81.8 | 80.1 | 79.3 | 65.6 | 65.8 | 65.8 | 67.9 | 63.9 | 67.0 | 62.5 | 80.1 | |

***Example 4: Identification of domains comprised in polypeptide sequences useful in the invention***

4.1. Glutamine synthase (GS1)

The Integrated Resource of Protein Families, Domains and Sites (interPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan of the polypeptide sequence as represented by SEQ ID NO: 2 are presented in Table C1.

**Table C1:** InterPro scan results (major accession numbers) of the polypeptide sequence as represented by SEQ ID NO: 2.

| Database | Accession number | Accession name | Amino acid coordinates on SEQ ID NO 2 |
|---|---|---|---|
| InterPro | IPR008146 | Glutamine synthetase, catalytic region | |
| PRODOM | PD001057 | Gln-synt-C | 153-370 |
| PFAM | PF00120 | Gln-synt-C | 132-381 |
| PROSITE | PS00181 | GLNA_ATP | 264-280 |
| InterPro | IPR008147 | Glutamine synthetase, beta-Grasp | |
| PFAM | PF03951 | Gln-synt_N | 36-116 |
| PROSITE | PS00180 | GLNA_1 | 74-91 |
| InterPro | IPR014746 | NGlutamine synthetase/ guanido kinase, catalytic region | |
| GENE3D | G3DSA:3.30.590.10 | no description | 135-376 |
| PANTHER | PTHR20852 | GLUTAMINE SYNTHETASE | 42-381 |
| PANTHER | PTHR20852:SF14 | GLUTAMINE SYNTHETASE (GLUTAMATE-AMMONIA LIGASE) (GS) | 42-381 |

4.2. Phosphoethanolamine N-methyltransferase (PEAMT)

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan of the polypeptide sequence as represented by SEQ ID NO: 58 are presented in Table C2.

**Table C2:** InterPro scan results (major accession numbers) of the polypeptide sequence as represented by SEQ ID NO: 58.

| Database | Accession number | Accession name | SEQ ID NO: | Amino acid coordinates on SEQ ID NO 58 |
|---|---|---|---|---|
| Interpro | IPR013216 | Methyltransferase type 11 | 86 | 34-143 |

(continued)

| Database | Accession number | Accession name | SEQ ID NO: | Amino acid coordinates on SEQ ID NO 58 |
|---|---|---|---|---|
| Interpro | IPR013216 | Methyltransferase type 11 | 87 | 263-370 |
| Interpro | IPR001601 | Generic methyltransferase | | 104-144 |
| Interpro | IPR001601 | Generic methyltransferase | | 333-371 |
| Interpro | IPR004033 | UbiE/COQ5 methyltransferase | 88 | 239-418 |

4.3. Fatty acyl-acyl carrier protein (ACP) thioesterase B (FATB)

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, Panther, ProDom and Pfam, Smart and TIGRFAMs. Interpro is hosted at the European Bioinformatics Institute in the United Kingdom.

The results of the InterPro scan of the polypeptide sequence as represented by SEQ ID NO: 93 are presented in Table C3.

**Table C3:** InterPro scan results of the polypeptide sequence as represented by SEQ ID NO: 93

| InterPro accession number and name | Integrated database name | Integrated database accession number | Integrated database accession name |
|---|---|---|---|
| IPR002864 Acyl-ACP thioesterase family | Pfam | PF01643 | Acyl-ACP_TE |
| No IPR integrated | G3DSA: 3.10.129.10 | CATH | G3DSA:3.10.129.10 |
| No IPR integrated | SSF54637 | Superfamily | SSF54637 Thioesterase/ thiol ester dehydrase-isomerase |

4.4. Leafy-like (LFY-like)

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan of the polypeptide sequence as represented by SEQ ID NO: 146 are presented in Table C4.

**Table C4:** InterPro scan results (major accession numbers) of the polypeptide sequence as represented by SEQ ID NO: 146.

| Database | Accession number | Accession name | Amino acid coordinates on SEQ ID NO 146 |
|---|---|---|---|
| InterPro | IPR002910 | Floricaula/leafy protein | |

(continued)

| Database | Accession number | Accession name | Amino acid coordinates on SEQ ID NO 146 |
|---|---|---|---|
| HMMPfam | PF01698 | FLO_LFY | T[1-395] 0.0 |

***Example 5: Topology prediction of the polypeptide sequences useful in the invention***

5.1. Glutamine synthase (GS1)

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

SEQ ID NO: 2 was analysed with TargetP 1.1. The "plant" organism group was selected, no cutoffs defined, and the predicted length of the transit peptide requested. The subcellular localization of the polypeptide sequence as represented by SEQ ID NO: 2 may be the cytoplasm or nucleus, no transit peptide is predicted (predicted localisation: Other: probability 0.737, reliability class 3). Predictions from other algorithms gave similar results:

| | |
|---|---|
| Psort: | peroxisome 0.503; cytoplasm 0.450 |
| PA-SUB: | cytoplasm, certainity 100% |
| PTS1: | not targeted to peroxisome |

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PLOC (Park and Kanehisa, Bioinformatics, 19, 1656-1663, 2003).

5.2. Fatty acyl-acyl carrier protein (ACP) thioesterase B (FATB)

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

TargetP v1.1 prediction results:
Number of query sequences: 1
Cleavage site predictions included.
Using PLANT networks.

| Name | Length | **cTP** | mTP | SP | other | Loc | RC | **TP length** |
|---|---|---|---|---|---|---|---|---|
| Sequence | 412 | **0.957** | 0.010 | 0.089 | 0.144 | C | 1 | **49** |

The subcellular localization of the polypeptide sequence as represented by SEQ ID NO: 93 is the chloroplast, and the predicted length of the transit peptide is of 49 amino acids starting from the N-terminus (not as reliable as the prediction of the subcellular localization itself, may vary in length by a few amino acids).

Many algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark

A transmembrane domain usually denotes a single transmembrane alpha helix of a transmembrane protein. It is called "domain" because an alpha-helix in membrane can be folded independently on the rest of the protein. More broadly, a transmembrane domain is any three-dimensional protein structure which is thermodynamically stable in membrane. This may be a single alpha helix, a stable complex of several transmembrane alpha helices, a transmembrane beta barrel, a beta-helix of gramicidin A, or any other structure.

The TMpred program makes a prediction of membrane-spanning regions and their orientation. The algorithm is based on the statistical analysis of TMbase, a database of naturally occuring transmembrane proteins. The prediction is made using a combination of several weight-matrices for scoring (K. Hofmann & W. Stoffel (1993) TMbase - A database of membrane spanning proteins segments. Biol. Chem. Hoppe-Seyler 374,166). TMpred is part of the European Molecular Biology network (EMBnet.ch) services and is maintained at the server of the Swiss Institute of Bioinformatics.

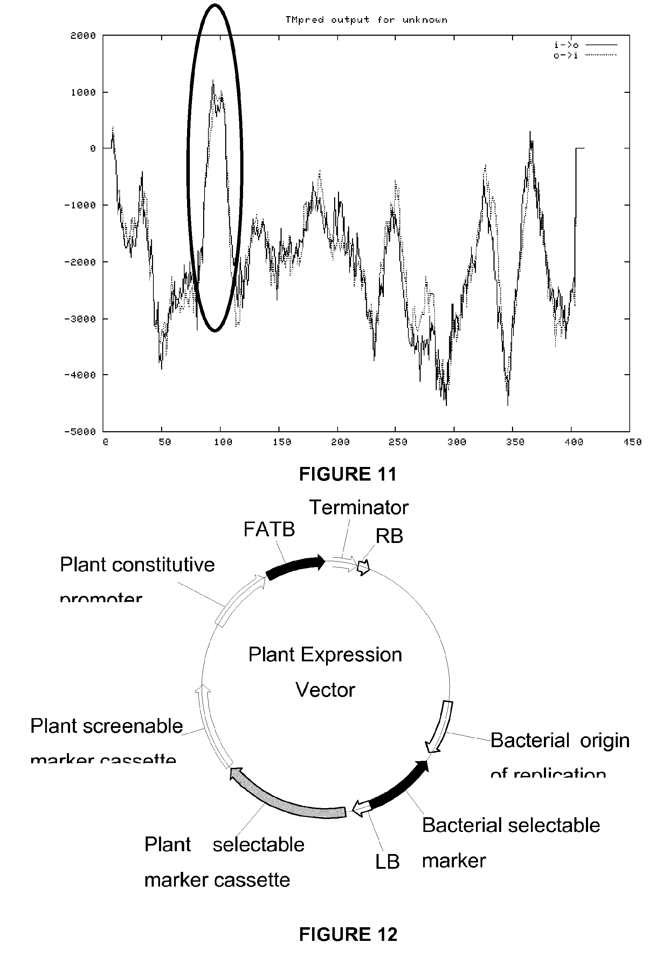

TMpred output (see Figure 11 for graphical output):

| | # | **from AA** | **To AA** | **length** | **Total score** |
|---|---|---|---|---|---|
| **Strongly preferred model** | 1 | 84 | 107 | 24 | 1214 |
| Alternative model | 1 | 89 | 113 | 25 | 1018 |

5.3. Leafy-like (LFY-like)

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 146 are presented Table D. The "plant" organism group has been selected, no cutoffs defined, and the predicted length of the transit peptide requested. The subcellular localization of the polypeptide sequence as represented by SEQ ID NO: 146 may be the mitochondrion, though the reliability of the prediction is low.

**Table D:** TargetP 1.1 analysis of Atleafy as represented by SEQ ID NO: 146, wherein Len is length of the protein, cTP: probability for a Chloroplastic transit peptide, mTP: probability for a Mitochondrial transit peptide, SP: probability for a Secretory pathway signal peptide, other: probability for a Other subcellular targeting, Loc: Predicted Location, RC: Reliability class, TPlen: Predicted transit peptide length:

| Name | Len | cTP | mTP | SP | other | Loc | RC | TPlen |
|---|---|---|---|---|---|---|---|---|
| Atleafy | 424 | 0.181 | 0.432 | 0.015 | 0.404 | M | 5 | 61 |

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark

***Example 6: Assay related to the polypeptide sequences useful in the invention***

6.1. Glutamine synthase (GS1)

Assay for glutamine synthase as commercialised by Sigma-Aldrich (modified from Kingdon, H.S., Hubbard, J.S., and Stadtman, E.R. (1968) Biochemistry 7, 2136-2142):

Principle:

ADP, generated by GS1 upon synthesis of glutamine, is used with phosphor(enol)pyruvate and pyruvate kinase to generate pyruvate and ATP. Pyruvate is converted by L-Lactic Dehydrogenase into L-Lactate with oxidation of β-NADH to β-NAD. The oxidation of NADH is followed spectrophotometrically at 340nm at 37°C with a light path of 1 cm in a buffer with pH 7.1.

Reagents:

A. 100 mM Imidazole HCl Buffer, pH 7.1 at 37°C
(Prepare 200 ml in deionized water using Imidazole, Sigma Prod. No. I-0250. Adjust to pH 7.1 at 37°C with 1 M HCl.)
B. 3 M Sodium Glutamate Solution (Glu)
(Prepare 10 ml in deionized water using L-Glutamic Acid, Monosodium Salt, Sigma Prod. No. G-1626.)
C. 250 mM Adenosine 5'-Triphosphate Solution (ATP)
(Prepare 5 ml in deionized water using Adenosine 5'-Triphosphate, Disodium Salt, Sigma Prod. No. A-5394. PREPARE FRESH.)
D. 33 mM Phospho(enol)pyruvate Solution (PEP)
(Prepare 10 ml in deionized water using Phospho(enol)pyruvate, Trisodium Salt, Hydrate, Sigma Prod. No. P-7002. PREPARE FRESH.)
E. 900 mM Magnesium Chloride Solution ($MgCl_2$)
(Prepare 10 ml in deionized water using Magnesium Chloride, Hexahydrate, Sigma Prod. No. M-0250.)
F. 1 M Potassium Chloride Solution (KCl)
(Prepare 5 ml in deionized water using Potassium Chloride, Sigma Prod. No. P-4504.)
G. 1.2 M Ammonium Chloride Solution (NH4Cl)
(Prepare 5 ml in deionized water using Ammonium Chloride, Sigma Prod. No. A-4514.)
H. 12.8 mM β-Nicotinamide Adenine Dinucleotide Solution, Reduced Form (β-NADH)
(Dissolve the contents of one 10 mg vial of β-Nicotinamide Adenine Dinucleotide, Reduced Form, Disodium Salt, Sigma Stock No. 340-110 in the appropriate volume of Reagent A. PREPARE FRESH.)
I. PK/LDH Enzymes Solution (PK/LDH)
(Use PK/LDH Enzymes Solution in 50% Glycerol, Sigma Prod. No. P-0294; contains approximately 700 units/ml pyruvate kinase and 1,000 units/ml lactic dehydrogenase. L-Lactic Dehydrogenase Unit Definition: One unit will

reduce 1.0 μmole of pyruvate to L-lactate per minute at pH 7.5 at 37°C. Pyruvate Kinase Unit Definition: One unit will convert 1.0 μmole of phospho(enol)pyruvate to pyruvate per minute at pH 7.6 at 37°C.)

J. Glutamine Synthetase Enzyme Solution

(Immediately before use, prepare a solution containing 4 - 8 units/ml of Glutamine Synthetase in cold deionized water).

Procedure:

Prepare a Reaction Cocktail by pipetting (in milliliters) the following reagents into a suitable container:

| | |
|---|---|
| Deionized Water | 20.60 |
| Reagent A (Buffer) | 17.20 |
| Reagent B (Glu) | 1.80 |
| Reagent C (ATP) | 1.80 |
| Reagent E ($MgCl_2$) | 3.55 |
| Reagent F (KCl) | 0.90 |
| Reagent G ($NH_4Cl$) | 1.80 |

Mix by stirring and adjust to pH 7.1 at 37°C with 0.1 N HCl or 0.1 N NaOH, if necessary. Pipette (in milliliters) the following reagents into suitable cuvettes:

| | Test | Blank |
|---|---|---|
| Reaction Cocktail | 2.70 | 2.70 |
| Reagent D (PEP) | 0.10 | 0.10 |
| Reagent H (β-NADH) | 0.06 | 0.06 |
| Mix by inversion and equilibrate to 37°C. Monitor the $A_{340}$nm until constant, using a suitably thermostatted spectrophotometer. Then add: | | |
| Reagent I (PK/LDH) | 0.04 | 0.04 |
| Mix by inversion and equilbrate to 37°C. Monitor the $A_{340}$nm until constant, using a suitably thermostatted spectrophotometer. Then add: | | |
| Deionized water | ------ | 0.10 |
| Reagent J (Enzyme Solution) | 0.10 | ------ |

Immediately mix by inversion and record the decrease in $A_{340}$nm for approximately 10 minutes. Obtain the $\Delta A_{340}$nm/min using the maximum linear rate for both the Test and Blank.

Calculations:

$$\text{Units/ml enzyme} = \frac{(\Delta A340\text{nm/min Test} - \Delta A340\text{nm/min Blank})(3)(15)}{(6.22)(0.1)}$$

3 = Total volume (in milliliters) of assay
15 = Conversion factor to 15 minutes (Unit Definition)
6.22 = Millimolar extinction coefficient of β-NADH at 340 nm
0.1 = Volume (in milliliter) of enzyme used

$$\text{Units/mg solid} = \frac{\text{units/ml enzyme}}{\text{mg solid/ml enzyme}}$$

$$\text{Units/mg protein} = \frac{\text{units/ml enzyme}}{\text{mg protein/ml enzyme}}$$

Unit definition:

One unit will convert 1.0 μmole of L-glutamate to L-glutamine in 15 minutes at pH 7.1 at 37°C.

Final assay concentrations:

In a 3.00 ml reaction mix, the final concentrations are 34.1 mM imidazole, 102 mM sodium glutamate, 8.5 mM adenosine 5'-triphosphate, 1.1 mM phosphoenolpyruvate, 60 mM magnesium chloride, 18.9 mM potassium chloride, 45 mM ammonium chloride, 0.25 mM β-nicotinamide adenine dinucleotide, 28 units pyruvate kinase, 40 units L-lactic dehydrogenase and 0.4 - 0.8 units glutamine synthetase.

6.2. Fatty acyl-acyl carrier protein (ACP) thioesterase B (FATB)

Polypeptides useful in performing the methods of the invention typically display thioesterase enzymatic activity. Many assays exist to measure such activity, for example, the FATB polypeptide can be expressed in an *E. coli* strain deficient in free fatty acid uptake from the medium. Thus, when a FATB polypeptide is functioning in this system, the free fatty acid product of the thioesterase raction accumulates in the medium. By measuring the free fatty acids in the medium, the enzymatic activity of the polypeptide can be identified (Mayer & Shanklin (2005) J Biol Chem 280: 3621). Thioesterase assays related to FATB polypeptide enzymatic activity can also performed, as described in Voelker et al. (1992; Science 257: 72-74).

A person skilled in the art is well aware of such experimental procedures to measure FATB polypeptide enzymatic activity, including the activity of a FATB polypeptide as represented by SEQ ID NO: 93.

***Example 7: Cloning of the nucleic acid sequence used in the methods of the invention***

7.1. Glutamine synthase (GS1)

The nucleic acid sequence used in the methods of the invention was amplified by PCR using as template a custom-made *Chlamydomonas reinhardtii* cDNA library (in pCMV Sport 6.0; Invitrogen, Paisley, UK). PCR was performed using Hifi Taq DNA polymerase in standard conditions, using 200 ng of template in a 50 μl PCR mix. The primers used were prm08458 (SEQ ID NO: 7; sense, start codon in bold): 5'-ggggacaagtttgtacaaaaaagcaggctt aaacaatggccgcgggatctgtt-3' and prm08459 (SEQ ID NO: 8, reverse, complementary): 5'-ggggaccactttgtacaagaaagctgggtgctgctcctgcgcttacagaa-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pGS1. Plasmid pDONR201 was purchased from Invitrogen, as part of the Gateway® technology.

The entry clone comprising SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice protochlorophyllide reductase promoter promoter (pPCR, SEQ ID NO: 6) for shoot specific expression was located upstream of this Gateway cassette.

After the LR recombination step, the resulting expression vector pPCR::GS1 (Figure 3) was transformed into *Agrobacterium* strain LBA4044 according to methods well known in the art.

7.2. Phosphoethanolamine N-methyltransferase (PEAMT)

The nucleic acid sequence used in the methods of the invention was amplified by PCR using as template a custom-made Arabidopsis thaliana seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used were primer: 5'-ggggacaagtttgtacaaaaaagcaggcttaaacaatggagcattctagtgatttg-3' (SEQ ID NO: 83; sense) and primer 5'-ggggaccactttgtacaagaaagctgggtcagagttttgggataaaaaca-3' (SEQ ID NO: 84; reverse, complementary): which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pArath_PEAMT_1. Plasmid pDONR201 was purchased from Invitrogen, as part of the Gateway® technology.

The entry clone comprising SEQ ID NO: 57 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 85) for constitutive expression was located upstream of this Gateway cassette.

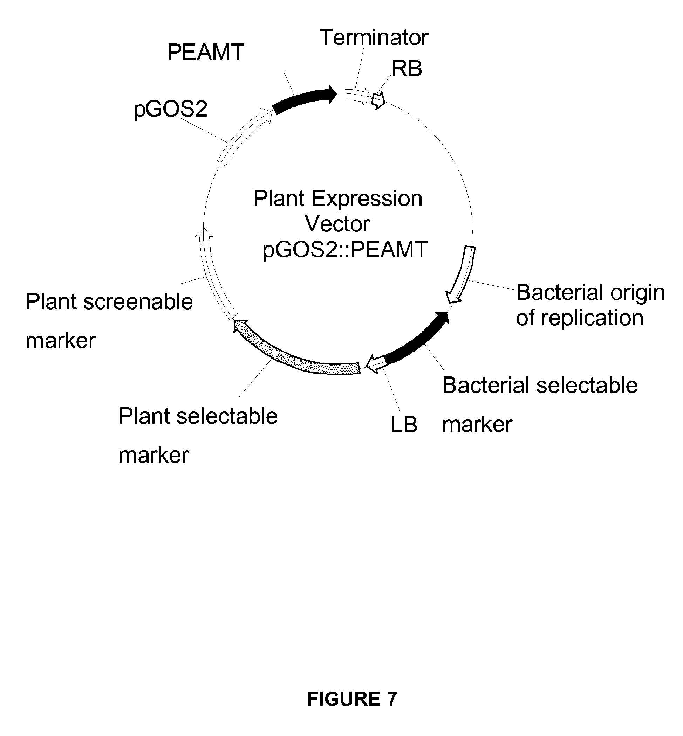

After the LR recombination step, the resulting expression vector pGOS2::Arath_PEAMT_1 (Figure 7) was transformed into Agrobacterium strain LBA4044 according to methods well known in the art.

7.3. Fatty acyl-acyl carrier protein (ACP) thioesterase B (FATB)

Unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

The *Arabidopsis thaliana* nucleic acid sequence encoding a FATB polypeptide sequence as represented by SEQ ID NO: 93 was amplified by PCR using as template a cDNA bank constructed using RNA from Arabidopsis plants at different developmental stages. The following primers, which include the AttB sites for Gateway recombination, were used for PCR amplification: prm08145: 5'-ggggacaagtttgtacaaaaaagcaggcttaaacaatggtggccacctc tgc-3' (SEQ ID NO: 142, sense) and prm08146: 5'-ggggaccactttgtacaagaaagctgggtttttt cttacggtgcagttcc-3' (SEQ ID NO: 143, reverse, complementary). PCR was performed using Hifi Taq DNA polymerase in standard conditions. A PCR fragment of the expected length (including attB sites) was amplified and purified also using standard methods. The first step of the Gateway procedure, the BP reaction, was then performed, during which the PCR fragment recombined in vivo with the pDONR201 plasmid to produce, according to the Gateway terminology, an "entry clone". Plasmid pDONR201 was purchased from Invitrogen, as part of the Gateway® technology.

The entry clone comprising SEQ ID NO: 92 was subsequently used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 144) for constitutive expression was located upstream of this Gateway cassette.

After the LR recombination step, the resulting expression vector pGOS2::FATB (Figure 12) for constitutive expression, was transformed into *Agrobacterium* strain LBA4044 according to methods well known in the art.

7.4. Leafy-like (LFY-like)

The nucleic acid sequence used in the methods of the invention was amplified by PCR using as template a custom-made *Arabidopsis thaliana* seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used were prm4841 (SEQ ID NO: 147; sense, start codon in bold): 5'-ggggacaagtttgtacaaaaaagcaggc ttaaaca**atg**gatcctgaaggtttcac-3' and prm4842 (SEQ ID NO: 148; reverse, complementary): 5'-ggggaccactttgtacaagaaagctgggtaaccaaactagaaacgcaagt -3', which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pLFY-like. Plasmid pDONR201 was purchased from Invitrogen, as part of the Gateway® technology.

The entry clone comprising SEQ ID NO: 145 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 5 for constitutive expression was located upstream of this Gateway cassette. In an alternative embodiment, a shoot-specific promoter was used (PCR, protochlorophyllid reductase promoter, SEQ ID NO: 150)

After the LR recombination step, the resulting expression vector pGOS2::LFY-like (Figure 16) or pPCR::LFY-like, was transformed into *Agrobacterium* strain LBA4044 according to methods well known in the art.

***Example 8: Plant transformation***

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water.

The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 μg/ml cefotaxime. The seeds are then transferred to SH-medium with 50μg/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μg/ml MgCL2, and with 50 to 100 μg/ml cefotaxime and 400-500 μg/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

***Example 9: Phenotypic evaluation procedure***

9.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development.

Four events were further evaluated following the same evaluation procedure as for the T2 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approach the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds were grown in potting soil under normal conditions except for the nutrient solution. The pots were watered from transplantation to maturation with a specific nutrient solution containing reduced N nitrogen (N) content, usually between 7 to 8 times less. The rest of the cultivation (plant maturation, seed harvest) was the same as for plants not grown under abiotic stress. Growth and yield parameters were recorded as detailed for growth under normal conditions.

*Salt stress screen*

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

9.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

9.3 Parameters measured

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. The Harvest Index (HI) in the present invention is defined as the ratio between the total seed yield and the above ground area ($mm^2$), multiplied by a factor $10^6$. The seed fill rate as defined in the present invention is the proportion (expressed as a %) of the number of filled seeds over the total number of seeds (or florets).

***Example 10: Results of the phenotypic evaluation of the transgenic plants***

10.1 Glutamine synthase (GS1)

Rice plants from T2 seeds were grown in potting soil under normal conditions except for the nutrient solution. The pots were watered from transplantation to maturation with a specific nutrient solution containing reduced N nitrogen (N) content, usually between 7 to 8 times less. The rest of the cultivation (plant maturation, seed harvest) was the same as for plants not grown under abiotic stress. Growth and yield parameters were recorded as detailed for growth under normal conditions.

The results of the evaluation of transgenic rice plants expressing a GS1 nucleic acid under conditions of nutrient deficiency are presented below in Table E1. An increase of more than 5 % was observed for total seed yield, number of filled seeds, fill rate, total number of seeds, and harvest index. These increases were confirmed in a subsequent experiment.

**Table E1:**

| | 1st experiment | | Confirmation experiment | |
|---|---|---|---|---|
| **parameter** | **% increase** | **p-value** | **% increase** | **p-value** |
| total seed yield | 17 | 0.011 | 18 | 0.000 |
| number of filled seeds | 16 | 0.014 | 18 | 0.000 |
| fill rate | 7 | 0.043 | 10 | 0.308 |
| total number of seeds | 26 | 0.117 | 15 | 0.000 |
| harvest index | 12 | 0.019 | 14 | 0.021 |

In addition, an increase was found for biomass (2 positive lines out of 4, overall increase 13%) and for early vigour (3 positive lines out of 4, overall increase 28%).

10.2. Phosphoethanolamine N-methyltransferase (PEAMT)

The results of the evaluation of transgenic rice plants expressing the Arath_PEAMT_1 nucleic acid under non-stress conditions are presented below. An increase of at least 5 % was observed for the total seed yield, seed fill rate, number of flowers per panicle and harvest index (Table E2).

**Table E2.** Results phenotypic evaluation under non-stress conditions.

| Parameter | % increase in transgenic plant versus control plant |
|---|---|
| Total Seed Yield | 12 |
| Flowers Per Panicle | 5,1 |
| See Fill Rate | 12 |
| Harvest Index | 3,4 |

Plants from T2 seeds were grown in potting soil under normal conditions until they approached the heading stage. They were then transferred to a "dry" section where irrigation was withheld. Humidity probes were inserted in randomly chosen pots to monitor the soil water content (SWC). When SWC went below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. Growth and yield parameters were recorded as detailed for growth under normal conditions.

The results of the evaluation of transgenic rice plants expressing a PEAMT nucleic acid under drought-stress conditions are presented hereunder. An increase was observed for total seed weight, number of filled seeds, fill rate, harvest index and thousand-kernel weight (Table E3). An increase of at least 5 % was observed for aboveground area (AreaMax; green biomass), emergence vigour (early vigour), and of 2.5% for thousand kernel weight.

**Table E3.** Results phenotypic evaluation under drought screen.

| Parameter | % increase in transgenic plant versus control plant |
|---|---|
| Aboveground Area | 5,4 |
| Emergence Vigour | 15 |
| Thousand Kernel Weight | 3 |

10.3. Fatty acyl-acyl carrier protein (ACP) thioesterase B (FATB)

The results of the evaluation of T1 and T2 generation transgenic rice plants expressing the nucleic acid sequence encoding a FATB polypeptide as represented by SEQ ID NO: 93, under the control of a GOS2 constitutive promoter, and grown under normal growth conditions, are presented below.

There was a significant increase in the early vigor, in the aboveground biomass, in the total seed yield per plant, in the total number of seeds, in the number of filled seeds, in the seed filling rate, and in the harvest index of the transgenic plants compared to corresponding nullizygotes (controls), as shown in Table E4

**Table E4:** Results of the evaluation of T1 and T2 generation transgenic rice plants expressing the nucleic acid sequence encoding a FATB polypeptide as represented by SEQ ID NO: 93, under the control of a GOS2 promoter for constitutive expression.

| Trait | overall average % increase in 6 events in the T1 generation | overall average % increase in 4 events in the T2 generation |
|---|---|---|
| Total seed yield per plant | 17% | 9% |
| Total number of seeds | 1% | 8% |
| Total number of filled seeds | 17% | 10% |

(continued)

| Trait | overall average % increase in 6 events in the T1 generation | overall average % increase in 4 events in the T2 generation |
|---|---|---|
| Seed filling rate | 14% | 2% |
| Harvest index | 17% | 6% |

10.4. Leafy-like (LFY-like)

Transgenic rice plants expressing a *LFY-like* nucleic acid under non-stress conditions showed increased seed yield. The plants expressing Atleafy under control of the constitutive promoter or the shoot specific promoter gave an increase in one or more of the following parameters: fillrate, harvest index, thousand kernel weight, flowers per panicle.

## Claims

1. A method for enhancing yield-related traits in plants relative to that of control plants, comprising modulating expression in a plant of a nucleic acid encoding a PEAMT polypeptide wherein said PEAMT polypeptide comprises a protein domain having in increasing order of preference at least 60%, 61 %, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, or 99% sequence identity to anyone of the protein domains set forth in Table C2.

2. Method according to claim 1, wherein the nucleic acid encodes a PEAMT polypeptide having in increasing order of preference at least 50%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid sequence represented by SEQ ID NO: 58.

3. Method according to claim 1 or 2, wherein said nucleic acid encoding a PEAMT polypeptide is a portion of the nucleic acid represented by SEQ ID NO: 57, or is a portion of a nucleic acid encoding an orthologue or paralogue of the amino acid sequence of SEQ ID NO: 58, wherein the portion is at least 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, consecutive nucleotides in length, the consecutive nucleotides being of SEQ ID NO: 57, or of a nucleic acid encoding an orthologue or paralogue of the amino acid sequence of SEQ ID NO: 58.

**4.** Method according to any one of claims 1 to 3, wherein the nucleic acid encoding a PEAMT polypeptide is capable of hybridising to the nucleic acid represented by SEQ ID NO: 1 or is capable of hybridising to a nucleic acid encoding an orthologue, paralogue or homologue of SEQ ID NO: 58.

**5.** Method according to any one of claims 1 to 4, wherein said nucleic acid encoding a PEAMT polypeptide encodes an orthologue or paralogue of the sequence represented by SEQ ID NO: 58.

**6.** Method according to any one of claims 1 to 5, wherein said modulated expression is effected by introducing and expressing in a plant a nucleic acid encoding a PEAMT polypeptide.

**7.** Method according to any one of claims 1 to 6, wherein said enhanced yield-related traits comprising increased yield, preferably increased biomass and/or increased seed yield relative to control plants is obtained under non-stress conditions.

**8.** Method according to any one of claims 1 to 7, wherein said enhanced yield-related traits comprising increased yield, preferably increased biomass and/or increased seed yield relative to control plants is obtained under conditions of drought stress.

**9.** Method according to claim 6, 7 or 8 wherein said nucleic acid is operably linked to a constitutive promoter, preferably to a GOS2 promoter, most preferably to a GOS2 promoter from rice.

**10.** Method according to any one of claims 1 to 9, wherein said nucleic acid encoding a PEAMT polypeptide is of plant origin, preferably from a dicotyledonous plant, further preferably from the family Brassicaceae, more preferably from the genus Arabidopsis, most preferably from *Arabidopsis thaliana.*

**11.** Plant or part thereof, including seeds, obtainable by a method according to any preceding claim, wherein said plant or part thereof comprises a recombinant nucleic acid encoding a PEAMT polypeptide.

**12.** Construct comprising:

(i) A nucleic acid encoding a PEAMT polypeptide as defined in any of claims 1 to 5;
(ii) one or more control sequences capable of driving expression of the nucleic acid sequence of (a); and optionally
(iii) a transcription termination sequence.

**13.** Construct according to claim 12, wherein one of said control sequences is a constitutive promoter, preferably a GOS2 promoter, most preferably a GOS2 promoter from rice.

**14.** Use of a construct according to claim 12 or 13 in a method for making plants having an altered yield-related traits relative to control plants.

**15.** Plant, plant part or plant cell transformed with a construct according to claim 12 or 13.

**16.** Method for the production of a transgenic plant having enhanced yield-related traits relative to control plants, comprising:

(i) introducing and expressing in a plant a nucleic acid encoding a PEAMT polypeptide as defined in any one of claims 1 to 5; and
(ii) cultivating the plant cell under conditions promoting plant growth and development.

**17.** Transgenic plant having enhanced yield-related traits relative to control plants, resulting from modulated expression of a nucleic acid encoding a PEAMT polypeptide as defined in any one of claims 1 to 5.

**18.** Transgenic plant according to claim 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

**19.** Products derived from a plant according to claim 18.

**20.** Use of a nucleic acid encoding a PEAMT polypeptide as defined in any of claims 1 to 5 in altering yield-related traits of plants relative to control plants.

MAAGSVGVFATDEKIGSLLDQSITRHFLSTVTDQQ**GK**

**ICAEYVWIGGSMHDVRSKSRTLSTI**PTKPEDLPHWNY

DGSSTGQAPGHDSEVYLIPRSIFKDPFRGGDNILVMC

------2------

DCYEPPKVNPDGTLAAPKPI*PTNTRFACAEVMEKAKK*

*EEPWFGIEQEYTLLNAITKWPLGWPKGGYPAPQGPYY*

*CSAGAGVAIGRDVAEVHYRLCLAAGVNISGVNAEVLP*

*SQWEYQVGPCEGITMGDHMWMSRYIMYRVCEMFNVEV*

*SFDPKPIPGDWNGSGGHTNYSTKATRTAPDGWKVIQE*

----3---

*HCAKLEARHAVHIAAYGEGNERRLTGKHETSSMSDFS*

*WGVANRGCSIRVGRMVPVEKSGYYEDRRPASNLDAYV*

----1---

*VTRLIVETTIL*L

**FIGURE 1**

```
CLUSTAL 2.0.3 multiple sequence alignment

C.reinhardtii_129468         ----------------------------MAFALRGVTAKASGRTAGARSS
C.reinhardtii_136895         --------------------------------MR-LNTQVSGRATGAPRQ
V.carterii_103492            -----------------------------MATMR-MSTKAQGR-VGIVRN
A.anophagefferens_20700      -----------------------------------------MASMDQAVL
T.pseudonana_26051           MKLSIALLSMAATATAFAPSLTTPSRTTSLSMVNPLEIRTGKAQLDHSVI
C.reinhardtii_133971         --------------------MAAGS------VGVFATDEKIGSLLDQSIT
V.carterii_77041             --------------------MAAGS------IGVFATDEKIGSLLDQSIT
Helicosporidum_DQ323125      --------------------MSPPTGEKYSLPPVFGTQGQITQLLDPIMA
C.reinhardtii_147468         --------------------MDLATALGLGIAPPPPADDSSHHSTTEACT


C.reinhardtii_129468         GRTLT-VRVQAYGMKAEYIWADGNEGKPEKGMIFNEMRSKTKCFEAPLGL
C.reinhardtii_136895         GRRLT-VRVQAYGMKAEYIWADGNEGKAEKGMIFNEMRSKTKCFEAPLGL
V.carterii_103492            TRTLT-VRVRAYGMKAEYIWADGNEGRPEKGMIFNEMRSKTKVFDEALPL
A.anophagefferens_20700      GKYMG-LDT-GDDCQVEYVFLDKD----------QVARSKCRTLPLKKVQ
T.pseudonana_26051           DRFNA-LPYPADKVLAEYVWVDAK----------GECRSKTRTLPVARTT
C.reinhardtii_133971         RHFLSTVTDQQGKICAEYVWIGGS---------MHDVRSKSRTLSTIPTK
V.carterii_77041             RHFLTNVTDQCGKITAEYVWIGGS---------MQDLRSKSRTLTSVPTK
Helicosporidum_DQ323125      ERFKD--LSQHGKVMAEYVWIGGT---------GSDLRCKTRVLDSVPNS
C.reinhardtii_147468         LPAYLRAPEVTAQVMAEYIWLMGG---------TGQLRSKTKVLDAKPSC
                                            .**::                 *.* : :

C.reinhardtii_129468         -DASEYPDWSFDGSSTGQAEGNNSDCILRPVRVVTDPIR----GAPHVLV
C.reinhardtii_136895         -DASEYPDWSFDGSSTGQAEGNNSDCILRPVRVVTDPIR----GAPHVLV
V.carterii_103492            -EAGQYPDWSFDGSSTGQAAGNNSDCILRPVRVIKDPIR----GEPHVLV
A.anophagefferens_20700      GPVDAYPKWNYDGSSTGQAPGDDSEVMIVPRAKYPDPFR----GGNHVLV
T.pseudonana_26051           -AVDNLPRWNFDGSSTGQAPGDDSEVILRPCRIFKDPFRPRNDGVDNILV
C.reinhardtii_133971         --PEDLPHWNYDGSSTGQAPGHDSEVYLIPRSIFKDPFR----GGDNILV
V.carterii_77041             --PEDLPHWNYDGSSTGQAPGHDSEVYLIPRRIFRDPFR----GGDNILV
Helicosporidum_DQ323125      --VEDLPVWNYDGSSTGQAPGDDSEVFLIPRAIYRDPFR----GGDNILV
C.reinhardtii_147468         --AEEAPIMIVESNPDGQLAEPNHELFLKPRKIFRDPFR----GGDHILV
                                   *    :... **    : :  : *     **:*    *  ::**
```

FIGURE 2

```
C.reinhardtii_129468          MCEVFAP-----DG--------KPHSTNTRAKLREIIDD-KVTAEDCWYG
C.reinhardtii_136895          MCEVFAP-----DG--------KPHSTNTRAKLREIIDD-KVTAEDCWYG
V.carterii_103492             MCEVFAP-----DG--------TPHPTNTRAKLRDIIDD-KVLAEDCWYG
A.anophagefferens_20700       LCDTYEP-----DG--------TPLPTNTRAPAVARFESGGAKEQVPWYG
T.pseudonana_26051            MCDTYTP-----AG--------EALPTNTRAIAAKAFE--GKEDEEIWFG
C.reinhardtii_133971          MCDCYEPPKVNPDGTLAA---PKPIPTNTRFACAEVMEK--AKKEEPWFG
V.carterii_77041              MCDCYEPPKANADGILQP---PKPIPTNTRYACAEAMEK--AKDEEPWFG
Helicosporidum_DQ323125       LADTYEPPRVLPNGKVSP---PVPLPTNSRHACAEAMDK--AAAHEPWFG
C.reinhardtii_147468          LCDTFIVAQVVAEAGAAPSTVLQPSETNSRVACENVLRV--AEQQEPVFA
                              :.: :        .         .  **:*      :       .   :.

C.reinhardtii_129468          FEQEYTMLA--KTSGHIYG-------WPAGGFPA-PQGPFYCGVGAESAF
C.reinhardtii_136895          FEQEYTMLA--KTSGHIYG-------WPAGGFPA-PQGPFYCGVGAESAF
V.carterii_103492             LEQEYTMLQ--KTTGQIYG-------WPSGGYPA-PQGPFYCGVGAESAF
A.anophagefferens_20700       LEQEYTLFN--LDGVTPLG-------WPVGGFPK-PQGPYYCGAGADRAF
T.pseudonana_26051            LEQEFTLFN--LDQRTPLG-------WPKGGVPARAQGPYYCSVGPENSF
C.reinhardtii_133971          IEQEYTLLN--AITKWPLG-------WPKGGYPA-PQGPYYCSAGAGVAI
V.carterii_77041              IEQEYTLLN--AITKWPLG-------WPKGGYPA-PQGPYYCSAGAGVAI
Helicosporidum_DQ323125       IEQEYTVLD--ARTKWPLG-------WPSNGFPG-PQGPYYCAAGAGCAI
C.reinhardtii_147468          VEQEYAIIHPAYPTKVPLGPRRPSTSRASSCHSGSRRSSYVSSGSARGGI
                              .***::::          *        . .  .   :..: ..  .. .:

C.reinhardtii_129468          G------------------------------------------------R
C.reinhardtii_136895          G------------------------------------------------R
V.carterii_103492             G------------------------------------------------R
A.anophagefferens_20700       G------------------------------------------------R
T.pseudonana_26051            G------------------------------------------------R
C.reinhardtii_133971          G------------------------------------------------R
V.carterii_77041              G------------------------------------------------R
Helicosporidum_DQ323125       G------------------------------------------------R
C.reinhardtii_147468          GKNSSHHGGKQSHAAAAAAAAAVAGIPWPSPDACEQTAQEASAARQKASR
                              *                                                *
```

FIGURE 2 (continued)

```
C.reinhardtii_129468        PLAEAHMEACMKAGLVISGINAEVMPGQWEYQIGPVGPLALGDEVMLSRW
C.reinhardtii_136895        PLAEAHMEACMKAGLVISGINAEVMPGQWEYQIGPVGPLALGDEVMLSRW
V.carterii_103492           PLAEAHMEACMKAGLKISGINAEVMPGQWEYQIGPVGPLEMGDEVMLSRW
A.anophagefferens_20700     AVSEAHYRACLYAGLEVSGTNAEVMPGQWEYQIGPSIGIDAADQLTISRY
T.pseudonana_26051          AITDTMYRACLYAGIEISGTNGEVMPGQQEYQVGPCVGIDAGDQLQMSRY
C.reinhardtii_133971        DVAEVHYRLCLAAGVNISGVNAEVLPSQWEYQVGPCEGITMGDHMWMSRY
V.carterii_77041            DVAEVHYRLCLYAGVNISGVNAEVLPSQWEYQVGPCEGIEMGDHMWMSRY
Helicosporidum_DQ323125     DLIEAHLKACLFAGINVSGVNAEVMPSQWEYQVGPCTGIESGDQMWMSRY
C.reinhardtii_147468        QLADSHLRCCLFAGVRVTGADVHSLDGLHSYKIGPSPGVDLGDDLWTSRY
                             : :   . *: **: ::* : . : .  .*::**   :  .*.:  **:

C.reinhardtii_129468        LLHRLGEDFGIVSTFNPKPVRTGDWNGTGAHTNFSTKGMR-VPGGMKVIE
C.reinhardtii_136895        LLHRLGEDFGIVSTFNPKPVRTGDWNGTGAHTNFSTKGMR-VPGGMKVIE
V.carterii_103492           LLHRLGEDFGIVCTFNPKPVRTGDWNGTGAHTNFSTKSMR-QPGGMKVIE
A.anophagefferens_20700     ILSRVCEDLGVIVTIDPKPIAG-DWNGAGMHINFSTESTR-KEGGLAVIE
T.pseudonana_26051          ILQRVCEEFQVYCTLHPKPIVEGDWNGAGMHTNVSTKSMR-EEGGLEVIK
C.reinhardtii_133971        IMYRVCEMFNVEVSFDPKPIPG-DWNGSGGHTNYSTKATRTAPDGWKVIQ
V.carterii_77041            IMYRVCEMFNVEVSFDPKPIPG-DWNGSGGHTNYSTKATRTAPNGWKAIQ
Helicosporidum_DQ323125     ILIRCAELYNVEVSFDPKPVPG-DWNGAGGHVNYSNKATRTAETGWAAIQ
C.reinhardtii_147468        LLQRVAEQHSASVSWEPDSMPS--ERPLGCHFKYSTASTRQAPHGLNAIE
                            :: *  *      : .*..:     .  * * : *. . *    *  .*:

C.reinhardtii_129468        EAVEKLSKTHIEHITQYGIGNEARLTGKHETCDINTFKHGVADRGSSIRI
C.reinhardtii_136895        EAVEKLSKTHIEHITQYGIGNEARLTGKHETCDINTFKHGVADRGSSIRI
V.carterii_103492           DAVEKLSKTHIEHITQYGLGNEARLTGKHETCDINTFKHGVADRGSSIRI
A.anophagefferens_20700     AMCEKLGAKHTEHIAAYGEGNERRLTGDCETASIDQFSYGVADRGCSIRI
T.pseudonana_26051          KAIYKLGAKHQEHIAVYGEGNELRLTGKHETASIDQFSFGVANRGASVRI
C.reinhardtii_133971        EHCAKLEARHAVHIAAYGEGNERRLTGKHETSSMSDFSWGVANRGCSIRV
V.carterii_77041            EHCQKLEARHAVHIAAYGEGNERRLTGKHETSSMNDFSWGVANRGCSIRV
Helicosporidum_DQ323125     QQVEKLGKRHAVHIAAYGEGNERRLTGKHETSSMNDFSWGVANRGASVRV
C.reinhardtii_147468        QQLVRLQATHVQHQVAYNDGRLDRLS----SPEASTFTHAVGSANASVVV
                                :*   *  * . *. *.  **:    : . . *. .*.. ..*: :
```

FIGURE 2 (continued)

```
C.reinhardtii_129468         PLPVMLKGYGYLEDRRPAANVDPYTVARLLIKTVLKG-------------
C.reinhardtii_136895         PLPVMLKGYGYLEDRRPAANVDPYTVARLLIKTVLKG-------------
V.carterii_103492            PLPVMLKGYGYLEDRRPAANVDPYTVARLLIKSILKGPQ-----------
A.anophagefferens_20700      PRDTAADKKGYLEDRRPASNVDPYVATSLIFATCTSA-------------
T.pseudonana_26051           GRDTEAEGKGYFEDRRPSSNADPYLVTGKIMATIMEDVDVPEISALDRAE
C.reinhardtii_133971         GRMVPVEKSGYYEDRRPASNLDAYVVTRLIVETTILL-------------
V.carterii_77041             GRMVPVEKCGYYEDRRPASNLDPYVVTKLIVETTVLL-------------
Helicosporidum_DQ323125      GRLVPVEKCGYYEDRRPASNLDPYVVTRLLVETTLLM-------------
C.reinhardtii_147468         PSLTFLQQGGYFTDRRPPSDADPYKVTLLLAATTLDIPLPKLPASSSAGN
                                .  .  **  ****.:: *.* .:  :  :

C.reinhardtii_129468         --------------------------------------------------
C.reinhardtii_136895         --------------------------------------------------
V.carterii_103492            --------------------------------------------------
A.anophagefferens_20700      --------------------------------------------------
T.pseudonana_26051           A-------------------------------------------------
C.reinhardtii_133971         --------------------------------------------------
V.carterii_77041             --------------------------------------------------
Helicosporidum_DQ323125      --------------------------------------------------
C.reinhardtii_147468         TAANCSGGMSAGPSSCPAAAALPFGSPMQSYLLAAAAAQRQQQQQHLMFD

C.reinhardtii_129468         --------------------------------------------------
C.reinhardtii_136895         --------------------------------------------------
V.carterii_103492            --------------------------------------------------
A.anophagefferens_20700      --------------------------------------------------
T.pseudonana_26051           --------------------------------------------------
C.reinhardtii_133971         --------------------------------------------------
V.carterii_77041             --------------------------------------------------
Helicosporidum_DQ323125      --------------------------------------------------
C.reinhardtii_147468         TESEECDSVDEDDAMTEDSAALLAKMDDDGGAAEASSCDSDFEDQDDASS
```

FIGURE 2 (continued)

```
C.reinhardtii_129468          --------------------
C.reinhardtii_136895          --------------------
V.carterii_103492             --------------------
A.anophagefferens_20700       --------------------
T.pseudonana_26051            --------------------
C.reinhardtii_133971          --------------------
V.carterii_77041              --------------------
Helicosporidum_DQ323125       --------------------
C.reinhardtii_147468          SPITGTWADNDCTHMLGAGI
```

FIGURE 2 (continued)

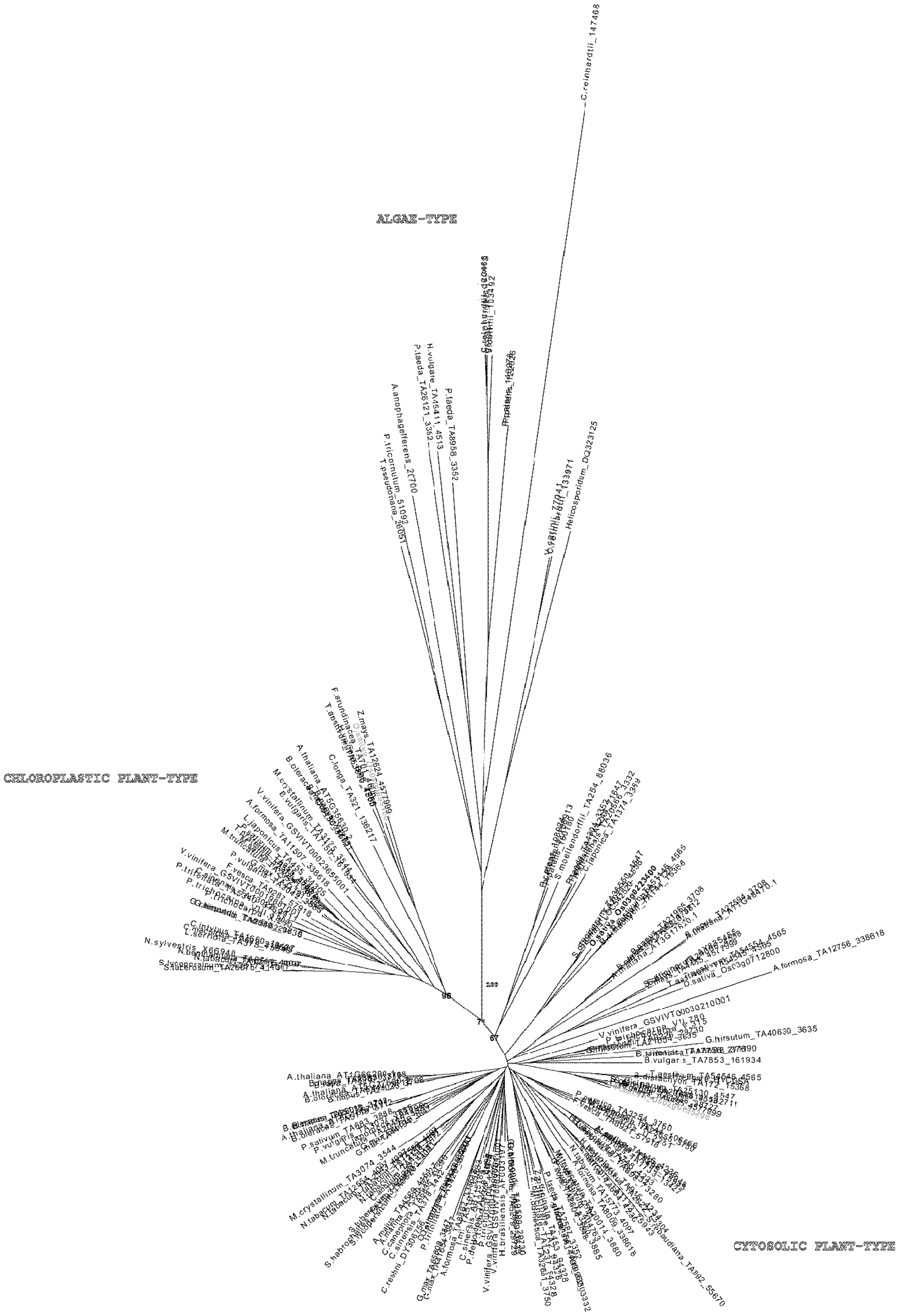
C.reinhardtii_147468
ALGAE-TYPE
CHLOROPLASTIC PLANT-TYPE
A.formosa_TA12756_338618
CYTOSOLIC PLANT-TYPE

Figure 3 A

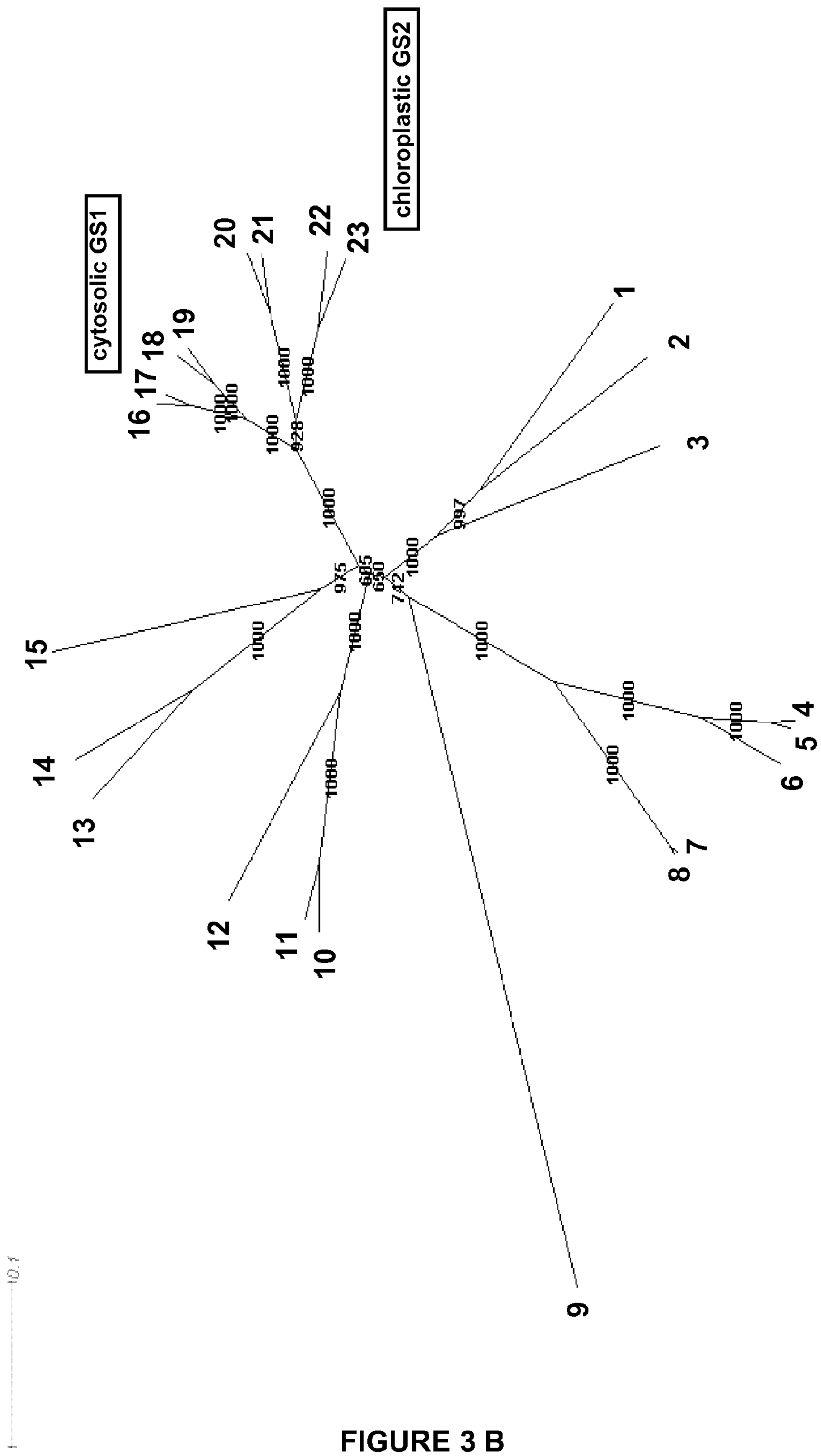
cytosolic GS1
chloroplastic GS2
1
2
3
4
5
6
7
8
9
10
11
12
13
14
15
16
17
18
19
20
21
22
23
0.1

FIGURE 3 B

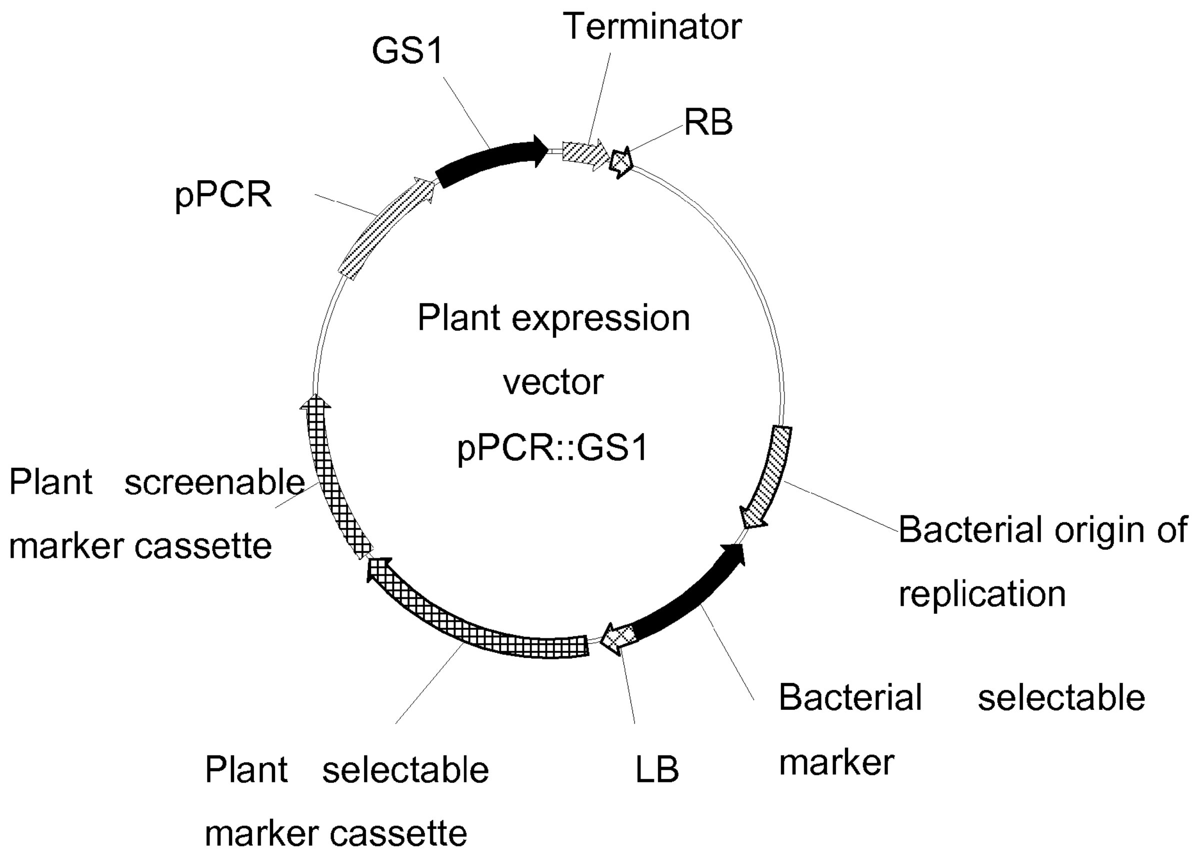
Terminator
GS1
RB
pPCR
Plant expression vector pPCR::GS1
Plant screenable marker cassette
Bacterial origin of replication
Bacterial selectable marker
Plant selectable marker cassette
LB

FIGURE 4

```
AT3gG18000        ------------------------------------------------------------
Arath_PEAMT_1     ------------------------------------------------------------
AT1G48600_1       ------------------------------------------------------------
Pt\PEAMT2         ------------------------------------------------------------
Pt\PEAMT1         ------------------------------------------------------------
AT1G73600_1       MVGEEDSERAQSSKMEIERESNLGSASVLMQSKVISVSNFFSIHRFHYPREKIVSFLFPS
Os05g47540_3      ------------------------------------------------------------
Os05g47540_2      ----------------------------------------------------MDAV----
Os05g47540_1      ----------------------------------------------------MRA-----
Zm\PEAMTa         ----------------------------------------------------MDTVGVPV
Os01g50030        ----------------------------------------------------MDAAAATA
Zm\PEAMTc         ------------------------------------------------------------
Zm\PEAMTb         ----------------------------------------------------M----AAA

AT3gG18000        ----MAASYEEERDIQKNYWIEHSADLTVEAMMLDSRASDLDKEERPEVLSLLPPYEGKS
Arath_PEAMT_1     --------------------MEHSSDLTVEAMMLDSKASDLDKEERPEVLSLIPPYEGKS
AT1G48600_1       --------------------MEHSSDLTVEAMMLDSKASDLDKEERPEVLSLIPPYEGKS
Pt\PEAMT2         -----------------------MTYVVLKGYLYDP------------------------
Pt\PEAMT1         -----MATHVEERDIQKKYWMDNISDLSVNAMMLDSKASELDKEERPEILSLLPPYEGKT
AT1G73600_1       VFSRIMASYGEEREIQKNYWKEHSVGLSVEAMMLDSKASDLDKEERPEILAFLPPIEGTT
Os05g47540_3      ------------------------------------------------------------
Os05g47540_2      --AANGIGEV-ERKAQRSYWEEHSKDLTVEAMMLDSRAADLDKEERPEVLSVLPSYKGKS
Os05g47540_1      -----GIGEV-ERKAQRSYWEEHSKDLTVEAMMLDSRAADLDKEERPEVLSVLPSYKGKS
Zm\PEAMTa         VAVANGIGEV-ERKVQKSYWEEHSKCLTVESMMLDSRAADLDKEERPEILSLLPSYKGKS
Os01g50030        V---NGVLEVEERKAQKSYWEEHSKDLTVEAMMLDSRAADLDKEERPEILSLLPPYEGKS
Zm\PEAMTc         ------------------------------------------------------------
Zm\PEAMTb         V---NGSLDVHERKAQKSYWEEHSGELNLEAIMLDSRAAELDKEERPEVLSLLPSYEGKS

AT3gG18000        VLELGAGIGRFTGELAQKAGELIALDFIDNVIKKNESINGHYKNVKFMCADVTSPDLKIT
Arath_PEAMT_1     VLELGAGIGRFTGELAQKAGEVIALDIIESAIQKNESVNGHYKNIKFMCADVTSPDLKIK
AT1G48600_1       VLELGAGIGRFTGELAQKAGEVIALDFIESAIQKNESVNGHYKNIKFMCADVTSPDLKIK
Pt\PEAMT2         ------------------------IDCVRHAV-------------------ATEPG----
Pt\PEAMT1         VLELGAGIGRFTGELAQKAGQVVALDFIESAIKKNENINGHYKNVKFMCADVTSPDLNIS
AT1G73600_1       VLEFGAGIGRFTTELAQKAGQVIAVDFIESVIKKNENINGHYKNVKFLCADVTSPNMNFP
Os05g47540_3      -----------------------------------------------MCADVTSPDLTIE
Os05g47540_2      VLELGAGIGRFTGELAKEAGHVLALDFIESVIKKNENINGHHKNITFMCADVTSPDLTIE
Os05g47540_1      VLELGAGIGRFTGELAKEAGHVLALDFIESVIKKNENINGHHKNITFMCADVTSPDLTIE
Zm\PEAMTa         VLELGAGIGRFTGDLAKEAGHVLALDFIESVIKKNQSINGHHKNITFRCADVTSNDLKIE
Os01g50030        VLELGAGIGRFTGELVKTAGHVLAMDFIESVIKKNESINGHHKNASFMCADVTCPDLMIE
Zm\PEAMTc         ------------------------------------------------------------
Zm\PEAMTb         ILELGAGIGRFTGELAKTSGHVFAVDFVESVIKKNGSINDHYGNTSFMCADVTSPDLMIE

AT3gG18000        DGSLDLIFSNWLLMYLSDKEVELLAERMVGWIKVGGYIFFRESCFHQSGDSKRKSNPTHY
Arath_PEAMT_1     DGSIDLIFSNWLLMYLSDKEVELMAERMIGWVKPGGYIFFRESCFHQSGDSKRKSNPTHY
AT1G48600_1       DGSIDLIFSNWLLMYLSDKEVELMAERMIGWVKPGGYIFFRESCFHQSGDSKRKSNPTHY
Pt\PEAMT2         -------------------KVENLVERMVKWLKVGGFIFFRESCFHQSGDSKRKYNPTHY
Pt\PEAMT1         EGSVDLIFSNWLLMYLSDKEVENLVERMVKWVKVDGFIFFRESCFHQSGDSKRKYNPTHY
AT1G73600_1       NESMDLIFSNWLLMYLSDQEVEDLAKKMLQWTKVGGYIFFRESCFHQSGDNKRKYNPTHY
Os05g47540_3      DNSIDLIFSNWLLMYLSDEEVEKLVGRMVKWLKVGGHIFFRESCFHQSGDSKRKVNPTHY
Os05g47540_2      DNSIDLIFSNWLLMYLSDEEVEKLVGRMVKWLKVGGHIFFRESCFHQSGDSKRKVNPTHY
Os05g47540_1      DNSIDLIFSNWLLMYLSDEEVEKLVGRMVKWLKVGGHIFFRESCFHQSGDSKRKVNPTHY
Zm\PEAMTa         DNSVDLIFSNWLLMYLSDEEVQKLVGKMVKWLKVGGHIFFRESCFHQSGDSKRKVNPTHY
Os01g50030        DNSIDLIFSNWLLMYLSDEEVEKLVKRMVRWLKVGGYIFFRESCFHQSGDSKRKVNPTHY
Zm\PEAMTc         -------------MYLSDEEVEQLVQRMVKWLKVGGYIFFRESCFHQSGDSKRKVNPTHY
Zm\PEAMTb         ANSIDLIFSNWLLMYLSDEEIDKLVERMVKWLKVGGYIFFRESCFHQSGDTERKFNPTHY
                                     ::: :. :*: * * .*.*************.:** *****
```

# FIGURE 5

```
AT3gG18000          REPRFYSKVFQECQTRDAAGNSFELSMIGCKCIGAYVKNKKNQNQICWIWQKVSSENDRG
Arath_PEAMT_1       REPRFYTKVFQECQTRDASGNSFELSMVGCKCIGAYVKNKKNQNQICWIWQKVSVESDKD
AT1G48600_1         REPRFYTKVFQECQTRDASGNSFELSMVGCKCIGAYVKNKKNQNQICWIWQKVSVENDKD
Pt\PEAMT2           REPRFYTK-------------------------------------ICWIWQKVSSNDDKG
Pt\PEAMT1           REPRFYTKVFKECHTRDGSGDSFELSLVGCKCISAY---------ICWIWQKVSSYEDKG
AT1G73600_1         REPKFYTKLFKECHMNDEDGNSYELSLVSCKCIGAYVRNKKNQNQICWLWQKVSSDNDRG
Os05g47540_3        REPRFYTKIFKECHSYDKDGGSYELSLETCKCIGAYVKSKKNQNQLCWLWEKVKSTEDRG
Os05g47540_2        REPRFYTKIFKECHSYDKDGGSYELSLETCKCIGAYVKSKKNQNQLCWLWEKVKSTEDRG
Os05g47540_1        REPRFYTKIFKECHSYDKDGGSYELSLETCKCIGAYVKSKKNQNQLCWLWEKVKSTEDRG
Zm\PEAMTa           REPRFYTKVFKEGHSFDQDGGSFELSLVTCKCIGAYVKNKKNQNQICWLWEKVKSTEDRD
Os01g50030          REPRFYTKVFKECQALDQDGNSFELSVLTCKCVGAYVKSKKNQNQICWLWQKVDSTEDRG
Zm\PEAMTc           REPSFYTKVFKECHTFDQDGNSFELSLVTCKCIGAYVKNKKNQNQICWLWQKVHSTEDKG
Zm\PEAMTb           REPRFYTKVFKECQTFNQDGTSFKLSLITFKCIGAYVNIKKDQNQICWLWKKVNSSEDGG
                    *** **:*                                     :**:*:**   .* .

AT3gG18000          FQRFLDNVQYKSSGILRYERVFGQGFVSTGGLETTKEFVEKMNLKPGQKVLDVGCGIGGG
Arath_PEAMT_1       FQRVLDNVQYKSSGILRYERVFGEGYVSTGGFETTKEFVAKMDLKPGQKVLDVGCGIGGG
AT1G48600_1         FQRFLDNVQYKSSGILRYERVFGEGYVSTGGFETTKEFVAKMDLKPGQKVLDVGCGIGGG
Pt\PEAMT2           FQRFLDNVQYKSNGILRYERVFGQGFVSTGGMETTKEFVEKLDLKPGQKVLDVGCGIGGG
Pt\PEAMT1           FQRFLDNVQYKSNGILRYERVFGQGYVSTGGIETTKEFVGKLDLKPGQKVLDVGCGIGGG
AT1G73600_1         FQRFLDNVQYKSSGILRYERVFGEGFVSTGGLETTKEFVDMLDLKPGQKVLDVGCGIGGG
Os05g47540_3        FQRFLDNVQYKTTGILRYERVFGEGYVSTGGIETTKEFVDKLDLKPGQKVLDVGCGIGGG
Os05g47540_2        FQRFLDNVQYKTTGILRYERVFGEGYVSTGGIETTKEFVDKLDLKPGQKVLDVGCGIGGG
Os05g47540_1        FQRFLDNVQYKTTGILRYERVFGEGYVSTGGIETTKEFVDKLDLKPGQKVLDVGCGIGGG
Zm\PEAMTa           FQRFLDNVQYKTSGILRYERVFGEGFVSTGGIETTKEFVGMLDLKPGQKVLDVGCGIGGG
Os01g50030          FQRFLDNVQYKASGILRYERIFGEGFVSTGGIETTKEFVDRLDLKPGQNVLDVGCGIGGG
Zm\PEAMTc           FQRFLDNVQYKASGILRYERIFGEGYVSTGGVETTKEFVDKLDLKPGHKVLDVGCGIGGG
Zm\PEAMTb           FQSFLDNVQYKATGILRYERIFGDGYVSTGGAETTKEFVEKLNLKPGQKVLDVGCGIGGG
                    ** .*******:.*******:**:*:***** *******  ::****::***********

AT3gG18000          DFYMAEKFDVHVVGIDLSVNMISFALERAIGLSCSVEFEVADCTTKHYPDNSFDVIYSRD
Arath_PEAMT_1       DFYMAENFDVHVVGIDLSVNMISFALERAIGLKCSVEFEVADCTTKTYPDNSFDVIYSRD
AT1G48600_1         DFYMAENFDVHVVGIDLSVNMISFALERAIGLKCSVEFEVADCTTKTYPDNSFDVIYSRD
Pt\PEAMT2           DFYMAENFEVEVVGIDLSVNMISFALERAIGLKCSVEFEVADCTTKTYPDNTFDVIYSRD
Pt\PEAMT1           DFYMAENFDVEVVGIDLSINMISFALERAIGLKCSVEFEVADCTTKTYPDNTFDVIYSRD
AT1G73600_1         DFYMAENFDVDVVGIDLSVNMISFALEHAIGLKCSVEFEVADCTKKEYPDNTFDVIYSRD
Os05g47540_3        DFYMAENYDAHVLGIDLSINMVSFAIERAIGRKCSVEFEVADCTTKTYAPNTFDVIYSRD
Os05g47540_2        DFYMAENYDAHVLGIDLSINMVSFAIERAIGRKCSVEFEVADCTTKTYAPNTFDVIYSRD
Os05g47540_1        DFYMAENYDAHVLGIDLSINMVSFAIERAIGRKCSVEFEVADCTTKTYAPNTFDVIYSRD
Zm\PEAMTa           DFYMAANYDVHVLGIDLSVNMVSFAIERAIGRKCSVEFEVADCTTKDYPENSFDVIYSRD
Os01g50030          DFYMADKYDVHVVGIDLSINMVSFALERAIGRKCSVEFEVADCTKKTYPDNTFDVIYSRD
Zm\PEAMTc           DFYMAEKYDAHVVGIDLSINMVSFALERAIGRSCSVEFEVADCTTKTYPDNTFDVIYSRD
Zm\PEAMTb           DFYMAEKYGTHVVGIDLSINMIMFALERSIGCKCLVEFEVADCTTKTYPDHMFDVIYSRD
                    ***** :: ..*:*****:**: **:*::** .* *********.* *. : ********

AT3gG18000          TILHIQDKPALFRTFFKWLKPGGKVLISDYCRSPKTPSAEFSEYIKQRGYDLHDVQAYGQ
Arath_PEAMT_1       TILHIQDKPALFRTFFKWLKPGGKVLITDYCRSAETPSPEFAEYIKQRGYDLHDVQAYGQ
AT1G48600_1         TILHIQDKPALFRTFFKWLKPGGKVLITDYCRSAETPSPEFAEYIKQRGYDLHDVQAYGQ
Pt\PEAMT2           TILHIQDKPALFRSFFKWLKPGGKVLISDYCKCAGTPSPEFAEYIKQRGYDLHDVKAYGQ
Pt\PEAMT1           TILHIQDKPALFRSFFKWLKPGGKVLISDYCKCDGTPSPEFAEYIKQRGYDLHDVKAYGQ
AT1G73600_1         TILHIQDKPALFRRFYKWLKPGGKVLITDYCRSPKTPSPDFAIYIKKRGYDLHDVQAYGQ
Os05g47540_3        TILHIHDKPALFRSFFKWLKPGGKVLISDYCRNPGKPSEEFAAYIKQRGYDLHDVKTYGK
Os05g47540_2        TILHIHDKPALFRSFFKWLKPGGKVLISDYCRNPGKPSEEFAAYIKQRGYDLHDVKTYGK
Os05g47540_1        TILHIHDKPALFRSFFKWLKPGGKVLISDYCRNPGKPSEEFAAYIKQRGYDLHDVKTYGK
Zm\PEAMTa           TILHIQDKPALFRSFFKWLKPGGKVLISDYCKNPGKPSEEFAAYIKQRGYDLHDVKAYGQ
Os01g50030          TILHIQDKPSLFKSFFKWLKPGGKVLISDYCKCPGKPSEEFAAYIKQRGYDLHDVRAYGQ
Zm\PEAMTc           TILHIHDKPSLFKSFFKWLKPGGKVLISDYCRSPGKPSEEFAAYIKQRGYDLHAVEAYGQ
Zm\PEAMTb           TILHIQDKPSLFKSFFKWLKPGGKVLISDYCKSPGKPSEEFATYIKQRGYDLHDVEAYGQ
                    *****:***:**: *:***********:***:   .** :*: ***:****** *.:**:
```

FIGURE 5 (continued)

```
AT3gG18000      MLKDAGFTDVIAEDRTDQFMQVLKRELDRVEKEKEKFISDFSKEDYDDIVGGWKSKLERC
Arath_PEAMT_1   MLKDAGFDDVIAEDRTDQFVQVLRRELEKVEKEKEEFISDFSEEDYNDIVGGWSAKLERT
AT1G48600_1     MLKDAGFDDVIAEDRTDQFVQVLRRELEKVEKEKEEFISDFSEEDYNDIVGGWSAKLERT
Pt\PEAMT2       MLRDAGFDEVIAEDRTDQFNQVLLRELKAIEKEKDEFIHDFSEEDYNDIVGGWKAKLIRS
Pt\PEAMT1       MLRDAGFDEVVAEDRTDQFNKVLQRELNAIEKDKDEFIHDFSEGDYNDIVGGWKAKLIRS
AT1G73600_1     MLRDAGFEEVIAEDRTDQFMKVLKRELDAVEKEKEEFISDFSKEDYEDIIGGWKSKLLRS
Os05g47540_3    MLEDAGFHHVIAEDRTDQFLRVLQRELAEVEKNKEAFMADFTQEDYDDIVNGWNAKLKRS
Os05g47540_2    MLEDAGFHHVIAEDRTDQFLRVLQRELAEVEKNKEAFMADFTQEDYDDIVNGWNAKLKRS
Os05g47540_1    MLEDAGFHHVIAEDRTDQFLRVLQRELAEVEKNKEAFMADFTQEDYDDIVNGWNAKLKRS
Zm\PEAMTa       MLKDAGFHNVIAEDRTEQFLNVLQRELGEVEKNKDAFLADFTQEDYDDIVNGWNAKLKRS
Os01g50030      MLENAGFHDVIAEDRTDQFLDVLERELAKVEKNKNEFVSDFSQEDYDAIVNGWKAKLQRS
Zm\PEAMTc       MLKSAGFRDVIAEDRTDQFLGVLDKELAEFEKNKDDFLSDFTQEDYDDIVNGWKAKLQRS
Zm\PEAMTb       MLKDAGFHNVIAEDRTEQFLNVLQREIGEVEKNKDAFLADFTQEDYDDIVNGWNAKLKRS
                **..*** .*:*****:**  ** :*:  .**:*: *: **:: **: *:.**.:** *

AT3gG18000      ASDEQKWGLFIANKN
Arath_PEAMT_1   ASGEQKWGLFIADKK
AT1G48600_1     ASGEQKWGLFIADKK
Pt\PEAMT2       SSGEQRWGLFIAKKK
Pt\PEAMT1       SSGEQRWGLFIAKKK
AT1G73600_1     SSGEQKWGLFIAKRN
Os05g47540_3    SAGEQRWGLFIATK-
Os05g47540_2    SAGEQRWGLFIATK-
Os05g47540_1    SAGEQRWGLFIATK-
Zm\PEAMTa       SAGEQRWGLFIATK-
Os01g50030      SAGEQRWGLFIATK-
Zm\PEAMTc       SAGEQRWGLFIATK-
Zm\PEAMTb       SGGEQRWGLFIATK-
```

## FIGURE 5 (continued)

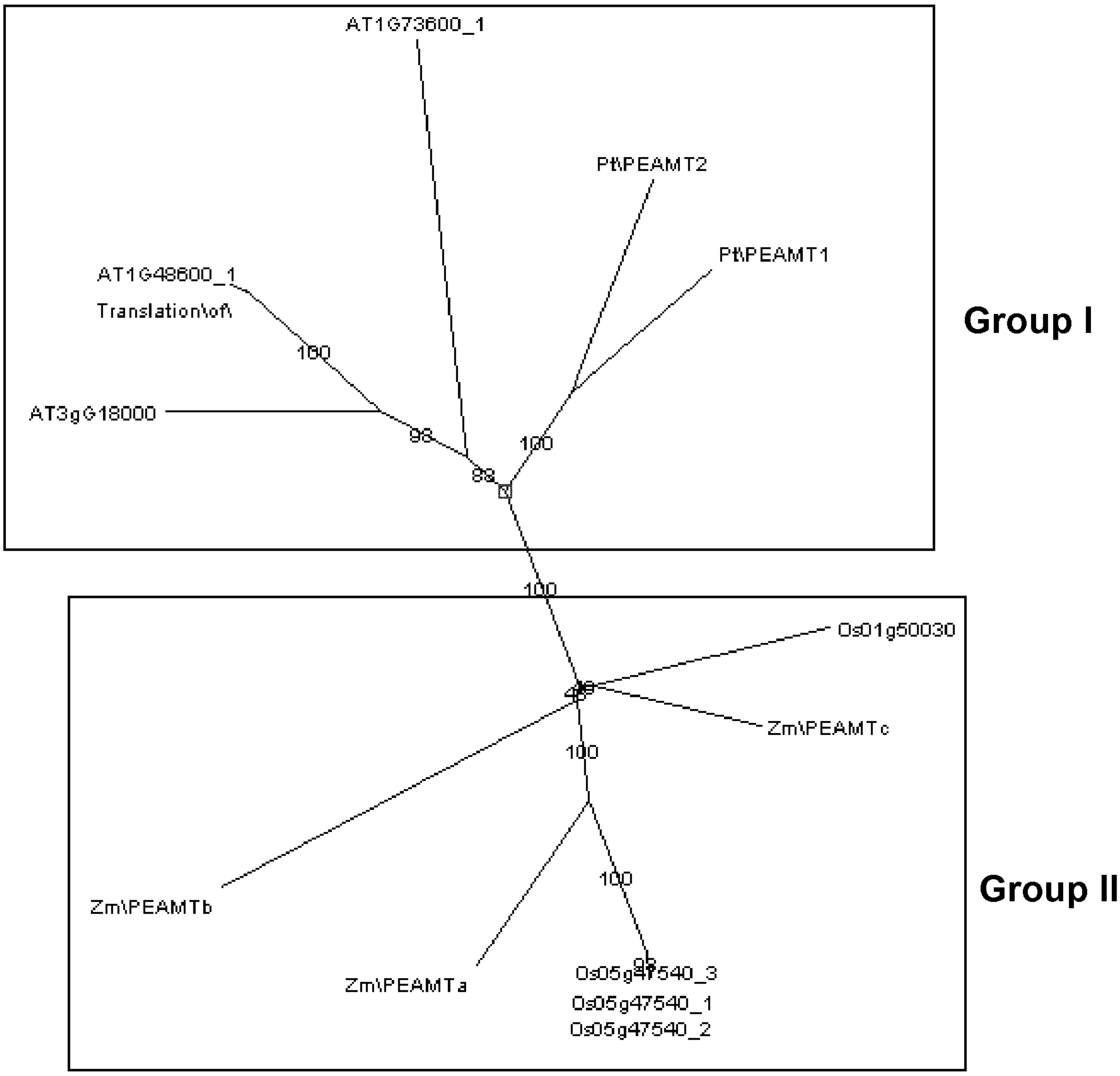
AT1G73600_1
Pt\PEAMT2
Pt\PEAMT1
AT1G48600_1
Translation\of\
100
AT3gG18000
98
100
88
Group I
100
Os01g50030
48
Zm\PEAMTc
100
Zm\PEAMTb
100
Group II
Zm\PEAMTa
Os05g47540_3
Os05g47540_1
Os05g47540_2

**FIGURE 6**

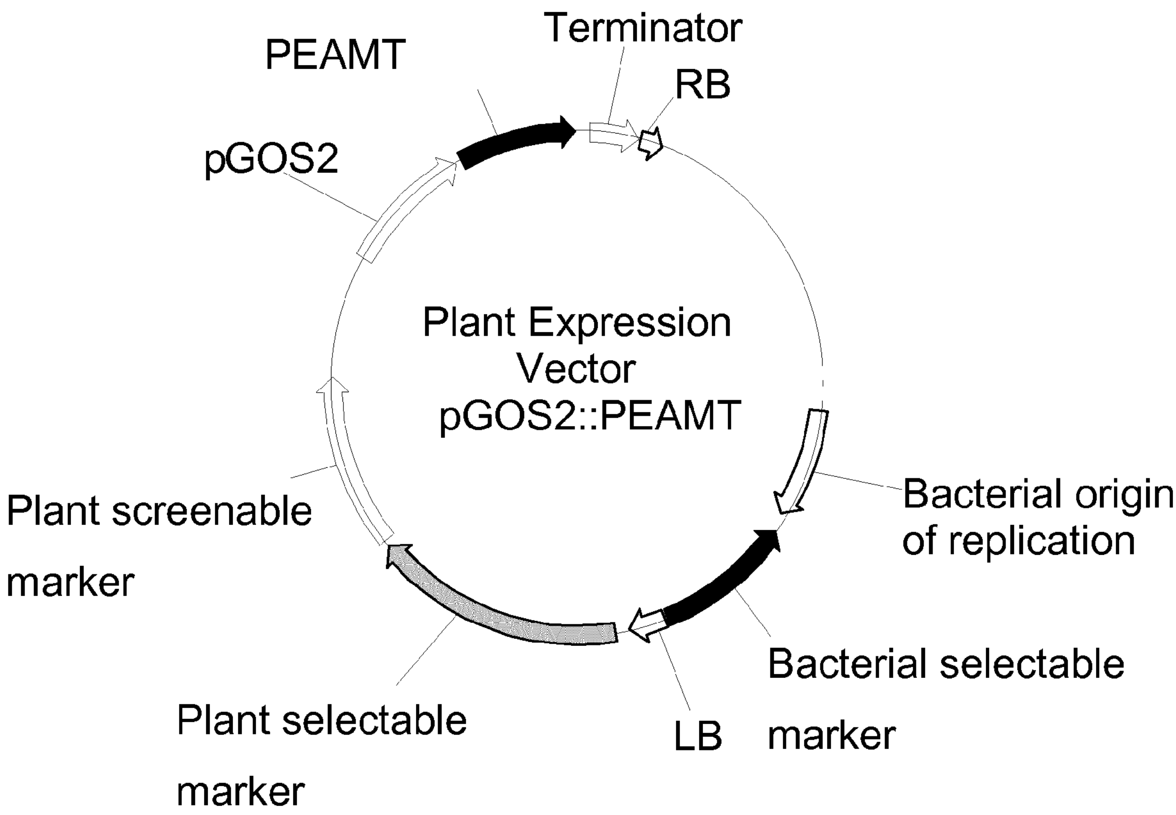
Terminator
PEAMT
RB
pGOS2
Plant Expression
Vector
pGOS2::PEAMT
Bacterial origin
of replication
Plant screenable
marker
Bacterial selectable
marker
Plant selectable
marker
LB

FIGURE 7

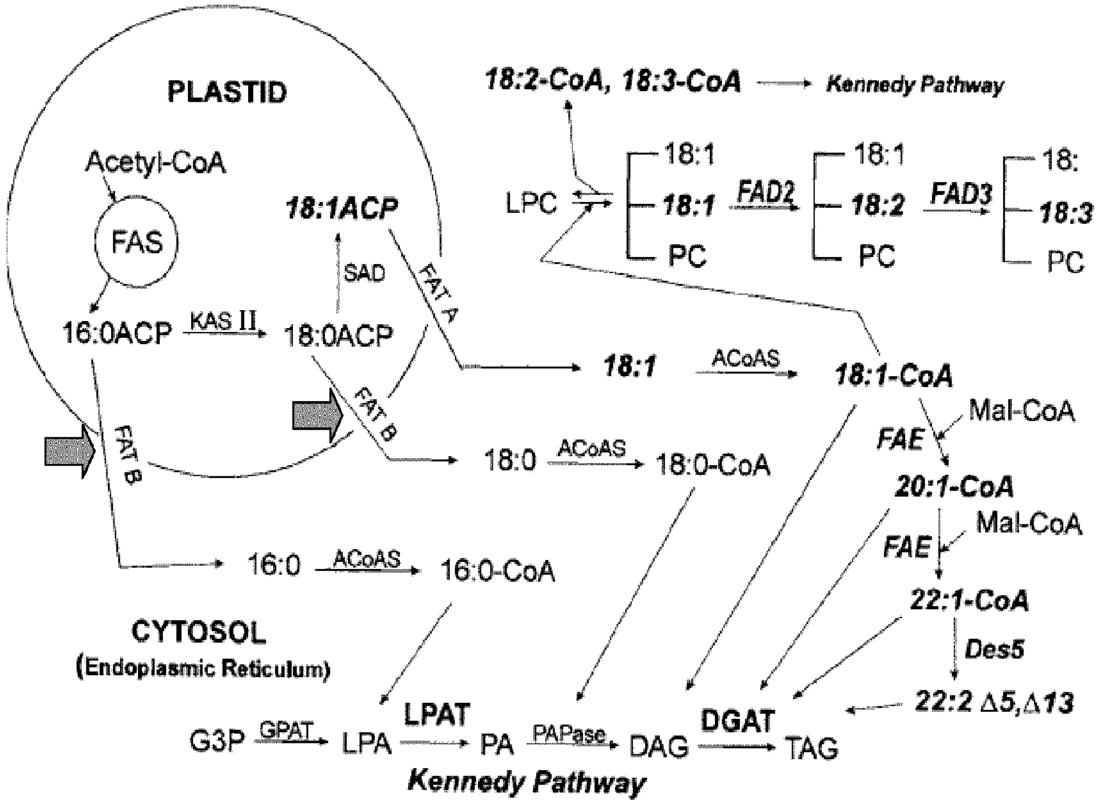


FIGURE 8

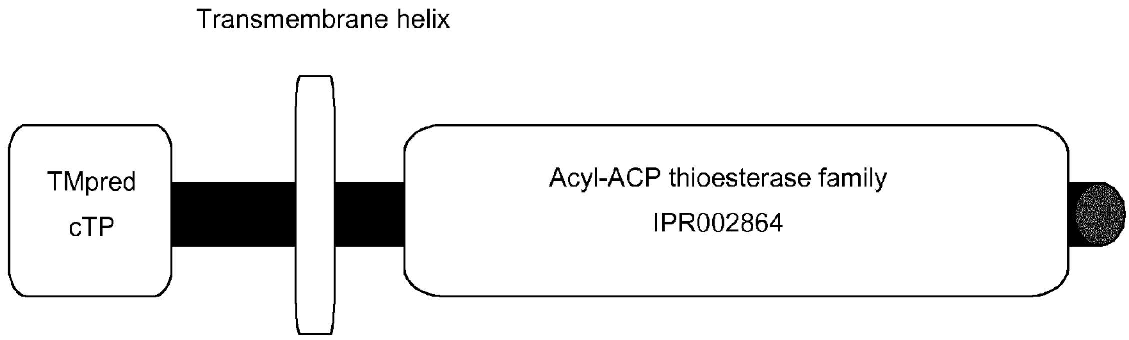


FIGURE 9

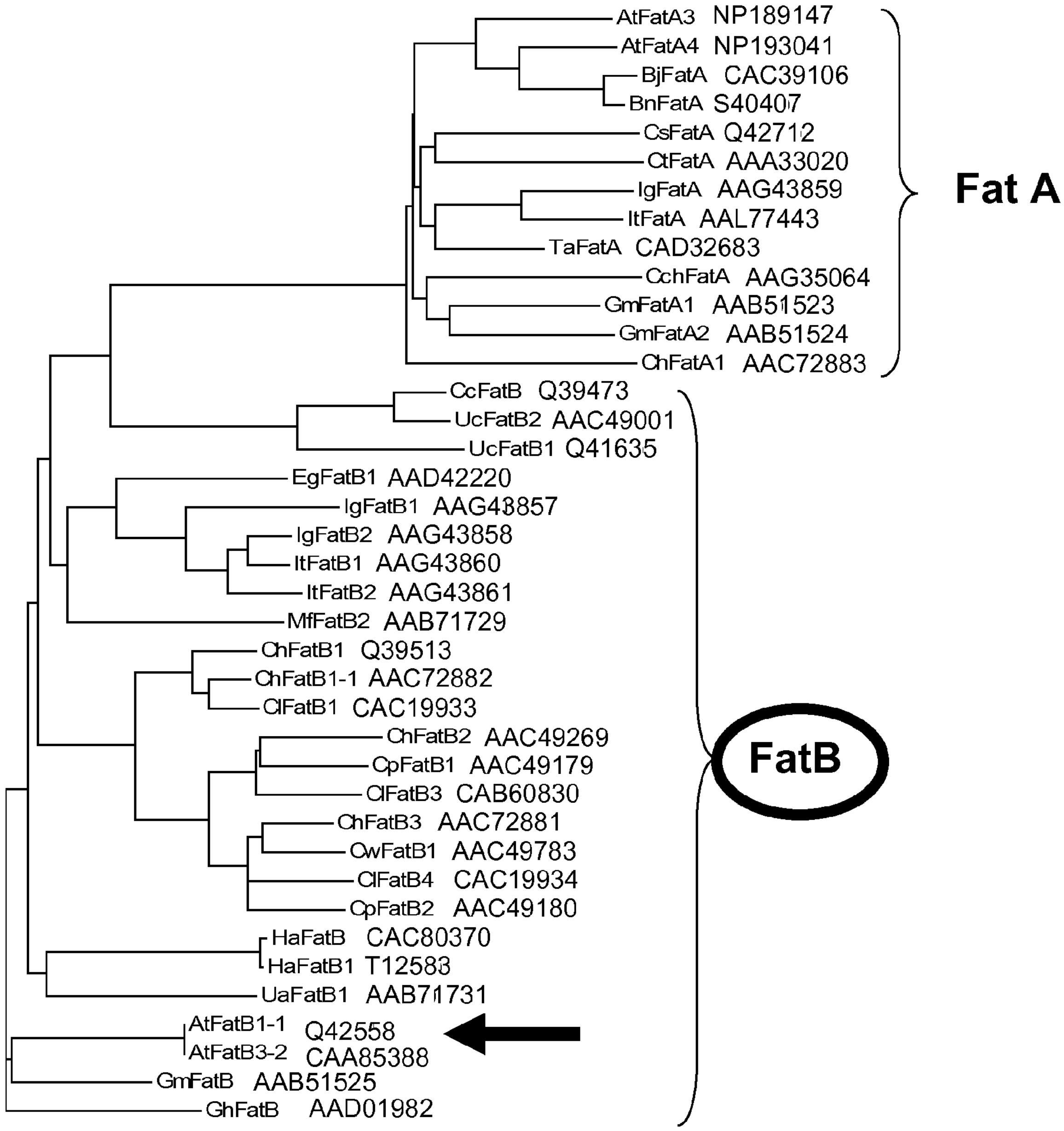
AtFatA3 NP189147
AtFatA4 NP193041
BjFatA CAC39106
BnFatA S40407
CsFatA Q42712
CtFatA AAA33020
IgFatA AAG43859
ItFatA AAL77443
TaFatA CAD32683
CchFatA AAG35064
GmFatA1 AAB51523
GmFatA2 AAB51524
ChFatA1 AAC72883
Fat A
CcFatB Q39473
UcFatB2 AAC49001
UcFatB1 Q41635
EgFatB1 AAD42220
IgFatB1 AAG43857
IgFatB2 AAG43858
ItFatB1 AAG43860
ItFatB2 AAG43861
MfFatB2 AAB71729
ChFatB1 Q39513
ChFatB1-1 AAC72882
ClFatB1 CAC19933
ChFatB2 AAC49269
CpFatB1 AAC49179
ClFatB3 CAB60830
ChFatB3 AAC72881
CwFatB1 AAC49783
ClFatB4 CAC19934
CpFatB2 AAC49180
HaFatB CAC80370
HaFatB1 T12583
UaFatB1 AAB71731
AtFatB1-1 Q42558
AtFatB3-2 CAA85388
GmFatB AAB51525
GhFatB AAD01982
FatB

FIGURE 10

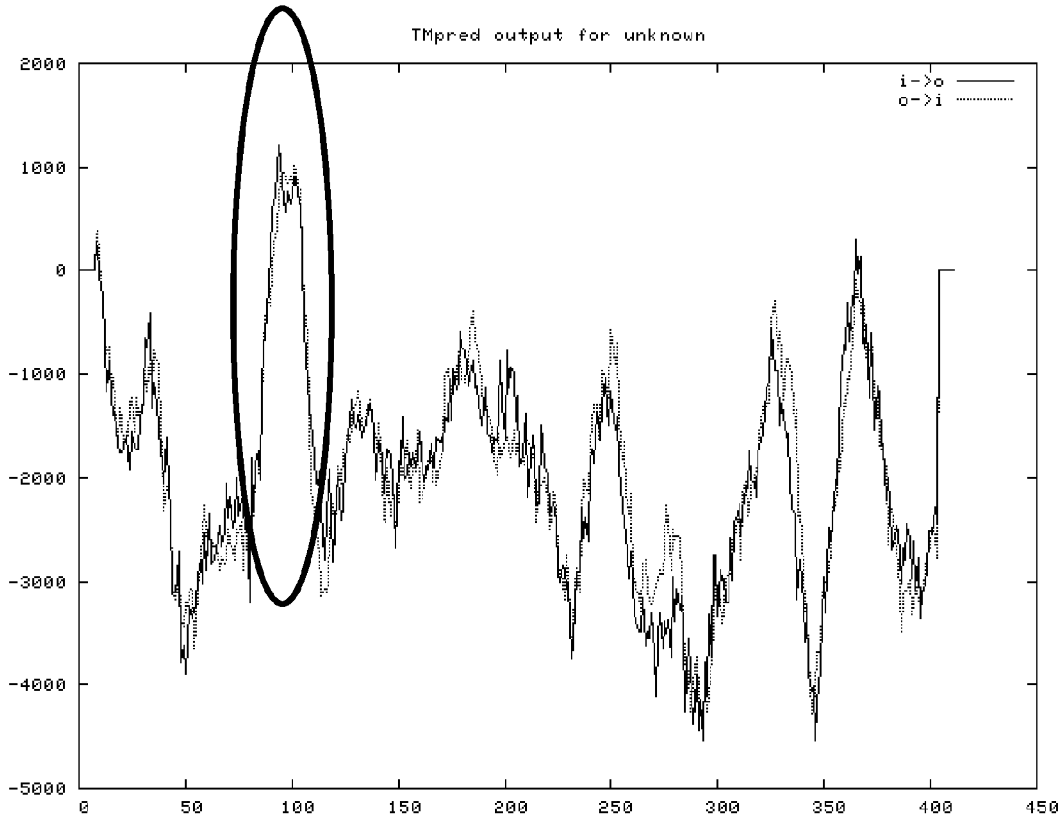


**FIGURE 11**

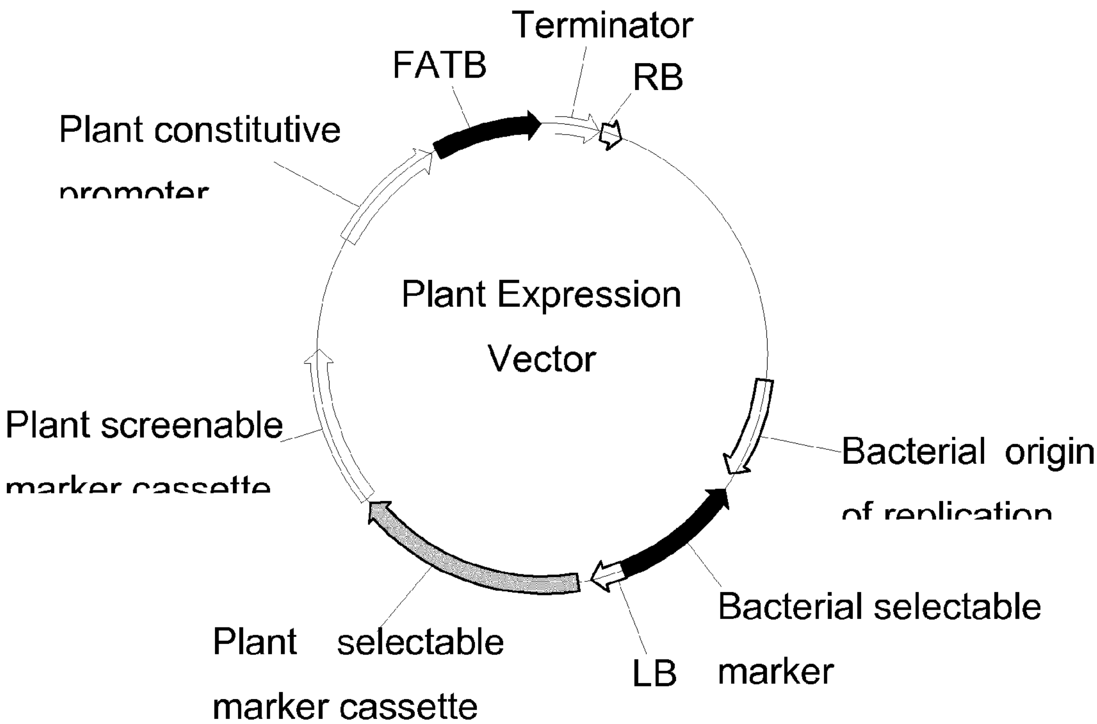


**FIGURE 12**

```
                              TargetP cTP
                   1                                                 50
Arath_FATB    (1) --MVATSATSSFFPVPSSSLDPN-G--------KGNKIG--S---TNLAG
Helan_FATB    (1) --MVAMSATASLFPVSSPKPHSGAK--------TSDKLGGEP-GSVAVRG
Soltu_FATB    (1) --MMATAATCAFFPAANPPPDSGAK--------SSGNLGGSLPGSIDTRG
Arahy_FATB    (1) ---MATAATASIFPVPSPS-PDAGA--------DGNKLVGGS---VKLQG
Glyma_FATB    (1) --MVATAATSSFFPVTSPS-PDSGG--------AGSKLGGGP---ANLGG
Maldo_FATB    (1) --MVATAATASFFPVSSPN-SDSSA--------KNAKLG------SANLG
Vitvi_FATB    (1) --MVATAATSAFFAVASPSSDPDAK--------PSTKPGVGS---AILRG
Garma_FATB    (1) --MVATAATSSFFPLTSPSGDAK-S--------GNPGKG--S---VSFGS
Jatcu_FATB    (1) --MVATAATSSFFPVPTSSADSK-S--------TKIGSG--S---ASLGG
Ricco_FATB    (1) MVATAAAATSSFFPVPSQSADAN-F--------DKAP--------ASLGG
Popto_FATB    (1) --MVATAATSSFFPVPSPPGDAK-S--------SKVGSG--S---ASLGG
Braju_FATB    (1) --MVATSATSLFFPLPSSSLDPNXK--------TNNRVT--S---TNFAG
Citsi_FATB    (1) --MVATAAASAFFPVSSPSGDSV-A--------KTKNLG--S---ANLGG
Goshi_FATB    (1) --MVATAVTSAFFPVTSSPDSSD-S--------KNKKLG--S---IKS--
Zeama_FATB    (1) --MAGSLAASAFFPGPGASPAASAK----------NLAGEVPDS-LSVRG
Brasy_FATB    (1) --MAGSLAASAFFPSPGSSPAALAKS-------SKNTSGELPET-LSVRG
Orysa_FATB    (1) --MAGSLAASAFFPVPGSSPAASARS-------SKNTTGELPEN-LSVRG
Aqufo_FATB    (1) --MVASAATAAFFPVTKASSTKASL--------VPGGGS----DNLDTRG
Irite_FATB    (1) --MVASVSASAFFPVPSSSSSSSSSSTGSTKPSSISLGKGPDA-LDARG
Tager_FATB    (1) --MVATAATASLFPVSSPQPDSGAK--------NSGNHKGGL-GSVDLRG
Elagu_FATB    (1) --MVASIVAWAFFPTPSFSPTASAK--------ASKTIGEGSEN-LNVRG
Picgl_FATB    (1) --MVAAAATMLMFSSSSQCNTQNKISSSASSGKPTMPVSSPERVDVKSKP
Zeama_FATBII  (1) --MAASIAASSFFPG---SPAPAAP---------KNGLGERPES-LDVRG
Phypa_FATB    (1) --------------------------------------------------
Arath_FATA    (1) --------------------------------------------------
Ostlu_FATA    (1) --MVSVAVARPRVAHASTHARERRQ-------------------R----
 Consensus    (1)   MVATAATSSFFPV S S  A              LG      L L G

                  TargetP cTP (cont'd)
                    51                                                100
Arath_FATB   (35) LNSAPNSG--RMKVKPNAQAPPKINGKKVGLPGSVDIVRTDTETSSHPAP
Helan_FATB   (40) IKTKSVNSG-GMKVKANAQAPTEVNGSRSR---ITHGFKTDDYSTS-PAP
Soltu_FATB   (41) LNVKKPSFG-SLQAKANAQAPPKVNGTKVG---VMDGFKNDDEVISSHHP
Arahy_FATB   (36) LKSKHASSGG-LQVKAHAQAPPKINGSTVES------LKHDDDLPS-PPP
Glyma_FATB   (37) LKSKSASSG---GLKAKAQAPSKINGTTVVTSK--ESFKHDDDLPS-PPP
Maldo_FATB   (34) LKSKSASGG--LQVKANAQAPSKINGTSVGLATVE-SGKHGDDISS-PPA
Vitvi_FATB   (38) IKSRNAPSGS-LQVKANAQAPPKINGTTVGYTSSAEGVKIEDDMSS-PPP
Garma_FATB   (35) MKSKSAASSRGLQVKANAQAPTKING-S------------TDDAQL-PAP
Jatcu_FATB   (35) IKSKPASSG-GLQVKANAQAPPKINGSTVGYTTPVDSVKNEGDTPS-PPP
Ricco_FATB   (34) IKLKSTSCSRGLQVKANAQAPPKINGSSVGFTTSVETVKNDGDMPLPPPP
Popto_FATB   (35) IKSKSASSG-ALQVKANAQAPPKINGSPVGLTASVETAKKEDVVSS-PAP
Braju_FATB   (36) LGPTPNSGG-RMKVKPNAQAPPKINGKKVGLPGSVEIETSQQQQ---PAP
Citsi_FATB   (35) IKSKSSSGS--LQVKANAQAPSKINGTSVGLTTPAESLKNGDISTSSPPP
Goshi_FATB   (33) -KPSVSSGS--LQVKANAQAPPKINGT-VASTTPVEGSKNDDGASS-PPP
Zeama_FATB   (38) IVAKPNANSGNMQVKAQAQTLPKVNGTKVNL-KNASSDTEEA--IPYTAP
Brasy_FATB   (41) IVAKPNTPPASMQVKTKAQALPKVNGTKVNL-KTSSSDKEDT--VPYSSS
Orysa_FATB   (41) IVAKPNPSPGAMQVKAQAQALPKVNGTKVNL-KTTSPDKEDI--IPYTAP
Aqufo_FATB   (37) INSSKPTSSGGLKVKANAQATPKINGTSIHYPPSSERLKNSDETS-IAPA
Irite_FATB   (48) LVAKPASNSGSLQVKVNAQAATRVNGSKVGL-KTDTNKLEDTPFFPSSAP
Tager_FATB   (40) IKSKSTSSN-GLQVKTNAQAPAKVNGTRVG---VMDGLKIDDSSSS-GAP
Elagu_FATB   (40) IIAKPTSSSAAKQGKVMAQAVPKINGAKVGL-KAESQKAEED-AAPSSAP
Picgl_FATB   (49) TAYKGLQVNGNSHGATNKINGTKVNGTAVDSMKHNVGLKEASEEES--TA
Zeama_FATBII (36) VAAKPGASSSAVRASKTRAHAAVPKMNGGGK-SAVADGEHET--VPSSVP
Phypa_FATB    (1) ----------------------------MSRVVPPILLEKDS--------
Arath_FATA    (1) --------------------------MLKLSCNVTDSKLQRSLLFFSHSYR
Ostlu_FATA   (25) --ASGARRSNAPRAFLASSTAVHANDASSCAMLKRASWRGKYALNVRASS
Consensus    (51) I SK  S     LQVKANAQAPPKINGT VG        K DD    S P P
```

**FIGURE 13**

```
                  TMpred predicted transmembrane helix
                   101                                               150
Arath_FATB    (83) RTFINQLPDWSMLLAAITTIFLAAEKQWMMLDWKPRRSDMLVD--PFGIG
Helan_FATB    (85) RTFINQLPDWSMLLAAITTIFLAAEKQWMMLEWKTKRPDMIADMDPFGLG
Soltu_FATB    (87) RTFINQLPDWSMLLAAITTIFLAAEKQWMMLDWKPKRPDMIAD--PFGLG
Arahy_FATB    (78) RTFINQLPDWSMLLAAITTIFLAAEKQWMMLDWKPRRSDMLID--PFGIG
Glyma_FATB    (81) RTFINQLPDWSMLLAAITTIFLAAEKQWMMLDWKPRRPDMLID--PFGIG
Maldo_FATB    (80) RTFINQLPDWSVLLAAITTIFLAAEKQWTMLDWKPKRPDMLID--PFGLG
Vitvi_FATB    (86) RTFINQLPDWSMLLAAITTIFLAAEKQWMMLDWKPRRSDMLID--PFGLG
Garma_FATB    (71) RTFINQLPDWSMLLAAITTVFLAAEKQWMMLDWKPRRPDMLID--TFGLG
Jatcu_FATB    (83) RTFINQLPDWSMLLAAITTIFLAAEKQWMMLDWKPRRPDMLID--PFGLG
Ricco_FATB    (84) RTFINQLPDWSMLLAAITTIFLAAEKQWMMLDWKPRRPDMLID--PFGIG
Popto_FATB    (83) RTFINQLPDWSMLLAAITTMFLAAEKQWMMLDWKPKRADMLID--PFGIG
Braju_FATB    (82) RTFINQLPDWSMLLAAITTVFLAAEKQWMMLDWKPRRSDMIME--PFGLG
Citsi_FATB    (83) RTFINQLPDWSMLLAAITTIFLAAEKQWMMLDWKPRRSDMLVD--PFGIG
Goshi_FATB    (78) RTFINQLPDWSMLLAAITTIFLAAEKQWMMLDWKPRRPDMVID--PFGIG
Zeama_FATB    (85) KTFYNQLPDWSMLLAAVTTIFLAAEKQWTLLDWKPKKPDMLVD--TFGFG
Brasy_FATB    (88) KTFYNQLPDWSMLLAAVTTIFLAAEKQWTMLDWKPKRPDMLVD--TFGFG
Orysa_FATB    (88) KTFYNQLPDWSMLLAAVTTIFLAAEKQWTLLDWKPKKPDMLAD--TFGFG
Aqufo_FATB    (86) RTFINQLPDWSVLLTAITAMFLAAEKQWTLLDWKPRRSDMLVD--PFGLG
Irite_FATB    (97) RTFYNQLPDWSVSFAAITTIFLAAEKQWTLIDWKPRRPDMLAD--AFGLG
Tager_FATB    (85) RTFINQLPDWSMLLAAITTIFLAAEKQWMMLDWKTKRPDMIADLDPFGFG
Elagu_FATB    (88) RTFYNQLPDWSVLLAAVTTIFLAAEKQWTLLDWKPRRPDMLTG--AFSLG
Picgl_FATB    (97) KSRINQLPDWSMLLATIATIILAAEKQWTNFDWKPRKTDVFGD--VFRLG
Zeama_FATBII  (83) KTFYNQLPDWSMLLAAITTIFLAAEKQWTMLDWKPRRPDMLTD--TFGFG
Phypa_FATB    (15) -------AAFRAILAAIAGVALAAENQRR-----HDKTEVPVD--VFRQG
Arath_FATA    (26) SDPVNFIRRRIVSCSQTKKTGLVPLRAVVSADQGSVVQGLATLADQLRLG
Ostlu_FATA    (73) TSSASEVADRNGADGGGEANGSATTGAGTSFTALDDSFRGLEG-TEWFSR
 Consensus   (101) RTFINQLPDWSMLLAAITTIFLAAEKQWMMLDWKPRRPDMLID  PFGLG

                   151                                               200
Arath_FATB   (131) RIVQDGLVFRQNFSIRSYEIGADRSASIETVMNHLQETALNHVKTAGLLG
Helan_FATB   (135) RIVQDGLVFRQNFSIRSYEIGADRTASIETLMNHLQETALNHVKSAGLLG
Soltu_FATB   (135) KIVQDGFVFRQNFSIRSYEIGADRTASIETMMNHLQETALNHVKSAGLMH
Arahy_FATB   (126) RIVQDGLVFRQNFSIRSYEIGADRTASIETVMNHLQETALNHVKTAGLLG
Glyma_FATB   (129) KIVQDGLVFRENFSIRSYEIGADRTASIETVMNHLQETALNHVKSAGLLG
Maldo_FATB   (128) RIVQDGLVFRQNFSIRSYEIGADRTASIETLMNHLQETALNHVKTAGLLG
Vitvi_FATB   (134) KIVQDGLVFRQNFSIRSYEIGADRTASIETLMNHLQETALNHVRTAGLLG
Garma_FATB   (119) RIVQDGLVFRQNFSIRSYEIGADRTASIETVMNHLQETALNHVKTAGLLG
Jatcu_FATB   (131) RIVQDGLVFRQNFSIRSYEIGADRTASIETLMNHLQETALNHVKTAGLLG
Ricco_FATB   (132) RIVQDGLIFRQNFSIRSYEIGADRTASIETLMNHLQETALNHVKTAGLLG
Popto_FATB   (131) RIVQDGLVFSQNFSIRSYEIGADRTASIETLMNHLQETALNHVKTAGLLG
Braju_FATB   (130) RIVQDGLVFRQNFSIRSYEIGADRSASIETVMNHLQETALNXVKTAGLLG
Citsi_FATB   (131) KIVQDGFIFRQNFSIRSYEIGADGTASIETLMNHLQETALNHVMTAGLLD
Goshi_FATB   (126) KIVQDGLVFSQNFSIRSYEIGADQTASIETLMNHLQETAINHCRSAGLLG
Zeama_FATB   (133) GIIQDGMVFRQNFIIRSYEIGADRTASIETLMNHLQETALNHVKTAGLLG
Brasy_FATB   (136) RIIQDGMVFRQNFLIRSYEIGADRTASIETLMNHLQETALNHVKTAGLLG
Orysa_FATB   (136) RIIQDGLVFRQNFLIRSYEIGADRTASIETLMNHLQETALNHVKTAGLLG
Aqufo_FATB   (134) KIVQDGLVFQQNFSIRSYEIGVDGTTSIESFMNHLQETALNHAKTVGLLG
Irite_FATB   (145) KIIENGLVYRQNFSIRSYEIGADQTASIETLMNHLQETALNHVKCAGLLG
Tager_FATB   (135) RIVEDGFVFRQNFSIRSYEIGADRTASVETLMNHLQETALNHVKNAGLLG
Elagu_FATB   (136) KIVQDGLVFRQNFSIRSYEIGADRTASIETLMNHLQETALNHVRNAGLLG
Picgl_FATB   (145) RFVEDSLVFRQNFAIRSYEIGADKTASIETLMNHLQETALNHVWLSGLAG
Zeama_FATBII (131) RIIHDGLMFRQNFSIRSYEIGADRTASIETLMNHLQETALNHVKTAGLLG
Phypa_FATB    (51) RLVESRLVYGQTFVIRSYEIGADRTASIETMMNHFQETALNHVWMSGLAG
Arath_FATA    (76) SLTEDGLSYKEKFVVRSYEVGSNKTATVETIANLLQEVGCNHAQSVGFST
Ostlu_FATA   (122) DFSESGRRFSEVFPVRFAEVGSNGEATMVTIADLIQECACNHAQ--GIWG
 Consensus   (151) RIVQDGLVFRQNFSIRSYEIGADRTASIETLMNHLQETALNHVKTAGLLG
 IPR002864              XXXXXXXXXXXXXXXXXXXXXXXXXXXXXXXXXXXXXXXXXXXXX
```

**FIGURE 13 (continued)**

```
                   201                                                50
Arath_FATB    (181) DGFGSTPEMFKKNLIWVVTRMQVVVDKYPTWGDVVEVDTWVSQSGKNGMR
Helan_FATB    (185) DGFGSTPEMCKKNLFWVVTKMQVIVDRYPTWGDVVQVDTWVAPNGKNGMR
Soltu_FATB    (185) GGFGSTPEMSKRNLIWVVTKMQVVVDRYPTWGDVVQVDTWVAASGKNGMR
Arahy_FATB    (176) DGFGSTPEMCKKNLIWVVTRMQVVVDRYPTWGDVVQVDTWVSASGKNGMR
Glyma_FATB    (179) DGFGSTPEMCKKNLIWVVTRMQVVVERYPTWGDIVQVDTWVSGSGKNGMR
Maldo_FATB    (178) DGFGSTPEMTVRNLIWVVTKMQVVVDRYPTWGDVVQVDTWVSASGKNGMR
Vitvi_FATB    (184) DGFGSTPEMSIRNLIWVVTRMQVVVDRYPTWGDVVQVDTWVCASGKNGMR
Garma_FATB    (169) DGFGSTPEMSKRNLIWVVTKMQVEVDRYPTWGDVVQVDTWVSASGKNGMR
Jatcu_FATB    (181) EGFGSTPEMSKRNLIWVVTRMQVLVDRYPTWGDVVEVDTWVSASGKNGMR
Ricco_FATB    (182) DGFGSTPEMSKRNLIWVVTRMQVLVDRYPTWGDVVQVDTWVSKSGKNGMR
Popto_FATB    (181) DGFGSTPEMSKRNLIWVVTRMQILVDRYPTWGDVVHVDTWVSASGKNGMR
Braju_FATB    (180) DGFGSTPEMVKKXLIWVVTRMQVVVDTYPTWGDVVEVDTWVSKSGKNGMR
Citsi_FATB    (181) AGFGATPAMAKKNLIWVVTRMQVVVDRYPTWNDVVNVETWVSASGKNGMR
Goshi_FATB    (176) EGFGATPEMCKKNLIWVVTRMQVVVDRYPTWGDVVQVDTWVSASGKNGMR
Zeama_FATB    (183) DGFGATPEMSKRNLIHEVSKIQLLVEKYPLWEDTVQVDTWVAAAGKNGMR
Brasy_FATB    (186) DGFGATQEMSKRNLIWVVSKIQLLVERYPSWEDMVQVDTWVASSGKNGMR
Orysa_FATB    (186) DGFGATPEMSKRNLIWVVSKIQLLVERYPSWGDMVQVDTWVAAAGKNGMR
Aqufo_FATB    (184) DGFGSTEAMSKRNLIWVVARMQILVNRYPTWGDTVQVDTWVAANGKNGMR
Irite_FATB    (195) NGFGSTPEMSKKNLIWVVTKMQVLVEHYPSWGNVIEVDTWAAVSGKNGMR
Tager_FATB    (185) DGFGSTPEMSKRNLFWVVTKMQVLVDRYPTWGDVVQVDTWVAASGKNGMR
Elagu_FATB    (186) DGFGATPEMSKRNLIWVVTKMQVLIEHYPSWGDVVEVDTWVGASGKNGMR
Picgl_FATB    (195) DGFGATLEMSRRNLLWVVARMQIQVERYPSWGDVVEIDTWVGPSGKNGMR
Zeama_FATBII(181) DGFGSTPEMSKRNLFWVVSQMQAIIERYPCWGDTVEVDTWVSANGKNGMR
Phypa_FATB    (101) DGFGATRAMSCNNLIWVVTRMQVHVEQYPAWGNIVEMDTWVAASGKNGMR
Arath_FATA    (126) DGFATTTTMRKLHLIWVTARMHIEIYKYPAWGDVVEIETWCQSEGRIGTR
Ostlu_FATA    (170) VGQSMPAEMARANLAWVCTRLHLRVRKYPKWGEKVAVSTWFEPQGKIAAR
 Consensus    (201) DGFGSTPEMSKRNLIWVVTRMQVLVDRYPTWGDVVQVDTWVSASGKNGMR
 IPR002864          XXXXXXXXXXXXXXXXXXXXXXXXXXXXXXXXXXXXXXXXXXXXXXXXXX

                    251                                              300
Arath_FATB    (231) RDWLVRDCNTGETLTRASSVWVMMNKLTRRLSKIPEEVRGEIEPYFV--N
Helan_FATB    (235) RDWLVRDYKTGEILTRASSNWVMMNKETRRLSKIPDEVRGEIEHYFV--D
Soltu_FATB    (235) RDWLLRDSNTGDIIMRASSQWVMMNKETRRLSKIPDEARAEIEGYFV--D
Arahy_FATB    (226) RDWLLRDCKTGEVLTRASSVWVMMNKLTRRLSKIPEEVRAEIASYFV--N
Glyma_FATB    (229) RDWLLRDCKTGEILTRASSVWVMMNKLTRRLSKIPEEVRQEIGSYFV--D
Maldo_FATB    (228) RDWIIQDLKTGQILTRASSVWVMMNKVTRRLSKMPDAVRGEIESFFM--N
Vitvi_FATB    (234) RDWIIRDCKTGETLTRASSVWVMMNKQTRRLSKIPDAVRAEIEPYFM--D
Garma_FATB    (219) RDWLLRDGNTGETLTRASSVWVMMNKLTRRLSKIPEEVREEIGSYFV--N
Jatcu_FATB    (231) RDWLVRDSKTGETLTRASSVWVMMNKLTRRLSKIPEEVRGEIEPYFL--N
Ricco_FATB    (232) RDWCVRDSRTGETLTRASSVWVMMNKLTRRLSKIPEEVRGEIEPYFL--N
Popto_FATB    (231) RDWLVRDAKTGETLTRASSLWVMMNKVTRRLSKIPEDVRGEIEPYFL--N
Braju_FATB    (230) RDWLVRDGNTGQILTRASSVWVMMNKLTRRLSKIPEEVRGEIEPYFV--D
Citsi_FATB    (231) RDWLIRNAKTGETLTRATSLWVMMNKLTRRLSKMPDEVRQEIEPYFL--N
Goshi_FATB    (226) RDWLVSNSETGEILTRATSVWVMMNKLTRRLSKIPEEVRGEIEPFFM--N
Zeama_FATB    (233) RDWHVLDCKSGCTILRATSVWVMMNKNTRRFSKMPDEVRAEIGPYFN--A
Brasy_FATB    (236) RDWHIRDYNSGQTILRATSVWVTMNKNTRKLSKMPDEVRAEIGEHFNN-D
Orysa_FATB    (236) RDWHVRDYNSGQTILRATSVWVMMNKNTRRLSKMPDEVRAEIGPYFN--G
Aqufo_FATB    (234) RDWLVRDGNSGETLARASSKWVMMNTSTRKLSKMPDDVRVEIEPYFM--D
Irite_FATB    (245) RDWHVRDCQTGQTIMRSSSNWVMMNKDTRRLSKFPEEVRAEIEPYFM--E
Tager_FATB    (235) RDWLIRDCKTGQILTRASSNWVMMNKVTRRLSKMPDEVRAEIEPYFV--D
Elagu_FATB    (236) RDWHVRDYRTGQTILRATSIWVMMDKHTRKLSKMPEEVRAEIGPYFM--E
Picgl_FATB    (245) RDWLVRDSKTNAILTRATSTWVMMNRKTRKLSKIPDAVKAEIQPYFT--E
Zeama_FATBII(231) RDWHIRDSMTGHTILKATSKWVMMNKLTRKLARIPDEVRTEIEPYFV--G
Phypa_FATB    (151) RDWLVRDYKSGQILARATSIWVMMNRKTRKLSKMPEEVRAEISPYFL--E
Arath_FATA    (176) RDWILKDSVTGEVTGRATSKWVMMNQDTRRLQKVSDDVRDEYLVFCPQEP
Ostlu_FATA    (220) RDYAITDAQTGECMGEATSQWVVFNLGSRRMARIPNSVLEDFKFQSL---
 Consensus    (251) RDWLVRD KTGETLTRASSVWVMMNKLTRRLSKIPDEVRAEIEPYFV
 IPR002864          XXXXXXXXXXXXXXXXXXXXXXXXXXXXXXXXXXXXXXXXXXXXXXXXXX
```

**FIGURE 13 (continued)**

```
                                                      Catalytic residues
                   301                                            350
Arath_FATB   (279) SDPVLAEDSR---KLTKIDDK-----TADYVRSGLTPRWS-DLDVNQHVN
Helan_FATB   (283) APPVVEDDSR---KLSKLDES-----TADYVRDGLIPRWS-DLDVNQHVN
Soltu_FATB   (283) SPPVIDEDSR---KLPKLDET-----TADYTRTGLTPRWS-DLDVNQHVN
Arahy_FATB   (274) SAPILEEDNR---KLSKLDDN-----TADYIRTGLSPRWN-DLDVNQHVN
Glyma_FATB   (277) SDPILEEDNR---KLTKLDDN-----TADYIRTGLSPRWS-DLDINQHVN
Maldo_FATB   (276) SPPVVEEDGR---KLPKLDDK-----TADVVLSGLTPRWS-DLDVNQHVN
Vitvi_FATB   (282) SAPIVDEDGR---KLPKLDDS-----TADYIRTGLTPRWS-DLDVNQHVN
Garma_FATB   (267) SDPVVEEDGR---KVTKLDDN-----TADFVRKGLTPKWN-DLDINQHVN
Jatcu_FATB   (279) SDPIVDEDGR---KLPKLDDN-----TADYVCKGLTPRWS-DLDVNQHVN
Ricco_FATB   (280) SDPIVDEDSR---KLPKLDDS-----NADYVRKGLTPRWS-DLDINQHVN
Popto_FATB   (279) SDPVVNEDST---KLPKLDDK-----TADYIRKGLTPRWN-DLDVNQHVN
Braju_FATB   (278) FDPVLAEDSR---KLTKLDDK-----TADYVRSGLTPRWS-DLDVNQHVN
Citsi_FATB   (279) SDPVVDEDSR---KLPKIGDS-----TADYVRRGLTPRWS-DLDVNQHVN
Goshi_FATB   (274) SDPVLAEDSQ---KIVKLDDS-----TAEHVCKGLTPKWS-DLDVNQHVN
Zeama_FATB   (281) RAAITDEQSE---KLAKPGSTAGGDAMKQFMRKGLTPRWGDLDVNQHVN
Brasy_FATB   (285) RSALTEEHSD---KLAKPGRKGGDPATKQFIRKGLTPKWG-DLDVNQHVN
Orysa_FATB   (284) RSAISEEQGE---KLPKPGTTFDGAATKQFTRKGLTPKWS-DLDVNQHVN
Aqufo_FATB   (282) CAPIVEEDGR---KLPKLDES-----TSDYVRNGLTPRWN-DLDINQHVN
Irite_FATB   (293) RVPVIDDDNR---KLPKLDD-----DTADHVRKGLTPRWS-DLDVNQHVN
Tager_FATB   (283) TPPVVDDDDR---KLPKLDEN-----TADHVRNGLTPKWS-DLDVNQHVN
Elagu_FATB   (284) HAAIVDEDSR---KLPKLDD-----DTADYIKWGLTPRWS-DLDVNQHVN
Picgl_FATB   (293) RNVFVAEDTR---KLHKLEDD-----TAQYICSDLTPRWS-DLDVNQHVN
Zeama_FATBII (279) RSAIVDEDNR---KLPKIPE-GQSTSAAKYVRTGLTPRWA-DLDINQHVN
Phypa_FATB   (199) RFAIKDEDEM---TQKICRLNG----SAEYVRSGLTPRRS-DLDMNQHVN
Arath_FATA   (226) RLAFPEENNRSLKKIPKIEDP------AQYSMIGLKPRRA-DLDMNQHVN
Ostlu_FATA   (267) -QQQVMEEGYAADKLPDVSEVG----GACAAPITHNVRRN-DMDMNGHVN
 Consensus   (301) S PIVDED R   KLPKLDD       TADYVR GLTPRWS DLDVNQHVN
 IPR002864         xxxxxxxxxxxxxxxxxxxxxxxxxxxxxxxxxxxxxxxxxxxxxxxxx

                                      Catalytic residue
                   351                                             400
Arath_FATB   (320) NVKYIGWILESAPVGIMERQKLKSMTLEYRRECGRDSVLQSLTAVTGCDI
Helan_FATB   (324) NVKYIGWILESAPQ-VVEKYELARITLEYRRECRKDSVVKSLTSVLGGGD
Soltu_FATB   (324) NVKYIGWILESAPMQILEGCELAAMTLEYRRECRRDSVLQSLTSVLDKGV
Arahy_FATB   (315) NVKYIGWILESAPQPILESHELSAMTLEYRRECGRDSVLQSLTAVSAADV
Glyma_FATB   (318) NVKYIGWILESAPQPILESHELSSMTLEYRRECGRDSVLDSLTAVSGADM
Maldo_FATB   (317) NVKYIGWILEGAPLPILESHELSSLTLEYRRECGRDSVLQSLTAVSGADI
Vitvi_FATB   (323) NVKYIGWILESAPLPILESHELSSMTLEYRRECGRDSVLQSLTAVCGTGV
Garma_FATB   (308) NVKYIGWILESAPQPILETRELSAVTLEYRRECGRDSVLRSLTAVSGGGV
Jatcu_FATB   (320) NVKYIGWILESAPLPILESHELSSIIMEYRRECGRDSVLQSLTAVSGTGL
Ricco_FATB   (321) NVKYIGWILESAPLPILESHELSAITLEYRRECGRDSVLQSLTAVSGNGI
Popto_FATB   (320) NVKYIGWILESAPPPILESHELAAITLEYRRECGRDSVLQSLTAVSGAGI
Braju_FATB   (319) NVKYIGWILESAPVGMMESQKLKSMTLEYRRECGRDSVLQSLTAVSGCDI
Citsi_FATB   (320) NVKYIGWILESAPQQILESHQLASVTLEYRRECGRDSVLQSLTAVSDKDI
Goshi_FATB   (315) NVKYIGWILESAPLPILESHELSALTLEYRRECGRDSVLQSLTTVSDSNT
Zeama_FATB   (328) NVKYIGWILESAPIAILEKHELASMTLDYRKECGRDSVLQSLTTVAGECV
Brasy_FATB   (331) NVKYIGWILESAPISILEKHELASMTLEYRKECGRDSVLQSLTNVIGECT
Orysa_FATB   (330) NVKYIGWILESAPISILEKHELASMTLDYRKECGRDSVLQSLTAVSGECD
Aqufo_FATB   (323) NVKYIGWILESSIS-MLENHELAGITLEYRKECRKDNVLQSLTAVSKDAK
Irite_FATB   (334) NVKYIGWILESAPISILESHELASMTLEYRRECGRDSMLQSLTSLSNDCT
Tager_FATB   (324) NVKYVGWILESAPQHVVENYELASLTLEYRRECMKDSVLQSLTSLLAGGE
Elagu_FATB   (325) NVKYIGWILESAPISILENHELASMTLEYRRECGRDSVLQSLTAVANDCT
Picgl_FATB   (334) NVKYIGWILESLPISVLEGNELANITLEYRRECGPTHVLQSLTSPQAGEV
Zeama_FATBII (324) NVKYIAWILESAPITIFENHELASIVLDYKRECVRDSVLQSHTSVHEDCN
Phypa_FATB   (241) NVKYIGWMLETVPPAVLDGYELVSMNLEYRRECGQSDVVQSMTTADGGN-
Arath_FATA   (269) NVTYIGWVLESIPQEIVDTHELQVITLDYRRECQQDDVVDSLTTTTSEIG
Ostlu_FATA   (311) NVVYVQWILESVPPETWEKHVLSEIILEYRSECNFGDSVTATCCEIEEAN
 Consensus   (351) NVKYIGWILESAPI ILESHELASMTLEYRRECGRDSVLQSLTAVSG   I
 IPR002864         xxxxxxxxxxxxxxxxxxxxxxxxxxxxxxxxxxxxxxxxxxxxxxxxxx
```

FIGURE 13 (continued)

```
                       401                                              450
Arath_FATB    (370) G---------------NLATAGDVECQHLLRLQDG------AEVVRGRTE
Helan_FATB    (373) DDNG------------GIGDSGRVDCQHVLLFAGGGDGTPGGEIVKGRTQ
Soltu_FATB    (374) G---------------DFTDFGNVECQHVLRLENG------GEVVKGRTE
Arahy_FATB    (365) G---------------NLAHRGQLECKHLLRLEDG------AEIVRGRTE
Glyma_FATB    (368) G---------------NLAHSGHVECKHLLRLENG------AEIVRGRTE
Maldo_FATB    (367) G---------------NLGSNGTVECQHMLRLEDG------AEIVRGRTE
Vitvi_FATB    (373) G---------------NLLDCGNVECQHLLRLEEG------AEIVKGRTE
Garma_FATB    (358) G---------------DLGHAGNVECQHVLRLEDG------AEIVRGRTE
Jatcu_FATB    (370) G---------------NLGNAGEIECQHLLRLEEG------AEIVRGRTA
Ricco_FATB    (371) G---------------NLGNAGDIECQHLLRLEDG------AEIVRGRTE
Popto_FATB    (370) G---------------NLGGPGKVECQHLLRHEDG------AEIVRGRTE
Braju_FATB    (369) G---------------NLGTAGEVECQHLLRLQDG------AEVVRGRTE
Citsi_FATB    (370) G---------------NLVNLGSVECQHLLRLEEG------AEVLRARTE
Goshi_FATB    (365) E---------------NAVNVGEFNCQHLLRLDDG------AEIVRGRTR
Zeama_FATB    (378) DG----------------DTDSTIQCDHLLQLETG------ADIVKAHTE
Brasy_FATB    (381) DG----------------SPESAIQCSHLLQLESG------TDIVKAHTK
Orysa_FATB    (380) DG----------------NTESSIQCDHLLQLESG------ADIVKAHTE
Aqufo_FATB    (372) G---------------------WPECVHLLRLDSG------AEVVRGSTM
Irite_FATB    (384) DG----------------LGELPIECQHLLRSRVG------LN-VKGRTE
Tager_FATB    (374) K-----------------ADSDDVDCQHLLRLEGG------GEIVKGRTK
Elagu_FATB    (375) GG----------------LPEASIECQHLLQLECG------AEIVRGRTQ
Picgl_FATB    (384) IAASAAPFSQRNDPPDTWKPLPALQFAHLLRLQDDR-----SEILRARSE
Zeama_FATBII  (374) IE----------------SGETTLHCEHVLSLESG------PTIVKARTM
Phypa_FATB    (290) -----------------------LQFVHLLRMESDG-----AEIVRGRTR
Arath_FATA    (319) GTNG--------SATSGTQGHNDSQFLHLLRLSGDG-----QEINRGTTL
Ostlu_FATA    (361) ---------------------DTYVLLHKLARGEG-------EIVRAKTV
 Consensus    (401) G                    G VECQHLLRLEDG      AEIVRGRTE
 IPR002864          XXXXXXXXXXXXXXXXXXXXXXXXXXXXXXXXXXXXXXXXXXXXXXXXXX

                       451                    473
Arath_FATB    (399) WSSKTPTTTWGTAP---------
Helan_FATB    (411) WRPKYEKQDGSVDHFSAGNV---
Soltu_FATB    (403) WRPKLVNGIGTLGGFDFA-----
Arahy_FATB    (394) WRPKPVSNFDIVNQVPAESI---
Glyma_FATB    (397) WRPKPVNNFGVVNQVPAEST---
Maldo_FATB    (396) WRPKYANNLGIVGHLPAESA---
Vitvi_FATB    (402) WRPKYAHSMGGVGQIPAESA---
Garma_FATB    (387) WRPKYINNFSIMG-QIPTDAS--
Jatcu_FATB    (399) WRPKYRSNFGIMG-QIPVESA--
Ricco_FATB    (400) WRPKYSSNFGIMG-QIPVESA--
Popto_FATB    (399) WRPKHANNFGMMGGQMPADESGA
Braju_FATB    (398) WSSKTPTTTWDITP---------
Citsi_FATB    (399) WRPKDAHNFGNVGPIPAEST---
Goshi_FATB    (394) WRPKHAKSSANMDQITAKRA---
Zeama_FATB    (406) WRPKRAHGEGTPMGGFPAESA--
Brasy_FATB    (409) WRPKRAQGEGN-TGLFPASSA--
Orysa_FATB    (408) WRPKRAQGEGN-MGFFPAESA--
Aqufo_FATB    (395) WRPKRINNFGSVGRIPTDGM---
Irite_FATB    (411) WRPK-KRAPFPVGSP--------
Tager_FATB    (401) WRPKYVKQIQEHQSFPY------
Elagu_FATB    (403) WRPRRASGPTSAGSA--------
Picgl_FATB    (429) WRSKAKNNLHDLA----------
Zeama_FATB II (402) WRPKGTKAQET---AVPSSF---
Phypa_FATB    (312) WRPKKLNHSQLS-----------
Arath_FATA    (356) WRKKPSS----------------
Ostlu_FATA    (383) WRK--------------------
 Consensus    (451) WRPK      G VG
 IPR002864          XXX
```

**FIGURE 13 (continued)**

**MDPEGFTSGLFRWNPTRALVQAPPPVPPPLQQQPVTPQT**

**AAFGMRLGGLEGLFGPYGIRFYTAAKIAELGFTASTLVG**

**MKDEELEEMMNSLSHIFRWELLVGERYGIKAAVRAERRR**

**LQEEEEEESSRRRHLLLSAAGDSGTHHALDALSQEDDWT**

**GLSEEPVQQQDQTDAAGNNGGGSGYWDAGQGKMKKQQQ**

**QRRRKKPMLTSVETDEDVNEGEDDDGMDNGNGGSGLGTE**

**RQREHPFIVTEPGEVARGKKNGLDYLFHLYEQCREFLLQ**

**VQTIAKDRGEKCPTKVTNQVFRYAKKSGASYINKPKMRH**

**YVHCYALHCLDEEASNALRRAFKERGENVGSWRQACYKP**

**LVNIACRHGWDIDAVFNAHPRLSIWYVPTKLRQLCHLER**

**NNAVA**AAAALVGGISCTGSSTSRGGCGGDDLRF

FIGURE 14

```
CLUSTAL 2.0.3 multiple sequence alignment

genpept7227884      MDP--EAFSASLFKWDP-RGAMPPPNRLLEAVAPPQPPP--PPLPPPQPLPP-AYSIR-T
genpept7658233      MDP--DAFSASLFKWDP-RGAMPPPSRLLEPVAPPQPPPSLPPPPPPQPLPTSSYSIRST
genpept7227893      MDP--EAFSASLFKWDP-RGAMPPPTRLLEAAVAPPPPP--PVLPPPQPLSA-AYSIR-T
genpept7227894      MDP--EAFSASLFKWDP-RGAMPPPTRLLEAAVAPPPPP--PALPPPQPLSA-AYSIK-T
genpept123096       MDP--DAF---LFKWDH-RTALPQPNRLLDAVAPPPPPP--PQAP--------SYSMR-P
genpept66864715     MDP--DGFSSSLFKWDPTRGIVQAPVRLLEAVAAAPTQA--------------AYGVR-P
Q1PDG5              MDP--EGFTSGLFRWNPTRALVQAP--------PPVPP---PLQQQPVTPQTAAFGMR--
Q1KLS1              MDP--EGFTSGLFRWNPTRALVQAP--------PPVPP---PLQQQPVTPQTAAFGMR--
Atleafy             MDP--EGFTSGLFRWNPTRALVQAP--------PPVPP---PLQQQPVTPQTAAFGMR--
Q8LSH1              MDP--EGFTSGLFRWNPTRAMVAAP--------PPVPP---QPQQQPATPQTRAFGMR--
Q3ZK20              ----------GLFRWNPMRAMVQAP--------PPVPPS--PQQQQPATPQTAAFGMR--
Q3LZW7              ---------SGLFRWNSTRALVQQP--------PPVPP---PQQQPPETPQTVAFGMR--
BOFH_BRAOB          MDP--EGFTSGLFRWNPTRVMVQAP--------TPIPP---PQQQSPATPQTAAFGMR--
Q6XPU8              MDP--EGFTSGLFRWNTTRAMVQHQ------PPPQVPPP--PSQQSPVTPQTAAFGMR--
Q3ZLR9              MDP--EGFTNGLFRWNTTRAMIQQQQ---QLPPPQITPP--P-QQSPATPQTAAFGMR--
Q6XPU7              MDP--EGFTSGLFRWNPTRATVQAL--------PPVPPP--L-QQQPATVQSAAFGTR--
Q3ZK15              ----------GLFRWNPTRATVQAL--------PPVPPP--P-QQQPATTQTAAFGMR--
Q3ZLS6              MDP--EGFTSGLFRWNPTRATVQAL--------APVPPP--L-QQQPATAQTAAFGMR--
Q6XPU5              MDP--DGFANGLFRWKPTRAMVQSP--------PPVPPP--PQQQQ--TAAAEAFGMR--
genpept27544560     -----MVLATSQQHHQHNPHEVQQH---------LQPHS------------TATESSR--
genpept86261940     MDPNDAFLAAHPFRWDLGPPAPAAVP-------PPPPPP---PPPPALPPA---NAPR--
genpept86261942     MDPNDAFLAAHPFRWDLGPPAPAAVP-------PPPPPP---PLPPALPPA---NAPR--
genpept11935156     MDPHDAFLAAHPFRWDLGPPAPAAVP-------PPPPLPM--PQTPALPPA---NSPR--
genpept2274790      MDPNDAFSAAHPFRWDLGPPAPAPVP-------PPPPPP------PPPPPA---NVPR--
genpept28974117     -DPNDAFSAAHPFRWDLGPPAPAAPA-------PPPPPP---PAPQLLPHAPLLSAPR--
genpept28974119     MDPNDAFSAAHPFRWDLGPPAHAAPA-------PAPPPP---PLAPLLLPP---HAPR--
                                 : .                    .                    :
```

FIGURE 15

```
genpept7227884      RELGGLEEMFQAYGIRYYTAAKITELGFTVNTLLDMKDDELDDMMNSLSQIFRWELLVGE
genpept7658233      RELGGLEELFQAYGIRYYTAAKIAELGFTVNTLLDMKDEELDDMMNSLSQIFRWDLLVGE
genpept7227893      RELGGLEELFQAYGIRYYTAAKIAELGFTVNTLLDMKDEELDDMMNSLSQIFRWELLVGE
genpept7227894      RELGGLEELFQAYGIRYYTAAKIAELGFTVNTLLDMKDEELDDMMNSLSQIFRWELLVGE
genpept123096       RELGGLEELFQAYGIRYYTAAKIAELGFTVNTLLDMRDEELDEMMNSLCQIFRWDLLVGE
genpept66864715     RELGGLEELFQDYGIRYFTAAKIAELGFTASTLVDMKDEELDEMMNSLSQIFRWELLVGE
Q1PDG5              --LGGLEGLFGPYGIRFYTAAKIAELGFTASTLVGMKDEELEEMMNSLSHIFRWELLVGE
Q1KLS1              --LGGLEGLFGPYGIRFYTAAKIAELGFTASTLVGMKDEELEEMMNSLSHIFRWELLVGE
Atleafy             --LGGLEGLFGPYGIRFYTAAKIAELGFTASTLVGMKDEELEEMMNSLSHIFRWELLVGE
Q8LSH1              --LGGLEGLFGAYGIRFYTAAKIAELGFTASTLVGMKDEELEEMMNSLSHIFRWELLVGE
Q3ZK20              --LGGLEGLFGAYGIRFYTAAKIAELGFTASTLVGMKDEELEEMMNSLSHIFRWELLVGE
Q3LZW7              --LGGLEGLFGAYGIRFYTAAKIAELGFTASTLVGMKDEELEDMMNSLSHIFRWELLVGE
BOFH_BRAOB          --LGGLEGLFGPYGVRFYTAAKIAELGFTASTLVGMKDEELEDMMNSLSHIFRWELLVGE
Q6XPU8              --LGGLEGLFGPYGIRFYTAAKIAELGFTASTLVGMKDEELEDMMNSLSHIFRWELLVGE
Q3ZLR9              --LGGLEGLFGPYGIRFYTAAKIAELGFTASTLVGMKDEELEDMMNSLSHIFRWELLVGE
Q6XPU7              --LGGLEGLFGVYGIRFYTAAKIAELGFTASTLVGMRDEELEEMMNSLSHIFRWELLVGE
Q3ZK15              --LGGLEGLFGAYGIRFYTAAKIAELGFTASTLVGMRDEELEEMMNSLSHIFRWELLVGE
Q3ZLS6              --LGGLEGLFGAYGIRFYTAAKIAELGFTASTLVGMRDEELEEMMNSLSHIFRWELLVGE
Q6XPU5              --VGGLEGLFRAYGIRFYTSAKIAELGFTASTLLNMKDEELDEMMNSLSHIFRWELLVGE
genpept27544560     ----ELEEVFEGYGVRYSTIARIGDLGFTASTLAGMREEEVDDMMAALSHLFRWDLLVGE
genpept86261940     ----ELEDLVVGYGVRASTVARISELGFTASTLLVMTERELDDMTAALAGLFRWDLLIGE
genpept86261942     ----ELEDLVVGYGVRASTVARISELGFTASTLLVMTESELDDMTAALAGLFRWDLLIGE
genpept11935156     ----ELEDLVAGYGVRGATVARISELGFTASTLLVMTDRELDDMTAALAGLFRWDLLIGE
genpept2274790      ----ELEELVAGYGVRMSTVARISELGFTASTLLAMTERELDDMMAALAGLFRWDLLLGE
genpept28974117     ----ELEDLVAGYGVRPSTVARISELGFTASTLLGMTERELDDMMAALAGLFRWDVLLGE
genpept28974119     ----ELEDLVAGYGVRPSTVARISELGFTASTLLGMTERELDDMMAALAGLFRWDVLLGE
                         ** :.  **:*  * *:*  :****..**  * : *:::*   :*. :***::*:**
```

FIGURE 15 (continued)

```
genpept7227884      RYGIKAAIRAERRRLEE---------------------------------EEGRRR-HIL
genpept7658233      RYGIKAAIRAEWRRLEE---------------------------------EEARRRGHIL
genpept7227893      RYGIKAAIRAERRRLEE---------------------------------EELRRRSHLL
genpept7227894      RYGIKAAIRAERRRLEE---------------------------------EELRRRGHLL
genpept123096       RYGIKAAVRAERRRIDE---------------------------------EEVRRR-HLL
genpept66864715     RYGIKAAVRAERRRLDD---------------------------------DDSRRR-QTL
Q1PDG5              RYGIKAAVRAERRRLQEEEE------------------------------EESSRRRHLL
Q1KLS1              RYGIKAAVRAERRRLQEEEE------------------------------EESSRRRHLL
Atleafy             RYGIKAAVRAERRRLQEEEE------------------------------EESSRRRHLL
Q8LSH1              RYGIKAAVTAERRRLQEEEE------------------------------EESSRRRHLL
Q3ZK20              RYGIKAAVRAERRRLQEEEE------------------------------E-SSRRRHLL
Q3LZW7              RYGIKAAVRAERRRLQEVEE------------------------------EESSRRRHLL
BOFH_BRAOB          RYGIKAAVRAERRRLQEEEE------------------------------EESSRRRHLL
Q6XPU8              RYGIKAAVRAERRRLQEEEE------------------------------DDSSRRRHLL
Q3ZLR9              RYGIKAAVRTERRRLQEEEE------------------------------EESSRRRHFM
Q6XPU7              RYGIKAAVRAERRRLQEEEE------------------------------EESSRRRHLL
Q3ZK15              RYGIKAAVRAERRRLQEEEE------------------------------EESSRRRHLL
Q3ZLS6              RYGIKAAVRAERRRLQEEEE------------------------------EESSRRRHLL
Q6XPU5              RYGIKAAVRAERRRVQEEEE------------------------------EESSRRRHLL
genpept27544560     RYGIKAAIRAERRRLEALIFSHVSGAARLSHHQHQMGYLFSSATTGYHLMPDDPRKRHLL
genpept86261940     RFGLRAALRAERGRLMSP----------------------------------GCRHHGYQ
genpept86261942     RFGLRAALRAERGRLMSP----------------------------------GCRHHGYQ
genpept11935156     RFGLRAALRAERGRLMALH---------------------------------GGRHHGHQ
genpept2274790      RFGLRAALRAERGRLMSL----------------------------------GGRHHGHQ
genpept28974117     RFGLRAALRAERGRVMSL----------------------------------GGR---FH
genpept28974119     RFGLRAALRAERGRVMSL----------------------------------GARC--FH
                    *:*::**: :*  *:                                       *
```

FIGURE 15 (continued)

```
genpept7227884      SDG-----GTNVLDALSQE----GLSEE----PVQQQ---EREAAGSGGGGTA------W
genpept7658233      SDG-----GTNVLDALSQE----GLSEE----PVQQQH--EREAAGSGGGGT-------W
genpept7227893      SDG-----GTNALDALSQE----GLSEE----PVQQQ---EREAVGSGGGGTT------W
genpept7227894      SDG-----GTNALDALSQE----GLSEE----PVQQQ---EREAVGSGGGGTT------W
genpept123096       LGD-----TTHALDALSQE----GLSEE----PVQQE----KEAMGSGGGGVGG----VW
genpept66864715     STD-----TTHALDALSQE----GLSEE----PVQQE----KEAAGSGGGTI-------W
Q1PDG5              LSAAGDSGTHHALDALSQE----GLSEE----PVQQQ--DQTDAAGNNGGGGSG----YW
Q1KLS1              LSAAGDSGTHHALDALSQE----ELSEE----PVQQQ--DQTDAAGNNGGGGSG----YW
Atleafy             LSAAGDSGTHHALDALSQEDDWTGLSEE----PVQQQ--DQTDAAGNNGGGGSG----YW
Q8LSH1              LSAAGDSGTHHALDALSQEDDWTGLSEELDREPVQQQ--NQTDAAGNNGGGGSG----YW
Q3ZK20              LSAAGDSGTHHALDALSQEDDWTGLSEE----PVQQQ--DQTDAAGNNGGGGSG----YW
Q3LZW7              LCAAGDSGTHHALDTLSQEDYWTGLSEE----PGQQQ--DQTDAAGNNGGNGGGEGGGYW
BOFH_BRAOB          LSAAGDSGTHLALDALSQEDDWTGLSQE----PVQHQ--DQTDAAGINGGGRGG----YW
Q6XPU8              LSAAGDSGTHHALDALSQEDDWTGLSEE----PVHQ---DQTDAAGNGG--FGG----YL
Q3ZLR9              LSAGGDSGTHHALDALSQEDDWTGLSEE----PVHQ---DQTDAAGNGG--FGG----YL
Q6XPU7              LSAAGDSGTHHALDALSQEDDWTGLSEE----PVQQI-DHLTDAVGNN----GG----YW
Q3ZK15              LSAAGDSGTHHALDALSQEDDWTGLSEE----PVQQQ-DHQTDAAGNN----GG----NW
Q3ZLS6              LSAAGDSGTHHALDALSQEDDWTGLSEE----PVQQQ-DHQTDAVGNN----GG----YW
Q6XPU5              LSAAGDSVAHHAL---SQEDDWTSLSEE----PVQQK--DQTDAAGSNG---GG---VYW
genpept27544560     LSP----DHHSALDALSQE----GLSEE----PVQLE----REAAGSGGEVVGR-----R
genpept86261940     SGS--------TIDGASQE----VLSNERDG-AASGG-IGEEDAMRMMA---SG----KK
genpept86261942     SGS--------TIDGASQE----VLSNERDG-AASGG-IGEDDAMRMMA---SG----KK
genpept11935156     SGS--------TIDGASQE----VLSNERDG-AASG----EDDAGRMML---SG----KK
genpept2274790      SGS--------TVDGASQE----VLSDEHDM-AGSGG-MGDDDNGRRMV---TG----KK
genpept28974117     TGS--------TLDAASQE----VLSDERDA-AASGG-LAEGEAGRRMVTTGK-----KK
genpept28974119     AGS--------TLDAASQE----ALSDERDA-AASGGGMAEGEAGRRMVTTTAG----KK
                                .:    ***       **:*      .           :
```

FIGURE 15 (continued)

```
genpept7227884      EVVAP-GGGRMRQRRRKKVV-VGRERRGSS-MEEDEDTEEGQ--------EDNEDYNINN
genpept7658233      EVAAG-GGGRMKQRRRKK---AGRERRG----EEDEETEELG--------EEDEENMNQG
genpept7227893      EVVAAVGGGRMKQRRRKKVVSTGRERRGRASAEEDEETEEGQ--------EDE---WNIN
genpept7227894      EVVAAAGGGRMKQRRRKKVVAAGREKRGGASAEEDEETEEGQ--------EDD---WNIN
genpept123096       EMMGA-GGRKAPQRRRKNYK--GRSRMAS--MEEDDDDDDDE--------TEG-----AE
genpept66864715     EVGPG----KKKQRRRKVVG------------EEEQEEEN--------------------
Q1PDG5              DAG-QGKMKKQQQQRRRKKP-------MLTSVETDEDVNEGE--------DDDGMDNGNG
Q1KLS1              DAG-QGKMKKQQQQRRRKKP-------MLTSVETDEDVNEGE--------DDDGMDNGNG
Atleafy             DAG-QGKMKKQQQQRRRKKP-------MLTSVETDEDVNEGE--------DDDGMDNGNG
Q8LSH1              EAG-QAKMKKQQQQRRRKKP-------MVTSVETDDDVNEGD--------DDDGMDNGNG
Q3ZK20              EAG-QAKMKKPQQ-RRRKKP-------MVASVETDDDGNEGE--------DDDGMDNGNG
Q3LZW7              EAG-QAKMKKPQQ-RRRKKS-------MVTSVEIDDECNEGE--------DDDGMDNCNG
BOFH_BRAOB          EAG-QTTIKKQQQRRRKKRL-------YVS--ETDDDGNEGE--------DDDGMDIVN-
Q6XPU8              ESSVHGKMKKHQPRRRKKPL-------VLTSVETDDDGNDNE--------DDDGMDNGNG
Q3ZLR9              ESG-HGKMKKQQQQKRRKKP-------LVTSVETDDDGND----------DDDGMDNGNG
Q6XPU7              EAN-KGKMKKQQQRRRKKP--------MLTSVETDDDINEGE--------DEDGMDNSNG
Q3ZK15              EAG-KGKMKKQQQRRRKKP--------MMTSVETDDDINEGE--------DEDGMDNGNG
Q3ZLS6              DEG-KGKMKKQQQRRRMKP--------LMTSVEPDNDMDECE--------DEDRMDNGNG
Q6XPU5              GAG-QAKMK--QKRRKKPTV-------MMTSVETDDEINECE--------DDDRMDNGNG
genpept27544560     DGKGKNQQRQTSAKKKDASSTKSKKKKKKGIEEGDDEEEEVEVWGRGASIENDEDDDGDE
genpept86261940     -QKNGSAGRKAKKARRKKVNDLR----LDMQGDEH---EEGGGG------RSESTESSAG
genpept86261942     -QKNGSAARKAKKARRNKVKELR----LDMQGDEH---EDGGGG------RSESTESSAG
genpept11935156     -LKNGSVARKAKKARRKKVDGLR----LDHMQEDE---REDGGG------RSESTESSAG
genpept2274790      QAKKGSAARKGKKARRKKVDDLR----LDMQEDEMDCCDEDGGG------GSESTESSAG
genpept28974117     GKKG-VGARKGKKARRK--KELRP---LDVLDDENDGDEDGGGG------GSDSTESSAG
genpept28974119     GKKGVVGTRKGKKARRK--KELRP---LNVLDDENDGDEYGG--------GSESTESSAG
                                  ::                :
```

FIGURE 15 (continued)

```
genpept7227884      EGG--GGISERQREHPFIVTEPGEVARGKKNGLDYLFHLYEQCRDFLIQ-VQNIAKERGE
genpept7658233      GGG--GGISERQREHPFIVTEPGEVARGKKNGLDYLFHLYEQCRDFLIQ-VQTIAKERGE
genpept7227893      DAG--GGISERQREHPFIVTEPGEVARGKKNGLDYLFHLYEQCRDFLIQ-VQNIAKERGE
genpept7227894      DAS--GGISERQREHPFIVTEPGEVARGKKNGLDYLFHLYEQCRDFLIQ-VQNIAKERGE
genpept123096       DDE--NIVSERQREHPFIVTEPGEVARGKKNGLDYLFHLYEQCRDFLIQ-VQTIAKERGE
genpept66864715     -----GGGSERQREHPFIVTEPGEVARGKKNGLDYLFHLYEQCRDFLIQ-VQNIAKERGE
Q1PDG5              GSG---LGTERQREHPFIVTEPGEVARGKKNGLDYLFHLYEQCREFLLQ-VQTIAKDRGE
Q1KLS1              GSG---LGTERQREHPFIVTEPGEVARGKKNGLDYLFHLYEQCREFLLQ-VQTIAKDRGE
Atleafy             GSG---LGTERQREHPFIVTEPGEVARGKKNGLDYLFHLYEQCREFLLQ-VQTIAKDRGE
Q8LSH1              GGGGG-LGTERQREHPFIVTEPGEVARGKKNGLDYLFHLYEQCREFLLQ-VQTIAKDRGE
Q3ZK20              GSGG--MGTERQREHPFIVTEPGEVARGKKNGLDYLFHLYEQCREFLLQVIQTIAKDRGE
Q3LZW7              GGGG--LGIERQREHPFIVTEPGEVARGKKNGLDYLFHLYEQCREFLLQ-VQTIAKDRGE
BOFH_BRAOB          GSG---VGMERQREHPFIVTEPGEVARGKKNGLDYLFHLYEQCREFLLQ-VQTIAKDRGE
Q6XPU8              G---IGLGTERQREHPFIVTEPGEVARGKKNGLDYLFHLYEQCREFLLQ-VQTIAKDRGE
Q3ZLR9              GS--SGLGTERQREHPFIVTEPGEVARGKKNGLDYLFHLYEQCREFLLQ-VQTIAKDRGE
Q6XPU7              G-----LGTERQREHPFIVTEPGEVARGKKNGLDYLFHLYEQCREFLLQ-VQTIAKDRGE
Q3ZK15              GGGGGGLGTERQREHPFIVTEPGEVARGKKNGLDYLFHLYEQCREFLLQ-VQTIAKDRGE
Q3ZLS6              GGGG--LGMERQREHPFIVTEPGEVARGKKNGLDYLFHLYEQCREFLLQ-VQLIAKDRGE
Q6XPU5              G-----MAIERQREHPFIVTEPGEVARGKKNGLDYLFHLYEQCREFLLQ-VQTIAKDRGE
genpept27544560     SQSEQSSAAERQREHPFIVTEPGEVARAKKNGLDYLFNLYEQCHEFLNQ-VQSVAKERGD
genpept86261940     GG----VGGERQREHPFVVTEPGEVARAKKNGLDYLFHLYEQCRLFLLQ-VQSMAKLHGQ
genpept86261942     G-----VGGERQREHPFVVTEPGEVARAKKNGLDYLFHLYEQRRLFLLQ-VQSMAKLHGQ
genpept11935156     GGG--GVGGERQREHPFVVTEPGEVARAKKNGLDYLFHLYEQCRLFLLQ-VQSMAKLHGH
genpept2274790      GG-----GGERQREHPFVVTEPGEVARAKKNGLDYLFHLYEQCRLFLLQ-VQSMAKLHGH
genpept28974117     GS----GGGERQREHPFVVTEPGEVARAKKNGLDYLFHLYEQCRVFLLQ-VQSLAKLGGH
genpept28974119     GS----G--ERQREHPFVVTEPGEVARAKKNGLDYLFHLYEQCRVFLLQ-VQSIAKLGGH
                             ********:*********.*********:**** : ** * :* :**  *.
```

**FIGURE 15 (continued)**

```
genpept7227884     KCPTKVTNQVFRFAKK-AGASYINKPKMRHYVHCYALHCLDEDASNALRRAFKERGENVG
genpept7658233     KCPTKVTNQVFRYAKK-AGASYINKPKMRHYVHCYALHCLDEDASNALRRAFKERGENVG
genpept7227893     KCPTKVTNQVFRYAKK-AGASYINKPKMRHYVHCYALHCLDEEASNALRRAFKERGENVG
genpept7227894     KCPTKVTNQVFRYAKK-AGASYINKPKMRHYVHCYALHCLDEEASNALRRAFKERGENVG
genpept123096      KCPTKVTNQVFRYAKK-AGANYINKPKMRHYVHCYALHCLDEAASNALRRAFKERGENVG
genpept66864715    KCPTKVTNQVFRYAKK-AGASYINKPKMRHYVHCYALHCLDEKESNALRTAFKERGENVG
Q1PDG5             KCPTKVTNQVFRYAKK-SGASYINKPKMRHYVHCYALHCLDEEASNALRRAFKERGENVG
Q1KLS1             KCPTKVTNQVFRYAKK-SGASYINKPKMRHYVHCYALHCLDEEASNALRRAFKERGENVG
Atleafy            KCPTKVTNQVFRYAKK-SGASYINKPKMRHYVHCYALHCLDEEASNALRRAFKERGENVG
Q8LSH1             KCPTKVTNQVFRYAKK-SGASYINKPKMRHYVHCYALHCLDEDASNALRRAFKERGENVG
Q3ZK20             KCPTKVTYQVFRYAKK-SGASYINKPKMRHYVHCYALHCLDEDASNALRRSFKERGENVG
Q3LZW7             KCPTKGTNQVFRYAKN-SGASYINKPKMRHYVHCYALHCLDEEASNALRRAFKERGENVG
BOFH_BRAOB         KCPTKVTNQVFRYAKK-SGANYINKPKMRHYVHCYALHCLDEEASNALRSAFKVRGENVG
Q6XPU8             KCPTKVTNQVFRYAKK-SGASYINKPKMRHYVHCYALHCLDEEASNALRRAFKERGENVG
Q3ZLR9             KCPTKVTNQVFRYAKK-SGASYINKPKMRHYVHCYALHCLDEDASNALRRAFKERGENVG
Q6XPU7             KCPTKVTNQVFRYAKK-SGASYINKPKMRHYVHCYALHCLDEEASNALRRAFKERGENVG
Q3ZK15             KC---VTNQVFRYAKK-SGASYINKPKMRRCVRCCALHCLDEDASSALRRAFKERGGNVG
Q3ZLS6             KCPTKVTNQVFRYAKK-SGASYINKPKMRHYVHCYALHCLDEDASNALRRAFKERGENVG
Q6XPU5             KCPTKVTNQVFRYAKK-SGASYINKPKMRHYVHCYALHCLDENASNALRRSFKERGENVG
genpept27544560    KCPTKVTNLVFRYAKKKVGASYINKPKMRHYVHCYALHVLDEDASNSLRRAFKERGENVG
genpept86261940    KSPTKVTNQVFRYASK-VGASYINKPKMRHYVHCYALHCLDEDASDALRRAYKARGENVG
genpept86261942    KSPTKVTNQVFRYASK-VGASYINKPKMRHYVHCYALHCLDEDASDALRRAYKARGENVG
genpept11935156    KSPTKVTNQVFRYASK-VGASYINKPKMRHYVHCYALHCLDQEASDALRRAYKARGENVG
genpept2274790     KSPTKVTNQVFRYAKK-VGASYINKPKMRHYVHCYALHCLDEEASDALRRAYKARGENVG
genpept28974117    KSPTKVTNQVFRYAKK-CGASYINKPKMRHYVHCYALHCLDEDASNALRRAYKARGENVG
genpept28974119    KSPTKVTNQVFRYANK-CGASYINKPKMRHYVHCYALHCLDEEASNALRRAYKSRGENVG
                   *.    *  ***:*.:  **.********: *:* *** **:  *.:** ::* ** ***
```

FIGURE 15 (continued)

```
genpept7227884      AWRQACYKPLVAIAARQGWDIDAIFNGHPRLSIWYVPTKLRQLCHSERSN-AAAAASTSV
genpept7658233      AWRQACYKPLVAIAARQGWDIDAIFNAHPRLAIWYVPTKLRQLCHSERSN-AAAAASSSV
genpept7227893      AWRQACYKPLVAIAARQGWDIDTIFNAHPRLAIWYVPTRLRQLCHSERSN-AAAAASSSV
genpept7227894      AWRQACYKPLVAIAARQGWDIDTIFNAHPRLAIWYVPTKLRQLCHSERSNAAAAAASSSV
genpept123096       AWRQACYKPLVAIAARQGWDIDTIFNAHPRLSIWYVPTKLRQLCHAERSS-AAVAATSSI
genpept66864715     SWRQACYKPLVAIAARQGWDIDAIFNAHPRLAIWYVPNKLRQLCHAERNN-TAIASTSAA
Q1PDG5              SWRQACYKPLVNIACRHGWDIDAVFNAHPRLSIWYVPTKLRQLCHLERNN--AVAAAAAL
Q1KLS1              SWRQACYKPLVNIACRHGWDIDAVFNAHPRLSIWYVPTKLRQLCHLERNN--AVAAAAAL
Atleafy             SWRQACYKPLVNIACRHGWDIDAVFNAHPRLSIWYVPTKLRQLCHLERNN--AVAAAAAL
Q8LSH1              SWRQACYKPLVNIACRHGWDIDAVFNAHPRLSIWYVPTKLRQLCHLERNN--AVAAAAAL
Q3ZK20              SWRQACYKPLVNIACRHGWDIDAVFNAHPRLSIWYVPTKLRQLCHLERNN--AVAAATAL
Q3LZW7              SWRQACYKPLVNIACRHGWDIDAVFNAHPHLSIWYVPTKLRQLCHLERNN--AVAAAAAL
BOFH_BRAOB          SWRQACYKPLVDIACRHGWDIDAVFNAHPRLSIWYVPTKLRQLCHLERNN--AEAAAATL
Q6XPU8              SWRQACYKPLVNIACRHGWDIDAVFNAHPRLSIWYVPTKLRQLCHLERNN--AVAAAAAL
Q3ZLR9              SWRQACYKPLVNIACRHGWDIDAVFNAHPRLSIWYVPTKLRQLCHLERNN--AVAAASAL
Q6XPU7              SWRQACYKPLVNIACRHGWDIDAVFNSHPRLSIWYVPTKLRQLCHMERNN--EVAAATVL
Q3ZK15              SWRQACCKPLVNIACRHGWDIDAVFNAHPRLSIWYVPTKLRQLCHLERNN--AVAAATVL
Q3ZLS6              SWRQARYKPLVDIACRHGWDIDAVFNAHPRLSIWYVPTKLRQLCHLERNN--AVAAAAVL
Q6XPU5              SWRQACYKPLVDVAFRHGGDIDAVFNAHPRLSIWYVPTKLRQLCHLERNN--AGSATAA-
genpept27544560     AWRLACYKPLVAISASHSFDIDAVFNAHPRLSIWYVPTKLRQLCHLARSSTSQFPLAVPR
genpept86261940     AWRQACYAPLVDIAARHGFDIDAVFAAHPRLAIWYVPTRLRQLCHQARSAHDT-AAAHAG
genpept86261942     AWRQACYAPLVDIAARHGFDIDAVFAAHPRLAIWYVPTRLRQLCHQARSAHDAAAAAHAG
genpept11935156     AWRQACYAPLVDIAAGHGFDVDAVFAAHPRLAIWYVPTRLRQLCHQARSAHEAAAANANA
genpept2274790      AWRQACYAPLVDISARHGFDIDAVFAAHPRLAIWYVPTRLRQLCHQARSSHAAAAA----
genpept28974117     AWRQACYAPLVEIAARHGFDIDAVFAAHPRLTIWYVPTRLRQLCHQARGSHAHAAAG---
genpept28974119     AWRQACYAPLVEIAARHGFDIDAVFAAHPRLAVWYVPTRLRQLCHQARGSHAHAAAG---
                    :** *   *** ::  :. *:*::* .**:*::****.:******  *.     .
```

FIGURE 15 (continued)

```
genpept7227884       SGGGV--DHLPHF-----------------
genpept7658233       SGGVA--DHLPHF-----------------
genpept7227893       SGGVG--DHLPHF-----------------
genpept7227894       SGGGGGGDHLPHF-----------------
genpept123096        TGGGP-ADHLPF------------------
genpept66864715      A------HHLPF------------------
Q1PDG5               VGGISCTGSSTSGRGGCGGDDLRF------
Q1KLS1               VGGISCTGSSTSGRGGCGGDDLRF------
Atleafy              VGGISCTGSSTSGRGGCGGDDLRF------
Q8LSH1               VGGISCTGSSTSGRGGCGGDDLRF------
Q3ZK20               VGGISC------------------------
Q3LZW7               VGGISC------------------------
BOFH_BRAOB           VGGISCRDRLRLDALGFN------------
Q6XPU8               VGGISCTGSSASGRGGCGGDEELRY-----
Q3ZLR9               VGGISC------------------------
Q6XPU7               VGGISCTGTSASGHGECGGELHY-------
Q3ZK15               VGGIS-------------------------
Q3ZLS6               VGGISC------------------------
Q6XPU5               ------------------------------
genpept27544560      TTGSSNQRVSSTVHVVEDSAAAHSFRPPMF
genpept86261940      --AMPPP----MF-----------------
genpept86261942      --SMPPP----MF-----------------
genpept11935156      NGAMPPPPPPPMF-----------------
genpept2274790       --ALPPP----LF-----------------
genpept28974117      ---LPPP---PMF-----------------
genpept28974119      ---LPPP---PMF-----------------
```

FIGURE 15 (continued)

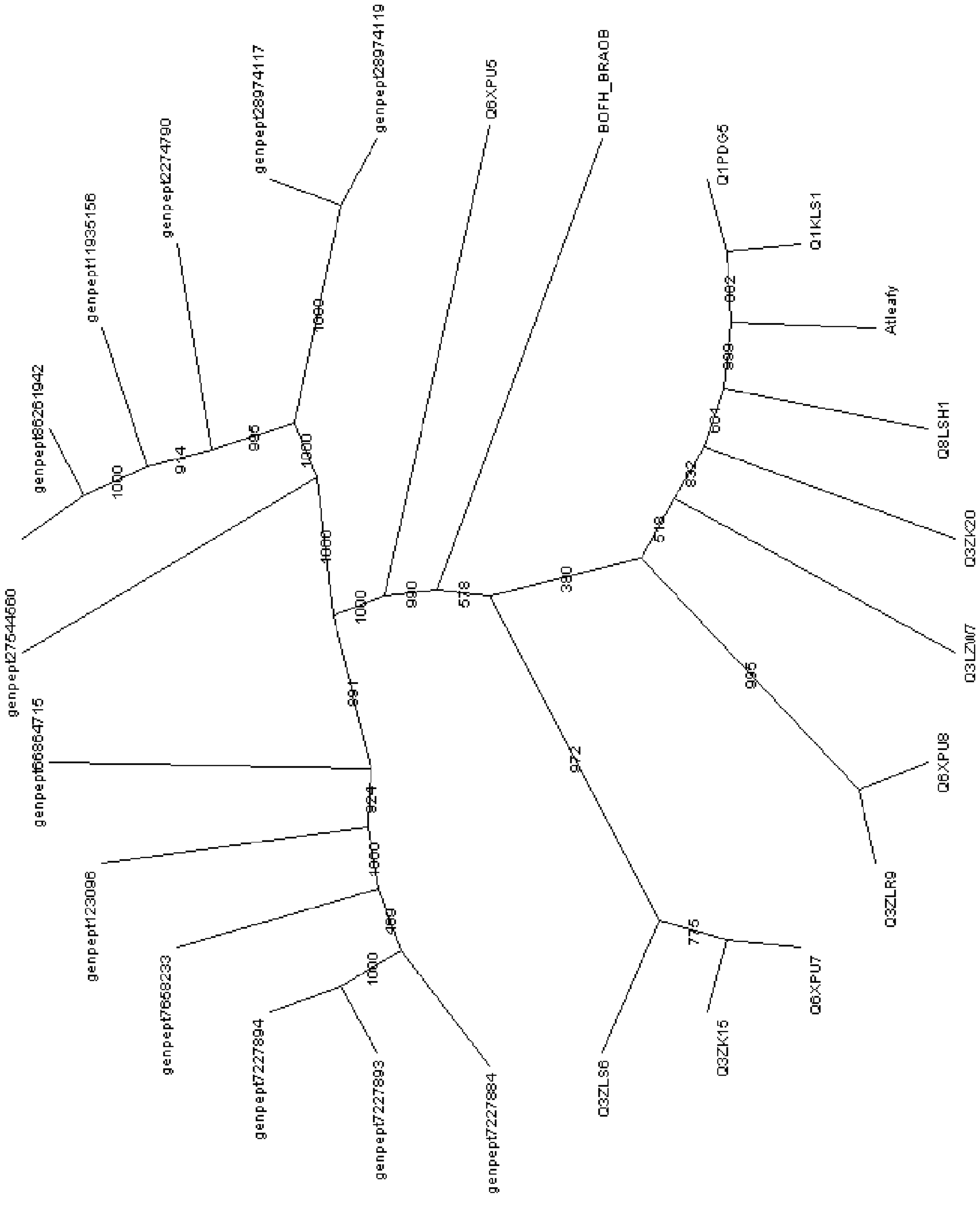
genpept28974119
genpept28974117
genpept2274790
genpept11935156
genpept36261942
genpept27544560
genpept66864715
genpept123096
genpept7658233
genpept7227894
genpept7227893
genpept7227884
Q6XPU5
BOFH_BRAOB
Q1PDG5
Q1KLS1
Atleafy
Q8LSH1
Q3ZK20
Q3LZW7
Q6XPU8
Q3ZLR9
Q6XPU7
Q3ZK15
Q3ZLS6

FIGURE 16

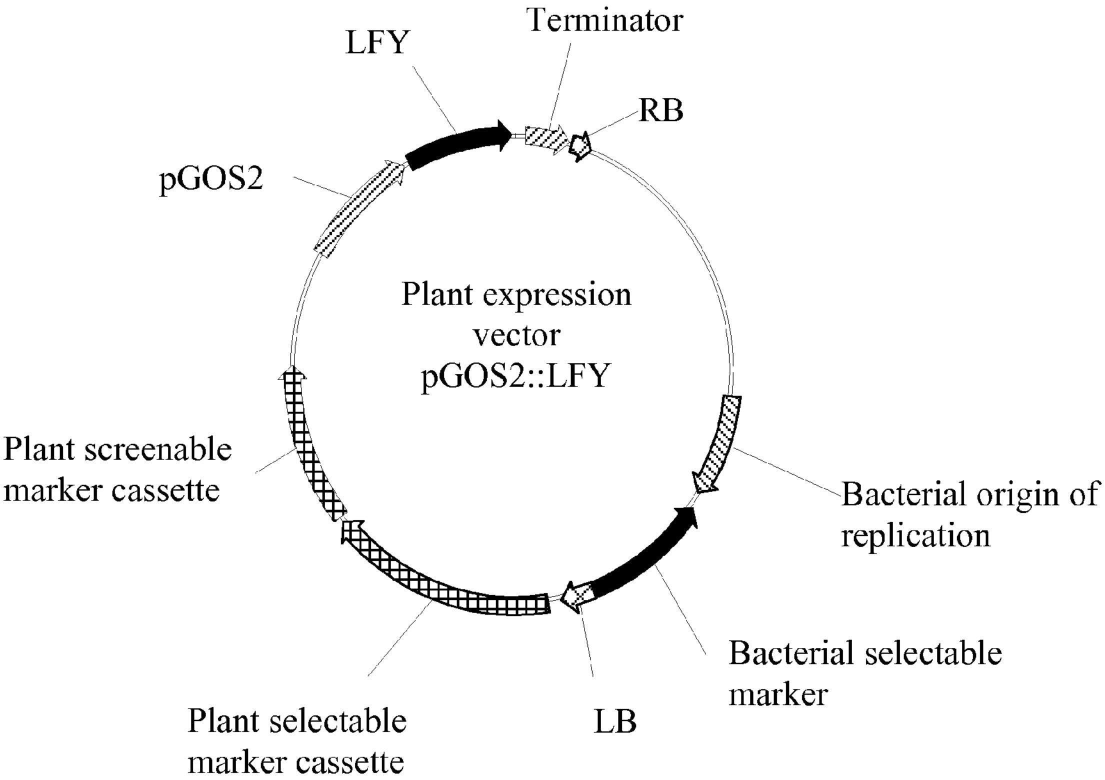
Terminator
LFY
RB
pGOS2
Plant expression vector pGOS2::LFY
Plant screenable marker cassette
Bacterial origin of replication
Bacterial selectable marker
LB
Plant selectable marker cassette

FIGURE 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008006171 A
- US 5811238 A
- US 6395547 A
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A
- WO 9738116 A
- WO 9836083 A
- WO 9915682 A
- WO 0015815 A
- EP 1198985 A1
- US 5164310 A
- US 5159135 A


### Non-patent literature cited in the description


- **TITTONELL et al.** *Agric Ecosys & Environ,* 2005, vol. 105, 213
- **FASOULA ; TOLLENAAR.** *Maydica,* 2005, vol. 50, 39
- **STEEGE et al.** *Plant Physiology,* 2005, vol. 139, 1078
- **HITTALMANI et al.** *Theoretical Applied Genetics,* 2003, vol. 107, 679
- **REBETZKE et al.** *Crop Science,* 2002, vol. 42, 739
- **GARDENER et al.** Physiology of Crop Plants. Iowa State University Press, 1985, 68-73
- **WANG et al.** *Planta,* 2003, vol. 218, 1-14
- **OLIVEIRA et al.** *Plant Physiol.,* 2002, vol. 129, 1170-1180
- **FUENTES et al.** *J. Exp. Bot.,* 2001, vol. 52, 1071-1081
- **MIGGE et al.** *Planta,* 2000, vol. 210, 252-260
- **MARTIN et al.** *Plant Cell,* vol. 18, 3252-3274
- **NUCCIO et al.** *J Biol Chem.,* 2000, vol. 275 (19), 14095-101
- **YANG et al.** *J. Mol. Biol.,* 2004, vol. 340, 695-706
- **DYKES et al.** *Biochem J.,* 1976, vol. 158 (3), 575-581
- **MCNEIL et al.** *PNAS,* 2001, vol. 98 (17), 10001-10005
- **VOELKER et al.** *Plant Physiol,* 1997, vol. 114, 669-677
- **BONAVENTURE et al.** *Plant Cell,* 2003, vol. 15, 1020-1033
- **DOERMANN et al.** *Plant Physiol,* 2000, vol. 123, 637-643
- **JONES et al.** *Plant Cell,* 1995, vol. 7, 359-371
- **WEIGEL et al.** *Cell,* 1992, vol. 69, 843-859
- **BOMBLIES et al.** *Development,* 2003, vol. 130, 2385-2395
- **CREIGHTON.** Proteins. 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462

- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic acid sequences Res,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **MUDGE et al.** *Plant J.,* 2002, vol. 31, 341
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* 1991, vol. 153, 386-395
- **DOWNEY et al.** *J. Biol. Chem.,* 2000, vol. 275, 39420
- **W SONG.** *PhD Thesis,* 1997
- **WANG et al.** *Plant Sci.,* 2002, vol. 163, 273
- **DIENER et al.** *Plant Cell,* 2001, vol. 13, 1625
- **QUESADA et al.** *lant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1995, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62

- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **SKRIVER et al.** *Proc Natl Acad Sci USA,* 1991, vol. 88, 7266-7270
- **WAGNER ; KOHORN.** *Plant Cell,* 2001, vol. 13 (2), 303-318
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1992, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- **B. JENES et al.** Techniques for Gene Transfer, in: Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- **F.F. WHITE.** Vectors for Gene Transfer in Higher Plants; in Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, Singapore, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. Singapore, World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84

- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **SCHULTZ et al.** *Proc. Natl. Acad. Sci. USA,* 1998, vol. 95, 5857-5864
- **LETUNIC et al.** *Nucleic acid sequences Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic acid sequences Research,* 2002, vol. 30 (1), 276-280
- **GASTEIGER et al.** ExPASy: the proteomics server for in-depth protein knowledge and analysis. *Nucleic acid sequences Res,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7
- **MARTIN et al.** *Anal. Biochem.,* 1982, vol. 125, 24-29
- **XUE.** *Plant J.,* 2005, vol. 41, 638-649
- Current Protocols in Molecular Biology
- **RABBANI et al.** *Plant Physiol,* 2003, vol. 133, 1755-1767
- **FRINK et al.** *Proc Natl Acad Sci USA,* 1999, vol. 96 (4), 1175-1180
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet,* 1991, vol. 7, 149-154
- **LAAN et al.** *Genome Res.,* 1995, vol. 5, 13-20
- **KAZAZIAN.** *J. Lab. Clin. Med,* 1999, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic acid sequence Res.,* 1990, vol. 18, 3671
- **WALTER et al.** *Nat. Genet.,* 1997, vol. 7, 22-28
- **DEAR ; COOK.** *Nucleic acid sequence Res,* 1989, vol. 17, 6795-6807
- **MARILLIA et al.** *Developments in Plant Genetics and Breeding,* 2000, vol. 5, 182-188
- **MAYER et al.** *BMC Plant Biology,* 2007
- **SAMBROOK.** Molecular Cloning: a laboratory manual. Cold Spring Harbor Laboratory Press, 2001
- **AUSUBEL et al.** Current Protocols in Molecular Biology. Current Protocols, 1994, vol. 1, 2
- **R.D.D. CROY.** Plant Molecular Biology Labfax. BIOS Scientific Publications Ltd, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **LARKIN et al.** *Bioinformatics,* 2007, vol. 23, 2947-2948
- **EDGAR.** *Nucleic Acids Research,* 2004, vol. 32 (5), 1792-97
- **HOWE et al.** *Bioinformatics,* 2002, vol. 18 (11), 1546-7
- **HUSON et al.** *BMC Bioinformatics,* 2007, vol. 8 (1), 460
- **THOMPSON et al.** *Nucleic Acids Res,* 1997, vol. 25, 4876-4882
- **CHENNA et al.** *Nucleic Acids Res,* 2003, vol. 31, 3497-3500
- *BMC Bioinformatics.,* 2003, vol. 4, 29
- *BMC Bioinformatics,* 2003, vol. 4, 29
- **PARK ; KANEHISA.** *Bioinformatics,* 2003, vol. 19, 1656-1663
- **KINGDON, H.S. ; HUBBARD, J.S. ; STADTMAN, E.R.** *Biochemistry,* 1968, vol. 7, 2136-2142
- **MAYER ; SHANKLIN.** *J Biol Chem,* 2005, vol. 280, 3621
- **VOELKER et al.** *Science,* 1992, vol. 257, 72-74
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158

SEQUENCE LISTING

<110> BASF Plant Science GmbH

<120> Plants having enhanced yield-related traits and a method for making the same

<130> PF60629

<150> EP 08158760.2
<151> 2008-06-20

<150> EP 08158760.2
<151> 2008-06-23

<150> US 61/074,712
<151> 2008-06-23

<150> US 61/074,686
<151> 2008-06-23

<150> US 61/075,784
<151> 2008-06-26

<150> EP 08159085.3
<151> 2008-06-26

<150> US 61/075,850
<151> 2008-06-26

<150> PCT/EP2009/057190
<151> 2009-06-10

<150> EP 09765788.6
<151> 2009-06-10

<160> 200

<170> PatentIn version 3.5

<210> 1
<211> 1149
<212> DNA
<213> Chlamydomonas reinhardtii

<400> 1
```
atggccgcgg gatctgttgg cgtcttcgcc accgatgaga agattggcag cctgctggac      60
cagtccatca cgcgccactt tctgtcgact gtgaccgacc agcagggcaa gatctgtgcc     120
gagtatgtgt ggatcggcgg ctccatgcac gacgtgcgct ccaagtcgcg caccctgtcc     180
accatcccca cgaagcccga ggacctgccc cactggaact acgacggctc ctccaccggc     240
caggcccccg gccacgactc agaggtctat ctcattcccc gctccatctt caaggacccc     300
ttccgcggcg gcgacaacat cctggtcatg tgcgactgct acgagccgcc caaggtcaac     360
cccgacggca ccctggccgc gcccaagccg atccccacga acacccgctt tgcctgcgcc     420
gaggtgatgg agaaggccaa gaaggaggag ccctggttcg gcattgagca ggagtacacg     480
ctgctcaacg ccatcaccaa gtggccgctg ggctggccca agggcggcta ccccgccccc     540
cagggcccct actactgctc ggccggcgcc ggcgtggcca tcggccgcga cgtggcggag     600
gtgcactacc gcctgtgcct ggccgcgggc gttaacatca gcggcgtgaa cgccgaggtg     660
ctgcccagcc agtgggagta ccaggtgggc ccgtgcgagg gcatcaccat gggcgaccac     720
atgtggatga gccgctatat catgtaccgc gtgtgcgaga tgttcaacgt ggaggtctcg     780
ttcgacccca agcccatccc cggcgactgg aacggctccg gcggccacac caactactcc     840
actaaggcca cccgcaccgc gcccgacggc tggaaggtca tccaggagca ctgcgccaag     900
ctggaggcgc gccacgccgt gcacatcgcc gcctacggcg agggcaacga gcgccgcctg     960
accggcaagc acgagaccag cagcatgagc gacttcagct ggggcgtggc caaccgcggc    1020
```

```
tgctccatcc gcgtgggccg catggtgccg gtggagaagt cgggctacta tgaggaccgc   1080
cggcctgcct ccaacctgga cgcctacgtc gtcacccgcc tcatcgtgga gaccaccatc   1140
cttctgtaa                                                           1149

<210>  2
<211>  382
<212>  PRT
<213>  Chlamydomonas reinhardtii

<400>  2
Met Ala Ala Gly Ser Val Gly Val Phe Ala Thr Asp Glu Lys Ile Gly
1               5                   10                  15
Ser Leu Leu Asp Gln Ser Ile Thr Arg His Phe Leu Ser Thr Val Thr
            20                  25                  30
Asp Gln Gln Gly Lys Ile Cys Ala Glu Tyr Val Trp Ile Gly Gly Ser
        35                  40                  45
Met His Asp Val Arg Ser Lys Ser Arg Thr Leu Ser Thr Ile Pro Thr
    50                  55                  60
Lys Pro Glu Asp Leu Pro His Trp Asn Tyr Asp Gly Ser Ser Thr Gly
65                  70                  75                  80
Gln Ala Pro Gly His Asp Ser Glu Val Tyr Leu Ile Pro Arg Ser Ile
                85                  90                  95
Phe Lys Asp Pro Phe Arg Gly Gly Asp Asn Ile Leu Val Met Cys Asp
            100                 105                 110
Cys Tyr Glu Pro Pro Lys Val Asn Pro Asp Gly Thr Leu Ala Ala Pro
        115                 120                 125
Lys Pro Ile Pro Thr Asn Thr Arg Phe Ala Cys Ala Glu Val Met Glu
    130                 135                 140
Lys Ala Lys Lys Glu Glu Pro Trp Phe Gly Ile Glu Gln Glu Tyr Thr
145                 150                 155                 160
Leu Leu Asn Ala Ile Thr Lys Trp Pro Leu Gly Trp Pro Lys Gly Gly
                165                 170                 175
Tyr Pro Ala Pro Gln Gly Pro Tyr Tyr Cys Ser Ala Gly Ala Gly Val
            180                 185                 190
Ala Ile Gly Arg Asp Val Ala Glu Val His Tyr Arg Leu Cys Leu Ala
        195                 200                 205
Ala Gly Val Asn Ile Ser Gly Val Asn Ala Glu Val Leu Pro Ser Gln
    210                 215                 220
Trp Glu Tyr Gln Val Gly Pro Cys Glu Gly Ile Thr Met Gly Asp His
225                 230                 235                 240
Met Trp Met Ser Arg Tyr Ile Met Tyr Arg Val Cys Glu Met Phe Asn
                245                 250                 255
Val Glu Val Ser Phe Asp Pro Lys Pro Ile Pro Gly Asp Trp Asn Gly
            260                 265                 270
Ser Gly Gly His Thr Asn Tyr Ser Thr Lys Ala Thr Arg Thr Ala Pro
        275                 280                 285
Asp Gly Trp Lys Val Ile Gln Glu His Cys Ala Lys Leu Glu Ala Arg
    290                 295                 300
His Ala Val His Ile Ala Ala Tyr Gly Glu Gly Asn Glu Arg Arg Leu
305                 310                 315                 320
Thr Gly Lys His Glu Thr Ser Ser Met Ser Asp Phe Ser Trp Gly Val
                325                 330                 335
Ala Asn Arg Gly Cys Ser Ile Arg Val Gly Arg Met Val Pro Val Glu
            340                 345                 350
Lys Ser Gly Tyr Tyr Glu Asp Arg Arg Pro Ala Ser Asn Leu Asp Ala
        355                 360                 365
Tyr Val Val Thr Arg Leu Ile Val Glu Thr Thr Ile Leu Leu
    370                 375                 380

<210>  3
<211>  15
<212>  PRT
<213>  Artificial sequence
```

<220>
<223> motif 1

<220>
<221> VARIANT
<222> (3)..(3)
<223> /replace = "Leu" /replace = "Phe"

<220>
<221> VARIANT
<222> (4)..(4)
<223> /replace = "Thr"

<220>
<221> VARIANT
<222> (9)..(9)
<223> /replace = "Ser" /replace = "Pro"

<220>
<221> VARIANT
<222> (10)..(10)
<223> /replace = "Ser"

<220>
<221> VARIANT
<222> (11)..(11)
<223> /replace = "Asp"

<220>
<221> VARIANT
<222> (12)..(12)
<223> /replace = "Leu" /replace = "Ala" /replace = "Met"

<220>
<221> VARIANT
<222> (14)..(14)
<223> /replace = "Ala"

<400> 3
Gly Tyr Tyr Glu Asp Arg Arg Pro Ala Ala Asn Val Asp Pro Tyr
1 5 10 15

<210> 4
<211> 13
<212> PRT
<213> Artificial sequence

<220>
<223> motif 2

<220>
<221> VARIANT
<222> (3)..(3)
<223> /replace = "Phe"

<220>
<221> VARIANT
<222> (6)..(6)
<223> /replace = "Glu" /replace = "Asp" /replace = "Ser" /replace = "Gly" /replace = "Leu" /replace = "Val"

<220>

<221> VARIANT
<222> (7)..(7)
<223> /replace = "Asn" /replace = "Asp"

<220>
<221> VARIANT
<222> (8)..(8)
<223> /replace = "Asn"

<220>
<221> VARIANT
<222> (9)..(9)
<223> /replace = "Ile"

<220>
<221> VARIANT
<222> (10)..(10)
<223> /replace = "Ile"

<220>
<221> VARIANT
<222> (12)..(12)
<223> /replace = "Ile" /replace = "Met"

<220>
<221> VARIANT
<222> (13)..(13)
<223> /replace = "Thr" /replace = "Ala"

<400> 4
Asp Pro Ile Arg Gly Ala Pro His Val Leu Val Leu Cys
1 5 10

<210> 5
<211> 8
<212> PRT
<213> Artificial sequence

<220>
<223> motif 3

<220>
<221> VARIANT
<222> (2)..(2)
<223> /replace = "Leu" /replace = "Gly" /replace = "Cys"

<220>
<221> VARIANT
<222> (4)..(4)
<223> /replace = "Ser" /replace = "Ile" /replace = "Val" /replace = "Phe"

<220>
<221> VARIANT
<222> (5)..(5)
<223> /replace = "Lys"

<220>
<221> VARIANT
<222> (6)..(6)
<223> /replace = "Tyr" /replace = "Val"

<220>

```
<221>  VARIANT
<222>  (8)..(8)
<223>  /replace = "Ser" /replace = "Asn"

<400>  5
Gly Ala His Thr Asn Phe Ser Thr
1               5

<210>  6
<211>  1179
<212>  DNA
<213>  Oryza sativa

<400>  6
ttgcagttgt gaccaagtaa gctgagcatg cccttaactt cacctagaaa aaagtatact      60
tggcttaact gctagtaaga catttcagaa ctgagactgg tgtacgcatt tcatgcaagc     120
cattaccact ttacctgaca ttttggacag agattagaaa tagtttcgta ctacctgcaa     180
gttgcaactt gaaaagtgaa atttgttcct tgctaatata ttggcgtgta attcttttat     240
gcgttagcgt aaaaagttga aatttgggtc aagttactgg tcagattaac cagtaactgg     300
ttaaagttga aagatggtct tttagtaatg gagggagtac tacactatcc tcagctgatt     360
taaatcttat tccgtcggtg gtgatttcgt caatctccca acttagtttt tcaatatatt     420
cataggatag agtgtgcata tgtgtgttta tagggatgag tctacgcgcc ttatgaacac     480
ctacttttgt actgtatttg tcaatgaaaa gaaaatctta ccaatgctgc gatgctgaca     540
ccaagaagag gcgatgaaaa gtgcaacgga tatcgtgcca cgtcggttgc caagtcagca     600
cagacccaat gggcctttcc tacgtgtctc ggccacagcc agtcgtttac cgcacgttca     660
catgggcacg aactcgcgtc atcttcccac gcaaaacgac agatctgccc tatctggtcc     720
cacccatcag tggcccacac ctcccatgct gcattatttg cgactcccat cccgtcctcc     780
acgcccaaac accgcacacg ggtcgcgata gccacgaccc aatcacacaa cgccacgtca     840
ccatatgtta cgggcagcca tgcgcagaag atcccgcgac gtcgctgtcc cccgtgtcgg     900
ttacgaaaaa atatcccacc acgtgtcgct ttcacaggac aatatctcga aggaaaaaaa     960
tcgtagcgga aaatccgagg cacgagctgc gattggctgg gaggcgtcca gcgtggtggg    1020
gggcccaccc ccttatcctt agcccgtggc gctcctcgct cctcgggtcc gtgtataaat    1080
accctccgga actcactctt gctggtcacc aacacgaagt aaaaggacac cagaaacata    1140
gtacacttga gctcactcca aactcaaaca ctcacacca                           1179

<210>  7
<211>  53
<212>  DNA
<213>  Artificial sequence

<220>
<223>  primer: prm08458

<400>  7
ggggacaagt ttgtacaaaa aagcaggctt aaacaatggc cgcgggatct gtt            53

<210>  8
<211>  50
<212>  DNA
<213>  Artificial sequence

<220>
<223>  primer: prm08459

<400>  8
ggggaccact ttgtacaaga aagctgggtg ctgctcctgc gcttacagaa                50

<210>  9
<211>  357
<212>  PRT
<213>  Aureococcus anophagefferens

<400>  9
```

Met Ala Ser Met Asp Gln Ala Val Leu Gly Lys Tyr Met Gly Leu Asp
1               5                   10                  15
Thr Gly Asp Asp Cys Gln Val Glu Tyr Val Phe Leu Asp Lys Asp Gln
            20                  25                  30
Val Ala Arg Ser Lys Cys Arg Thr Leu Pro Leu Lys Lys Val Gln Gly
        35                  40                  45
Pro Val Asp Ala Tyr Pro Lys Trp Asn Tyr Asp Gly Ser Ser Thr Gly
    50                  55                  60
Gln Ala Pro Gly Asp Asp Ser Glu Val Met Ile Val Pro Arg Ala Lys
65                  70                  75                  80
Tyr Pro Asp Pro Phe Arg Gly Gly Asn His Val Leu Val Leu Cys Asp
                85                  90                  95
Thr Tyr Glu Pro Asp Gly Thr Pro Leu Pro Thr Asn Thr Arg Ala Pro
            100                 105                 110
Ala Val Ala Arg Phe Glu Ser Gly Gly Ala Lys Glu Gln Val Pro Trp
        115                 120                 125
Tyr Gly Leu Glu Gln Glu Tyr Thr Leu Phe Asn Leu Asp Gly Val Thr
    130                 135                 140
Pro Leu Gly Trp Pro Val Gly Gly Phe Pro Lys Pro Gln Gly Pro Tyr
145                 150                 155                 160
Tyr Cys Gly Ala Gly Ala Asp Arg Ala Phe Gly Arg Ala Val Ser Glu
                165                 170                 175
Ala His Tyr Arg Ala Cys Leu Tyr Ala Gly Leu Glu Val Ser Gly Thr
            180                 185                 190
Asn Ala Glu Val Met Pro Gly Gln Trp Glu Tyr Gln Ile Gly Pro Ser
        195                 200                 205
Ile Gly Ile Asp Ala Ala Asp Gln Leu Thr Ile Ser Arg Tyr Ile Leu
    210                 215                 220
Ser Arg Val Cys Glu Asp Leu Gly Val Ile Val Thr Ile Asp Pro Lys
225                 230                 235                 240
Pro Ile Ala Gly Asp Trp Asn Gly Ala Gly Met His Ile Asn Phe Ser
                245                 250                 255
Thr Glu Ser Thr Arg Lys Glu Gly Gly Leu Ala Val Ile Glu Ala Met
            260                 265                 270
Cys Glu Lys Leu Gly Ala Lys His Thr Glu His Ile Ala Ala Tyr Gly
        275                 280                 285
Glu Gly Asn Glu Arg Arg Leu Thr Gly Asp Cys Glu Thr Ala Ser Ile
    290                 295                 300
Asp Gln Phe Ser Tyr Gly Val Ala Asp Arg Gly Cys Ser Ile Arg Ile
305                 310                 315                 320
Pro Arg Asp Thr Ala Ala Asp Lys Lys Gly Tyr Leu Glu Asp Arg Arg
                325                 330                 335
Pro Ala Ser Asn Val Asp Pro Tyr Val Ala Thr Ser Leu Ile Phe Ala
            340                 345                 350
Thr Cys Thr Ser Ala
        355

<210> 10
<211> 380
<212> PRT
<213> Chlamydomonas reinhardtii

<400> 10
Met Ala Phe Ala Leu Arg Gly Val Thr Ala Lys Ala Ser Gly Arg Thr
1               5                   10                  15
Ala Gly Ala Arg Ser Ser Gly Arg Thr Leu Thr Val Arg Val Gln Ala
            20                  25                  30
Tyr Gly Met Lys Ala Glu Tyr Ile Trp Ala Asp Gly Asn Glu Gly Lys
        35                  40                  45
Pro Glu Lys Gly Met Ile Phe Asn Glu Met Arg Ser Lys Thr Lys Cys
    50                  55                  60
Phe Glu Ala Pro Leu Gly Leu Asp Ala Ser Glu Tyr Pro Asp Trp Ser
65                  70                  75                  80

Phe Asp Gly Ser Ser Thr Gly Gln Ala Glu Gly Asn Asn Ser Asp Cys
85 90 95
Ile Leu Arg Pro Val Arg Val Val Thr Asp Pro Ile Arg Gly Ala Pro
100 105 110
His Val Leu Val Met Cys Glu Val Phe Ala Pro Asp Gly Lys Pro His
115 120 125
Ser Thr Asn Thr Arg Ala Lys Leu Arg Glu Ile Ile Asp Asp Lys Val
130 135 140
Thr Ala Glu Asp Cys Trp Tyr Gly Phe Glu Gln Glu Tyr Thr Met Leu
145 150 155 160
Ala Lys Thr Ser Gly His Ile Tyr Gly Trp Pro Ala Gly Gly Phe Pro
165 170 175
Ala Pro Gln Gly Pro Phe Tyr Cys Gly Val Gly Ala Glu Ser Ala Phe
180 185 190
Gly Arg Pro Leu Ala Glu Ala His Met Glu Ala Cys Met Lys Ala Gly
195 200 205
Leu Val Ile Ser Gly Ile Asn Ala Glu Val Met Pro Gly Gln Trp Glu
210 215 220
Tyr Gln Ile Gly Pro Val Gly Pro Leu Ala Leu Gly Asp Glu Val Met
225 230 235 240
Leu Ser Arg Trp Leu Leu His Arg Leu Gly Glu Asp Phe Gly Ile Val
245 250 255
Ser Thr Phe Asn Pro Lys Pro Val Arg Thr Gly Asp Trp Asn Gly Thr
260 265 270
Gly Ala His Thr Asn Phe Ser Thr Lys Gly Met Arg Val Pro Gly Gly
275 280 285
Met Lys Val Ile Glu Glu Ala Val Glu Lys Leu Ser Lys Thr His Ile
290 295 300
Glu His Ile Thr Gln Tyr Gly Ile Gly Asn Glu Ala Arg Leu Thr Gly
305 310 315 320
Lys His Glu Thr Cys Asp Ile Asn Thr Phe Lys His Gly Val Ala Asp
325 330 335
Arg Gly Ser Ser Ile Arg Ile Pro Leu Pro Val Met Leu Lys Gly Tyr
340 345 350
Gly Tyr Leu Glu Asp Arg Arg Pro Ala Ala Asn Val Asp Pro Tyr Thr
355 360 365
Val Ala Arg Leu Leu Ile Lys Thr Val Leu Lys Gly
370 375 380

<210> 11
<211> 375
<212> PRT
<213> Chlamydomonas reinhardtii

<400> 11
Met Arg Leu Asn Thr Gln Val Ser Gly Arg Ala Thr Gly Ala Pro Arg
1 5 10 15
Gln Gly Arg Arg Leu Thr Val Arg Val Gln Ala Tyr Gly Met Lys Ala
20 25 30
Glu Tyr Ile Trp Ala Asp Gly Asn Glu Gly Lys Ala Glu Lys Gly Met
35 40 45
Ile Phe Asn Glu Met Arg Ser Lys Thr Lys Cys Phe Glu Ala Pro Leu
50 55 60
Gly Leu Asp Ala Ser Glu Tyr Pro Asp Trp Ser Phe Asp Gly Ser Ser
65 70 75 80
Thr Gly Gln Ala Glu Gly Asn Asn Ser Asp Cys Ile Leu Arg Pro Val
85 90 95
Arg Val Val Thr Asp Pro Ile Arg Gly Ala Pro His Val Leu Val Met
100 105 110
Cys Glu Val Phe Ala Pro Asp Gly Lys Pro His Ser Thr Asn Thr Arg
115 120 125
Ala Lys Leu Arg Glu Ile Ile Asp Asp Lys Val Thr Ala Glu Asp Cys
130 135 140

```
Trp Tyr Gly Phe Glu Gln Glu Tyr Thr Met Leu Ala Lys Thr Ser Gly
145                 150                 155                 160
His Ile Tyr Gly Trp Pro Ala Gly Gly Phe Pro Ala Pro Gln Gly Pro
                165                 170                 175
Phe Tyr Cys Gly Val Gly Ala Glu Ser Ala Phe Gly Arg Pro Leu Ala
            180                 185                 190
Glu Ala His Met Glu Ala Cys Met Lys Ala Gly Leu Val Ile Ser Gly
        195                 200                 205
Ile Asn Ala Glu Val Met Pro Gly Gln Trp Glu Tyr Gln Ile Gly Pro
    210                 215                 220
Val Gly Pro Leu Ala Leu Gly Asp Glu Val Met Leu Ser Arg Trp Leu
225                 230                 235                 240
Leu His Arg Leu Gly Glu Asp Phe Gly Ile Val Ser Thr Phe Asn Pro
                245                 250                 255
Lys Pro Val Arg Thr Gly Asp Trp Asn Gly Thr Gly Ala His Thr Asn
            260                 265                 270
Phe Ser Thr Lys Gly Met Arg Val Pro Gly Gly Met Lys Val Ile Glu
        275                 280                 285
Glu Ala Val Glu Lys Leu Ser Lys Thr His Ile Glu His Ile Thr Gln
    290                 295                 300
Tyr Gly Ile Gly Asn Glu Ala Arg Leu Thr Gly Lys His Glu Thr Cys
305                 310                 315                 320
Asp Ile Asn Thr Phe Lys His Gly Val Ala Asp Arg Gly Ser Ser Ile
                325                 330                 335
Arg Ile Pro Leu Pro Val Met Leu Lys Gly Tyr Gly Tyr Leu Glu Asp
            340                 345                 350
Arg Arg Pro Ala Ala Asn Val Asp Pro Tyr Thr Val Ala Arg Leu Leu
        355                 360                 365
Ile Lys Thr Val Leu Lys Gly
    370                 375
```

<210> 12
<211> 577
<212> PRT
<213> Chlamydomonas reinhardtii

<400> 12

```
Met Asp Leu Ala Thr Ala Leu Gly Leu Gly Ile Ala Pro Pro Pro Pro
1               5                   10                  15
Ala Asp Asp Ser Ser His His Ser Thr Thr Glu Ala Cys Thr Leu Pro
            20                  25                  30
Ala Tyr Leu Arg Ala Pro Glu Val Thr Ala Gln Val Met Ala Glu Tyr
        35                  40                  45
Ile Trp Leu Met Gly Gly Thr Gly Gln Leu Arg Ser Lys Thr Lys Val
    50                  55                  60
Leu Asp Ala Lys Pro Ser Cys Ala Glu Glu Ala Pro Ile Met Ile Val
65                  70                  75                  80
Glu Ser Asn Pro Asp Gly Gln Leu Ala Glu Pro Asn His Glu Leu Phe
                85                  90                  95
Leu Lys Pro Arg Lys Ile Phe Arg Asp Pro Phe Arg Gly Gly Asp His
            100                 105                 110
Ile Leu Val Leu Cys Asp Thr Phe Ile Val Ala Gln Val Val Ala Glu
        115                 120                 125
Ala Gly Ala Ala Pro Ser Thr Val Leu Gln Pro Ser Glu Thr Asn Ser
    130                 135                 140
Arg Val Ala Cys Glu Asn Val Leu Arg Val Ala Glu Gln Gln Glu Pro
145                 150                 155                 160
Val Phe Ala Val Glu Gln Glu Tyr Ala Ile Ile His Pro Ala Tyr Pro
                165                 170                 175
Thr Lys Val Pro Leu Gly Pro Arg Arg Pro Ser Thr Ser Arg Ala Ser
            180                 185                 190
Ser Cys His Ser Gly Ser Arg Arg Ser Ser Tyr Val Ser Ser Gly Ser
        195                 200                 205
```

Ala Arg Gly Gly Ile Gly Lys Asn Ser Ser His His Gly Gly Lys Gln
    210                 215                 220
Ser His Ala Ala Ala Ala Ala Ala Ala Ala Ala Val Ala Gly Ile Pro
225                 230                 235                 240
Trp Pro Ser Pro Asp Ala Cys Glu Gln Thr Ala Gln Glu Ala Ser Ala
                245                 250                 255
Ala Arg Gln Lys Ala Ser Arg Gln Leu Ala Asp Ser His Leu Arg Cys
            260                 265                 270
Cys Leu Phe Ala Gly Val Arg Val Thr Gly Ala Asp Val His Ser Leu
        275                 280                 285
Asp Gly Leu His Ser Tyr Lys Ile Gly Pro Ser Pro Gly Val Asp Leu
    290                 295                 300
Gly Asp Asp Leu Trp Thr Ser Arg Tyr Leu Leu Gln Arg Val Ala Glu
305                 310                 315                 320
Gln His Ser Ala Ser Val Ser Trp Glu Pro Asp Ser Met Pro Ser Glu
                325                 330                 335
Arg Pro Leu Gly Cys His Phe Lys Tyr Ser Thr Ala Ser Thr Arg Gln
            340                 345                 350
Ala Pro His Gly Leu Asn Ala Ile Glu Gln Gln Leu Val Arg Leu Gln
        355                 360                 365
Ala Thr His Val Gln His Gln Val Ala Tyr Asn Asp Gly Arg Leu Asp
    370                 375                 380
Arg Leu Ser Ser Pro Glu Ala Ser Thr Phe Thr His Ala Val Gly Ser
385                 390                 395                 400
Ala Asn Ala Ser Val Val Val Pro Ser Leu Thr Phe Leu Gln Gln Gly
                405                 410                 415
Gly Tyr Phe Thr Asp Arg Arg Pro Pro Ser Asp Ala Asp Pro Tyr Lys
            420                 425                 430
Val Thr Leu Leu Leu Ala Ala Thr Thr Leu Asp Ile Pro Leu Pro Lys
        435                 440                 445
Leu Pro Ala Ser Ser Ser Ala Gly Asn Thr Ala Ala Asn Cys Ser Gly
    450                 455                 460
Gly Met Ser Ala Gly Pro Ser Ser Cys Pro Ala Ala Ala Ala Leu Pro
465                 470                 475                 480
Phe Gly Ser Pro Met Gln Ser Tyr Leu Leu Ala Ala Ala Ala Ala Gln
                485                 490                 495
Arg Gln Gln Gln Gln Gln His Leu Met Phe Asp Thr Glu Ser Glu Glu
            500                 505                 510
Cys Asp Ser Val Asp Glu Asp Asp Ala Met Thr Glu Asp Ser Ala Ala
        515                 520                 525
Leu Leu Ala Lys Met Asp Asp Asp Gly Gly Ala Ala Glu Ala Ser Ser
    530                 535                 540
Cys Asp Ser Asp Phe Glu Asp Gln Asp Asp Ala Ser Ser Ser Pro Ile
545                 550                 555                 560
Thr Gly Thr Trp Ala Asp Asn Asp Cys Thr His Met Leu Gly Ala Gly
                565                 570                 575
Ile

<210> 13
<211> 386
<212> PRT
<213> Helicosporidum sp.

<400> 13
Met Ser Pro Pro Thr Gly Glu Lys Tyr Ser Leu Pro Pro Val Phe Gly
1               5                   10                  15
Thr Gln Gly Gln Ile Thr Gln Leu Leu Asp Pro Ile Met Ala Glu Arg
            20                  25                  30
Phe Lys Asp Leu Ser Gln His Gly Lys Val Met Ala Glu Tyr Val Trp
        35                  40                  45
Ile Gly Gly Thr Gly Ser Asp Leu Arg Cys Lys Thr Arg Val Leu Asp
    50                  55                  60

Ser Val Pro Asn Ser Val Glu Asp Leu Pro Val Trp Asn Tyr Asp Gly
65 70 75 80
Ser Ser Thr Gly Gln Ala Pro Gly Asp Asp Ser Glu Val Phe Leu Ile
85 90 95
Pro Arg Ala Ile Tyr Arg Asp Pro Phe Arg Gly Gly Asp Asn Ile Leu
100 105 110
Val Leu Ala Asp Thr Tyr Glu Pro Pro Arg Val Leu Pro Asn Gly Lys
115 120 125
Val Ser Pro Pro Val Pro Leu Pro Thr Asn Ser Arg His Ala Cys Ala
130 135 140
Glu Ala Met Asp Lys Ala Ala Ala His Glu Pro Trp Phe Gly Ile Glu
145 150 155 160
Gln Glu Tyr Thr Val Leu Asp Ala Arg Thr Lys Trp Pro Leu Gly Trp
165 170 175
Pro Ser Asn Gly Phe Pro Gly Pro Gln Gly Pro Tyr Tyr Cys Ala Ala
180 185 190
Gly Ala Gly Cys Ala Ile Gly Arg Asp Leu Ile Glu Ala His Leu Lys
195 200 205
Ala Cys Leu Phe Ala Gly Ile Asn Val Ser Gly Val Asn Ala Glu Val
210 215 220
Met Pro Ser Gln Trp Glu Tyr Gln Val Gly Pro Cys Thr Gly Ile Glu
225 230 235 240
Ser Gly Asp Gln Met Trp Met Ser Arg Tyr Ile Leu Ile Arg Cys Ala
245 250 255
Glu Leu Tyr Asn Val Glu Val Ser Phe Asp Pro Lys Pro Val Pro Gly
260 265 270
Asp Trp Asn Gly Ala Gly Gly His Val Asn Tyr Ser Asn Lys Ala Thr
275 280 285
Arg Thr Ala Glu Thr Gly Trp Ala Ala Ile Gln Gln Gln Val Glu Lys
290 295 300
Leu Gly Lys Arg His Ala Val His Ile Ala Ala Tyr Gly Glu Gly Asn
305 310 315 320
Glu Arg Arg Leu Thr Gly Lys His Glu Thr Ser Ser Met Asn Asp Phe
325 330 335
Ser Trp Gly Val Ala Asn Arg Gly Ala Ser Val Arg Val Gly Arg Leu
340 345 350
Val Pro Val Glu Lys Cys Gly Tyr Tyr Glu Asp Arg Arg Pro Ala Ser
355 360 365
Asn Leu Asp Pro Tyr Val Val Thr Arg Leu Leu Val Glu Thr Thr Leu
370 375 380
Leu Met
385

<210> 14
<211> 416
<212> PRT
<213> Thalassiosira pseudonana

<400> 14
Met Lys Leu Ser Ile Ala Leu Leu Ser Met Ala Ala Thr Ala Thr Ala
1 5 10 15
Phe Ala Pro Ser Leu Thr Thr Pro Ser Arg Thr Thr Ser Leu Ser Met
20 25 30
Val Asn Pro Leu Glu Ile Arg Thr Gly Lys Ala Gln Leu Asp His Ser
35 40 45
Val Ile Asp Arg Phe Asn Ala Leu Pro Tyr Pro Ala Asp Lys Val Leu
50 55 60
Ala Glu Tyr Val Trp Val Asp Ala Lys Gly Glu Cys Arg Ser Lys Thr
65 70 75 80
Arg Thr Leu Pro Val Ala Arg Thr Thr Ala Val Asp Asn Leu Pro Arg
85 90 95
Trp Asn Phe Asp Gly Ser Ser Thr Gly Gln Ala Pro Gly Asp Asp Ser
100 105 110

Glu Val Ile Leu Arg Pro Cys Arg Ile Phe Lys Asp Pro Phe Arg Pro
        115                 120                 125
Arg Asn Asp Gly Val Asp Asn Ile Leu Val Met Cys Asp Thr Tyr Thr
    130                 135                 140
Pro Ala Gly Glu Ala Leu Pro Thr Asn Thr Arg Ala Ile Ala Ala Lys
145                 150                 155                 160
Ala Phe Glu Gly Lys Glu Asp Glu Glu Ile Trp Phe Gly Leu Glu Gln
                165                 170                 175
Glu Phe Thr Leu Phe Asn Leu Asp Gln Arg Thr Pro Leu Gly Trp Pro
            180                 185                 190
Lys Gly Gly Val Pro Ala Arg Ala Gln Gly Pro Tyr Tyr Cys Ser Val
        195                 200                 205
Gly Pro Glu Asn Ser Phe Gly Arg Ala Ile Thr Asp Thr Met Tyr Arg
    210                 215                 220
Ala Cys Leu Tyr Ala Gly Ile Glu Ile Ser Gly Thr Asn Gly Glu Val
225                 230                 235                 240
Met Pro Gly Gln Gln Glu Tyr Gln Val Gly Pro Cys Val Gly Ile Asp
                245                 250                 255
Ala Gly Asp Gln Leu Gln Met Ser Arg Tyr Ile Leu Gln Arg Val Cys
            260                 265                 270
Glu Glu Phe Gln Val Tyr Cys Thr Leu His Pro Lys Pro Ile Val Glu
        275                 280                 285
Gly Asp Trp Asn Gly Ala Gly Met His Thr Asn Val Ser Thr Lys Ser
    290                 295                 300
Met Arg Glu Glu Gly Gly Leu Glu Val Ile Lys Lys Ala Ile Tyr Lys
305                 310                 315                 320
Leu Gly Ala Lys His Gln Glu His Ile Ala Val Tyr Gly Glu Gly Asn
                325                 330                 335
Glu Leu Arg Leu Thr Gly Lys His Glu Thr Ala Ser Ile Asp Gln Phe
            340                 345                 350
Ser Phe Gly Val Ala Asn Arg Gly Ala Ser Val Arg Ile Gly Arg Asp
        355                 360                 365
Thr Glu Ala Glu Gly Lys Gly Tyr Phe Glu Asp Arg Arg Pro Ser Ser
    370                 375                 380
Asn Ala Asp Pro Tyr Leu Val Thr Gly Lys Ile Met Ala Thr Ile Met
385                 390                 395                 400
Glu Asp Val Asp Val Pro Glu Ile Ser Ala Leu Asp Arg Ala Glu Ala
                405                 410                 415

<210> 15
<211> 379
<212> PRT
<213> Volvox carterii

<400> 15
Met Ala Thr Met Arg Met Ser Thr Lys Ala Gln Gly Arg Val Gly Ile
1               5                   10                  15
Val Arg Asn Thr Arg Thr Leu Thr Val Arg Val Arg Ala Tyr Gly Met
            20                  25                  30
Lys Ala Glu Tyr Ile Trp Ala Asp Gly Asn Glu Gly Arg Pro Glu Lys
        35                  40                  45
Gly Met Ile Phe Asn Glu Met Arg Ser Lys Thr Lys Val Phe Asp Glu
    50                  55                  60
Ala Leu Pro Leu Glu Ala Gly Gln Tyr Pro Asp Trp Ser Phe Asp Gly
65                  70                  75                  80
Ser Ser Thr Gly Gln Ala Ala Gly Asn Asn Ser Asp Cys Ile Leu Arg
                85                  90                  95
Pro Val Arg Val Ile Lys Asp Pro Ile Arg Gly Glu Pro His Val Leu
            100                 105                 110
Val Met Cys Glu Val Phe Ala Pro Asp Gly Thr Pro His Pro Thr Asn
        115                 120                 125
Thr Arg Ala Lys Leu Arg Asp Ile Ile Asp Asp Lys Val Leu Ala Glu
    130                 135                 140

Asp Cys Trp Tyr Gly Leu Glu Gln Glu Tyr Thr Met Leu Gln Lys Thr
145 150 155 160
Thr Gly Gln Ile Tyr Gly Trp Pro Ser Gly Gly Tyr Pro Ala Pro Gln
165 170 175
Gly Pro Phe Tyr Cys Gly Val Gly Ala Glu Ser Ala Phe Gly Arg Pro
180 185 190
Leu Ala Glu Ala His Met Glu Ala Cys Met Lys Ala Gly Leu Lys Ile
195 200 205
Ser Gly Ile Asn Ala Glu Val Met Pro Gly Gln Trp Glu Tyr Gln Ile
210 215 220
Gly Pro Val Gly Pro Leu Glu Met Gly Asp Glu Val Met Leu Ser Arg
225 230 235 240
Trp Leu Leu His Arg Leu Gly Glu Asp Phe Gly Ile Val Cys Thr Phe
245 250 255
Asn Pro Lys Pro Val Arg Thr Gly Asp Trp Asn Gly Thr Gly Ala His
260 265 270
Thr Asn Phe Ser Thr Lys Ser Met Arg Gln Pro Gly Gly Met Lys Val
275 280 285
Ile Glu Asp Ala Val Glu Lys Leu Ser Lys Thr His Ile Glu His Ile
290 295 300
Thr Gln Tyr Gly Leu Gly Asn Glu Ala Arg Leu Thr Gly Lys His Glu
305 310 315 320
Thr Cys Asp Ile Asn Thr Phe Lys His Gly Val Ala Asp Arg Gly Ser
325 330 335
Ser Ile Arg Ile Pro Leu Pro Val Met Leu Lys Gly Tyr Gly Tyr Leu
340 345 350
Glu Asp Arg Arg Pro Ala Ala Asn Val Asp Pro Tyr Thr Val Ala Arg
355 360 365
Leu Leu Ile Lys Ser Ile Leu Lys Gly Pro Gln
370 375

<210> 16
<211> 382
<212> PRT
<213> Volvox carterii

<400> 16
Met Ala Ala Gly Ser Ile Gly Val Phe Ala Thr Asp Glu Lys Ile Gly
1 5 10 15
Ser Leu Leu Asp Gln Ser Ile Thr Arg His Phe Leu Thr Asn Val Thr
20 25 30
Asp Gln Cys Gly Lys Ile Thr Ala Glu Tyr Val Trp Ile Gly Gly Ser
35 40 45
Met Gln Asp Leu Arg Ser Lys Ser Arg Thr Leu Thr Ser Val Pro Thr
50 55 60
Lys Pro Glu Asp Leu Pro His Trp Asn Tyr Asp Gly Ser Ser Thr Gly
65 70 75 80
Gln Ala Pro Gly His Asp Ser Glu Val Tyr Leu Ile Pro Arg Arg Ile
85 90 95
Phe Arg Asp Pro Phe Arg Gly Gly Asp Asn Ile Leu Val Met Cys Asp
100 105 110
Cys Tyr Glu Pro Pro Lys Ala Asn Ala Asp Gly Ile Leu Gln Pro Pro
115 120 125
Lys Pro Ile Pro Thr Asn Thr Arg Tyr Ala Cys Ala Glu Ala Met Glu
130 135 140
Lys Ala Lys Asp Glu Glu Pro Trp Phe Gly Ile Glu Gln Glu Tyr Thr
145 150 155 160
Leu Leu Asn Ala Ile Thr Lys Trp Pro Leu Gly Trp Pro Lys Gly Gly
165 170 175
Tyr Pro Ala Pro Gln Gly Pro Tyr Tyr Cys Ser Ala Gly Ala Gly Val
180 185 190
Ala Ile Gly Arg Asp Val Ala Glu Val His Tyr Arg Leu Cys Leu Tyr
195 200 205

```
Ala Gly Val Asn Ile Ser Gly Val Asn Ala Glu Val Leu Pro Ser Gln
    210                 215                 220
Trp Glu Tyr Gln Val Gly Pro Cys Glu Gly Ile Glu Met Gly Asp His
225                 230                 235                 240
Met Trp Met Ser Arg Tyr Ile Met Tyr Arg Val Cys Glu Met Phe Asn
                245                 250                 255
Val Glu Val Ser Phe Asp Pro Lys Pro Ile Pro Gly Asp Trp Asn Gly
            260                 265                 270
Ser Gly Gly His Thr Asn Tyr Ser Thr Lys Ala Thr Arg Thr Ala Pro
        275                 280                 285
Asn Gly Trp Lys Ala Ile Gln Glu His Cys Gln Lys Leu Glu Ala Arg
    290                 295                 300
His Ala Val His Ile Ala Ala Tyr Gly Glu Gly Asn Glu Arg Arg Leu
305                 310                 315                 320
Thr Gly Lys His Glu Thr Ser Ser Met Asn Asp Phe Ser Trp Gly Val
                325                 330                 335
Ala Asn Arg Gly Cys Ser Ile Arg Val Gly Arg Met Val Pro Val Glu
            340                 345                 350
Lys Cys Gly Tyr Tyr Glu Asp Arg Arg Pro Ala Ser Asn Leu Asp Pro
        355                 360                 365
Tyr Val Val Thr Lys Leu Ile Val Glu Thr Thr Val Leu Leu
    370                 375                 380

<210>  17
<211>  356
<212>  PRT
<213>  Arabidopsis thaliana

<400>  17
Met Ser Leu Leu Ala Asp Leu Val Asn Leu Asp Ile Ser Asp Asn Ser
1               5                   10                  15
Glu Lys Ile Ile Ala Glu Tyr Ile Trp Val Gly Gly Ser Gly Met Asp
            20                  25                  30
Met Arg Ser Lys Ala Arg Thr Leu Pro Gly Pro Val Thr Asp Pro Ser
        35                  40                  45
Lys Leu Pro Lys Trp Asn Tyr Asp Gly Ser Ser Thr Gly Gln Ala Pro
    50                  55                  60
Gly Gln Asp Ser Glu Val Ile Leu Tyr Pro Gln Ala Ile Phe Lys Asp
65                  70                  75                  80
Pro Phe Arg Arg Gly Asn Asn Ile Leu Val Met Cys Asp Ala Tyr Thr
                85                  90                  95
Pro Ala Gly Glu Pro Ile Pro Thr Asn Lys Arg His Ala Ala Ala Glu
            100                 105                 110
Ile Phe Ala Asn Pro Asp Val Ile Ala Glu Val Pro Trp Tyr Gly Ile
        115                 120                 125
Glu Gln Glu Tyr Thr Leu Leu Gln Lys Asp Val Asn Trp Pro Leu Gly
    130                 135                 140
Trp Pro Ile Gly Gly Phe Pro Gly Pro Gln Gly Pro Tyr Tyr Cys Ser
145                 150                 155                 160
Ile Gly Ala Asp Lys Ser Phe Gly Arg Asp Ile Val Asp Ala His Tyr
                165                 170                 175
Lys Ala Ser Leu Tyr Ala Gly Ile Asn Ile Ser Gly Ile Asn Gly Glu
            180                 185                 190
Val Met Pro Gly Gln Trp Glu Phe Gln Val Gly Pro Ser Val Gly Ile
        195                 200                 205
Ser Ala Ala Asp Glu Ile Trp Ile Ala Arg Tyr Ile Leu Glu Arg Ile
    210                 215                 220
Thr Glu Ile Ala Gly Val Val Val Ser Phe Asp Pro Lys Pro Ile Pro
225                 230                 235                 240
Gly Asp Trp Asn Gly Ala Gly Ala His Thr Asn Tyr Ser Thr Lys Ser
                245                 250                 255
Met Arg Glu Glu Gly Gly Tyr Glu Ile Ile Lys Lys Ala Ile Glu Lys
            260                 265                 270
```

```
Leu Gly Leu Arg His Lys Glu His Ile Ser Ala Tyr Gly Glu Gly Asn
        275                 280                 285
Glu Arg Arg Leu Thr Gly His His Glu Thr Ala Asp Ile Asn Thr Phe
    290                 295                 300
Leu Trp Gly Val Ala Asn Arg Gly Ala Ser Ile Arg Val Gly Arg Asp
305                 310                 315                 320
Thr Glu Lys Glu Gly Lys Gly Tyr Phe Glu Asp Arg Arg Pro Ala Ser
                325                 330                 335
Asn Met Asp Pro Tyr Val Val Thr Ser Met Ile Ala Glu Thr Thr Leu
            340                 345                 350
Leu Trp Asn Pro
        355

<210>  18
<211>  430
<212>  PRT
<213>  Arabidopsis thaliana

<400>  18
Met Ala Gln Ile Leu Ala Ala Ser Pro Thr Cys Gln Met Arg Val Pro
1               5                   10                  15
Lys His Ser Ser Val Ile Ala Ser Ser Ser Lys Leu Trp Ser Ser Val
            20                  25                  30
Val Leu Lys Gln Lys Lys Gln Ser Asn Asn Lys Val Arg Gly Phe Arg
        35                  40                  45
Val Leu Ala Leu Gln Ser Asp Asn Ser Thr Val Asn Arg Val Glu Thr
    50                  55                  60
Leu Leu Asn Leu Asp Thr Lys Pro Tyr Ser Asp Arg Ile Ile Ala Glu
65                  70                  75                  80
Tyr Ile Trp Ile Gly Gly Ser Gly Ile Asp Leu Arg Ser Lys Ser Arg
                85                  90                  95
Thr Ile Glu Lys Pro Val Glu Asp Pro Ser Glu Leu Pro Lys Trp Asn
            100                 105                 110
Tyr Asp Gly Ser Ser Thr Gly Gln Ala Pro Gly Glu Asp Ser Glu Val
        115                 120                 125
Ile Leu Tyr Pro Gln Ala Ile Phe Arg Asp Pro Phe Arg Gly Gly Asn
    130                 135                 140
Asn Ile Leu Val Ile Cys Asp Thr Trp Thr Pro Ala Gly Glu Pro Ile
145                 150                 155                 160
Pro Thr Asn Lys Arg Ala Lys Ala Ala Glu Ile Phe Ser Asn Lys Lys
                165                 170                 175
Val Ser Gly Glu Val Pro Trp Phe Gly Ile Glu Gln Glu Tyr Thr Leu
            180                 185                 190
Leu Gln Gln Asn Val Lys Trp Pro Leu Gly Trp Pro Val Gly Ala Phe
        195                 200                 205
Pro Gly Pro Gln Gly Pro Tyr Tyr Cys Gly Val Gly Ala Asp Lys Ile
    210                 215                 220
Trp Gly Arg Asp Ile Ser Asp Ala His Tyr Lys Ala Cys Leu Tyr Ala
225                 230                 235                 240
Gly Ile Asn Ile Ser Gly Thr Asn Gly Glu Val Met Pro Gly Gln Trp
                245                 250                 255
Glu Phe Gln Val Gly Pro Ser Val Gly Ile Asp Ala Gly Asp His Val
            260                 265                 270
Trp Cys Ala Arg Tyr Leu Leu Glu Arg Ile Thr Glu Gln Ala Gly Val
        275                 280                 285
Val Leu Thr Leu Asp Pro Lys Pro Ile Glu Gly Asp Trp Asn Gly Ala
    290                 295                 300
Gly Cys His Thr Asn Tyr Ser Thr Lys Ser Met Arg Glu Glu Gly Gly
305                 310                 315                 320
Phe Glu Val Ile Lys Lys Ala Ile Leu Asn Leu Ser Leu Arg His Lys
                325                 330                 335
Glu His Ile Ser Ala Tyr Gly Glu Gly Asn Glu Arg Arg Leu Thr Gly
            340                 345                 350
```

```
Lys His Glu Thr Ala Ser Ile Asp Gln Phe Ser Trp Gly Val Ala Asn
        355                 360                 365
Arg Gly Cys Ser Ile Arg Val Gly Arg Asp Thr Glu Ala Lys Gly Lys
    370                 375                 380
Gly Tyr Leu Glu Asp Arg Arg Pro Ala Ser Asn Met Asp Pro Tyr Ile
385                 390                 395                 400
Val Thr Ser Leu Leu Ala Glu Thr Thr Leu Leu Trp Glu Pro Thr Leu
                405                 410                 415
Glu Ala Glu Ala Leu Ala Ala Gln Lys Leu Ser Leu Asn Val
            420                 425                 430

<210>  19
<211>  356
<212>  PRT
<213>  Brassica napus

<400>  19
Met Ser Leu Leu Thr Asp Leu Val Asn Leu Asp Leu Ser Asp Asn Thr
1               5                   10                  15
Glu Lys Ile Ile Ala Glu Tyr Ile Trp Val Gly Gly Ser Gly Met Asp
            20                  25                  30
Met Arg Ser Lys Ala Arg Thr Leu Pro Gly Pro Val Thr Asp Pro Ser
        35                  40                  45
Lys Leu Pro Lys Trp Asn Tyr Asp Gly Ser Ser Thr Gly Gln Ala Pro
    50                  55                  60
Gly Glu Asp Ser Glu Val Ile Leu Tyr Pro Gln Ala Ile Phe Lys Asp
65                  70                  75                  80
Pro Phe Arg Arg Gly Asn Asn Ile Leu Val Met Cys Asp Thr Tyr Thr
                85                  90                  95
Pro Ala Gly Glu Pro Ile Pro Thr Asn Lys Arg His Ala Ala Ala Gln
            100                 105                 110
Ile Phe Ser Asn Pro Asp Val Val Ala Glu Val Pro Trp Tyr Gly Ile
        115                 120                 125
Glu Gln Glu Tyr Thr Leu Leu Gln Lys Asp Val Asn Trp Pro Val Gly
    130                 135                 140
Trp Pro Ile Gly Gly Phe Pro Gly Pro Gln Gly Pro Tyr Tyr Cys Ser
145                 150                 155                 160
Val Gly Ala Asp Lys Ser Phe Gly Arg Asp Ile Val Asp Ala His Tyr
                165                 170                 175
Lys Ala Cys Leu Tyr Ala Gly Ile Asn Ile Ser Gly Ile Asn Gly Glu
            180                 185                 190
Val Met Pro Gly Gln Trp Glu Phe Gln Val Gly Pro Ser Val Gly Ile
        195                 200                 205
Ser Ala Ala Asp Glu Val Trp Ile Ala Arg Tyr Ile Leu Glu Arg Ile
    210                 215                 220
Thr Glu Ile Ala Gly Val Val Val Ser Phe Asp Pro Lys Pro Ile Pro
225                 230                 235                 240
Gly Asp Trp Asn Gly Ala Gly Ala His Thr Asn Tyr Ser Thr Lys Ser
                245                 250                 255
Met Arg Glu Glu Gly Gly Tyr Glu Ile Ile Lys Lys Ala Ile Asp Lys
            260                 265                 270
Leu Gly Leu Arg His Lys Glu His Ile Ser Ala Tyr Gly Glu Gly Asn
        275                 280                 285
Glu Arg Arg Leu Thr Gly His His Glu Thr Ala Asp Ile Asn Thr Phe
    290                 295                 300
Lys Trp Gly Val Ala Asn Arg Gly Ala Ser Ile Arg Val Gly Arg Asp
305                 310                 315                 320
Thr Glu Lys Glu Gly Lys Gly Tyr Phe Glu Asp Arg Arg Pro Ala Ser
                325                 330                 335
Asn Met Asp Pro Tyr Thr Val Thr Ser Met Ile Ala Glu Thr Thr Leu
            340                 345                 350
Leu Trp Asn Pro
        355
```

<210> 20
<211> 428
<212> PRT
<213> Brassica napus

<400> 20
Met Ala Gln Ile Leu Ala Ala Ser Pro Thr Cys Gln Met Arg Leu Thr
1 5 10 15
Lys Pro Ser Ser Ile Ala Ser Ser Lys Leu Trp Asn Ser Val Val Leu
20 25 30
Lys Gln Lys Lys Gln Ser Ser Ser Lys Val Arg Ser Phe Lys Val Met
35 40 45
Ala Leu Gln Ser Asp Asn Ser Thr Ile Asn Arg Val Glu Ser Leu Leu
50 55 60
Asn Leu Asp Thr Lys Pro Phe Thr Asp Arg Ile Ile Ala Glu Tyr Ile
65 70 75 80
Trp Ile Gly Gly Ser Gly Ile Asp Leu Arg Ser Lys Ser Arg Thr Leu
85 90 95
Glu Lys Pro Val Glu Asp Pro Ser Glu Leu Pro Lys Trp Asn Tyr Asp
100 105 110
Gly Ser Ser Thr Gly Gln Ala Pro Gly Glu Asp Ser Glu Val Ile Leu
115 120 125
Tyr Pro Gln Ala Ile Phe Arg Asp Pro Phe Arg Gly Gly Asn Asn Ile
130 135 140
Leu Val Ile Cys Asp Thr Tyr Thr Pro Ala Gly Glu Pro Ile Pro Thr
145 150 155 160
Asn Lys Arg Ala Arg Ala Ala Glu Ile Phe Ser Asn Lys Lys Val Asn
165 170 175
Glu Glu Ile Pro Trp Phe Gly Ile Glu Gln Glu Tyr Thr Leu Leu Gln
180 185 190
Pro Asn Val Asn Trp Pro Leu Gly Trp Pro Val Gly Ala Tyr Pro Gly
195 200 205
Pro Gln Gly Pro Tyr Tyr Cys Gly Val Gly Ala Glu Lys Ser Trp Gly
210 215 220
Arg Asp Ile Ser Asp Ala His Tyr Lys Ala Cys Leu Tyr Ala Gly Ile
225 230 235 240
Asn Ile Ser Gly Thr Asn Gly Glu Val Met Pro Gly Gln Trp Glu Phe
245 250 255
Gln Val Gly Pro Ser Val Gly Ile Glu Ala Gly Asp His Val Trp Cys
260 265 270
Ala Arg Tyr Leu Leu Glu Arg Ile Thr Glu Gln Ala Gly Val Val Leu
275 280 285
Thr Leu Asp Pro Lys Pro Ile Glu Gly Asp Trp Asn Gly Ala Gly Cys
290 295 300
His Thr Asn Tyr Ser Thr Lys Ser Met Arg Glu Asp Gly Gly Phe Glu
305 310 315 320
Val Ile Lys Lys Ala Ile Leu Asn Leu Ser Leu Arg His Met Glu His
325 330 335
Ile Ser Ala Tyr Gly Glu Gly Asn Glu Arg Arg Leu Thr Gly Lys His
340 345 350
Glu Thr Ala Ser Ile Asp Gln Phe Ser Trp Gly Val Ala Asn Arg Gly
355 360 365
Cys Ser Ile Arg Val Gly Arg Asp Thr Glu Lys Lys Gly Lys Gly Tyr
370 375 380
Leu Glu Asp Arg Arg Pro Ala Ser Asn Met Asp Pro Tyr Ile Val Thr
385 390 395 400
Ser Leu Leu Ala Glu Thr Thr Leu Leu Trp Glu Pro Thr Leu Glu Ala
405 410 415
Glu Ala Leu Ala Ala Gln Lys Leu Ser Leu Lys Val
420 425

<210> 21

```
<211>  364
<212>  PRT
<213>  Hordeum vulgare

<400>  21
Met Ala Ala Ala Thr Thr Asn Val Ser Tyr Thr Thr Asn Leu Leu Lys
1               5                   10                  15
Tyr Met Gly Leu Asp Gln Lys Gly Ser Ala Met Ala Glu Tyr Ile Trp
            20                  25                  30
Ile Asp Ala Val Gly Gly Val Arg Ser Lys Ser Lys Thr Leu Thr Ser
        35                  40                  45
Ile Pro Pro Ser Gly Glu Phe Thr Val Asp Asp Leu Pro Glu Trp Asn
    50                  55                  60
Phe Asp Gly Ser Ser Thr Gly Gln Ala Pro Gly Asp Asn Ser Asp Val
65                  70                  75                  80
Tyr Leu Arg Pro Val Ala Val Phe Pro Asp Pro Phe Arg Gly Ala Pro
                85                  90                  95
Asn Ile Leu Val Ile Thr Glu Cys Trp Asp Pro Asp Gly Thr Pro Asn
            100                 105                 110
Lys Tyr Asn His Arg His Glu Ala Ala Lys Leu Met Glu Ala His Lys
        115                 120                 125
Ala Gln Lys Pro Trp Phe Gly Leu Glu Gln Glu Tyr Thr Leu Leu Asp
    130                 135                 140
Met His Asp Arg Pro Tyr Gly Trp Pro Ala Gly Gly Phe Pro Gly Pro
145                 150                 155                 160
Gln Gly Pro Tyr Tyr Cys Gly Val Gly Ser Gly Lys Val Tyr Cys Arg
                165                 170                 175
Asp Ile Val Glu Ala His Tyr Lys Ala Cys Leu Phe Ala Gly Val Lys
            180                 185                 190
Ile Ser Gly Thr Asn Ala Glu Val Met Pro Ala Gln Trp Glu Phe Gln
        195                 200                 205
Val Gly Pro Cys Glu Gly Ile Glu Leu Gly Asp Gln Leu Trp Leu Ala
    210                 215                 220
Arg Phe Leu Leu His Arg Ile Ala Glu Glu Phe Gly Ala Lys Ile Ser
225                 230                 235                 240
Phe His Pro Lys Pro Ile Pro Gly Asp Trp Asn Gly Ala Gly Leu His
                245                 250                 255
Ser Asn Phe Ser Ser Glu Glu Met Arg Lys Pro Gly Gly Met Lys Ala
            260                 265                 270
Ile Glu Ala Ala Met Lys Lys Leu Glu Ala Arg His Lys Glu His Ile
        275                 280                 285
Ala Val Tyr Gly Glu Asp Asn Thr Met Arg Leu Thr Gly Arg His Glu
    290                 295                 300
Thr Gly Asn Ile Asp Ser Phe Thr Tyr Gly Val Ala Asn Arg Gly Thr
305                 310                 315                 320
Ser Ile Arg Ile Pro Arg Glu Val Ser Gln Lys Gly Phe Gly Tyr Phe
                325                 330                 335
Glu Asp Arg Arg Pro Ala Ser Asn Ala Asp Pro Tyr Gln Ile Thr Gly
            340                 345                 350
Ile Met Val Glu Thr Ile Phe Gly Gly Leu Asp Lys
        355                 360

<210>  22
<211>  356
<212>  PRT
<213>  Oryza sativa

<400>  22
Met Ala Ser Leu Thr Asp Leu Val Asn Leu Asn Leu Ser Asp Thr Thr
1               5                   10                  15
Glu Lys Ile Ile Ala Glu Tyr Ile Trp Ile Gly Gly Ser Gly Met Asp
            20                  25                  30
Leu Arg Ser Lys Ala Arg Thr Leu Ser Gly Pro Val Thr Asp Pro Ser
```

```
        35                  40                  45
Lys Leu Pro Lys Trp Asn Tyr Asp Gly Ser Ser Thr Gly Gln Ala Pro
    50                  55                  60
Gly Glu Asp Ser Glu Val Ile Leu Tyr Pro Gln Ala Ile Phe Lys Asp
65                  70                  75                  80
Pro Phe Arg Lys Gly Asn Asn Ile Leu Val Met Cys Asp Cys Tyr Thr
                85                  90                  95
Pro Ala Gly Glu Pro Ile Pro Thr Asn Lys Arg His Asn Ala Ala Lys
            100                 105                 110
Ile Phe Ser Ser Pro Glu Val Ala Ser Glu Glu Pro Trp Tyr Gly Ile
        115                 120                 125
Glu Gln Glu Tyr Thr Leu Leu Gln Lys Asp Ile Asn Trp Pro Leu Gly
    130                 135                 140
Trp Pro Val Gly Gly Phe Pro Gly Pro Gln Gly Pro Tyr Tyr Cys Gly
145                 150                 155                 160
Ile Gly Ala Asp Lys Ser Phe Gly Arg Asp Ile Val Asp Ser His Tyr
                165                 170                 175
Lys Ala Cys Leu Tyr Ala Gly Ile Asn Ile Ser Gly Ile Asn Gly Glu
            180                 185                 190
Val Met Pro Gly Gln Trp Glu Phe Gln Val Gly Pro Ser Val Gly Ile
        195                 200                 205
Ser Ala Gly Asp Gln Val Trp Val Ala Arg Tyr Ile Leu Glu Arg Ile
    210                 215                 220
Thr Glu Ile Ala Gly Val Val Val Ser Phe Asp Pro Lys Pro Ile Pro
225                 230                 235                 240
Gly Asp Trp Asn Gly Ala Gly Ala His Thr Asn Tyr Ser Thr Lys Ser
                245                 250                 255
Met Arg Asn Asp Gly Gly Tyr Glu Ile Ile Lys Ser Ala Ile Glu Lys
            260                 265                 270
Leu Lys Leu Arg His Lys Glu His Ile Ser Ala Tyr Gly Glu Gly Asn
        275                 280                 285
Glu Arg Arg Leu Thr Gly Arg His Glu Thr Ala Asp Ile Asn Thr Phe
    290                 295                 300
Ser Trp Gly Val Ala Asn Arg Gly Ala Ser Val Arg Val Gly Arg Glu
305                 310                 315                 320
Thr Glu Gln Asn Gly Lys Gly Tyr Phe Glu Asp Arg Arg Pro Ala Ser
                325                 330                 335
Asn Met Asp Pro Tyr Ile Val Thr Ser Met Ile Ala Glu Thr Thr Ile
            340                 345                 350
Ile Trp Lys Pro
        355
```

<210> 23
<211> 428
<212> PRT
<213> Oryza sativa

<400> 23

```
Met Ala Gln Ala Val Val Pro Ala Met Gln Cys Gln Val Gly Ala Val
1               5                   10                  15
Arg Ala Arg Pro Ala Ala Ala Ala Ala Ala Ala Gly Gly Arg Val Trp
            20                  25                  30
Gly Val Arg Arg Thr Gly Arg Gly Thr Ser Gly Phe Arg Val Met Ala
        35                  40                  45
Val Ser Thr Glu Thr Thr Gly Val Val Thr Arg Met Glu Gln Leu Leu
    50                  55                  60
Asn Met Asp Thr Thr Pro Phe Thr Asp Lys Ile Ile Ala Glu Tyr Ile
65                  70                  75                  80
Trp Val Gly Gly Thr Gly Ile Asp Leu Arg Ser Lys Ser Arg Thr Ile
                85                  90                  95
Ser Lys Pro Val Glu Asp Pro Ser Glu Leu Pro Lys Trp Asn Tyr Asp
            100                 105                 110
Gly Ser Ser Thr Gly Gln Ala Pro Gly Glu Asp Ser Glu Val Ile Leu
```

```
        115                 120                 125
Tyr Pro Gln Ala Ile Phe Lys Asp Pro Phe Arg Gly Gly Asn Asn Ile
    130                 135                 140
Leu Val Met Cys Asp Thr Tyr Thr Pro Ala Gly Glu Pro Ile Pro Thr
145                 150                 155                 160
Asn Lys Arg Asn Arg Ala Ala Gln Val Phe Ser Asp Pro Lys Val Val
                165                 170                 175
Ser Gln Val Pro Trp Phe Gly Ile Glu Gln Glu Tyr Thr Leu Leu Gln
            180                 185                 190
Arg Asp Val Asn Trp Pro Leu Gly Trp Pro Val Gly Gly Tyr Pro Gly
        195                 200                 205
Pro Gln Gly Pro Tyr Tyr Cys Ala Val Gly Ser Asp Lys Ser Phe Gly
    210                 215                 220
Arg Asp Ile Ser Asp Ala His Tyr Lys Ala Cys Leu Tyr Ala Gly Ile
225                 230                 235                 240
Asn Ile Ser Gly Thr Asn Gly Glu Val Met Pro Gly Gln Trp Glu Tyr
                245                 250                 255
Gln Val Gly Pro Ser Val Gly Ile Glu Ala Gly Asp His Ile Trp Ile
            260                 265                 270
Ser Arg Tyr Ile Leu Glu Arg Ile Thr Glu Gln Ala Gly Val Val Leu
        275                 280                 285
Thr Leu Asp Pro Lys Pro Ile Gln Gly Asp Trp Asn Gly Ala Gly Cys
    290                 295                 300
His Thr Asn Tyr Ser Thr Lys Ser Met Arg Glu Asp Gly Gly Phe Glu
305                 310                 315                 320
Val Ile Lys Lys Ala Ile Leu Asn Leu Ser Leu Arg His Asp Leu His
                325                 330                 335
Ile Ser Ala Tyr Gly Glu Gly Asn Glu Arg Arg Leu Thr Gly Leu His
            340                 345                 350
Glu Thr Ala Ser Ile Asp Asn Phe Ser Trp Gly Val Ala Asn Arg Gly
        355                 360                 365
Cys Ser Ile Arg Val Gly Arg Asp Thr Glu Ala Lys Gly Lys Gly Tyr
    370                 375                 380
Leu Glu Asp Arg Arg Pro Ala Ser Asn Met Asp Pro Tyr Val Val Thr
385                 390                 395                 400
Ala Leu Leu Ala Glu Thr Thr Ile Leu Trp Glu Pro Thr Leu Glu Ala
                405                 410                 415
Glu Val Leu Ala Ala Lys Lys Leu Ala Leu Lys Val
            420                 425
```

<210> 24
<211> 346
<212> PRT
<213> Physcomitrella patens

<400> 24

```
Met Ala Leu Ala Gln Lys Ala Glu Tyr Ile Trp Met Asp Gly Gln Glu
1               5                   10                  15
Gly Gln Lys Gly Ile Arg Phe Asn Glu Met Arg Ser Lys Thr Lys Val
            20                  25                  30
Ile Gln Glu Pro Ile Lys Ala Gly Ser Leu Asp Phe Pro Lys Trp Ser
        35                  40                  45
Phe Asp Gly Ser Ser Thr Gly Gln Ala Glu Gly Arg Phe Ser Asp Cys
    50                  55                  60
Ile Leu Asn Pro Val Phe Ser Cys Leu Asp Pro Ile Arg Gly Asp Asn
65                  70                  75                  80
His Val Leu Val Leu Cys Glu Val Leu Asn Pro Asp Ser Thr Pro His
                85                  90                  95
Glu Thr Asn Thr Arg Arg Lys Ile Glu Glu Leu Leu Thr Pro Asp Val
            100                 105                 110
Leu Ala Glu Glu Thr Leu Phe Gly Phe Glu Gln Glu Tyr Thr Met Phe
        115                 120                 125
Asn Lys Ala Gly Lys Val Tyr Gly Trp Pro Glu Gly Gly Phe Pro His
```

```
        130                 135                 140
    Pro Gln Gly Pro Phe Tyr Cys Gly Val Gly Leu Glu Ala Val Tyr Gly
    145                 150                 155                 160
    Arg Pro Leu Val Glu Ala His Met Asp Ala Cys Ile Lys Ala Gly Leu
                    165                 170                 175
    Lys Ile Ser Gly Ile Asn Ala Glu Val Met Pro Gly Gln Trp Glu Phe
                180                 185                 190
    Gln Ile Gly Pro Ala Gly Pro Leu Glu Val Gly Asp His Val Met Ile
            195                 200                 205
    Ala Arg Trp Leu Leu His Arg Leu Gly Glu Asp Phe Gly Ile Thr Cys
        210                 215                 220
    Thr Phe Glu Pro Lys Pro Met Glu Gly Asp Trp Asn Gly Ala Gly Ala
    225                 230                 235                 240
    His Thr Asn Tyr Ser Thr Lys Ser Met Arg Val Asp Gly Gly Ile Lys
                    245                 250                 255
    Ala Ile His Ala Ala Ile Glu Lys Leu Ser Lys Lys His Val Glu His
                260                 265                 270
    Ile Ser Ser Tyr Gly Leu Gly Asn Glu Arg Arg Leu Thr Gly Lys His
            275                 280                 285
    Glu Thr Ala Asn Ile Asn Thr Phe Lys Ser Gly Val Ala Asp Arg Gly
        290                 295                 300
    Ala Ser Ile Arg Ile Pro Leu Gly Val Ser Leu Asp Gly Lys Gly Tyr
    305                 310                 315                 320
    Leu Glu Asp Arg Arg Pro Ala Ala Asn Val Asp Pro Tyr Val Val Ala
                    325                 330                 335
    Arg Met Leu Ile Gln Thr Thr Leu Lys Asn
                340                 345

    <210>  25
    <211>  346
    <212>  PRT
    <213>  Physcomitrella patens

    <400>  25
    Met Ala Leu Ala Gln Lys Ala Glu Tyr Ile Trp Met Asp Gly Gln Glu
    1               5                   10                  15
    Gly Gln Lys Gly Ile Arg Phe Asn Glu Met Arg Ser Lys Thr Lys Val
                20                  25                  30
    Ile Gln Glu Pro Ile Lys Ala Gly Ser Leu Asp Phe Pro Lys Trp Ser
            35                  40                  45
    Phe Asp Gly Ser Ser Thr Gly Gln Ala Glu Gly Arg Phe Ser Asp Cys
        50                  55                  60
    Ile Leu Asn Pro Val Phe Ser Cys Pro Asp Pro Ile Arg Gly Asp Asn
    65                  70                  75                  80
    His Val Leu Val Leu Cys Glu Val Leu Asn Pro Asp Ser Thr Pro His
                    85                  90                  95
    Glu Thr Asn Thr Arg Arg Lys Ile Glu Glu Leu Leu Thr Pro Asp Val
                100                 105                 110
    Leu Ala Glu Glu Thr Leu Phe Gly Phe Glu Gln Glu Tyr Thr Met Phe
            115                 120                 125
    Asn Lys Ala Ala Lys Val Tyr Gly Trp Pro Glu Gly Gly Phe Pro His
        130                 135                 140
    Pro Gln Gly Pro Phe Tyr Cys Gly Val Gly Leu Glu Ala Val Tyr Gly
    145                 150                 155                 160
    Arg Pro Leu Val Glu Ala His Met Asp Ala Cys Ile Lys Ala Gly Leu
                    165                 170                 175
    Lys Ile Ser Gly Ile Asn Ala Glu Val Met Pro Gly Gln Trp Glu Phe
                180                 185                 190
    Gln Ile Gly Pro Ala Gly Pro Leu Glu Val Gly Asp His Val Met Val
            195                 200                 205
    Ala Arg Trp Leu Leu His Arg Leu Gly Glu Asp Phe Gly Ile Thr Cys
        210                 215                 220
    Thr Phe Glu Pro Lys Pro Met Glu Gly Asp Trp Asn Gly Ala Gly Ala
```

```
225                 230                 235                 240
His Thr Asn Tyr Ser Thr Lys Ser Met Arg Val Asp Gly Gly Ile Lys
                245                 250                 255
Ala Ile His Ala Ala Ile Glu Lys Leu Ser Lys Lys His Ala Glu His
            260                 265                 270
Ile Ser Ser Tyr Gly Leu Gly Asn Glu Arg Arg Leu Thr Gly Lys His
        275                 280                 285
Glu Thr Ala Asn Ile Asn Thr Phe Lys Ser Gly Val Ala Asp Arg Gly
    290                 295                 300
Ala Ser Ile Arg Ile Pro Leu Gly Val Ser Leu Glu Gly Lys Gly Tyr
305                 310                 315                 320
Leu Glu Asp Arg Arg Pro Ala Ala Asn Val Asp Pro Tyr Val Val Ala
                325                 330                 335
Arg Met Leu Ile Gln Thr Thr Leu Lys Asn
            340                 345

<210>  26
<211>  371
<212>  PRT
<213>  Pinus taeda

<400>  26
Met Ala Thr Pro Ile Thr Ser Arg Thr Glu Thr Leu Gln Lys Tyr Leu
1               5                   10                  15
Lys Leu Asp Gln Lys Gly Met Ile Met Ala Glu Tyr Val Trp Val Asp
            20                  25                  30
Ala Asp Gly Gly Thr Arg Ser Lys Ser Arg Thr Leu Pro Glu Lys Glu
        35                  40                  45
Tyr Lys Pro Glu Asp Leu Pro Val Trp Asn Phe Asp Gly Ser Ser Thr
    50                  55                  60
Asn Gln Ala Pro Gly Asp Asn Ser Asp Val Tyr Leu Arg Pro Cys Ala
65                  70                  75                  80
Val Tyr Pro Asp Pro Phe Arg Gly Ser Pro Asn Ile Ile Val Leu Ala
                85                  90                  95
Glu Cys Trp Asn Ala Asp Gly Thr Pro Asn Lys Tyr Asn Phe Arg His
            100                 105                 110
Asp Cys Val Lys Val Met Asp Thr Tyr Ala Asp Asp Glu Pro Trp Phe
        115                 120                 125
Gly Leu Glu Gln Glu Tyr Thr Leu Leu Gly Ser Asp Asn Arg Pro Tyr
    130                 135                 140
Gly Trp Pro Ala Gly Gly Phe Pro Ala Pro Gln Gly Glu Tyr Tyr Cys
145                 150                 155                 160
Gly Val Gly Thr Gly Lys Val Val Gln Arg Asp Ile Val Glu Ala His
                165                 170                 175
Tyr Lys Ala Cys Leu Tyr Ala Gly Ile Gln Ile Ser Gly Thr Asn Ala
            180                 185                 190
Glu Val Met Pro Ala Gln Trp Glu Tyr Gln Val Gly Pro Cys Thr Gly
        195                 200                 205
Ile Ala Met Gly Asp Gln Leu Trp Ile Ser Arg Phe Phe Leu His Arg
    210                 215                 220
Val Ala Glu Glu Phe Gly Ala Lys Val Ser Leu His Pro Lys Pro Ile
225                 230                 235                 240
Ala Gly Asp Trp Asn Gly Ala Leu Ser Phe Pro Gly Leu Cys Phe Ile
                245                 250                 255
Ser Val Ile Leu Ile Ser Leu Gln Gly Leu His Ser Asn Phe Ser Thr
            260                 265                 270
Lys Ala Met Arg Glu Glu Gly Gly Met Lys Val Ile Glu Glu Ala Leu
        275                 280                 285
Lys Lys Leu Glu Pro His His Val Glu Cys Ile Ala Glu Tyr Gly Glu
    290                 295                 300
Asp Asn Glu Leu Arg Leu Thr Gly Arg His Glu Thr Gly Ser Ile Asp
305                 310                 315                 320
Ser Phe Ser Trp Gly Val Ala Asn Arg Gly Thr Ser Ile Arg Val Pro
```

```
                325                 330                 335
Arg Glu Thr Ala Ala Lys Gly Tyr Gly Tyr Phe Glu Asp Arg Arg Pro
            340                 345                 350
Ala Ser Asn Ala Asp Pro Tyr Arg Val Thr Lys Val Leu Leu Gln Phe
        355                 360                 365
Ser Met Ala
    370

<210>  27
<211>  354
<212>  PRT
<213>  Pinus taeda

<400>  27
Met Ala Tyr Ala Tyr Arg Pro Glu Leu Leu Ala Pro Tyr Leu Ser Leu
1               5                   10                  15
Pro Gln Gly Glu Lys Val Gln Ala Glu Tyr Val Trp Val Asp Gly Asp
            20                  25                  30
Gly Gly Leu Arg Ser Lys Thr Cys Thr Val Asp Lys Lys Val Thr Asp
        35                  40                  45
Ile Gly Gln Leu Arg Val Trp Asp Phe Asp Gly Ser Ser Thr Asn Gln
    50                  55                  60
Ala Pro Gly Gly Asn Ser Asp Val Tyr Leu Arg Pro Ala Ala Ile Phe
65                  70                  75                  80
Lys Asp Pro Phe Arg Gly Gly Asp Asn Ile Leu Val Leu Ala Glu Cys
                85                  90                  95
Tyr Asn Asn Asp Gly Thr Pro Asn Lys Thr Asn His Arg His His Ala
            100                 105                 110
Ala Lys Val Met Glu Leu Ala Lys Asp Gln Lys Pro Trp Phe Gly Leu
        115                 120                 125
Glu Gln Glu Tyr Thr Leu Phe Asp Val Asp Gly Thr Pro Phe Gly Trp
    130                 135                 140
Pro Lys Gly Gly Phe Pro Gly Pro Gln Gly Pro Tyr Tyr Cys Gly Ala
145                 150                 155                 160
Gly Ala Gly Lys Val Tyr Ala Arg Asp Leu Ile Glu Ala His Tyr Arg
                165                 170                 175
Val Cys Leu Tyr Ala Gly Ile Lys Ile Ser Gly Val Asn Ala Glu Val
            180                 185                 190
Met Pro Ala Gln Trp Glu Phe Gln Val Gly Pro Cys Glu Gly Ile Glu
        195                 200                 205
Met Gly Asp His Leu Trp Met Ala Arg Tyr Leu Leu Ile Arg Leu Ala
    210                 215                 220
Glu Gln Trp Gly Ile Lys Val Ser Phe His Pro Lys Pro Leu Ala Gly
225                 230                 235                 240
Asp Trp Asn Gly Ser Gly Cys His Thr Asn Tyr Ser Thr Ala Pro Met
                245                 250                 255
Arg Glu Glu Gly Gly Met Lys His Ile Glu Ala Ala Ile Glu Lys Leu
            260                 265                 270
Ala Gln Lys His Asp Glu His Ile Ala Val Tyr Gly Asp Asp Asn Asp
        275                 280                 285
Met Arg Leu Thr Gly Arg His Glu Thr Gly His Ile Gly Thr Phe Ser
    290                 295                 300
Ser Gly Val Ala Asn Arg Gly Ala Ser Ile Arg Ile Pro Arg His Val
305                 310                 315                 320
Ala Ala Lys Gly Tyr Gly Tyr Leu Glu Asp Arg Arg Pro Ala Ser Asn
                325                 330                 335
Val Asp Pro Tyr Arg Val Thr Ser Ile Ile Val Glu Thr Thr Val Thr
            340                 345                 350
Asn Ala


<210>  28
<211>  416
```

<212> PRT
<213> Phaedactylum tricornutum

<400> 28

```
Met Lys Leu Asn Ile Ala Ala Ile Ala Leu Phe Ala Ala Ser Ala Ser
1               5                   10                  15
Ala Phe Ala Pro Arg Phe Ala Ser Pro Arg Ser His Ala Thr Val Leu
            20                  25                  30
Ser Ala Val Leu Glu Glu Arg Thr Gly Gln Ser Gln Leu Asp Pro Ala
        35                  40                  45
Val Ile Glu Arg Tyr Ala Ala Leu Pro Tyr Pro Asp Asp Thr Val Leu
    50                  55                  60
Ala Glu Tyr Val Trp Val Asp Ala Val Gly Asn Thr Arg Ser Lys Thr
65                  70                  75                  80
Arg Thr Leu Pro Ala Lys Lys Ala Ala Ser Val Glu Ala Leu Pro Lys
                85                  90                  95
Trp Asn Phe Asp Gly Ser Ser Thr Asp Gln Ala Pro Gly Asp Asp Ser
            100                 105                 110
Glu Val Ile Leu Arg Pro Cys Arg Ile Phe Lys Asp Pro Phe Arg Pro
        115                 120                 125
Arg Asn Asp Gly Leu Asp Asn Val Leu Val Met Cys Asp Cys Tyr Thr
    130                 135                 140
Pro Asn Gly Glu Ala Ile Pro Thr Asn His Arg Ala Lys Ala Met Glu
145                 150                 155                 160
Ser Phe Glu Ser Arg Glu Asp Glu Glu Ile Trp Phe Gly Leu Glu Gln
                165                 170                 175
Glu Phe Thr Leu Phe Asn Leu Asp Lys Arg Thr Pro Leu Gly Trp Pro
            180                 185                 190
Glu Gly Gly Met Pro Asn Arg Pro Gln Gly Pro Tyr Tyr Cys Ser Val
        195                 200                 205
Gly Pro Glu Asn Asn Phe Gly Arg His Ile Thr Glu Ser Met Tyr Arg
    210                 215                 220
Ala Cys Leu Tyr Ala Gly Ile Asn Ile Ser Gly Thr Asn Gly Glu Val
225                 230                 235                 240
Met Pro Gly Gln Gln Glu Tyr Gln Val Gly Pro Cys Val Gly Ile Asp
                245                 250                 255
Ala Gly Asp Gln Leu Met Met Ser Arg Tyr Ile Leu Gln Arg Val Cys
            260                 265                 270
Glu Asp Phe Gln Val Tyr Cys Thr Leu His Pro Lys Pro Ile Val Asp
        275                 280                 285
Gly Asp Trp Asn Gly Ala Gly Met His Thr Asn Val Ser Thr Lys Ser
    290                 295                 300
Met Arg Glu Glu Gly Gly Leu Glu Val Ile Lys Lys Ala Ile Tyr Lys
305                 310                 315                 320
Leu Gly Ala Lys His Leu Glu His Ile Ala Val Tyr Gly Glu Gly Asn
                325                 330                 335
Glu Leu Arg Leu Thr Gly Lys His Glu Thr Ala Ser Met Asp Lys Phe
            340                 345                 350
Cys Tyr Gly Val Ala Asn Arg Gly Ala Ser Ile Arg Ile Gly Arg Asp
        355                 360                 365
Thr Glu Ala Glu Gly Lys Gly Tyr Phe Glu Asp Arg Arg Pro Ser Ser
    370                 375                 380
Asn Ala Asp Pro Tyr Ile Val Thr Gly Lys Ile Met Asn Thr Ile Met
385                 390                 395                 400
Glu Asp Val Glu Val Pro Asp Ile Ala Pro Met Asp Lys Ala Val Ala
                405                 410                 415
```

<210> 29
<211> 423
<212> PRT
<213> Zea mays

<400> 29

Met Ala Gln Ala Val Val Pro Ala Met Gln Cys Arg Val Gly Val Lys
1 5 10 15
Ala Ala Ala Gly Arg Val Trp Ser Ala Gly Arg Thr Arg Thr Gly Arg
20 25 30
Gly Gly Ala Ser Pro Gly Phe Lys Val Met Ala Val Ser Thr Gly Ser
35 40 45
Thr Gly Val Val Pro Arg Leu Glu Gln Leu Leu Asn Met Asp Thr Thr
50 55 60
Pro Tyr Thr Asp Lys Val Ile Ala Glu Tyr Ile Trp Val Gly Gly Ser
65 70 75 80
Gly Ile Asp Ile Arg Ser Lys Ser Arg Thr Ile Ser Lys Pro Val Glu
85 90 95
Asp Pro Ser Glu Leu Pro Lys Trp Asn Tyr Asp Gly Ser Ser Thr Gly
100 105 110
Gln Ala Pro Gly Glu Asp Ser Glu Val Ile Leu Tyr Pro Gln Ala Ile
115 120 125
Phe Lys Asp Pro Phe Arg Gly Gly Asn Asn Val Leu Val Ile Cys Asp
130 135 140
Thr Tyr Thr Pro Gln Gly Glu Pro Leu Pro Thr Asn Lys Arg His Arg
145 150 155 160
Ala Ala Gln Ile Phe Ser Asp Pro Lys Val Ala Glu Gln Val Pro Trp
165 170 175
Phe Gly Ile Glu Gln Glu Tyr Thr Leu Leu Gln Lys Asp Val Asn Trp
180 185 190
Pro Leu Gly Trp Pro Val Gly Gly Phe Pro Gly Pro Gln Gly Pro Tyr
195 200 205
Tyr Cys Ala Val Gly Ala Asp Lys Ser Phe Gly Arg Asp Ile Ser Asp
210 215 220
Ala His Tyr Lys Ala Cys Leu Tyr Ala Gly Ile Asn Ile Ser Gly Thr
225 230 235 240
Asn Gly Glu Val Met Pro Gly Gln Trp Glu Tyr Gln Val Gly Pro Ser
245 250 255
Val Gly Ile Glu Ala Gly Asp His Ile Trp Ile Ser Arg Tyr Ile Leu
260 265 270
Glu Arg Ile Thr Glu Gln Ala Gly Val Val Leu Thr Leu Asp Pro Lys
275 280 285
Pro Ile Gln Gly Asp Trp Asn Gly Ala Gly Cys His Thr Asn Tyr Ser
290 295 300
Thr Lys Thr Met Arg Glu Asp Gly Gly Phe Glu Glu Ile Lys Arg Ala
305 310 315 320
Ile Leu Asn Leu Ser Leu Arg His Asp Leu His Ile Ser Ala Tyr Gly
325 330 335
Glu Gly Asn Glu Arg Arg Leu Thr Gly Lys His Glu Thr Ala Ser Ile
340 345 350
Gly Thr Phe Ser Trp Gly Val Ala Asn Arg Gly Cys Ser Ile Arg Val
355 360 365
Gly Arg Asp Thr Glu Ala Lys Gly Lys Gly Tyr Leu Glu Asp Arg Arg
370 375 380
Pro Ala Ser Asn Met Asp Pro Tyr Ile Val Thr Gly Leu Leu Ala Glu
385 390 395 400
Thr Thr Ile Leu Trp Gln Pro Ser Leu Glu Ala Glu Ala Leu Ala Ala
405 410 415
Lys Lys Leu Ala Leu Lys Val
420

<210> 30
<211> 356
<212> PRT
<213> Zea mays

<400> 30
Met Ala Cys Leu Thr Asp Leu Val Asn Leu Asn Leu Ser Asp Thr Thr
1 5 10 15

```
Glu Lys Ile Ile Ala Glu Tyr Ile Trp Ile Gly Gly Ser Gly Met Asp
            20                  25                  30
Leu Arg Ser Lys Ala Arg Thr Leu Pro Gly Pro Val Thr Asp Pro Ser
        35                  40                  45
Lys Leu Pro Lys Trp Asn Tyr Asp Gly Ser Ser Thr Gly Gln Ala Pro
    50                  55                  60
Gly Glu Asp Ser Glu Val Ile Leu Tyr Pro Gln Ala Ile Phe Lys Asp
65                  70                  75                  80
Pro Phe Arg Arg Gly Asn Asn Ile Leu Val Met Cys Asp Cys Tyr Thr
                85                  90                  95
Pro Ala Gly Glu Pro Ile Pro Thr Asn Lys Arg Tyr Ser Ala Ala Lys
            100                 105                 110
Ile Phe Ser Ser Leu Glu Val Ala Ala Glu Glu Pro Trp Tyr Gly Ile
        115                 120                 125
Glu Gln Glu Tyr Thr Leu Leu Gln Lys Asp Thr Asn Trp Pro Leu Gly
    130                 135                 140
Trp Pro Ile Gly Gly Phe Pro Gly Pro Gln Gly Pro Tyr Tyr Cys Gly
145                 150                 155                 160
Ile Gly Ala Glu Lys Ser Phe Gly Arg Asp Ile Val Asp Ala His Tyr
                165                 170                 175
Lys Ala Cys Leu Tyr Ala Gly Ile Asn Ile Ser Gly Ile Asn Gly Glu
            180                 185                 190
Val Met Pro Gly Gln Trp Glu Phe Gln Val Gly Pro Ser Val Gly Ile
        195                 200                 205
Ser Ser Gly Asp Gln Val Trp Val Ala Arg Tyr Ile Leu Glu Arg Ile
    210                 215                 220
Thr Glu Ile Ala Gly Val Val Val Thr Phe Asp Pro Lys Pro Ile Pro
225                 230                 235                 240
Gly Asp Trp Asn Gly Ala Gly Ala His Thr Asn Tyr Ser Thr Glu Ser
                245                 250                 255
Met Arg Lys Glu Gly Gly Tyr Glu Val Ile Lys Ala Ala Ile Glu Lys
            260                 265                 270
Leu Lys Leu Arg His Lys Glu His Ile Ala Ala Tyr Gly Glu Gly Asn
        275                 280                 285
Glu Arg Arg Leu Thr Gly Arg His Glu Thr Ala Asp Ile Asn Thr Phe
    290                 295                 300
Ser Trp Gly Val Ala Asn Arg Gly Ala Ser Val Arg Val Gly Arg Glu
305                 310                 315                 320
Thr Glu Gln Asn Gly Lys Gly Tyr Phe Glu Asp Arg Arg Pro Ala Ser
                325                 330                 335
Asn Met Asp Pro Tyr Val Val Thr Ser Met Ile Ala Glu Thr Thr Ile
            340                 345                 350
Val Trp Lys Pro
        355
```

<210> 31
<211> 1074
<212> DNA
<213> Aureococcus anophagefferens

<400> 31

```
atggcgtcca tggaccaggc cgtgctcggc aagtacatgg gcctcgacac gggcgacgac      60
tgccaggtcg agtacgtctt cctcgacaag gaccaggtcg cgcggtccaa gtgccgcacg     120
ctgcccctca agaaggtcca gggccccgtg gacgcgtacc ccaagtggaa ctacgacggc     180
tcgtcgacgg gacaggcgcc cggcgacgac tccgaggtca tgatcgtgcc ccgcgccaag     240
taccccgacc ccttccgcgg cgggaaccac gtcctcgtgc tctgcgacac ctacgagccc     300
gacgggacgc ctctaccgac gaacacgcgc gcgcccgccg tcgcccgctt cgagtcgggc     360
ggcgcgaagg agcaggtgcc ctggtacggc ctcgagcagg agtacacgct cttcaacctc     420
gacggcgtca cgcccctggg ctggcccgtc ggcggcttcc ccaagcccca gggcccctac     480
tactgcggcg cgggcgcgga ccgcgcgttc ggccgcgccg tgtccgaggc gcactaccgc     540
gcgtgcctct acgcgggcct cgaggtctcg ggcacgaacg ccgaggtcat gcccggccag     600
tgggagtacc agatcggccc ctccatcggc atcgacgccg cggaccagct cacgatctcg     660
cgctacatcc tcagccgcgt ctgcgaggac ctcggcgtca tcgtcaccat cgaccccaag     720
```

cccatcgccg gcgactggaa cggcgcgggc atgcacatca acttctccac cgagtccacg 780
cgcaaggagg gcggcctcgc ggtcatcgag gccatgtgcg agaagctcgg cgcgaagcac 840
acggagcaca tcgccgcgta cggcgagggc aacgagcgcc gcctcacggg cgactgcgag 900
acggcctcca tcgaccagtt ctcctacggc gtcgccgacc gcggctgctc catccgcatc 960
ccccgcgaca ccgcggccga caagaagggc tacctcgagg accgccgccc cgcgtccaac 1020
gtggatccct acgtcgcgac gtcgctcatc ttcgcgacct gcacgtccgc ctag 1074

<210> 32
<211> 2031
<212> DNA
<213> Chlamydomonas reinhardtii

<400> 32
ctcacacacg cacaattctt tactctgctg cctgtccact cgcctgtcca actactacca 60
gtcggggatt tcttctcctg aaggtctaac catggccgcg ggatctgttg gcgtcttcgc 120
caccgatgag aagattggca gcctgctgga ccagtccatc acgcgccact ttctgtcgac 180
tgtgaccgac cagcagggca agatctgtgc cgagtatgtg tggatcggcg gctccatgca 240
cgacgtgcgc tccaagtcgc gcaccctgtc caccatcccc acgaagcccg aggacctgcc 300
ccactggaac tacgacggct cctccaccgg ccaggccccc ggccacgact cagaggtcta 360
tctcattccc cgctccatct tcaaggaccc cttccgcggc ggcgacaaca tcctggtcat 420
gtgcgactgc tacgagccgc ccaaggtcaa ccccgacggc accctggccg cgcccaagcc 480
gatccccacg aacacccgct ttgcctgcgc cgaggtgatg gagaaggcca agaaggagga 540
gccctggttc ggcattgagc aggagtacac gctgctcaac gccatcacca agtggccgct 600
gggctggccc aagggcggct accccgcccc ccagggcccc tactactgct cggccggcgc 660
cggcgtggcc atcggccgcg acgtggcgga ggtgcactac cgcctgtgcc tggccgcggg 720
cgttaacatc agcggcgtga acgccgaggt gctgcccagc cagtgggagt accaggtggg 780
cccgtgcgag ggcatcacca tgggcgacca catgtggatg agccgctata tcatgtaccg 840
cgtgtgcgag atgttcaacg tggaggtctc gttcgacccc aagcccatcc ccggcgactg 900
gaacggctcc ggcggccaca ccaactactc cactaaggcc acccgcaccg cgcccgacgg 960
ctggaaggtc atccaggagc actgcgccaa gctggaggcg cgccacgccg tgcacatcgc 1020
cgcctacggc gagggcaacg agcgccgcct gaccggcaag cacgagacca gcagcatgag 1080
cgacttcagc tggggcgtgg ccaaccgcgg ctgctccatc cgcgtgggcc gcatggtgcc 1140
ggtggagaag tcgggctact atgaggaccg ccggcctgcc tccaacctgg acgcctacgt 1200
cgtcacccgc ctcatcgtgg agaccaccat ccttctgtaa gcgcaggagc agcggcacgc 1260
aggagcagca gtgggcgatg gtggtggtgg cgtttgtgct ggcctgagcg aggggggggc 1320
cacggaaggg cgatcgtggc caaggcggag ggaagcggcg gtcgagccgc gtggtgatca 1380
aggtgaggcg tggtgcgcgt gtttgcattg acatgcgggc tcgtttggcg ccgtggcttg 1440
aagctggagc aattccaact gcatttggtt tgccgggacg tgtagcggtt caggaaagat 1500
ggggtgacgg cagcgaggac ccgctgtgtg ttctggtcca gtctgccaaa gggacttcgg 1560
acgcaggatg ctgcatcatc tgtggcgcag tcaactgatc tctacgaaga gccgcagtgc 1620
cataccattt gtcgtgtgcg tttcgagcct ggctgtgtgg acgccggcgc agaggtcgcc 1680
tggttgtgtg caagtgtatg ccgtcggcga cggaagggag cgtacaccgt gcggccaagc 1740
gacacggcgc tctgtacgtg cccgtcgtca agtgcatgag cggaggaccc cgcgcagcgc 1800
ggggtgcgtg gcatgacgtg agctcttatt ggctgtgcgc gacgcatgcg ttccctcatg 1860
taggggaggc gttgcataca ggaacggtcg cggccgtgtt tggtgttcaa actgtgtttt 1920
gtcttggtat tgtgtcctgg ttccaacagt ggttgggtga ttgtgcactt gaaacattct 1980
ttgtgtgggt cggacccact ctgtcgttct gtaacacggg aaagcatacg g 2031

<210> 33
<211> 3248
<212> DNA
<213> Chlamydomonas reinhardtii

<400> 33
cctgttacag caacatacag ttctagccaa gtagcaacgc acaacttcaa gccttgatca 60
atcagtatgg acctcgccac cgcgcttgga ctgggcatag ccccccgcc gcccgcggac 120
gactcctccc accacagcac cacggaagca tgcactctgc cggcgtatct gcgcgcgccg 180
gaggtgacgg cccaggttat ggccgagtac atctggctga tgggtgggac cggccagctg 240
cgcagcaaga ctaaggtgct ggacgccaag ccgtcttgtg ccgaggaggc ccctatcatg 300
attgtggaga gcaacccaga cggccagctc gccgagccga accatgagct tttcctcaag 360
ccccgcaaga tcttccggga ccccttccgc ggcggcgacc acattctggt cctctgcgac 420
acattcatcg tcgcccaggt tgtcgcggag gctggtgcgg ctccctcgac cgtgctgcag 480
cccagcgaga ccaacagccg cgttgcgtgc gagaacgtcc tgcgcgttgc cgagcagcag 540

gagcccgtgt ttgcggtgga gcaggagtac gccatcatcc acccggcgta ccccacgaag 600
gttccgctgg gacctcggcg cccttcgacc tcgcgcgcca gcagctgcca cagcggctcg 660
cgccgcagca gctacgtgtc cagtggctca gcgcgcggcg ggatcggcaa gaacagcagc 720
caccacggcg gcaagcagtc gcacgccgct gccgccgccg ctgcggcggc ggtcgccggc 780
atcccttggc ccagcccgga cgcatgtgag cagacggccc aagaagcgag cgcagcgagg 840
cagaaggcgt cgagacagct tgcggactcg cacctgcgct gttgcctctt tgcgggcgtg 900
agggtgacgg gcgcggacgt gcactcgctt gacggtctgc actcgtacaa gatcgggccg 960
tcgccggggg tggacctcgg cgatgacctc tggaccagca gatacctgct acagcgggtc 1020
gcagagcagc acagcgcatc ggtgtcgtgg gaacccgact caatgccgtc ggaacggccg 1080
ctgggctgcc acttcaaata cagtacggcg tcgacgcggc aggcgccaca cggcttgaac 1140
gcgatagaac agcagctcgt gcggctgcag gctacgcacg ttcagcacca ggtggcctac 1200
aacgacggca ggctggaccg gctgtcctcg ccggaggcct ccacgtttac gcacgcggtc 1260
ggctcggcca acgcctccgt cgtagtgccc agcctaacct tcctgcagca gggcggctac 1320
ttcacggacc gccgcccgcc gtcggatgcc gacccctaca aggtgaccct gctcctggca 1380
gcgaccacgc tggacatccc cctgcccaag ctgcccgcgt cctcgtccgc cggcaacacg 1440
gcggccaact gcagtggcgg catgtcggcg ggcccgtcct cgtgtcccgc tgctgctgcc 1500
ctgcctttcg gcagtcccat gcagagctac ctgctggccg ctgcggccgc ccaacggcag 1560
cagcagcagc agcacctgat gttcgacacg gagagcgagg agtgcgactc cgtcgacgaa 1620
gatgatgcga tgactgaaga ctcggcagct ctgctggcca agatggacga cgatggcggc 1680
gctgcagagg cgtcgtcgtg cgactcggac ttcgaggacc aggacgatgc cagctccagc 1740
cctatcaccg gcacctgggc ggacaacgac tgcacccaca tgctgggtgc tggcatttaa 1800
gactactaga ctgagaatgg aaccttgttt gcctcttgta ttgcttcgtg cagttcaaag 1860
tgtgcatgtc cgggtgctcg agtgtgtgcg cgttcccata atgcgcgtgt tccagtagta 1920
cgtgtgccca tgttccagta gtagttctcg ctgcagggtt attgttgaca agctttgtct 1980
gatgcctttc tcgtgcgttt tttcctcgtg cacatggacg cgagatgttc tggtctgatg 2040
gatccgagat tttgagcggc atgcaatcac gccggagcgc ggccgcaccc ctctcactgc 2100
tatatcgata ccctggtcag ggtttacgcg cgtcatcccg tagatggagt gggagcgaaa 2160
gagacttgtg caagctgtac accgcaattg gcgctttggc tgattgttcc gtgccagttc 2220
tgcatgccgt gacggtatcg aaatgaatgt gtccaagcat ttggctgggt ggcgattgaa 2280
ggatcgggat ggacctgatg ggcatcacct ggtgcatgtg cgctcaagcc gttcaatgga 2340
aagaatggca agatgggttt gcagtgtgca catgcctaat gctacctagt gaacacgtgt 2400
gcctgccgtg aatgtgtgtg tgtgtgtgtt taggttcacc ctgtttaccg agctatccgg 2460
ggcagacatc cctccgatta tcatcataaa tgcatggctg gcatggggag ctgactacaa 2520
ccggggggttt caagatttac acaaccgcca gccgacttgc ggtgctggcg gatcggactg 2580
acgtagtggg ctcatccttg aggcgtgtag agtgtgcagt actgactggt ggcagcgctg 2640
tagtagcggc gtacgagccg catatcaagc attacgggtg accatttcca aatgattaca 2700
atctggtgcg gcggcggagg tgcggcttgg gttctgcagc ccattctatc actggcgcgg 2760
aggtcatcaa gccggagccg acctgacacg ggcccgtaag ggatgcacgg tcaacaggcc 2820
aggaaagcag gatggcacag gccgtgtgtc gtgtgacgcg atccatgtca cggtggctgg 2880
atgaagttag cagtatcaat ggcatgactg cgagcatggt cgctgtgtgg cgccaggcca 2940
aacatagacg gtcaagcagt atcatgcagg tgaaccccgt gaagggatgt gcacgcatga 3000
gcagtatcaa tggcatgact gcgagcatgg tcgctgtgtg gcgccaggcc aaacatagac 3060
ggtcaagcag tatcatgcag gtgaaccccg tgaagggatg tgcacgcatg aactctaatg 3120
ttgattagca agtgtacggt tgttctgtat gtcgtggggc gtctgttcgc gggtggtgca 3180
tgggtgcatt gacctggctg tgagtattca tgtaaacgtt ttgggattct gtacatctcc 3240
agaacccg 3248

<210> 34
<211> 1593
<212> DNA
<213> Chlamydomonas reinhardtii

<400> 34
ctttacctcg ttgcaaagat ggcgttcgct ctgcgtggtg ttaccgctaa ggcctcgggc 60
cgcactgctg gcgcccgctc gtcgggccgc accctgacgg tgcgcgtcca ggcctatggc 120
atgaaggctg agtacatctg ggcggatggc aacgagggca agcctgagaa gggcatgatc 180
ttcaacgaga tgcgctcgaa gaccaagtgc ttcgaggccc ccctgggcct ggacgcctcg 240
gagtaccccg actggtcgtt cgatggctcg tccaccggcc aggctgaggg caacaactcg 300
gactgcatcc tgcgccccgt gcgcgtggtg accgacccca tccgcggtgc cccccacgtg 360
ctggtgatgt gcgaggtgtt cgccccccgat ggcaagcccc actccaccaa cacccgcgcc 420
aagctccgcg agatcattga cgacaaggtc actgccgagg actgctggta cggcttcgag 480
caggagtaca ccatgctggc caagacctct ggccacatct acggctggcc cgctggcggc 540
ttccctgctc cccagggccc cttctactgc ggtgtgggcg ctgagtccgc cttcggccgc 600

cccctggctg aggcccacat ggaggcctgc atgaaggccg gtctggtcat ctccggcatc 660
aacgccgagg tgatgcccgg ccagtgggag taccagatcg gccccgtcgg ccctctggcc 720
ctgggcgacg aggtgatgct gtcccgctgg ctgctgcacc gcctgggcga ggacttcggc 780
attgtgtcga ccttcaaccc caagcccgtg cgcaccggtg actggaacgg cactggcgcc 840
cacactaact tctcgaccaa gggcatgcgc gtgcccggcg gcatgaaggt gatcgaggag 900
gccgtggaga agctgtccaa gacccacatc gagcacatca cccagtacgg cattggcaac 960
gaggcgcgcc tgaccggcaa gcacgagacc tgcgacatca acaccttcaa gcacggtgtg 1020
gctgaccgcg gctcttccat ccgcattccc ctgcccgtca tgctcaaggg ctacggctac 1080
ctggaggacc gccgccccgc tgccaacgtc gacccctaca ccgtggcgcg cctgctgatc 1140
aagaccgtgc tcaagggcta aatgcccagc atgcgccagc taataagggc agcgatgagg 1200
cggagggtg cgtgactcgg atgtgagctg tgatgagggg gttgcttcta tcggctaagg 1260
gtgtgtgtgt gtgtgtctgt ctatgctggg ccgggtatgt ggaccggcga cctgacgttt 1320
ggaatgcgtg cgtgtgcaca ctgcccggtt gcagtgtctg cgcatgtatt tcctggcaac 1380
tccaaagcct acggttgagc aagtgacctg tctttggttg gacgattgtt ctgacacgtc 1440
gattgctgct aggttaacgg gaggttgcgg cgtgagccct gcgacgagct gcgtaatact 1500
atttccttgt acttcttcct cgcgcgccct cctgggtgct gacgcattgt caggtttgct 1560
caggtcgcca catgtaatcg aacacgtcaa cag 1593

<210> 35
<211> 1328
<212> DNA
<213> Helicosporidum sp.

<400> 35
catttcttat tcctttggag ctgtgctcct tttggttttg tgcagttgtg tactgccggc 60
actccttcgc cttcggtgct ttctgcgtag agctcaagca tgtctcctcc cactggcgaa 120
aagtactctc tgccccccgt cttcgggacg caggggcaga tcacccagct gcttgaccct 180
atcatggctg agcgcttcaa ggacctctct cagcacggca aagtgatggc ggagtacgtc 240
tggattggcg gcacgggcag cgacctgcgg tgcaagaccc gcgttctgga ctcggtcccc 300
aacagcgtcg aggatctgcc ggtgtggaac tacgacggct cctccacagg ccaggccccc 360
ggcgacgatt cggaagtatt cctcatcccc cgcgccatct accgcgatcc tttccgcggc 420
ggggacaaca tcctggtgct ggcggacacg tacgagcccc cacgcgtgct ccccaacggc 480
aaggtttccc cccccgtgcc gctgcccacc aactcccgcc acgcctgcgc cgaggccatg 540
gacaaggctg cggcgcacga gccctggttc gggatcgagc aggagtacac ggtgctggac 600
gcccgcacca agtggcccct gggctggccc tccaacggct tccccggtcc ccaaggccct 660
tactactgcg cggctggcgc ggggtgtgcc atcggccgag acctgatcga ggcgcatctc 720
aaggcgtgcc tgttcgcggg catcaacgtc tcgggcgtga acgccgaggt gatgcccagc 780
cagtgggagt accaggtggg tccctgcacc ggcatcgaaa gcggagacca gatgtggatg 840
agccggtaca ttctcatccg gtgcgccgag ctctacaacg tggaggtttc tttcgacccc 900
aagcccgtgc ctggcgactg gaacggcgcc ggcgggcacg tcaactactc caacaaggcc 960
acccgcacgg ccgagacggg ctgggcggcc atccagcagc aagtcgagaa gctgggcaag 1020
cgccatgccg tgcacatcgc cgcttacggc gagggcaacg agcgccgcct cacgggcaag 1080
cacgagacca gctccatgaa cgacttctcg tggggcgtgg ccaaccgcgg cgcctcggtg 1140
cgggtgggcc gtctcgtgcc ggtggagaag tgcggctact acgaagaccg acgcccggcc 1200
tccaacctgg acccttacgt ggtcacgcgc ctgctggtgg agaccacgct gctcatgtag 1260
atatgcaggg gggtgggtgg gagatggcaa cggctgtgac ttgcgtggat gtagatagtt 1320
ttcgggtg 1328

<210> 36
<211> 1470
<212> DNA
<213> Thalassiosira pseudonana

<400> 36
caaaatcaac caaccatgaa gctctccatc gccctcctct ccatggccgc gacggccaca 60
gccttcgccc catccctcac cacccccctcc cgcaccacct ccctctccat ggtaaacccc 120
ctcgagatca gaaccggaaa agcccaacta gaccactccg tcatcgaccg cttcaacgca 180
cttccctacc ccgctgacaa agtactggcc gaatacgtct gggtcgacgc caagggagag 240
tgccgttcaa agacgcgtac tcttcccgtg gctcgtacca cggctgtgga caatttgcct 300
cgttggaact ttgatggaag ttcgacaggt caggctcctg gtgatgatag tgaggttatc 360
ttgagaccgt gtaggatctt caaggatcct ttcaggccac gtaatgacgg tgtggacaac 420
atcttggtga tgtgtgatac ttatactcct gccggagagg ctttgcctac gaatacgagg 480
gcgattgccg caaaagcctt tgaaggaaag gaagacgaag aaatctggtt cggcctcgaa 540

```
caagaattca ccctcttcaa cctcgaccaa cgcacccccc tcggctggcc caagggaggc    600
gtccccgccc gcgcccaagg cccctactac tgctccgtcg gacccgagaa ctccttcgga    660
cgtgccatca ccgacaccat gtaccgtgcc tgtctctacg ccggtattga gatcagcggt    720
accaatggag aggtcatgcc cggtcagcaa gagtatcagg ttggaccatg tgtaggaatt    780
gatgctggtg atcagcttca gatgtcacga tacattcttc aacgtgtgtg tgaggagttc    840
caggtctact gtactctaca ccccaagcct attgtggagg gagattggaa cggagccggt    900
atgcacacca atgtctccac caaatccatg cgtgaggagg gaggacttga ggtcatcaaa    960
aaggcaattt acaaacttgg agccaagcat caagagcaca tcgctgttta cggagagggc   1020
aatgagttgc gtttgactgg aaaacacgag actgcaagta ttgatcagtt ctcgtttgga   1080
gttgcaaata ggggagctag tgtgaggatt ggaagggata ccgaggctga gggtaaggga   1140
tactttgagg acaggaggcc tagttcgaat gctgatcctt atttggttac tggaaagatt   1200
atggctacca tcatggagga cgttgacgtt ccagaaatca gtgcccttga ccgtgccgag   1260
gcctaagcac ttcttctttc ttcccaaaca cactccttct ttctctttgg agaacttttg   1320
aacatgacga gtaggaatac gacactgatt gcacaattca aatgagtttg gcaagtgtac   1380
agtcttcttt gttgagagaa tgtctcattt ttcatgccga ggctacgata attgactaat   1440
gctactaaag gagaatagtt tgctgaattg                                    1470
```

<210> 37
<211> 2446
<212> DNA
<213> Volvox carterii

<400> 37

```
caaaatctgt aatcatggct accatgcgca tgtccacgaa ggctcagggc cgcgtcggga     60
ttgtccgcaa cacgcggacc ctgacagtgc gcgtacgtgc gtatggtatg aaggccgaat    120
atatctgggc cgatggaaat gagggccggc ccgagaaggg catgatcttt aacgagatgc    180
gctcgaagac gaaggtcttt gatgaggctc taccccctgga agctggccag taccccgact    240
ggtccttcga tggctcttcg accggccagg ccgccggcaa caactccgac tgcatcctca    300
ggcccgtccg cgtcatcaag gaccccatcc gcggtgagcc gcacgtgctg gtgatgtgcg    360
aggtgttcgc ccctgatggc accccgcacc ctaccaacac tcgtgccaag ctgcgcgaca    420
tcattgacga caaggtcctt gccgaggact gctggtacgg tctggagcag gagtacacca    480
tgcttcaaaa gaccaccggc cagatctacg gctggcccag cggcggttac cctgcacccc    540
agggcccctt ctactgcggt gtcggtgcgg agtcggcgtt cggccggccc ctggctgagg    600
ctcacatgga ggcttgcatg aaggctggtc ttaagatctc tggcatcaac gccgaggtga    660
tgccaggcca gtgggagtac cagattggcc cggtgggtcc cttggagatg ggcgatgagg    720
tgatgctgtc gcgctggctg ctgcaccgtc tgggcgagga tttcggcatt gtctgcacct    780
tcaaccccaa gcctgtccgc accggcgact ggaacggcac tggcgcgcac accaacttct    840
cgaccaagtc catgcgccag cctggcggca tgaaggtgat tgaggacgcc gtggagaagc    900
tctccaagac ccacattgag cacatcaccc agtacggtct gggcaatgag gctcgtctga    960
ccggcaagca cgagacgtgc gacatcaaca ccttcaagca cggtgttgcg gaccgcggct   1020
cgtccattcg catcccgttg ccggtcatgc tgaagggcta cggctacctg gaggaccgtc   1080
gcccggctgc caacgttgac ccgtacactg tcgcccgcct gctcatcaaa tccatcctca   1140
agggcccgca gtaaatgatc cctcgtactg agccacttcg gtcattccga cgcacccata   1200
ggcaacttac ggttacctag tctcggacgt tcttgtgaat gggttggcct catttgcagg   1260
atggcatgat gggacaggtg taagatgttc tagaggctct ggagtgggct tggggctgga   1320
gataccccgg tgcatgtttg tagctgtggg ttggctggta cgatgtgaca agaaccgtcc   1380
ggaactatta agaagttcat tggatcaatg gacaatatat ttattgcgga aatgtctttt   1440
tgcgcgttga caagtggcta gctgctactg atcctactat tatctgccat acttacgcag   1500
tttaattttc ggcatcagtg cacacgttct cctgtaatgg ttaggaaaca tgtgctattg   1560
aggagacgtg cgtgtgactg atatcctgac acgcctaggt atcggagtgt acgttgagtt   1620
ccagttcacg ggttcatgcg gctagcgggc atgccttggc gacggctgca attgcaccga   1680
gttgccgagg ggtgcatgtg catagcgggt tgtcgcatac ggagataatg ctctttgtgt   1740
ttggggcttt ttttcctgtg tgtagctctc ttctacagca gctaagcgag cttaattcgt   1800
gaggtacaga gagtttcatc tactgtatag attactttat ttccttccgg gtattgaacg   1860
attgcatgcc gtacctgggc atgtagtctt cgacgtacgt gtgctaagct tctgcggttc   1920
tcatgaagtg gagatgccgc atttgtatca tgattgcaca aatataacga tcttggtgtg   1980
tcaggcccgg gcaagctgct gtgcaatcac ctgatactgt ctcgattgat actgtcctaa   2040
aacgattgtt atcattactg cgtgacatcg tacgacgcag cgtaactttt cttcaagcac   2100
aactgtcatt gacatactgt cttaacgaca ggcacaaatc atcaagtgtt taaaacggca   2160
ggccgttccg cgctgacctg gtgctggcat ggctcctcat ccagcgaatg gcaatcaaag   2220
tgggtaggaa acccaacttg atttaaacac ttgtttggta tagtacggca aaagtcaacg   2280
acccccgaac ttggctgtac agcatgggtg gtgattttct tcagggacac ttgaaacttt   2340
gtatacacat gccggaatac agtcaacaat atttattaaa gcaattgatt acagaagtct   2400
```

taacagtgat aggacactca tttagttggc agttgtaaaa tgttat 2446

<210> 38
<211> 2269
<212> DNA
<213> Volvox carterii

<400> 38
aaggactttc gtcggcaact caccgcgtcg cacacagttc tgcttcaggc cagcttgaga 60
taaatggctg ctggatcaat tggcgttttt gcaactgatg agaagattgg aagccttctg 120
gaccagtcca ttacccgcca cttcctgacc aatgtaacgg atcagtgtgg caagatcacc 180
gcggagtatg tgtggattgg cgggagcatg caggacttga ggtctaagtc ccgcaccctg 240
acttctgttc ccacaaaacc cgaggacctt ccgcattgga actacgacgg ttcgtccacg 300
ggccaagcgc cgggccacga ctcagaggtg tacctcatcc cccgccgcat tttccgggat 360
ccgtttcggg ggggtgacaa catccttgtc atgtgcgatt gctacgagcc gcccaaggcc 420
aacgcggacg gtattctgca accgcccaag cccatcccaa ccaacactcg ctacgcgtgc 480
gccgaggcta tggagaaagc caaggatgag gagccatggt tcggcattga gcaggagtac 540
acgctgctga acgcgattac caagtggccg cttggctggc ccaagggcgg ttaccccgca 600
ccgcagggcc cgtactactg ctctgccggt gcaggtgtgg ctataggccg cgacgttgcc 660
gaggttcact acaggttgtg tctgtacgct ggggtcaaca tcagcggcgt gaacgctgag 720
gtgctgccat cgcaatggga gtaccaggtg ggcccatgcg agggcattga gatgggcgac 780
cacatgtgga tgtcccgtta catcatgtac cgcgtatgtg agatgttcaa cgtggaggtg 840
tcgttcgacc ccaagcccat tcccggcgac tggaacggct caggtggcca caccaactac 900
tccaccaagg ccacacgcac tgcgcctaac ggctggaagg ccatccaaga gcactgccag 960
aagctggaag cgcgccacgc ggttcacatt gccgcctatg gtgagggcaa cgagcgccgc 1020
ctgacgggaa agcacgagac gtcgtccatg aacgacttct catggggcgt cgcgaaccgc 1080
ggctgctcca tccgcgttgg ccgcatggtg cccgtggaga agtgcggcta ttacgaggat 1140
cgccgccccg cctccaacct ggacccgtac gtggtcacca agctcatcgt tgagaccacg 1200
gtcctcctgt aatggcgtgg gtcagcaaaa tggtgggtcg gcatgttcat taggtgtagt 1260
tgtaacggca atccgggtgg atagtgctca gtcgcggcgt gtttgtggac gttatcatca 1320
gcgtgctata gtgatgggcg gctgagaccg tatgagactc gcgcgcaatg gcggttgtgg 1380
caaggttttt aagtgtcccc gccatcttat tccatgcccc ggctttcgga ggctgctgct 1440
gaatgaagcg tccggggttg gcctacccca ctggggctgc tgtcggcaaa acaaggtgca 1500
acgccagacg gtgtaggctg ttggatctgg gtgcttcgat gtgccgggca ctggaggaca 1560
caatctaagc aagggccgag cggtttcatc gttaggaaac tgatttgacg ttggctgtat 1620
acaggaacgg agatttatga ctcgcgtcca tgctcattgc aggggcatgc tggtacaagg 1680
gtaatgtgtc ctttggctgt gtgaaccgct cgccatgcag gattgtgctg gcgagtccgg 1740
gattgcgtcg cacttggcta attgtagcac taaaacgctt tttacagtaa aatacgacca 1800
cctggacgac tgacacgact acactggttt gatggactgc aggcagaggc cgtctgcaga 1860
tgttattgtg catcctcgtg gatatgggtg ttttgttgtt cggatgatgt aggcgtccgg 1920
atgaggtgat ggctcgtggg gacagattac aaatgtcgtt ggtgcatatt ttttagtatc 1980
gcgatgatgg tttggagcga aacgtattgt cgccagtgca atatatacac gcgagccacc 2040
gcgtaagtag tgaggatcct cggccatacc tttcttatat cgaacccctc cattgtgtca 2100
tcaccttttg gccacgaaat acacagattt ccatattttg gtgctatcta tatgatgagt 2160
taagtccctg atgccgtctt tttgacgtcc gaggagttgg tacgtgacgg gcaagtgaca 2220
gctatcaaaa actttcgatg gtagcttttg taatcaccgg tcgccgcac 2269

<210> 39
<211> 1341
<212> DNA
<213> Arabidopsis thaliana

<400> 39
ctctataaac acacactctc aggagagaag ttgtattgat cgtcttctct ttccctaaac 60
acactgatta ttttctctcc gacgccgcca tgtctctgct ctcagatctc gttaacctca 120
acctcaccga tgccaccggg aaaatcatcg ccgaatacat atggatcggt ggatctggaa 180
tggatatcag aagcaaagcc aggacactac caggaccagt gactgatcca tcaaagcttc 240
ccaagtggaa ctacgacgga tccagcaccg gtcaggctgc tggagaagac agtgaagtca 300
ttctataccc tcaggcaata ttcaaggatc ccttcaggaa aggcaacaac atcctggtga 360
tgtgtgatgc ttacacacca gctggtgatc ctattccaac caacaagagg cacaacgctg 420
ctaagatctt cagccacccc gacgttgcca aggaggagcc ttggtatggg attgagcaag 480
aatacacttt gatgcaaaag gatgtgaact ggccaattgg ttggcctgtt ggtggctacc 540
ctggccctca gggaccttac tactgtggtg tgggagctga caaagccatt ggtcgtgaca 600

```
ttgtggatgc tcactacaag gcctgtcttt acgccggtat tggtatttct ggtatcaatg    660
gagaagtcat gccaggccag tgggagttcc aagtcggccc tgttgagggt attagttctg    720
gtgatcaagt ctgggttgct cgataccttc tcgagaggat cactgagatc tctggtgtaa    780
ttgtcagctt cgacccgaaa ccagtcccgg gtgactggaa tggagctgga gctcactgca    840
actacagcac taagacaatg agaaacgatg gaggattaga agtgatcaag aaagcgatag    900
ggaagcttca gctgaaacac aaagaacaca ttgctgctta cggtgaagga aacgagcgtc    960
gtctcactgg aaagcacgaa accgcagaca tcaacacatt ctcttgggga gtcgcgaacc   1020
gtggagcgtc agtgagagtg ggacgtgaca cagagaagga aggtaaaggg tacttcgaag   1080
acagaaggcc agcttctaac atggatcctt acgttgtcac ctccatgatc gctgagacga   1140
ccatactcgg ttgatgacac atttcatgat ttgatttctc tccaatttgg tttttttttt   1200
ttccctttg attgcacttt tcgataataa aaaaataatt cttattatgg gcgtattgtt    1260
gtgacatttt gtgttttgtt tcgaataatt aaataagcgc ttcttaaggt gaaaataaat   1320
aataattagt gatttttaat c                                              1341
```

<210> 40
<211> 1494
<212> DNA
<213> Arabidopsis thaliana

<400> 40

```
tgtggagagc caaaaagtct ccaaagtctt cacgtcaccc tcttcctcaa tctctgcacc     60
cacccctcct ccttctataa gtactactct tcatatctct ctctaccaaa atatcaaaac    120
acgagacaga tttgattcca tttttattac tgttactatc atccaaaccc ttggtatttg    180
tagccatgag tcttgtttca gatctcatca accttaacct ctcagactcc actgacaaaa    240
tcattgctga atacatatgg gttggtggtt ctggaatgga catgagaagc aaagccagga    300
ctctacctgg accagtgact gacccttcgc agctaccaaa gtggaactat gatggttcaa    360
gcacaggcca agctcctggt gaagacagtg aagtcatctt ataccctcaa gccatattca    420
aggatccttt ccgtagagga aacaacattc ttgtcatgtg cgatgcgtac actcccgcgg    480
gtgaaccaat cccgactaac aaaagacacg ctgcggctaa ggtctttagc aaccctgatg    540
ttgcagctga agtgccatgg tatggtattg agcaagaata cactttactc cagaaagatg    600
tgaagtggcc tgttggttgg cctattggtg gttatcccgg ccctcaggga ccgtactatt    660
gcggtattgg agcagacaaa tcttttggca gagatgttgt tgattctcac tacaaggcct    720
gcttatacgc tgggatcaac attagtggca tcaatggaga agtcatgccg ggtcagtggg    780
agttccaggt cggtccagct gttggtatct cggctgctga tgaaatttgg gtcgctcgtt    840
acattttgga gaggatcaca gagattgctg gtgtagtggt atcttttgac ccgaaaccga    900
ttcccggtga ctggaacggt gctggtgctc actgcaacta cagtaccaag tcaatgaggg    960
aagaaggcgg ttacgagatc atcaagaaag caatcgataa attgggactg agacacaaag   1020
aacacattgc tgcttacggt gaaggcaatg agcgtcgtct cacaggacac cacgagactg   1080
ctgacatcaa cactttcctt tggggtgttg cgaaccgtgg agcatcgatc cgagtaggac   1140
gtgatacgga gaaagaaggg aaaggatact ttgaggacag gaggccagct tcgaacatgg   1200
atccttacat tgtcacttcc atgattgcag agactacaat cctctggaat ccttgatgat   1260
catcagatca agaaaaaatc ttgaatgtca ctcaaatttg tgtttcttgc aagattcaaa   1320
gtttgtgttc tctatcaagc aatgtcttag gataagtcaa agatttgctc tgcttattct   1380
gctttttatt tacttcacat cctattgaaa acatttctgt gtattattta tgaataaaca   1440
ttatcttaaa agggctgatt tatttactaa tgcatgcatt caccacttaa gatc          1494
```

<210> 41
<211> 1317
<212> DNA
<213> Brassica napus

<400> 41

```
ggctcacctc agactgatta ttataactcg atcgtcatct tcttcggctt gatggaaaca     60
gaaaaaatgt ctccactctc agatctccta aacctcaacc tcgacaccaa gcaaatcatc    120
gctgaataca tatggatcgg tgggtctgga atggacatta gaagcaaagg caggacatta    180
ccaggaccag taagtgatcc atcaaagctt ccgaaatgga actacgatgg atccagcacc    240
aatcaagccg ccggagatga cagtgaagtc attctatatc ctcaggcgat tttaaagac     300
ccgttcagga aagggaataa cattctcgtg atgtgtgatg cttacacacc gaaaggagat    360
ccaatcccga ccaacaatag gcacaaagcc gtgaaaatct tcgatcatcc caatgtgaag    420
gctgaagagc cttggtttgg gatagagcaa gaatacacat tacttaagaa agacgtcaag    480
tggccattgg gttggcccct tggtggcttt cctggtcctc agggaccgta ctattgtgcg    540
gtcggtgcag acaaagcctt tgggcgtgac attgtggatg gtcactacaa agcttgtctt    600
tacgctggtt taagcatagg tggtgccaat ggtgaagtca tgcctggtca atgggagttt    660
```

```
caaatcagcc ctactgttgg tattggtgca ggtgatcagt tatgggttgc tcgctacata    720
ctcgagagga ttactgagat atgcggcgtg attgtctcat ttgatcccaa accaatcgag    780
ggtgattgga acggagcagc tgctcataca aacttcagta caaaatcaat gaggaaagaa    840
ggaggattgg acttgataaa aaaagcaata gggaagcttg aagtgaagca taaacaacac    900
attgctgctt atggtgaagg caatgagagg cgcctcactg ggaagcatga aaccgcagac    960
atcaacaagt tctcttgggg agttgcggat cgtggagcat cggtgagagt gggaagagat   1020
acggagaaag aagggaaagg ttattttgaa gatcgaagac cttcgtctaa tatggatcct   1080
tatcttgtta cctccatgat agctgaaacc accatcctcg gctaagcttt cttttgaagt   1140
tgttgcatac gttcttttgt ttcttcatgt ttcggtttaa tttcggtttg agactttttt   1200
ttttggtgct aataattcat gggatggtct tgatcctatt gtttgtttat cctggttcag   1260
ttgttagtgt taaacaaaat tgaattggga aaataaaggt tcttagttct tacttttt     1317

<210>  42
<211>  1555
<212>  DNA
<213>  Brassica napus

<400>  42
ttcatatttg tcaactcttc ctttgccatt tgttgcaaac actcaagtct cctgatatca     60
gagttagagt cttcttcaag ttccagggat aaaaatggcg cagatcttgg cagcttctcc    120
aacatgtcaa atgagattga ctaaacccag ctccattgca tcgtcaaagt tatggaactc    180
ggttgtgttg aaacagaaga aacagagcag cagcaaagtc agaagcttca aagtgatggc    240
tctccaatct gataacagca caatcaacag agttgagagt cttctcaatc tagacaccaa    300
acctttcact gaccggatca tcgctgagta catctggatt ggcggatctg gaattgacct    360
taggagcaag tcaaggacgc ttgaaaagcc cgtggaagat ccttctgaac ttcccaagtg    420
gaactatgat ggttcaagta ccggtcaagc acctggtgaa gatagtgaag tgattctcta    480
tccgcaagct atcttcaggg atcctttccg tggaggcaat aacatattgg ttatctgtga    540
tacctacaca ccagctggtg agccaattcc aacaaacaaa cgtgcaagag ctgctgagat    600
tttcagcaac aagaaggtca atgaagagat tccatggttt ggcattgaac aagagtacac    660
tttacttcag ccaaacgtga actggccttt gggttggccc gttggagcgt atcctggtcc    720
ccagggtcct tactactgtg gagttggagc tgaaaagtct tggggccgtg acatttcaga    780
tgctcattac aaagcttgtt tgtatgctgg aattaacatc agtggtacta atggtgaagt    840
tatgccagga cagtgggaat tccaagttgg cccgagcgta ggaatcgaag caggtgatca    900
cgtttggtgt gctagatacc ttcttgagag aatcacagaa caagctggtg ttgtcctaac    960
acttgatccc aaaccgattg agggtgactg gaacggtgct ggttgccata ccaattacag   1020
cacaaagagc atgagagagg acggaggatt tgaggtgatt aaaaaggcaa tcttgaacct   1080
ctcgcttcgt cacatggagc acatcagtgc ctacggtgaa ggcaatgaga gaaggttgac   1140
tggaaagcac gagacagcca gtatcgacca attctcatgg ggagtggcta accgtggatg   1200
ctcaattcgt gtgggacgtg ataccgagaa gaaaggaaaa ggttacttgg aagatcggcg   1260
tccagcgtct aacatggacc catacattgt gacttcactg ttggcagaga ccacacttct   1320
ctgggagcca acccttgagg ctgaagcact tgctgctcag aagctttctt taaaagttta   1380
atttattaat gaacacacat gtctgtttat gtggtcttcc cgggatcatc agtcttgttt   1440
agaacacgtg ttcggattac gacattcttg tctctttttt ttcatttgca ttgtttaaaa   1500
aacccagaat ttcgtggaca atgttcatcc ttttctattg gttgtttatg gtctt        1555

<210>  43
<211>  1456
<212>  DNA
<213>  Hordeum vulgare

<220>
<221>  misc_feature
<222>  (1237)..(1237)
<223>  n is a, c, g, or t

<220>
<221>  misc_feature
<222>  (1240)..(1240)
<223>  n is a, c, g, or t

<220>
<221>  misc_feature
<222>  (1324)..(1324)
```

<223> n is a, c, g, or t

<220>
<221> misc_feature
<222> (1405)..(1405)
<223> n is a, c, g, or t

<400> 43
gaattccctc cctccctgcc ctcagtcgtc cagccgggtt cctccatccc tcccgccatg 60
gcgctcctca ccgatctcct caacctcgac ctctccggct ccacggagaa gatcatcgcc 120
gagtacatat ggatcggcgg atctggcatg gatctcagga gcaaggccag gcacctcccc 180
ggcccggtca cccaccccag caagctgccc aagtggaact acgacggctc cagcaccggc 240
caggccccgg gcgaggacag cgaggtcatc ctgtacccac aggccatcct caaggacccg 300
ttcagggagg gaaacaacat ccttgtcatg tgcgattgct acaccccacg tggagagcca 360
atccccacca acaagagata caacgctgct aagatcctta gcaaccccga tgttgccaag 420
gaggagccat ggtacggtat tgagcaggag tacaccctcc tacagaagga catcaactgg 480
cctctcggct ggcctgttgg tggcttccct ggtcctcagg gtccctacta ctgtggtatt 540
ggtgctgaca agtcgtttgg gcgtgacata gttgactccc actacaaggc ttgcctcttt 600
ggcggcgtca acatcagtgg catcaacggc gaggtcatgc ccggacagtg ggagttccaa 660
gttggcccga ctgttggcat ttctgctggt gaccaagtgt gggtcgctcg ctacattctt 720
gagaggatca ccgagatcgc cggagttgtc gtcacgtttg accccaagcc catcccaggc 780
gactggaacg gtgctggtgc tcacacgaac tacagtaccg agtcgatgag gaatgacggt 840
gggttcaagg tcatcgtgga cgcggtcgag aagctcaagc tgaagcacaa ggagcacatc 900
gcggcctacg gcgagggcaa cgagcgccgt ctgaccggca agcacgagac ggccgacatc 960
aacacctcca gctggggtgt ggcaaaccgt ggcgcgtcgg tgcgcgtggg ccgggagacg 1020
gagcagaacg gcaagggcta cttcgaggac cgccggccgg cgtccaacat ggacccctac 1080
gtggtcacct ccatgatcgc ccagaccacc atcctgtgga agccctgaag ctccgatcgc 1140
cgtgtgatgg accgtcggtg atggggtccg gtggtggcca ttggaggatt cgtgccttgg 1200
gcgaaaattc ttccagcatt ttccttttac gtgtggntgn atactactcc tagtccgctt 1260
aggtaggtca catcatcatg gtcatctcat cagggtgtct ggtctctctt ctcgctctcg 1320
tctntgggtg ggtggtgggt gatgggtggc aaggggcgtg tcaaagcaga ttgatatggt 1380
aataaaacaa gattactaca gtatntgggt gattgttaac ccttgccgtc tggatgctat 1440
ggtctcgtgt aatctc 1456

<210> 44
<211> 1495
<212> DNA
<213> Oryza sativa

<400> 44
attgatagcc tgtgcgtctc caagaagagg cttgccgctg ccgccattgg agccctctcg 60
tttctgctcg agctctgcat ttcttcagta ggaggaggag gaggaagagt tggagtcgcc 120
atgtcgtcgt ccctgctcac tgacctcgtt aacctcgacc tgtcggagag cacggacaag 180
gtcatcgccg agtacatatg ggttggtggt actgggatgg atgtgaggag caaagccaga 240
acgttgtctg gacctgttga tgacccaagc aagcttccaa agtggaactt tgatggctcc 300
agcaccggtc aggctaccgg tgacgacagt gaagtcatcc tccaccctca agccatcttc 360
agagacccat tcaggaaggg gaagaacatc ctggtcatgt gtgactgtta tgcgccgaat 420
ggcgagccga ttccgacgaa caaccggtac aatgcagcaa ggatcttcag tcatcctgat 480
gtcaaggctg aagagccatg gtatgggatt gagcaggagt acacccttct tcagaagcac 540
atcaactggc ctcttggctg gccactaggt ggctatccag gccctcaggg tccgtactac 600
tgtgcggcgg gagccgataa atcgtacggg cgcgacatcg ttgatgccca ctacaaggcc 660
tgcctgtttg ccggcatcaa catcagcggg atcaacgcag aagtcatgcc ggggcagtgg 720
gagttccaga ttggccctgt cgttggcgtc tccgcagggg atcatgtctg ggtggcacgc 780
tacattcttg agaggatcac tgagattgct ggcgtcgtcg tgtccttcga ccccaagccc 840
attccgggag actggaatgg cgccggtgct cacaccaact acagcaccaa gtcgatgagg 900
agcaatggcg gctacgaggt gatcaagaaa gcgatcaaga agcttggcat gcgccaccgt 960
gagcacatcg ccgcctacgg cgacggcaac gagcgccgcc tcaccggccg ccacgagacc 1020
gccgacatca acaacttcgt ctggggcgta gcgaaccgcg gcgcgtcggt gcgtgtcggc 1080
cgggacaccg agaaggacgg caaaggttac ttcgaggaca ggaggccggc gtccaacatg 1140
gacccgtacc tggtgaccgc catgatcgcc gagaccacca tcctctggga gcccagccac 1200
ggccacggcc acggccaatc caacggcaag tgaggaggag tcgcctcgcc cgggttgatg 1260
aactgctttc tcgcgttctg ggtttcatgg aaatctgtgt gtgtgtgttc tctgacgctg 1320
gtgctgttag aaacttccaa taattcagaa ataactgcga tgtgctctca aatttctcat 1380

```
gaggccatca cctgcagcat ctcatgaaat agatctattg caatgacaat accaatggca   1440
acgcaaaatt ttatggtacc tccagatacc atctactctc ctcaataatg acaat        1495

<210>  45
<211>  1677
<212>  DNA
<213>  Oryza sativa

<400>  45
atcgacgtcg cctcctctcc tcctcctcct cgtcgctgca ttccggttga gtgagttggt     60
gattatctgt aggggggtgaa aatggcgcag gcggtggtgc cggcgatgca gtgccaggtc   120
ggggccgtgc gggcgaggcc ggcggcggct gcggcggcgg cggggggag ggtgtgggga     180
gtcaggagga ccgggcgcgg cacgtcgggg ttcagggtga tggccgtgag cacggagacc   240
accggggtgg tgacgcggat ggagcagctg ctcaacatgg acaccacccc cttcaccgac   300
aagatcatcg ccgagtacat ctgggttgga ggaactggaa ttgacctcag aagcaaatca   360
aggacaatat caaaaccagt ggaggacccc tcggagctac caaaatggaa ctacgatgga   420
tcaagcacag ggcaagctcc aggagaagat agtgaagtca tcttataccc acaggctata   480
ttcaaggacc catttcgagg tggcaacaac atattggtta tgtgtgatac ctacacacca   540
gctggggaac ccatccctac taacaaacgt aacagggctg cacaagtatt cagtgatcca   600
aaggttgtca gccaagtgcc atggtttgga atagaacagg agtacacttt gctccagaga   660
gacgtaaact ggcctcttgg ctggcccgtt ggaggctacc ctgggcccca gggtccatac   720
tactgcgctg taggatcgga caaatcgttt ggccgtgaca tatcagatgc tcactacaag   780
gcatgtcttt atgctggaat taacattagt ggaacaaatg gagaggtcat gcctggtcag   840
tgggagtacc aggttggacc tagtgtcggt attgaagctg gagaccacat atggatttca   900
agatatattc ttgagagaat aacggagcag gctggtgtag tgcttaccct tgaccccaaa   960
ccaattcagg gagactggaa tggagctggg tgccacacaa actacagcac caagagtatg  1020
cgtgaagatg gaggatttga ggtgatcaag aaggcaatcc taaacctatc acttcgccat  1080
gacttgcata taagtgcata tggtgaagga aatgaaagga ggttgacagg tttacacgag  1140
acagctagca ttgacaattt ctcatggggt gtggcaaacc gtggatgctc tattcgggtg  1200
gggcgagaca ccgaggcgaa gggaaaaggc tacttggaag accgtcgccc ggcatcaaac  1260
atggacccgt acgtcgtgac agcgctattg gctgaaacca caattctttg ggagccaacc  1320
ctcgaagcgg aggttcttgc tgctaagaag ttggccctga aggtatgaag aacttggacg  1380
atgaatcggg gcaaataaat cccagcaaaa tttgtttgct gcccaccagt cttgatcttg  1440
tatttcttct gtctggggat tggtctgtac aaatctgcag tttctagaaa accacgccac  1500
cttccattcg ccagttaaca ttttggttga acaccacact tgatctgggt ctgtattttg  1560
agtccatttg tgagtgacag aacggatgat gaaacacatc aggacactt ttaagtttct    1620
tcagtcctgc gtccttccct cgaaataaaa atgtttcctt gttttttatc ccgggct      1677

<210>  46
<211>  1041
<212>  DNA
<213>  Physcomitrella patens

<400>  46
atggccttgg cacagaaggc agagtacatc tggatggatg gacaggaggg tcagaaaggg     60
atccgcttca acgaaatgcg atccaagacc aaggtgatcc aggagcccat caaggccgga   120
tctttggact tccccaagtg gtcattcgac ggttccagca ctgggcaagc agaggggcga   180
ttctccgact gtatcctgaa ccccgtgttt agctgccttg accccatccg cggggacaac   240
cacgtgctgg ttctgtgtga ggtgttgaac cccgacagca caccccatga aaccaacacc   300
cggcgcaaga tcgaggaatt gttgaccccg gatgtgctgg cagaggagac actgttcgga   360
tttgagcagg agtatacgat gttcaacaag gccggaaagg tatacgggtg gccagaagga   420
ggtttcccac acccacaggg ccccttctac tgtggagtgg gtctggaggc ggtttacggg   480
cgacctctgg tggaggcgca catggatgcg tgcatcaagg ctgggctgaa gatcagtggt   540
atcaatgccg aggtcatgcc gggacagtgg gagttccaga tcggccccgc tggacctttg   600
gaagtgggtg accacgtcat gatcgcacgt tggttgcttc accgcttggg tgaggacttc   660
ggcattactt gcacgttcga gcccaagccc atggaaggtg actggaatgg tgctggagct   720
cacaccaact actcgacgaa gtcaatgagg gtggacggcg gtatcaaggc catccacgcc   780
gccattgaga agttgtccaa gaagcacgtg gagcacatct cctcatacgg gttgggcaat   840
gagcgtcgtc tgactggaaa gcacgagact gccaacatca acactttcaa atcggggggtc  900
gcagacagag gtgcatcgat ccgtatccct cttggagtgt ctcttgacgg caagggttat   960
ttggaggatc gcagacccgc ggcgaatgtg gacccttacg tggtggcacg catgctgatc  1020
cagacgactt tgaagaacta g                                             1041
```

<210> 47
<211> 1041
<212> DNA
<213> Physcomitrella patens

<400> 47
atggccttgg cacagaaggc agagtacatc tggatggatg gacaggaggg tcagaaaggg 60
atccgcttta acgaaatgcg atccaagacc aaggtgatcc aggagcccat caaggccgga 120
tctttggact tccccaagtg gtctttcgat ggttctagca ctgggcaagc agaagggcga 180
ttctccgact gcattctgaa ccccgtgttc agctgccccg accccatccg cggggacaac 240
cacgtgctgg ttctgtgcga ggtgttgaac cccgacagca caccccatga aaccaacacc 300
cggcgcaaga tcgaggaact attgaccccg gatgtgctgg cagaggagac actgttcgga 360
tttgagcagg agtacaccat gttcaacaag gccgcgaagg tgtacgggtg gccagaggga 420
ggtttcccac acccacaagg gccctttac tgtggagtgg gtcttgaggc ggtttacggg 480
cgacctctgg tggaggcgca catggatgcg tgcatcaagg ccgggctgaa gatcagtggt 540
attaatgccg aggtgatgcc gggacagtgg gagttccaga tcggccccgc tggacctctg 600
gaggtgggtg accacgtcat ggtcgcgcgt tggctgcttc accgcttggg tgaggacttt 660
ggcattactt gcactttcga gcccaagccc atggaaggag actggaacgg tgctggagct 720
cacaccaact actcgacgaa gtcgatgagg gtggacggcg gtatcaaggc catccacgcg 780
gccattgaga agctgtccaa gaagcacgcg gagcacatct cctcatacgg gttgggcaat 840
gagcgtcgtc tgacaggcaa gcacgagacc gccaacatca acacattcaa gtcgggagtt 900
gcggacagag gtgcgtcgat ccgtattccg cttggagtgt ccctggaggg caaaggttac 960
ttggaagacc gtaggccagc ggcgaacgtg gacccttacg tagtggcccg catgcttatc 1020
caaacgactt tgaagaacta g 1041

<210> 48
<211> 1584
<212> DNA
<213> Pinus taeda

<400> 48
ttcctttgcc ttaaaaaata gaggtttctt aataccccgt cttcgttcat tggtttctat 60
aaattcttcc tcaggttggg gttgctcttt gcatcaattg ctataaattc ttatttcagt 120
ggcctttatt tcgaaatagc agatcaaagg ccttcactgc ttgcagaatt atacttgtgc 180
gggagcctgt gattttgtgg tacatccaag atgtctctac tgacggattt gatcaacttg 240
gatctctctg atgtcactga gaagatcatc gctgagtaca tatggatcgg aggctctggc 300
atggatatcc gcagcaaggc caggacctta tctcacccag ttacggaccc caaagatcta 360
cccaagtgga attatgatgg atccagtact ggacaggctc ctggaaagga cagtgaagtc 420
atcctttacc ctcaggctat cttcagggat ccattccgca ggggtaacaa catcttggtg 480
atttgtgata catatacccc agctggagaa cctattccta ctaacaagag agcaaatgct 540
gctaaaatat ttagccatcc agatgttgtt gccgaggaac catggtacgg gattgaacaa 600
gaatacactc ttctgcaaaa ggatgtgaat tggccgcttg gatggcccgt aggtggttac 660
cctggtcctc agggtcctta ttattgtgga actggagcag acaaagccta cggccgtgat 720
attgtcgatg cccactataa ggcttgcctg tatgcaggaa tcaacattag tggcatcaat 780
ggagaagtca tgcccggtca atgggaattt caagttggcc cgacggttgg tatttcagct 840
ggtgatcaag tctgggctgc acgttacctt cttgagagaa tcacagaagt ggctggtgtt 900
gtcctctcat ttgaccccaa acccattcag ggtgattgga atggtgctgg tgctcacact 960
aactacagta cgaaatcaat gagggaagaa gggggaatta aagtgatcaa aacggccatt 1020
gaaaagttag ggttgaggca taaggaacac attgctgcct atggagaggg caacgagagg 1080
cgtttgactg gccgacatga gacagcagac ataaacacat tttcatgggg agttgcaaat 1140
cgtggagctt ctattcgagt tggacgtgac acggaacgtg aaggcaaagg gtacttcgaa 1200
gaccgcaggc cagcttccaa catggacccc tatatagtaa catctatgat tgctgagaca 1260
accatccttt tgaagtgaga gtaacattgt ttactgaatg aataaagatg ccgatacgat 1320
tgaagtgttc ttgatgctag tcaaattgcg aagggatccc caattgtttg tggggcatat 1380
tctcatttga atttctttat gtgcctaaag tatttcccct atttctgtta ataagaacat 1440
tctggaaata ggacttgaga tttagggtgc tttatattca gtgtctaatt tgtctttcag 1500
attttcattg ttccatgact ctgatatgat tggtgtgcaa ttgaatttaa tgaattcaga 1560
agttctttta ttgcttgtga aaaa 1584

<210> 49
<211> 1304
<212> DNA
<213> Pinus taeda

<400> 49
tttgtatctc gtttcgtatt tcctcactcg caatccatct tatccccgta tcacaaccac 60
attcacaatg gctactccta tcacctcacg gacggagact ctccagaagt atctcaagct 120
tgatcagaag ggtatgatca tggctgagta cgtctgggtt gatgccgatg gtggcactcg 180
ttccaagtct cgcacattgc ccgagaaaga atacaagccc gaggatcttc ccgtttggaa 240
cttcgatggt tcttccacta accaggcccc tggtgacaac tccgatgtct acctccgtcc 300
ctgcgccgtc taccctgatc ccttccgcgg ctctcccaac atcattgttc ttgctgagtg 360
ctggaacgcc gatggcactc ccaacaaata caacttccgt cacgattgcg tgaaggtcat 420
ggacacctac gccgacgacg agccttggtt tggcctcgag caggagtata ccctcctcgg 480
ctctgacaac cgaccctatg gctggcccgc cggtggtttc cctgctcccc aaggcgagta 540
ctactgtggt gtgggcactg gaaaggttgt ccagcgcgat atcgtcgagg cccattataa 600
agcctgtttg tacgccggca tccagatctc tggaacgaac gccgaggtca tgcctgctca 660
gtgggaatat caggtcggcc cctgcactgg cattgcaatg ggcgaccaac tctggatttc 720
gcgattcttt ttacatcgag tcgctgagga attcggtgca aaggtttctt tgcaccccaa 780
gcccattgct ggcgattgga acggagcttt aagtttccct ggtctctgtt tcatatccgt 840
gatactaata tctttacagg gtttgcactc caacttctcc acgaaagcaa tgcgcgagga 900
gggtggtatg aaggttattg aggaggccct gaagaagctt gaacctcacc acgtcgagtg 960
tatcgcagag tatggtgagg ataacgaatt gcgtttgacc ggccgtcacg agacgggatc 1020
catcgacagc ttttcttggg gtgtcgccaa ccgtggcaca agcatccgcg tgccacgcga 1080
aacggctgct aagggctatg gctactttga ggaccgccgt cctgcttcca acgccgatcc 1140
ctaccgcgtt accaaggttc tcctccaatt ttctatggct tagagcgagt tttagagttt 1200
ttgctttctg atgacatggt ctacggcgtg aaggtttggg aaactattga ttacatagat 1260
agcatgaaag cttgtcctga aggacagtaa tgacaaccaa tcag 1304

<210> 50
<211> 1251
<212> DNA
<213> Phaedactylum tricornutum

<400> 50
atgaaattaa acattgctgc tattgcgcta tttgctgcat cggcttcggc ctttgctcct 60
cgatttgcgt cgcctcgctc ccacgctacc gtactgtccg cggtcctcga agaacgaacg 120
gggcagtctc agctcgaccc tgccgtcatc gagcgatacg ctgcgcttcc ctacccggat 180
gataccgttc ttgccgaata tgtatgggtc gatgccgtgg gtaacacgcg ctccaagaca 240
cgcacgcttc ctgccaagaa ggctgcatct gtcgaggctc ttcccaagtg gaactttgat 300
ggctcttcga cggaccaggc tcccggagac gactcggaag ttattctacg tccttgccgt 360
atcttcaaag atcctttccg acctcgtaac gatggtctcg acaatgttct cgtcatgtgc 420
gattgctaca caccgaacgg cgaagcaatt cccacgaacc accgtgccaa ggctatggaa 480
tcttttgaat ccagggaaga cgaagagatc tggttcgggc tcgaacagga atttacgctg 540
ttcaacttgg acaagcgtac ccctctcggc tggccagaag gcggcatgcc caatcgccct 600
caaggacctt actattgtag tgttggaccc gaaaataact tcggacgtca cattacggaa 660
tccatgtacc gggcttgtct ctacgcaggc atcaacattt cgggaacgaa tggagaagtc 720
atgcccggac aacaggaata ccaggttgga ccctgcgtgg gaattgacgc aggggatcag 780
ctcatgatga gccgatacat tcttcagcgt gtctgcgagg atttccaggt atattgtaca 840
ctccatccca agcccatcgt tgacggtgac tggaacggcg ccggcatgca caccaatgtt 900
tctactaaat ccatgcgcga ggaaggtggc cttgaagtta tcaaaaaggc gatttacaag 960
ttgggggcca agcaccttga gcacatcgct gtgtacggtg aaggtaacga acttcgcctg 1020
acaggcaagc acgaaacggc cagcatggac aagttttgct acggtgttgc caaccgtgga 1080
gcgtccattc gaattggtcg cgacaccgaa gccgagggga agggatactt cgaggatcgt 1140
cgtccgtcat ctaacgccga tccttacatt gttacgggaa agatcatgaa tacaattatg 1200
gaagatgtgg aagtccccga tattgctcca atggacaagg ccgtggccta a 1251

<210> 51
<211> 1768
<212> DNA
<213> Zea mays

<400> 51
caacgacagc gagccctatc ccctcagcaa aagccagatg cctgttgccg tcgcggccac 60
tggatgccaa gtacttttta tatacgccgt ccgcgcccac gacccccgag acccgcctcc 120
cctcgtcgtc tcgtctcgcc tcgcgtcgtc tgcgctcgcg gctcgtcaca ggtgaggtct 180
cggcgggaga ggggcggcgg ccggtccgtg tccgtgtccg tcgacggttg gttcgggaat 240

```
ggcgcaggcg gtggtgccgg cgatgcagtg ccgggtcgga gtgaaggcgg cggcggggag    300
ggtgtggagc gccggcagga ctaggaccgg ccgcggcggc gcctcgccgg ggttcaaggt    360
catggccgtc agcacgggca gcaccggggt ggtgccgcgc ctcgagcagc tgctcaacat    420
ggacaccacg ccctacaccg acaaggtcat cgccgagtac atctgggtcg gaggatctgg    480
aatcgacatc cgaagcaaat caaggacgat ttcgaaaccc gtggaggatc cctcggaact    540
accaaaatgg aactacgatg gatctagcac aggacaagcc ccgggagaag acagtgaagt    600
cattctatac ccccaggcta tcttcaagga cccattccga ggtggcaaca acgttttggt    660
tatctgtgac acctacacgc cacaggggga acccccttcca actaacaaac gccacagggc    720
tgcgcaaatt ttcagtgacc caaaggtcgc tgaacaagtg ccatggtttg gcatagagca    780
agagtacact ttgctccaga aagatgtaaa ttggcctctt ggttggcctg ttggaggctt    840
ccctggtccc cagggtccat actactgtgc cgtaggagcc gacaaatcat ttggccgtga    900
catatcagat gctcactaca aggcatgcct ctacgctgga atcaacatta gtggaacaaa    960
cggggaggtc atgcctggtc agtgggagta ccaagttgga cctagtgttg gtattgaagc   1020
aggagatcac atatggattt cgagatacat tctcgagaga atcacagagc aagctggggt   1080
tgtccttacc cttgatccaa aaccaattca gggtgactgg aacggagctg gctgccacac   1140
aaattacagc acaaagacca tgcgcgaaga cggcgggttt gaagagatca agagagcaat   1200
cctgaacctt tctctgcgcc atgatctgca tattagtgca tacggagaag gaaatgaaag   1260
aagactgact gggaaacatg agactgcgag catcggaacg ttctcatggg gtgtggcaaa   1320
ccgcggctgc tctatccgtg tggggcggga taccgaggca aaagggaaag gttacctgga   1380
agaccgtcgg ccggcatcaa acatggaccc gtacattgtg acggggctac tggccgagac   1440
cacgatcctc tggcagccat ccctcgaggc ggaggctctt gccgccaaga agctggcgct   1500
gaaggtgtga agcagctgaa ggatggttca ggcaccaata taaaccggtc cgcgacaaga   1560
ttgatctttg tgtccatggc gtgggtcttg cgactctctg ctcggcggtg ccactctgta   1620
caaaatcacg gctgtctttg attcatcgga tattcggata cgtttgtttg ttactttttg   1680
cttggacacc caccatgttt ggaacttttt tgggctccgt ttgggggctg aacgatggtc   1740
agtggaaatt ttaaaaattc gtcgtctc                                      1768
```

<210> 52
<211> 1531
<212> DNA
<213> Zea mays

<400> 52

```
cacgccacat cctcccctcc ttcctccttg ggttcccagc ccgtgcgccc gcctgtcgca     60
gtcgcaccgc agccgccggc catggcctgc ctcaccgacc tcgtcaacct caacctctcg    120
gacaccacag agaagatcat cgccgagtac atatggatcg gtggatctgg catggatctc    180
aggagcaaag ccaggaccct cccgggcccg gtgaccgatc ccagcaagct gcccaagtgg    240
aactacgacg gctccagcac cggccaggcc cccggcgagg acagcgaggt catcctgtac    300
ccgcaggcca tcttcaagga cccattcagg aggggcaaca acatccttgt catgtgcgat    360
tgctacaccc cagctggcga gccaattccc accaacaaga ggtacagcgc cgccaagatc    420
ttcagcagcc ttgaggtcgc tgccgaggag ccctggtatg gtatcgagca ggagtacacc    480
ctccttcaga aggacaccaa ctggcccctc gggtggccta ttggcggctt ccctggccct    540
cagggtcctt actactgtgg aatcggcgcg gagaaatcgt tcgggcgtga catagtcgac    600
gcccactaca aggcctgcct gtacgcaggc atcaacatca gtggcatcaa cggggaggtc    660
atgccggggc agtgggagtt ccaggtcgga ccgtccgtcg gcatctcttc gggcgatcag    720
gtgtgggttg ctcgctacat tcttgagagg atcaccgaga tcgccggcgt ggtggtgacg    780
ttcgacccga agccgatccc gggcgactgg aacggcgcgg gcgcccacac caactacagc    840
accgagtcca tgaggaagga gggcgggtac gaggtgatca aggcggccat cgagaagctg    900
aagctgcggc acaaggagca catcgcggcc tacggcgagg gcaacgagcg ccggctcacc    960
ggcaggcacg agaccgccga catcaacacc ttcagctggg gagtcgccaa ccgtggcgcg   1020
tcggtgcgcg tgggccgcga gacggagcag aacggcaagg gctacttcga ggaccgccgg   1080
ccggcgtcca acatggaccc ctacgtggtc acctccatga tcgccgagac caccatcgtc   1140
tggaagccct gaggcacccc gtggccgtgt cgtgtcggtt tgctccgcgt acggcgctgg   1200
ccgttgcatc gcagggccca gcggttgcgc aactattttc ccttccccgt tctgtttgct   1260
tgtactacta ctctaccgct agtcctgcat agcattttag ctagaacaca acaacagcca   1320
aaaaaaagta ttgttgcttg cttcgacgct tgccaccact tccattccat gccgtccgtc   1380
cgcttccttc ctgtgtaatc ctcctccaat aatagacgtg ccatgttgca tcctctattc   1440
ctctgcattg tataaaagtg gtgtaattct tttgctacgc ctccaatgtc tgggctttta   1500
gctgctgatg cgatgtcaga ttctgtcacg g                                  1531
```

<210> 53
<211> 354
<212> PRT

<213> Hordeum vulgare

<400> 53

```
Met Ala Ser Leu Ala Asp Leu Val Asn Leu Asn Leu Ser Asp Cys Thr
1               5                   10                  15
Asp Lys Val Ile Val Glu Tyr Leu Trp Val Gly Gly Ser Gly Ile Asp
            20                  25                  30
Ile Arg Ser Lys Ala Arg Thr Val Asn Gly Pro Ile Thr Asp Ala Ser
        35                  40                  45
Gln Leu Pro Lys Trp Asn Tyr Asp Gly Ser Ser Thr Gly Gln Ala Pro
    50                  55                  60
Gly Glu Asp Ser Glu Val Ile Leu Tyr Pro Gln Ala Ile Phe Lys Asp
65                  70                  75                  80
Pro Phe Arg Arg Gly Asp Asn Ile Leu Val Met Cys Asp Cys Tyr Thr
                85                  90                  95
Pro Gln Gly Val Pro Ile Pro Thr Asn Lys Arg His Asn Ala Ala Lys
            100                 105                 110
Ile Phe Asn Ser Ala Lys Val Ala Ala Glu Glu Thr Trp Tyr Gly Ile
        115                 120                 125
Glu Gln Glu Tyr Thr Leu Leu Gln Lys Asp Val Asn Trp Pro Leu Gly
    130                 135                 140
Trp Pro Ile Gly Gly Tyr Pro Gly Pro Gln Gly Pro Tyr Tyr Cys Ala
145                 150                 155                 160
Ala Gly Ala Asp Lys Ala Phe Gly Arg Asp Ile Val Asp Ala His Tyr
                165                 170                 175
Lys Ala Cys Leu Tyr Ala Gly Ile Asn Ile Ser Gly Ile Asn Gly Glu
            180                 185                 190
Val Met Pro Gly Gln Trp Glu Phe Gln Val Gly Pro Ser Val Gly Ile
        195                 200                 205
Ala Ala Ser Asp Gln Leu Trp Val Ala Arg Tyr Ile Leu Glu Arg Ile
    210                 215                 220
Thr Glu Val Ala Gly Val Val Leu Ser Leu Asp Pro Lys Pro Ile Pro
225                 230                 235                 240
Gly Asp Trp Asn Gly Ala Gly Ala His Thr Asn Tyr Ser Thr Lys Ser
                245                 250                 255
Met Arg Gln Ala Gly Gly Tyr Glu Val Ile Lys Lys Ala Ile Glu Lys
            260                 265                 270
Leu Gly Lys Arg His Met Gln His Ile Ala Ala Tyr Gly Glu Gly Asn
        275                 280                 285
Glu Arg Arg Leu Thr Gly His His Glu Thr Ala Asp Ile Asn Thr Phe
    290                 295                 300
Lys Trp Gly Val Ala Asp Arg Gly Ala Ser Ile Arg Val Gly Arg Asp
305                 310                 315                 320
Thr Glu Lys Asp Gly Lys Gly Tyr Phe Glu Asp Arg Arg Pro Ala Ser
                325                 330                 335
Asn Met Asp Pro Tyr Val Val Thr Ser Met Ile Ala Glu Thr Thr Leu
            340                 345                 350
Leu Leu
```

<210> 54
<211> 427
<212> PRT
<213> Hordeum vulgare

<400> 54

```
Met Ala Gln Ala Val Val Gln Ala Met Gln Cys Gln Val Gly Val Arg
1               5                   10                  15
Gly Arg Thr Ala Val Pro Ala Arg Gln Pro Ala Gly Arg Val Trp Gly
            20                  25                  30
Val Arg Arg Ala Ala Arg Ala Thr Ser Gly Phe Lys Val Leu Ala Leu
        35                  40                  45
Gly Pro Glu Thr Thr Gly Val Ile Gln Arg Met Gln Gln Leu Leu Asp
```

50 55 60
Met Asp Thr Thr Pro Phe Thr Asp Lys Ile Ile Ala Glu Tyr Ile Trp
65 70 75 80
Val Gly Gly Ser Gly Ile Asp Leu Arg Ser Lys Ser Arg Thr Ile Ser
85 90 95
Lys Pro Val Glu Asp Pro Ser Glu Leu Pro Lys Trp Asn Tyr Asp Gly
100 105 110
Ser Ser Thr Gly Gln Ala Pro Gly Glu Asp Ser Glu Val Ile Leu Tyr
115 120 125
Pro Gln Ala Ile Phe Lys Asp Pro Phe Arg Gly Gly Asn Asn Ile Leu
130 135 140
Val Ile Cys Asp Thr Tyr Thr Pro Gln Gly Glu Pro Ile Pro Thr Asn
145 150 155 160
Lys Arg His Met Ala Ala Gln Ile Phe Ser Asp Pro Lys Val Thr Ser
165 170 175
Gln Val Pro Trp Phe Gly Ile Glu Gln Glu Tyr Thr Leu Met Gln Arg
180 185 190
Asp Val Asn Trp Pro Leu Gly Trp Pro Val Gly Gly Tyr Pro Gly Pro
195 200 205
Gln Gly Pro Tyr Tyr Cys Ala Val Gly Ser Asp Lys Ser Phe Gly Arg
210 215 220
Asp Ile Ser Asp Ala His Tyr Lys Ala Cys Leu Tyr Ala Gly Ile Glu
225 230 235 240
Ile Ser Gly Thr Asn Gly Glu Val Met Pro Gly Gln Trp Glu Tyr Gln
245 250 255
Val Gly Pro Ser Val Gly Ile Asp Ala Gly Asp His Ile Trp Ala Ser
260 265 270
Arg Tyr Ile Leu Glu Arg Ile Thr Glu Gln Ala Gly Val Val Leu Thr
275 280 285
Leu Asp Pro Lys Pro Ile Gln Gly Asp Trp Asn Gly Ala Gly Cys His
290 295 300
Thr Asn Tyr Ser Thr Leu Ser Met Arg Glu Asp Gly Gly Phe Asp Val
305 310 315 320
Ile Lys Lys Ala Ile Leu Asn Leu Ser Leu Arg His Asp Leu His Ile
325 330 335
Ala Ala Tyr Gly Glu Gly Asn Glu Arg Arg Leu Thr Gly Leu His Glu
340 345 350
Thr Ala Ser Ile Ser Asp Phe Ser Trp Gly Val Ala Asn Arg Gly Cys
355 360 365
Ser Ile Arg Val Gly Arg Asp Thr Glu Ala Lys Gly Lys Gly Tyr Leu
370 375 380
Glu Asp Arg Arg Pro Ala Ser Asn Met Asp Pro Tyr Thr Val Thr Ala
385 390 395 400
Leu Leu Ala Glu Thr Thr Ile Leu Trp Glu Pro Thr Leu Glu Ala Glu
405 410 415
Ala Leu Ala Ala Lys Lys Leu Ala Leu Lys Val
420 425

<210> 55
<211> 1455
<212> PRT
<213> Hordeum vulgare

<400> 55
Gly Cys Thr Cys Gly Ala Gly Cys Thr Gly Cys Ala Cys Ala Cys Cys
1 5 10 15
Thr Cys Ala Thr Cys Thr Cys Ala Thr Cys Ala Thr Cys Gly Thr Cys
20 25 30
Thr Thr Cys Cys Cys Cys Cys Cys Ala Thr Thr Gly Cys Cys Ala Thr
35 40 45
Cys Gly Ala Cys Cys Thr Cys Cys Cys Thr Cys Cys Cys Thr Gly Cys
50 55 60
Gly Ala Gly Cys Ala Gly Cys Ala Gly Cys Ala Gly Cys Ala Gly Cys

```
65                  70                  75                  80
Ala Ala Thr Gly Gly Cys Cys Ala Gly Cys Cys Thr Cys Gly Cys Cys
                85                  90                  95
Gly Ala Cys Cys Thr Cys Gly Thr Thr Ala Ala Thr Cys Thr Cys Ala
            100                 105                 110
Ala Cys Cys Thr Cys Ala Gly Cys Gly Ala Cys Thr Gly Cys Ala Cys
        115                 120                 125
Gly Gly Ala Cys Ala Ala Gly Gly Thr Cys Ala Thr Cys Gly Thr Cys
    130                 135                 140
Gly Ala Gly Thr Ala Cys Cys Thr Cys Thr Gly Gly Gly Thr Thr Gly
145                 150                 155                 160
Gly Ala Gly Gly Ala Thr Cys Thr Gly Gly Thr Ala Thr Cys Gly Ala
                165                 170                 175
Cys Ala Thr Cys Ala Gly Gly Ala Gly Cys Ala Ala Ala Gly Cys Ala
            180                 185                 190
Ala Gly Gly Ala Cys Gly Gly Thr Gly Ala Ala Cys Gly Gly Ala Cys
        195                 200                 205
Cys Cys Ala Thr Cys Ala Cys Cys Gly Ala Cys Gly Cys Gly Ala Gly
    210                 215                 220
Cys Cys Ala Gly Cys Thr Gly Cys Cys Cys Ala Ala Gly Thr Gly Gly
225                 230                 235                 240
Ala Ala Cys Thr Ala Cys Gly Ala Cys Gly Gly Cys Thr Cys Cys Ala
                245                 250                 255
Gly Cys Ala Cys Cys Gly Gly Cys Cys Ala Gly Gly Cys Thr Cys Cys
            260                 265                 270
Cys Gly Gly Ala Gly Ala Gly Gly Ala Cys Ala Gly Cys Gly Ala Ala
        275                 280                 285
Gly Thr Cys Ala Thr Cys Cys Thr Cys Thr Ala Cys Cys Cys Cys Cys
    290                 295                 300
Ala Gly Gly Cys Cys Ala Thr Thr Thr Cys Ala Ala Gly Gly Ala
305                 310                 315                 320
Cys Cys Cys Gly Thr Thr Cys Ala Gly Gly Ala Gly Gly Gly Gly Thr
                325                 330                 335
Gly Ala Cys Ala Ala Cys Ala Thr Cys Cys Thr Thr Gly Thr Thr Ala
            340                 345                 350
Thr Gly Thr Gly Cys Gly Ala Cys Thr Gly Cys Thr Ala Cys Ala Cys
        355                 360                 365
Ala Cys Cys Ala Cys Ala Ala Gly Gly Thr Gly Thr Gly Cys Cys Ala
    370                 375                 380
Ala Thr Thr Cys Cys Cys Ala Cys Thr Ala Ala Cys Ala Ala Gly Ala
385                 390                 395                 400
Gly Gly Cys Ala Cys Ala Ala Thr Gly Cys Thr Gly Cys Cys Ala Ala
                405                 410                 415
Gly Ala Thr Cys Thr Thr Cys Ala Ala Cys Ala Gly Cys Gly Cys Thr
            420                 425                 430
Ala Ala Gly Gly Thr Thr Gly Cys Ala Gly Cys Thr Gly Ala Gly Gly
        435                 440                 445
Ala Gly Ala Cys Ala Thr Gly Gly Thr Ala Thr Gly Gly Thr Ala Thr
    450                 455                 460
Thr Gly Ala Gly Cys Ala Gly Gly Ala Gly Thr Ala Cys Ala Cys Ala
465                 470                 475                 480
Cys Thr Cys Cys Thr Cys Cys Ala Gly Ala Ala Gly Gly Ala Thr Gly
                485                 490                 495
Thr Gly Ala Ala Cys Thr Gly Gly Cys Cys Thr Cys Thr Thr Gly Gly
            500                 505                 510
Cys Thr Gly Gly Cys Cys Ala Ala Thr Thr Gly Gly Thr Gly Gly Cys
        515                 520                 525
Thr Ala Cys Cys Cys Thr Gly Gly Thr Cys Cys Thr Cys Ala Gly Gly
    530                 535                 540
Gly Ala Cys Cys Ala Thr Ala Cys Thr Ala Cys Thr Gly Cys Gly Cys
545                 550                 555                 560
Cys Gly Cys Cys Gly Gly Thr Gly Cys Cys Gly Ala Cys Ala Ala Gly
                565                 570                 575
```

Gly Cys Gly Thr Thr Cys Gly Gly Gly Cys Gly Thr Gly Ala Cys Ala
580 585 590
Thr Cys Gly Thr Gly Gly Ala Cys Gly Cys Cys Cys Ala Cys Thr Ala
595 600 605
Cys Ala Ala Gly Gly Cys Gly Thr Gly Cys Cys Thr Cys Thr Ala Cys
610 615 620
Gly Cys Cys Gly Gly Gly Ala Thr Cys Ala Ala Cys Ala Thr Cys Ala
625 630 635 640
Gly Cys Gly Gly Cys Ala Thr Cys Ala Ala Cys Gly Gly Gly Gly Ala
645 650 655
Gly Gly Thr Cys Ala Thr Gly Cys Cys Cys Gly Gly Cys Cys Ala Gly
660 665 670
Thr Gly Gly Gly Ala Gly Thr Thr Cys Cys Ala Ala Gly Thr Thr Gly
675 680 685
Gly Gly Cys Cys Gly Thr Cys Cys Gly Thr Cys Gly Gly Gly Ala Thr
690 695 700
Cys Gly Cys Cys Gly Cys Cys Thr Cys Cys Gly Ala Cys Cys Ala Gly
705 710 715 720
Cys Thr Gly Thr Gly Gly Gly Thr Gly Gly Cys Gly Cys Gly Cys Thr
725 730 735
Ala Cys Ala Thr Cys Cys Thr Cys Gly Ala Gly Ala Gly Gly Ala Thr
740 745 750
Cys Ala Cys Ala Gly Ala Gly Gly Thr Thr Gly Cys Cys Gly Gly Gly
755 760 765
Gly Thr Gly Gly Thr Gly Cys Thr Gly Thr Cys Cys Cys Thr Gly Gly
770 775 780
Ala Cys Cys Cys Gly Ala Ala Gly Cys Cys Gly Ala Thr Cys Cys Cys
785 790 795 800
Gly Gly Gly Thr Gly Ala Cys Thr Gly Gly Ala Ala Cys Gly Gly Cys
805 810 815
Gly Cys Gly Gly Gly Cys Gly Cys Gly Cys Ala Cys Ala Cys Cys Ala
820 825 830
Ala Cys Thr Ala Cys Ala Gly Cys Ala Cys Cys Ala Ala Gly Thr Cys
835 840 845
Cys Ala Thr Gly Ala Gly Gly Cys Ala Gly Gly Cys Cys Gly Gly Cys
850 855 860
Gly Gly Cys Thr Ala Cys Gly Ala Gly Gly Thr Gly Ala Thr Cys Ala
865 870 875 880
Ala Gly Ala Ala Gly Gly Cys Cys Ala Thr Cys Gly Ala Gly Ala Ala
885 890 895
Gly Cys Thr Thr Gly Gly Cys Ala Ala Gly Cys Gly Cys Cys Ala Cys
900 905 910
Ala Thr Gly Cys Ala Gly Cys Ala Cys Ala Thr Cys Gly Cys Cys Gly
915 920 925
Cys Cys Thr Ala Cys Gly Gly Cys Gly Ala Gly Gly Gly Cys Ala Ala
930 935 940
Cys Gly Ala Gly Cys Gly Cys Cys Gly Cys Cys Thr Cys Ala Cys Cys
945 950 955 960
Gly Gly Cys Cys Ala Cys Cys Ala Cys Gly Ala Gly Ala Cys Cys Gly
965 970 975
Cys Cys Gly Ala Cys Ala Thr Cys Ala Ala Cys Ala Cys Cys Thr Thr
980 985 990
Cys Ala Ala Ala Thr Gly Gly Gly Gly Cys Gly Thr Gly Gly Cys Gly
995 1000 1005
Gly Ala Cys Cys Gly Cys Gly Gly Cys Gly Cys Gly Thr Cys Cys
1010 1015 1020
Ala Thr Cys Cys Gly Cys Gly Thr Gly Gly Gly Gly Cys Gly Cys
1025 1030 1035
Gly Ala Cys Ala Cys Gly Gly Ala Gly Ala Ala Gly Gly Ala Cys
1040 1045 1050
Gly Gly Cys Ala Ala Gly Gly Gly Cys Thr Ala Cys Thr Thr Cys
1055 1060 1065
Gly Ala Gly Gly Ala Cys Cys Gly Cys Ala Gly Gly Cys Cys Gly

```
    1070                1075                1080
Gly Cys  Cys Thr Cys Cys Ala  Ala Cys Ala Thr Gly  Gly Ala Cys
    1085                1090                1095
Cys Cys  Cys Thr Ala Cys Gly  Thr Cys Gly Thr Cys  Ala Cys Cys
    1100                1105                1110
Thr Cys  Cys Ala Thr Gly Ala  Thr Cys Gly Cys Cys  Gly Ala Gly
    1115                1120                1125
Ala Cys  Cys Ala Cys Gly Cys  Thr Thr Cys Thr Cys  Cys Thr Cys
    1130                1135                1140
Thr Gly  Ala Gly Cys Ala Cys  Ala Cys Gly Gly Cys  Cys Gly Gly
    1145                1150                1155
Cys Ala  Ala Thr Gly Cys Cys  Thr Ala Cys Thr Cys  Cys Ala Cys
    1160                1165                1170
Cys Gly  Cys Cys Ala Gly Ala  Thr Gly Ala Cys Ala  Cys Thr Thr
    1175                1180                1185
Thr Gly  Gly Gly Cys Ala Gly  Gly Cys Thr Cys Thr  Cys Gly Thr
    1190                1195                1200
Cys Thr  Cys Gly Ala Cys Thr  Cys Thr Cys Thr Cys  Gly Ala Thr
    1205                1210                1215
Cys Gly  Ala Gly Gly Gly Thr  Gly Gly Thr Gly Ala  Thr Thr Gly
    1220                1225                1230
Ala Thr  Thr Thr Cys Thr Gly  Cys Ala Ala Ala Ala  Cys Ala Thr
    1235                1240                1245
Thr Thr  Cys Cys Cys Gly Thr  Thr Thr Cys Cys Gly  Thr Thr Thr
    1250                1255                1260
Cys Thr  Thr Thr Thr Gly Cys  Ala Ala Thr Thr Gly  Cys Ala Ala
    1265                1270                1275
Gly Gly  Thr Cys Thr Ala Gly  Thr Cys Thr Gly Thr  Thr Thr Thr
    1280                1285                1290
Thr Gly  Gly Gly Gly Cys Gly  Thr Gly Cys Cys Thr  Thr Thr Gly
    1295                1300                1305
Gly Thr  Ala Thr Cys Thr Thr  Thr Cys Ala Thr Ala  Gly Thr Ala
    1310                1315                1320
Gly Thr  Ala Cys Gly Thr Cys  Thr Ala Cys Thr Gly  Cys Thr Cys
    1325                1330                1335
Thr Thr  Cys Ala Gly Gly Ala  Thr Ala Ala Gly Ala  Ala Gly Ala
    1340                1345                1350
Gly Thr  Cys Thr Thr Cys Ala  Gly Thr Gly Thr Ala  Cys Thr Cys
    1355                1360                1365
Thr Gly  Ala Ala Ala Ala Thr  Ala Ala Thr Gly Thr  Thr Gly Thr
    1370                1375                1380
Thr Thr  Cys Cys Gly Cys Ala  Thr Thr Cys Thr Gly  Ala Thr Ala
    1385                1390                1395
Ala Ala  Ala Thr Gly Gly Ala  Ala Thr Cys Ala Thr  Gly Gly Ala
    1400                1405                1410
Ala Cys  Cys Gly Gly Thr Thr  Gly Thr Gly Ala Thr  Thr Cys Thr
    1415                1420                1425
Gly Thr  Cys Thr Gly Thr Thr  Cys Ala Ala Ala Ala  Ala Ala Ala
    1430                1435                1440
Ala Ala  Ala Ala Ala Ala Ala  Ala Ala Ala Ala Ala
    1445                1450                1455
```

<210>  56
<211>  1775
<212>  DNA
<213>  Hordeum vulgare

<220>
<221>  misc_feature
<222>  (1724)..(1724)
<223>  n is a, c, g, or t

<400>  56

```
tcgcccctct cctccctcgc cccctcgcct cgctcctctc gcccgcgtcg ctgtctctgg      60
tttcggggcg gcggagtcgc tgtacgtaag taagtaagta cgtagagacg acgatggcgc     120
aggcggttgt gcaggcgatg cagtgccagg tgggggtgag gggcaggacg gccgtcccgg     180
cgaggcagcc cgcgggcagg gtgtggggcg tcaggagggc cgcccgcgcc acctccgggt     240
tcaaggtgct ggcgctcggc ccggagacca ccggggtcat ccagaggatg cagcagctgc     300
tcgacatgga caccacgccc ttcaccgaca agatcatcgc cgagtacatc tgggttggag     360
gatctggaat tgacctcaga agcaaatcaa ggacgatttc gaagccagtg gaggacccgt     420
cagagctgcc gaaatggaac tacgacggat cgagcacggg gcaggctcct ggggaagaca     480
gtgaagtcat cctataccca caggccatat tcaaggaccc attccgagga ggcaacaaca     540
tactggttat ctgtgacacc tacacaccac agggggaacc catccctact aacaaacgcc     600
acatggctgc acaaatcttc agtgacccca aggtcacttc acaagtgcca tggttcggaa     660
tcgaacagga gtacactctg atgcagaggg atgtgaactg gcctcttggc tggcctgttg     720
gagggtaccc tggcccccag ggtccatact actgcgccgt aggatcagac aagtcatttg     780
gccgtgacat atcagatgct cactacaagg cgtgccttta cgctggaatt gaaatcagtg     840
gaacaaacgg ggaggtcatg cctggtcagt gggagtacca ggttggaccc agcgttggta     900
ttgatgcagg agaccacata tgggcttcca gatacattct cgagagaatc acggagcaag     960
ctggtgtggt gctcaccctt gacccaaaac caatccaggg tgactggaac ggagctggct    1020
gccacacaaa ctacagcaca ttgagcatgc gcgaggatgg aggtttcgac gtgatcaaga    1080
aggcaatcct gaacctttca cttcgccatg acttgcacat agccgcatat ggtgaaggaa    1140
acgagcggag gttgacaggg ctacacgaga cagctagcat atcagacttc tcatggggtg    1200
tggcgaaccg tggctgctct attcgtgtgg ggcgagacac cgaggcgaag ggcaaaggat    1260
acctggagga ccgtcgcccg gcctccaaca tggacccgta caccgtgacg gcgctgctgg    1320
ccgagaccac gatcctgtgg gagccgaccc tcgaggcgga ggccctcgct gccaagaagc    1380
tggcgctgaa ggtatgaagg acctgaaaaa aggacgaatt cttcttccgg ggaaaagaaa    1440
ataaatcggc gagcggcgag accgttggcc gtccattctt gttgatcctg tggttccgtc    1500
ggggcactgc ctgtacaaaa tcctcacagt ttgtagaacc actcccgcgt gtgtttttcc    1560
gcttgaactg agtccatttg atctgttggg actgtacact cactgtacct gagtccatat    1620
ggagaactac gttattataa aacgataatg aatcgcaaaa aaaaaaaaaa aaaaagtcac    1680
aaaacagaaa aaaaaaaact caaggggggg cccgggcccc agtnccgcct atcggaggtc    1740
tgtgtacatc cattggcccc ccctgcacaa ccccc                               1775
```

<210> 57
<211> 1428
<212> DNA
<213> Arabidopsis thaliana

<400> 57

```
atggagcatt ctagtgattt gactgttgaa gctatgatgc ttgactctaa agcttctgat      60
cttgacaaag aagaacgtcc tgaggtactc tctttaatcc caccatatga agggaaatct     120
gtgcttgaac ttggagctgg tattggtcgt ttcactggtg aattggctca aaggctggt     180
gaagttatcg ctcttgacat catcgaaagc gcgattcaga agaatgaaag tgttaatggg     240
cattacaaga acatcaagtt tatgtgtgct gatgtaacat ctccagactt gaaaatcaaa     300
gatggatcta tcgacttgat tttctcaaac tggttgctca tgtatctctc tgataaagag     360
gtggaactaa tggcagagag aatgattgga tgggtcaagc caggggggata cattttcttc     420
agagaatctt gcttccatca atctggggac agcaagcgaa agtcaaaccc cactcactac     480
cgtgaaccca gattctacac aaaggttttc caggaatgtc agacacgtga tgcttctggc     540
aattcatttg agctctctat ggttggctgc aaatgcattg gggcttatgt gaagaacaag     600
aagaatcaga atcagatttg ctggatatgg caaaaagtca gcgtggagag tgacaaggat     660
ttccagcgtg tcttggacaa tgttcaatac aagtctagtg ggatcttgcg ctatgagcgt     720
gtctttgggg aaggatatgt gagcactggt ggatttgaga caactaaaga atttgtggcg     780
aagatggacc ttaaaccggg acagaaagtc ctagatgttg gttgtggtat cggtggaggt     840
gacttctaca tggctgagaa tttcgatgtt catgttgttg gaatcgatct gtcggtcaac     900
atgatctctt tcgcactgga gcgggccatt ggactcaaat gctcagtcga gtttgaagtc     960
gctgattgca ccaccaaaac atatcccgat aattcctttg atgtcattta cagccgtgac    1020
actattctgc acatccaaga caagccagct ctattcagga cattcttcaa gtggcttaaa    1080
ccagggggta aagttctcat cactgactat tgtagaagtg ctgaaactcc gtctcctgaa    1140
ttcgcagagt acataaaaca aagaggatat gatctacatg atgttcaagc ttacggacag    1200
atgctgaaag acgcaggctt tgacgacgtt atcgctgagg accgtactga tcagtttgta    1260
caagtcctca ggcgtgaatt agaaaaagtg gagaaagaaa aggaagaatt catcagcgac    1320
ttctcagaag aggattacaa tgacattgtt ggaggatggt cggcaaagct tgaaaggact    1380
gcatctggtg aacagaaatg gggattattc atagccgaca agaagtaa                 1428
```

<210> 58

<211> 475
<212> PRT
<213> Arabidopsis thaliana

<400> 58
```
Met Glu His Ser Ser Asp Leu Thr Val Glu Ala Met Met Leu Asp Ser
1               5                   10                  15
Lys Ala Ser Asp Leu Asp Lys Glu Glu Arg Pro Glu Val Leu Ser Leu
            20                  25                  30
Ile Pro Pro Tyr Glu Gly Lys Ser Val Leu Glu Leu Gly Ala Gly Ile
        35                  40                  45
Gly Arg Phe Thr Gly Glu Leu Ala Gln Lys Ala Gly Glu Val Ile Ala
    50                  55                  60
Leu Asp Ile Ile Glu Ser Ala Ile Gln Lys Asn Glu Ser Val Asn Gly
65                  70                  75                  80
His Tyr Lys Asn Ile Lys Phe Met Cys Ala Asp Val Thr Ser Pro Asp
                85                  90                  95
Leu Lys Ile Lys Asp Gly Ser Ile Asp Leu Ile Phe Ser Asn Trp Leu
            100                 105                 110
Leu Met Tyr Leu Ser Asp Lys Glu Val Glu Leu Met Ala Glu Arg Met
        115                 120                 125
Ile Gly Trp Val Lys Pro Gly Gly Tyr Ile Phe Phe Arg Glu Ser Cys
    130                 135                 140
Phe His Gln Ser Gly Asp Ser Lys Arg Lys Ser Asn Pro Thr His Tyr
145                 150                 155                 160
Arg Glu Pro Arg Phe Tyr Thr Lys Val Phe Gln Glu Cys Gln Thr Arg
                165                 170                 175
Asp Ala Ser Gly Asn Ser Phe Glu Leu Ser Met Val Gly Cys Lys Cys
            180                 185                 190
Ile Gly Ala Tyr Val Lys Asn Lys Lys Asn Gln Asn Gln Ile Cys Trp
        195                 200                 205
Ile Trp Gln Lys Val Ser Val Glu Ser Asp Lys Asp Phe Gln Arg Val
    210                 215                 220
Leu Asp Asn Val Gln Tyr Lys Ser Ser Gly Ile Leu Arg Tyr Glu Arg
225                 230                 235                 240
Val Phe Gly Glu Gly Tyr Val Ser Thr Gly Gly Phe Glu Thr Thr Lys
                245                 250                 255
Glu Phe Val Ala Lys Met Asp Leu Lys Pro Gly Gln Lys Val Leu Asp
            260                 265                 270
Val Gly Cys Gly Ile Gly Gly Gly Asp Phe Tyr Met Ala Glu Asn Phe
        275                 280                 285
Asp Val His Val Val Gly Ile Asp Leu Ser Val Asn Met Ile Ser Phe
    290                 295                 300
Ala Leu Glu Arg Ala Ile Gly Leu Lys Cys Ser Val Glu Phe Glu Val
305                 310                 315                 320
Ala Asp Cys Thr Thr Lys Thr Tyr Pro Asp Asn Ser Phe Asp Val Ile
                325                 330                 335
Tyr Ser Arg Asp Thr Ile Leu His Ile Gln Asp Lys Pro Ala Leu Phe
            340                 345                 350
Arg Thr Phe Phe Lys Trp Leu Lys Pro Gly Gly Lys Val Leu Ile Thr
        355                 360                 365
Asp Tyr Cys Arg Ser Ala Glu Thr Pro Ser Pro Glu Phe Ala Glu Tyr
    370                 375                 380
Ile Lys Gln Arg Gly Tyr Asp Leu His Asp Val Gln Ala Tyr Gly Gln
385                 390                 395                 400
Met Leu Lys Asp Ala Gly Phe Asp Asp Val Ile Ala Glu Asp Arg Thr
                405                 410                 415
Asp Gln Phe Val Gln Val Leu Arg Arg Glu Leu Glu Lys Val Glu Lys
            420                 425                 430
Glu Lys Glu Glu Phe Ile Ser Asp Phe Ser Glu Glu Asp Tyr Asn Asp
        435                 440                 445
Ile Val Gly Gly Trp Ser Ala Lys Leu Glu Arg Thr Ala Ser Gly Glu
    450                 455                 460
```

Gln Lys Trp Gly Leu Phe Ile Ala Asp Lys Lys
465 470 475

<210> 59
<211> 1428
<212> DNA
<213> Arabidopsis thaliana

<400> 59
atggagcatt ctagtgattt gactgttgaa gctatgatgc ttgactctaa agcttctgat 60
cttgacaaag aagaacgtcc tgaggtactc tctttaatcc caccatatga agggaaatct 120
gtgcttgaac ttggagctgg tattggtcgt ttcactggtg aattggctca aaaggctggt 180
gaagttatcg ctcttgactt catcgaaagc gcgattcaga agaatgaaag tgttaatggg 240
cattacaaga acatcaagtt tatgtgtgct gatgtaacat ctccagactt gaaaatcaaa 300
gatggatcta tcgacttgat tttctcaaac tggttgctca tgtatctctc tgataaagag 360
gtggaactaa tggcagagag aatgattgga tgggtcaagc caggggggata cattttcttc 420
agagaatctt gcttccatca atctggggac agcaagcgaa agtcaaaccc cactcactac 480
cgtgaaccca gattctacac aaaggttttc caggaatgtc agacacgtga tgcttctggc 540
aattcatttg agctctctat ggttggctgc aaatgcattg ggcttatgt gaagaacaag 600
aagaatcaga atcagatttg ctggatatgg caaaaagtca gcgtggagaa tgacaaggat 660
ttccagcgtt tcttggacaa tgttcaatac aagtctagtg ggatcttgcg ctatgagcgt 720
gtctttgggg aaggatatgt gagcactggt ggatttgaga caactaaaga atttgtggcg 780
aagatggacc ttaaaccggg acagaaagtc ctagatgttg gttgtggtat cggtggaggt 840
gacttctaca tggctgagaa tttcgatgtt catgttgttg gaatcgatct gtcggtcaac 900
atgatctctt tcgcactgga gcgggccatt ggactcaaat gctcagtcga gtttgaagtc 960
gctgattgca ccaccaaaac atatcccgat aattcctttg atgtcattta cagccgtgac 1020
actattctgc acatccaaga caagccagct ctattcagga cattcttcaa gtggcttaaa 1080
ccagggggta aagttctcat cactgactat tgcagaagtg ctgaaactcc gtctcctgaa 1140
ttcgcagagt acataaaaca aagaggatat gatctacatg atgttcaagc ttacggacag 1200
atgctgaaag acgcaggctt tgacgacgtt atcgctgagg accgtactga tcagtttgta 1260
caagtcctca ggcgtgaatt agaaaaagtg gagaaagaaa aggaagaatt catcagcgac 1320
ttctcagaag aggattacaa tgacattgtt ggaggatggt cggcaaagct tgaaaggact 1380
gcatctggtg aacagaaatg gggattattc atagccgaca agaagtaa 1428

<210> 60
<211> 475
<212> PRT
<213> Arabidopsis thaliana

<400> 60
Met Glu His Ser Ser Asp Leu Thr Val Glu Ala Met Met Leu Asp Ser
1 5 10 15
Lys Ala Ser Asp Leu Asp Lys Glu Glu Arg Pro Glu Val Leu Ser Leu
20 25 30
Ile Pro Pro Tyr Glu Gly Lys Ser Val Leu Glu Leu Gly Ala Gly Ile
35 40 45
Gly Arg Phe Thr Gly Glu Leu Ala Gln Lys Ala Gly Glu Val Ile Ala
50 55 60
Leu Asp Phe Ile Glu Ser Ala Ile Gln Lys Asn Glu Ser Val Asn Gly
65 70 75 80
His Tyr Lys Asn Ile Lys Phe Met Cys Ala Asp Val Thr Ser Pro Asp
85 90 95
Leu Lys Ile Lys Asp Gly Ser Ile Asp Leu Ile Phe Ser Asn Trp Leu
100 105 110
Leu Met Tyr Leu Ser Asp Lys Glu Val Glu Leu Met Ala Glu Arg Met
115 120 125
Ile Gly Trp Val Lys Pro Gly Gly Tyr Ile Phe Phe Arg Glu Ser Cys
130 135 140
Phe His Gln Ser Gly Asp Ser Lys Arg Lys Ser Asn Pro Thr His Tyr
145 150 155 160
Arg Glu Pro Arg Phe Tyr Thr Lys Val Phe Gln Glu Cys Gln Thr Arg
165 170 175
Asp Ala Ser Gly Asn Ser Phe Glu Leu Ser Met Val Gly Cys Lys Cys

```
            180                 185                 190
Ile Gly Ala Tyr Val Lys Asn Lys Lys Asn Gln Asn Gln Ile Cys Trp
        195                 200                 205
Ile Trp Gln Lys Val Ser Val Glu Asn Asp Lys Asp Phe Gln Arg Phe
    210                 215                 220
Leu Asp Asn Val Gln Tyr Lys Ser Ser Gly Ile Leu Arg Tyr Glu Arg
225                 230                 235                 240
Val Phe Gly Glu Gly Tyr Val Ser Thr Gly Gly Phe Glu Thr Thr Lys
                245                 250                 255
Glu Phe Val Ala Lys Met Asp Leu Lys Pro Gly Gln Lys Val Leu Asp
            260                 265                 270
Val Gly Cys Gly Ile Gly Gly Gly Asp Phe Tyr Met Ala Glu Asn Phe
        275                 280                 285
Asp Val His Val Val Gly Ile Asp Leu Ser Val Asn Met Ile Ser Phe
    290                 295                 300
Ala Leu Glu Arg Ala Ile Gly Leu Lys Cys Ser Val Glu Phe Glu Val
305                 310                 315                 320
Ala Asp Cys Thr Thr Lys Thr Tyr Pro Asp Asn Ser Phe Asp Val Ile
                325                 330                 335
Tyr Ser Arg Asp Thr Ile Leu His Ile Gln Asp Lys Pro Ala Leu Phe
            340                 345                 350
Arg Thr Phe Phe Lys Trp Leu Lys Pro Gly Gly Lys Val Leu Ile Thr
        355                 360                 365
Asp Tyr Cys Arg Ser Ala Glu Thr Pro Ser Pro Glu Phe Ala Glu Tyr
    370                 375                 380
Ile Lys Gln Arg Gly Tyr Asp Leu His Asp Val Gln Ala Tyr Gly Gln
385                 390                 395                 400
Met Leu Lys Asp Ala Gly Phe Asp Asp Val Ile Ala Glu Asp Arg Thr
                405                 410                 415
Asp Gln Phe Val Gln Val Leu Arg Arg Glu Leu Glu Lys Val Glu Lys
            420                 425                 430
Glu Lys Glu Glu Phe Ile Ser Asp Phe Ser Glu Glu Asp Tyr Asn Asp
        435                 440                 445
Ile Val Gly Gly Trp Ser Ala Lys Leu Glu Arg Thr Ala Ser Gly Glu
    450                 455                 460
Gln Lys Trp Gly Leu Phe Ile Ala Asp Lys Lys
465                 470                 475
```

<210> 61
<211> 1668
<212> DNA
<213> Arabidopsis thaliana

<400> 61

```
atggtcggcg aagaagatag cgagagagct cagtccagta agatggagat cgagcgagaa      60
tcgaatttgg gatctgcgag tgtgctgatg cagtcgaagg tcatctccgt ctcgaatttc     120
ttctctattc atagatttca ttaccctcgt gaaaaaatcg tctctttttt gtttcctagt     180
gtgttctcaa ggataatggc ttcgtatggc gaggagcgtg aaatccaaaa gaattactgg     240
aaagagcatt cagtgggatt gagtgttgaa gctatgatgc ttgattccaa agcttctgac     300
ctcgacaaag aagaacgtcc tgagatactt gcgtttcttc cacctattga agggacaaca     360
gtgctagagt ttggtgctgg aattggtcgt tttactactg aattagctca gaaggccggc     420
caggtcattg cggttgactt cattgaaagt gttatcaaaa agaatgagaa cattaacggt     480
cactacaaga acgtcaaatt tctgtgcgct gatgtcacat caccaaatat gaactttcca     540
aatgagtcta tggatctgat attctccaac tggctgctaa tgtatctctc tgatcaagag     600
gttgaagatt tggcgaaaaa gatgttacaa tggacaaagg ttggcgggta tattttcttt     660
cgggagtctt gtttccatca gtctggtgat aacaagcgga agtacaaccc aacacactac     720
cgtgaaccta aattttacac aaagcttttc aaagaatgcc atatgaatga cgaagatggg     780
aattcgtatg aactctcttt ggttagctgt aaatgcattg gagcttatgt gagaaacaaa     840
aagaaccaga accagatatg ctggctttgg cagaaagtca gttcggataa tgataggggc     900
ttccaacgct tcttggacaa tgtccagtat aagtctagtg gtatcttacg ctatgagcgt     960
gtctttggag aagggtttgt tagcacaggg ggactcgaga caacaaagga attcgtggat    1020
atgctggatc tgaaacctgg ccaaaaagtt ctagacgttg ggtgcggaat aggaggaggg    1080
gacttctaca tggctgagaa ctttgacgtg gatgttgtgg gcattgatct atctgtaaac    1140
```

```
atgatctctt ttgcgcttga acacgcaata ggactcaaat gctctgtaga attcgaagta   1200
gctgattgca ccaagaagga gtatcctgat aacacctttg atgttattta tagcagagac   1260
accattctac atatccaaga caagccagca ttgttcagaa gattctacaa atggttgaag   1320
ccgggaggga aagttctcat cactgattac tgcagaagcc ccaaaacccc atctccagac   1380
tttgcaatct acatcaagaa acgaggttat gatcttcatg atgtacaagc atacggtcag   1440
atgctgagag atgctggttt cgaggaggta atcgcggagg atagaaccga tcagttcatg   1500
aaagtcctga aacgggaact ggatgcagtg gagaaggaga aggaagaatt catcagtgac   1560
ttctcgaaag aggattacga ggatattata ggcgggtgga agtcaaagct acttaggagc   1620
tcaagtggtg agcagaagtg ggtttgttc atcgccaaga gaaactga             1668
```

<210> 62
<211> 555
<212> PRT
<213> Arabidopsis thaliana

<400> 62

```
Met Val Gly Glu Glu Asp Ser Glu Arg Ala Gln Ser Ser Lys Met Glu
1               5                   10                  15
Ile Glu Arg Glu Ser Asn Leu Gly Ser Ala Ser Val Leu Met Gln Ser
            20                  25                  30
Lys Val Ile Ser Val Ser Asn Phe Phe Ser Ile His Arg Phe His Tyr
        35                  40                  45
Pro Arg Glu Lys Ile Val Ser Phe Leu Phe Pro Ser Val Phe Ser Arg
    50                  55                  60
Ile Met Ala Ser Tyr Gly Glu Glu Arg Glu Ile Gln Lys Asn Tyr Trp
65                  70                  75                  80
Lys Glu His Ser Val Gly Leu Ser Val Glu Ala Met Met Leu Asp Ser
                85                  90                  95
Lys Ala Ser Asp Leu Asp Lys Glu Glu Arg Pro Glu Ile Leu Ala Phe
            100                 105                 110
Leu Pro Pro Ile Glu Gly Thr Thr Val Leu Glu Phe Gly Ala Gly Ile
        115                 120                 125
Gly Arg Phe Thr Thr Glu Leu Ala Gln Lys Ala Gly Gln Val Ile Ala
    130                 135                 140
Val Asp Phe Ile Glu Ser Val Ile Lys Lys Asn Glu Asn Ile Asn Gly
145                 150                 155                 160
His Tyr Lys Asn Val Lys Phe Leu Cys Ala Asp Val Thr Ser Pro Asn
                165                 170                 175
Met Asn Phe Pro Asn Glu Ser Met Asp Leu Ile Phe Ser Asn Trp Leu
            180                 185                 190
Leu Met Tyr Leu Ser Asp Gln Glu Val Glu Asp Leu Ala Lys Lys Met
        195                 200                 205
Leu Gln Trp Thr Lys Val Gly Gly Tyr Ile Phe Phe Arg Glu Ser Cys
    210                 215                 220
Phe His Gln Ser Gly Asp Asn Lys Arg Lys Tyr Asn Pro Thr His Tyr
225                 230                 235                 240
Arg Glu Pro Lys Phe Tyr Thr Lys Leu Phe Lys Glu Cys His Met Asn
                245                 250                 255
Asp Glu Asp Gly Asn Ser Tyr Glu Leu Ser Leu Val Ser Cys Lys Cys
            260                 265                 270
Ile Gly Ala Tyr Val Arg Asn Lys Lys Asn Gln Asn Gln Ile Cys Trp
        275                 280                 285
Leu Trp Gln Lys Val Ser Ser Asp Asn Asp Arg Gly Phe Gln Arg Phe
    290                 295                 300
Leu Asp Asn Val Gln Tyr Lys Ser Ser Gly Ile Leu Arg Tyr Glu Arg
305                 310                 315                 320
Val Phe Gly Glu Gly Phe Val Ser Thr Gly Gly Leu Glu Thr Thr Lys
                325                 330                 335
Glu Phe Val Asp Met Leu Asp Leu Lys Pro Gly Gln Lys Val Leu Asp
            340                 345                 350
Val Gly Cys Gly Ile Gly Gly Gly Asp Phe Tyr Met Ala Glu Asn Phe
        355                 360                 365
Asp Val Asp Val Val Gly Ile Asp Leu Ser Val Asn Met Ile Ser Phe
```

370 375 380
Ala Leu Glu His Ala Ile Gly Leu Lys Cys Ser Val Glu Phe Glu Val
385 390 395 400
Ala Asp Cys Thr Lys Lys Glu Tyr Pro Asp Asn Thr Phe Asp Val Ile
405 410 415
Tyr Ser Arg Asp Thr Ile Leu His Ile Gln Asp Lys Pro Ala Leu Phe
420 425 430
Arg Arg Phe Tyr Lys Trp Leu Lys Pro Gly Gly Lys Val Leu Ile Thr
435 440 445
Asp Tyr Cys Arg Ser Pro Lys Thr Pro Ser Pro Asp Phe Ala Ile Tyr
450 455 460
Ile Lys Lys Arg Gly Tyr Asp Leu His Asp Val Gln Ala Tyr Gly Gln
465 470 475 480
Met Leu Arg Asp Ala Gly Phe Glu Glu Val Ile Ala Glu Asp Arg Thr
485 490 495
Asp Gln Phe Met Lys Val Leu Lys Arg Glu Leu Asp Ala Val Glu Lys
500 505 510
Glu Lys Glu Glu Phe Ile Ser Asp Phe Ser Lys Glu Asp Tyr Glu Asp
515 520 525
Ile Ile Gly Gly Trp Lys Ser Lys Leu Leu Arg Ser Ser Ser Gly Glu
530 535 540
Gln Lys Trp Gly Leu Phe Ile Ala Lys Arg Asn
545 550 555

<210> 63
<211> 1476
<212> DNA
<213> Arabidopsis thaliana

<400> 63
atggctgcat cgtacgaaga agagcgtgat attcagaaga attactggat agagcattcc 60
gctgatctga ctgttgaagc tatgatgctt gactcgagag cttctgatct cgacaaggaa 120
gaacgtcctg aggtactctc tttgctccct ccatatgaag gcaaatcagt gttggaactt 180
ggagctggta ttggtcgttt cactggtgaa ttagctcaaa aggctggtga actcattgct 240
cttgacttca ttgataacgt tatcaagaag aatgaaagta tcaatggca ttacaagaat 300
gtcaagttta tgtgtgctga tgttacatcc cctgacctca agatcactga tggatctctt 360
gacttgattt tctccaactg gctgctcatg tatctttctg acaaagaggt ggagcttttg 420
gcagaaagga tggtcggttg gatcaaggtt ggaggataca ttttcttccg tgaatcttgc 480
ttccaccaat caggggacag taagcggaaa tccaacccca ctcactaccg tgaacccgt 540
ttctattcca aggtctttca agagtgtcag actcgggatg ctgctggaaa ttcatttgag 600
ctctctatga tcggatgcaa gtgcattgga gcttatgtca agaacaagaa gaatcagaat 660
cagatttgtt ggatatggca gaaggtcagc tcagaaaatg acagaggctt ccaacgtttc 720
ttggacaatg tccaatacaa atccagtgga atcctacgct atgagcgtgt ctttggccaa 780
gggtttgtga gcactggtgg acttgagaca accaaagaat ttgtggagaa aatgaatctg 840
aaaccaggac agaaagtctt agatgttggg tgtggcattg gtggaggtga cttctacatg 900
gctgagaagt ttgatgttca cgttgttggt atcgatcttt ctgtcaacat gatctctttc 960
gcattggaac gtgctattgg actcagctgc tcggttgagt ttgaggttgc tgattgcacc 1020
acaaaacact acccagataa ttcgtttgat gtcatttaca gccgtgacac tattctgcac 1080
atccaagaca aaccagcctt gtttaggact ttcttcaaat ggcttaaacc gggaggtaaa 1140
gttctcatca gcgactactg tagaagcccc aaaactccat ctgctgagtt ttcagagtac 1200
atcaaacaga gaggatatga tctccatgac gttcaagctt atggacagat gctaaaagac 1260
gctggcttca ctgatgtgat cgcagaggac cgtactgatc agtttatgca agtcctgaaa 1320
cgtgaattag acagggtgga gaaagaaaag gaaaaattca tctccgactt ctccaaagag 1380
gattacgatg acattgttgg aggatggaag tcaaagctgg agaggtgtgc atcggatgag 1440
cagaaatggg gacttttcat cgccaacaag aattaa 1476

<210> 64
<211> 491
<212> PRT
<213> Arabidopsis thaliana

<400> 64
Met Ala Ala Ser Tyr Glu Glu Glu Arg Asp Ile Gln Lys Asn Tyr Trp

```
1               5                   10                  15
Ile Glu His Ser Ala Asp Leu Thr Val Glu Ala Met Met Leu Asp Ser
            20                  25                  30
Arg Ala Ser Asp Leu Asp Lys Glu Glu Arg Pro Glu Val Leu Ser Leu
        35                  40                  45
Leu Pro Pro Tyr Glu Gly Lys Ser Val Leu Glu Leu Gly Ala Gly Ile
    50                  55                  60
Gly Arg Phe Thr Gly Glu Leu Ala Gln Lys Ala Gly Glu Leu Ile Ala
65                  70                  75                  80
Leu Asp Phe Ile Asp Asn Val Ile Lys Lys Asn Glu Ser Ile Asn Gly
                85                  90                  95
His Tyr Lys Asn Val Lys Phe Met Cys Ala Asp Val Thr Ser Pro Asp
            100                 105                 110
Leu Lys Ile Thr Asp Gly Ser Leu Asp Leu Ile Phe Ser Asn Trp Leu
        115                 120                 125
Leu Met Tyr Leu Ser Asp Lys Glu Val Glu Leu Leu Ala Glu Arg Met
    130                 135                 140
Val Gly Trp Ile Lys Val Gly Gly Tyr Ile Phe Phe Arg Glu Ser Cys
145                 150                 155                 160
Phe His Gln Ser Gly Asp Ser Lys Arg Lys Ser Asn Pro Thr His Tyr
                165                 170                 175
Arg Glu Pro Arg Phe Tyr Ser Lys Val Phe Gln Glu Cys Gln Thr Arg
            180                 185                 190
Asp Ala Ala Gly Asn Ser Phe Glu Leu Ser Met Ile Gly Cys Lys Cys
        195                 200                 205
Ile Gly Ala Tyr Val Lys Asn Lys Lys Asn Gln Asn Gln Ile Cys Trp
    210                 215                 220
Ile Trp Gln Lys Val Ser Ser Glu Asn Asp Arg Gly Phe Gln Arg Phe
225                 230                 235                 240
Leu Asp Asn Val Gln Tyr Lys Ser Ser Gly Ile Leu Arg Tyr Glu Arg
                245                 250                 255
Val Phe Gly Gln Gly Phe Val Ser Thr Gly Gly Leu Glu Thr Thr Lys
            260                 265                 270
Glu Phe Val Glu Lys Met Asn Leu Lys Pro Gly Gln Lys Val Leu Asp
        275                 280                 285
Val Gly Cys Gly Ile Gly Gly Gly Asp Phe Tyr Met Ala Glu Lys Phe
    290                 295                 300
Asp Val His Val Val Gly Ile Asp Leu Ser Val Asn Met Ile Ser Phe
305                 310                 315                 320
Ala Leu Glu Arg Ala Ile Gly Leu Ser Cys Ser Val Glu Phe Glu Val
                325                 330                 335
Ala Asp Cys Thr Thr Lys His Tyr Pro Asp Asn Ser Phe Asp Val Ile
            340                 345                 350
Tyr Ser Arg Asp Thr Ile Leu His Ile Gln Asp Lys Pro Ala Leu Phe
        355                 360                 365
Arg Thr Phe Phe Lys Trp Leu Lys Pro Gly Gly Lys Val Leu Ile Ser
    370                 375                 380
Asp Tyr Cys Arg Ser Pro Lys Thr Pro Ser Ala Glu Phe Ser Glu Tyr
385                 390                 395                 400
Ile Lys Gln Arg Gly Tyr Asp Leu His Asp Val Gln Ala Tyr Gly Gln
                405                 410                 415
Met Leu Lys Asp Ala Gly Phe Thr Asp Val Ile Ala Glu Asp Arg Thr
            420                 425                 430
Asp Gln Phe Met Gln Val Leu Lys Arg Glu Leu Asp Arg Val Glu Lys
        435                 440                 445
Glu Lys Glu Lys Phe Ile Ser Asp Phe Ser Lys Glu Asp Tyr Asp Asp
    450                 455                 460
Ile Val Gly Gly Trp Lys Ser Lys Leu Glu Arg Cys Ala Ser Asp Glu
465                 470                 475                 480
Gln Lys Trp Gly Leu Phe Ile Ala Asn Lys Asn
                485                 490
```

<210> 65

<211> 1500
<212> DNA
<213> Oryza sativa

<400> 65
atggacgccg cggccgccac cgctgttaat ggagtgcttg aggtggagga gaggaaggcg 60
cagaagagct actgggagga gcactccaag gacctcaccg tcgaggccat gatgctcgac 120
tcccgcgccg ccgatctcga caaggaggag cgccccgaga tattgtcttt acttcctcct 180
tacgaaggaa aatcagtact ggaacttggt gctggaatag gtcgcttcac tggagaacta 240
gtgaaaacag ctgggcatgt tcttgcaatg gatttcattg aaagtgtgat taagaagaat 300
gaaagcataa acggtcacca caagaatgca tcctttatgt gtgcggatgt cacatgtcca 360
gacctgatga ttgaggataa ctccattgat ctgatatttt caaactggtt actgatgtat 420
ctttcagacg aggaggttga gaagctagta aagagaatgg taagatggct aaaggttggc 480
ggctatatct tctttaggga atcttgtttc catcagtctg gagattcaaa aaggaaagtg 540
aatcctacac attaccggga gccaaggttt tacactaagg tgtttaaaga gtgtcaagct 600
cttgatcaag atgggaattc ctttgaactc tctgtactta cttgcaagtg tgttggagct 660
tacgtgaaaa gcaagaaaaa tcaaaaccag atatgttggc tatggcaaaa ggttgattca 720
acagaagatc ggggggtttca aagattttg gacaatgtgc agtacaaagc cagtggaata 780
ttacgctatg aacgcatctt tggagaaggc tttgtgagca ctggtggaat tgaaactaca 840
aaagaatttg tggacaggct ggatctcaaa cctggccaga acgttcttga tgttggatgt 900
ggaattgggg gcggtgattt ttatatggct gacaagtatg atgttcatgt tgttggtatt 960
gatctttcga taaacatggt ttcttttgca cttgagcgtg ctattgggcg taagtgctca 1020
gttgagtttg aagtcgctga ttgcaccaaa aagacatacc cagacaacac gtttgacgtc 1080
atctacagtc gtgatactat ccttcacata caagataaac cctcactatt taaaagtttc 1140
ttcaagtggc tcaaacctgg gggtaaggtc ctaattagtg attactgcaa gtgccctggg 1200
aaacccttcag aagagttcgc agcttacatt aagcaaaggg gttatgacct tcacgacgtc 1260
agggcttacg gacagatgct tgagaatgct ggtttccatg atgtcattgc tgaagaccgc 1320
accgatcagt tcctcgatgt tctagagagg gagcttgcta aagttgaaaa gaacaaaaac 1380
gagttcgtct ctgatttcag ccaggaggac tacgacgcca ttgtgaatgg atggaaggca 1440
aaacttcaaa ggagttctgc tggtgagcag aggtgggggc tgttcatcgc gaccaagtga 1500

<210> 66
<211> 499
<212> PRT
<213> Oryza sativa

<400> 66
Met Asp Ala Ala Ala Ala Thr Ala Val Asn Gly Val Leu Glu Val Glu
1 5 10 15
Glu Arg Lys Ala Gln Lys Ser Tyr Trp Glu Glu His Ser Lys Asp Leu
20 25 30
Thr Val Glu Ala Met Met Leu Asp Ser Arg Ala Ala Asp Leu Asp Lys
35 40 45
Glu Glu Arg Pro Glu Ile Leu Ser Leu Leu Pro Pro Tyr Glu Gly Lys
50 55 60
Ser Val Leu Glu Leu Gly Ala Gly Ile Gly Arg Phe Thr Gly Glu Leu
65 70 75 80
Val Lys Thr Ala Gly His Val Leu Ala Met Asp Phe Ile Glu Ser Val
85 90 95
Ile Lys Lys Asn Glu Ser Ile Asn Gly His His Lys Asn Ala Ser Phe
100 105 110
Met Cys Ala Asp Val Thr Cys Pro Asp Leu Met Ile Glu Asp Asn Ser
115 120 125
Ile Asp Leu Ile Phe Ser Asn Trp Leu Leu Met Tyr Leu Ser Asp Glu
130 135 140
Glu Val Glu Lys Leu Val Lys Arg Met Val Arg Trp Leu Lys Val Gly
145 150 155 160
Gly Tyr Ile Phe Phe Arg Glu Ser Cys Phe His Gln Ser Gly Asp Ser
165 170 175
Lys Arg Lys Val Asn Pro Thr His Tyr Arg Glu Pro Arg Phe Tyr Thr
180 185 190
Lys Val Phe Lys Glu Cys Gln Ala Leu Asp Gln Asp Gly Asn Ser Phe
195 200 205

```
Glu Leu Ser Val Leu Thr Cys Lys Cys Val Gly Ala Tyr Val Lys Ser
    210                 215                 220
Lys Lys Asn Gln Asn Gln Ile Cys Trp Leu Trp Gln Lys Val Asp Ser
225                 230                 235                 240
Thr Glu Asp Arg Gly Phe Gln Arg Phe Leu Asp Asn Val Gln Tyr Lys
                245                 250                 255
Ala Ser Gly Ile Leu Arg Tyr Glu Arg Ile Phe Gly Glu Gly Phe Val
            260                 265                 270
Ser Thr Gly Gly Ile Glu Thr Thr Lys Glu Phe Val Asp Arg Leu Asp
        275                 280                 285
Leu Lys Pro Gly Gln Asn Val Leu Asp Val Gly Cys Gly Ile Gly Gly
    290                 295                 300
Gly Asp Phe Tyr Met Ala Asp Lys Tyr Asp Val His Val Val Gly Ile
305                 310                 315                 320
Asp Leu Ser Ile Asn Met Val Ser Phe Ala Leu Glu Arg Ala Ile Gly
                325                 330                 335
Arg Lys Cys Ser Val Glu Phe Glu Val Ala Asp Cys Thr Lys Lys Thr
            340                 345                 350
Tyr Pro Asp Asn Thr Phe Asp Val Ile Tyr Ser Arg Asp Thr Ile Leu
        355                 360                 365
His Ile Gln Asp Lys Pro Ser Leu Phe Lys Ser Phe Phe Lys Trp Leu
    370                 375                 380
Lys Pro Gly Gly Lys Val Leu Ile Ser Asp Tyr Cys Lys Cys Pro Gly
385                 390                 395                 400
Lys Pro Ser Glu Glu Phe Ala Ala Tyr Ile Lys Gln Arg Gly Tyr Asp
                405                 410                 415
Leu His Asp Val Arg Ala Tyr Gly Gln Met Leu Glu Asn Ala Gly Phe
            420                 425                 430
His Asp Val Ile Ala Glu Asp Arg Thr Asp Gln Phe Leu Asp Val Leu
        435                 440                 445
Glu Arg Glu Leu Ala Lys Val Glu Lys Asn Lys Asn Glu Phe Val Ser
    450                 455                 460
Asp Phe Ser Gln Glu Asp Tyr Asp Ala Ile Val Asn Gly Trp Lys Ala
465                 470                 475                 480
Lys Leu Gln Arg Ser Ser Ala Gly Glu Gln Arg Trp Gly Leu Phe Ile
                485                 490                 495
Ala Thr Lys


<210>  67
<211>  1476
<212>  DNA
<213>  Oryza sativa

<400>  67
atgcgtgcag ggatcgggga ggtggagagg aaggcgcagc ggagctactg ggaggagcac      60
tccaaggacc tcaccgtcga ggccatgatg ctcgactccc gcgccgccga cctcgacaag     120
gaggagcgcc ccgaggtcct gtctgtactc ccttcttaca aagggaaatc agtactggag     180
cttggtgctg gaataggacg ctttactggg gaactggcaa aagaagctgg ccatgtttta     240
gccctagact tcattgaaag tgtgattaag aagaatgaga acataaatgg gcatcacaag     300
aacataacct ttatgtgcgc tgatgtcacg tctccggacc tgacgatcga agataactct     360
attgatctca tattctcaaa ctggctacta atgtaccttt cagatgagga ggtcgagaag     420
ctagtaggaa gaatggtgaa atggctgaag gtaggtggcc atatattctt tagggagtca     480
tgctttcacc aatctggaga ttccaaaagg aaggtgaatc caacacatta ccgggagcca     540
aggttctata caaagatatt taaagaatgc cattcctatg ataaagatgg gggttcttat     600
gaactttctc tagaaacatg caagtgcatt ggggcttatg tgaaaagcaa gaaaaatcaa     660
aatcagttat gttggctatg ggaaaaggtt aagtcaacag aagacagagg attccaaaga     720
ttcctggaca atgtgcagta caaaaccact ggaatcttac gctatgagcg tgtcttcgga     780
gagggttatg tcagcactgg tggaattgaa accacaaagg aatttgtgga taagctggat     840
cttaaacctg gacagaaagt gcttgatgtt gggtgcggaa ttggaggcgg cgacttctat     900
atggctgaaa actacgatgc ccatgttctt ggtattgatc tttcaatcaa catggtttca     960
tttgcaatcg aacgtgccat tggacgcaag tgttcggttg agtttgaagt agctgattgc    1020
accacaaaga cctacgcacc aaatacattt gatgtgatct acagccgtga caccattctt    1080
```

```
cacatacatg ataaacctgc tttgttcaga agtttcttca agtggctgaa acctgggggc   1140
aaagtcctca tcagtgatta ctgtaggaat cctgggaaac catcagaaga atttgctgct   1200
tacattaagc agagaggcta tgacctccac gatgtgaaga cttacggaaa gatgcttgag   1260
gatgctggtt tccatcatgt cattgctgaa gaccgcacgg accagttcct gcgtgttctt   1320
caaagggagc ttgctgaagt tgagaagaac aaagaagcct tcatggcaga cttcacccag   1380
gaggactacg atgacattgt gaacggctgg aacgcgaagc tgaagcggag ctctgccggt   1440
gagcagaggt gggggctgtt cattgcaacc aaatga                             1476
```

<210> 68
<211> 491
<212> PRT
<213> Oryza sativa

<400> 68

```
Met Arg Ala Gly Ile Gly Glu Val Glu Arg Lys Ala Gln Arg Ser Tyr
1               5                   10                  15
Trp Glu Glu His Ser Lys Asp Leu Thr Val Glu Ala Met Met Leu Asp
            20                  25                  30
Ser Arg Ala Ala Asp Leu Asp Lys Glu Glu Arg Pro Glu Val Leu Ser
        35                  40                  45
Val Leu Pro Ser Tyr Lys Gly Lys Ser Val Leu Glu Leu Gly Ala Gly
    50                  55                  60
Ile Gly Arg Phe Thr Gly Glu Leu Ala Lys Glu Ala Gly His Val Leu
65                  70                  75                  80
Ala Leu Asp Phe Ile Glu Ser Val Ile Lys Lys Asn Glu Asn Ile Asn
                85                  90                  95
Gly His His Lys Asn Ile Thr Phe Met Cys Ala Asp Val Thr Ser Pro
            100                 105                 110
Asp Leu Thr Ile Glu Asp Asn Ser Ile Asp Leu Ile Phe Ser Asn Trp
        115                 120                 125
Leu Leu Met Tyr Leu Ser Asp Glu Glu Val Glu Lys Leu Val Gly Arg
    130                 135                 140
Met Val Lys Trp Leu Lys Val Gly Gly His Ile Phe Phe Arg Glu Ser
145                 150                 155                 160
Cys Phe His Gln Ser Gly Asp Ser Lys Arg Lys Val Asn Pro Thr His
                165                 170                 175
Tyr Arg Glu Pro Arg Phe Tyr Thr Lys Ile Phe Lys Glu Cys His Ser
            180                 185                 190
Tyr Asp Lys Asp Gly Gly Ser Tyr Glu Leu Ser Leu Glu Thr Cys Lys
        195                 200                 205
Cys Ile Gly Ala Tyr Val Lys Ser Lys Lys Asn Gln Asn Gln Leu Cys
    210                 215                 220
Trp Leu Trp Glu Lys Val Lys Ser Thr Glu Asp Arg Gly Phe Gln Arg
225                 230                 235                 240
Phe Leu Asp Asn Val Gln Tyr Lys Thr Thr Gly Ile Leu Arg Tyr Glu
                245                 250                 255
Arg Val Phe Gly Glu Gly Tyr Val Ser Thr Gly Gly Ile Glu Thr Thr
            260                 265                 270
Lys Glu Phe Val Asp Lys Leu Asp Leu Lys Pro Gly Gln Lys Val Leu
        275                 280                 285
Asp Val Gly Cys Gly Ile Gly Gly Gly Asp Phe Tyr Met Ala Glu Asn
    290                 295                 300
Tyr Asp Ala His Val Leu Gly Ile Asp Leu Ser Ile Asn Met Val Ser
305                 310                 315                 320
Phe Ala Ile Glu Arg Ala Ile Gly Arg Lys Cys Ser Val Glu Phe Glu
                325                 330                 335
Val Ala Asp Cys Thr Thr Lys Thr Tyr Ala Pro Asn Thr Phe Asp Val
            340                 345                 350
Ile Tyr Ser Arg Asp Thr Ile Leu His Ile His Asp Lys Pro Ala Leu
        355                 360                 365
Phe Arg Ser Phe Phe Lys Trp Leu Lys Pro Gly Gly Lys Val Leu Ile
    370                 375                 380
Ser Asp Tyr Cys Arg Asn Pro Gly Lys Pro Ser Glu Glu Phe Ala Ala
```

```
385                 390                 395                 400
Tyr Ile Lys Gln Arg Gly Tyr Asp Leu His Asp Val Lys Thr Tyr Gly
                405                 410                 415
Lys Met Leu Glu Asp Ala Gly Phe His His Val Ile Ala Glu Asp Arg
            420                 425                 430
Thr Asp Gln Phe Leu Arg Val Leu Gln Arg Glu Leu Ala Glu Val Glu
        435                 440                 445
Lys Asn Lys Glu Ala Phe Met Ala Asp Phe Thr Gln Glu Asp Tyr Asp
    450                 455                 460
Asp Ile Val Asn Gly Trp Asn Ala Lys Leu Lys Arg Ser Ser Ala Gly
465                 470                 475                 480
Glu Gln Arg Trp Gly Leu Phe Ile Ala Thr Lys
                485                 490

<210>  69
<211>  1488
<212>  DNA
<213>  Oryza sativa

<400>  69
atggacgccg tcgcggcgaa tgggatcggg gaggtggaga ggaaggcgca gcggagctac     60
tgggaggagc actccaagga cctcaccgtc gaggccatga tgctcgactc ccgcgccgcc    120
gacctcgaca aggaggagcg ccccgaggtc ctgtctgtac tcccttctta caaagggaaa    180
tcagtactgg agcttggtgc tggaatagga cgctttactg ggaactggc aaaagaagct     240
ggccatgttt tagccctaga cttcattgaa agtgtgatta agaagaatga gaacataaat    300
gggcatcaca agaacataac ctttatgtgc gctgatgtca cgtctccgga cctgacgatc    360
gaagataact ctattgatct catattctca aactggctac taatgtacct ttcagatgag    420
gaggtcgaga agctagtagg aagaatggtg aaatggctga aggtaggtgg ccatatattc    480
tttagggagt catgctttca ccaatctgga gattccaaaa ggaaggtgaa tccaacacat    540
taccgggagc caaggttcta tacaaagata tttaaagaat gccattccta tgataaagat    600
gggggttctt atgaactttc tctagaaaca tgcaagtgca ttggggctta tgtgaaaagc    660
aagaaaaatc aaaatcagtt atgttggcta tgggaaaagg ttaagtcaac agaagacaga    720
ggattccaaa gattcctgga caatgtgcag tacaaaacca ctggaatctt acgctatgag    780
cgtgtcttcg gagagggtta tgtcagcact ggtggaattg aaaccacaaa ggaatttgtg    840
gataagctgg atcttaaacc tggacagaaa gtgcttgatg ttgggtgcgg aattggaggc    900
ggcgacttct atatggctga aaactacgat gcccatgttc ttggtattga tctttcaatc    960
aacatggttt catttgcaat cgaacgtgcc attggacgca agtgttcggt tgagtttgaa   1020
gtagctgatt gcaccacaaa gacctacgca ccaaatacat ttgatgtgat ctacagccgt   1080
gacaccattc ttcacataca tgataaacct gctttgttca gaagtttctt caagtggctg   1140
aaacctgggg gcaaagtcct catcagtgat tactgtagga atcctgggaa accatcagaa   1200
gaatttgctg cttacattaa gcagagaggc tatgacctcc acgatgtgaa gacttacgga   1260
aagatgcttg aggatgctgg tttccatcat gtcattgctg aagaccgcac ggaccagttc   1320
ctgcgtgttc ttcaaaggga gcttgctgaa gttgagaaga acaaagaagc cttcatggca   1380
gacttcaccc aggaggacta cgatgacatt gtgaacggct ggaacgcgaa gctgaagcgg   1440
agctctgccg gtgagcagag gtgggggctg ttcattgcaa ccaaatga                1488

<210>  70
<211>  495
<212>  PRT
<213>  Oryza sativa

<400>  70
Met Asp Ala Val Ala Ala Asn Gly Ile Gly Glu Val Glu Arg Lys Ala
1               5                   10                  15
Gln Arg Ser Tyr Trp Glu Glu His Ser Lys Asp Leu Thr Val Glu Ala
            20                  25                  30
Met Met Leu Asp Ser Arg Ala Ala Asp Leu Asp Lys Glu Glu Arg Pro
        35                  40                  45
Glu Val Leu Ser Val Leu Pro Ser Tyr Lys Gly Lys Ser Val Leu Glu
    50                  55                  60
Leu Gly Ala Gly Ile Gly Arg Phe Thr Gly Glu Leu Ala Lys Glu Ala
65                  70                  75                  80
Gly His Val Leu Ala Leu Asp Phe Ile Glu Ser Val Ile Lys Lys Asn
```

```
                85                  90                  95
Glu Asn Ile Asn Gly His His Lys Asn Ile Thr Phe Met Cys Ala Asp
            100                 105                 110
Val Thr Ser Pro Asp Leu Thr Ile Glu Asp Asn Ser Ile Asp Leu Ile
        115                 120                 125
Phe Ser Asn Trp Leu Leu Met Tyr Leu Ser Asp Glu Glu Val Glu Lys
    130                 135                 140
Leu Val Gly Arg Met Val Lys Trp Leu Lys Val Gly Gly His Ile Phe
145                 150                 155                 160
Phe Arg Glu Ser Cys Phe His Gln Ser Gly Asp Ser Lys Arg Lys Val
                165                 170                 175
Asn Pro Thr His Tyr Arg Glu Pro Arg Phe Tyr Thr Lys Ile Phe Lys
            180                 185                 190
Glu Cys His Ser Tyr Asp Lys Asp Gly Gly Ser Tyr Glu Leu Ser Leu
        195                 200                 205
Glu Thr Cys Lys Cys Ile Gly Ala Tyr Val Lys Ser Lys Lys Asn Gln
    210                 215                 220
Asn Gln Leu Cys Trp Leu Trp Glu Lys Val Lys Ser Thr Glu Asp Arg
225                 230                 235                 240
Gly Phe Gln Arg Phe Leu Asp Asn Val Gln Tyr Lys Thr Thr Gly Ile
                245                 250                 255
Leu Arg Tyr Glu Arg Val Phe Gly Glu Gly Tyr Val Ser Thr Gly Gly
            260                 265                 270
Ile Glu Thr Thr Lys Glu Phe Val Asp Lys Leu Asp Leu Lys Pro Gly
        275                 280                 285
Gln Lys Val Leu Asp Val Gly Cys Gly Ile Gly Gly Gly Asp Phe Tyr
    290                 295                 300
Met Ala Glu Asn Tyr Asp Ala His Val Leu Gly Ile Asp Leu Ser Ile
305                 310                 315                 320
Asn Met Val Ser Phe Ala Ile Glu Arg Ala Ile Gly Arg Lys Cys Ser
                325                 330                 335
Val Glu Phe Glu Val Ala Asp Cys Thr Thr Lys Thr Tyr Ala Pro Asn
            340                 345                 350
Thr Phe Asp Val Ile Tyr Ser Arg Asp Thr Ile Leu His Ile His Asp
        355                 360                 365
Lys Pro Ala Leu Phe Arg Ser Phe Phe Lys Trp Leu Lys Pro Gly Gly
    370                 375                 380
Lys Val Leu Ile Ser Asp Tyr Cys Arg Asn Pro Gly Lys Pro Ser Glu
385                 390                 395                 400
Glu Phe Ala Ala Tyr Ile Lys Gln Arg Gly Tyr Asp Leu His Asp Val
                405                 410                 415
Lys Thr Tyr Gly Lys Met Leu Glu Asp Ala Gly Phe His His Val Ile
            420                 425                 430
Ala Glu Asp Arg Thr Asp Gln Phe Leu Arg Val Leu Gln Arg Glu Leu
        435                 440                 445
Ala Glu Val Glu Lys Asn Lys Glu Ala Phe Met Ala Asp Phe Thr Gln
    450                 455                 460
Glu Asp Tyr Asp Asp Ile Val Asn Gly Trp Asn Ala Lys Leu Lys Arg
465                 470                 475                 480
Ser Ser Ala Gly Glu Gln Arg Trp Gly Leu Phe Ile Ala Thr Lys
                485                 490                 495
```

<210> 71
<211> 1164
<212> DNA
<213> Oryza sativa

<400> 71

```
atgtgcgctg atgtcacgtc tccggacctg acgatcgaag ataactctat tgatctcata      60
ttctcaaact ggctactaat gtacctttca gatgaggagg tcgagaagct agtaggaaga     120
atggtgaaat ggctgaaggt aggtggccat atattcttta gggagtcatg ctttcaccaa     180
tctggagatt ccaaaaggaa ggtgaatcca acacattacc gggagccaag gttctataca     240
aagatattta aagaatgcca ttcctatgat aaagatgggg gttcttatga actttctcta     300
```

```
gaaacatgca agtgcattgg ggcttatgtg aaaagcaaga aaaatcaaaa tcagttatgt     360
tggctatggg aaaaggttaa gtcaacagaa gacagaggat tccaaagatt cctggacaat     420
gtgcagtaca aaaccactgg aatcttacgc tatgagcgtg tcttcggaga gggttatgtc     480
agcactggtg gaattgaaac cacaaaggaa tttgtggata agctggatct taaacctgga     540
cagaaagtgc ttgatgttgg gtgcggaatt ggaggcggcg acttctatat ggctgaaaac     600
tacgatgccc atgttcttgg tattgatctt tcaatcaaca tggtttcatt tgcaatcgaa     660
cgtgccattg gacgcaagtg ttcggttgag tttgaagtag ctgattgcac cacaaagacc     720
tacgcaccaa atacatttga tgtgatctac agccgtgaca ccattcttca catacatgat     780
aaacctgctt tgttcagaag tttcttcaag tggctgaaac ctgggggcaa agtcctcatc     840
agtgattact gtaggaatcc tgggaaacca tcagaagaat ttgctgctta cattaagcag     900
agaggctatg acctccacga tgtgaagact tacggaaaga tgcttgagga tgctggtttc     960
catcatgtca ttgctgaaga ccgcacggac cagttcctgc gtgttcttca aagggagctt    1020
gctgaagttg agaagaacaa agaagccttc atggcagact tcacccagga ggactacgat    1080
gacattgtga acggctggaa cgcgaagctg aagcggagct ctgccggtga gcagaggtgg    1140
gggctgttca ttgcaaccaa atga                                           1164
```

<210> 72
<211> 387
<212> PRT
<213> Oryza sativa

<400> 72

```
Met Cys Ala Asp Val Thr Ser Pro Asp Leu Thr Ile Glu Asp Asn Ser
1               5                   10                  15
Ile Asp Leu Ile Phe Ser Asn Trp Leu Leu Met Tyr Leu Ser Asp Glu
            20                  25                  30
Glu Val Glu Lys Leu Val Gly Arg Met Val Lys Trp Leu Lys Val Gly
        35                  40                  45
Gly His Ile Phe Phe Arg Glu Ser Cys Phe His Gln Ser Gly Asp Ser
    50                  55                  60
Lys Arg Lys Val Asn Pro Thr His Tyr Arg Glu Pro Arg Phe Tyr Thr
65                  70                  75                  80
Lys Ile Phe Lys Glu Cys His Ser Tyr Asp Lys Asp Gly Gly Ser Tyr
                85                  90                  95
Glu Leu Ser Leu Glu Thr Cys Lys Cys Ile Gly Ala Tyr Val Lys Ser
            100                 105                 110
Lys Lys Asn Gln Asn Gln Leu Cys Trp Leu Trp Glu Lys Val Lys Ser
        115                 120                 125
Thr Glu Asp Arg Gly Phe Gln Arg Phe Leu Asp Asn Val Gln Tyr Lys
    130                 135                 140
Thr Thr Gly Ile Leu Arg Tyr Glu Arg Val Phe Gly Glu Gly Tyr Val
145                 150                 155                 160
Ser Thr Gly Gly Ile Glu Thr Thr Lys Glu Phe Val Asp Lys Leu Asp
                165                 170                 175
Leu Lys Pro Gly Gln Lys Val Leu Asp Val Gly Cys Gly Ile Gly Gly
            180                 185                 190
Gly Asp Phe Tyr Met Ala Glu Asn Tyr Asp Ala His Val Leu Gly Ile
        195                 200                 205
Asp Leu Ser Ile Asn Met Val Ser Phe Ala Ile Glu Arg Ala Ile Gly
    210                 215                 220
Arg Lys Cys Ser Val Glu Phe Glu Val Ala Asp Cys Thr Thr Lys Thr
225                 230                 235                 240
Tyr Ala Pro Asn Thr Phe Asp Val Ile Tyr Ser Arg Asp Thr Ile Leu
                245                 250                 255
His Ile His Asp Lys Pro Ala Leu Phe Arg Ser Phe Phe Lys Trp Leu
            260                 265                 270
Lys Pro Gly Gly Lys Val Leu Ile Ser Asp Tyr Cys Arg Asn Pro Gly
        275                 280                 285
Lys Pro Ser Glu Glu Phe Ala Ala Tyr Ile Lys Gln Arg Gly Tyr Asp
    290                 295                 300
Leu His Asp Val Lys Thr Tyr Gly Lys Met Leu Glu Asp Ala Gly Phe
305                 310                 315                 320
His His Val Ile Ala Glu Asp Arg Thr Asp Gln Phe Leu Arg Val Leu
```

```
                    325                 330                 335
Gln Arg Glu Leu Ala Glu Val Glu Lys Asn Lys Glu Ala Phe Met Ala
                340                 345                 350
Asp Phe Thr Gln Glu Asp Tyr Asp Asp Ile Val Asn Gly Trp Asn Ala
            355                 360                 365
Lys Leu Lys Arg Ser Ser Ala Gly Glu Gln Arg Trp Gly Leu Phe Ile
    370                 375                 380
Ala Thr Lys
385
```

<210> 73
<211> 1446
<212> DNA
<213> Populus trichocarpa

<400> 73

```
atggctactc atgtggaaga acgcgatatt cagaagaagt attggatgga taacatttcc      60
gatttgagtg tgaatgcaat gatgcttgac tcgaaagcat ccgaacttga caaggaagaa     120
cgacctgaga tactttctct gcttccacct tatgaaggaa aaacagtttt ggaactcgga     180
gctggtattg gccgtttcac aggggaatta gcacagaagg ctggccaagt agtggctttg     240
gacttcattg agagtgcaat aaaaaagaat gaaaatatca acggacacta taagaatgtc     300
aagtttatgt gcgctgatgt gacatcccca gatctgaata tttcagaggg gtcggtggat     360
ttgatattct caaattggct tctcatgtat ctctctgaca aagaggtgga gaatctggta     420
gaaaggatgg tcaaatgggt gaaggttgat gggtttattt tcttcagaga gtcttgtttt     480
catcaatctg gagattctaa gcgaaaatac aacccaaccc attaccggga acccagattc     540
tacacgaagg tgtttaaaga atgccatacg cgtgatgggt ctggagattc tttcgaactc     600
tctcttgttg gctgcaaatg catctcagct tatatttgtt ggatatggca gaaagttagt     660
tcatatgagg ataaggggtt ccagcgattc ttagataatg ttcagtataa atccaatggc     720
atattacgtt atgagcgtgt ctttggacaa ggttatgtga gtacaggagg aattgaaaca     780
actaaagaat ttgtgggaaa actggatctt aagcctggcc agaaagtcct agatgttggc     840
tgtgggattg gggaggtga cttttacatg gctgagaact ttgatgtgga ggttgtaggc      900
attgacctct ccataaatat gatttcgttt gcccttgaac gtgccattgg gctcaaatgt     960
tctgtggagt ttgaagttgc tgattgtact acaaagacat atcctgacaa cacatttgat    1020
gttatctaca gccgtgacac cattttgcac attcaagaca aacctgcatt atttagatct    1080
ttcttcaagt ggttgaagcc tggaggtaaa gtacttatca gtgattactg caagtgtgat    1140
ggaactccat caccagaatt cgccgagtac attaaacaga gaggatatga tcttcatgat    1200
gtaaaagcat atggccagat gcttagggat gctggttttg atgaggtcgt tgcagaggac    1260
cgaactgatc agttcaacaa agttctgcaa agggagttaa atgctataga gaaggacaag    1320
gatgagttca tccacgactt ttccgaaggg gactataatg atatagttgg tggatggaag    1380
gcaaagctga tcaggagttc atctggggag cagcgatggg gcctgttcat cgccaagaaa    1440
aaatga                                                               1446
```

<210> 74
<211> 481
<212> PRT
<213> Populus trichocarpa

<400> 74

```
Met Ala Thr His Val Glu Glu Arg Asp Ile Gln Lys Lys Tyr Trp Met
1               5                   10                  15
Asp Asn Ile Ser Asp Leu Ser Val Asn Ala Met Met Leu Asp Ser Lys
            20                  25                  30
Ala Ser Glu Leu Asp Lys Glu Glu Arg Pro Glu Ile Leu Ser Leu Leu
        35                  40                  45
Pro Pro Tyr Glu Gly Lys Thr Val Leu Glu Leu Gly Ala Gly Ile Gly
    50                  55                  60
Arg Phe Thr Gly Glu Leu Ala Gln Lys Ala Gly Gln Val Val Ala Leu
65                  70                  75                  80
Asp Phe Ile Glu Ser Ala Ile Lys Lys Asn Glu Asn Ile Asn Gly His
                85                  90                  95
Tyr Lys Asn Val Lys Phe Met Cys Ala Asp Val Thr Ser Pro Asp Leu
            100                 105                 110
Asn Ile Ser Glu Gly Ser Val Asp Leu Ile Phe Ser Asn Trp Leu Leu
```

```
        115                 120                 125
Met Tyr Leu Ser Asp Lys Glu Val Glu Asn Leu Val Glu Arg Met Val
    130                 135                 140
Lys Trp Val Lys Val Asp Gly Phe Ile Phe Phe Arg Glu Ser Cys Phe
145                 150                 155                 160
His Gln Ser Gly Asp Ser Lys Arg Lys Tyr Asn Pro Thr His Tyr Arg
                165                 170                 175
Glu Pro Arg Phe Tyr Thr Lys Val Phe Lys Glu Cys His Thr Arg Asp
            180                 185                 190
Gly Ser Gly Asp Ser Phe Glu Leu Ser Leu Val Gly Cys Lys Cys Ile
        195                 200                 205
Ser Ala Tyr Ile Cys Trp Ile Trp Gln Lys Val Ser Ser Tyr Glu Asp
    210                 215                 220
Lys Gly Phe Gln Arg Phe Leu Asp Asn Val Gln Tyr Lys Ser Asn Gly
225                 230                 235                 240
Ile Leu Arg Tyr Glu Arg Val Phe Gly Gln Gly Tyr Val Ser Thr Gly
                245                 250                 255
Gly Ile Glu Thr Thr Lys Glu Phe Val Gly Lys Leu Asp Leu Lys Pro
            260                 265                 270
Gly Gln Lys Val Leu Asp Val Gly Cys Gly Ile Gly Gly Gly Asp Phe
        275                 280                 285
Tyr Met Ala Glu Asn Phe Asp Val Glu Val Val Gly Ile Asp Leu Ser
    290                 295                 300
Ile Asn Met Ile Ser Phe Ala Leu Glu Arg Ala Ile Gly Leu Lys Cys
305                 310                 315                 320
Ser Val Glu Phe Glu Val Ala Asp Cys Thr Thr Lys Thr Tyr Pro Asp
                325                 330                 335
Asn Thr Phe Asp Val Ile Tyr Ser Arg Asp Thr Ile Leu His Ile Gln
            340                 345                 350
Asp Lys Pro Ala Leu Phe Arg Ser Phe Phe Lys Trp Leu Lys Pro Gly
        355                 360                 365
Gly Lys Val Leu Ile Ser Asp Tyr Cys Lys Cys Asp Gly Thr Pro Ser
    370                 375                 380
Pro Glu Phe Ala Glu Tyr Ile Lys Gln Arg Gly Tyr Asp Leu His Asp
385                 390                 395                 400
Val Lys Ala Tyr Gly Gln Met Leu Arg Asp Ala Gly Phe Asp Glu Val
                405                 410                 415
Val Ala Glu Asp Arg Thr Asp Gln Phe Asn Lys Val Leu Gln Arg Glu
            420                 425                 430
Leu Asn Ala Ile Glu Lys Asp Lys Asp Glu Phe Ile His Asp Phe Ser
        435                 440                 445
Glu Gly Asp Tyr Asn Asp Ile Val Gly Gly Trp Lys Ala Lys Leu Ile
    450                 455                 460
Arg Ser Ser Ser Gly Glu Gln Arg Trp Gly Leu Phe Ile Ala Lys Lys
465                 470                 475                 480
Lys
```

<210> 75
<211> 1038
<212> DNA
<213> Populus trichocarpa

<400> 75

```
atgacttatg ttgtgttgaa aggatatcta tatgatccga ttgattgcgt aaggcacgcg      60
gtagcaacgg aaccggggaa agtggagaat ctggttgaaa ggatggtcaa atggctaaag     120
gttggggggt tcattttctt tagagagtct tgttttcatc aatctggaga ttccaagcga     180
aaatacaacc caacccacta ccgtgaaccc agattctaca caaagatttg ttggatatgg     240
cagaaagtca gttcaaatga tgataagggg ttccagcgat tcttagataa tgtccaatat     300
aaatctaatg gcatattacg ttatgagcgc gtctttggtc aaggttttgt gagcacagga     360
ggaatggaga caactaaaga atttgtggaa aagctggatc ttaagcctgg ccagaaagtc     420
ctagatgttg gctgtgggat tgggggaggt gacttttaca tggctgagaa ctttgaagtg     480
gaggttgtag gcattgacct ctccgtaaat atgatttcat ttgctctcga acgtgccatt     540
```

```
ggactcaaat gctctgttga gtttgaagtt gctgattgca ctacgaagac atatcctgac     600
aatacttttg atgttatcta cagccgggac accattttgc acattcaaga caaacctgca     660
ttatttagat ctttcttcaa gtggctgaag cctggaggta aagtacttat cagtgattac     720
tgcaagtgtg ctggaactcc atcaccagaa tttgcagagt acattaaaca gagaggatat     780
gatcttcatg atgtgaaagc atatggccag atgcttaggg atgctggttt tgatgaggtc     840
attgcagaag accgaactga tcagttcaac caagttctgc taagggaatt aaaagctata     900
gaaaaggaga aggatgaatt tatccatgac ttctctgaag aagactataa tgatatagtt     960
ggtggatgga aggcaaagct gatcaggagt tcatctggcg agcagcgatg ggcctgttc    1020
attgccaaga aaaaatga                                                  1038

<210>  76
<211>  345
<212>  PRT
<213>  Populus trichocarpa

<400>  76
Met Thr Tyr Val Val Leu Lys Gly Tyr Leu Tyr Asp Pro Ile Asp Cys
1               5                   10                  15
Val Arg His Ala Val Ala Thr Glu Pro Gly Lys Val Glu Asn Leu Val
            20                  25                  30
Glu Arg Met Val Lys Trp Leu Lys Val Gly Gly Phe Ile Phe Phe Arg
        35                  40                  45
Glu Ser Cys Phe His Gln Ser Gly Asp Ser Lys Arg Lys Tyr Asn Pro
    50                  55                  60
Thr His Tyr Arg Glu Pro Arg Phe Tyr Thr Lys Ile Cys Trp Ile Trp
65                  70                  75                  80
Gln Lys Val Ser Ser Asn Asp Asp Lys Gly Phe Gln Arg Phe Leu Asp
                85                  90                  95
Asn Val Gln Tyr Lys Ser Asn Gly Ile Leu Arg Tyr Glu Arg Val Phe
            100                 105                 110
Gly Gln Gly Phe Val Ser Thr Gly Gly Met Glu Thr Thr Lys Glu Phe
        115                 120                 125
Val Glu Lys Leu Asp Leu Lys Pro Gly Gln Lys Val Leu Asp Val Gly
    130                 135                 140
Cys Gly Ile Gly Gly Gly Asp Phe Tyr Met Ala Glu Asn Phe Glu Val
145                 150                 155                 160
Glu Val Val Gly Ile Asp Leu Ser Val Asn Met Ile Ser Phe Ala Leu
                165                 170                 175
Glu Arg Ala Ile Gly Leu Lys Cys Ser Val Glu Phe Glu Val Ala Asp
            180                 185                 190
Cys Thr Thr Lys Thr Tyr Pro Asp Asn Thr Phe Asp Val Ile Tyr Ser
        195                 200                 205
Arg Asp Thr Ile Leu His Ile Gln Asp Lys Pro Ala Leu Phe Arg Ser
    210                 215                 220
Phe Phe Lys Trp Leu Lys Pro Gly Gly Lys Val Leu Ile Ser Asp Tyr
225                 230                 235                 240
Cys Lys Cys Ala Gly Thr Pro Ser Pro Glu Phe Ala Glu Tyr Ile Lys
                245                 250                 255
Gln Arg Gly Tyr Asp Leu His Asp Val Lys Ala Tyr Gly Gln Met Leu
            260                 265                 270
Arg Asp Ala Gly Phe Asp Glu Val Ile Ala Glu Asp Arg Thr Asp Gln
        275                 280                 285
Phe Asn Gln Val Leu Leu Arg Glu Leu Lys Ala Ile Glu Lys Glu Lys
    290                 295                 300
Asp Glu Phe Ile His Asp Phe Ser Glu Glu Asp Tyr Asn Asp Ile Val
305                 310                 315                 320
Gly Gly Trp Lys Ala Lys Leu Ile Arg Ser Ser Ser Gly Glu Gln Arg
                325                 330                 335
Trp Gly Leu Phe Ile Ala Lys Lys Lys
            340                 345

<210>  77
<211>  1506
```

<212> DNA
<213> Zea Mays

<400> 77
atggacaccg tcggcgtccc cgtggtggcc gttgcgaatg ggatcgggga ggtggagcgc 60
aaggtgcaga agagctactg ggaggagcac tccaagtgcc tcactgtcga gtccatgatg 120
ctcgactccc gcgccgccga cctcgacaag gaagagcgac ccgagatcct gtctttgctt 180
ccctcttaca aagggaaatc agttctagaa ctcggtgctg gaattggacg ctttactgga 240
gatctggcaa aagaagctgg gcacgttctg gcactagact ttattgaaag tgtgattaag 300
aagaaccaaa gcataaatgg gcatcacaag aacataacct tcaggtgtgc cgatgtgaca 360
tctaacgact tgaagattga agataactct gttgatctga tattttcaaa ctggctatta 420
atgtatcttt cagatgagga ggtccaaaag cttgtgggga aaatggtaaa atggttaaag 480
gtcggaggcc atattttctt tagagaatca tgttttcacc aatctggaga ttccaaaagg 540
aaggtgaacc caacacacta tcgagaacca aggttttata ccaaggtatt taaagagggc 600
cattcatttg atcaagatgg aggttcgttt gaactttctc tagtgacctg taaatgcatt 660
ggggcttatg tcaaaaacaa gaagaatcaa aaccagatat gctggttatg ggaaaaggta 720
aaatcaacag aagacagaga ttttcaaaga ttcctggaca acgtgcaata caaaacaagt 780
gggatattac gttacgagcg tgtctttggt gaaggttttg tgagcactgg tggaatcgag 840
acaacaaagg aatttgtggg catgctcgat cttaaaccgg gccagaaagt acttgatgtc 900
ggatgtggaa ttggaggcgg cgacttttac atggctgcaa actatgatgt ccatgttctt 960
ggtattgatc tttcggtgaa catggtttca tttgcaattg aacgtgccat tggacgcaag 1020
tgctctgttg aatttgaagt tgctgattgc accacaaagg attacccaga aaatagtttt 1080
gacgtcatct acagccgtga caccatcctt cacatacaag acaagcctgc tctgttcaga 1140
agcttcttca aatggctaaa gcccggtggc aaagtcctaa tcagcgacta ctgtaagaat 1200
cctggaaaac catcagaaga atttgctgcg tacattaagc agagaggcta tgaccttcac 1260
gacgtgaagg cttatggaca gatgctgaag gatgctggtt ttcataatgt catcgcggaa 1320
gatcgcactg agcagttctt gaatgttcta cagagggagc taggtgaagt tgaaaagaac 1380
aaagacgctt tcctggcaga cttcacccag gaggactatg acgacattgt gaatggctgg 1440
aacgcgaagc tgaaacggag ctctgccggc gagcagaggt gggggttgtt cattgccacc 1500
aagtga 1506

<210> 78
<211> 501
<212> PRT
<213> Zea Mays

<400> 78
Met Asp Thr Val Gly Val Pro Val Val Ala Val Ala Asn Gly Ile Gly
1 5 10 15
Glu Val Glu Arg Lys Val Gln Lys Ser Tyr Trp Glu Glu His Ser Lys
20 25 30
Cys Leu Thr Val Glu Ser Met Met Leu Asp Ser Arg Ala Ala Asp Leu
35 40 45
Asp Lys Glu Glu Arg Pro Glu Ile Leu Ser Leu Leu Pro Ser Tyr Lys
50 55 60
Gly Lys Ser Val Leu Glu Leu Gly Ala Gly Ile Gly Arg Phe Thr Gly
65 70 75 80
Asp Leu Ala Lys Glu Ala Gly His Val Leu Ala Leu Asp Phe Ile Glu
85 90 95
Ser Val Ile Lys Lys Asn Gln Ser Ile Asn Gly His His Lys Asn Ile
100 105 110
Thr Phe Arg Cys Ala Asp Val Thr Ser Asn Asp Leu Lys Ile Glu Asp
115 120 125
Asn Ser Val Asp Leu Ile Phe Ser Asn Trp Leu Leu Met Tyr Leu Ser
130 135 140
Asp Glu Glu Val Gln Lys Leu Val Gly Lys Met Val Lys Trp Leu Lys
145 150 155 160
Val Gly Gly His Ile Phe Phe Arg Glu Ser Cys Phe His Gln Ser Gly
165 170 175
Asp Ser Lys Arg Lys Val Asn Pro Thr His Tyr Arg Glu Pro Arg Phe
180 185 190
Tyr Thr Lys Val Phe Lys Glu Gly His Ser Phe Asp Gln Asp Gly Gly
195 200 205

```
Ser Phe Glu Leu Ser Leu Val Thr Cys Lys Cys Ile Gly Ala Tyr Val
    210                 215                 220
Lys Asn Lys Lys Asn Gln Asn Gln Ile Cys Trp Leu Trp Glu Lys Val
225                 230                 235                 240
Lys Ser Thr Glu Asp Arg Asp Phe Gln Arg Phe Leu Asp Asn Val Gln
                245                 250                 255
Tyr Lys Thr Ser Gly Ile Leu Arg Tyr Glu Arg Val Phe Gly Glu Gly
            260                 265                 270
Phe Val Ser Thr Gly Gly Ile Glu Thr Thr Lys Glu Phe Val Gly Met
        275                 280                 285
Leu Asp Leu Lys Pro Gly Gln Lys Val Leu Asp Val Gly Cys Gly Ile
    290                 295                 300
Gly Gly Gly Asp Phe Tyr Met Ala Ala Asn Tyr Asp Val His Val Leu
305                 310                 315                 320
Gly Ile Asp Leu Ser Val Asn Met Val Ser Phe Ala Ile Glu Arg Ala
                325                 330                 335
Ile Gly Arg Lys Cys Ser Val Glu Phe Glu Val Ala Asp Cys Thr Thr
            340                 345                 350
Lys Asp Tyr Pro Glu Asn Ser Phe Asp Val Ile Tyr Ser Arg Asp Thr
        355                 360                 365
Ile Leu His Ile Gln Asp Lys Pro Ala Leu Phe Arg Ser Phe Phe Lys
    370                 375                 380
Trp Leu Lys Pro Gly Gly Lys Val Leu Ile Ser Asp Tyr Cys Lys Asn
385                 390                 395                 400
Pro Gly Lys Pro Ser Glu Glu Phe Ala Ala Tyr Ile Lys Gln Arg Gly
                405                 410                 415
Tyr Asp Leu His Asp Val Lys Ala Tyr Gly Gln Met Leu Lys Asp Ala
            420                 425                 430
Gly Phe His Asn Val Ile Ala Glu Asp Arg Thr Glu Gln Phe Leu Asn
        435                 440                 445
Val Leu Gln Arg Glu Leu Gly Glu Val Glu Lys Asn Lys Asp Ala Phe
    450                 455                 460
Leu Ala Asp Phe Thr Gln Glu Asp Tyr Asp Asp Ile Val Asn Gly Trp
465                 470                 475                 480
Asn Ala Lys Leu Lys Arg Ser Ser Ala Gly Glu Gln Arg Trp Gly Leu
                485                 490                 495
Phe Ile Ala Thr Lys
            500
```

<210> 79
<211> 1488
<212> DNA
<213> Zea Mays

<400> 79

```
atggccgccg ccgtgaatgg gagcctagac gtgcatgaga ggaaggcgca gaagagctac      60
tgggaggagc actccgggga gctcaacctc gaggccatta tgctcgactc ccgtgccgcc     120
gaactcgaca aggaggagcg ccccgaggtt ctgtctttac ttccttcata tgaagggaaa     180
tctatactgg agctgggagc tggaataggc cgctttactg gtgaactggc taaaacatct     240
gggcatgttt ttgcagtgga tttcgttgaa agtgtgatta aaaagaatgg aagtataaat     300
gatcactatg gcaacacatc ctttatgtgt gctgatgtta catccccgga cctgatgatt     360
gaagcaaact ccattgatct gatattttca aactggttgc tgatgtatct ttcagatgag     420
gagattgaca agttggtaga aagaatggta aaatggttga aggtcggtgg ttatatcttc     480
tttagggaat cttgcttcca tcaatccgga gatacagaaa ggaaatttaa tccaacacac     540
tatcgagaac caaggtttta taccaaggta tttaaagaat gccaaacctt taatcaggat     600
ggcacttcct tcaaactttc tttgattaca ttcaaatgca ttggagctta tgtaaacatc     660
aagaaagatc aaaaccagat atgttggcta tggaaaaaag taaactcatc agaagatggg     720
ggatttcaaa gtttttggga caatgtgcag tacaaagcca ctggaatact acgctatgaa     780
cgtatctttg gagatggcta cgtgagtact ggtggagctg agactacaaa agaatttgtg     840
gagaaactga atcttaagcc tgggcagaag gtgcttgatg ttggatgtgg aattggggga     900
ggtgactttt atatggctga gaagtatggt acacatgtcg ttggtattga cctttccatt     960
aacatgataa tgtttgccct tgagcgttcc attgggtgta agtgcttagt tgagtttgaa    1020
gttgctgatt gcaccacaaa gacataccca gaccacatgt ttgatgtcat ctacagtcgt    1080
```

```
gacactatcc ttcatataca agataaaccc tccttgttta aaagtttctt caaatggctg   1140
aaacctgggg gaaaggttct aatcagtgat tactgcaaga gtcctggaaa accatcagaa   1200
gagtttgcaa catacattaa gcagaggggt tatgatctcc atgacgtgga ggcttatgga   1260
cagatgctga aggatgctgg ttttcataat gtcatcgcgg aagatcgcac tgagcagttc   1320
ttgaatgttc tacagaggga gataggtgaa gttgaaaaga acaaagacgc tttcctggca   1380
gacttcaccc aggaggacta tgacgacatt gtgaacggct ggaacgcgaa gctgaaacgg   1440
agctctggcg gtgagcagag gtgggggttg ttcattgcca ccaagtga                 1488
```

```
<210>  80
<211>  495
<212>  PRT
<213>  Zea Mays

<400>  80
Met Ala Ala Ala Val Asn Gly Ser Leu Asp Val His Glu Arg Lys Ala
1               5                   10                  15
Gln Lys Ser Tyr Trp Glu Glu His Ser Gly Glu Leu Asn Leu Glu Ala
            20                  25                  30
Ile Met Leu Asp Ser Arg Ala Ala Glu Leu Asp Lys Glu Glu Arg Pro
        35                  40                  45
Glu Val Leu Ser Leu Leu Pro Ser Tyr Glu Gly Lys Ser Ile Leu Glu
    50                  55                  60
Leu Gly Ala Gly Ile Gly Arg Phe Thr Gly Glu Leu Ala Lys Thr Ser
65                  70                  75                  80
Gly His Val Phe Ala Val Asp Phe Val Glu Ser Val Ile Lys Lys Asn
                85                  90                  95
Gly Ser Ile Asn Asp His Tyr Gly Asn Thr Ser Phe Met Cys Ala Asp
            100                 105                 110
Val Thr Ser Pro Asp Leu Met Ile Glu Ala Asn Ser Ile Asp Leu Ile
        115                 120                 125
Phe Ser Asn Trp Leu Leu Met Tyr Leu Ser Asp Glu Glu Ile Asp Lys
    130                 135                 140
Leu Val Glu Arg Met Val Lys Trp Leu Lys Val Gly Gly Tyr Ile Phe
145                 150                 155                 160
Phe Arg Glu Ser Cys Phe His Gln Ser Gly Asp Thr Glu Arg Lys Phe
                165                 170                 175
Asn Pro Thr His Tyr Arg Glu Pro Arg Phe Tyr Thr Lys Val Phe Lys
            180                 185                 190
Glu Cys Gln Thr Phe Asn Gln Asp Gly Thr Ser Phe Lys Leu Ser Leu
        195                 200                 205
Ile Thr Phe Lys Cys Ile Gly Ala Tyr Val Asn Ile Lys Lys Asp Gln
    210                 215                 220
Asn Gln Ile Cys Trp Leu Trp Lys Lys Val Asn Ser Ser Glu Asp Gly
225                 230                 235                 240
Gly Phe Gln Ser Phe Leu Asp Asn Val Gln Tyr Lys Ala Thr Gly Ile
                245                 250                 255
Leu Arg Tyr Glu Arg Ile Phe Gly Asp Gly Tyr Val Ser Thr Gly Gly
            260                 265                 270
Ala Glu Thr Thr Lys Glu Phe Val Glu Lys Leu Asn Leu Lys Pro Gly
        275                 280                 285
Gln Lys Val Leu Asp Val Gly Cys Gly Ile Gly Gly Gly Asp Phe Tyr
    290                 295                 300
Met Ala Glu Lys Tyr Gly Thr His Val Val Gly Ile Asp Leu Ser Ile
305                 310                 315                 320
Asn Met Ile Met Phe Ala Leu Glu Arg Ser Ile Gly Cys Lys Cys Leu
                325                 330                 335
Val Glu Phe Glu Val Ala Asp Cys Thr Thr Lys Thr Tyr Pro Asp His
            340                 345                 350
Met Phe Asp Val Ile Tyr Ser Arg Asp Thr Ile Leu His Ile Gln Asp
        355                 360                 365
Lys Pro Ser Leu Phe Lys Ser Phe Phe Lys Trp Leu Lys Pro Gly Gly
    370                 375                 380
Lys Val Leu Ile Ser Asp Tyr Cys Lys Ser Pro Gly Lys Pro Ser Glu
```

385 390 395 400
Glu Phe Ala Thr Tyr Ile Lys Gln Arg Gly Tyr Asp Leu His Asp Val
405 410 415
Glu Ala Tyr Gly Gln Met Leu Lys Asp Ala Gly Phe His Asn Val Ile
420 425 430
Ala Glu Asp Arg Thr Glu Gln Phe Leu Asn Val Leu Gln Arg Glu Ile
435 440 445
Gly Glu Val Glu Lys Asn Lys Asp Ala Phe Leu Ala Asp Phe Thr Gln
450 455 460
Glu Asp Tyr Asp Asp Ile Val Asn Gly Trp Asn Ala Lys Leu Lys Arg
465 470 475 480
Ser Ser Gly Gly Glu Gln Arg Trp Gly Leu Phe Ile Ala Thr Lys
485 490 495

<210> 81
<211> 1086
<212> DNA
<213> Zea Mays

<400> 81
atgtatcttt cagatgaaga ggttgaacag ctagttcaga gaatggtaaa atggttgaag 60
gttggtggct atatcttctt tagggaatct tgcttccatc aatctggaga ttcaaaaagg 120
aaagttaatc cgacacacta tagggaacca agtttttata ctaaggtttt caaagaatgc 180
catacctttg atcaagatgg gaattctttc gaactttctc tggttacttg caagtgtatt 240
ggtgcttatg ttaaaaacaa gaaaaaccaa aaccagatat gttggctatg gcaaaaggtc 300
cattctacag aagataaagg atttcaaaga tttttggaca atgtgcagta caaagccagt 360
ggaatattac gttacgagcg catttttgga gaaggttatg tgagcactgg tggagttgag 420
actacaaaag aatttgtgga caagctggat ctcaaacctg gacataaggt gcttgatgtt 480
ggatgtggaa ttgggggagg tgacttttat atggccgaaa aatatgatgc tcatgttgtt 540
ggtattgatc tttccataaa catggtatca tttgcacttg agcgtgccat tgggcgcagt 600
tgctcagtgg agtttgaagt tgctgattgc actacgaaga catacccaga caacacattt 660
gatgtcatat acagccgtga tactatcctt cacatacatg acaaaccctc tttgttcaaa 720
agtttttttca agtggctgaa gcctgggggc aaggtcctta tcagtgacta ctgcaggagt 780
cctgggaaac catcagagga atttgcagcg tacattaagc agagaggtta tgacctacat 840
gctgtggagg cttatggaca gatgttgaag agtgctggtt ttcgtgatgt cattgctgag 900
gatcgaactg atcagttcct tggtgtttta gataaggagt tagctgaatt tgaaaagaac 960
aaggacgatt tcctgtctga cttcacccag gaggactacg atgatatcgt gaacggttgg 1020
aaggcaaaac tgcagaggag ttctgctggt gaacagaggt gggggctgtt catcgccacc 1080
aaatga 1086

<210> 82
<211> 361
<212> PRT
<213> Zea Mays

<400> 82
Met Tyr Leu Ser Asp Glu Glu Val Glu Gln Leu Val Gln Arg Met Val
1 5 10 15
Lys Trp Leu Lys Val Gly Gly Tyr Ile Phe Phe Arg Glu Ser Cys Phe
20 25 30
His Gln Ser Gly Asp Ser Lys Arg Lys Val Asn Pro Thr His Tyr Arg
35 40 45
Glu Pro Ser Phe Tyr Thr Lys Val Phe Lys Glu Cys His Thr Phe Asp
50 55 60
Gln Asp Gly Asn Ser Phe Glu Leu Ser Leu Val Thr Cys Lys Cys Ile
65 70 75 80
Gly Ala Tyr Val Lys Asn Lys Lys Asn Gln Asn Gln Ile Cys Trp Leu
85 90 95
Trp Gln Lys Val His Ser Thr Glu Asp Lys Gly Phe Gln Arg Phe Leu
100 105 110
Asp Asn Val Gln Tyr Lys Ala Ser Gly Ile Leu Arg Tyr Glu Arg Ile
115 120 125
Phe Gly Glu Gly Tyr Val Ser Thr Gly Gly Val Glu Thr Thr Lys Glu

```
    130                 135                 140
Phe Val Asp Lys Leu Asp Leu Lys Pro Gly His Lys Val Leu Asp Val
145                 150                 155                 160
Gly Cys Gly Ile Gly Gly Gly Asp Phe Tyr Met Ala Glu Lys Tyr Asp
                165                 170                 175
Ala His Val Val Gly Ile Asp Leu Ser Ile Asn Met Val Ser Phe Ala
            180                 185                 190
Leu Glu Arg Ala Ile Gly Arg Ser Cys Ser Val Glu Phe Glu Val Ala
        195                 200                 205
Asp Cys Thr Thr Lys Thr Tyr Pro Asp Asn Thr Phe Asp Val Ile Tyr
    210                 215                 220
Ser Arg Asp Thr Ile Leu His Ile His Asp Lys Pro Ser Leu Phe Lys
225                 230                 235                 240
Ser Phe Phe Lys Trp Leu Lys Pro Gly Gly Lys Val Leu Ile Ser Asp
                245                 250                 255
Tyr Cys Arg Ser Pro Gly Lys Pro Ser Glu Glu Phe Ala Ala Tyr Ile
            260                 265                 270
Lys Gln Arg Gly Tyr Asp Leu His Ala Val Glu Ala Tyr Gly Gln Met
        275                 280                 285
Leu Lys Ser Ala Gly Phe Arg Asp Val Ile Ala Glu Asp Arg Thr Asp
    290                 295                 300
Gln Phe Leu Gly Val Leu Asp Lys Glu Leu Ala Glu Phe Glu Lys Asn
305                 310                 315                 320
Lys Asp Asp Phe Leu Ser Asp Phe Thr Gln Glu Asp Tyr Asp Asp Ile
                325                 330                 335
Val Asn Gly Trp Lys Ala Lys Leu Gln Arg Ser Ser Ala Gly Glu Gln
            340                 345                 350
Arg Trp Gly Leu Phe Ile Ala Thr Lys
        355                 360

<210>  83
<211>  56
<212>  DNA
<213>  Artificial sequence

<220>
<223>  primer 1

<400>  83
ggggacaagt ttgtacaaaa aagcaggctt aaacaatgga gcattctagt gatttg         56

<210>  84
<211>  50
<212>  DNA
<213>  Artificial sequence

<220>
<223>  primer 2

<400>  84
ggggaccact ttgtacaaga aagctgggtc agagttttgg gataaaaaca                50

<210>  85
<211>  2194
<212>  DNA
<213>  Oryza sativa

<400>  85
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
```

```
aaaaaatctt tctagctgaa ctcaatgggt aaagagagag attttttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc ttttttcgat tagatgcaag    540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa cctttaaca gcaggctttg cggccaggag agaggaggag aggcaaagaa    960
aaccaagcat cctccttctc ccatctataa attcctcccc ccttttcccc tctctatata   1020
ggaggcatcc aagccaagaa gagggagagc accaaggaca cgcgactagc agaagccgag   1080
cgaccgcctt ctcgatccat atcttccggt cgagttcttg gtcgatctct tccctcctcc   1140
acctcctcct cacagggtat gtgcctccct tcggttgttc ttggatttat tgttctaggt   1200
tgtgtagtac gggcgttgat gttaggaaag gggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aattttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
ctgtcctcaa ttttgtttc aaattcacat cgattatcta tgcattatcc tcttgtatct   2040
acctgtagaa gtttcttttt ggttattcct tgactgcttg attacagaaa gaaatttatg   2100
aagctgtaat cgggatagtt atactgcttg ttcttatgat tcatttcctt tgtgcagttc   2160
ttggtgtagc ttgccacttt caccagcaaa gttc                                2194
```

<210> 86
<211> 110
<212> PRT
<213> Artificial sequence

<220>
<223> Methyltransferase type 11 domain

<400> 86

```
Pro Pro Tyr Glu Gly Lys Ser Val Leu Glu Leu Gly Ala Gly Ile Gly
1               5                   10                  15
Arg Phe Thr Gly Glu Leu Ala Gln Lys Ala Gly Glu Val Ile Ala Leu
            20                  25                  30
Asp Ile Ile Glu Ser Ala Ile Gln Lys Asn Glu Ser Val Asn Gly His
        35                  40                  45
Tyr Lys Asn Ile Lys Phe Met Cys Ala Asp Val Thr Ser Pro Asp Leu
    50                  55                  60
Lys Ile Lys Asp Gly Ser Ile Asp Leu Ile Phe Ser Asn Trp Leu Leu
65                  70                  75                  80
Met Tyr Leu Ser Asp Lys Glu Val Glu Leu Met Ala Glu Arg Met Ile
                85                  90                  95
Gly Trp Val Lys Pro Gly Gly Tyr Ile Phe Phe Arg Glu Ser
            100                 105                 110
```

<210> 87
<211> 108
<212> PRT
<213> Artificial sequence

<220>

<223> Methyltransferase type 11 domain

<400> 87
Asp Leu Lys Pro Gly Gln Lys Val Leu Asp Val Gly Cys Gly Ile Gly
1 5 10 15
Gly Gly Asp Phe Tyr Met Ala Glu Asn Phe Asp Val His Val Val Gly
20 25 30
Ile Asp Leu Ser Val Asn Met Ile Ser Phe Ala Leu Glu Arg Ala Ile
35 40 45
Gly Leu Lys Cys Ser Val Glu Phe Glu Val Ala Asp Cys Thr Thr Lys
50 55 60
Thr Tyr Pro Asp Asn Ser Phe Asp Val Ile Tyr Ser Arg Asp Thr Ile
65 70 75 80
Leu His Ile Gln Asp Lys Pro Ala Leu Phe Arg Thr Phe Phe Lys Trp
85 90 95
Leu Lys Pro Gly Gly Lys Val Leu Ile Thr Asp Tyr
100 105

<210> 88
<211> 180
<212> PRT
<213> Artificial sequence

<220>
<223> ubiE/COQ5 methyltransferase domain

<400> 88
Glu Arg Val Phe Gly Glu Gly Tyr Val Ser Thr Gly Gly Phe Glu Thr
1 5 10 15
Thr Lys Glu Phe Val Ala Lys Met Asp Leu Lys Pro Gly Gln Lys Val
20 25 30
Leu Asp Val Gly Cys Gly Ile Gly Gly Gly Asp Phe Tyr Met Ala Glu
35 40 45
Asn Phe Asp Val His Val Val Gly Ile Asp Leu Ser Val Asn Met Ile
50 55 60
Ser Phe Ala Leu Glu Arg Ala Ile Gly Leu Lys Cys Ser Val Glu Phe
65 70 75 80
Glu Val Ala Asp Cys Thr Thr Lys Thr Tyr Pro Asp Asn Ser Phe Asp
85 90 95
Val Ile Tyr Ser Arg Asp Thr Ile Leu His Ile Gln Asp Lys Pro Ala
100 105 110
Leu Phe Arg Thr Phe Phe Lys Trp Leu Lys Pro Gly Gly Lys Val Leu
115 120 125
Ile Thr Asp Tyr Cys Arg Ser Ala Glu Thr Pro Ser Pro Glu Phe Ala
130 135 140
Glu Tyr Ile Lys Gln Arg Gly Tyr Asp Leu His Asp Val Gln Ala Tyr
145 150 155 160
Gly Gln Met Leu Lys Asp Ala Gly Phe Asp Asp Val Ile Ala Glu Asp
165 170 175
Arg Thr Asp Gln
180

<210> 89
<211> 13
<212> PRT
<213> Artificial sequence

<220>
<223> motif 5

<400> 89
Ile Phe Phe Arg Glu Ser Cys Phe His Gln Ser Gly Asp
1 5 10

<210> 90
<211> 6
<212> PRT
<213> Artificial sequence

<220>
<223> motif 6

<400> 90
Glu Tyr Ile Lys Gln Arg
1 5

<210> 91
<211> 6
<212> PRT
<213> Artificial sequence

<220>
<223> motif 7

<400> 91
Trp Gly Leu Phe Ile Ala
1 5

<210> 92
<211> 1239
<212> DNA
<213> Arabidopsis thaliana

<400> 92
atggtggcca cctctgctac gtcgtcattc tttcctgtac catcttcttc acttgatcct 60
aatggaaaag gcaataagat tgggtctacg aatcttgctg gactcaattc tgcacctaac 120
tctggtagga tgaaggttaa accaaacgct caggctccac ctaagattaa tgggaaaaag 180
gttggtttgc ctggttctgt agatattgta aggactgata ccgagacctc atcacaccct 240
gcgccgagaa ctttcatcaa ccagttacct gactggagca tgcttcttgc tgctataact 300
acgatttctt tagcggctga gaaacagtgg atgatgcttg attggaaacc taggcgttct 360
gacatgctgg tggatccttt tggtataggg agaattgttc aggatggcct tgtgttccgt 420
cagaattttt ctattaggtc atatgaaata ggtgctgatc gctctgcatc tatagaaacc 480
gtcatgaatc atctgcagga aacggcgctt aatcatgtta agactgctgg attgcttgga 540
gatgggtttg gctctacacc tgagatgttt aagaagaact tgatatgggt tgtcactcgt 600
atgcaggttg tggttgataa atatcctact tggggagatg ttgttgaagt agacacctgg 660
gtcagtcagt ctggaaagaa tggtatgcgt cgtgattggc tagttcggga ctgtaatact 720
ggagaaacct taacacgagc atcaagtgtg tgggtgatga tgaataaact gacaaggaga 780
ttgtcaaaga ttcctgaaga ggttcgaggg gaaatagagc cttattttgt gaattctgat 840
cctgtccttg ccgaggacag cagaaagtta acaaaaattg atgacaagac tgctgactat 900
gttcgatctg gtctcactcc tcgatggagt gacctagatg ttaaccagca tgtgaataat 960
gtaaagtaca ttgggtggat cctggagagt gctccagtgg gaataatgga gaggcagaag 1020
ctgaaaagca tgactctgga gtatcggagg gaatgcggga gagacagtgt gcttcagtcc 1080
ctcactgcag ttacgggttg cgatatcggt aacctggcaa cagcggggga tgtggaatgt 1140
cagcatttgc tccgactcca ggatggagcg gaagtggtga gaggaagaac agagtggagt 1200
agtaaaacac caacaacaac ttggggaact gcaccgtaa 1239

<210> 93
<211> 412
<212> PRT
<213> Arabidopsis thaliana

<400> 93
Met Val Ala Thr Ser Ala Thr Ser Ser Phe Phe Pro Val Pro Ser Ser
1 5 10 15
Ser Leu Asp Pro Asn Gly Lys Gly Asn Lys Ile Gly Ser Thr Asn Leu
20 25 30

```
Ala Gly Leu Asn Ser Ala Pro Asn Ser Gly Arg Met Lys Val Lys Pro
        35                  40                  45
Asn Ala Gln Ala Pro Pro Lys Ile Asn Gly Lys Lys Val Gly Leu Pro
    50                  55                  60
Gly Ser Val Asp Ile Val Arg Thr Asp Thr Glu Thr Ser Ser His Pro
65                  70                  75                  80
Ala Pro Arg Thr Phe Ile Asn Gln Leu Pro Asp Trp Ser Met Leu Leu
                85                  90                  95
Ala Ala Ile Thr Thr Ile Phe Leu Ala Ala Glu Lys Gln Trp Met Met
            100                 105                 110
Leu Asp Trp Lys Pro Arg Arg Ser Asp Met Leu Val Asp Pro Phe Gly
        115                 120                 125
Ile Gly Arg Ile Val Gln Asp Gly Leu Val Phe Arg Gln Asn Phe Ser
    130                 135                 140
Ile Arg Ser Tyr Glu Ile Gly Ala Asp Arg Ser Ala Ser Ile Glu Thr
145                 150                 155                 160
Val Met Asn His Leu Gln Glu Thr Ala Leu Asn His Val Lys Thr Ala
                165                 170                 175
Gly Leu Leu Gly Asp Gly Phe Gly Ser Thr Pro Glu Met Phe Lys Lys
            180                 185                 190
Asn Leu Ile Trp Val Val Thr Arg Met Gln Val Val Val Asp Lys Tyr
        195                 200                 205
Pro Thr Trp Gly Asp Val Val Glu Val Asp Thr Trp Val Ser Gln Ser
    210                 215                 220
Gly Lys Asn Gly Met Arg Arg Asp Trp Leu Val Arg Asp Cys Asn Thr
225                 230                 235                 240
Gly Glu Thr Leu Thr Arg Ala Ser Ser Val Trp Val Met Met Asn Lys
                245                 250                 255
Leu Thr Arg Arg Leu Ser Lys Ile Pro Glu Glu Val Arg Gly Glu Ile
            260                 265                 270
Glu Pro Tyr Phe Val Asn Ser Asp Pro Val Leu Ala Glu Asp Ser Arg
        275                 280                 285
Lys Leu Thr Lys Ile Asp Asp Lys Thr Ala Asp Tyr Val Arg Ser Gly
    290                 295                 300
Leu Thr Pro Arg Trp Ser Asp Leu Asp Val Asn Gln His Val Asn Asn
305                 310                 315                 320
Val Lys Tyr Ile Gly Trp Ile Leu Glu Ser Ala Pro Val Gly Ile Met
                325                 330                 335
Glu Arg Gln Lys Leu Lys Ser Met Thr Leu Glu Tyr Arg Arg Glu Cys
            340                 345                 350
Gly Arg Asp Ser Val Leu Gln Ser Leu Thr Ala Val Thr Gly Cys Asp
        355                 360                 365
Ile Gly Asn Leu Ala Thr Ala Gly Asp Val Glu Cys Gln His Leu Leu
    370                 375                 380
Arg Leu Gln Asp Gly Ala Glu Val Val Arg Gly Arg Thr Glu Trp Ser
385                 390                 395                 400
Ser Lys Thr Pro Thr Thr Thr Trp Gly Thr Ala Pro
                405                 410
```

<210> 94
<211> 1245
<212> DNA
<213> Aquilegia formosa x Aquilegia pubescens

<400> 94

```
atggtcgcat ccgccgctac cgcagcattc tttcccgtta ctaaagcttc ttctacaaag      60
gcttcacttg tgcctggtgg aggatcagat aatttggaca ctcgaggaat caattcgtcg     120
aaacctactt cttctggagg tttgaaagtt aaggctaatg cacaagcaac tcctaaaatt     180
aatggaactt ctattcatta cccaccatca tctgaacgtt tgaagaattc cgatgaaact     240
tcaattgcac ctgccagaac atttatcaat caattgcctg attggagtgt tcttcttacc     300
gccatcaccg caatgttctt agcagctgag aaacagtgga cacttcttga ttggaaaccg     360
aggagatccg acatgcttgt tgatcctttt ggtttaggga agattgttca ggatgggctt     420
gtttttcaac agaatttctc aattagatcg tatgaaatag gtgttgatgg gacgacgtct     480
```

```
atagaatcat ttatgaacca tttgcaggaa actgctctta accatgctaa gactgtgggg    540
cttcttggcg atggcttcgg ttcaactgaa gctatgagca aaagaaactt gatctgggtg    600
gtagctagga tgcagattct tgtgaataga tatcctacgt ggggtgatac tgttcaggta    660
gatacttggg ttgctgcaaa tgggaagaat ggtatgcgtc gtgattggct tgttcgtgac    720
gggaattctg ggaaaccct tgcaagagct tcaagcaagt gggtgatgat gaatacaagt    780
acgcggaaac tatctaaaat gccagatgat gttagggttg aaatagagcc ttattttatg    840
gattgtgctc ctattgttga ggaagatggc agaaagctgc caaagcttga tgaaagcaca    900
tcagattatg ttcgaaatgg cctaacgcct cgatggaatg atctggatct caatcagcat    960
gtgaacaatg tcaagtacat aggctggatt cttgagagtt ctatctcaat gttggagaat   1020
catgagcttg caggcatcac tctagagtat cggaaggagt gtcggaagga caatgtgctg   1080
caatccttga ctgctgtcag caaagatgcc aaaggctggc ctgagtgtgt tcacttgctt   1140
cgtcttgaca gtggggctga ggttgtcagg ggaagcacta tgtggaggcc gaagcgcatc   1200
aacaactttg gatctgtggg ccgaattcct accgatggca tgtag                   1245

<210>  95
<211>  414
<212>  PRT
<213>  Aquilegia formosa x Aquilegia pubescens

<400>  95
Met Val Ala Ser Ala Ala Thr Ala Ala Phe Phe Pro Val Thr Lys Ala
1               5                   10                  15
Ser Ser Thr Lys Ala Ser Leu Val Pro Gly Gly Gly Ser Asp Asn Leu
            20                  25                  30
Asp Thr Arg Gly Ile Asn Ser Ser Lys Pro Thr Ser Ser Gly Gly Leu
        35                  40                  45
Lys Val Lys Ala Asn Ala Gln Ala Thr Pro Lys Ile Asn Gly Thr Ser
    50                  55                  60
Ile His Tyr Pro Pro Ser Ser Glu Arg Leu Lys Asn Ser Asp Glu Thr
65                  70                  75                  80
Ser Ile Ala Pro Ala Arg Thr Phe Ile Asn Gln Leu Pro Asp Trp Ser
                85                  90                  95
Val Leu Leu Thr Ala Ile Thr Ala Met Phe Leu Ala Ala Glu Lys Gln
            100                 105                 110
Trp Thr Leu Leu Asp Trp Lys Pro Arg Arg Ser Asp Met Leu Val Asp
        115                 120                 125
Pro Phe Gly Leu Gly Lys Ile Val Gln Asp Gly Leu Val Phe Gln Gln
    130                 135                 140
Asn Phe Ser Ile Arg Ser Tyr Glu Ile Gly Val Asp Gly Thr Thr Ser
145                 150                 155                 160
Ile Glu Ser Phe Met Asn His Leu Gln Glu Thr Ala Leu Asn His Ala
                165                 170                 175
Lys Thr Val Gly Leu Leu Gly Asp Gly Phe Gly Ser Thr Glu Ala Met
            180                 185                 190
Ser Lys Arg Asn Leu Ile Trp Val Val Ala Arg Met Gln Ile Leu Val
        195                 200                 205
Asn Arg Tyr Pro Thr Trp Gly Asp Thr Val Gln Val Asp Thr Trp Val
    210                 215                 220
Ala Ala Asn Gly Lys Asn Gly Met Arg Arg Asp Trp Leu Val Arg Asp
225                 230                 235                 240
Gly Asn Ser Gly Glu Thr Leu Ala Arg Ala Ser Ser Lys Trp Val Met
                245                 250                 255
Met Asn Thr Ser Thr Arg Lys Leu Ser Lys Met Pro Asp Asp Val Arg
            260                 265                 270
Val Glu Ile Glu Pro Tyr Phe Met Asp Cys Ala Pro Ile Val Glu Glu
        275                 280                 285
Asp Gly Arg Lys Leu Pro Lys Leu Asp Glu Ser Thr Ser Asp Tyr Val
    290                 295                 300
Arg Asn Gly Leu Thr Pro Arg Trp Asn Asp Leu Asp Leu Asn Gln His
305                 310                 315                 320
Val Asn Asn Val Lys Tyr Ile Gly Trp Ile Leu Glu Ser Ser Ile Ser
                325                 330                 335
Met Leu Glu Asn His Glu Leu Ala Gly Ile Thr Leu Glu Tyr Arg Lys
```

```
            340                 345                 350
Glu Cys Arg Lys Asp Asn Val Leu Gln Ser Leu Thr Ala Val Ser Lys
        355                 360                 365
Asp Ala Lys Gly Trp Pro Glu Cys Val His Leu Leu Arg Leu Asp Ser
    370                 375                 380
Gly Ala Glu Val Val Arg Gly Ser Thr Met Trp Arg Pro Lys Arg Ile
385                 390                 395                 400
Asn Asn Phe Gly Ser Val Gly Arg Ile Pro Thr Asp Gly Met
                405                 410
```

<210> 96
<211> 1242
<212> DNA
<213> Arachis hypogaea

<400> 96

```
atggcaactg ctgctactgc ttccattttc cctgttcctt caccctcacc agatgcaggt     60
gcagatggca acaaacttgt tggtggctct gttaaacttc aagggctcaa atctaaacat    120
gcatcttctg gtggcttgca agttaaagct catgcccaag ctccacccaa gattaatgga    180
agcacagtag aaagcttgaa gcatgatgat gatttgcctt cccctccccc caggactttt    240
attaaccagt tacctgattg gagcatgctt cttgctgcta taactacaat tttcctggca    300
gcagaaaagc agtggatgat gcttgattgg aaaccaaggc gatctgacat gcttattgat    360
ccctttggaa taggaagaat tgttcaagat ggtctagtgt tccgtcaaaa cttttctatt    420
agatcatatg aaattggtgc cgatcgaaca gcatctatag agacagtaat gaaccatctg    480
caggaaactg cacttaatca tgtcaagact gctggacttc ttggtgatgg ctttggttcc    540
acaccagaaa tgtgcaaaaa gaacttgata tgggtagtca cacggatgca ggttgtggtt    600
gatcgttatc ctacatgggg tgatgttgtt caagtagata cttgggtatc tgcatctggg    660
aagaatggca tgcgtcgtga ttggcttctg cgtgactgca aaactggtga agtattgacg    720
agagcctcca gtgtttgggt catgatgaat aaactaacaa ggaggctatc taaaattcca    780
gaagaagtca gagcggagat agcatcttat tttgtgaatt ccgctccaat tctggaagag    840
gataacagaa aactatctaa acttgatgac aataccgctg attacattcg cacgggtctt    900
agtcctagat ggaatgatct agatgtcaat cagcatgtta acaatgtgaa gtacattggc    960
tggattctgg agagtgctcc gcagccaatc ttggagagtc atgagctttc tgcaatgact   1020
ttggagtata ggagggagtg tggtagggac agtgtgctgc agtccctcac tgctgtgtct   1080
gctgccgacg tcggcaatct tgctcacagg gggcaactcg agtgcaagca tttgcttcga   1140
cttgaagatg gtgctgaaat tgtgaggggt aggactgagt ggaggcccaa acctgtgagc   1200
aactttgaca ttgtgaatca ggttccagcc gaaagcatct aa                        1242
```

<210> 97
<211> 413
<212> PRT
<213> Arachis hypogaea

<400> 97

```
Met Ala Thr Ala Ala Thr Ala Ser Ile Phe Pro Val Pro Ser Pro Ser
1               5                   10                  15
Pro Asp Ala Gly Ala Asp Gly Asn Lys Leu Val Gly Gly Ser Val Lys
            20                  25                  30
Leu Gln Gly Leu Lys Ser Lys His Ala Ser Ser Gly Gly Leu Gln Val
        35                  40                  45
Lys Ala His Ala Gln Ala Pro Pro Lys Ile Asn Gly Ser Thr Val Glu
    50                  55                  60
Ser Leu Lys His Asp Asp Asp Leu Pro Ser Pro Pro Pro Arg Thr Phe
65                  70                  75                  80
Ile Asn Gln Leu Pro Asp Trp Ser Met Leu Leu Ala Ala Ile Thr Thr
                85                  90                  95
Ile Phe Leu Ala Ala Glu Lys Gln Trp Met Met Leu Asp Trp Lys Pro
            100                 105                 110
Arg Arg Ser Asp Met Leu Ile Asp Pro Phe Gly Ile Gly Arg Ile Val
        115                 120                 125
Gln Asp Gly Leu Val Phe Arg Gln Asn Phe Ser Ile Arg Ser Tyr Glu
    130                 135                 140
Ile Gly Ala Asp Arg Thr Ala Ser Ile Glu Thr Val Met Asn His Leu
```

```
145                 150                 155                 160
Gln Glu Thr Ala Leu Asn His Val Lys Thr Ala Gly Leu Leu Gly Asp
                165                 170                 175
Gly Phe Gly Ser Thr Pro Glu Met Cys Lys Lys Asn Leu Ile Trp Val
            180                 185                 190
Val Thr Arg Met Gln Val Val Val Asp Arg Tyr Pro Thr Trp Gly Asp
        195                 200                 205
Val Val Gln Val Asp Thr Trp Val Ser Ala Ser Gly Lys Asn Gly Met
    210                 215                 220
Arg Arg Asp Trp Leu Leu Arg Asp Cys Lys Thr Gly Glu Val Leu Thr
225                 230                 235                 240
Arg Ala Ser Ser Val Trp Val Met Met Asn Lys Leu Thr Arg Arg Leu
                245                 250                 255
Ser Lys Ile Pro Glu Glu Val Arg Ala Glu Ile Ala Ser Tyr Phe Val
            260                 265                 270
Asn Ser Ala Pro Ile Leu Glu Glu Asp Asn Arg Lys Leu Ser Lys Leu
        275                 280                 285
Asp Asp Asn Thr Ala Asp Tyr Ile Arg Thr Gly Leu Ser Pro Arg Trp
    290                 295                 300
Asn Asp Leu Asp Val Asn Gln His Val Asn Asn Val Lys Tyr Ile Gly
305                 310                 315                 320
Trp Ile Leu Glu Ser Ala Pro Gln Pro Ile Leu Glu Ser His Glu Leu
                325                 330                 335
Ser Ala Met Thr Leu Glu Tyr Arg Arg Glu Cys Gly Arg Asp Ser Val
            340                 345                 350
Leu Gln Ser Leu Thr Ala Val Ser Ala Ala Asp Val Gly Asn Leu Ala
        355                 360                 365
His Arg Gly Gln Leu Glu Cys Lys His Leu Leu Arg Leu Glu Asp Gly
    370                 375                 380
Ala Glu Ile Val Arg Gly Arg Thr Glu Trp Arg Pro Lys Pro Val Ser
385                 390                 395                 400
Asn Phe Asp Ile Val Asn Gln Val Pro Ala Glu Ser Ile
                405                 410
```

<210> 98
<211> 1236
<212> DNA
<213> Brassica juncea

<400> 98

```
atggtggcca cctctgctac gtccttattc tttcctctcc catcttcctc cctcgacccc      60
aacgyaaaaa ccaacaacag agtcacctcc accaacttcg ccggactcgg tccaacgcca     120
aactctggcg gcaggatgaa ggttaaacca aacgcccagg ctccrcccaa gatcaacggs     180
aagaaagttg gtctccctgg ctcggtagag atcgagacct cacaacaaca acaacccgca     240
ccgaggacgt tcatcaacca gctgcctgac tggagcatgc ttctcgccgc cattacgacc     300
gtcttcctag cggctgagaa acagtggatg atgcttgact ggaaaccgag gcgttccgac     360
atgattatgg aaccgtttgg tctagggaga atcgttcagg atgggcttgt gttccgtcag     420
aatttttcta ttaggtctta tgagataggt gctgatcgct ctgcatctat agaaacggtt     480
atgaatcatt tacaggaaac ggccctaaac yatgttaaga ctgctggact gctgggggat     540
gggtttggtt ctaccccctga gatggttaag aagascttga tatgggtcgt tactcgtatg     600
caggttgttg ttgataccta tcctacttgg ggagatgttg ttgaagtaga tacatgggtc     660
agcaagtctg gaaagaatgg tatgcgtcgt gattggctag tccgggatgg caatactgga     720
caaattttaa caagagcatc aagtgtatgg gtgatgatga ataaactgac gagaagatta     780
tcaaagattc ctgaagaggt tcgaggggag atagagcctt actttgtgga ttttgaccct     840
gtccttgccg aggacagcag gaagttaaca aaactggatg acaaaactgc tgactatgtc     900
cgttctggtc tcactccgcg ttggagtgac ttagatgtta accagcatgt taacaatgta     960
aagtacatag ggtggatact ggagagtgct ccagtgggga tgatggagag tcagaagctg    1020
aaaagcatga ctctggagta tcgcagggag tgcgggaggg acagtgtgct tcagtccctc    1080
accgcggttt cgggctgcga tatcggtaac ctcgggacag ctggtgaagt tgaatgtcag    1140
catctgctcc gactccagga tggagctgaa gtggtgagag gaagaacaga gtggagttcc    1200
aaaacaccaa caacaacttg ggacattaca ccgtga                              1236
```

<210> 99

<211> 411
<212> PRT
<213> Brassica juncea

<220>
<221> UNSURE
<222> (22)..(22)
<223> Unknown amino acid

<220>
<221> UNSURE
<222> (171)..(171)
<223> Unknown amino acid

<220>
<221> UNSURE
<222> (192)..(192)
<223> Unknown amino acid

<400> 99

```
Met Val Ala Thr Ser Ala Thr Ser Leu Phe Phe Pro Leu Pro Ser Ser
1               5                   10                  15
Ser Leu Asp Pro Asn Xaa Lys Thr Asn Asn Arg Val Thr Ser Thr Asn
            20                  25                  30
Phe Ala Gly Leu Gly Pro Thr Pro Asn Ser Gly Gly Arg Met Lys Val
        35                  40                  45
Lys Pro Asn Ala Gln Ala Pro Pro Lys Ile Asn Gly Lys Lys Val Gly
    50                  55                  60
Leu Pro Gly Ser Val Glu Ile Glu Thr Ser Gln Gln Gln Gln Pro Ala
65                  70                  75                  80
Pro Arg Thr Phe Ile Asn Gln Leu Pro Asp Trp Ser Met Leu Leu Ala
                85                  90                  95
Ala Ile Thr Thr Val Phe Leu Ala Ala Glu Lys Gln Trp Met Met Leu
            100                 105                 110
Asp Trp Lys Pro Arg Arg Ser Asp Met Ile Met Glu Pro Phe Gly Leu
        115                 120                 125
Gly Arg Ile Val Gln Asp Gly Leu Val Phe Arg Gln Asn Phe Ser Ile
    130                 135                 140
Arg Ser Tyr Glu Ile Gly Ala Asp Arg Ser Ala Ser Ile Glu Thr Val
145                 150                 155                 160
Met Asn His Leu Gln Glu Thr Ala Leu Asn Xaa Val Lys Thr Ala Gly
                165                 170                 175
Leu Leu Gly Asp Gly Phe Gly Ser Thr Pro Glu Met Val Lys Lys Xaa
            180                 185                 190
Leu Ile Trp Val Val Thr Arg Met Gln Val Val Val Asp Thr Tyr Pro
        195                 200                 205
Thr Trp Gly Asp Val Val Glu Val Asp Thr Trp Val Ser Lys Ser Gly
    210                 215                 220
Lys Asn Gly Met Arg Arg Asp Trp Leu Val Arg Asp Gly Asn Thr Gly
225                 230                 235                 240
Gln Ile Leu Thr Arg Ala Ser Ser Val Trp Val Met Met Asn Lys Leu
                245                 250                 255
Thr Arg Arg Leu Ser Lys Ile Pro Glu Glu Val Arg Gly Glu Ile Glu
            260                 265                 270
Pro Tyr Phe Val Asp Phe Asp Pro Val Leu Ala Glu Asp Ser Arg Lys
        275                 280                 285
Leu Thr Lys Leu Asp Asp Lys Thr Ala Asp Tyr Val Arg Ser Gly Leu
    290                 295                 300
Thr Pro Arg Trp Ser Asp Leu Asp Val Asn Gln His Val Asn Asn Val
305                 310                 315                 320
Lys Tyr Ile Gly Trp Ile Leu Glu Ser Ala Pro Val Gly Met Met Glu
                325                 330                 335
Ser Gln Lys Leu Lys Ser Met Thr Leu Glu Tyr Arg Arg Glu Cys Gly
```

```
                340                 345                 350
Arg Asp Ser Val Leu Gln Ser Leu Thr Ala Val Ser Gly Cys Asp Ile
        355                 360                 365
Gly Asn Leu Gly Thr Ala Gly Glu Val Glu Cys Gln His Leu Leu Arg
    370                 375                 380
Leu Gln Asp Gly Ala Glu Val Val Arg Gly Arg Thr Glu Trp Ser Ser
385                 390                 395                 400
Lys Thr Pro Thr Thr Thr Trp Asp Ile Thr Pro
                405                 410
```

<210> 100
<211> 1287
<212> DNA
<213> Brachypodium sylvaticum

<400> 100

```
atggcagggt cccttgccgc ctcggcgttc ttccccagcc caggatcttc accagctgca      60
ttggctaaaa gctccaagaa cacgtccggt gaattacctg agactttgag tgtccgtgga     120
attgtcgcaa agcctaacac gcctcctgcg tccatgcaag tgaaaactaa ggcccaagcg     180
ctccccaagg ttaatggcac caaggttaat ctcaagactt caagctctga caaggaagac     240
acagtgccgt acagttcttc aaagacattc tataaccaac tgccagattg gagcatgctg     300
cttgcagctg tcacgaccat cttcctggcc gcagagaagc agtggacaat gcttgattgg     360
aaaccgaaga ggcctgacat gcttgtcgac acatttggct ttggcagaat catccaggat     420
gggatggttt ttaggcagaa ctttttgatt agatcctacg agattggtgc tgatcgtaca     480
gcttctatag agacattaat gaatcattta caggaaacag ctcttaacca tgtcaagact     540
gctggtctcc ttggagatgg ctttggtgct actcaggaga tgagtaaacg gaacttgatc     600
tgggttgtca gcaaaattca gcttcttgta gagcgatatc catcgtggga agatatggtt     660
caagtcgata catgggtagc ttcttctgga aaaaatggca tgcgtcgaga ttggcatatc     720
cgtgactaca attcggggca aacgatcttg agagctacaa gtgtttgggt tacgatgaat     780
aagaacacta gaaaactttc aaaaatgcct gatgaagtta gggctgaaat aggcccgcac     840
ttcaacaatg accgttccgc tttaacagag gagcatagtg acaagttagc taagccaggg     900
aggaaaggtg gtgaccctgc taccaaacag ttcataagga aggggcttac cccaaaatgg     960
ggtgaccttg atgtcaacca acatgtgaac aatgtgaagt atattgggtg gattcttgag    1020
agtgctccaa tttcaatact ggaaaagcat gagcttgcaa gcatgacact ggaatacagg    1080
aaggagtgtg gccgtgacag cgtgctgcag tctcttacca atgtcatagg tgagtgcacc    1140
gacggcagcc cagagtctgc tatccagtgc agccatctgc tccagctgga gtctggaact    1200
gacatcgtga aggctcacac aaagtggcga ccgaagagag cgcagggcga aggaaacaca    1260
gggttgttcc cagcttcgag tgcataa                                        1287
```

<210> 101
<211> 428
<212> PRT
<213> Brachypodium sylvaticum

<400> 101

```
Met Ala Gly Ser Leu Ala Ala Ser Ala Phe Phe Pro Ser Pro Gly Ser
1               5                   10                  15
Ser Pro Ala Ala Leu Ala Lys Ser Ser Lys Asn Thr Ser Gly Glu Leu
            20                  25                  30
Pro Glu Thr Leu Ser Val Arg Gly Ile Val Ala Lys Pro Asn Thr Pro
        35                  40                  45
Pro Ala Ser Met Gln Val Lys Thr Lys Ala Gln Ala Leu Pro Lys Val
    50                  55                  60
Asn Gly Thr Lys Val Asn Leu Lys Thr Ser Ser Ser Asp Lys Glu Asp
65                  70                  75                  80
Thr Val Pro Tyr Ser Ser Ser Lys Thr Phe Tyr Asn Gln Leu Pro Asp
                85                  90                  95
Trp Ser Met Leu Leu Ala Ala Val Thr Thr Ile Phe Leu Ala Ala Glu
            100                 105                 110
Lys Gln Trp Thr Met Leu Asp Trp Lys Pro Lys Arg Pro Asp Met Leu
        115                 120                 125
Val Asp Thr Phe Gly Phe Gly Arg Ile Ile Gln Asp Gly Met Val Phe
    130                 135                 140
```

```
Arg Gln Asn Phe Leu Ile Arg Ser Tyr Glu Ile Gly Ala Asp Arg Thr
145                 150                 155                 160
Ala Ser Ile Glu Thr Leu Met Asn His Leu Gln Glu Thr Ala Leu Asn
                165                 170                 175
His Val Lys Thr Ala Gly Leu Leu Gly Asp Gly Phe Gly Ala Thr Gln
            180                 185                 190
Glu Met Ser Lys Arg Asn Leu Ile Trp Val Val Ser Lys Ile Gln Leu
        195                 200                 205
Leu Val Glu Arg Tyr Pro Ser Trp Glu Asp Met Val Gln Val Asp Thr
    210                 215                 220
Trp Val Ala Ser Ser Gly Lys Asn Gly Met Arg Arg Asp Trp His Ile
225                 230                 235                 240
Arg Asp Tyr Asn Ser Gly Gln Thr Ile Leu Arg Ala Thr Ser Val Trp
                245                 250                 255
Val Thr Met Asn Lys Asn Thr Arg Lys Leu Ser Lys Met Pro Asp Glu
            260                 265                 270
Val Arg Ala Glu Ile Gly Pro His Phe Asn Asn Asp Arg Ser Ala Leu
        275                 280                 285
Thr Glu Glu His Ser Asp Lys Leu Ala Lys Pro Gly Arg Lys Gly Gly
    290                 295                 300
Asp Pro Ala Thr Lys Gln Phe Ile Arg Lys Gly Leu Thr Pro Lys Trp
305                 310                 315                 320
Gly Asp Leu Asp Val Asn Gln His Val Asn Asn Val Lys Tyr Ile Gly
                325                 330                 335
Trp Ile Leu Glu Ser Ala Pro Ile Ser Ile Leu Glu Lys His Glu Leu
            340                 345                 350
Ala Ser Met Thr Leu Glu Tyr Arg Lys Glu Cys Gly Arg Asp Ser Val
        355                 360                 365
Leu Gln Ser Leu Thr Asn Val Ile Gly Glu Cys Thr Asp Gly Ser Pro
    370                 375                 380
Glu Ser Ala Ile Gln Cys Ser His Leu Leu Gln Leu Glu Ser Gly Thr
385                 390                 395                 400
Asp Ile Val Lys Ala His Thr Lys Trp Arg Pro Lys Arg Ala Gln Gly
                405                 410                 415
Glu Gly Asn Thr Gly Leu Phe Pro Ala Ser Ser Ala
            420                 425
```

```
<210>  102
<211>  1257
<212>  DNA
<213>  Citrus sinensis

<400>  102
atggttgcta ctgccgcagc ttctgcgttc ttcccagttt cctcaccatc tggggattct      60
gttgcaaaga ccaaaaatct cggatctgct aatctgggag gtattaagtc aaaatcctct     120
tctgggagtt tgcaggttaa ggctaatgcg caagcacctt ccaagataaa tggtacttca     180
gttggtttga caacaccagc agaaagtttg aagaatggtg atatctccac gtcatcacct     240
cctcctagga cttttattaa ccagttacct gactggagta tgcttcttgc tgctataaca     300
acaatcttct tggcagcaga gaagcagtgg atgatgcttg attggaaacc aaggcgatct     360
gacatgcttg tggacccatt tgggattggg aaaatagttc aggatggttt cattttccgg     420
caaaatttct caattagatc atatgagata ggtgctgatg gtactgcatc tatagagaca     480
ttaatgaatc atttacagga aacagcgctt aatcatgtta tgactgctgg tcttctagat     540
gctggctttg gtgcaacccc agcgatggct aaaaagaacc tgatatgggt ggttactcgg     600
atgcaggttg ttgtagaccg ctatcccact tggaatgatg ttgtaaatgt agaaacttgg     660
gttagtgcat ctggaaaaaa tggtatgcgg cgtgattggc tcattcgcaa tgctaagaca     720
ggtgaaacat taacaagagc aaccagtctg tgggtaatga tgaataaact gactaggagg     780
ttgtccaaaa tgcccgatga agttcgtcag gaaattgaac cgtattttct gaattctgac     840
cctgttgtcg atgaggatag caggaaatta ccaaaacttg gcgacagtac tgcagattat     900
gttcgtagag gtttaactcc taggtggagt gatttagatg tcaaccagca tgtcaataat     960
gtgaagtaca ttggctggat cctagagagt gctcctcagc agatcttgga gagtcatcag    1020
ctggcatctg tgaccctgga gtataggagg gagtgcggaa gggacagtgt gttgcagtcc    1080
ctgactgctg tctcagacaa ggacattggc aatttggtga acttgggcag tgtggagtgc    1140
cagcacttgc tccgactaga ggaaggtgct gaagttttga gagcaaggac tgaatggagg    1200
```

```
ccaaaggatg cccacaactt tgggaatgtt ggtccaatcc ctgcagaaag cacttaa      1257

<210>  103
<211>  418
<212>  PRT
<213>  Citrus sinensis

<400>  103
Met Val Ala Thr Ala Ala Ala Ser Ala Phe Phe Pro Val Ser Ser Pro
1               5                   10                  15
Ser Gly Asp Ser Val Ala Lys Thr Lys Asn Leu Gly Ser Ala Asn Leu
            20                  25                  30
Gly Gly Ile Lys Ser Lys Ser Ser Ser Gly Ser Leu Gln Val Lys Ala
        35                  40                  45
Asn Ala Gln Ala Pro Ser Lys Ile Asn Gly Thr Ser Val Gly Leu Thr
    50                  55                  60
Thr Pro Ala Glu Ser Leu Lys Asn Gly Asp Ile Ser Thr Ser Ser Pro
65                  70                  75                  80
Pro Pro Arg Thr Phe Ile Asn Gln Leu Pro Asp Trp Ser Met Leu Leu
                85                  90                  95
Ala Ala Ile Thr Thr Ile Phe Leu Ala Ala Glu Lys Gln Trp Met Met
            100                 105                 110
Leu Asp Trp Lys Pro Arg Arg Ser Asp Met Leu Val Asp Pro Phe Gly
        115                 120                 125
Ile Gly Lys Ile Val Gln Asp Gly Phe Ile Phe Arg Gln Asn Phe Ser
    130                 135                 140
Ile Arg Ser Tyr Glu Ile Gly Ala Asp Gly Thr Ala Ser Ile Glu Thr
145                 150                 155                 160
Leu Met Asn His Leu Gln Glu Thr Ala Leu Asn His Val Met Thr Ala
                165                 170                 175
Gly Leu Leu Asp Ala Gly Phe Gly Ala Thr Pro Ala Met Ala Lys Lys
            180                 185                 190
Asn Leu Ile Trp Val Val Thr Arg Met Gln Val Val Val Asp Arg Tyr
        195                 200                 205
Pro Thr Trp Asn Asp Val Val Asn Val Glu Thr Trp Val Ser Ala Ser
    210                 215                 220
Gly Lys Asn Gly Met Arg Arg Asp Trp Leu Ile Arg Asn Ala Lys Thr
225                 230                 235                 240
Gly Glu Thr Leu Thr Arg Ala Thr Ser Leu Trp Val Met Met Asn Lys
                245                 250                 255
Leu Thr Arg Arg Leu Ser Lys Met Pro Asp Glu Val Arg Gln Glu Ile
            260                 265                 270
Glu Pro Tyr Phe Leu Asn Ser Asp Pro Val Val Asp Glu Asp Ser Arg
        275                 280                 285
Lys Leu Pro Lys Leu Gly Asp Ser Thr Ala Asp Tyr Val Arg Arg Gly
    290                 295                 300
Leu Thr Pro Arg Trp Ser Asp Leu Asp Val Asn Gln His Val Asn Asn
305                 310                 315                 320
Val Lys Tyr Ile Gly Trp Ile Leu Glu Ser Ala Pro Gln Gln Ile Leu
                325                 330                 335
Glu Ser His Gln Leu Ala Ser Val Thr Leu Glu Tyr Arg Arg Glu Cys
            340                 345                 350
Gly Arg Asp Ser Val Leu Gln Ser Leu Thr Ala Val Ser Asp Lys Asp
        355                 360                 365
Ile Gly Asn Leu Val Asn Leu Gly Ser Val Glu Cys Gln His Leu Leu
    370                 375                 380
Arg Leu Glu Glu Gly Ala Glu Val Leu Arg Ala Arg Thr Glu Trp Arg
385                 390                 395                 400
Pro Lys Asp Ala His Asn Phe Gly Asn Val Gly Pro Ile Pro Ala Glu
                405                 410                 415
Ser Thr
```

<210> 104
<211> 1254
<212> DNA
<213> Elaeis guineensis

<400> 104
```
atggttgctt cgattgtcgc ttgggccttt ttccccacac catctttctc ccccacggca      60
tcagcaaaag cttcgaagac cattggtgaa ggctccgaga atttgaatgt tcggggtatc     120
atagccaaac ccacttcttc ttcggcggct aagcagggta aggtgatggc ccaagccgtc     180
cccaagatca atggcgcgaa ggttggcctg aaagctgaat cccaaaaggc cgaggaagat     240
gctgcccctt cctcagcccc gaggacattc tataatcaac tacctgactg gagcgtgctc     300
cttgccgccg taacaacgat ctttttggct gccgagaagc agtggaccct tcttgattgg     360
aagccacggc gtcccgacat gcttactggt gcatttagcc ttgggaagat tgtgcaggat     420
ggactagttt tcaggcagaa cttttccatc aggtcatatg agattggggc tgatcggacg     480
gcttctatag aaacgttaat gaaccattta caggaaacag cacttaatca tgtgaggaat     540
gctgggcttc tgggcgatgg ttttggtgcc acaccagaga tgagtaaaag aaatttgatt     600
tgggttgtca ctaaaatgca ggtcctgatt gagcactatc cttcctgggg ggatgttgtt     660
gaagtagata catgggttgg tgcatctggt aaaaatggga tgcgtcgtga ttggcatgtt     720
cgtgactacc gaacaggcca aactatattg agagccacca gtatctgggt gatgatggat     780
aaacacacta ggaagttgtc taaaatgccc gaagaagtca gagcagagat aggGccttac     840
tttatggaac atgctgctat tgtggacgag gacagcagaa agcttccaaa gcttgatgat     900
gatactgcag attatattaa atggggcctg actcctcgat ggagtgattt agatgtgaat     960
cagcatgtga acaatgtcaa atatataggc tggattcttg agagcgctcc aatatcaatc    1020
ctggagaatc acgagctggc gagtatgact ctggaatata ggagggagtg tgggagggac    1080
agcgttctgc aatccctcac cgcagtcgct aatgactgca ctggtggcct tccagaagct    1140
agcatcgagt gccagcatct gctgcagctg gaatgcgggg ccgagattgt taggggacgg    1200
acacagtgga ggcccaggcg tgcctccggt cccacttcag ctggaagtgc ttga          1254
```

<210> 105
<211> 417
<212> PRT
<213> Elaeis guineensis

<400> 105
```
Met Val Ala Ser Ile Val Ala Trp Ala Phe Phe Pro Thr Pro Ser Phe
1               5                   10                  15
Ser Pro Thr Ala Ser Ala Lys Ala Ser Lys Thr Ile Gly Glu Gly Ser
            20                  25                  30
Glu Asn Leu Asn Val Arg Gly Ile Ile Ala Lys Pro Thr Ser Ser Ser
        35                  40                  45
Ala Ala Lys Gln Gly Lys Val Met Ala Gln Ala Val Pro Lys Ile Asn
    50                  55                  60
Gly Ala Lys Val Gly Leu Lys Ala Glu Ser Gln Lys Ala Glu Glu Asp
65                  70                  75                  80
Ala Ala Pro Ser Ser Ala Pro Arg Thr Phe Tyr Asn Gln Leu Pro Asp
                85                  90                  95
Trp Ser Val Leu Leu Ala Ala Val Thr Thr Ile Phe Leu Ala Ala Glu
            100                 105                 110
Lys Gln Trp Thr Leu Leu Asp Trp Lys Pro Arg Arg Pro Asp Met Leu
        115                 120                 125
Thr Gly Ala Phe Ser Leu Gly Lys Ile Val Gln Asp Gly Leu Val Phe
    130                 135                 140
Arg Gln Asn Phe Ser Ile Arg Ser Tyr Glu Ile Gly Ala Asp Arg Thr
145                 150                 155                 160
Ala Ser Ile Glu Thr Leu Met Asn His Leu Gln Glu Thr Ala Leu Asn
                165                 170                 175
His Val Arg Asn Ala Gly Leu Leu Gly Asp Gly Phe Gly Ala Thr Pro
            180                 185                 190
Glu Met Ser Lys Arg Asn Leu Ile Trp Val Val Thr Lys Met Gln Val
        195                 200                 205
Leu Ile Glu His Tyr Pro Ser Trp Gly Asp Val Val Glu Val Asp Thr
    210                 215                 220
Trp Val Gly Ala Ser Gly Lys Asn Gly Met Arg Arg Asp Trp His Val
```

```
225                 230                 235                 240
Arg Asp Tyr Arg Thr Gly Gln Thr Ile Leu Arg Ala Thr Ser Ile Trp
                245                 250                 255
Val Met Met Asp Lys His Thr Arg Lys Leu Ser Lys Met Pro Glu Glu
            260                 265                 270
Val Arg Ala Glu Ile Gly Pro Tyr Phe Met Glu His Ala Ala Ile Val
        275                 280                 285
Asp Glu Asp Ser Arg Lys Leu Pro Lys Leu Asp Asp Asp Thr Ala Asp
    290                 295                 300
Tyr Ile Lys Trp Gly Leu Thr Pro Arg Trp Ser Asp Leu Asp Val Asn
305                 310                 315                 320
Gln His Val Asn Asn Val Lys Tyr Ile Gly Trp Ile Leu Glu Ser Ala
                325                 330                 335
Pro Ile Ser Ile Leu Glu Asn His Glu Leu Ala Ser Met Thr Leu Glu
            340                 345                 350
Tyr Arg Arg Glu Cys Gly Arg Asp Ser Val Leu Gln Ser Leu Thr Ala
        355                 360                 365
Val Ala Asn Asp Cys Thr Gly Gly Leu Pro Glu Ala Ser Ile Glu Cys
    370                 375                 380
Gln His Leu Leu Gln Leu Glu Cys Gly Ala Glu Ile Val Arg Gly Arg
385                 390                 395                 400
Thr Gln Trp Arg Pro Arg Arg Ala Ser Gly Pro Thr Ser Ala Gly Ser
                405                 410                 415
Ala
```

<210> 106
<211> 1221
<212> DNA
<213> Garcinia mangostana

<400> 106

```
atggttgcta ctgccgccac gtcatcattc tttccgttga cttccccttc tggggatgcc     60
aaatcgggca atcccggaaa agggtcggtg agttttgggt caatgaagtc gaaatccgcg    120
gcttcctcga ggggtttaca agtgaaggcc aatgcacagg cacccactaa gatcaatgga    180
tccacggatg atgctcaatt gcctgccccg aggactttta ttaaccagtt gcctgattgg    240
agcatgcttc ttgctgctat tactaccgtg tttttggcag ccgagaagca gtggatgatg    300
ttggattgga agcctaggag gcccgacatg cttattgaca cgtttggttt ggggaggatt    360
gtgcaggatg gtcttgtttt tcgacagaat ttctcgatta ggtcctatga aattggtgct    420
gatcgtactg cgtctataga gacggttatg aatcatctgc aagaaactgc cctcaatcat    480
gttaagactg caggacttct gggtgatgga ttcggttcaa caccagagat gtctaaaagg    540
aatctcatat gggttgttac taagatgcag gtcgaagtcg atcggtatcc tacatggggt    600
gacgttgttc aggtagatac ttgggtgagt gcatcaggaa agaatggaat gcgtcgagat    660
tggcttcttc gtgatggtaa tactggggag acattaacca gagcttcaag tgtgtgggtg    720
atgatgaata aactgacaag gagattgtct aaaattcccg aagaagttcg gaggaaata     780
ggatcttact ttgtgaattc tgatcctgtt gtggaggagg atggtagaaa ggtgacaaaa    840
cttgatgaca acactgcaga ttttgttcgc aaagggttaa ctcctaaatg gaatgacttg    900
gacatcaatc agcatgtgaa taatgtgaag tatattggct ggatccttga gagcgctcca    960
cagccaatcc tggaaacccg tgagctctca gcggtgactt tggagtatag gagggagtgt   1020
ggaagggaca gtgtgctgcg gtctctgacc gccgtttctg gcggtggcgt tggtgattta   1080
ggacacgctg gtaacgtcga gtgccagcac gtgcttcgct tggaggatgg agctgagatt   1140
gttcgtggaa ggaccgagtg gaggcccaaa tacattaaca acttcagtat catgggccag   1200
attccgacag atgcttctta g                                             1221
```

<210> 107
<211> 406
<212> PRT
<213> Garcinia mangostana

<400> 107

```
Met Val Ala Thr Ala Ala Thr Ser Ser Phe Phe Pro Leu Thr Ser Pro
1               5                   10                  15
Ser Gly Asp Ala Lys Ser Gly Asn Pro Gly Lys Gly Ser Val Ser Phe
```

```
                20                  25                  30
Gly Ser Met Lys Ser Lys Ser Ala Ala Ser Ser Arg Gly Leu Gln Val
        35                  40                  45
Lys Ala Asn Ala Gln Ala Pro Thr Lys Ile Asn Gly Ser Thr Asp Asp
    50                  55                  60
Ala Gln Leu Pro Ala Pro Arg Thr Phe Ile Asn Gln Leu Pro Asp Trp
65                  70                  75                  80
Ser Met Leu Leu Ala Ala Ile Thr Thr Val Phe Leu Ala Ala Glu Lys
                85                  90                  95
Gln Trp Met Met Leu Asp Trp Lys Pro Arg Arg Pro Asp Met Leu Ile
            100                 105                 110
Asp Thr Phe Gly Leu Gly Arg Ile Val Gln Asp Gly Leu Val Phe Arg
        115                 120                 125
Gln Asn Phe Ser Ile Arg Ser Tyr Glu Ile Gly Ala Asp Arg Thr Ala
    130                 135                 140
Ser Ile Glu Thr Val Met Asn His Leu Gln Glu Thr Ala Leu Asn His
145                 150                 155                 160
Val Lys Thr Ala Gly Leu Leu Gly Asp Gly Phe Gly Ser Thr Pro Glu
                165                 170                 175
Met Ser Lys Arg Asn Leu Ile Trp Val Val Thr Lys Met Gln Val Glu
            180                 185                 190
Val Asp Arg Tyr Pro Thr Trp Gly Asp Val Val Gln Val Asp Thr Trp
        195                 200                 205
Val Ser Ala Ser Gly Lys Asn Gly Met Arg Arg Asp Trp Leu Leu Arg
    210                 215                 220
Asp Gly Asn Thr Gly Glu Thr Leu Thr Arg Ala Ser Ser Val Trp Val
225                 230                 235                 240
Met Met Asn Lys Leu Thr Arg Arg Leu Ser Lys Ile Pro Glu Glu Val
                245                 250                 255
Arg Glu Glu Ile Gly Ser Tyr Phe Val Asn Ser Asp Pro Val Val Glu
            260                 265                 270
Glu Asp Gly Arg Lys Val Thr Lys Leu Asp Asp Asn Thr Ala Asp Phe
        275                 280                 285
Val Arg Lys Gly Leu Thr Pro Lys Trp Asn Asp Leu Asp Ile Asn Gln
    290                 295                 300
His Val Asn Asn Val Lys Tyr Ile Gly Trp Ile Leu Glu Ser Ala Pro
305                 310                 315                 320
Gln Pro Ile Leu Glu Thr Arg Glu Leu Ser Ala Val Thr Leu Glu Tyr
                325                 330                 335
Arg Arg Glu Cys Gly Arg Asp Ser Val Leu Arg Ser Leu Thr Ala Val
            340                 345                 350
Ser Gly Gly Gly Val Gly Asp Leu Gly His Ala Gly Asn Val Glu Cys
        355                 360                 365
Gln His Val Leu Arg Leu Glu Asp Gly Ala Glu Ile Val Arg Gly Arg
    370                 375                 380
Thr Glu Trp Arg Pro Lys Tyr Ile Asn Asn Phe Ser Ile Met Gly Gln
385                 390                 395                 400
Ile Pro Thr Asp Ala Ser
                405
```

<210> 108
<211> 1251
<212> DNA
<213> Glycine max

<400> 108

```
atggtggcaa cagctgctac ttcatcattt ttccctgtta cttcaccctc gccggactct      60
ggtggagcag gcagcaaact tggtggtggg cctgcaaacc ttggaggact aaaatccaaa     120
tctgcgtctt ctggtggctt gaaggcaaag gcgcaagccc cttcgaaaat taatggaacc     180
acagttgtta catctaaaga aagcttcaag catgatgatg atctaccttc gcctcccccc     240
agaactttta tcaaccagtt gcctgattgg agcatgcttc ttgctgctat cacaacaatt     300
ttcttggccg ctgaaaagca gtggatgatg cttgattgga agccaaggcg acctgacatg     360
cttattgacc cctttgggat aggaaaaatt gttcaggatg gtcttgtgtt ccgtgaaaac     420
```

```
ttttctatta gatcatatga gattggcgct gatcgaaccg catctataga aacagtaatg     480
aaccatttgc aagaaactgc acttaatcat gttaaaagtg ctgggcttct tggtgatggc     540
tttggttcca cgccagaaat gtgcaaaaag aacttgatat gggtggttac tcggatgcag     600
gttgtggtgg aacgctatcc tacatggggt gacatagttc aagtggacac ttgggtttct     660
ggatcaggga agaatggtat gcgccgtgat tggcttttac gtgactgcaa aactggtgaa     720
atcttgacaa gagcttccag tgtttgggtc atgatgaata agctaacacg gaggctgtct     780
aaaattccag aagaagtcag acaggagata ggatcttatt ttgtggattc tgatccaatt     840
ctggaagagg ataacagaaa actgactaaa cttgacgaca acacagcgga ttatattcgt     900
accggtttaa gtcctaggtg gagtgatcta gatatcaatc agcatgtcaa caatgtgaag     960
tacattggct ggattctgga gagtgctcca cagccaatct tggagagtca tgagctttct    1020
tccatgactt tagagtatag gagagagtgt ggtagggaca gtgtgctgga ttccctgact    1080
gctgtatctg ggccgacat gggcaatcta gctcacagcg ggcatgttga gtgcaagcat     1140
ttgcttcgac tggaaaatgg tgctgagatt gtgaggggca ggactgagtg gaggcccaaa    1200
cctgtgaaca actttggtgt tgtgaaccag gttccagcag aaagcaccta a             1251

<210>  109
<211>  416
<212>  PRT
<213>  Glycine max

<400>  109
Met Val Ala Thr Ala Ala Thr Ser Ser Phe Phe Pro Val Thr Ser Pro
1               5                   10                  15
Ser Pro Asp Ser Gly Gly Ala Gly Ser Lys Leu Gly Gly Gly Pro Ala
            20                  25                  30
Asn Leu Gly Gly Leu Lys Ser Lys Ser Ala Ser Ser Gly Gly Leu Lys
        35                  40                  45
Ala Lys Ala Gln Ala Pro Ser Lys Ile Asn Gly Thr Thr Val Val Thr
    50                  55                  60
Ser Lys Glu Ser Phe Lys His Asp Asp Asp Leu Pro Ser Pro Pro Pro
65                  70                  75                  80
Arg Thr Phe Ile Asn Gln Leu Pro Asp Trp Ser Met Leu Leu Ala Ala
                85                  90                  95
Ile Thr Thr Ile Phe Leu Ala Ala Glu Lys Gln Trp Met Met Leu Asp
            100                 105                 110
Trp Lys Pro Arg Arg Pro Asp Met Leu Ile Asp Pro Phe Gly Ile Gly
        115                 120                 125
Lys Ile Val Gln Asp Gly Leu Val Phe Arg Glu Asn Phe Ser Ile Arg
    130                 135                 140
Ser Tyr Glu Ile Gly Ala Asp Arg Thr Ala Ser Ile Glu Thr Val Met
145                 150                 155                 160
Asn His Leu Gln Glu Thr Ala Leu Asn His Val Lys Ser Ala Gly Leu
                165                 170                 175
Leu Gly Asp Gly Phe Gly Ser Thr Pro Glu Met Cys Lys Lys Asn Leu
            180                 185                 190
Ile Trp Val Val Thr Arg Met Gln Val Val Val Glu Arg Tyr Pro Thr
        195                 200                 205
Trp Gly Asp Ile Val Gln Val Asp Thr Trp Val Ser Gly Ser Gly Lys
    210                 215                 220
Asn Gly Met Arg Arg Asp Trp Leu Leu Arg Asp Cys Lys Thr Gly Glu
225                 230                 235                 240
Ile Leu Thr Arg Ala Ser Ser Val Trp Val Met Met Asn Lys Leu Thr
                245                 250                 255
Arg Arg Leu Ser Lys Ile Pro Glu Glu Val Arg Gln Glu Ile Gly Ser
            260                 265                 270
Tyr Phe Val Asp Ser Asp Pro Ile Leu Glu Glu Asp Asn Arg Lys Leu
        275                 280                 285
Thr Lys Leu Asp Asp Asn Thr Ala Asp Tyr Ile Arg Thr Gly Leu Ser
    290                 295                 300
Pro Arg Trp Ser Asp Leu Asp Ile Asn Gln His Val Asn Asn Val Lys
305                 310                 315                 320
Tyr Ile Gly Trp Ile Leu Glu Ser Ala Pro Gln Pro Ile Leu Glu Ser
                325                 330                 335
```

```
His Glu Leu Ser Ser Met Thr Leu Glu Tyr Arg Arg Glu Cys Gly Arg
            340                 345                 350
Asp Ser Val Leu Asp Ser Leu Thr Ala Val Ser Gly Ala Asp Met Gly
        355                 360                 365
Asn Leu Ala His Ser Gly His Val Glu Cys Lys His Leu Leu Arg Leu
    370                 375                 380
Glu Asn Gly Ala Glu Ile Val Arg Gly Arg Thr Glu Trp Arg Pro Lys
385                 390                 395                 400
Pro Val Asn Asn Phe Gly Val Val Asn Gln Val Pro Ala Glu Ser Thr
                405                 410                 415

<210>  110
<211>  1242
<212>  DNA
<213>  Gossypium hirsutum

<400>  110
atggttgcta ctgctgtgac atcggcgttt ttcccagtca cttcttcacc tgactcctct      60
gactcgaaaa acaagaagct cggaagcatc aagtcgaagc catcggtttc ttctggaagt     120
ttgcaagtca aggcaaatgc tcaagcacct ccgaaaataa acggcactgt ggcgtcgacg     180
actcccgtgg aaggttccaa gaacgatgac ggtgcaagtt cccctcctcc taggacgttt     240
atcaaccagt tacctgattg gagcatgctt cttgctgcta tcacaaccat tttcttggct     300
gctgagaagc agtggatgat gcttgattgg aagccgaggc ggcctgacat ggtcattgat     360
ccgtttggca tagggaagat tgttcaggat ggtcttgttt tcagtcagaa cttctcgatt     420
agatcatatg agataggcgc tgatcaaaca gcatccatag agacactaat gaatcattta     480
caggaaacag ctataaatca ttgtcgaagt gctggactgc ttggagaagg ttttggtgca     540
acacctgaga tgtgcaagaa gaacctaata tgggttgtca cacggatgca agttgtggtt     600
gatcgctatc ctacttgggg tgatgttgtt caagtcgaca cttgggtcag tgcatcgggg     660
aagaatggca tgcgaagaga ttggcttgtc agcaatagtg aaactggtga aattttaaca     720
cgagccacaa gtgtatgggt gatgatgaat aaactgacta gaaggttatc taaaatccca     780
gaagaggttc gaggggaaat agaacctttt tttatgaatt cagatcctgt tctggctgag     840
gatagccaga aactagtgaa actcgatgac agcacagctg aacacgtgtg caaaggttta     900
actcctaaat ggagcgactt ggatgtcaac cagcatgtca ataatgtgaa gtacattggc     960
tggatccttg agagtgctcc attaccaatc ttggagagtc acgagctttc cgccttgact    1020
ctggaatata ggagggagtg cgggagggac agcgtgctgc agtcactgac cactgtgtct    1080
gattccaata cggaaaatgc agtaaatgtt ggtgaattta attgccaaca tttgctccga    1140
ctcgacgatg gagctgagat tgtgagaggc aggacccgat ggaggcctaa acatgccaaa    1200
agttccgcta acatggatca aattaccgca aaaagggcat ag                       1242

<210>  111
<211>  413
<212>  PRT
<213>  Gossypium hirsutum

<400>  111
Met Val Ala Thr Ala Val Thr Ser Ala Phe Phe Pro Val Thr Ser Ser
1               5                   10                  15
Pro Asp Ser Ser Asp Ser Lys Asn Lys Lys Leu Gly Ser Ile Lys Ser
            20                  25                  30
Lys Pro Ser Val Ser Ser Gly Ser Leu Gln Val Lys Ala Asn Ala Gln
        35                  40                  45
Ala Pro Pro Lys Ile Asn Gly Thr Val Ala Ser Thr Thr Pro Val Glu
    50                  55                  60
Gly Ser Lys Asn Asp Asp Gly Ala Ser Ser Pro Pro Pro Arg Thr Phe
65                  70                  75                  80
Ile Asn Gln Leu Pro Asp Trp Ser Met Leu Leu Ala Ala Ile Thr Thr
                85                  90                  95
Ile Phe Leu Ala Ala Glu Lys Gln Trp Met Met Leu Asp Trp Lys Pro
            100                 105                 110
Arg Arg Pro Asp Met Val Ile Asp Pro Phe Gly Ile Gly Lys Ile Val
        115                 120                 125
Gln Asp Gly Leu Val Phe Ser Gln Asn Phe Ser Ile Arg Ser Tyr Glu
    130                 135                 140
```

Ile Gly Ala Asp Gln Thr Ala Ser Ile Glu Thr Leu Met Asn His Leu
145 150 155 160
Gln Glu Thr Ala Ile Asn His Cys Arg Ser Ala Gly Leu Leu Gly Glu
165 170 175
Gly Phe Gly Ala Thr Pro Glu Met Cys Lys Lys Asn Leu Ile Trp Val
180 185 190
Val Thr Arg Met Gln Val Val Val Asp Arg Tyr Pro Thr Trp Gly Asp
195 200 205
Val Val Gln Val Asp Thr Trp Val Ser Ala Ser Gly Lys Asn Gly Met
210 215 220
Arg Arg Asp Trp Leu Val Ser Asn Ser Glu Thr Gly Glu Ile Leu Thr
225 230 235 240
Arg Ala Thr Ser Val Trp Val Met Met Asn Lys Leu Thr Arg Arg Leu
245 250 255
Ser Lys Ile Pro Glu Glu Val Arg Gly Glu Ile Glu Pro Phe Phe Met
260 265 270
Asn Ser Asp Pro Val Leu Ala Glu Asp Ser Gln Lys Leu Val Lys Leu
275 280 285
Asp Asp Ser Thr Ala Glu His Val Cys Lys Gly Leu Thr Pro Lys Trp
290 295 300
Ser Asp Leu Asp Val Asn Gln His Val Asn Asn Val Lys Tyr Ile Gly
305 310 315 320
Trp Ile Leu Glu Ser Ala Pro Leu Pro Ile Leu Glu Ser His Glu Leu
325 330 335
Ser Ala Leu Thr Leu Glu Tyr Arg Arg Glu Cys Gly Arg Asp Ser Val
340 345 350
Leu Gln Ser Leu Thr Thr Val Ser Asp Ser Asn Thr Glu Asn Ala Val
355 360 365
Asn Val Gly Glu Phe Asn Cys Gln His Leu Leu Arg Leu Asp Asp Gly
370 375 380
Ala Glu Ile Val Arg Gly Arg Thr Arg Trp Arg Pro Lys His Ala Lys
385 390 395 400
Ser Ser Ala Asn Met Asp Gln Ile Thr Ala Lys Arg Ala
405 410

<210> 112
<211> 1293
<212> DNA
<213> Helianthus annuus

<400> 112
atggtagcta tgagtgctac tgcgtcgctg tttccggttt cttccccaaa acctcactct 60
ggagccaaga catctgataa gcttggaggt gaaccaggta gtgttgctgt gcgcggaatc 120
aagacaaaat ctgttaattc cggtggtatg aaagttaagg ctaacgcaca ggctcctact 180
gaggtgaatg ggagtagatc acgtatcacg catggcttca aaaccgatga ttattctaca 240
tcacctgccc cgagaacctt tatcaaccaa ttgcccgatt ggagcatgct tcttgctgca 300
atcacaacaa tcttcttggc tgcagagaag caatggatga tgctggaatg gaagaccaaa 360
cgcccccgata tgattgctga tatggatcct ttcggtttag ggaggattgt tcaagatggc 420
cttgtattcc gtcaaaactt ctctattaga tcatatgaaa taggggctga tcgaactgca 480
tcgatagaaa ccctaatgaa tcatttacaa gaaacggccc ttaatcatgt aaagtctgcg 540
ggtcttctgg gcgatggatt cggttcaaca ccagaaatgt gcaagaagaa tctattttgg 600
gtggtgacaa agatgcaggt gatagttgac cgttatccaa cttggggtga tgttgttcaa 660
gtagatactt gggtagcccc aaatgggaaa aatggtatgc gccgtgattg gctcgttcgc 720
gattataaaa caggcgagat tttaacaaga gcctcaagta actgggttat gatgaataaa 780
gagacaagga ggttatcgaa aatcccagat gaagttcgag gtgaaataga gcattacttt 840
gtagatgcac ctccggttgt ggaggatgat tctagaaaat tatctaaact tgacgaaagc 900
actgctgact atgttcgcga cggtttgatt ccaagatgga gtgatttgga tgtcaaccag 960
catgttaaca atgtgaagta tattggctgg atccttgaga gtgctccaca agttgtggag 1020
aagtacgagc ttgctcgcat tactctcgag taccgtagag aatgtaggaa ggatagtgtg 1080
gtgaaatcac tgacctcggt attaggtggt ggcgacgacg acaatggtgg aataggcgat 1140
tctggccgtg ttgattgcca acatgtgctc ttgtttgcgg gtggyggaga tggtactcct 1200
ggtggcgaga ttgtgaaggg aaggacccag tggcggccga aatatgagaa acaagatggg 1260
agtgttgatc acttctctgc tggaaatgtt taa 1293

<210> 113
<211> 430
<212> PRT
<213> Helianthus annuus

<400> 113

```
Met Val Ala Met Ser Ala Thr Ala Ser Leu Phe Pro Val Ser Ser Pro
1               5                   10                  15
Lys Pro His Ser Gly Ala Lys Thr Ser Asp Lys Leu Gly Gly Glu Pro
            20                  25                  30
Gly Ser Val Ala Val Arg Gly Ile Lys Thr Lys Ser Val Asn Ser Gly
        35                  40                  45
Gly Met Lys Val Lys Ala Asn Ala Gln Ala Pro Thr Glu Val Asn Gly
    50                  55                  60
Ser Arg Ser Arg Ile Thr His Gly Phe Lys Thr Asp Asp Tyr Ser Thr
65                  70                  75                  80
Ser Pro Ala Pro Arg Thr Phe Ile Asn Gln Leu Pro Asp Trp Ser Met
                85                  90                  95
Leu Leu Ala Ala Ile Thr Thr Ile Phe Leu Ala Ala Glu Lys Gln Trp
            100                 105                 110
Met Met Leu Glu Trp Lys Thr Lys Arg Pro Asp Met Ile Ala Asp Met
        115                 120                 125
Asp Pro Phe Gly Leu Gly Arg Ile Val Gln Asp Gly Leu Val Phe Arg
    130                 135                 140
Gln Asn Phe Ser Ile Arg Ser Tyr Glu Ile Gly Ala Asp Arg Thr Ala
145                 150                 155                 160
Ser Ile Glu Thr Leu Met Asn His Leu Gln Glu Thr Ala Leu Asn His
                165                 170                 175
Val Lys Ser Ala Gly Leu Leu Gly Asp Gly Phe Gly Ser Thr Pro Glu
            180                 185                 190
Met Cys Lys Lys Asn Leu Phe Trp Val Val Thr Lys Met Gln Val Ile
        195                 200                 205
Val Asp Arg Tyr Pro Thr Trp Gly Asp Val Val Gln Val Asp Thr Trp
    210                 215                 220
Val Ala Pro Asn Gly Lys Asn Gly Met Arg Arg Asp Trp Leu Val Arg
225                 230                 235                 240
Asp Tyr Lys Thr Gly Glu Ile Leu Thr Arg Ala Ser Ser Asn Trp Val
                245                 250                 255
Met Met Asn Lys Glu Thr Arg Arg Leu Ser Lys Ile Pro Asp Glu Val
            260                 265                 270
Arg Gly Glu Ile Glu His Tyr Phe Val Asp Ala Pro Pro Val Val Glu
        275                 280                 285
Asp Asp Ser Arg Lys Leu Ser Lys Leu Asp Glu Ser Thr Ala Asp Tyr
    290                 295                 300
Val Arg Asp Gly Leu Ile Pro Arg Trp Ser Asp Leu Asp Val Asn Gln
305                 310                 315                 320
His Val Asn Asn Val Lys Tyr Ile Gly Trp Ile Leu Glu Ser Ala Pro
                325                 330                 335
Gln Val Val Glu Lys Tyr Glu Leu Ala Arg Ile Thr Leu Glu Tyr Arg
            340                 345                 350
Arg Glu Cys Arg Lys Asp Ser Val Val Lys Ser Leu Thr Ser Val Leu
        355                 360                 365
Gly Gly Gly Asp Asp Asp Asn Gly Gly Ile Gly Asp Ser Gly Arg Val
    370                 375                 380
Asp Cys Gln His Val Leu Leu Phe Ala Gly Gly Gly Asp Gly Thr Pro
385                 390                 395                 400
Gly Gly Glu Ile Val Lys Gly Arg Thr Gln Trp Arg Pro Lys Tyr Glu
                405                 410                 415
Lys Gln Asp Gly Ser Val Asp His Phe Ser Ala Gly Asn Val
            420                 425                 430
```

<210> 114

<211> 1275
<212> DNA
<213> Iris tectorum

<400> 114
```
atggttgctt ccgtgtccgc ctcggccttc ttcccggtcc cctcctcctc gtcctcctct     60
tcctcttcga gctctaccgg gtccacaaaa ccctcgtcca tctccctcgg gaaagggccc    120
gatgccctcg atgcccgggg cctcgtggcc aaacccgcat ccaattccgg cagcttacaa    180
gtaaaggtca atgcccaagc cgccaccagg gttaatggat ccaaggtcgg gttgaaaacc    240
gataccaaca agcttgagga cacacccttt tttccttcct ccgccccgag gactttctac    300
aaccaattgc cagactggag cgtctccttt gctgccatca ccaccatctt cttggctgct    360
gagaagcaat ggacgcttat cgattggaag ccaaggcggc ccgacatgct cgccgatgca    420
ttcggccttg gaaagattat tgagaatgga cttgtctaca ggcagaactt ctccataagg    480
tcatatgaga ttggggcgga tcagacggca tctatagaga cgttaatgaa tcatttacag    540
gaaacggcgt taaaccatgt gaagtgtgcc ggactcttgg gtaatgggtt tggttccacg    600
ccggagatga gtaaaaagaa tttaatatgg gtcgtcacca aaatgcaggt ccttgtggag    660
cattatcctt cctgggggaa tgttattgaa gtagatacat gggctgcggt atctggaaag    720
aatggaatgc ggcgtgattg gcatgttcgg gactgccaaa ccggtcaaac tatcatgaga    780
agctccagca attgggtgat gatgaacaag gacaccagga ggttgtctaa atttcctgaa    840
gaagttagag ctgaaataga accctacttc atggagcgtg ttcctgtcat tgatgatgac    900
aacaggaagc tccctaagct tgatgatgat actgctgatc atgttcgcaa gggtctaact    960
ccaagatgga gtgacttgga tgtcaatcag catgtgaaca atgtcaagta cattggatgg   1020
atccttgaga gtgctccaat ctccatcctg gagagtcatg agcttgcaag catgactctt   1080
gagtacagga gggagtgtgg aagggacagc atgctgcagt ccctcacctc actttctaac   1140
gattgcactg atgggctcgg cgagcttccc attgaatgtc agcatctact ccgctcgagg   1200
gtgggcctga atgtgaaagg acgaactgag tggaggccca agaaacgtgc cccctccct   1260
gttgggagcc catga                                                    1275
```

<210> 115
<211> 424
<212> PRT
<213> Iris tectorum

<400> 115
```
Met Val Ala Ser Val Ser Ala Ser Ala Phe Phe Pro Val Pro Ser Ser
1               5                   10                  15
Ser Ser Ser Ser Ser Ser Ser Ser Ser Thr Gly Ser Thr Lys Pro Ser
            20                  25                  30
Ser Ile Ser Leu Gly Lys Gly Pro Asp Ala Leu Asp Ala Arg Gly Leu
        35                  40                  45
Val Ala Lys Pro Ala Ser Asn Ser Gly Ser Leu Gln Val Lys Val Asn
    50                  55                  60
Ala Gln Ala Ala Thr Arg Val Asn Gly Ser Lys Val Gly Leu Lys Thr
65                  70                  75                  80
Asp Thr Asn Lys Leu Glu Asp Thr Pro Phe Phe Pro Ser Ser Ala Pro
                85                  90                  95
Arg Thr Phe Tyr Asn Gln Leu Pro Asp Trp Ser Val Ser Phe Ala Ala
            100                 105                 110
Ile Thr Thr Ile Phe Leu Ala Ala Glu Lys Gln Trp Thr Leu Ile Asp
        115                 120                 125
Trp Lys Pro Arg Arg Pro Asp Met Leu Ala Asp Ala Phe Gly Leu Gly
    130                 135                 140
Lys Ile Ile Glu Asn Gly Leu Val Tyr Arg Gln Asn Phe Ser Ile Arg
145                 150                 155                 160
Ser Tyr Glu Ile Gly Ala Asp Gln Thr Ala Ser Ile Glu Thr Leu Met
                165                 170                 175
Asn His Leu Gln Glu Thr Ala Leu Asn His Val Lys Cys Ala Gly Leu
            180                 185                 190
Leu Gly Asn Gly Phe Gly Ser Thr Pro Glu Met Ser Lys Lys Asn Leu
        195                 200                 205
Ile Trp Val Val Thr Lys Met Gln Val Leu Val Glu His Tyr Pro Ser
    210                 215                 220
Trp Gly Asn Val Ile Glu Val Asp Thr Trp Ala Ala Val Ser Gly Lys
```

```
225                 230                 235                 240
Asn Gly Met Arg Arg Asp Trp His Val Arg Asp Cys Gln Thr Gly Gln
                245                 250                 255
Thr Ile Met Arg Ser Ser Ser Asn Trp Val Met Met Asn Lys Asp Thr
            260                 265                 270
Arg Arg Leu Ser Lys Phe Pro Glu Glu Val Arg Ala Glu Ile Glu Pro
        275                 280                 285
Tyr Phe Met Glu Arg Val Pro Val Ile Asp Asp Asp Asn Arg Lys Leu
    290                 295                 300
Pro Lys Leu Asp Asp Asp Thr Ala Asp His Val Arg Lys Gly Leu Thr
305                 310                 315                 320
Pro Arg Trp Ser Asp Leu Asp Val Asn Gln His Val Asn Asn Val Lys
                325                 330                 335
Tyr Ile Gly Trp Ile Leu Glu Ser Ala Pro Ile Ser Ile Leu Glu Ser
            340                 345                 350
His Glu Leu Ala Ser Met Thr Leu Glu Tyr Arg Arg Glu Cys Gly Arg
        355                 360                 365
Asp Ser Met Leu Gln Ser Leu Thr Ser Leu Ser Asn Asp Cys Thr Asp
    370                 375                 380
Gly Leu Gly Glu Leu Pro Ile Glu Cys Gln His Leu Leu Arg Ser Arg
385                 390                 395                 400
Val Gly Leu Asn Val Lys Gly Arg Thr Glu Trp Arg Pro Lys Lys Arg
                405                 410                 415
Ala Pro Phe Pro Val Gly Ser Pro
            420
```

<210> 116
<211> 1257
<212> DNA
<213> Jatropha curcas

<400> 116

```
atggttgcta ctgctgctac ttcctcgttc ttccctgttc ctacttcatc tgcagattcc     60
aagtccacca agattggtag tgggtctgca agtttgggag gaatcaaatc aaaacctgct    120
tcttctgggg gcttgcaagt caaggcaaat gcccaagccc ctcccaagat aaatggatcc    180
acagtaggct atacaacacc tgtggacagt gtgaaaaatg agggtgacac gccatcaccg    240
cccccaagga cctttatcaa ccaattacct gattggagca tgcttcttgc tgctattaca    300
actatattct tggcagcaga gaagcagtgg atgatgcttg actggaaacc acggcgacct    360
gacatgctta ttgacccttt tggtctaggg agaattgttc aggatggcct tgtgttcagg    420
cagaacttct ccatccgatc atatgaaatt ggcgcggatc ggacagcatc catagagaca    480
ttgatgaatc atttacaaga aacagccctc aaccatgtta agactgctgg acttcttggt    540
gaggggtttg gttcaacacc agagatgagt aaaaggaacc tgatatgggt ggttactcgg    600
atgcaggtcc tggtggatcg ttatccaacg tggggtgatg ttgttgaagt agatacttgg    660
gtgagtgcat caggaaaaaa tggcatgcgc cgcgattggc ttgttcgtga cagtaaaacc    720
ggtgaaactc taacaagagc ctccagtgtg tgggtaatga tgaataaact gactaggaga    780
ttatctaaaa ttcctgaaga ggttaggggg gaaatagagc cttacttttt gaattctgat    840
cctattgtgg atgaggatgg cagaaaactg ccaaaacttg atgacaacac tgcggattat    900
gtttgcaaag gtttaactcc tagatggagt gatttagatg tcaaccaaca tgttaacaat    960
gtgaagtaca ttggctggat ccttgagagt gctccgctgc cgatcctgga gagtcatgag   1020
ctatcatcca ttattatgga atataggagg gagtgtggaa gggatagtgt gcttcagtcg   1080
ctgactgctg tctctggcac cggcttagga aatttaggaa atgctggtga aattgagtgt   1140
cagcacttgc ttcgactgga ggaaggtgct gagatagtaa ggggaaggac tgcgtggagg   1200
ccaaagtatc gcagcaactt tggaattatg ggtcagattc cagttgaaag tgcctaa      1257
```

<210> 117
<211> 418
<212> PRT
<213> Jatropha curcas

<400> 117

```
Met Val Ala Thr Ala Ala Thr Ser Ser Phe Phe Pro Val Pro Thr Ser
1               5                   10                  15
Ser Ala Asp Ser Lys Ser Thr Lys Ile Gly Ser Gly Ser Ala Ser Leu
```

```
            20                  25                  30
Gly Gly Ile Lys Ser Lys Pro Ala Ser Ser Gly Gly Leu Gln Val Lys
        35                  40                  45
Ala Asn Ala Gln Ala Pro Pro Lys Ile Asn Gly Ser Thr Val Gly Tyr
    50                  55                  60
Thr Thr Pro Val Asp Ser Val Lys Asn Glu Gly Asp Thr Pro Ser Pro
65                  70                  75                  80
Pro Pro Arg Thr Phe Ile Asn Gln Leu Pro Asp Trp Ser Met Leu Leu
                85                  90                  95
Ala Ala Ile Thr Thr Ile Phe Leu Ala Ala Glu Lys Gln Trp Met Met
            100                 105                 110
Leu Asp Trp Lys Pro Arg Arg Pro Asp Met Leu Ile Asp Pro Phe Gly
        115                 120                 125
Leu Gly Arg Ile Val Gln Asp Gly Leu Val Phe Arg Gln Asn Phe Ser
    130                 135                 140
Ile Arg Ser Tyr Glu Ile Gly Ala Asp Arg Thr Ala Ser Ile Glu Thr
145                 150                 155                 160
Leu Met Asn His Leu Gln Glu Thr Ala Leu Asn His Val Lys Thr Ala
                165                 170                 175
Gly Leu Leu Gly Glu Gly Phe Gly Ser Thr Pro Glu Met Ser Lys Arg
            180                 185                 190
Asn Leu Ile Trp Val Val Thr Arg Met Gln Val Leu Val Asp Arg Tyr
        195                 200                 205
Pro Thr Trp Gly Asp Val Val Glu Val Asp Thr Trp Val Ser Ala Ser
    210                 215                 220
Gly Lys Asn Gly Met Arg Arg Asp Trp Leu Val Arg Asp Ser Lys Thr
225                 230                 235                 240
Gly Glu Thr Leu Thr Arg Ala Ser Ser Val Trp Val Met Met Asn Lys
                245                 250                 255
Leu Thr Arg Arg Leu Ser Lys Ile Pro Glu Glu Val Arg Gly Glu Ile
            260                 265                 270
Glu Pro Tyr Phe Leu Asn Ser Asp Pro Ile Val Asp Glu Asp Gly Arg
        275                 280                 285
Lys Leu Pro Lys Leu Asp Asp Asn Thr Ala Asp Tyr Val Cys Lys Gly
    290                 295                 300
Leu Thr Pro Arg Trp Ser Asp Leu Asp Val Asn Gln His Val Asn Asn
305                 310                 315                 320
Val Lys Tyr Ile Gly Trp Ile Leu Glu Ser Ala Pro Leu Pro Ile Leu
                325                 330                 335
Glu Ser His Glu Leu Ser Ser Ile Ile Met Glu Tyr Arg Arg Glu Cys
            340                 345                 350
Gly Arg Asp Ser Val Leu Gln Ser Leu Thr Ala Val Ser Gly Thr Gly
        355                 360                 365
Leu Gly Asn Leu Gly Asn Ala Gly Glu Ile Glu Cys Gln His Leu Leu
    370                 375                 380
Arg Leu Glu Glu Gly Ala Glu Ile Val Arg Gly Arg Thr Ala Trp Arg
385                 390                 395                 400
Pro Lys Tyr Arg Ser Asn Phe Gly Ile Met Gly Gln Ile Pro Val Glu
                405                 410                 415
Ser Ala
```

```
<210>  118
<211>  1248
<212>  DNA
<213>  Malus domestica

<400>  118
atggttgcca ctgctgctac tgcctcgttc tttccggttt cttctcccaa ctcagactca      60
agcgccaaga acgccaagct cgggtcagcc aatttaggac tcaaatcgaa gtctgcatct     120
ggtggtttgc aggtaaaggc aaatgctcaa gccccttcaa agataaatgg aactagtgtt     180
ggtttggcaa ctgtggaaag tgggaagcat ggggatgaca tttcatcccc tccggcacgg     240
actttcatta accaattacc tgattggagt gtgctccttg ctgctattac cacaatcttc     300
```

```
ttggctgcag agaagcaatg gacaatgctt gattggaaac ccaagcgacc tgacatgctc     360
attgacccat ttggtctagg acgaattgtt caggatggtc ttgtctttcg ccagaacttc     420
tcaattagat catatgaaat aggtgctgat cgtacggctt caatagagac gttaatgaat     480
catttacagg aaacagcact taatcatgtt aagactgctg gacttctggg agatggtttt     540
ggttcaactc cagagatgac tgtaagaaac ctgatatggg tggtaacgaa gatgcaggtt     600
gtggtagacc gctatcctac ttggggtgac gttgttcaag ttgacacttg ggttagtgcc     660
tctgggaaga atggaatgcg tcgtgattgg attatccagg atttgaaaac tggtcaaatt     720
ctaacaagag cctccagtgt gtgggtgatg atgaataaag tgacgaggag attatcaaag     780
atgcctgatg cagttcgcgg tgaaatagag tcctttttta tgaattctcc tcctgttgtg     840
gaggaagatg gcaggaaact gccgaaactt gatgacaaaa cagcggacgt tgttctctct     900
ggtttgactc ctagatggag tgatttagat gtcaaccagc atgttaataa cgtgaagtac     960
attggctgga tccttgaggg tgctcccttg ccaatcctgg agagtcatga gctctcttct    1020
ttgactctgg agtataggag ggagtgcggg agggacagtg tgcttcagtc tctgactgca    1080
gtctcaggtg ctgatatcgg caacctggga agtaatggca cggtggagtg ccagcacatg    1140
cttcgacttg aggatggggc tgagattgtg aggggaagga ctgagtggag gcccaaatat    1200
gccaacaatc ttgggattgt gggtcatctt ccagcagaaa gcgcatag                 1248

<210>  119
<211>  415
<212>  PRT
<213>  Malus domestica

<400>  119
Met Val Ala Thr Ala Ala Thr Ala Ser Phe Phe Pro Val Ser Ser Pro
1               5                   10                  15
Asn Ser Asp Ser Ser Ala Lys Asn Ala Lys Leu Gly Ser Ala Asn Leu
            20                  25                  30
Gly Leu Lys Ser Lys Ser Ala Ser Gly Gly Leu Gln Val Lys Ala Asn
        35                  40                  45
Ala Gln Ala Pro Ser Lys Ile Asn Gly Thr Ser Val Gly Leu Ala Thr
    50                  55                  60
Val Glu Ser Gly Lys His Gly Asp Asp Ile Ser Ser Pro Pro Ala Arg
65                  70                  75                  80
Thr Phe Ile Asn Gln Leu Pro Asp Trp Ser Val Leu Leu Ala Ala Ile
                85                  90                  95
Thr Thr Ile Phe Leu Ala Ala Glu Lys Gln Trp Thr Met Leu Asp Trp
            100                 105                 110
Lys Pro Lys Arg Pro Asp Met Leu Ile Asp Pro Phe Gly Leu Gly Arg
        115                 120                 125
Ile Val Gln Asp Gly Leu Val Phe Arg Gln Asn Phe Ser Ile Arg Ser
    130                 135                 140
Tyr Glu Ile Gly Ala Asp Arg Thr Ala Ser Ile Glu Thr Leu Met Asn
145                 150                 155                 160
His Leu Gln Glu Thr Ala Leu Asn His Val Lys Thr Ala Gly Leu Leu
                165                 170                 175
Gly Asp Gly Phe Gly Ser Thr Pro Glu Met Thr Val Arg Asn Leu Ile
            180                 185                 190
Trp Val Val Thr Lys Met Gln Val Val Val Asp Arg Tyr Pro Thr Trp
        195                 200                 205
Gly Asp Val Val Gln Val Asp Thr Trp Val Ser Ala Ser Gly Lys Asn
    210                 215                 220
Gly Met Arg Arg Asp Trp Ile Ile Gln Asp Leu Lys Thr Gly Gln Ile
225                 230                 235                 240
Leu Thr Arg Ala Ser Ser Val Trp Val Met Met Asn Lys Val Thr Arg
                245                 250                 255
Arg Leu Ser Lys Met Pro Asp Ala Val Arg Gly Glu Ile Glu Ser Phe
            260                 265                 270
Phe Met Asn Ser Pro Pro Val Val Glu Glu Asp Gly Arg Lys Leu Pro
        275                 280                 285
Lys Leu Asp Asp Lys Thr Ala Asp Val Val Leu Ser Gly Leu Thr Pro
    290                 295                 300
Arg Trp Ser Asp Leu Asp Val Asn Gln His Val Asn Asn Val Lys Tyr
305                 310                 315                 320
```

Ile Gly Trp Ile Leu Glu Gly Ala Pro Leu Pro Ile Leu Glu Ser His
325 330 335
Glu Leu Ser Ser Leu Thr Leu Glu Tyr Arg Arg Glu Cys Gly Arg Asp
340 345 350
Ser Val Leu Gln Ser Leu Thr Ala Val Ser Gly Ala Asp Ile Gly Asn
355 360 365
Leu Gly Ser Asn Gly Thr Val Glu Cys Gln His Met Leu Arg Leu Glu
370 375 380
Asp Gly Ala Glu Ile Val Arg Gly Arg Thr Glu Trp Arg Pro Lys Tyr
385 390 395 400
Ala Asn Asn Leu Gly Ile Val Gly His Leu Pro Ala Glu Ser Ala
405 410 415

<210> 120
<211> 1284
<212> DNA
<213> Oryza sativa

<400> 120
atggctggtt ctcttgcggc gtctgcattc ttccctgtcc cagggtcttc ccctgcagct 60
tcggctagaa gctctaagaa cacaaccggt gaattgccag agaatttgag tgtccgcgga 120
atcgtcgcga agcctaatcc gtctccaggg gccatgcaag tcaaggcgca ggcgcaagcc 180
cttcctaagg ttaatggaac caaggttaac ctgaagacta caagcccaga caaggaggat 240
ataataccgt acactgctcc gaagacattc tataaccaat tgccagactg gagcatgctt 300
cttgcagctg tcacgaccat tttcctggca gctgagaagc agtggactct gcttgactgg 360
aagccgaaga agcctgacat gctggctgac acattcggct ttggtaggat catccaagac 420
gggctggtgt ttaggcaaaa cttcttgatt cggtcctacg agattggtgc tgatcgtaca 480
gcttctattg agacattaat gaatcattta caggaaacag ctctgaacca tgtgaaaact 540
gctggtctct taggtgatgg ttttggtgct acgccggaga tgagcaaacg gaacttaata 600
tgggttgtca gcaaaattca gcttcttgtt gagcgatacc catcatgggg agatatggtc 660
caagttgaca catgggtagc tgctgctggc aaaaatggca tgcgtcgaga ttggcatgtt 720
cgggactaca actctggtca aacaatcttg agggctacaa gtgtttgggt gatgatgaat 780
aagaacacta gaagactttc aaaaatgcca gatgaagtta gagctgaaat aggcccgtat 840
ttcaatggcc gttctgctat atcagaggag cagggtgaaa agttgcctaa gccagggacc 900
acatttgatg gcgctgctac caaacaattc acaagaaaag ggcttactcc gaagtggagt 960
gaccttgatg tcaaccagca tgtgaacaat gtgaagtata ttggttggat acttgagagt 1020
gctccaattt cgatactgga gaagcacgag cttgcaagca tgaccttgga ttacaggaag 1080
gagtgtggcc gtgacagtgt gcttcagtcg cttaccgctg tttcaggtga atgcgatgat 1140
ggcaacacag aatcctccat ccagtgtgac catctgcttc agctggagtc cggagcagac 1200
attgtgaagg ctcacacaga gtggcgaccg aagcgagctc agggcgaggg gaacatgggc 1260
tttttcccag ctgagagtgc atga 1284

<210> 121
<211> 427
<212> PRT
<213> Oryza sativa

<400> 121
Met Ala Gly Ser Leu Ala Ala Ser Ala Phe Phe Pro Val Pro Gly Ser
1 5 10 15
Ser Pro Ala Ala Ser Ala Arg Ser Ser Lys Asn Thr Thr Gly Glu Leu
20 25 30
Pro Glu Asn Leu Ser Val Arg Gly Ile Val Ala Lys Pro Asn Pro Ser
35 40 45
Pro Gly Ala Met Gln Val Lys Ala Gln Ala Gln Ala Leu Pro Lys Val
50 55 60
Asn Gly Thr Lys Val Asn Leu Lys Thr Thr Ser Pro Asp Lys Glu Asp
65 70 75 80
Ile Ile Pro Tyr Thr Ala Pro Lys Thr Phe Tyr Asn Gln Leu Pro Asp
85 90 95
Trp Ser Met Leu Leu Ala Ala Val Thr Thr Ile Phe Leu Ala Ala Glu
100 105 110
Lys Gln Trp Thr Leu Leu Asp Trp Lys Pro Lys Lys Pro Asp Met Leu

```
        115                 120                 125
Ala Asp Thr Phe Gly Phe Gly Arg Ile Ile Gln Asp Gly Leu Val Phe
    130                 135                 140
Arg Gln Asn Phe Leu Ile Arg Ser Tyr Glu Ile Gly Ala Asp Arg Thr
145                 150                 155                 160
Ala Ser Ile Glu Thr Leu Met Asn His Leu Gln Glu Thr Ala Leu Asn
                165                 170                 175
His Val Lys Thr Ala Gly Leu Leu Gly Asp Gly Phe Gly Ala Thr Pro
            180                 185                 190
Glu Met Ser Lys Arg Asn Leu Ile Trp Val Val Ser Lys Ile Gln Leu
        195                 200                 205
Leu Val Glu Arg Tyr Pro Ser Trp Gly Asp Met Val Gln Val Asp Thr
    210                 215                 220
Trp Val Ala Ala Ala Gly Lys Asn Gly Met Arg Arg Asp Trp His Val
225                 230                 235                 240
Arg Asp Tyr Asn Ser Gly Gln Thr Ile Leu Arg Ala Thr Ser Val Trp
                245                 250                 255
Val Met Met Asn Lys Asn Thr Arg Arg Leu Ser Lys Met Pro Asp Glu
            260                 265                 270
Val Arg Ala Glu Ile Gly Pro Tyr Phe Asn Gly Arg Ser Ala Ile Ser
        275                 280                 285
Glu Glu Gln Gly Glu Lys Leu Pro Lys Pro Gly Thr Thr Phe Asp Gly
    290                 295                 300
Ala Ala Thr Lys Gln Phe Thr Arg Lys Gly Leu Thr Pro Lys Trp Ser
305                 310                 315                 320
Asp Leu Asp Val Asn Gln His Val Asn Asn Val Lys Tyr Ile Gly Trp
                325                 330                 335
Ile Leu Glu Ser Ala Pro Ile Ser Ile Leu Glu Lys His Glu Leu Ala
            340                 345                 350
Ser Met Thr Leu Asp Tyr Arg Lys Glu Cys Gly Arg Asp Ser Val Leu
        355                 360                 365
Gln Ser Leu Thr Ala Val Ser Gly Glu Cys Asp Asp Gly Asn Thr Glu
    370                 375                 380
Ser Ser Ile Gln Cys Asp His Leu Leu Gln Leu Glu Ser Gly Ala Asp
385                 390                 395                 400
Ile Val Lys Ala His Thr Glu Trp Arg Pro Lys Arg Ala Gln Gly Glu
                405                 410                 415
Gly Asn Met Gly Phe Phe Pro Ala Glu Ser Ala
            420                 425
```

<210> 122
<211> 1326
<212> DNA
<213> Picea glauca

<400> 122

```
atggtagccg ccgctgcaac aatgctaatg ttttcttcaa gctctcagtg caacacacag     60
aacaagatct cgtcatctgc ttcatcaggg aagcccacaa tgccagttag ctctcctgag    120
cgtgttgatg ttaagtccaa acccactgca tacaagggac tccaagtcaa tggaaattcc    180
cacggagcta ctaataagat aaatggcact aaggtgaacg gaacagcagt ggatagcatg    240
aagcataacg ttggcctgaa ggaagcatcc gaggaagaaa gcactgctaa gagcaggatc    300
aatcagctcc cagattggag tatgcttctc gcaactattg ctaccattat tctggcagcc    360
gaaaagcagt ggaccaattt tgattggaag ccaaggaaaa cagacgtgtt tggtgacgtt    420
ttcaggctgg gcaggtttgt ggaagacagt ctggttttcc ggcagaactt cgccataaga    480
tcttatgaaa ttggtgcaga caaaacggct tctattgaaa ccttgatgaa ccatcttcag    540
gaaactgccc ttaatcatgt ttggctttct gggctagctg gggatggatt cggtgctact    600
cttgagatga gccggagaaa tctcctatgg gttgtggctc gcatgcaaat tcaagttgaa    660
cgatatccct catggggtga tgttgtggag atagatacat gggttgggcc atcaggtaaa    720
aatggcatgc ggcgtgattg gcttgttcga gattcgaaga cgaatgccat ccttacacga    780
gctactagta cctgggtaat gatgaataga aagacaagaa aactgtccaa aattcctgat    840
gctgtcaaag cagagataca gccttatttc acagaaagaa atgtctttgt ggcagaagac    900
accagaaagt tgcataagct ggaggatgac actgcccagt acatctgttc ggatttaaca    960
ccgcggtgga gtgatttgga tgtgaatcag catgtcaata atgttaaata tattggttgg   1020
```

```
attttggaga gtttacccat ctctgtttta gagggcaacg aactagctaa tataacgttg   1080
gagtacagac gtgaatgtgg accgacgcat gtactccaat cattgacaag tccacaggct   1140
ggtgaggtga ttgctgcttc agctgcacca ttttcacaga gaaatgatcc tccagacacc   1200
tggaaaccct tgcctgcatt gcagtttgca cacttgcttc gattgcaaga tgacagatcg   1260
gaaattctga gggcaaggtc agagtggagg tcaaaggcaa agaacaacct tcacgacctt   1320
gcttga                                                              1326

<210>  123
<211>  441
<212>  PRT
<213>  Picea glauca

<400>  123
Met Val Ala Ala Ala Ala Thr Met Leu Met Phe Ser Ser Ser Ser Gln
1               5                   10                  15
Cys Asn Thr Gln Asn Lys Ile Ser Ser Ser Ala Ser Ser Gly Lys Pro
            20                  25                  30
Thr Met Pro Val Ser Ser Pro Glu Arg Val Asp Val Lys Ser Lys Pro
        35                  40                  45
Thr Ala Tyr Lys Gly Leu Gln Val Asn Gly Asn Ser His Gly Ala Thr
    50                  55                  60
Asn Lys Ile Asn Gly Thr Lys Val Asn Gly Thr Ala Val Asp Ser Met
65                  70                  75                  80
Lys His Asn Val Gly Leu Lys Glu Ala Ser Glu Glu Glu Ser Thr Ala
                85                  90                  95
Lys Ser Arg Ile Asn Gln Leu Pro Asp Trp Ser Met Leu Leu Ala Thr
            100                 105                 110
Ile Ala Thr Ile Ile Leu Ala Ala Glu Lys Gln Trp Thr Asn Phe Asp
        115                 120                 125
Trp Lys Pro Arg Lys Thr Asp Val Phe Gly Asp Val Phe Arg Leu Gly
    130                 135                 140
Arg Phe Val Glu Asp Ser Leu Val Phe Arg Gln Asn Phe Ala Ile Arg
145                 150                 155                 160
Ser Tyr Glu Ile Gly Ala Asp Lys Thr Ala Ser Ile Glu Thr Leu Met
                165                 170                 175
Asn His Leu Gln Glu Thr Ala Leu Asn His Val Trp Leu Ser Gly Leu
            180                 185                 190
Ala Gly Asp Gly Phe Gly Ala Thr Leu Glu Met Ser Arg Arg Asn Leu
        195                 200                 205
Leu Trp Val Val Ala Arg Met Gln Ile Gln Val Glu Arg Tyr Pro Ser
    210                 215                 220
Trp Gly Asp Val Val Glu Ile Asp Thr Trp Val Gly Pro Ser Gly Lys
225                 230                 235                 240
Asn Gly Met Arg Arg Asp Trp Leu Val Arg Asp Ser Lys Thr Asn Ala
                245                 250                 255
Ile Leu Thr Arg Ala Thr Ser Thr Trp Val Met Met Asn Arg Lys Thr
            260                 265                 270
Arg Lys Leu Ser Lys Ile Pro Asp Ala Val Lys Ala Glu Ile Gln Pro
        275                 280                 285
Tyr Phe Thr Glu Arg Asn Val Phe Val Ala Glu Asp Thr Arg Lys Leu
    290                 295                 300
His Lys Leu Glu Asp Asp Thr Ala Gln Tyr Ile Cys Ser Asp Leu Thr
305                 310                 315                 320
Pro Arg Trp Ser Asp Leu Asp Val Asn Gln His Val Asn Asn Val Lys
                325                 330                 335
Tyr Ile Gly Trp Ile Leu Glu Ser Leu Pro Ile Ser Val Leu Glu Gly
            340                 345                 350
Asn Glu Leu Ala Asn Ile Thr Leu Glu Tyr Arg Arg Glu Cys Gly Pro
        355                 360                 365
Thr His Val Leu Gln Ser Leu Thr Ser Pro Gln Ala Gly Glu Val Ile
    370                 375                 380
Ala Ala Ser Ala Ala Pro Phe Ser Gln Arg Asn Asp Pro Pro Asp Thr
385                 390                 395                 400
```

```
Trp Lys Pro Leu Pro Ala Leu Gln Phe Ala His Leu Leu Arg Leu Gln
                405                 410                 415
Asp Asp Arg Ser Glu Ile Leu Arg Ala Arg Ser Glu Trp Arg Ser Lys
            420                 425                 430
Ala Lys Asn Asn Leu His Asp Leu Ala
        435                 440

<210>  124
<211>  1266
<212>  DNA
<213>  Populus tomentosiformis

<400>  124
atggttgcca cagcagctac ttcatcattt ttcccagttc cttcaccacc tggagatgcc      60
aagtcctcca aggttggtag tggttctgca agtttgggag gaatcaaatc gaaatctgct     120
tcctctggag ctttgcaggt taaggcaaat gcccaagctc ctccgaagat aaatggctct     180
ccagttggct tgacagcatc agtggaaact gcgaagaagg aggatgttgt ctcatcaccg     240
gcaccccgga catttatcaa ccaattacct gattggagca tgcttcttgc tgcaattaca     300
accatgtttt tggcagcaga gaagcagtgg atgatgcttg attggaaacc aaagcgagct     360
gacatgctta ttgatccctt tggtattgga agaattgtcc aagatggtct tgtcttcagc     420
cagaatttct caattaggtc atatgaaatt ggtgcagatc gtactgcgtc tatagagacg     480
ttgatgaacc atttacaaga aacagcactt aatcatgtta agactgctgg gcttcttgga     540
gatggatttg gttcaacccc agagatgtcc aaaaggaacc tgatatgggt ggtaactcga     600
atgcagattc tagtcgatcg ttatcctaca tggggtgatg ttgtccatgt ggatacttgg     660
gtgagtgcat caggaaagaa tggtatgcgc cgtgattggc ttgtccgtga tgctaaaact     720
ggtgaaactc ttacaagagc ctccagtttg tgggtgatga tgaataaagt gacaaggagg     780
ttatctaaaa ttcctgaaga tgttcgaggt gaaatagagc cttattttct gaattctgat     840
cctgttgtga atgaggacag cacaaaactg ccaaaacttg acgacaagac ggcggactat     900
atccgcaaag gcctaactcc tagatggaat gatttagatg tcaaccagca tgttaacaat     960
gtgaaataca taggctggat ccttgagagc gctcctcccc caatcctgga gagtcatgag    1020
cttgctgcca ttactttgga gtacaggagg gagtgtggca gggacagcgt gctgcagtcc    1080
ttgactgctg tatctggcgc tggcattgga aatttgggcg gtcctggtaa agttgagtgt    1140
caacatttgc tgcgacatga ggatggtgct gagatcgtga ggggaaggac cgagtggagg    1200
cccaaacatg ccaacaattt tggcatgatg ggtggtcaga tgccagctga tgagagcggt    1260
gcttaa                                                               1266

<210>  125
<211>  421
<212>  PRT
<213>  Populus tomentosiformis

<400>  125
Met Val Ala Thr Ala Ala Thr Ser Ser Phe Phe Pro Val Pro Ser Pro
1               5                   10                  15
Pro Gly Asp Ala Lys Ser Ser Lys Val Gly Ser Gly Ser Ala Ser Leu
            20                  25                  30
Gly Gly Ile Lys Ser Lys Ser Ala Ser Ser Gly Ala Leu Gln Val Lys
        35                  40                  45
Ala Asn Ala Gln Ala Pro Pro Lys Ile Asn Gly Ser Pro Val Gly Leu
    50                  55                  60
Thr Ala Ser Val Glu Thr Ala Lys Lys Glu Asp Val Val Ser Ser Pro
65                  70                  75                  80
Ala Pro Arg Thr Phe Ile Asn Gln Leu Pro Asp Trp Ser Met Leu Leu
                85                  90                  95
Ala Ala Ile Thr Thr Met Phe Leu Ala Ala Glu Lys Gln Trp Met Met
            100                 105                 110
Leu Asp Trp Lys Pro Lys Arg Ala Asp Met Leu Ile Asp Pro Phe Gly
        115                 120                 125
Ile Gly Arg Ile Val Gln Asp Gly Leu Val Phe Ser Gln Asn Phe Ser
    130                 135                 140
Ile Arg Ser Tyr Glu Ile Gly Ala Asp Arg Thr Ala Ser Ile Glu Thr
145                 150                 155                 160
Leu Met Asn His Leu Gln Glu Thr Ala Leu Asn His Val Lys Thr Ala
```

```
                165                 170                 175
Gly Leu Leu Gly Asp Gly Phe Gly Ser Thr Pro Glu Met Ser Lys Arg
            180                 185                 190
Asn Leu Ile Trp Val Val Thr Arg Met Gln Ile Leu Val Asp Arg Tyr
        195                 200                 205
Pro Thr Trp Gly Asp Val Val His Val Asp Thr Trp Val Ser Ala Ser
    210                 215                 220
Gly Lys Asn Gly Met Arg Arg Asp Trp Leu Val Arg Asp Ala Lys Thr
225                 230                 235                 240
Gly Glu Thr Leu Thr Arg Ala Ser Ser Leu Trp Val Met Met Asn Lys
                245                 250                 255
Val Thr Arg Arg Leu Ser Lys Ile Pro Glu Asp Val Arg Gly Glu Ile
            260                 265                 270
Glu Pro Tyr Phe Leu Asn Ser Asp Pro Val Val Asn Glu Asp Ser Thr
        275                 280                 285
Lys Leu Pro Lys Leu Asp Asp Lys Thr Ala Asp Tyr Ile Arg Lys Gly
    290                 295                 300
Leu Thr Pro Arg Trp Asn Asp Leu Asp Val Asn Gln His Val Asn Asn
305                 310                 315                 320
Val Lys Tyr Ile Gly Trp Ile Leu Glu Ser Ala Pro Pro Pro Ile Leu
                325                 330                 335
Glu Ser His Glu Leu Ala Ala Ile Thr Leu Glu Tyr Arg Arg Glu Cys
            340                 345                 350
Gly Arg Asp Ser Val Leu Gln Ser Leu Thr Ala Val Ser Gly Ala Gly
        355                 360                 365
Ile Gly Asn Leu Gly Gly Pro Gly Lys Val Glu Cys Gln His Leu Leu
    370                 375                 380
Arg His Glu Asp Gly Ala Glu Ile Val Arg Gly Arg Thr Glu Trp Arg
385                 390                 395                 400
Pro Lys His Ala Asn Asn Phe Gly Met Met Gly Gly Gln Met Pro Ala
                405                 410                 415
Asp Glu Ser Gly Ala
            420
```

<210> 126
<211> 1260
<212> DNA
<213> Ricinus communis

<400> 126

```
atggttgcta ctgcggctgc tgctacttcc tctttctttc cagttccttc tcaatctgcg      60
gatgctaatt tcgataaggc acctgcaagc ttaggtggaa tcaaattaaa atctacctct     120
tgctctcggg gtttacaggt taaggcaaat gcgcaagccc ctcccaagat aaatggatcc     180
tcggtaggat tcacaacatc tgtggaaact gtgaagaatg acggtgacat gccattacca     240
ccacccccta ggacttttat caaccaatta cctgattgga gcatgcttct tgctgctatt     300
acaactatct tttggctgc tgaaaagcag tggatgatgc ttgactggaa accaaggcgg     360
cctgacatgc ttatcgaccc gtttggtata ggtagaattg ttcaggatgg tcttattttt     420
cgccagaact tctccataag atcatatgaa attggtgctg atcgtacagc atccatagag     480
acattaatga atcatttaca agaaacggcc ctcaatcatg ttaagactgc tggacttctt     540
ggggatggat ttggttcaac cccagagatg agcaaaagga acctcatatg ggtggttact     600
cggatgcagg ttctggtgga tcgttaccca acatggggtg atgttgttca agtagatact     660
tgggtgagta aatcaggaaa gaatggcatg cggcgtgatt ggtgcgtccg tgatagtaga     720
actggtgaaa ctttaacgag agcatccagc gtgtgggtga tgatgaataa actgactagg     780
aggttatcta aaattcccga agaagttcga ggagaaatag agccttattt tctgaattct     840
gatcctattg tggatgagga tagcagaaaa ctgccaaagc ttgatgatag caatgcggac     900
tatgtccgca aaggtctaac tcctagatgg agtgatctag atatcaacca acatgttaac     960
aatgtgaaat acattggctg gattcttgag agtgctccac tgccaatact ggagagtcat    1020
gaactctctg ccattactct ggagtatagg agggagtgcg ggagggacag tgtactgcag    1080
tctctgactg ctgtatccgg taatggtatt ggaaatttgg gaaatgctgg tgatattgag    1140
tgccagcact tgcttcgact tgaggatggg gctgagatag tgaggggaag gaccgagtgg    1200
aggccaaagt acagcagcaa ctttggtatt atgggtcaga ttccagtcga aagtgcttaa    1260
```

<210> 127

<211> 419
<212> PRT
<213> Ricinus communis

<400> 127
Met Val Ala Thr Ala Ala Ala Ala Thr Ser Ser Phe Phe Pro Val Pro
1 5 10 15
Ser Gln Ser Ala Asp Ala Asn Phe Asp Lys Ala Pro Ala Ser Leu Gly
20 25 30
Gly Ile Lys Leu Lys Ser Thr Ser Cys Ser Arg Gly Leu Gln Val Lys
35 40 45
Ala Asn Ala Gln Ala Pro Pro Lys Ile Asn Gly Ser Ser Val Gly Phe
50 55 60
Thr Thr Ser Val Glu Thr Val Lys Asn Asp Gly Asp Met Pro Leu Pro
65 70 75 80
Pro Pro Pro Arg Thr Phe Ile Asn Gln Leu Pro Asp Trp Ser Met Leu
85 90 95
Leu Ala Ala Ile Thr Thr Ile Phe Leu Ala Ala Glu Lys Gln Trp Met
100 105 110
Met Leu Asp Trp Lys Pro Arg Arg Pro Asp Met Leu Ile Asp Pro Phe
115 120 125
Gly Ile Gly Arg Ile Val Gln Asp Gly Leu Ile Phe Arg Gln Asn Phe
130 135 140
Ser Ile Arg Ser Tyr Glu Ile Gly Ala Asp Arg Thr Ala Ser Ile Glu
145 150 155 160
Thr Leu Met Asn His Leu Gln Glu Thr Ala Leu Asn His Val Lys Thr
165 170 175
Ala Gly Leu Leu Gly Asp Gly Phe Gly Ser Thr Pro Glu Met Ser Lys
180 185 190
Arg Asn Leu Ile Trp Val Val Thr Arg Met Gln Val Leu Val Asp Arg
195 200 205
Tyr Pro Thr Trp Gly Asp Val Val Gln Val Asp Thr Trp Val Ser Lys
210 215 220
Ser Gly Lys Asn Gly Met Arg Arg Asp Trp Cys Val Arg Asp Ser Arg
225 230 235 240
Thr Gly Glu Thr Leu Thr Arg Ala Ser Ser Val Trp Val Met Met Asn
245 250 255
Lys Leu Thr Arg Arg Leu Ser Lys Ile Pro Glu Glu Val Arg Gly Glu
260 265 270
Ile Glu Pro Tyr Phe Leu Asn Ser Asp Pro Ile Val Asp Glu Asp Ser
275 280 285
Arg Lys Leu Pro Lys Leu Asp Asp Ser Asn Ala Asp Tyr Val Arg Lys
290 295 300
Gly Leu Thr Pro Arg Trp Ser Asp Leu Asp Ile Asn Gln His Val Asn
305 310 315 320
Asn Val Lys Tyr Ile Gly Trp Ile Leu Glu Ser Ala Pro Leu Pro Ile
325 330 335
Leu Glu Ser His Glu Leu Ser Ala Ile Thr Leu Glu Tyr Arg Arg Glu
340 345 350
Cys Gly Arg Asp Ser Val Leu Gln Ser Leu Thr Ala Val Ser Gly Asn
355 360 365
Gly Ile Gly Asn Leu Gly Asn Ala Gly Asp Ile Glu Cys Gln His Leu
370 375 380
Leu Arg Leu Glu Asp Gly Ala Glu Ile Val Arg Gly Arg Thr Glu Trp
385 390 395 400
Arg Pro Lys Tyr Ser Ser Asn Phe Gly Ile Met Gly Gln Ile Pro Val
405 410 415
Glu Ser Ala

<210> 128
<211> 1263
<212> DNA

<213> Solanum tuberosum

<400> 128
atgatggcca ctgctgctac ttgtgcattc ttccctgctg ctaatccacc tcctgactct 60
ggagctaaat cgtctggaaa tttaggagga agtcttcctg gaagtataga tacacggggg 120
cttaatgtta agaagccttc ttttgggagc ctacaagcta aggccaatgc acaagcacca 180
cctaaggtga atggaacaaa ggtaggcgtt atggatggct tcaaaaatga cgatgaggtg 240
atttcttcac atcacccaag gacttttatc aaccagttac ctgattggag catgctcctc 300
gccgccatca cgacaatttt tttagctgct gagaagcaat ggatgatgct tgattggaag 360
cctaagcgtc ctgatatgct cgctgatcca tttggattag gaaaaattgt gcaggatggc 420
tttgttttcc gtcaaaattt cagcatcagg tcttatgaaa taggggctga taggactgcg 480
tctatagaaa caatgatgaa tcatttacag gaaactgctc ttaaccatgt caagagtgct 540
ggactcatgc atggtgggtt cggatcaact ccagagatgt ccaagagaaa tttgatctgg 600
gtcgttacta aaatgcaggt tgtggtggac cgttatccta cttggggtga tgttgttcaa 660
gtagacactt gggtagctgc atcggggaaa aatggtatgc gcagagattg gctcctccgc 720
gatagtaata caggggatat attgatgaga gcttccagcc aatgggttat gatgaataag 780
gagacgagga gattatctaa aataccagat gaggctcggg ctgaaattga aggttatttt 840
gttgattcac ctcctgttat tgatgaggac agcaggaagt taccaaaact tgatgagaca 900
acagcagact acactcgaac tggtttaact ccaagatgga gtgatttaga tgttaaccag 960
catgttaata atgtcaagta cattggctgg attcttgaga gtgcacccat gcaaatacta 1020
gagggttgtg agcttgctgc catgactttg gagtaccgca gggagtgcag aagggacagt 1080
gtgcttcagt ctcttacctc tgtacttgac aaaggagtcg gtgacttcac cgactttggg 1140
aatgttgagt gtcaacacgt ccttcgactt gaaaatggcg gagaggttgt taagggacga 1200
actgagtgga ggccgaaact tgtcaatgga attgggaccc taggcggatt cgacttcgcc 1260
tga 1263

<210> 129
<211> 420
<212> PRT
<213> Solanum tuberosum

<400> 129
Met Met Ala Thr Ala Ala Thr Cys Ala Phe Phe Pro Ala Ala Asn Pro
1 5 10 15
Pro Pro Asp Ser Gly Ala Lys Ser Ser Gly Asn Leu Gly Gly Ser Leu
20 25 30
Pro Gly Ser Ile Asp Thr Arg Gly Leu Asn Val Lys Lys Pro Ser Phe
35 40 45
Gly Ser Leu Gln Ala Lys Ala Asn Ala Gln Ala Pro Pro Lys Val Asn
50 55 60
Gly Thr Lys Val Gly Val Met Asp Gly Phe Lys Asn Asp Asp Glu Val
65 70 75 80
Ile Ser Ser His His Pro Arg Thr Phe Ile Asn Gln Leu Pro Asp Trp
85 90 95
Ser Met Leu Leu Ala Ala Ile Thr Thr Ile Phe Leu Ala Ala Glu Lys
100 105 110
Gln Trp Met Met Leu Asp Trp Lys Pro Lys Arg Pro Asp Met Leu Ala
115 120 125
Asp Pro Phe Gly Leu Gly Lys Ile Val Gln Asp Gly Phe Val Phe Arg
130 135 140
Gln Asn Phe Ser Ile Arg Ser Tyr Glu Ile Gly Ala Asp Arg Thr Ala
145 150 155 160
Ser Ile Glu Thr Met Met Asn His Leu Gln Glu Thr Ala Leu Asn His
165 170 175
Val Lys Ser Ala Gly Leu Met His Gly Gly Phe Gly Ser Thr Pro Glu
180 185 190
Met Ser Lys Arg Asn Leu Ile Trp Val Val Thr Lys Met Gln Val Val
195 200 205
Val Asp Arg Tyr Pro Thr Trp Gly Asp Val Val Gln Val Asp Thr Trp
210 215 220
Val Ala Ala Ser Gly Lys Asn Gly Met Arg Arg Asp Trp Leu Leu Arg
225 230 235 240
Asp Ser Asn Thr Gly Asp Ile Leu Met Arg Ala Ser Ser Gln Trp Val

```
                245                 250                 255
Met Met Asn Lys Glu Thr Arg Arg Leu Ser Lys Ile Pro Asp Glu Ala
            260                 265                 270
Arg Ala Glu Ile Glu Gly Tyr Phe Val Asp Ser Pro Pro Val Ile Asp
        275                 280                 285
Glu Asp Ser Arg Lys Leu Pro Lys Leu Asp Glu Thr Thr Ala Asp Tyr
    290                 295                 300
Thr Arg Thr Gly Leu Thr Pro Arg Trp Ser Asp Leu Asp Val Asn Gln
305                 310                 315                 320
His Val Asn Asn Val Lys Tyr Ile Gly Trp Ile Leu Glu Ser Ala Pro
                325                 330                 335
Met Gln Ile Leu Glu Gly Cys Glu Leu Ala Ala Met Thr Leu Glu Tyr
            340                 345                 350
Arg Arg Glu Cys Arg Arg Asp Ser Val Leu Gln Ser Leu Thr Ser Val
        355                 360                 365
Leu Asp Lys Gly Val Gly Asp Phe Thr Asp Phe Gly Asn Val Glu Cys
    370                 375                 380
Gln His Val Leu Arg Leu Glu Asn Gly Gly Glu Val Val Lys Gly Arg
385                 390                 395                 400
Thr Glu Trp Arg Pro Lys Leu Val Asn Gly Ile Gly Thr Leu Gly Gly
                405                 410                 415
Phe Asp Phe Ala
            420

<210>  130
<211>  1254
<212>  DNA
<213>  Tagetes erecta

<400>  130
atggttgcta cggctgcaac tgcatcgtta tttccggttt cttcaccaca acctgactct      60
ggtgctaaga attctggcaa tcacaaaggc ggattgggta gtgttgactt acgtggaatt     120
aagtcaaagt caacgtcttc taatggtttg caagttaaga cgaatgcaca agctcctgcg     180
aaggtgaatg ggaccagggt aggtgttatg gatggactga aaattgatga cagttcatca     240
tcgggtgccc caagaacatt tattaaccaa ctgcctgatt ggagcatgct tcttgctgct     300
attactacta ttttcttggc tgctgaaaag caatggatga tgctggattg gaagactaaa     360
cgtccggaca tgcttgctga tcttgatcct tttggtttcg ggcgaattgt tgaggatgga     420
tttgtatttc gtcaaaactt ttcaattaga tcatatgaaa taggggcgga tcgaactgcg     480
tcggttgaaa cgttgatgaa tcatttgcag gaaacggccc ttaatcatgt aaaaaatgct     540
ggactcctcg gtgatggctt tggctcaaca cctgaaatgt ctaaaaggaa tctgttctgg     600
gtggtaacta agatgcaagt gctagtagac cgttatccaa cttggggtga cgtggttcaa     660
gtagatactt gggtagctgc ttctgggaaa aatggcatgc gtcgtgattg gttgattcgt     720
gattgcaaaa cgggtcagat actaacaaga gcctcaagta attgggttat gatgaataaa     780
gttacaagga ggttatcaaa aatgcccgat gaagttcggg ctgaaattga gccgtatttt     840
gttgacacgc ctcctgtggt tgatgatgat gatagaaaat taccaaaact tgatgagaac     900
actgctgacc atgttcgtaa tggtttaact ccaaagtgga gtgatttgga tgtcaatcag     960
catgtcaaca atgtgaagta tgttggctgg attcttgaga gtgcaccaca gcatgtggta    1020
gagaactatg agcttgcaag cctcaccctt gagtaccgcc gtgagtgtat gaaagacagc    1080
gtgctgcagt cactcacttc cttgctggcg ggtggtgaga aggcggattc tgatgatgtg    1140
gactgtcaac acctgcttcg actagaaggt ggcggtgaga ttgtgaaggg aaggaccaaa    1200
tggaggccca aatatgtgaa acagattcaa gaacatcaat catttcccta ctga          1254

<210>  131
<211>  417
<212>  PRT
<213>  Tagetes erecta

<400>  131
Met Val Ala Thr Ala Ala Thr Ala Ser Leu Phe Pro Val Ser Ser Pro
1               5                   10                  15
Gln Pro Asp Ser Gly Ala Lys Asn Ser Gly Asn His Lys Gly Gly Leu
            20                  25                  30
Gly Ser Val Asp Leu Arg Gly Ile Lys Ser Lys Ser Thr Ser Ser Asn
```

35 40 45
Gly Leu Gln Val Lys Thr Asn Ala Gln Ala Pro Ala Lys Val Asn Gly
50 55 60
Thr Arg Val Gly Val Met Asp Gly Leu Lys Ile Asp Asp Ser Ser Ser
65 70 75 80
Ser Gly Ala Pro Arg Thr Phe Ile Asn Gln Leu Pro Asp Trp Ser Met
85 90 95
Leu Leu Ala Ala Ile Thr Thr Ile Phe Leu Ala Ala Glu Lys Gln Trp
100 105 110
Met Met Leu Asp Trp Lys Thr Lys Arg Pro Asp Met Leu Ala Asp Leu
115 120 125
Asp Pro Phe Gly Phe Gly Arg Ile Val Glu Asp Gly Phe Val Phe Arg
130 135 140
Gln Asn Phe Ser Ile Arg Ser Tyr Glu Ile Gly Ala Asp Arg Thr Ala
145 150 155 160
Ser Val Glu Thr Leu Met Asn His Leu Gln Glu Thr Ala Leu Asn His
165 170 175
Val Lys Asn Ala Gly Leu Leu Gly Asp Gly Phe Gly Ser Thr Pro Glu
180 185 190
Met Ser Lys Arg Asn Leu Phe Trp Val Val Thr Lys Met Gln Val Leu
195 200 205
Val Asp Arg Tyr Pro Thr Trp Gly Asp Val Val Gln Val Asp Thr Trp
210 215 220
Val Ala Ala Ser Gly Lys Asn Gly Met Arg Arg Asp Trp Leu Ile Arg
225 230 235 240
Asp Cys Lys Thr Gly Gln Ile Leu Thr Arg Ala Ser Ser Asn Trp Val
245 250 255
Met Met Asn Lys Val Thr Arg Arg Leu Ser Lys Met Pro Asp Glu Val
260 265 270
Arg Ala Glu Ile Glu Pro Tyr Phe Val Asp Thr Pro Pro Val Val Asp
275 280 285
Asp Asp Asp Arg Lys Leu Pro Lys Leu Asp Glu Asn Thr Ala Asp His
290 295 300
Val Arg Asn Gly Leu Thr Pro Lys Trp Ser Asp Leu Asp Val Asn Gln
305 310 315 320
His Val Asn Asn Val Lys Tyr Val Gly Trp Ile Leu Glu Ser Ala Pro
325 330 335
Gln His Val Val Glu Asn Tyr Glu Leu Ala Ser Leu Thr Leu Glu Tyr
340 345 350
Arg Arg Glu Cys Met Lys Asp Ser Val Leu Gln Ser Leu Thr Ser Leu
355 360 365
Leu Ala Gly Gly Glu Lys Ala Asp Ser Asp Asp Val Asp Cys Gln His
370 375 380
Leu Leu Arg Leu Glu Gly Gly Gly Glu Ile Val Lys Gly Arg Thr Lys
385 390 395 400
Trp Arg Pro Lys Tyr Val Lys Gln Ile Gln Glu His Gln Ser Phe Pro
405 410 415
Tyr

<210> 132
<211> 1266
<212> DNA
<213> Vitis vinifera

<400> 132
atggttgcca ctgcagccac ttctgcattc tttgcagttg cttctccatc ttctgatcca 60
gatgccaaac cttccaccaa gccgggggtt gggtctgcaa ttttgagggg aatcaagtca 120
agaaatgctc cttcaggcag tttgcaagtt aaggcaaatg cccaagcccc tcctaagata 180
aatggtacca cagttggtta tacctcctcg gcggaaggcg tgaagattga ggatgacatg 240
tcgtcgcctc cacctaggac tttcatcaac caattgccag actggagcat gcttcttgct 300
gctattacaa ccatcttctt ggcagctgag aagcagtgga tgatgcttga ctggaaacca 360
aggaggtctg acatgctaat cgacccattt ggcttaggga aaattgtcca agatggtctt 420

```
gttttcaggc aaaacttctc gattagatca tatgaaatag gtgctgatcg aaccgcatcc    480
atagaaacgt tgatgaatca tttacaggaa actgcactta accatgttag gactgctggt    540
cttctgggtg atggttttgg ttcaacgcca gagatgagca taaggaacct aatatgggtg    600
gtcactcgaa tgcaggttgt ggtagatcgg taccctactt ggggtgatgt tgttcaagtg    660
gatacttggg tatgtgcatc tgggaagaat ggcatgcgtc gtgattggat aatccgtgat    720
tgcaaaactg ggaaactct aaccagagcc tccagtgtgt gggtgatgat gaataagcag    780
accaggagat tatcaaaaat tccagatgca gttcgagctg aaatagagcc ttattttatg    840
gattctgctc ctattgtgga tgaggatggc agaaaactgc ccaaacttga tgacagcact    900
gcggattata tccgcacagg actaactcct agatggagtg atttagatgt caatcagcat    960
gttaacaatg ttaagtacat cggttggatc cttgagagtg ctccactgcc aatcttggag   1020
agtcacgagc tttcttccat gactctggag tacaggaggg agtgtggaag ggacagtgtg   1080
ctgcagtccc tcactgctgt ctgcggaact ggtgttggta atttgctgga ttgtggaaat   1140
gttgagtgcc agcaccttct tcgacttgag gaaggagctg agattgttaa gggaaggact   1200
gagtggaggc caaagtatgc ccacagcatg gggggtgtgg gccagatccc agcagaaagt   1260
gcttga                                                              1266
```

<210> 133
<211> 421
<212> PRT
<213> Vitis vinifera

<400> 133

```
Met Val Ala Thr Ala Ala Thr Ser Ala Phe Phe Ala Val Ala Ser Pro
1               5                   10                  15
Ser Ser Asp Pro Asp Ala Lys Pro Ser Thr Lys Pro Gly Val Gly Ser
            20                  25                  30
Ala Ile Leu Arg Gly Ile Lys Ser Arg Asn Ala Pro Ser Gly Ser Leu
        35                  40                  45
Gln Val Lys Ala Asn Ala Gln Ala Pro Pro Lys Ile Asn Gly Thr Thr
    50                  55                  60
Val Gly Tyr Thr Ser Ser Ala Glu Gly Val Lys Ile Glu Asp Asp Met
65                  70                  75                  80
Ser Ser Pro Pro Pro Arg Thr Phe Ile Asn Gln Leu Pro Asp Trp Ser
                85                  90                  95
Met Leu Leu Ala Ala Ile Thr Thr Ile Phe Leu Ala Ala Glu Lys Gln
            100                 105                 110
Trp Met Met Leu Asp Trp Lys Pro Arg Arg Ser Asp Met Leu Ile Asp
        115                 120                 125
Pro Phe Gly Leu Gly Lys Ile Val Gln Asp Gly Leu Val Phe Arg Gln
    130                 135                 140
Asn Phe Ser Ile Arg Ser Tyr Glu Ile Gly Ala Asp Arg Thr Ala Ser
145                 150                 155                 160
Ile Glu Thr Leu Met Asn His Leu Gln Glu Thr Ala Leu Asn His Val
                165                 170                 175
Arg Thr Ala Gly Leu Leu Gly Asp Gly Phe Gly Ser Thr Pro Glu Met
            180                 185                 190
Ser Ile Arg Asn Leu Ile Trp Val Val Thr Arg Met Gln Val Val Val
        195                 200                 205
Asp Arg Tyr Pro Thr Trp Gly Asp Val Val Gln Val Asp Thr Trp Val
    210                 215                 220
Cys Ala Ser Gly Lys Asn Gly Met Arg Arg Asp Trp Ile Ile Arg Asp
225                 230                 235                 240
Cys Lys Thr Gly Glu Thr Leu Thr Arg Ala Ser Ser Val Trp Val Met
                245                 250                 255
Met Asn Lys Gln Thr Arg Arg Leu Ser Lys Ile Pro Asp Ala Val Arg
            260                 265                 270
Ala Glu Ile Glu Pro Tyr Phe Met Asp Ser Ala Pro Ile Val Asp Glu
        275                 280                 285
Asp Gly Arg Lys Leu Pro Lys Leu Asp Asp Ser Thr Ala Asp Tyr Ile
    290                 295                 300
Arg Thr Gly Leu Thr Pro Arg Trp Ser Asp Leu Asp Val Asn Gln His
305                 310                 315                 320
Val Asn Asn Val Lys Tyr Ile Gly Trp Ile Leu Glu Ser Ala Pro Leu
```

```
                325                 330                 335
Pro Ile Leu Glu Ser His Glu Leu Ser Ser Met Thr Leu Glu Tyr Arg
            340                 345                 350
Arg Glu Cys Gly Arg Asp Ser Val Leu Gln Ser Leu Thr Ala Val Cys
        355                 360                 365
Gly Thr Gly Val Gly Asn Leu Leu Asp Cys Gly Asn Val Glu Cys Gln
    370                 375                 380
His Leu Leu Arg Leu Glu Glu Gly Ala Glu Ile Val Lys Gly Arg Thr
385                 390                 395                 400
Glu Trp Arg Pro Lys Tyr Ala His Ser Met Gly Gly Val Gly Gln Ile
                405                 410                 415
Pro Ala Glu Ser Ala
            420

<210>  134
<211>  1281
<212>  DNA
<213>  Zea mays

<400>  134
atggctggct cccttgctgc ctcagccttc ttccctggcc caggggcgtc tccagcagca      60
tccgcgaaga acttggctgg tgaagtaccg gatagtttga gcgtccgtgg tattgtcgca     120
aagcctaatg ccaattctgg gaacatgcaa gtgaaggctc aagcacaaac ccttcccaag     180
gttaatggca ccaaggttaa cctcaagaat gcaagctcag acacagagga ggcgataccc     240
tacactgctc ccaagacatt ctacaaccaa ctgccagatt ggagcatgct tcttgcggct     300
gtcactacca tcttcctggc agcagagaag cagtggacac tgcttgactg gaagccgaag     360
aaacccgaca tgcttgttga tacatttggt tttggtggga tcatccagga tgggatggtg     420
tttaggcaaa acttcattat tcggtcctat gagattggtg ccgatcgtac tgcttctata     480
gagacattaa tgaatcactt acaggaaaca gctcttaacc atgtgaagac agctggcctt     540
cttggagatg gttttggcgc cacgccagag atgagcaaac gaaacttgat ccacgaggtc     600
agcaaaattc agcttcttgt tgagaagtac cccttgtggg aagacacggt tcaagtggac     660
acgtgggtag ctgccgctgg gaaaaatggc atgcgtcgag actggcatgt cctcgactgc     720
aagtctggat gtacgatctt gagagctaca agtgtttggg tgatgatgaa taagaacact     780
agaaggtttt caaaaatgcc ggacgaagta agggctgaga taggcccgta tttcaacgcc     840
cgcgcagcca taacagatga gcagagcgag aaactggcta agccagggag cactgctggt     900
ggcgatgcta tgaagcagtt catgagaaag ggctcactc ctaggtggtg gggtgacctt      960
gatgtcaacc agcacgtgaa taacgtcaag tacatcggtt ggattcttga gagtgctccg    1020
atcgcgatcc tggagaagca cgagctcgca agcatgacgc tggattacag gaaggagtgc    1080
ggacgcgaca gcgtgctgca gtcgctcacc accgtcgcgg gtgaatgcgt agacggcgac    1140
acagactcca ccatccagtg cgaccacctg ctccagctgg aaacaggagc cgatattgtg    1200
aaggcgcaca cggagtggcg cccgaagcgg gcgcatggtg aggggacccc catggggggt    1260
ttcccggcgg agagcgcgtg a                                               1281

<210>  135
<211>  426
<212>  PRT
<213>  Zea mays

<400>  135
Met Ala Gly Ser Leu Ala Ala Ser Ala Phe Phe Pro Gly Pro Gly Ala
1               5                   10                  15
Ser Pro Ala Ala Ser Ala Lys Asn Leu Ala Gly Glu Val Pro Asp Ser
            20                  25                  30
Leu Ser Val Arg Gly Ile Val Ala Lys Pro Asn Ala Asn Ser Gly Asn
        35                  40                  45
Met Gln Val Lys Ala Gln Ala Gln Thr Leu Pro Lys Val Asn Gly Thr
    50                  55                  60
Lys Val Asn Leu Lys Asn Ala Ser Ser Asp Thr Glu Glu Ala Ile Pro
65                  70                  75                  80
Tyr Thr Ala Pro Lys Thr Phe Tyr Asn Gln Leu Pro Asp Trp Ser Met
                85                  90                  95
Leu Leu Ala Ala Val Thr Thr Ile Phe Leu Ala Ala Glu Lys Gln Trp
            100                 105                 110
```

Thr Leu Leu Asp Trp Lys Pro Lys Lys Pro Asp Met Leu Val Asp Thr
115 120 125
Phe Gly Phe Gly Gly Ile Ile Gln Asp Gly Met Val Phe Arg Gln Asn
130 135 140
Phe Ile Ile Arg Ser Tyr Glu Ile Gly Ala Asp Arg Thr Ala Ser Ile
145 150 155 160
Glu Thr Leu Met Asn His Leu Gln Glu Thr Ala Leu Asn His Val Lys
165 170 175
Thr Ala Gly Leu Leu Gly Asp Gly Phe Gly Ala Thr Pro Glu Met Ser
180 185 190
Lys Arg Asn Leu Ile His Glu Val Ser Lys Ile Gln Leu Leu Val Glu
195 200 205
Lys Tyr Pro Leu Trp Glu Asp Thr Val Gln Val Asp Thr Trp Val Ala
210 215 220
Ala Ala Gly Lys Asn Gly Met Arg Arg Asp Trp His Val Leu Asp Cys
225 230 235 240
Lys Ser Gly Cys Thr Ile Leu Arg Ala Thr Ser Val Trp Val Met Met
245 250 255
Asn Lys Asn Thr Arg Arg Phe Ser Lys Met Pro Asp Glu Val Arg Ala
260 265 270
Glu Ile Gly Pro Tyr Phe Asn Ala Arg Ala Ala Ile Thr Asp Glu Gln
275 280 285
Ser Glu Lys Leu Ala Lys Pro Gly Ser Thr Ala Gly Gly Asp Ala Met
290 295 300
Lys Gln Phe Met Arg Lys Gly Leu Thr Pro Arg Trp Trp Gly Asp Leu
305 310 315 320
Asp Val Asn Gln His Val Asn Asn Val Lys Tyr Ile Gly Trp Ile Leu
325 330 335
Glu Ser Ala Pro Ile Ala Ile Leu Glu Lys His Glu Leu Ala Ser Met
340 345 350
Thr Leu Asp Tyr Arg Lys Glu Cys Gly Arg Asp Ser Val Leu Gln Ser
355 360 365
Leu Thr Thr Val Ala Gly Glu Cys Val Asp Gly Asp Thr Asp Ser Thr
370 375 380
Ile Gln Cys Asp His Leu Leu Gln Leu Glu Thr Gly Ala Asp Ile Val
385 390 395 400
Lys Ala His Thr Glu Trp Arg Pro Lys Arg Ala His Gly Glu Gly Thr
405 410 415
Pro Met Gly Gly Phe Pro Ala Glu Ser Ala
420 425

<210> 136
<211> 1257
<212> DNA
<213> Zea mays

<400> 136
atggccgcct ccatcgcggc ctcgtccttc tttccagggt caccggcgcc ggccgctcct 60
aagaacggcc ttggggagcg cccagagagc ctggacgtcc gcggcgttgc ggcgaagccg 120
ggagcctcgt ctagtgccgt gagggcgagc aagacgcgcg cccacgctgc ggtccccaag 180
atgaacggtg gggcaagtc cgcggtggcg gatggggagc acgaaaccgt accttcttcg 240
gtgccgaaga ctttctacaa ccagcttccc gactggagca tgctccttgc ggccatcacc 300
accatcttct tggccgcaga gaagcagtgg acgatgcttg actggaagcc taggaggcct 360
gacatgctca ctgacacgtt tgggtttggc cggatcatac atgatgggct catgttcagg 420
cagaacttct ccattaggtc ctatgagatt ggggcagata ggacggcatc tatagagacg 480
ctgatgaacc atttgcagga aacggcactc aatcatgtga agaccgctgg gctgctaggt 540
gatggatttg gctccacacc agagatgagt aaacgaaact tgttctgggt ggttagccaa 600
atgcaggcca tcatcgagcg ttatccatgc tggggtgata ctgttgaagt agatacatgg 660
gttagtgcta atggtaaaaa tggaatgcgt agggattggc atatacgtga ttctatgaca 720
ggccacacaa tactgaaggc gacaagtaaa tgggttatga tgaacaaact cactaggaag 780
cttgcaagaa ttccagatga agtgcggact gaaatagagc catactttgt tgggcgttct 840
gctattgttg atgaagacaa ccgcaagctt ccaaaactgc cagaggtcaa aagcacttct 900
gcagctaaat atgtgaggac aggcctgact cctcgttggg ctgatcttga tataaaccag 960

```
catgtcaata atgttaaata cattgcgtgg attcttgaga gtgcaccgat tactattttt   1020
gagaatcatg agctggccag cattgtgctg gattacaaaa gggagtgtgt ccgcgatagt   1080
gtgctgcagt cacacacctc tgtccatgag gattgcaaca ttgagtctgg agaaacaacc   1140
ttgcactgtg agcatgtgct gagccttgaa tcaggtccga ccatagtgaa ggcccggacc   1200
atgtggaggc ctaagggaac caaggcccaa gaaacagcgg ttccatcttc attctga      1257
```

<210> 137
<211> 418
<212> PRT
<213> Zea mays

<400> 137

```
Met Ala Ala Ser Ile Ala Ala Ser Ser Phe Phe Pro Gly Ser Pro Ala
1               5                   10                  15
Pro Ala Ala Pro Lys Asn Gly Leu Gly Glu Arg Pro Glu Ser Leu Asp
            20                  25                  30
Val Arg Gly Val Ala Ala Lys Pro Gly Ala Ser Ser Ser Ala Val Arg
        35                  40                  45
Ala Ser Lys Thr Arg Ala His Ala Ala Val Pro Lys Met Asn Gly Gly
    50                  55                  60
Gly Lys Ser Ala Val Ala Asp Gly Glu His Glu Thr Val Pro Ser Ser
65                  70                  75                  80
Val Pro Lys Thr Phe Tyr Asn Gln Leu Pro Asp Trp Ser Met Leu Leu
                85                  90                  95
Ala Ala Ile Thr Thr Ile Phe Leu Ala Ala Glu Lys Gln Trp Thr Met
            100                 105                 110
Leu Asp Trp Lys Pro Arg Arg Pro Asp Met Leu Thr Asp Thr Phe Gly
        115                 120                 125
Phe Gly Arg Ile Ile His Asp Gly Leu Met Phe Arg Gln Asn Phe Ser
    130                 135                 140
Ile Arg Ser Tyr Glu Ile Gly Ala Asp Arg Thr Ala Ser Ile Glu Thr
145                 150                 155                 160
Leu Met Asn His Leu Gln Glu Thr Ala Leu Asn His Val Lys Thr Ala
                165                 170                 175
Gly Leu Leu Gly Asp Gly Phe Gly Ser Thr Pro Glu Met Ser Lys Arg
            180                 185                 190
Asn Leu Phe Trp Val Val Ser Gln Met Gln Ala Ile Ile Glu Arg Tyr
        195                 200                 205
Pro Cys Trp Gly Asp Thr Val Glu Val Asp Thr Trp Val Ser Ala Asn
    210                 215                 220
Gly Lys Asn Gly Met Arg Arg Asp Trp His Ile Arg Asp Ser Met Thr
225                 230                 235                 240
Gly His Thr Ile Leu Lys Ala Thr Ser Lys Trp Val Met Met Asn Lys
                245                 250                 255
Leu Thr Arg Lys Leu Ala Arg Ile Pro Asp Glu Val Arg Thr Glu Ile
            260                 265                 270
Glu Pro Tyr Phe Val Gly Arg Ser Ala Ile Val Asp Glu Asp Asn Arg
        275                 280                 285
Lys Leu Pro Lys Leu Pro Glu Gly Gln Ser Thr Ser Ala Ala Lys Tyr
    290                 295                 300
Val Arg Thr Gly Leu Thr Pro Arg Trp Ala Asp Leu Asp Ile Asn Gln
305                 310                 315                 320
His Val Asn Asn Val Lys Tyr Ile Ala Trp Ile Leu Glu Ser Ala Pro
                325                 330                 335
Ile Thr Ile Phe Glu Asn His Glu Leu Ala Ser Ile Val Leu Asp Tyr
            340                 345                 350
Lys Arg Glu Cys Val Arg Asp Ser Val Leu Gln Ser His Thr Ser Val
        355                 360                 365
His Glu Asp Cys Asn Ile Glu Ser Gly Glu Thr Thr Leu His Cys Glu
    370                 375                 380
His Val Leu Ser Leu Glu Ser Gly Pro Thr Ile Val Lys Ala Arg Thr
385                 390                 395                 400
Met Trp Arg Pro Lys Gly Thr Lys Ala Gln Glu Thr Ala Val Pro Ser
```

```
                405                 410                 415
Ser Phe


<210>  138
<211>  1257
<212>  DNA
<213>  Populus trichocarpa

<400>  138
atggttgccg ctgcagctgc ttcatcattt ttcccagttc cttcgccatc tggagatgcc      60
aaggcctcca agtttggtag tgtgtctgca agtttgggag gaatcaaaac gaaatctgct     120
tcctctgggg ctttgcaagt taacacaaat gcccaagctc ctccaaagat aaatggccct     180
ccagttggct tgacagcatc agtggaaact ctgaagaatg aggatgttgt gtcgtcaccg     240
gcacctcgga cgttcatcaa ccaattacct gattggagca tgcttcttgc tgcaattaca     300
accatgtttt tggcagcaga gaagcagtgg atgatgcttg attggaaacc aaagcgacct     360
gatatgctta ttgacccctt tggtattggg agaattgtcc aagatggtct tgtcttccgc     420
cagaatttct caattaggtc atatgaaatt ggtgcagatc gtacagcatc tatagagacg     480
ttgatgaacc atttacaaga aactgcactt aatcatgtta agactgctgg gctccttggc     540
gatggatttg gtgcaacccc agagatgtcc aaaaggaacc tgatatgggt ggtaactcgt     600
atgcagattc tggtagatcg ttatcctaca tggggtgatg ttgttcaagt agatacttgg     660
gtgagtgcat cgggaaagaa tggcatgcgc cgtgattggc ttctccgtga tgctaaaact     720
ggtgaaacgt tgaccagagc ctccagtgtg tgggtgatga tgaataaagt gacaaggagg     780
ttatccaaaa ttcctgaaga agttcgaggg gaaatagagc ctcatttttct gacttctgat     840
cctgttgtga atgaggacag cagaaaactt ccaaaaattg atgacaatac agcggactat     900
atctgcgaaa gtctaactcc tagatggaat gatttagatg tcaaccaaca tgttaacaat     960
gtgaagtaca taggctggat ccttgagagc gctcctccac caatcatgga gagtcatgag    1020
cttgctgcca ttactttgga gtacaggagg gagtgtggca gggacagcgt gctgcagtcc    1080
ttgactgctg tatctgacac tggcattgga aatttaggca gccctggtga agttgagttc    1140
caacacttgc tccggtttga ggagggtgct gagattgtga ggggaaggac tgagtggaga    1200
cccaaacatg ccgacaattt tggtatcatg ggtcagatcc cagctgtgag cgcttaa       1257


<210>  139
<211>  418
<212>  PRT
<213>  Populus trichocarpa

<400>  139
Met Val Ala Ala Ala Ala Ala Ser Ser Phe Phe Pro Val Pro Ser Pro
1               5                   10                  15
Ser Gly Asp Ala Lys Ala Ser Lys Phe Gly Ser Val Ser Ala Ser Leu
            20                  25                  30
Gly Gly Ile Lys Thr Lys Ser Ala Ser Ser Gly Ala Leu Gln Val Asn
        35                  40                  45
Thr Asn Ala Gln Ala Pro Pro Lys Ile Asn Gly Pro Pro Val Gly Leu
    50                  55                  60
Thr Ala Ser Val Glu Thr Leu Lys Asn Glu Asp Val Val Ser Ser Pro
65                  70                  75                  80
Ala Pro Arg Thr Phe Ile Asn Gln Leu Pro Asp Trp Ser Met Leu Leu
                85                  90                  95
Ala Ala Ile Thr Thr Met Phe Leu Ala Ala Glu Lys Gln Trp Met Met
            100                 105                 110
Leu Asp Trp Lys Pro Lys Arg Pro Asp Met Leu Ile Asp Pro Phe Gly
        115                 120                 125
Ile Gly Arg Ile Val Gln Asp Gly Leu Val Phe Arg Gln Asn Phe Ser
    130                 135                 140
Ile Arg Ser Tyr Glu Ile Gly Ala Asp Arg Thr Ala Ser Ile Glu Thr
145                 150                 155                 160
Leu Met Asn His Leu Gln Glu Thr Ala Leu Asn His Val Lys Thr Ala
                165                 170                 175
Gly Leu Leu Gly Asp Gly Phe Gly Ala Thr Pro Glu Met Ser Lys Arg
            180                 185                 190
Asn Leu Ile Trp Val Val Thr Arg Met Gln Ile Leu Val Asp Arg Tyr
```

```
        195                 200                 205
Pro Thr Trp Gly Asp Val Val Gln Val Asp Thr Trp Val Ser Ala Ser
    210                 215                 220
Gly Lys Asn Gly Met Arg Arg Asp Trp Leu Leu Arg Asp Ala Lys Thr
225                 230                 235                 240
Gly Glu Thr Leu Thr Arg Ala Ser Ser Val Trp Val Met Met Asn Lys
                245                 250                 255
Val Thr Arg Arg Leu Ser Lys Ile Pro Glu Glu Val Arg Gly Glu Ile
            260                 265                 270
Glu Pro His Phe Leu Thr Ser Asp Pro Val Val Asn Glu Asp Ser Arg
        275                 280                 285
Lys Leu Pro Lys Ile Asp Asp Asn Thr Ala Asp Tyr Ile Cys Glu Ser
    290                 295                 300
Leu Thr Pro Arg Trp Asn Asp Leu Asp Val Asn Gln His Val Asn Asn
305                 310                 315                 320
Val Lys Tyr Ile Gly Trp Ile Leu Glu Ser Ala Pro Pro Pro Ile Met
                325                 330                 335
Glu Ser His Glu Leu Ala Ala Ile Thr Leu Glu Tyr Arg Arg Glu Cys
            340                 345                 350
Gly Arg Asp Ser Val Leu Gln Ser Leu Thr Ala Val Ser Asp Thr Gly
        355                 360                 365
Ile Gly Asn Leu Gly Ser Pro Gly Glu Val Glu Phe Gln His Leu Leu
    370                 375                 380
Arg Phe Glu Glu Gly Ala Glu Ile Val Arg Gly Arg Thr Glu Trp Arg
385                 390                 395                 400
Pro Lys His Ala Asp Asn Phe Gly Ile Met Gly Gln Ile Pro Ala Val
                405                 410                 415
Ser Ala
```

<210> 140
<211> 266
<212> PRT
<213> Artificial sequence

<220>
<223> IPR002864 Acyl-ACP thioesterase family comprised in SEQ ID NO: 2

<400> 140

```
Gly Leu Val Phe Arg Gln Asn Phe Ser Ile Arg Ser Tyr Glu Ile Gly
1               5                   10                  15
Ala Asp Arg Ser Ala Ser Ile Glu Thr Val Met Asn His Leu Gln Glu
            20                  25                  30
Thr Ala Leu Asn His Val Lys Thr Ala Gly Leu Leu Gly Asp Gly Phe
        35                  40                  45
Gly Ser Thr Pro Glu Met Phe Lys Lys Asn Leu Ile Trp Val Val Thr
    50                  55                  60
Arg Met Gln Val Val Val Asp Lys Tyr Pro Thr Trp Gly Asp Val Val
65                  70                  75                  80
Glu Val Asp Thr Trp Val Ser Gln Ser Gly Lys Asn Gly Met Arg Arg
                85                  90                  95
Asp Trp Leu Val Arg Asp Cys Asn Thr Gly Glu Thr Leu Thr Arg Ala
            100                 105                 110
Ser Ser Val Trp Val Met Met Asn Lys Leu Thr Arg Arg Leu Ser Lys
        115                 120                 125
Ile Pro Glu Glu Val Arg Gly Glu Ile Glu Pro Tyr Phe Val Asn Ser
    130                 135                 140
Asp Pro Val Leu Ala Glu Asp Ser Arg Lys Leu Thr Lys Ile Asp Asp
145                 150                 155                 160
Lys Thr Ala Asp Tyr Val Arg Ser Gly Leu Thr Pro Arg Trp Ser Asp
                165                 170                 175
Leu Asp Val Asn Gln His Val Asn Asn Val Lys Tyr Ile Gly Trp Ile
            180                 185                 190
```

```
Leu Glu Ser Ala Pro Val Gly Ile Met Glu Arg Gln Lys Leu Lys Ser
        195                 200                 205
Met Thr Leu Glu Tyr Arg Arg Glu Cys Gly Arg Asp Ser Val Leu Gln
    210                 215                 220
Ser Leu Thr Ala Val Thr Gly Cys Asp Ile Gly Asn Leu Ala Thr Ala
225                 230                 235                 240
Gly Asp Val Glu Cys Gln His Leu Leu Arg Leu Gln Asp Gly Ala Glu
                245                 250                 255
Val Val Arg Gly Arg Thr Glu Trp Ser Ser
            260                 265
```

<210> 141
<211> 24
<212> PRT
<213> Artificial sequence

<220>
<223> TMpred predicted transmembrane helix

<400> 141

```
Thr Phe Ile Asn Gln Leu Pro Asp Trp Ser Met Leu Leu Ala Ala Ile
1               5                   10                  15
Thr Thr Ile Phe Leu Ala Ala Glu
            20
```

<210> 142
<211> 52
<212> DNA
<213> Artificial sequence

<220>
<223> primer: Prm 08145

<400> 142

```
ggggacaagt ttgtacaaaa aagcaggctt aaacaatggt ggccacctct gc             52
```

<210> 143
<211> 50
<212> DNA
<213> Artificial sequence

<220>
<223> primer: Prm 08146

<400> 143

```
ggggaccact ttgtacaaga aagctgggtt ttttcttacg gtgcagttcc                50
```

<210> 144
<211> 2194
<212> DNA
<213> Oryza sativa

<400> 144

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
```

tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa cctttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgtttc aaattcacat cgattatcta tgcattatcc tcttgtatct 2040
acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

<210> 145
<211> 1275
<212> DNA
<213> Arabidopsis thaliana

<400> 145
atggatcctg aaggtttcac gagtggctta ttccggtgga acccaacgag agcattggtt 60
caagcaccac ctccggttcc acctccgctg cagcaacagc cggtgacacc gcagacggct 120
gcttttggga tgcgacttgg tggtttagag ggactattcg gtccgtacgg tatacgtttc 180
tacacggcgg cgaagatagc ggagttaggt tttacggcga gcacgcttgt gggtatgaag 240
gacgaggagc ttgaagagat gatgaatagt ctctctcata tctttcgttg ggagcttctt 300
gttggtgaac ggtacggtat caaagctgcc gttagagctg aacggagacg attgcaagaa 360
gaggaggaag aggaatcttc tagacgccgt catttgctac tctccgccgc tggtgattcc 420
ggtactcatc acgctcttga tgctctctcc caagaagatg attggacagg gttatctgag 480
gaaccggtgc agcaacaaga ccagactgat gcggcgggga ataacggcgg aggaggaagt 540
ggttactggg acgcaggtca aggaaagatg aagaagcaac agcagcagag acggagaaag 600
aaaccaatgc tgacgtcagt ggaaaccgac gaagacgtca acgaaggtga ggatgacgac 660
gggatggata acggcaacgg aggtagtggt ttggggacag agagacagag ggagcatccg 720
tttatcgtaa cggagcctgg ggaagtggca cgtggcaaaa agaacggctt agattatctg 780
ttccacttgt acgaacaatg ccgtgagttc cttcttcagg tccagacaat tgctaaagac 840
cgtggcgaaa aatgccccac caaggtgacg aaccaagtat tcaggtacgc gaagaaatca 900
ggagcgagtt acataaacaa gcctaaaatg cgacactacg ttcactgtta cgctctccac 960
tgcctagacg aagaagcttc aaatactctc agaagagcgt ttaaagaacg cggtgagaac 1020
gttggctcat ggcgtcaggc ttgttacaag ccacttgtga acatcgcttg tcgtcatggc 1080
tgggatatag acgccgtctt taacgctcat cctcgtctct ctatttggta tgttccaaca 1140
aagctgcgtc agctttgcca tttggagcgg aacaatgcgg ttgctgcggc tgcggcttta 1200
gttggcggta ttagctgtac cggatcgtcg acgtctggac gtggtggatg cggcggcgac 1260
gacttgcgtt tctag 1275

<210> 146
<211> 424
<212> PRT
<213> Arabidopsis thaliana

<400> 146

Met Asp Pro Glu Gly Phe Thr Ser Gly Leu Phe Arg Trp Asn Pro Thr
1               5                   10                  15
Arg Ala Leu Val Gln Ala Pro Pro Pro Val Pro Pro Pro Leu Gln Gln
            20                  25                  30
Gln Pro Val Thr Pro Gln Thr Ala Ala Phe Gly Met Arg Leu Gly Gly
        35                  40                  45
Leu Glu Gly Leu Phe Gly Pro Tyr Gly Ile Arg Phe Tyr Thr Ala Ala
    50                  55                  60
Lys Ile Ala Glu Leu Gly Phe Thr Ala Ser Thr Leu Val Gly Met Lys
65                  70                  75                  80
Asp Glu Glu Leu Glu Glu Met Met Asn Ser Leu Ser His Ile Phe Arg
                85                  90                  95
Trp Glu Leu Leu Val Gly Glu Arg Tyr Gly Ile Lys Ala Ala Val Arg
            100                 105                 110
Ala Glu Arg Arg Arg Leu Gln Glu Glu Glu Glu Glu Ser Ser Arg
        115                 120                 125
Arg Arg His Leu Leu Leu Ser Ala Ala Gly Asp Ser Gly Thr His His
    130                 135                 140
Ala Leu Asp Ala Leu Ser Gln Glu Asp Asp Trp Thr Gly Leu Ser Glu
145                 150                 155                 160
Glu Pro Val Gln Gln Gln Asp Gln Thr Asp Ala Ala Gly Asn Asn Gly
                165                 170                 175
Gly Gly Gly Ser Gly Tyr Trp Asp Ala Gly Gln Gly Lys Met Lys Lys
            180                 185                 190
Gln Gln Gln Gln Arg Arg Arg Lys Lys Pro Met Leu Thr Ser Val Glu
        195                 200                 205
Thr Asp Glu Asp Val Asn Glu Gly Glu Asp Asp Asp Gly Met Asp Asn
    210                 215                 220
Gly Asn Gly Gly Ser Gly Leu Gly Thr Glu Arg Gln Arg Glu His Pro
225                 230                 235                 240
Phe Ile Val Thr Glu Pro Gly Glu Val Ala Arg Gly Lys Lys Asn Gly
                245                 250                 255
Leu Asp Tyr Leu Phe His Leu Tyr Glu Gln Cys Arg Glu Phe Leu Leu
            260                 265                 270
Gln Val Gln Thr Ile Ala Lys Asp Arg Gly Glu Lys Cys Pro Thr Lys
        275                 280                 285
Val Thr Asn Gln Val Phe Arg Tyr Ala Lys Lys Ser Gly Ala Ser Tyr
    290                 295                 300
Ile Asn Lys Pro Lys Met Arg His Tyr Val His Cys Tyr Ala Leu His
305                 310                 315                 320
Cys Leu Asp Glu Glu Ala Ser Asn Ala Leu Arg Arg Ala Phe Lys Glu
                325                 330                 335
Arg Gly Glu Asn Val Gly Ser Trp Arg Gln Ala Cys Tyr Lys Pro Leu
            340                 345                 350
Val Asn Ile Ala Cys Arg His Gly Trp Asp Ile Asp Ala Val Phe Asn
        355                 360                 365
Ala His Pro Arg Leu Ser Ile Trp Tyr Val Pro Thr Lys Leu Arg Gln
    370                 375                 380
Leu Cys His Leu Glu Arg Asn Asn Ala Val Ala Ala Ala Ala Ala Leu
385                 390                 395                 400
Val Gly Gly Ile Ser Cys Thr Gly Ser Ser Thr Ser Gly Arg Gly Gly
                405                 410                 415
Cys Gly Gly Asp Asp Leu Arg Phe
            420

<210> 147
<211> 55
<212> DNA
<213> Artificial sequence

<220>
<223> primer: prm4841

<400> 147
ggggacaagt ttgtacaaaa aagcaggctt aaacaatgga tcctgaaggt ttcac 55

<210> 148
<211> 50
<212> DNA
<213> Artificial sequence

<220>
<223> primer: prm4842

<400> 148
ggggaccact ttgtacaaga aagctgggta accaaactag aaacgcaagt 50

<210> 149
<211> 2194
<212> DNA
<213> Oryza sativa

<400> 149
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa cctttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040
acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

<210> 150
<211> 1179
<212> DNA
<213> Oryza sativa

<400> 150

```
ttgcagttgt gaccaagtaa gctgagcatg cccttaactt cacctagaaa aaagtatact      60
tggcttaact gctagtaaga catttcagaa ctgagactgg tgtacgcatt tcatgcaagc     120
cattaccact ttacctgaca ttttggacag agattagaaa tagtttcgta ctacctgcaa     180
gttgcaactt gaaaagtgaa atttgttcct tgctaatata ttggcgtgta attcttttat     240
gcgttagcgt aaaaagttga aatttgggtc aagttactgg tcagattaac cagtaactgg     300
ttaaagttga aagatggtct tttagtaatg gagggagtac tacactatcc tcagctgatt     360
taaatcttat tccgtcggtg gtgatttcgt caatctccca acttagtttt tcaatatatt     420
cataggatag agtgtgcata tgtgtgttta tagggatgag tctacgcgcc ttatgaacac     480
ctacttttgt actgtatttg tcaatgaaaa gaaaatctta ccaatgctgc gatgctgaca     540
ccaagaagag gcgatgaaaa gtgcaacgga tatcgtgcca cgtcggttgc caagtcagca     600
cagacccaat gggcctttcc tacgtgtctc ggccacagcc agtcgtttac cgcacgttca     660
catgggcacg aactcgcgtc atcttcccac gcaaaacgac agatctgccc tatctggtcc     720
cacccatcag tggcccacac ctcccatgct gcattatttg cgactcccat cccgtcctcc     780
acgcccaaac accgcacacg ggtcgcgata gccacgaccc aatcacacaa cgccacgtca     840
ccatatgtta cgggcagcca tgcgcagaag atcccgcgac gtcgctgtcc cccgtgtcgg     900
ttacgaaaaa atatcccacc acgtgtcgct ttcacaggac aatatctcga aggaaaaaaa     960
tcgtagcgga aaatccgagg cacgagctgc gattggctgg gaggcgtcca gcgtggtggg    1020
gggcccaccc ccttatcctt agcccgtggc gctcctcgct cctcgggtcc gtgtataaat    1080
accctccgga actcactctt gctggtcacc aacacgaagt aaaaggacac cagaaacata    1140
gtacacttga gctcactcca aactcaaaca ctcacacca                           1179
```

<210> 151
<211> 420
<212> PRT
<213> Arabidopsis thaliana

<400> 151

```
Met Asp Pro Glu Gly Phe Thr Ser Gly Leu Phe Arg Trp Asn Pro Thr
1               5                   10                  15
Arg Ala Leu Val Gln Ala Pro Pro Pro Val Pro Pro Pro Leu Gln Gln
            20                  25                  30
Gln Pro Val Thr Pro Gln Thr Ala Ala Phe Gly Met Arg Leu Gly Gly
        35                  40                  45
Leu Glu Gly Leu Phe Gly Pro Tyr Gly Ile Arg Phe Tyr Thr Ala Ala
    50                  55                  60
Lys Ile Ala Glu Leu Gly Phe Thr Ala Ser Thr Leu Val Gly Met Lys
65                  70                  75                  80
Asp Glu Glu Leu Glu Glu Met Met Asn Ser Leu Ser His Ile Phe Arg
                85                  90                  95
Trp Glu Leu Leu Val Gly Glu Arg Tyr Gly Ile Lys Ala Ala Val Arg
            100                 105                 110
Ala Glu Arg Arg Arg Leu Gln Glu Glu Glu Glu Glu Glu Ser Ser Arg
        115                 120                 125
Arg Arg His Leu Leu Leu Ser Ala Ala Gly Asp Ser Gly Thr His His
    130                 135                 140
Ala Leu Asp Ala Leu Ser Gln Glu Gly Leu Ser Glu Glu Pro Val Gln
145                 150                 155                 160
Gln Gln Asp Gln Thr Asp Ala Ala Gly Asn Asn Gly Gly Gly Gly Ser
                165                 170                 175
Gly Tyr Trp Asp Ala Gly Gln Gly Lys Met Lys Lys Gln Gln Gln Gln
            180                 185                 190
Arg Arg Arg Lys Lys Pro Met Leu Thr Ser Val Glu Thr Asp Glu Asp
        195                 200                 205
Val Asn Glu Gly Glu Asp Asp Asp Gly Met Asp Asn Gly Asn Gly Gly
    210                 215                 220
Ser Gly Leu Gly Thr Glu Arg Gln Arg Glu His Pro Phe Ile Val Thr
225                 230                 235                 240
Glu Pro Gly Glu Val Ala Arg Gly Lys Lys Asn Gly Leu Asp Tyr Leu
                245                 250                 255
Phe His Leu Tyr Glu Gln Cys Arg Glu Phe Leu Leu Gln Val Gln Thr
            260                 265                 270
Ile Ala Lys Asp Arg Gly Glu Lys Cys Pro Thr Lys Val Thr Asn Gln
```

```
        275                     280                     285
Val Phe Arg Tyr Ala Lys Lys Ser Gly Ala Ser Tyr Ile Asn Lys Pro
    290                     295                     300
Lys Met Arg His Tyr Val His Cys Tyr Ala Leu His Cys Leu Asp Glu
305                     310                     315                     320
Glu Ala Ser Asn Ala Leu Arg Arg Ala Phe Lys Glu Arg Gly Glu Asn
                325                     330                     335
Val Gly Ser Trp Arg Gln Ala Cys Tyr Lys Pro Leu Val Asn Ile Ala
            340                     345                     350
Cys Arg His Gly Trp Asp Ile Asp Ala Val Phe Asn Ala His Pro Arg
        355                     360                     365
Leu Ser Ile Trp Tyr Val Pro Thr Lys Leu Arg Gln Leu Cys His Leu
    370                     375                     380
Glu Arg Asn Asn Ala Val Ala Ala Ala Ala Ala Leu Val Gly Gly Ile
385                     390                     395                     400
Ser Cys Thr Gly Ser Ser Thr Ser Gly Arg Gly Gly Cys Gly Gly Asp
                405                     410                     415
Asp Leu Arg Phe
            420

<210>  152
<211>  420
<212>  PRT
<213>  Brassica juncea

<400>  152
Met Asp Pro Glu Gly Phe Thr Ser Gly Leu Phe Arg Trp Asn Pro Thr
1               5                   10                  15
Arg Ala Leu Val Gln Ala Pro Pro Pro Val Pro Pro Pro Leu Gln Gln
            20                  25                  30
Gln Pro Val Thr Pro Gln Thr Ala Ala Phe Gly Met Arg Leu Gly Gly
        35                  40                  45
Leu Glu Gly Leu Phe Gly Pro Tyr Gly Ile Arg Phe Tyr Thr Ala Ala
    50                  55                  60
Lys Ile Ala Glu Leu Gly Phe Thr Ala Ser Thr Leu Val Gly Met Lys
65                  70                  75                  80
Asp Glu Glu Leu Glu Glu Met Met Asn Ser Leu Ser His Ile Phe Arg
                85                  90                  95
Trp Glu Leu Leu Val Gly Glu Arg Tyr Gly Ile Lys Ala Ala Val Arg
            100                 105                 110
Ala Glu Arg Arg Arg Leu Gln Glu Glu Glu Glu Glu Glu Ser Ser Arg
        115                 120                 125
Arg Arg His Leu Leu Leu Ser Ala Ala Gly Asp Ser Gly Thr His His
    130                 135                 140
Ala Leu Asp Ala Leu Ser Gln Glu Glu Leu Ser Glu Glu Pro Val Gln
145                 150                 155                 160
Gln Gln Asp Gln Thr Asp Ala Ala Gly Asn Asn Gly Gly Gly Gly Ser
                165                 170                 175
Gly Tyr Trp Asp Ala Gly Gln Gly Lys Met Lys Lys Gln Gln Gln Gln
            180                 185                 190
Arg Arg Arg Lys Lys Pro Met Leu Thr Ser Val Glu Thr Asp Glu Asp
        195                 200                 205
Val Asn Glu Gly Glu Asp Asp Asp Gly Met Asp Asn Gly Asn Gly Gly
    210                 215                 220
Ser Gly Leu Gly Thr Glu Arg Gln Arg Glu His Pro Phe Ile Val Thr
225                 230                 235                 240
Glu Pro Gly Glu Val Ala Arg Gly Lys Lys Asn Gly Leu Asp Tyr Leu
                245                 250                 255
Phe His Leu Tyr Glu Gln Cys Arg Glu Phe Leu Leu Gln Val Gln Thr
            260                 265                 270
Ile Ala Lys Asp Arg Gly Glu Lys Cys Pro Thr Lys Val Thr Asn Gln
        275                 280                 285
Val Phe Arg Tyr Ala Lys Lys Ser Gly Ala Ser Tyr Ile Asn Lys Pro
```

```
    290                 295                 300
Lys Met Arg His Tyr Val His Cys Tyr Ala Leu His Cys Leu Asp Glu
305                 310                 315                 320
Glu Ala Ser Asn Ala Leu Arg Arg Ala Phe Lys Glu Arg Gly Glu Asn
                325                 330                 335
Val Gly Ser Trp Arg Gln Ala Cys Tyr Lys Pro Leu Val Asn Ile Ala
            340                 345                 350
Cys Arg His Gly Trp Asp Ile Asp Ala Val Phe Asn Ala His Pro Arg
        355                 360                 365
Leu Ser Ile Trp Tyr Val Pro Thr Lys Leu Arg Gln Leu Cys His Leu
    370                 375                 380
Glu Arg Asn Asn Ala Val Ala Ala Ala Ala Ala Leu Val Gly Gly Ile
385                 390                 395                 400
Ser Cys Thr Gly Ser Ser Thr Ser Gly Arg Gly Gly Cys Gly Gly Asp
                405                 410                 415
Asp Leu Arg Phe
            420

<210>  153
<211>  426
<212>  PRT
<213>  Ionopsidium acaule

<400>  153
Met Asp Pro Glu Gly Phe Thr Ser Gly Leu Phe Arg Trp Asn Thr Thr
1               5                   10                  15
Arg Ala Met Val Gln His Gln Pro Pro Pro Gln Val Pro Pro Pro Pro
            20                  25                  30
Ser Gln Gln Ser Pro Val Thr Pro Gln Thr Ala Ala Phe Gly Met Arg
        35                  40                  45
Leu Gly Gly Leu Glu Gly Leu Phe Gly Pro Tyr Gly Ile Arg Phe Tyr
    50                  55                  60
Thr Ala Ala Lys Ile Ala Glu Leu Gly Phe Thr Ala Ser Thr Leu Val
65                  70                  75                  80
Gly Met Lys Asp Glu Glu Leu Glu Asp Met Met Asn Ser Leu Ser His
                85                  90                  95
Ile Phe Arg Trp Glu Leu Leu Val Gly Glu Arg Tyr Gly Ile Lys Ala
            100                 105                 110
Ala Val Arg Ala Glu Arg Arg Arg Leu Gln Glu Glu Glu Glu Asp Asp
        115                 120                 125
Ser Ser Arg Arg Arg His Leu Leu Leu Ser Ala Ala Gly Asp Ser Gly
    130                 135                 140
Thr His His Ala Leu Asp Ala Leu Ser Gln Glu Asp Asp Trp Thr Gly
145                 150                 155                 160
Leu Ser Glu Glu Pro Val His Gln Asp Gln Thr Asp Ala Ala Gly Asn
                165                 170                 175
Gly Gly Phe Gly Gly Tyr Leu Glu Ser Ser Val His Gly Lys Met Lys
            180                 185                 190
Lys His Gln Pro Arg Arg Arg Lys Lys Pro Leu Val Leu Thr Ser Val
        195                 200                 205
Glu Thr Asp Asp Asp Gly Asn Asp Asn Glu Asp Asp Asp Gly Met Asp
    210                 215                 220
Asn Gly Asn Gly Gly Ile Gly Leu Gly Thr Glu Arg Gln Arg Glu His
225                 230                 235                 240
Pro Phe Ile Val Thr Glu Pro Gly Glu Val Ala Arg Gly Lys Lys Asn
                245                 250                 255
Gly Leu Asp Tyr Leu Phe His Leu Tyr Glu Gln Cys Arg Glu Phe Leu
            260                 265                 270
Leu Gln Val Gln Thr Ile Ala Lys Asp Arg Gly Glu Lys Cys Pro Thr
        275                 280                 285
Lys Val Thr Asn Gln Val Phe Arg Tyr Ala Lys Lys Ser Gly Ala Ser
    290                 295                 300
Tyr Ile Asn Lys Pro Lys Met Arg His Tyr Val His Cys Tyr Ala Leu
```

305 310 315 320
His Cys Leu Asp Glu Glu Ala Ser Asn Ala Leu Arg Arg Ala Phe Lys
325 330 335
Glu Arg Gly Glu Asn Val Gly Ser Trp Arg Gln Ala Cys Tyr Lys Pro
340 345 350
Leu Val Asn Ile Ala Cys Arg His Gly Trp Asp Ile Asp Ala Val Phe
355 360 365
Asn Ala His Pro Arg Leu Ser Ile Trp Tyr Val Pro Thr Lys Leu Arg
370 375 380
Gln Leu Cys His Leu Glu Arg Asn Asn Ala Val Ala Ala Ala Ala Ala
385 390 395 400
Leu Val Gly Gly Ile Ser Cys Thr Gly Ser Ser Ala Ser Gly Arg Gly
405 410 415
Gly Cys Gly Gly Asp Glu Glu Leu Arg Tyr
420 425

<210> 154
<211> 417
<212> PRT
<213> Leavenworthia crassa

<400> 154
Met Asp Pro Glu Gly Phe Thr Ser Gly Leu Phe Arg Trp Asn Pro Thr
1 5 10 15
Arg Ala Thr Val Gln Ala Leu Pro Pro Val Pro Pro Pro Leu Gln Gln
20 25 30
Gln Pro Ala Thr Val Gln Ser Ala Ala Phe Gly Thr Arg Leu Gly Gly
35 40 45
Leu Glu Gly Leu Phe Gly Val Tyr Gly Ile Arg Phe Tyr Thr Ala Ala
50 55 60
Lys Ile Ala Glu Leu Gly Phe Thr Ala Ser Thr Leu Val Gly Met Arg
65 70 75 80
Asp Glu Glu Leu Glu Glu Met Met Asn Ser Leu Ser His Ile Phe Arg
85 90 95
Trp Glu Leu Leu Val Gly Glu Arg Tyr Gly Ile Lys Ala Ala Val Arg
100 105 110
Ala Glu Arg Arg Arg Leu Gln Glu Glu Glu Glu Glu Glu Ser Ser Arg
115 120 125
Arg Arg His Leu Leu Leu Ser Ala Ala Gly Asp Ser Gly Thr His His
130 135 140
Ala Leu Asp Ala Leu Ser Gln Glu Asp Asp Trp Thr Gly Leu Ser Glu
145 150 155 160
Glu Pro Val Gln Gln Ile Asp His Leu Thr Asp Ala Val Gly Asn Asn
165 170 175
Gly Gly Tyr Trp Glu Ala Asn Lys Gly Lys Met Lys Lys Gln Gln Gln
180 185 190
Arg Arg Arg Lys Lys Pro Met Leu Thr Ser Val Glu Thr Asp Asp Asp
195 200 205
Ile Asn Glu Gly Glu Asp Glu Asp Gly Met Asp Asn Ser Asn Gly Gly
210 215 220
Leu Gly Thr Glu Arg Gln Arg Glu His Pro Phe Ile Val Thr Glu Pro
225 230 235 240
Gly Glu Val Ala Arg Gly Lys Lys Asn Gly Leu Asp Tyr Leu Phe His
245 250 255
Leu Tyr Glu Gln Cys Arg Glu Phe Leu Leu Gln Val Gln Thr Ile Ala
260 265 270
Lys Asp Arg Gly Glu Lys Cys Pro Thr Lys Val Thr Asn Gln Val Phe
275 280 285
Arg Tyr Ala Lys Lys Ser Gly Ala Ser Tyr Ile Asn Lys Pro Lys Met
290 295 300
Arg His Tyr Val His Cys Tyr Ala Leu His Cys Leu Asp Glu Glu Ala
305 310 315 320
Ser Asn Ala Leu Arg Arg Ala Phe Lys Glu Arg Gly Glu Asn Val Gly

```
                325                 330                 335
Ser Trp Arg Gln Ala Cys Tyr Lys Pro Leu Val Asn Ile Ala Cys Arg
            340                 345                 350
His Gly Trp Asp Ile Asp Ala Val Phe Asn Ser His Pro Arg Leu Ser
        355                 360                 365
Ile Trp Tyr Val Pro Thr Lys Leu Arg Gln Leu Cys His Met Glu Arg
    370                 375                 380
Asn Asn Glu Val Ala Ala Ala Thr Val Leu Val Gly Gly Ile Ser Cys
385                 390                 395                 400
Thr Gly Thr Ser Ala Ser Gly His Gly Glu Cys Gly Gly Glu Leu His
                405                 410                 415
Tyr
```

<210> 155
<211> 403
<212> PRT
<213> Selenia aurea

<400> 155

```
Met Asp Pro Glu Gly Phe Thr Ser Gly Leu Phe Arg Trp Asn Pro Thr
1               5                   10                  15
Arg Ala Thr Val Gln Ala Leu Ala Pro Val Pro Pro Pro Leu Gln Gln
            20                  25                  30
Gln Pro Ala Thr Ala Gln Thr Ala Ala Phe Gly Met Arg Leu Gly Gly
        35                  40                  45
Leu Glu Gly Leu Phe Gly Ala Tyr Gly Ile Arg Phe Tyr Thr Ala Ala
    50                  55                  60
Lys Ile Ala Glu Leu Gly Phe Thr Ala Ser Thr Leu Val Gly Met Arg
65                  70                  75                  80
Asp Glu Glu Leu Glu Glu Met Met Asn Ser Leu Ser His Ile Phe Arg
                85                  90                  95
Trp Glu Leu Leu Val Gly Glu Arg Tyr Gly Ile Lys Ala Ala Val Arg
            100                 105                 110
Ala Glu Arg Arg Arg Leu Gln Glu Glu Glu Glu Glu Ser Ser Arg
        115                 120                 125
Arg Arg His Leu Leu Leu Ser Ala Ala Gly Asp Ser Gly Thr His His
    130                 135                 140
Ala Leu Asp Ala Leu Ser Gln Glu Asp Asp Trp Thr Gly Leu Ser Glu
145                 150                 155                 160
Glu Pro Val Gln Gln Gln Asp His Gln Thr Asp Ala Val Gly Asn Asn
                165                 170                 175
Gly Gly Tyr Trp Asp Glu Gly Lys Gly Lys Met Lys Lys Gln Gln Gln
            180                 185                 190
Arg Arg Arg Met Lys Pro Leu Met Thr Ser Val Glu Pro Asp Asn Asp
        195                 200                 205
Met Asp Glu Cys Glu Asp Glu Asp Arg Met Asp Asn Gly Asn Gly Gly
    210                 215                 220
Gly Gly Gly Leu Gly Met Glu Arg Gln Arg Glu His Pro Phe Ile Val
225                 230                 235                 240
Thr Glu Pro Gly Glu Val Ala Arg Gly Lys Lys Asn Gly Leu Asp Tyr
                245                 250                 255
Leu Phe His Leu Tyr Glu Gln Cys Arg Glu Phe Leu Leu Gln Val Gln
            260                 265                 270
Leu Ile Ala Lys Asp Arg Gly Glu Lys Cys Pro Thr Lys Val Thr Asn
        275                 280                 285
Gln Val Phe Arg Tyr Ala Lys Lys Ser Gly Ala Ser Tyr Ile Asn Lys
    290                 295                 300
Pro Lys Met Arg His Tyr Val His Cys Tyr Ala Leu His Cys Leu Asp
305                 310                 315                 320
Glu Asp Ala Ser Asn Ala Leu Arg Arg Ala Phe Lys Glu Arg Gly Glu
                325                 330                 335
Asn Val Gly Ser Trp Arg Gln Ala Arg Tyr Lys Pro Leu Val Asp Ile
```

```
            340                 345                 350
Ala Cys Arg His Gly Trp Asp Ile Asp Ala Val Phe Asn Ala His Pro
        355                 360                 365
Arg Leu Ser Ile Trp Tyr Val Pro Thr Lys Leu Arg Gln Leu Cys His
    370                 375                 380
Leu Glu Arg Asn Asn Ala Val Ala Ala Ala Ala Val Leu Val Gly Gly
385                 390                 395                 400
Ile Ser Cys


<210>  156
<211>  430
<212>  PRT
<213>  Arabidopsis lyrata

<400>  156
Met Asp Pro Glu Gly Phe Thr Ser Gly Leu Phe Arg Trp Asn Pro Thr
1               5                   10                  15
Arg Ala Met Val Ala Ala Pro Pro Pro Val Pro Pro Gln Pro Gln Gln
            20                  25                  30
Gln Pro Ala Thr Pro Gln Thr Arg Ala Phe Gly Met Arg Leu Gly Gly
        35                  40                  45
Leu Glu Gly Leu Phe Gly Ala Tyr Gly Ile Arg Phe Tyr Thr Ala Ala
    50                  55                  60
Lys Ile Ala Glu Leu Gly Phe Thr Ala Ser Thr Leu Val Gly Met Lys
65                  70                  75                  80
Asp Glu Glu Leu Glu Glu Met Met Asn Ser Leu Ser His Ile Phe Arg
                85                  90                  95
Trp Glu Leu Leu Val Gly Glu Arg Tyr Gly Ile Lys Ala Ala Val Thr
            100                 105                 110
Ala Glu Arg Arg Arg Leu Gln Glu Glu Glu Glu Glu Ser Ser Arg
        115                 120                 125
Arg Arg His Leu Leu Leu Ser Ala Ala Gly Asp Ser Gly Thr His His
    130                 135                 140
Ala Leu Asp Ala Leu Ser Gln Glu Asp Asp Trp Thr Gly Leu Ser Glu
145                 150                 155                 160
Glu Leu Asp Arg Glu Pro Val Gln Gln Gln Asn Gln Thr Asp Ala Ala
                165                 170                 175
Gly Asn Asn Gly Gly Gly Gly Ser Gly Tyr Trp Glu Ala Gly Gln Ala
            180                 185                 190
Lys Met Lys Lys Gln Gln Gln Gln Arg Arg Arg Lys Lys Pro Met Val
        195                 200                 205
Thr Ser Val Glu Thr Asp Asp Asp Val Asn Glu Gly Asp Asp Asp Asp
    210                 215                 220
Gly Met Asp Asn Gly Asn Gly Gly Gly Gly Gly Gly Leu Gly Thr Glu
225                 230                 235                 240
Arg Gln Arg Glu His Pro Phe Ile Val Thr Glu Pro Gly Glu Val Ala
                245                 250                 255
Arg Gly Lys Lys Asn Gly Leu Asp Tyr Leu Phe His Leu Tyr Glu Gln
            260                 265                 270
Cys Arg Glu Phe Leu Leu Gln Val Gln Thr Ile Ala Lys Asp Arg Gly
        275                 280                 285
Glu Lys Cys Pro Thr Lys Val Thr Asn Gln Val Phe Arg Tyr Ala Lys
    290                 295                 300
Lys Ser Gly Ala Ser Tyr Ile Asn Lys Pro Lys Met Arg His Tyr Val
305                 310                 315                 320
His Cys Tyr Ala Leu His Cys Leu Asp Glu Asp Ala Ser Asn Ala Leu
                325                 330                 335
Arg Arg Ala Phe Lys Glu Arg Gly Glu Asn Val Gly Ser Trp Arg Gln
            340                 345                 350
Ala Cys Tyr Lys Pro Leu Val Asn Ile Ala Cys Arg His Gly Trp Asp
        355                 360                 365
Ile Asp Ala Val Phe Asn Ala His Pro Arg Leu Ser Ile Trp Tyr Val
```

370 375 380
Pro Thr Lys Leu Arg Gln Leu Cys His Leu Glu Arg Asn Asn Ala Val
385 390 395 400
Ala Ala Ala Ala Ala Leu Val Gly Gly Ile Ser Cys Thr Gly Ser Ser
405 410 415
Thr Ser Gly Arg Gly Gly Cys Gly Gly Asp Asp Leu Arg Phe
420 425 430

<210> 157
<211> 403
<212> PRT
<213> Streptanthus glandulosus

<400> 157
Ser Gly Leu Phe Arg Trp Asn Ser Thr Arg Ala Leu Val Gln Gln Pro
1 5 10 15
Pro Pro Val Pro Pro Pro Gln Gln Gln Pro Pro Glu Thr Pro Gln Thr
20 25 30
Val Ala Phe Gly Met Arg Leu Gly Gly Leu Glu Gly Leu Phe Gly Ala
35 40 45
Tyr Gly Ile Arg Phe Tyr Thr Ala Ala Lys Ile Ala Glu Leu Gly Phe
50 55 60
Thr Ala Ser Thr Leu Val Gly Met Lys Asp Glu Glu Leu Glu Asp Met
65 70 75 80
Met Asn Ser Leu Ser His Ile Phe Arg Trp Glu Leu Leu Val Gly Glu
85 90 95
Arg Tyr Gly Ile Lys Ala Ala Val Arg Ala Glu Arg Arg Arg Leu Gln
100 105 110
Glu Val Glu Glu Glu Glu Ser Ser Arg Arg Arg His Leu Leu Leu Cys
115 120 125
Ala Ala Gly Asp Ser Gly Thr His His Ala Leu Asp Thr Leu Ser Gln
130 135 140
Glu Asp Tyr Trp Thr Gly Leu Ser Glu Glu Pro Gly Gln Gln Gln Asp
145 150 155 160
Gln Thr Asp Ala Ala Gly Asn Asn Gly Gly Asn Gly Gly Gly Glu Gly
165 170 175
Gly Gly Tyr Trp Glu Ala Gly Gln Ala Lys Met Lys Lys Pro Gln Gln
180 185 190
Arg Arg Arg Lys Lys Ser Met Val Thr Ser Val Glu Ile Asp Asp Glu
195 200 205
Cys Asn Glu Gly Glu Asp Asp Asp Gly Met Asp Asn Cys Asn Gly Gly
210 215 220
Gly Gly Gly Leu Gly Ile Glu Arg Gln Arg Glu His Pro Phe Ile Val
225 230 235 240
Thr Glu Pro Gly Glu Val Ala Arg Gly Lys Lys Asn Gly Leu Asp Tyr
245 250 255
Leu Phe His Leu Tyr Glu Gln Cys Arg Glu Phe Leu Leu Gln Val Gln
260 265 270
Thr Ile Ala Lys Asp Arg Gly Glu Lys Cys Pro Thr Lys Gly Thr Asn
275 280 285
Gln Val Phe Arg Tyr Ala Lys Asn Ser Gly Ala Ser Tyr Ile Asn Lys
290 295 300
Pro Lys Met Arg His Tyr Val His Cys Tyr Ala Leu His Cys Leu Asp
305 310 315 320
Glu Glu Ala Ser Asn Ala Leu Arg Arg Ala Phe Lys Glu Arg Gly Glu
325 330 335
Asn Val Gly Ser Trp Arg Gln Ala Cys Tyr Lys Pro Leu Val Asn Ile
340 345 350
Ala Cys Arg His Gly Trp Asp Ile Asp Ala Val Phe Asn Ala His Pro
355 360 365
His Leu Ser Ile Trp Tyr Val Pro Thr Lys Leu Arg Gln Leu Cys His
370 375 380
Leu Glu Arg Asn Asn Ala Val Ala Ala Ala Ala Ala Leu Val Gly Gly

385 390 395 400
Ile Ser Cys

<210> 158
<211> 407
<212> PRT
<213> Cochlearia officinalis

<400> 158
Met Asp Pro Glu Gly Phe Thr Asn Gly Leu Phe Arg Trp Asn Thr Thr
1 5 10 15
Arg Ala Met Ile Gln Gln Gln Gln Gln Leu Pro Pro Pro Gln Ile Thr
20 25 30
Pro Pro Pro Gln Gln Ser Pro Ala Thr Pro Gln Thr Ala Ala Phe Gly
35 40 45
Met Arg Leu Gly Gly Leu Glu Gly Leu Phe Gly Pro Tyr Gly Ile Arg
50 55 60
Phe Tyr Thr Ala Ala Lys Ile Ala Glu Leu Gly Phe Thr Ala Ser Thr
65 70 75 80
Leu Val Gly Met Lys Asp Glu Glu Leu Glu Asp Met Met Asn Ser Leu
85 90 95
Ser His Ile Phe Arg Trp Glu Leu Leu Val Gly Glu Arg Tyr Gly Ile
100 105 110
Lys Ala Ala Val Arg Thr Glu Arg Arg Arg Leu Gln Glu Glu Glu Glu
115 120 125
Glu Glu Ser Ser Arg Arg Arg His Phe Met Leu Ser Ala Gly Gly Asp
130 135 140
Ser Gly Thr His His Ala Leu Asp Ala Leu Ser Gln Glu Asp Asp Trp
145 150 155 160
Thr Gly Leu Ser Glu Glu Pro Val His Gln Asp Gln Thr Asp Ala Ala
165 170 175
Gly Asn Gly Gly Phe Gly Gly Tyr Leu Glu Ser Gly His Gly Lys Met
180 185 190
Lys Lys Gln Gln Gln Gln Lys Arg Arg Lys Lys Pro Leu Val Thr Ser
195 200 205
Val Glu Thr Asp Asp Asp Gly Asn Asp Asp Asp Asp Gly Met Asp Asn
210 215 220
Gly Asn Gly Gly Ser Ser Gly Leu Gly Thr Glu Arg Gln Arg Glu His
225 230 235 240
Pro Phe Ile Val Thr Glu Pro Gly Glu Val Ala Arg Gly Lys Lys Asn
245 250 255
Gly Leu Asp Tyr Leu Phe His Leu Tyr Glu Gln Cys Arg Glu Phe Leu
260 265 270
Leu Gln Val Gln Thr Ile Ala Lys Asp Arg Gly Glu Lys Cys Pro Thr
275 280 285
Lys Val Thr Asn Gln Val Phe Arg Tyr Ala Lys Lys Ser Gly Ala Ser
290 295 300
Tyr Ile Asn Lys Pro Lys Met Arg His Tyr Val His Cys Tyr Ala Leu
305 310 315 320
His Cys Leu Asp Glu Asp Ala Ser Asn Ala Leu Arg Arg Ala Phe Lys
325 330 335
Glu Arg Gly Glu Asn Val Gly Ser Trp Arg Gln Ala Cys Tyr Lys Pro
340 345 350
Leu Val Asn Ile Ala Cys Arg His Gly Trp Asp Ile Asp Ala Val Phe
355 360 365
Asn Ala His Pro Arg Leu Ser Ile Trp Tyr Val Pro Thr Lys Leu Arg
370 375 380
Gln Leu Cys His Leu Glu Arg Asn Asn Ala Val Ala Ala Ala Ser Ala
385 390 395 400
Leu Val Gly Gly Ile Ser Cys
405

<210> 159
<211> 415
<212> PRT
<213> Brassica oleracea var. botrytis

<400> 159
Met Asp Pro Glu Gly Phe Thr Ser Gly Leu Phe Arg Trp Asn Pro Thr
1 5 10 15
Arg Val Met Val Gln Ala Pro Thr Pro Ile Pro Pro Pro Gln Gln Gln
20 25 30
Ser Pro Ala Thr Pro Gln Thr Ala Ala Phe Gly Met Arg Leu Gly Gly
35 40 45
Leu Glu Gly Leu Phe Gly Pro Tyr Gly Val Arg Phe Tyr Thr Ala Ala
50 55 60
Lys Ile Ala Glu Leu Gly Phe Thr Ala Ser Thr Leu Val Gly Met Lys
65 70 75 80
Asp Glu Glu Leu Glu Asp Met Met Asn Ser Leu Ser His Ile Phe Arg
85 90 95
Trp Glu Leu Leu Val Gly Glu Arg Tyr Gly Ile Lys Ala Ala Val Arg
100 105 110
Ala Glu Arg Arg Arg Leu Gln Glu Glu Glu Glu Glu Ser Ser Arg
115 120 125
Arg Arg His Leu Leu Leu Ser Ala Ala Gly Asp Ser Gly Thr His Leu
130 135 140
Ala Leu Asp Ala Leu Ser Gln Glu Asp Asp Trp Thr Gly Leu Ser Gln
145 150 155 160
Glu Pro Val Gln His Gln Asp Gln Thr Asp Ala Ala Gly Ile Asn Gly
165 170 175
Gly Gly Arg Gly Gly Tyr Trp Glu Ala Gly Gln Thr Thr Ile Lys Lys
180 185 190
Gln Gln Gln Arg Arg Arg Lys Lys Arg Leu Tyr Val Ser Glu Thr Asp
195 200 205
Asp Asp Gly Asn Glu Gly Glu Asp Asp Asp Gly Met Asp Ile Val Asn
210 215 220
Gly Ser Gly Val Gly Met Glu Arg Gln Arg Glu His Pro Phe Ile Val
225 230 235 240
Thr Glu Pro Gly Glu Val Ala Arg Gly Lys Lys Asn Gly Leu Asp Tyr
245 250 255
Leu Phe His Leu Tyr Glu Gln Cys Arg Glu Phe Leu Leu Gln Val Gln
260 265 270
Thr Ile Ala Lys Asp Arg Gly Glu Lys Cys Pro Thr Lys Val Thr Asn
275 280 285
Gln Val Phe Arg Tyr Ala Lys Lys Ser Gly Ala Asn Tyr Ile Asn Lys
290 295 300
Pro Lys Met Arg His Tyr Val His Cys Tyr Ala Leu His Cys Leu Asp
305 310 315 320
Glu Glu Ala Ser Asn Ala Leu Arg Ser Ala Phe Lys Val Arg Gly Glu
325 330 335
Asn Val Gly Ser Trp Arg Gln Ala Cys Tyr Lys Pro Leu Val Asp Ile
340 345 350
Ala Cys Arg His Gly Trp Asp Ile Asp Ala Val Phe Asn Ala His Pro
355 360 365
Arg Leu Ser Ile Trp Tyr Val Pro Thr Lys Leu Arg Gln Leu Cys His
370 375 380
Leu Glu Arg Asn Asn Ala Glu Ala Ala Ala Ala Thr Leu Val Gly Gly
385 390 395 400
Ile Ser Cys Arg Asp Arg Leu Arg Leu Asp Ala Leu Gly Phe Asn
405 410 415

<210> 160
<211> 389
<212> PRT
<213> Idahoa scapigera

<400> 160
Met Asp Pro Asp Gly Phe Ala Asn Gly Leu Phe Arg Trp Lys Pro Thr
1 5 10 15
Arg Ala Met Val Gln Ser Pro Pro Pro Val Pro Pro Pro Pro Gln Gln
20 25 30
Gln Gln Thr Ala Ala Ala Glu Ala Phe Gly Met Arg Val Gly Gly Leu
35 40 45
Glu Gly Leu Phe Arg Ala Tyr Gly Ile Arg Phe Tyr Thr Ser Ala Lys
50 55 60
Ile Ala Glu Leu Gly Phe Thr Ala Ser Thr Leu Leu Asn Met Lys Asp
65 70 75 80
Glu Glu Leu Asp Glu Met Met Asn Ser Leu Ser His Ile Phe Arg Trp
85 90 95
Glu Leu Leu Val Gly Glu Arg Tyr Gly Ile Lys Ala Ala Val Arg Ala
100 105 110
Glu Arg Arg Arg Val Gln Glu Glu Glu Glu Glu Glu Ser Ser Arg Arg
115 120 125
Arg His Leu Leu Leu Ser Ala Ala Gly Asp Ser Val Ala His His Ala
130 135 140
Leu Ser Gln Glu Asp Asp Trp Thr Ser Leu Ser Glu Glu Pro Val Gln
145 150 155 160
Gln Lys Asp Gln Thr Asp Ala Ala Gly Ser Asn Gly Gly Gly Val Tyr
165 170 175
Trp Gly Ala Gly Gln Ala Lys Met Lys Gln Lys Arg Arg Lys Lys Pro
180 185 190
Thr Val Met Met Thr Ser Val Glu Thr Asp Asp Glu Ile Asn Glu Cys
195 200 205
Glu Asp Asp Asp Arg Met Asp Asn Gly Asn Gly Gly Met Ala Ile Glu
210 215 220
Arg Gln Arg Glu His Pro Phe Ile Val Thr Glu Pro Gly Glu Val Ala
225 230 235 240
Arg Gly Lys Lys Asn Gly Leu Asp Tyr Leu Phe His Leu Tyr Glu Gln
245 250 255
Cys Arg Glu Phe Leu Leu Gln Val Gln Thr Ile Ala Lys Asp Arg Gly
260 265 270
Glu Lys Cys Pro Thr Lys Val Thr Asn Gln Val Phe Arg Tyr Ala Lys
275 280 285
Lys Ser Gly Ala Ser Tyr Ile Asn Lys Pro Lys Met Arg His Tyr Val
290 295 300
His Cys Tyr Ala Leu His Cys Leu Asp Glu Asn Ala Ser Asn Ala Leu
305 310 315 320
Arg Arg Ser Phe Lys Glu Arg Gly Glu Asn Val Gly Ser Trp Arg Gln
325 330 335
Ala Cys Tyr Lys Pro Leu Val Asp Val Ala Phe Arg His Gly Gly Asp
340 345 350
Ile Asp Ala Val Phe Asn Ala His Pro Arg Leu Ser Ile Trp Tyr Val
355 360 365
Pro Thr Lys Leu Arg Gln Leu Cys His Leu Glu Arg Asn Asn Ala Gly
370 375 380
Ser Ala Thr Ala Ala
385

<210> 161
<211> 399
<212> PRT
<213> Capsella bursa-pastoris

<400> 161
Gly Leu Phe Arg Trp Asn Pro Met Arg Ala Met Val Gln Ala Pro Pro
1 5 10 15
Pro Val Pro Pro Ser Pro Gln Gln Gln Gln Pro Ala Thr Pro Gln Thr
20 25 30

Ala Ala Phe Gly Met Arg Leu Gly Gly Leu Glu Gly Leu Phe Gly Ala
35 40 45
Tyr Gly Ile Arg Phe Tyr Thr Ala Ala Lys Ile Ala Glu Leu Gly Phe
50 55 60
Thr Ala Ser Thr Leu Val Gly Met Lys Asp Glu Glu Leu Glu Glu Met
65 70 75 80
Met Asn Ser Leu Ser His Ile Phe Arg Trp Glu Leu Leu Val Gly Glu
85 90 95
Arg Tyr Gly Ile Lys Ala Ala Val Arg Ala Glu Arg Arg Arg Leu Gln
100 105 110
Glu Glu Glu Glu Glu Ser Ser Arg Arg Arg His Leu Leu Leu Ser Ala
115 120 125
Ala Gly Asp Ser Gly Thr His His Ala Leu Asp Ala Leu Ser Gln Glu
130 135 140
Asp Asp Trp Thr Gly Leu Ser Glu Glu Pro Val Gln Gln Gln Asp Gln
145 150 155 160
Thr Asp Ala Ala Gly Asn Asn Gly Gly Gly Gly Ser Gly Tyr Trp Glu
165 170 175
Ala Gly Gln Ala Lys Met Lys Lys Pro Gln Gln Arg Arg Arg Lys Lys
180 185 190
Pro Met Val Ala Ser Val Glu Thr Asp Asp Asp Gly Asn Glu Gly Glu
195 200 205
Asp Asp Asp Gly Met Asp Asn Gly Asn Gly Gly Ser Gly Gly Met Gly
210 215 220
Thr Glu Arg Gln Arg Glu His Pro Phe Ile Val Thr Glu Pro Gly Glu
225 230 235 240
Val Ala Arg Gly Lys Lys Asn Gly Leu Asp Tyr Leu Phe His Leu Tyr
245 250 255
Glu Gln Cys Arg Glu Phe Leu Leu Gln Val Ile Gln Thr Ile Ala Lys
260 265 270
Asp Arg Gly Glu Lys Cys Pro Thr Lys Val Thr Tyr Gln Val Phe Arg
275 280 285
Tyr Ala Lys Lys Ser Gly Ala Ser Tyr Ile Asn Lys Pro Lys Met Arg
290 295 300
His Tyr Val His Cys Tyr Ala Leu His Cys Leu Asp Glu Asp Ala Ser
305 310 315 320
Asn Ala Leu Arg Arg Ser Phe Lys Glu Arg Gly Glu Asn Val Gly Ser
325 330 335
Trp Arg Gln Ala Cys Tyr Lys Pro Leu Val Asn Ile Ala Cys Arg His
340 345 350
Gly Trp Asp Ile Asp Ala Val Phe Asn Ala His Pro Arg Leu Ser Ile
355 360 365
Trp Tyr Val Pro Thr Lys Leu Arg Gln Leu Cys His Leu Glu Arg Asn
370 375 380
Asn Ala Val Ala Ala Ala Thr Ala Leu Val Gly Gly Ile Ser Cys
385 390 395

<210> 162
<211> 393
<212> PRT
<213> Barbarea vulgaris

<400> 162
Gly Leu Phe Arg Trp Asn Pro Thr Arg Ala Thr Val Gln Ala Leu Pro
1 5 10 15
Pro Val Pro Pro Pro Pro Gln Gln Gln Pro Ala Thr Thr Gln Thr Ala
20 25 30
Ala Phe Gly Met Arg Leu Gly Gly Leu Glu Gly Leu Phe Gly Ala Tyr
35 40 45
Gly Ile Arg Phe Tyr Thr Ala Ala Lys Ile Ala Glu Leu Gly Phe Thr
50 55 60
Ala Ser Thr Leu Val Gly Met Arg Asp Glu Glu Leu Glu Glu Met Met
65 70 75 80

```
Asn Ser Leu Ser His Ile Phe Arg Trp Glu Leu Leu Val Gly Glu Arg
                85                  90                  95
Tyr Gly Ile Lys Ala Ala Val Arg Ala Glu Arg Arg Arg Leu Gln Glu
            100                 105                 110
Glu Glu Glu Glu Glu Ser Ser Arg Arg Arg His Leu Leu Leu Ser Ala
        115                 120                 125
Ala Gly Asp Ser Gly Thr His His Ala Leu Asp Ala Leu Ser Gln Glu
    130                 135                 140
Asp Asp Trp Thr Gly Leu Ser Glu Glu Pro Val Gln Gln Gln Asp His
145                 150                 155                 160
Gln Thr Asp Ala Ala Gly Asn Asn Gly Gly Asn Trp Glu Ala Gly Lys
                165                 170                 175
Gly Lys Met Lys Lys Gln Gln Gln Arg Arg Arg Lys Lys Pro Met Met
            180                 185                 190
Thr Ser Val Glu Thr Asp Asp Asp Ile Asn Glu Gly Glu Asp Glu Asp
        195                 200                 205
Gly Met Asp Asn Gly Asn Gly Gly Gly Gly Gly Gly Gly Leu Gly Thr
    210                 215                 220
Glu Arg Gln Arg Glu His Pro Phe Ile Val Thr Glu Pro Gly Glu Val
225                 230                 235                 240
Ala Arg Gly Lys Lys Asn Gly Leu Asp Tyr Leu Phe His Leu Tyr Glu
                245                 250                 255
Gln Cys Arg Glu Phe Leu Leu Gln Val Gln Thr Ile Ala Lys Asp Arg
            260                 265                 270
Gly Glu Lys Cys Val Thr Asn Gln Val Phe Arg Tyr Ala Lys Lys Ser
        275                 280                 285
Gly Ala Ser Tyr Ile Asn Lys Pro Lys Met Arg Arg Cys Val Arg Cys
    290                 295                 300
Cys Ala Leu His Cys Leu Asp Glu Asp Ala Ser Ser Ala Leu Arg Arg
305                 310                 315                 320
Ala Phe Lys Glu Arg Gly Gly Asn Val Gly Ser Trp Arg Gln Ala Cys
                325                 330                 335
Cys Lys Pro Leu Val Asn Ile Ala Cys Arg His Gly Trp Asp Ile Asp
            340                 345                 350
Ala Val Phe Asn Ala His Pro Arg Leu Ser Ile Trp Tyr Val Pro Thr
        355                 360                 365
Lys Leu Arg Gln Leu Cys His Leu Glu Arg Asn Asn Ala Val Ala Ala
    370                 375                 380
Ala Thr Val Leu Val Gly Gly Ile Ser
385                 390
```

<210> 163
<211> 412
<212> PRT
<213> Petunia hybrida

<400> 163

```
Met Asp Pro Glu Ala Phe Ser Ala Ser Leu Phe Lys Trp Asp Pro Arg
1               5                   10                  15
Gly Ala Met Pro Pro Pro Asn Arg Leu Leu Glu Ala Val Ala Pro Pro
            20                  25                  30
Gln Pro Pro Pro Pro Pro Leu Pro Pro Pro Gln Pro Leu Pro Pro Ala
        35                  40                  45
Tyr Ser Ile Arg Thr Arg Glu Leu Gly Gly Leu Glu Glu Met Phe Gln
    50                  55                  60
Ala Tyr Gly Ile Arg Tyr Tyr Thr Ala Ala Lys Ile Thr Glu Leu Gly
65                  70                  75                  80
Phe Thr Val Asn Thr Leu Leu Asp Met Lys Asp Asp Glu Leu Asp Asp
                85                  90                  95
Met Met Asn Ser Leu Ser Gln Ile Phe Arg Trp Glu Leu Leu Val Gly
            100                 105                 110
Glu Arg Tyr Gly Ile Lys Ala Ala Ile Arg Ala Glu Arg Arg Arg Leu
        115                 120                 125
```

Glu Glu Glu Glu Gly Arg Arg Arg His Ile Leu Ser Asp Gly Gly Thr
    130                 135                 140
Asn Val Leu Asp Ala Leu Ser Gln Glu Gly Leu Ser Glu Glu Pro Val
145                 150                 155                 160
Gln Gln Gln Glu Arg Glu Ala Ala Gly Ser Gly Gly Gly Gly Thr Ala
                165                 170                 175
Trp Glu Val Val Ala Pro Gly Gly Gly Arg Met Arg Gln Arg Arg Arg
            180                 185                 190
Lys Lys Val Val Val Gly Arg Glu Arg Arg Gly Ser Ser Met Glu Glu
        195                 200                 205
Asp Glu Asp Thr Glu Glu Gly Gln Glu Asp Asn Glu Asp Tyr Asn Ile
    210                 215                 220
Asn Asn Glu Gly Gly Gly Gly Ile Ser Glu Arg Gln Arg Glu His Pro
225                 230                 235                 240
Phe Ile Val Thr Glu Pro Gly Glu Val Ala Arg Gly Lys Lys Asn Gly
                245                 250                 255
Leu Asp Tyr Leu Phe His Leu Tyr Glu Gln Cys Arg Asp Phe Leu Ile
            260                 265                 270
Gln Val Gln Asn Ile Ala Lys Glu Arg Gly Glu Lys Cys Pro Thr Lys
        275                 280                 285
Val Thr Asn Gln Val Phe Arg Phe Ala Lys Lys Ala Gly Ala Ser Tyr
    290                 295                 300
Ile Asn Lys Pro Lys Met Arg His Tyr Val His Cys Tyr Ala Leu His
305                 310                 315                 320
Cys Leu Asp Glu Asp Ala Ser Asn Ala Leu Arg Arg Ala Phe Lys Glu
                325                 330                 335
Arg Gly Glu Asn Val Gly Ala Trp Arg Gln Ala Cys Tyr Lys Pro Leu
            340                 345                 350
Val Ala Ile Ala Ala Arg Gln Gly Trp Asp Ile Asp Ala Ile Phe Asn
        355                 360                 365
Gly His Pro Arg Leu Ser Ile Trp Tyr Val Pro Thr Lys Leu Arg Gln
    370                 375                 380
Leu Cys His Ser Glu Arg Ser Asn Ala Ala Ala Ala Ala Ser Thr Ser
385                 390                 395                 400
Val Ser Gly Gly Gly Val Asp His Leu Pro His Phe
                405                 410

<210> 164
<211> 396
<212> PRT
<213> Antirhinum majus

<400> 164
Met Asp Pro Asp Ala Phe Leu Phe Lys Trp Asp His Arg Thr Ala Leu
1               5                   10                  15
Pro Gln Pro Asn Arg Leu Leu Asp Ala Val Ala Pro Pro Pro Pro Pro
            20                  25                  30
Pro Pro Gln Ala Pro Ser Tyr Ser Met Arg Pro Arg Glu Leu Gly Gly
        35                  40                  45
Leu Glu Glu Leu Phe Gln Ala Tyr Gly Ile Arg Tyr Tyr Thr Ala Ala
    50                  55                  60
Lys Ile Ala Glu Leu Gly Phe Thr Val Asn Thr Leu Leu Asp Met Arg
65                  70                  75                  80
Asp Glu Glu Leu Asp Glu Met Met Asn Ser Leu Cys Gln Ile Phe Arg
                85                  90                  95
Trp Asp Leu Leu Val Gly Glu Arg Tyr Gly Ile Lys Ala Ala Val Arg
            100                 105                 110
Ala Glu Arg Arg Arg Ile Asp Glu Glu Glu Val Arg Arg Arg His Leu
        115                 120                 125
Leu Leu Gly Asp Thr Thr His Ala Leu Asp Ala Leu Ser Gln Glu Gly
    130                 135                 140
Leu Ser Glu Glu Pro Val Gln Gln Glu Lys Glu Ala Met Gly Ser Gly
145                 150                 155                 160

Gly Gly Gly Val Gly Gly Val Trp Glu Met Met Gly Ala Gly Gly Arg
165 170 175
Lys Ala Pro Gln Arg Arg Arg Lys Asn Tyr Lys Gly Arg Ser Arg Met
180 185 190
Ala Ser Met Glu Glu Asp Asp Asp Asp Asp Asp Asp Glu Thr Glu Gly
195 200 205
Ala Glu Asp Asp Glu Asn Ile Val Ser Glu Arg Gln Arg Glu His Pro
210 215 220
Phe Ile Val Thr Glu Pro Gly Glu Val Ala Arg Gly Lys Lys Asn Gly
225 230 235 240
Leu Asp Tyr Leu Phe His Leu Tyr Glu Gln Cys Arg Asp Phe Leu Ile
245 250 255
Gln Val Gln Thr Ile Ala Lys Glu Arg Gly Glu Lys Cys Pro Thr Lys
260 265 270
Val Thr Asn Gln Val Phe Arg Tyr Ala Lys Lys Ala Gly Ala Asn Tyr
275 280 285
Ile Asn Lys Pro Lys Met Arg His Tyr Val His Cys Tyr Ala Leu His
290 295 300
Cys Leu Asp Glu Ala Ala Ser Asn Ala Leu Arg Arg Ala Phe Lys Glu
305 310 315 320
Arg Gly Glu Asn Val Gly Ala Trp Arg Gln Ala Cys Tyr Lys Pro Leu
325 330 335
Val Ala Ile Ala Ala Arg Gln Gly Trp Asp Ile Asp Thr Ile Phe Asn
340 345 350
Ala His Pro Arg Leu Ser Ile Trp Tyr Val Pro Thr Lys Leu Arg Gln
355 360 365
Leu Cys His Ala Glu Arg Ser Ser Ala Ala Val Ala Ala Thr Ser Ser
370 375 380
Ile Thr Gly Gly Gly Pro Ala Asp His Leu Pro Phe
385 390 395

<210> 165
<211> 413
<212> PRT
<213> Nicotiana tabacum

<400> 165
Met Asp Pro Glu Ala Phe Ser Ala Ser Leu Phe Lys Trp Asp Pro Arg
1 5 10 15
Gly Ala Met Pro Pro Pro Thr Arg Leu Leu Glu Ala Ala Val Ala Pro
20 25 30
Pro Pro Pro Pro Pro Val Leu Pro Pro Pro Gln Pro Leu Ser Ala Ala
35 40 45
Tyr Ser Ile Arg Thr Arg Glu Leu Gly Gly Leu Glu Glu Leu Phe Gln
50 55 60
Ala Tyr Gly Ile Arg Tyr Tyr Thr Ala Ala Lys Ile Ala Glu Leu Gly
65 70 75 80
Phe Thr Val Asn Thr Leu Leu Asp Met Lys Asp Glu Glu Leu Asp Asp
85 90 95
Met Met Asn Ser Leu Ser Gln Ile Phe Arg Trp Glu Leu Leu Val Gly
100 105 110
Glu Arg Tyr Gly Ile Lys Ala Ala Ile Arg Ala Glu Arg Arg Arg Leu
115 120 125
Glu Glu Glu Glu Leu Arg Arg Arg Ser His Leu Leu Ser Asp Gly Gly
130 135 140
Thr Asn Ala Leu Asp Ala Leu Ser Gln Glu Gly Leu Ser Glu Glu Pro
145 150 155 160
Val Gln Gln Gln Glu Arg Glu Ala Val Gly Ser Gly Gly Gly Gly Thr
165 170 175
Thr Trp Glu Val Val Ala Ala Val Gly Gly Gly Arg Met Lys Gln Arg
180 185 190
Arg Arg Lys Lys Val Val Ser Thr Gly Arg Glu Arg Arg Gly Arg Ala
195 200 205

Ser Ala Glu Glu Asp Glu Glu Thr Glu Glu Gly Gln Glu Asp Glu Trp
    210                 215                 220
Asn Ile Asn Asp Ala Gly Gly Gly Ile Ser Glu Arg Gln Arg Glu His
225                 230                 235                 240
Pro Phe Ile Val Thr Glu Pro Gly Glu Val Ala Arg Gly Lys Lys Asn
                245                 250                 255
Gly Leu Asp Tyr Leu Phe His Leu Tyr Glu Gln Cys Arg Asp Phe Leu
            260                 265                 270
Ile Gln Val Gln Asn Ile Ala Lys Glu Arg Gly Glu Lys Cys Pro Thr
        275                 280                 285
Lys Val Thr Asn Gln Val Phe Arg Tyr Ala Lys Lys Ala Gly Ala Ser
    290                 295                 300
Tyr Ile Asn Lys Pro Lys Met Arg His Tyr Val His Cys Tyr Ala Leu
305                 310                 315                 320
His Cys Leu Asp Glu Glu Ala Ser Asn Ala Leu Arg Arg Ala Phe Lys
                325                 330                 335
Glu Arg Gly Glu Asn Val Gly Ala Trp Arg Gln Ala Cys Tyr Lys Pro
            340                 345                 350
Leu Val Ala Ile Ala Ala Arg Gln Gly Trp Asp Ile Asp Thr Ile Phe
        355                 360                 365
Asn Ala His Pro Arg Leu Ala Ile Trp Tyr Val Pro Thr Arg Leu Arg
    370                 375                 380
Gln Leu Cys His Ser Glu Arg Ser Asn Ala Ala Ala Ala Ala Ser Ser
385                 390                 395                 400
Ser Val Ser Gly Gly Val Gly Asp His Leu Pro His Phe
                405                 410

<210> 166
<211> 416
<212> PRT
<213> Nicotiana tabacum

<400> 166
Met Asp Pro Glu Ala Phe Ser Ala Ser Leu Phe Lys Trp Asp Pro Arg
1               5                   10                  15
Gly Ala Met Pro Pro Pro Thr Arg Leu Leu Glu Ala Ala Val Ala Pro
            20                  25                  30
Pro Pro Pro Pro Pro Ala Leu Pro Pro Pro Gln Pro Leu Ser Ala Ala
        35                  40                  45
Tyr Ser Ile Lys Thr Arg Glu Leu Gly Gly Leu Glu Glu Leu Phe Gln
    50                  55                  60
Ala Tyr Gly Ile Arg Tyr Tyr Thr Ala Ala Lys Ile Ala Glu Leu Gly
65                  70                  75                  80
Phe Thr Val Asn Thr Leu Leu Asp Met Lys Asp Glu Glu Leu Asp Asp
                85                  90                  95
Met Met Asn Ser Leu Ser Gln Ile Phe Arg Trp Glu Leu Leu Val Gly
            100                 105                 110
Glu Arg Tyr Gly Ile Lys Ala Ala Ile Arg Ala Glu Arg Arg Arg Leu
        115                 120                 125
Glu Glu Glu Glu Leu Arg Arg Arg Gly His Leu Leu Ser Asp Gly Gly
    130                 135                 140
Thr Asn Ala Leu Asp Ala Leu Ser Gln Glu Gly Leu Ser Glu Glu Pro
145                 150                 155                 160
Val Gln Gln Gln Glu Arg Glu Ala Val Gly Ser Gly Gly Gly Gly Thr
                165                 170                 175
Thr Trp Glu Val Val Ala Ala Ala Gly Gly Gly Arg Met Lys Gln Arg
            180                 185                 190
Arg Arg Lys Lys Val Val Ala Ala Gly Arg Glu Lys Arg Gly Gly Ala
        195                 200                 205
Ser Ala Glu Glu Asp Glu Glu Thr Glu Glu Gly Gln Glu Asp Asp Trp
    210                 215                 220
Asn Ile Asn Asp Ala Ser Gly Gly Ile Ser Glu Arg Gln Arg Glu His
225                 230                 235                 240

Pro Phe Ile Val Thr Glu Pro Gly Glu Val Ala Arg Gly Lys Lys Asn
245 250 255
Gly Leu Asp Tyr Leu Phe His Leu Tyr Glu Gln Cys Arg Asp Phe Leu
260 265 270
Ile Gln Val Gln Asn Ile Ala Lys Glu Arg Gly Glu Lys Cys Pro Thr
275 280 285
Lys Val Thr Asn Gln Val Phe Arg Tyr Ala Lys Lys Ala Gly Ala Ser
290 295 300
Tyr Ile Asn Lys Pro Lys Met Arg His Tyr Val His Cys Tyr Ala Leu
305 310 315 320
His Cys Leu Asp Glu Glu Ala Ser Asn Ala Leu Arg Arg Ala Phe Lys
325 330 335
Glu Arg Gly Glu Asn Val Gly Ala Trp Arg Gln Ala Cys Tyr Lys Pro
340 345 350
Leu Val Ala Ile Ala Ala Arg Gln Gly Trp Asp Ile Asp Thr Ile Phe
355 360 365
Asn Ala His Pro Arg Leu Ala Ile Trp Tyr Val Pro Thr Lys Leu Arg
370 375 380
Gln Leu Cys His Ser Glu Arg Ser Asn Ala Ala Ala Ala Ala Ala Ser
385 390 395 400
Ser Ser Val Ser Gly Gly Gly Gly Gly Gly Asp His Leu Pro His Phe
405 410 415

<210> 167
<211> 392
<212> PRT
<213> Triticum aestivum

<400> 167
Met Asp Pro Asn Asp Ala Phe Leu Ala Ala His Pro Phe Arg Trp Asp
1 5 10 15
Leu Gly Pro Pro Ala Pro Ala Ala Val Pro Pro Pro Pro Pro Pro Pro
20 25 30
Pro Pro Pro Pro Ala Leu Pro Pro Ala Asn Ala Pro Arg Glu Leu Glu
35 40 45
Asp Leu Val Val Gly Tyr Gly Val Arg Ala Ser Thr Val Ala Arg Ile
50 55 60
Ser Glu Leu Gly Phe Thr Ala Ser Thr Leu Leu Val Met Thr Glu Arg
65 70 75 80
Glu Leu Asp Asp Met Thr Ala Ala Leu Ala Gly Leu Phe Arg Trp Asp
85 90 95
Leu Leu Ile Gly Glu Arg Phe Gly Leu Arg Ala Ala Leu Arg Ala Glu
100 105 110
Arg Gly Arg Leu Met Ser Pro Gly Cys Arg His His Gly Tyr Gln Ser
115 120 125
Gly Ser Thr Ile Asp Gly Ala Ser Gln Glu Val Leu Ser Asn Glu Arg
130 135 140
Asp Gly Ala Ala Ser Gly Gly Ile Gly Glu Glu Asp Ala Met Arg Met
145 150 155 160
Met Ala Ser Gly Lys Lys Gln Lys Asn Gly Ser Ala Gly Arg Lys Ala
165 170 175
Lys Lys Ala Arg Arg Lys Lys Val Asn Asp Leu Arg Leu Asp Met Gln
180 185 190
Gly Asp Glu His Glu Glu Gly Gly Gly Gly Arg Ser Glu Ser Thr Glu
195 200 205
Ser Ser Ala Gly Gly Gly Val Gly Gly Glu Arg Gln Arg Glu His Pro
210 215 220
Phe Val Val Thr Glu Pro Gly Glu Val Ala Arg Ala Lys Lys Asn Gly
225 230 235 240
Leu Asp Tyr Leu Phe His Leu Tyr Glu Gln Cys Arg Leu Phe Leu Leu
245 250 255
Gln Val Gln Ser Met Ala Lys Leu His Gly Gln Lys Ser Pro Thr Lys
260 265 270

Val Thr Asn Gln Val Phe Arg Tyr Ala Ser Lys Val Gly Ala Ser Tyr
275 280 285
Ile Asn Lys Pro Lys Met Arg His Tyr Val His Cys Tyr Ala Leu His
290 295 300
Cys Leu Asp Glu Asp Ala Ser Asp Ala Leu Arg Arg Ala Tyr Lys Ala
305 310 315 320
Arg Gly Glu Asn Val Gly Ala Trp Arg Gln Ala Cys Tyr Ala Pro Leu
325 330 335
Val Asp Ile Ala Ala Arg His Gly Phe Asp Ile Asp Ala Val Phe Ala
340 345 350
Ala His Pro Arg Leu Ala Ile Trp Tyr Val Pro Thr Arg Leu Arg Gln
355 360 365
Leu Cys His Gln Ala Arg Ser Ala His Asp Thr Ala Ala Ala His Ala
370 375 380
Gly Ala Met Pro Pro Pro Met Phe
385 390

<210> 168
<211> 392
<212> PRT
<213> Triticum aestivum

<400> 168
Met Asp Pro Asn Asp Ala Phe Leu Ala Ala His Pro Phe Arg Trp Asp
1 5 10 15
Leu Gly Pro Pro Ala Pro Ala Ala Val Pro Pro Pro Pro Pro Pro Pro
20 25 30
Pro Leu Pro Pro Ala Leu Pro Pro Ala Asn Ala Pro Arg Glu Leu Glu
35 40 45
Asp Leu Val Val Gly Tyr Gly Val Arg Ala Ser Thr Val Ala Arg Ile
50 55 60
Ser Glu Leu Gly Phe Thr Ala Ser Thr Leu Leu Val Met Thr Glu Ser
65 70 75 80
Glu Leu Asp Asp Met Thr Ala Ala Leu Ala Gly Leu Phe Arg Trp Asp
85 90 95
Leu Leu Ile Gly Glu Arg Phe Gly Leu Arg Ala Ala Leu Arg Ala Glu
100 105 110
Arg Gly Arg Leu Met Ser Pro Gly Cys Arg His His Gly Tyr Gln Ser
115 120 125
Gly Ser Thr Ile Asp Gly Ala Ser Gln Glu Val Leu Ser Asn Glu Arg
130 135 140
Asp Gly Ala Ala Ser Gly Gly Ile Gly Glu Asp Asp Ala Met Arg Met
145 150 155 160
Met Ala Ser Gly Lys Lys Gln Lys Asn Gly Ser Ala Ala Arg Lys Ala
165 170 175
Lys Lys Ala Arg Arg Asn Lys Val Lys Glu Leu Arg Leu Asp Met Gln
180 185 190
Gly Asp Glu His Glu Asp Gly Gly Gly Gly Arg Ser Glu Ser Thr Glu
195 200 205
Ser Ser Ala Gly Gly Val Gly Gly Glu Arg Gln Arg Glu His Pro Phe
210 215 220
Val Val Thr Glu Pro Gly Glu Val Ala Arg Ala Lys Lys Asn Gly Leu
225 230 235 240
Asp Tyr Leu Phe His Leu Tyr Glu Gln Arg Arg Leu Phe Leu Leu Gln
245 250 255
Val Gln Ser Met Ala Lys Leu His Gly Gln Lys Ser Pro Thr Lys Val
260 265 270
Thr Asn Gln Val Phe Arg Tyr Ala Ser Lys Val Gly Ala Ser Tyr Ile
275 280 285
Asn Lys Pro Lys Met Arg His Tyr Val His Cys Tyr Ala Leu His Cys
290 295 300
Leu Asp Glu Asp Ala Ser Asp Ala Leu Arg Arg Ala Tyr Lys Ala Arg
305 310 315 320

Gly Glu Asn Val Gly Ala Trp Arg Gln Ala Cys Tyr Ala Pro Leu Val
325 330 335
Asp Ile Ala Ala Arg His Gly Phe Asp Ile Asp Ala Val Phe Ala Ala
340 345 350
His Pro Arg Leu Ala Ile Trp Tyr Val Pro Thr Arg Leu Arg Gln Leu
355 360 365
Cys His Gln Ala Arg Ser Ala His Asp Ala Ala Ala Ala Ala His Ala
370 375 380
Gly Ser Met Pro Pro Pro Met Phe
385 390

<210> 169
<211> 400
<212> PRT
<213> Lolium temulentum

<400> 169
Met Asp Pro His Asp Ala Phe Leu Ala Ala His Pro Phe Arg Trp Asp
1 5 10 15
Leu Gly Pro Pro Ala Pro Ala Ala Val Pro Pro Pro Pro Pro Leu Pro
20 25 30
Met Pro Gln Thr Pro Ala Leu Pro Pro Ala Asn Ser Pro Arg Glu Leu
35 40 45
Glu Asp Leu Val Ala Gly Tyr Gly Val Arg Gly Ala Thr Val Ala Arg
50 55 60
Ile Ser Glu Leu Gly Phe Thr Ala Ser Thr Leu Leu Val Met Thr Asp
65 70 75 80
Arg Glu Leu Asp Asp Met Thr Ala Ala Leu Ala Gly Leu Phe Arg Trp
85 90 95
Asp Leu Leu Ile Gly Glu Arg Phe Gly Leu Arg Ala Ala Leu Arg Ala
100 105 110
Glu Arg Gly Arg Leu Met Ala Leu His Gly Gly Arg His His Gly His
115 120 125
Gln Ser Gly Ser Thr Ile Asp Gly Ala Ser Gln Glu Val Leu Ser Asn
130 135 140
Glu Arg Asp Gly Ala Ala Ser Gly Glu Asp Asp Ala Gly Arg Met Met
145 150 155 160
Leu Ser Gly Lys Lys Leu Lys Asn Gly Ser Val Ala Arg Lys Ala Lys
165 170 175
Lys Ala Arg Arg Lys Lys Val Asp Gly Leu Arg Leu Asp His Met Gln
180 185 190
Glu Asp Glu Arg Glu Asp Gly Gly Gly Arg Ser Glu Ser Thr Glu Ser
195 200 205
Ser Ala Gly Gly Gly Gly Gly Val Gly Gly Glu Arg Gln Arg Glu His
210 215 220
Pro Phe Val Val Thr Glu Pro Gly Glu Val Ala Arg Ala Lys Lys Asn
225 230 235 240
Gly Leu Asp Tyr Leu Phe His Leu Tyr Glu Gln Cys Arg Leu Phe Leu
245 250 255
Leu Gln Val Gln Ser Met Ala Lys Leu His Gly His Lys Ser Pro Thr
260 265 270
Lys Val Thr Asn Gln Val Phe Arg Tyr Ala Ser Lys Val Gly Ala Ser
275 280 285
Tyr Ile Asn Lys Pro Lys Met Arg His Tyr Val His Cys Tyr Ala Leu
290 295 300
His Cys Leu Asp Gln Glu Ala Ser Asp Ala Leu Arg Arg Ala Tyr Lys
305 310 315 320
Ala Arg Gly Glu Asn Val Gly Ala Trp Arg Gln Ala Cys Tyr Ala Pro
325 330 335
Leu Val Asp Ile Ala Ala Gly His Gly Phe Asp Val Asp Ala Val Phe
340 345 350
Ala Ala His Pro Arg Leu Ala Ile Trp Tyr Val Pro Thr Arg Leu Arg
355 360 365

Gln Leu Cys His Gln Ala Arg Ser Ala His Glu Ala Ala Ala Ala Asn
    370                 375                 380
Ala Asn Ala Asn Gly Ala Met Pro Pro Pro Pro Pro Pro Pro Met Phe
385                 390                 395                 400

<210> 170
<211> 389
<212> PRT
<213> Oryza sativa

<400> 170
Met Asp Pro Asn Asp Ala Phe Ser Ala Ala His Pro Phe Arg Trp Asp
1               5                   10                  15
Leu Gly Pro Pro Ala Pro Ala Pro Val Pro Pro Pro Pro Pro Pro Pro
            20                  25                  30
Pro Pro Pro Pro Pro Ala Asn Val Pro Arg Glu Leu Glu Glu Leu Val
        35                  40                  45
Ala Gly Tyr Gly Val Arg Met Ser Thr Val Ala Arg Ile Ser Glu Leu
    50                  55                  60
Gly Phe Thr Ala Ser Thr Leu Leu Ala Met Thr Glu Arg Glu Leu Asp
65                  70                  75                  80
Asp Met Met Ala Ala Leu Ala Gly Leu Phe Arg Trp Asp Leu Leu Leu
                85                  90                  95
Gly Glu Arg Phe Gly Leu Arg Ala Ala Leu Arg Ala Glu Arg Gly Arg
            100                 105                 110
Leu Met Ser Leu Gly Gly Arg His His Gly His Gln Ser Gly Ser Thr
        115                 120                 125
Val Asp Gly Ala Ser Gln Glu Val Leu Ser Asp Glu His Asp Met Ala
    130                 135                 140
Gly Ser Gly Gly Met Gly Asp Asp Asp Asn Gly Arg Arg Met Val Thr
145                 150                 155                 160
Gly Lys Lys Gln Ala Lys Lys Gly Ser Ala Ala Arg Lys Gly Lys Lys
                165                 170                 175
Ala Arg Arg Lys Lys Val Asp Asp Leu Arg Leu Asp Met Gln Glu Asp
            180                 185                 190
Glu Met Asp Cys Cys Asp Glu Asp Gly Gly Gly Gly Ser Glu Ser Thr
        195                 200                 205
Glu Ser Ser Ala Gly Gly Gly Gly Gly Glu Arg Gln Arg Glu His Pro
    210                 215                 220
Phe Val Val Thr Glu Pro Gly Glu Val Ala Arg Ala Lys Lys Asn Gly
225                 230                 235                 240
Leu Asp Tyr Leu Phe His Leu Tyr Glu Gln Cys Arg Leu Phe Leu Leu
                245                 250                 255
Gln Val Gln Ser Met Ala Lys Leu His Gly His Lys Ser Pro Thr Lys
            260                 265                 270
Val Thr Asn Gln Val Phe Arg Tyr Ala Lys Lys Val Gly Ala Ser Tyr
        275                 280                 285
Ile Asn Lys Pro Lys Met Arg His Tyr Val His Cys Tyr Ala Leu His
    290                 295                 300
Cys Leu Asp Glu Glu Ala Ser Asp Ala Leu Arg Arg Ala Tyr Lys Ala
305                 310                 315                 320
Arg Gly Glu Asn Val Gly Ala Trp Arg Gln Ala Cys Tyr Ala Pro Leu
                325                 330                 335
Val Asp Ile Ser Ala Arg His Gly Phe Asp Ile Asp Ala Val Phe Ala
            340                 345                 350
Ala His Pro Arg Leu Ala Ile Trp Tyr Val Pro Thr Arg Leu Arg Gln
        355                 360                 365
Leu Cys His Gln Ala Arg Ser Ser His Ala Ala Ala Ala Ala Ala Leu
    370                 375                 380
Pro Pro Pro Leu Phe
385

<210> 171

<211> 393
<212> PRT
<213> Zea mays

<400> 171

```
Asp Pro Asn Asp Ala Phe Ser Ala Ala His Pro Phe Arg Trp Asp Leu
1               5                   10                  15
Gly Pro Pro Ala Pro Ala Ala Pro Ala Pro Pro Pro Pro Pro Pro Pro
            20                  25                  30
Ala Pro Gln Leu Leu Pro His Ala Pro Leu Leu Ser Ala Pro Arg Glu
        35                  40                  45
Leu Glu Asp Leu Val Ala Gly Tyr Gly Val Arg Pro Ser Thr Val Ala
    50                  55                  60
Arg Ile Ser Glu Leu Gly Phe Thr Ala Ser Thr Leu Leu Gly Met Thr
65                  70                  75                  80
Glu Arg Glu Leu Asp Asp Met Met Ala Ala Leu Ala Gly Leu Phe Arg
                85                  90                  95
Trp Asp Val Leu Leu Gly Glu Arg Phe Gly Leu Arg Ala Ala Leu Arg
            100                 105                 110
Ala Glu Arg Gly Arg Val Met Ser Leu Gly Gly Arg Phe His Thr Gly
        115                 120                 125
Ser Thr Leu Asp Ala Ala Ser Gln Glu Val Leu Ser Asp Glu Arg Asp
    130                 135                 140
Ala Ala Ala Ser Gly Gly Leu Ala Glu Gly Glu Ala Gly Arg Arg Met
145                 150                 155                 160
Val Thr Thr Gly Lys Lys Lys Gly Lys Lys Gly Val Gly Ala Arg Lys
                165                 170                 175
Gly Lys Lys Ala Arg Arg Lys Lys Glu Leu Arg Pro Leu Asp Val Leu
            180                 185                 190
Asp Asp Glu Asn Asp Gly Asp Glu Asp Gly Gly Gly Gly Gly Ser Asp
        195                 200                 205
Ser Thr Glu Ser Ser Ala Gly Gly Ser Gly Gly Gly Glu Arg Gln Arg
    210                 215                 220
Glu His Pro Phe Val Val Thr Glu Pro Gly Glu Val Ala Arg Ala Lys
225                 230                 235                 240
Lys Asn Gly Leu Asp Tyr Leu Phe His Leu Tyr Glu Gln Cys Arg Val
                245                 250                 255
Phe Leu Leu Gln Val Gln Ser Leu Ala Lys Leu Gly Gly His Lys Ser
            260                 265                 270
Pro Thr Lys Val Thr Asn Gln Val Phe Arg Tyr Ala Lys Lys Cys Gly
        275                 280                 285
Ala Ser Tyr Ile Asn Lys Pro Lys Met Arg His Tyr Val His Cys Tyr
    290                 295                 300
Ala Leu His Cys Leu Asp Glu Asp Ala Ser Asn Ala Leu Arg Arg Ala
305                 310                 315                 320
Tyr Lys Ala Arg Gly Glu Asn Val Gly Ala Trp Arg Gln Ala Cys Tyr
                325                 330                 335
Ala Pro Leu Val Glu Ile Ala Ala Arg His Gly Phe Asp Ile Asp Ala
            340                 345                 350
Val Phe Ala Ala His Pro Arg Leu Thr Ile Trp Tyr Val Pro Thr Arg
        355                 360                 365
Leu Arg Gln Leu Cys His Gln Ala Arg Gly Ser His Ala His Ala Ala
    370                 375                 380
Ala Gly Leu Pro Pro Pro Pro Met Phe
385                 390
```

<210> 172
<211> 391
<212> PRT
<213> Zea mays

<400> 172

```
Met Asp Pro Asn Asp Ala Phe Ser Ala Ala His Pro Phe Arg Trp Asp
```

```
1               5                   10                  15
Leu Gly Pro Pro Ala His Ala Ala Pro Ala Pro Ala Pro Pro Pro Pro
            20                  25                  30
Pro Leu Ala Pro Leu Leu Leu Pro Pro His Ala Pro Arg Glu Leu Glu
        35                  40                  45
Asp Leu Val Ala Gly Tyr Gly Val Arg Pro Ser Thr Val Ala Arg Ile
    50                  55                  60
Ser Glu Leu Gly Phe Thr Ala Ser Thr Leu Leu Gly Met Thr Glu Arg
65                  70                  75                  80
Glu Leu Asp Asp Met Met Ala Ala Leu Ala Gly Leu Phe Arg Trp Asp
                85                  90                  95
Val Leu Leu Gly Glu Arg Phe Gly Leu Arg Ala Ala Leu Arg Ala Glu
            100                 105                 110
Arg Gly Arg Val Met Ser Leu Gly Ala Arg Cys Phe His Ala Gly Ser
        115                 120                 125
Thr Leu Asp Ala Ala Ser Gln Glu Ala Leu Ser Asp Glu Arg Asp Ala
    130                 135                 140
Ala Ala Ser Gly Gly Gly Met Ala Glu Gly Glu Ala Gly Arg Arg Met
145                 150                 155                 160
Val Thr Thr Thr Ala Gly Lys Lys Gly Lys Lys Gly Val Val Gly Thr
                165                 170                 175
Arg Lys Gly Lys Lys Ala Arg Arg Lys Lys Glu Leu Arg Pro Leu Asn
            180                 185                 190
Val Leu Asp Asp Glu Asn Asp Gly Asp Glu Tyr Gly Gly Gly Ser Glu
        195                 200                 205
Ser Thr Glu Ser Ser Ala Gly Gly Ser Gly Glu Arg Gln Arg Glu His
    210                 215                 220
Pro Phe Val Val Thr Glu Pro Gly Glu Val Ala Arg Ala Lys Lys Asn
225                 230                 235                 240
Gly Leu Asp Tyr Leu Phe His Leu Tyr Glu Gln Cys Arg Val Phe Leu
                245                 250                 255
Leu Gln Val Gln Ser Ile Ala Lys Leu Gly Gly His Lys Ser Pro Thr
            260                 265                 270
Lys Val Thr Asn Gln Val Phe Arg Tyr Ala Asn Lys Cys Gly Ala Ser
        275                 280                 285
Tyr Ile Asn Lys Pro Lys Met Arg His Tyr Val His Cys Tyr Ala Leu
    290                 295                 300
His Cys Leu Asp Glu Glu Ala Ser Asn Ala Leu Arg Arg Ala Tyr Lys
305                 310                 315                 320
Ser Arg Gly Glu Asn Val Gly Ala Trp Arg Gln Ala Cys Tyr Ala Pro
                325                 330                 335
Leu Val Glu Ile Ala Ala Arg His Gly Phe Asp Ile Asp Ala Val Phe
            340                 345                 350
Ala Ala His Pro Arg Leu Ala Val Trp Tyr Val Pro Thr Arg Leu Arg
        355                 360                 365
Gln Leu Cys His Gln Ala Arg Gly Ser His Ala His Ala Ala Ala Gly
    370                 375                 380
Leu Pro Pro Pro Pro Met Phe
385                 390

<210>  173
<211>  456
<212>  PRT
<213>  Ophrys tenthredinifera

<400>  173
Met Val Leu Ala Thr Ser Gln Gln His His Gln His Asn Pro His Glu
1               5                   10                  15
Val Gln Gln His Leu Gln Pro His Ser Thr Ala Thr Glu Ser Ser Arg
            20                  25                  30
Glu Leu Glu Glu Val Phe Glu Gly Tyr Gly Val Arg Tyr Ser Thr Ile
        35                  40                  45
Ala Arg Ile Gly Asp Leu Gly Phe Thr Ala Ser Thr Leu Ala Gly Met
```

50 55 60
Arg Glu Glu Glu Val Asp Asp Met Met Ala Ala Leu Ser His Leu Phe
65 70 75 80
Arg Trp Asp Leu Leu Val Gly Glu Arg Tyr Gly Ile Lys Ala Ala Ile
85 90 95
Arg Ala Glu Arg Arg Arg Leu Glu Ala Leu Ile Phe Ser His Val Ser
100 105 110
Gly Ala Ala Arg Leu Ser His His Gln His Gln Met Gly Tyr Leu Phe
115 120 125
Ser Ser Ala Thr Thr Gly Tyr His Leu Met Pro Asp Asp Pro Arg Lys
130 135 140
Arg His Leu Leu Leu Ser Pro Asp His His Ser Ala Leu Asp Ala Leu
145 150 155 160
Ser Gln Glu Gly Leu Ser Glu Glu Pro Val Gln Leu Glu Arg Glu Ala
165 170 175
Ala Gly Ser Gly Gly Glu Val Val Gly Arg Arg Asp Gly Lys Gly Lys
180 185 190
Asn Gln Gln Arg Gln Thr Ser Ala Lys Lys Lys Asp Ala Ser Ser Thr
195 200 205
Lys Ser Lys Lys Lys Lys Lys Lys Gly Ile Glu Glu Gly Asp Asp Glu
210 215 220
Glu Glu Glu Val Glu Val Trp Gly Arg Gly Ala Ser Ile Glu Asn Asp
225 230 235 240
Glu Asp Asp Asp Gly Asp Glu Ser Gln Ser Glu Gln Ser Ser Ala Ala
245 250 255
Glu Arg Gln Arg Glu His Pro Phe Ile Val Thr Glu Pro Gly Glu Val
260 265 270
Ala Arg Ala Lys Lys Asn Gly Leu Asp Tyr Leu Phe Asn Leu Tyr Glu
275 280 285
Gln Cys His Glu Phe Leu Asn Gln Val Gln Ser Val Ala Lys Glu Arg
290 295 300
Gly Asp Lys Cys Pro Thr Lys Val Thr Asn Leu Val Phe Arg Tyr Ala
305 310 315 320
Lys Lys Lys Val Gly Ala Ser Tyr Ile Asn Lys Pro Lys Met Arg His
325 330 335
Tyr Val His Cys Tyr Ala Leu His Val Leu Asp Glu Asp Ala Ser Asn
340 345 350
Ser Leu Arg Arg Ala Phe Lys Glu Arg Gly Glu Asn Val Gly Ala Trp
355 360 365
Arg Leu Ala Cys Tyr Lys Pro Leu Val Ala Ile Ser Ala Ser His Ser
370 375 380
Phe Asp Ile Asp Ala Val Phe Asn Ala His Pro Arg Leu Ser Ile Trp
385 390 395 400
Tyr Val Pro Thr Lys Leu Arg Gln Leu Cys His Leu Ala Arg Ser Ser
405 410 415
Thr Ser Gln Phe Pro Leu Ala Val Pro Arg Thr Thr Gly Ser Ser Asn
420 425 430
Gln Arg Val Ser Ser Thr Val His Val Val Glu Asp Ser Ala Ala Ala
435 440 445
His Ser Phe Arg Pro Pro Met Phe
450 455

<210> 174
<211> 412
<212> PRT
<213> Lycopersicon esculentum

<400> 174
Met Asp Pro Asp Ala Phe Ser Ala Ser Leu Phe Lys Trp Asp Pro Arg
1 5 10 15
Gly Ala Met Pro Pro Pro Ser Arg Leu Leu Glu Pro Val Ala Pro Pro
20 25 30
Gln Pro Pro Pro Ser Leu Pro Pro Pro Pro Pro Pro Gln Pro Leu Pro

```
        35                  40                  45
Thr Ser Ser Tyr Ser Ile Arg Ser Thr Arg Glu Leu Gly Gly Leu Glu
    50                  55                  60
Glu Leu Phe Gln Ala Tyr Gly Ile Arg Tyr Tyr Thr Ala Ala Lys Ile
65                  70                  75                  80
Ala Glu Leu Gly Phe Thr Val Asn Thr Leu Leu Asp Met Lys Asp Glu
                85                  90                  95
Glu Leu Asp Asp Met Met Asn Ser Leu Ser Gln Ile Phe Arg Trp Asp
            100                 105                 110
Leu Leu Val Gly Glu Arg Tyr Gly Ile Lys Ala Ala Ile Arg Ala Glu
        115                 120                 125
Trp Arg Arg Leu Glu Glu Glu Glu Ala Arg Arg Arg Gly His Ile Leu
    130                 135                 140
Ser Asp Gly Gly Thr Asn Val Leu Asp Ala Leu Ser Gln Glu Gly Leu
145                 150                 155                 160
Ser Glu Glu Pro Val Gln Gln Gln His Glu Arg Glu Ala Ala Gly Ser
                165                 170                 175
Gly Gly Gly Gly Thr Trp Glu Val Ala Ala Gly Gly Gly Gly Arg Met
            180                 185                 190
Lys Gln Arg Arg Arg Lys Lys Ala Gly Arg Glu Arg Arg Gly Glu Glu
        195                 200                 205
Asp Glu Glu Thr Glu Glu Leu Gly Glu Glu Asp Glu Glu Asn Met Asn
    210                 215                 220
Gln Gly Gly Gly Gly Gly Gly Ile Ser Glu Arg Gln Arg Glu His Pro
225                 230                 235                 240
Phe Ile Val Thr Glu Pro Gly Glu Val Ala Arg Gly Lys Lys Asn Gly
                245                 250                 255
Leu Asp Tyr Leu Phe His Leu Tyr Glu Gln Cys Arg Asp Phe Leu Ile
            260                 265                 270
Gln Val Gln Thr Ile Ala Lys Glu Arg Gly Glu Lys Cys Pro Thr Lys
        275                 280                 285
Val Thr Asn Gln Val Phe Arg Tyr Ala Lys Lys Ala Gly Ala Ser Tyr
    290                 295                 300
Ile Asn Lys Pro Lys Met Arg His Tyr Val His Cys Tyr Ala Leu His
305                 310                 315                 320
Cys Leu Asp Glu Asp Ala Ser Asn Ala Leu Arg Arg Ala Phe Lys Glu
                325                 330                 335
Arg Gly Glu Asn Val Gly Ala Trp Arg Gln Ala Cys Tyr Lys Pro Leu
            340                 345                 350
Val Ala Ile Ala Ala Arg Gln Gly Trp Asp Ile Asp Ala Ile Phe Asn
        355                 360                 365
Ala His Pro Arg Leu Ala Ile Trp Tyr Val Pro Thr Lys Leu Arg Gln
    370                 375                 380
Leu Cys His Ser Glu Arg Ser Asn Ala Ala Ala Ala Ala Ser Ser Ser
385                 390                 395                 400
Val Ser Gly Gly Val Ala Asp His Leu Pro His Phe
                405                 410

<210>  175
<211>  367
<212>  PRT
<213>  Carica papaya

<400>  175
Met Asp Pro Asp Gly Phe Ser Ser Ser Leu Phe Lys Trp Asp Pro Thr
1               5                   10                  15
Arg Gly Ile Val Gln Ala Pro Val Arg Leu Leu Glu Ala Val Ala Ala
            20                  25                  30
Ala Pro Thr Gln Ala Ala Tyr Gly Val Arg Pro Arg Glu Leu Gly Gly
        35                  40                  45
Leu Glu Glu Leu Phe Gln Asp Tyr Gly Ile Arg Tyr Phe Thr Ala Ala
    50                  55                  60
Lys Ile Ala Glu Leu Gly Phe Thr Ala Ser Thr Leu Val Asp Met Lys
```

```
65                  70                  75                  80
Asp Glu Glu Leu Asp Glu Met Met Asn Ser Leu Ser Gln Ile Phe Arg
                85                  90                  95
Trp Glu Leu Leu Val Gly Glu Arg Tyr Gly Ile Lys Ala Ala Val Arg
            100                 105                 110
Ala Glu Arg Arg Arg Leu Asp Asp Asp Asp Ser Arg Arg Arg Gln Thr
        115                 120                 125
Leu Ser Thr Asp Thr Thr His Ala Leu Asp Ala Leu Ser Gln Glu Gly
    130                 135                 140
Leu Ser Glu Glu Pro Val Gln Gln Glu Lys Glu Ala Ala Gly Ser Gly
145                 150                 155                 160
Gly Gly Thr Ile Trp Glu Val Gly Pro Gly Lys Lys Lys Gln Arg Arg
                165                 170                 175
Arg Lys Val Val Gly Glu Glu Glu Gln Glu Glu Glu Asn Gly Gly Gly
            180                 185                 190
Ser Glu Arg Gln Arg Glu His Pro Phe Ile Val Thr Glu Pro Gly Glu
        195                 200                 205
Val Ala Arg Gly Lys Lys Asn Gly Leu Asp Tyr Leu Phe His Leu Tyr
    210                 215                 220
Glu Gln Cys Arg Asp Phe Leu Ile Gln Val Gln Asn Ile Ala Lys Glu
225                 230                 235                 240
Arg Gly Glu Lys Cys Pro Thr Lys Val Thr Asn Gln Val Phe Arg Tyr
                245                 250                 255
Ala Lys Lys Ala Gly Ala Ser Tyr Ile Asn Lys Pro Lys Met Arg His
            260                 265                 270
Tyr Val His Cys Tyr Ala Leu His Cys Leu Asp Glu Lys Glu Ser Asn
        275                 280                 285
Ala Leu Arg Thr Ala Phe Lys Glu Arg Gly Glu Asn Val Gly Ser Trp
    290                 295                 300
Arg Gln Ala Cys Tyr Lys Pro Leu Val Ala Ile Ala Ala Arg Gln Gly
305                 310                 315                 320
Trp Asp Ile Asp Ala Ile Phe Asn Ala His Pro Arg Leu Ala Ile Trp
                325                 330                 335
Tyr Val Pro Asn Lys Leu Arg Gln Leu Cys His Ala Glu Arg Asn Asn
            340                 345                 350
Thr Ala Ile Ala Ser Thr Ser Ala Ala Ala His His Leu Pro Phe
        355                 360                 365
```

<210> 176
<211> 1263
<212> DNA
<213> Arabidopsis thaliana

<400> 176

```
atggatcctg aaggtttcac gagtggctta ttccggtgga acccaacgag agcattggtt      60
caagcaccac ctccggttcc acctccgctg cagcaacagc cggtgacacc gcagacggct     120
gcttttggga tgcgacttgg tggtttagag ggactattcg gtccgtacgg tatacgtttc     180
tacacggcgg cgaagatagc ggagttaggt tttacggcga gcacgcttgt gggtatgaag     240
gacgaggagc ttgaagagat gatgaatagt ctctctcata tctttcgttg ggagcttctt     300
gttggtgaac ggtacggtat caaagctgcc gttagagctg aacggagacg attgcaagaa     360
gaggaggaag aggaatcttc tagacgccgt catttgctac tctccgccgc tggtgattcc     420
ggtactcatc acgctcttga tgctctctcc caagaagggt tatctgagga accggtgcag     480
caacaagacc agactgatgc ggcggggaat aacggcggag gaggaagtgg ttactgggac     540
gcaggtcaag gaaagatgaa gaagcaacag cagcagagac ggagaaagaa accaatgctg     600
acgtcagtgg aaaccgacga agacgtcaac gaaggtgagg atgacgacgg gatggataac     660
ggcaacggag gtagtggttt ggggacagag agacagaggg agcatccgtt tatcgtaacg     720
gagcctgggg aagtggcacg tggcaaaaag aacggcttag attatctgtt ccacttgtac     780
gaacaatgcc gtgagttcct tcttcaggtc cagacaattg ctaaagaccg tggcgaaaaa     840
tgccccacca aggtgacgaa ccaagtattc aggtacgcga agaaatcagg agcgagttac     900
ataaacaagc ctaaaatgcg acactacgtt cactgttacg ctctccactg cctagacgaa     960
gaagcttcaa atgctctcag aagagcgttt aaagaacgcg gtgagaacgt tggctcatgg    1020
cgtcaggctt gttacaagcc acttgtgaac atcgcttgtc gtcatggctg ggatatagac    1080
gccgtcttta acgctcatcc tcgtctctct atttggtatg ttccaacaaa gctgcgtcag    1140
```

ctttgccatt tggagcggaa caatgcggtt gctgcggctg cggctttagt tggcggtatt 1200
agctgtaccg gatcgtcgac gtctggacgt ggtggatgcg gcggcgacga cttgcgtttc 1260
tga 1263

<210> 177
<211> 1263
<212> DNA
<213> Brassica juncea

<400> 177
atggatcctg aaggtttcac gagtggctta ttccggtgga acccaacgag agcattggtt 60
caagcaccac ctccggttcc acctccgctg cagcaacagc cggtgacacc gcagacggct 120
gcttttggga tgcgacttgg tggtttagag ggactattcg gtccatacgg tatacgtttc 180
tacacggcgg cgaagatagc ggagttaggc tttacggcga gcacgcttgt gggtatgaag 240
gacgaggagc ttgaagagat gatgaatagt ctctctcata tctttcgttg ggagcttctt 300
gttggtgaac ggtacggtat caaagctgcc gttagagctg aacggagacg attgcaagaa 360
gaggaggagg aggaatcttc tagacgccgt catttgctac tctccgccgc tggtgattcc 420
ggtactcatc acgctcttga tgctctctcc caagaagagt tatctgagga accggtgcag 480
caacaagacc agactgatgc ggcggggaat aacggcggag gaggaagtgg ttactgggac 540
gcaggtcaag gaaagatgaa gaagcaacag cagcagagac ggagaaagaa accaatgctg 600
acgtcagtgg aaaccgacga agacgtcaac gaaggtgagg atgacgacgg gatggataac 660
ggcaacggag gtagtggttt ggggacagag agacagaggg agcatccgtt tatcgtaacg 720
gagcctgggg aagtggcacg tggcaaaaag aacggcttag attatctgtt ccacttgtac 780
gaacaatgcc gtgagttcct tcttcaggtc cagacaattg ctaaagaccg tggcgaaaaa 840
tgccccacca aggtgacgaa ccaagtattc aggtacgcga agaaatcagg agcgagttac 900
ataaacaagc ctaaaatgcg acactacgtt cactgttacg ctctccactg cctagacgaa 960
gaagcttcaa atgctctcag aagagcgttt aaagaacgcg gtgagaacgt tggctcatgg 1020
cgtcaggctt gttacaagcc acttgtgaac atcgcttgtc gtcatggctg ggatatagac 1080
gccgtcttta acgctcatcc tcgcctctct atttggtatg ttccaacaaa gctgcgtcag 1140
ctttgccatt tggagcggaa caatgcggtt gctgcggctg cggctttagt tggcggtatt 1200
agctgtaccg gatcgtcgac gtctggacgt ggtggatgcg gcggcgacga cttgcgtttc 1260
tag 1263

<210> 178
<211> 1281
<212> DNA
<213> Ionopsidium acaule

<400> 178
atggaccccg aaggtttcac gagtggctta ttccgatgga acacaacaag agcaatggtt 60
caacatcaac caccaccaca agtccctcct cctccgtcgc agcaatctcc ggtaacacca 120
caaacggcgg cgtttgggat gagattaggt ggtctagaag gtttgttcgg tccttacggg 180
atacgttttt acacggcggc gaagatagcc gagttaggtt tcacggcgag cacgctcgtt 240
ggtatgaaag acgaagagct tgaagatatg atgaatagtc tctctcatat ctttcgttgg 300
gagcttcttg ttggtgaacg ttacggtatc aaagctgccg ttagagctga acggaggaga 360
ttgcaagaag aggaggagga tgattcttct agacgccgtc atttgcttct ctccgccgct 420
ggtgattccg gcactcacca cgctcttgat gctctctctc aagaagatga ttggacaggc 480
ttatcagagg aaccggtgca tcaagaccaa actgacgcgg cgggtaacgg cggattcggt 540
ggttatttgg aatcatcagt acacggaaag atgaagaaac atcaaccaag acgtagaaag 600
aaaccgttgg tactgacgtc agttgaaacc gacgatgacg gcaacgataa cgaggatgac 660
gacgggatgg ataacggtaa cggaggtatt gggttaggga cggagagaca gagagaacat 720
ccgtttattg taactgagcc tggggaagtg gcacgtggca aaaagaacgg tttggattat 780
cttttccact tgtacgaaca atgccgtgag ttccttcttc aggtccagac tattgctaaa 840
gaccgtggcg aaaaatgccc caccaaggtg acgaaccaag tgtttaggta cgctaaaaaa 900
tcaggagcga gttacataaa caaaccaaaa atgcgacact acgtccattg ctacgctctc 960
cactgcctag acgaagaagc atcaaacgct cttagaagag cgtttaaaga acgcggcgag 1020
aacgttggct cgtggcgtca ggcttgttac aagccgctag tgaacatagc ctgtcgtcat 1080
ggctgggaca tagacgccgt tttcaacgca catcctcgtc tatctatttg gtacgttcca 1140
actaaactgc gtcagctttg ccatttggag cgtaacaacg ccgttgctgc ggcggctgct 1200
ttggttggtg gtattagctg caccggctct tctgcgtctg gacgcggtgg ttgcggcggc 1260
gacgaggagt tacgttacta g 1281

<210> 179

<211> 1254
<212> DNA
<213> Leavenworthia crassa

<400> 179
atggatcctg aaggtttcac gagtggctta ttccgatgga acccaacgag agcaacggtt 60
caagcactac ctccggttcc tcctccacta cagcaacagc cagcaacagt acagtcagcg 120
gcttttggga cgcgacttgg tggtttagag ggacttttcg gtgtttatgg gatacgtttt 180
tacacggcgg cgaagatagc cgagttaggt tttacggcga gcacgcttgt gggtatgagg 240
gatgaggagc ttgaggaaat gatgaatagc ctctctcata tctttcggtg ggagcttctt 300
gttggtgaac ggtacggtat caaagctgcc gttagagctg aacggagaag attgcaagaa 360
gaagaggagg aggaatcttc tagacgacgt catttgttac tctccgccgc aggtgattcc 420
ggcactcatc acgctcttga tgctctctcc caagaagatg attggacagg tttatcagag 480
gagccggtac agcaaataga tcacctgact gatgcggtgg ggaataacgg tggttattgg 540
gaagcaaaca aaggaaagat gaagaagcaa caacaaagaa ggagaaagaa accgatgctg 600
acatcagttg aaacagacga tgacatcaac gaaggtgagg atgaagatgg aatggataac 660
agtaacggag gattagggac agagagacaa agggagcatc cgtttattgt aacggagcct 720
ggggaagtag cacgtggcaa aaagaacggt ttagattacc tcttccattt gtacgaacaa 780
tgtcgtgagt tccttcttca ggttcagaca atagctaaag atcgtggcga gaaatgtccc 840
accaaggtga cgaaccaagt gtttaggtac gcaaaaaaat caggagcaag ttacataaac 900
aagcccaaaa tgcgacacta cgtccactgt tacgctcttc actgcttaga cgaagaagcc 960
tcaaacgctc tccgacgagc gttcaaggaa cgcggtgaga acgttggctc ttggcgtcag 1020
gcttgttaca agccacttgt gaacatcgct tgtcgtcatg gttgggatat agacgccgtc 1080
tttaactctc atcctcgtct ctctatttgg tatgtcccaa ccaagctgcg tcagctctgt 1140
catatggaga ggaacaatga ggttgctgca gctacggttt tggttggcgg tattagctgt 1200
acgggaacgt cagcgtctgg acacggtgaa tgtggaggcg agttacatta ttag 1254

<210> 180
<211> 1210
<212> DNA
<213> Selenia aurea

<400> 180
atggatcctg aaggtttcac gagtggctta ttccgatgga acccaacgag agcaacggtt 60
caagcactag ctccggttcc tcctccattg cagcaacaac cagcaacagc acagacggcg 120
gcttttggga tgcgacttgg tggtttagaa ggactctttg gtgcttacgg aatacgtttt 180
tacacggcgg cgaagatagc agagttaggt tttacggcga gcacgcttgt gggtatgagg 240
gacgaggagc ttgaggaaat gatgaatagt ctctctcata tctttcggtg ggagcttctt 300
gttggtgaac ggtacggtat caaagctgcc gttagagctg aacgaagaag attgcaggag 360
gaagaggaag aggaatcttc tagacgacgt catttgctac tctccgccgc aggtgattcc 420
ggcactcatc acgctcttga tgctctctcc caagaagatg attggacagg cttatcagaa 480
gagccggtgc agcagcaaga tcatcagact gatgcggtgg gtaataacgg cggttactgg 540
gatgaaggta aaggaaagat gaagaagcaa caacaaagaa ggaggatgaa accgttgatg 600
acgtcagtgg aacccgacaa tgacatggac gagtgtgagg atgaagatag gatggataac 660
ggtaacggag gaggtggtgg attggggatg gagagacaga gggagcatcc gtttattgta 720
acggagcctg gggaagtggc acgtggcaaa aagaacggtt tagattacct gttccatttg 780
tacgaacaat gccgtgagtt ccttcttcag gtccaattaa ttgccaaaga tcgtggcgag 840
aaatgcccta ccaaggtgac gaaccaagtg tttaggtacg cgaagaaatc aggagcgagt 900
tacataaaca agcctaaaat gagacactac gtccactgtt acgctttaca ctgcttagat 960
gaagacgcct caaacgctct ccgacgagcg ttcaaggaac gcggtgagaa cgttgggtca 1020
tggcgtcagg ctcgttacaa gccacttgtg gatatcgctt gtcgtcatgg ctgggatata 1080
gacgccgtct ttaacgctca tcctcgtctc tctatttggt atgttcctac caagctacgt 1140
cagctctgcc atttggagag aaacaatgcg gttgcggctg ctgcggtttt agttggcggt 1200
attagctgta 1210

<210> 181
<211> 1293
<212> DNA
<213> Arabidopsis lyrata

<400> 181
atggatcctg aaggtttcac gagtggctta ttccgatgga acccaacgag agcaatggtt 60
gcagcaccac ctccggttcc acctcagccg cagcaacagc cggcaacacc tcagacgcgc 120

gcttttggga tgcgacttgg tggtttagag ggactgttcg gagcttacgg tatacgtttt 180
tacacggcgg cgaagatagc ggagttaggt tttacggcga gcacgcttgt gggtatgaag 240
gacgaggagc ttgaggagat gatgaatagt ctctctcaca tctttcgttg ggagcttctt 300
gttggtgaac ggtacggtat caaagctgcc gttacagctg aacggagacg attgcaagaa 360
gaggaggagg aggaatcttc tagacgccgt catttgctac tctccgccgc tggtgattcc 420
ggtactcatc acgctcttga tgctctctcc caagaagatg attggacagg gttatctgag 480
gaactggaca gggaaccggt gcaacagcaa aaccagacag atgcggctgg gaataacggc 540
ggaggaggaa gtggttactg ggaagcaggt caagcaaaga tgaagaagca acagcagcag 600
agacggagaa agaaaccgat ggtgacgtca gtggaaaccg acgatgacgt caacgaaggt 660
gatgatgacg acgggatgga taacggcaac ggaggtggtg gtggtggatt ggggacagag 720
agacagaggg agcacccgtt tatcgtaacg gagcctgggg aagtggcacg tggcaaaaag 780
aacggtttag attatctgtt ccacttgtac gaacaatgcc gtgagttcct tcttcaggtc 840
cagacaattg ctaaagaccg tggcgaaaaa tgtcccacca aggtgacgaa ccaagtgttc 900
aggtacgcga agaaatcggg agcgagttac ataaacaagc ccaaaatgcg acactacgtc 960
cactgttacg ctctccactg cctagacgaa gacgcttcaa acgctctccg aagagcgttt 1020
aaagaacgcg gtgagaacgt tggctcgtgg cgtcaggctt gttacaagcc acttgtgaac 1080
attgcttgtc gtcatggctg ggatatagac gccgtcttta acgctcatcc tcgtctctct 1140
atttggtacg ttccaactaa gctgcgtcag ctttgccatt tggagcggaa caatgccgtg 1200
gctgcggccg cggcgttggt tggcggtatt agctgtaccg gatcgtctac gtctggccgt 1260
ggtggttgcg gcggcgacga cttgcgtttc tag 1293

<210> 182
<211> 1209
<212> DNA
<213> Streptanthus glandulosus

<400> 182
agtggcttat tccgatggaa ctcaacgaga gcactggttc aacaaccacc tccagttcct 60
ccaccgcagc agcaaccgcc ggaaacaccg cagacggtag cgtttggaat gcgactaggt 120
gggttggagg gtttgttcgg tgcttacgga atacgttttt acacggcggc aaagatagcg 180
gagttaggtt ttacggctag cacgcttgtt ggcatgaagg acgaggagct tgaggatatg 240
atgaatagcc tctctcatat ctttcgttgg gaacttctcg tcggtgaacg gtacggtatc 300
aaagctgccg ttagagctga acggagacga ttgcaagaag tggaagagga ggaatcttct 360
agacgccgtc atttgctact ctgcgccgca ggtgattcag gcactcatca cgctcttgat 420
actctctcac aagaagatta ttggacagga ttatcagagg agccggggca gcagcaagat 480
cagactgatg cggcgggaaa caacggcgga aacggcggag gagaaggagg aggctattgg 540
gaagcagggc aggcgaagat gaagaagcca cagcaaagac gtagaaagaa atcgatggtg 600
acgtcagtgg aaatcgatga tgaatgcaac gaaggtgagg atgacgatgg gatggataac 660
tgtaacggag gaggtggtgg gttggggata gagagacaaa gggagcatcc gtttatagta 720
acggagccag gggaagtggc acgtggcaaa aagaacggtt tggattatct tttccacttg 780
tacgaacaat gccgcgagtt ccttcttcag gtccagacaa ttgctaaaga ccgtggcgaa 840
aaatgcccca ccaagggtac gaaccaagtt ttcaggtacg caaagaattc gggagcgagt 900
tacataaaca agccgaaaat gcgacactac gttcattgtt acgcactcca ctgcctcgac 960
gaagaagctt caaacgctct ccgaagagcg tttaaagaac gcggtgagaa cgttgggtcg 1020
tggcgtcagg cctgttacaa gccacttgtg aacatcgctt gtcgtcatgg ctgggatata 1080
gacgccgttt ttaacgctca tcctcacctc tccatttggt atgttcccac taagctgcgt 1140
cagctctgcc atttagagcg gaacaatgcg gttgctgcgg ctgcagcttt agttggcggt 1200
attagctgt 1209

<210> 183
<211> 1221
<212> DNA
<213> Cochlearia officinalis

<400> 183
atggatcctg aaggtttcac gaatggctta ttccgatgga acacaacaag agcaatgatt 60
caacaacaac aacaattacc accgcctcaa atcactcctc cgccgcaaca atcaccggca 120
acaccacaaa cggcggcgtt tgggatgaga ctaggtggtt tagaaggttt gttcggtcct 180
tacgggatac gtttttacac ggcggcgaag atagctgagc taggtttcac ggcgagcacg 240
cttgttggta tgaaagacga agagcttgaa gatatgatga atagtctctc acatatcttt 300
cgttgggagc ttcttgtcgg tgaacgttac ggtatcaaag ctgccgttag aactgaacgg 360
aggagattgc aagaagagga agaggaggaa tcttctagac gccgtcattt tatgctctcc 420
gccggtggtg attccggcac tcaccacgct cttgatgctc tctctcaaga agatgattgg 480

acaggtttat cagaggaacc ggtgcatcaa gaccaaactg acgcggcggg taacggcgga 540
ttcggtggtt atttagaatc aggacacggt aaaatgaaga aacagcaaca acaaaaacgt 600
agaaagaaac cgttagtgac gtcagtggaa acagacgatg acggtaacga tgatgacgac 660
gggatggata acggtaacgg agggagtagc gggttgggaa cggagagaca gagagaacat 720
ccgtttatcg taacggagcc tggggaagtg gcacgtggca aaaagaacgg tttggattat 780
cttttccact tgtacgaaca atgccgtgag tttcttcttc aggttcagac tattgctaaa 840
gaccgtggcg aaaaatgtcc caccaaggtg acgaaccaag tgtttaggta cgccaaaaaa 900
tcaggagcga gttacataaa caaaccaaaa atgcgacact acgtccattg ttacgcccta 960
cactgcctag acgaagacgc ttcaaacgct ctcagaagag cgttcaaaga acgcggcgaa 1020
aacgttggct cgtggcggca ggcctgttat aaaccgctag tcaacatcgc gtgccgtcac 1080
ggttgggaca tagacgccgt tttcaacgca catccacgtc tatctatttg gtacgttccg 1140
acaaaactgc gtcagctttg ccatttggag cgtaacaacg cggttgctgc ggcttcggct 1200
ttagttggcg gtattagctg t 1221

<210> 184
<211> 1248
<212> DNA
<213> Brassica oleracea

<400> 184
atggatcctg aaggtttcac gagtggctta ttccgatgga acccaactag agtaatggtt 60
caagcaccaa ctccgattcc tccaccgcag cagcaatcgc cagcaacacc gcagaccgca 120
gcgtttggaa tgcgactagg tggtttggag ggtttgttcg gtccttacgg tgtacgtttt 180
tacacggcgg caaagatagc tgagttaggt tttacggcga gcacactggt gggcatgaag 240
gacgaggagc ttgaggatat gatgaatagc ctctctcata tctttcgttg ggagcttctc 300
gtcggtgaac ggtacggcat caaagctgcc gttagagctg aacggagacg attgcaagaa 360
gaggaagagg aggaatcgtc tagacgccgt catttgctac tctccgccgc aggtgattcc 420
ggcactcatc ttgctcttga tgctctctcc caagaagatg actggacagg gttgtcacag 480
gagccggttc agcaccaaga tcagactgat gcggcgggga tcaacggcgg aggaagagga 540
ggttattggg aagcagggca gacgacaata aaaaagcaac agcagagacg cagaaagaag 600
cgattgtacg tcagtgaaac tgatgatgac ggcaacgaag gtgaggatga cgacgggatg 660
gatattgtta acggaagtgg tgtagggatg gagagacaaa gggagcaccc gtttattgta 720
acggagccag gggaagtagc acgtggcaaa aagaacggtt tggattatct tttccacttg 780
tacgaacagt gccgcgagtt ccttcttcag gtccagacca ttgctaaaga tcgtggcgaa 840
aaatgcccta ccaaggtgac gaaccaggtg ttcaggtacg ctaagaaatc gggggcaaat 900
tacataaata agccaaaaat gcgacactac gttcattgtt acgcactcca ttgcctcgac 960
gaagaagctt caaacgctct ccgaagtgcg tttaaagttc gcggtgagaa cgttgggtcg 1020
tggcgtcagg cttgttacaa gccacttgtg gacattgctt gtcgtcatgg ctgggatata 1080
gacgccgttt ttaacgctca tcctcgcctt tccatttggt atgttcccac taagctgcgt 1140
cagctctgcc atttggagcg gaacaatgcg gaagcagcgg cagcgacttt ggttggtggt 1200
attagctgca gggatcgcct gcgtctggac gctttggggt ttaattag 1248

<210> 185
<211> 1167
<212> DNA
<213> Idahoa scapigera

<400> 185
atggatcctg atggtttcgc gaatggttta ttccgatgga aaccaacgag agcaatggtt 60
caatcaccac ctcctgttcc tcctccacct cagcaacaac agacggcggc tgcagaggct 120
tttgggatgc gagtaggcgg tttagaaggt ctcttccgtg cttacggtat acgtttttac 180
acgtcggcga aaatagcgga gttaggtttt acggcgagca cacttctgaa tatgaaggat 240
gaagagcttg atgaaatgat gaatagcctt tctcatatct ttcggtggga gcttcttgtc 300
ggtgaacggt acggtatcaa agctgccgtt agagctgaaa ggagacgagt gcaagaagaa 360
gaggaggaag aatcttctcg acggcgtcat ttgttactct ccgctgccgg ggattccgtc 420
gctcatcacg ctctctctca agaagatgac tggacaagct tgtcagagga gccggtgcag 480
caaaaagatc agactgatgc ggcggggagt aacggtggag gagtttattg gggggcaggt 540
caagcaaaga tgaagcaaaa acggagaaag aaaccgacgg tgatgatgac gtcagtggaa 600
acagatgacg aaattaacga atgtgaggat gacgacagga tggataacgg taacggtgga 660
atggcgatag agagacagag agagcatccg tttattgtaa cggagcctgg ggaagtggca 720
cgtggcaaaa agaacggttt ggattatttg tttcatttgt acgaacaatg ccgtgagttc 780
cttcttcagg ttcagacaat tgctaaagac cgtggcgaaa aatgccccac caaggtgaca 840
aaccaagtgt tcagatacgc gaagaaatca ggagcgagtt acataaataa accaaaaatg 900

cgacattacg tccactgcta cgctttacat tgcctagacg aaaacgcttc aaacgctctc 960
cgaagatcat ttaaggaacg tggcgaaaac gttggatcgt ggcgtcaggc ttgttacaag 1020
ccacttgttg acgttgcttt tcgtcatggt ggggatatag atgctgtctt taacgctcat 1080
cctcgcctct ctatttggta tgtcccaact aagctgcgtc agctctgcca tttggagcgg 1140
aacaatgcgg gttctgcaac tgcggct 1167

<210> 186
<211> 1199
<212> DNA
<213> Capsella bursa-pastoris

<400> 186
gtggcttatt ccgatggaac ccaatgagag caatggttca agcaccacct ccggttcctc 60
cttcgccgca gcagcaacag ccggcaacac ctcagacggc ggctttcggg atgcgacttg 120
gtggcttaga gggactcttt ggtgcttacg gtatccgttt ctacacggcg gcgaagatag 180
cggagttggg ttttacggcc agcacgctcg ttggtatgaa ggacgaggag cttgaggaga 240
tgatgaacag tctctctcac atctttaggt gggagcttct cgttggtgaa cggtacggta 300
tcaaagctgc cgtaagagct gaacggagac gattgcaaga agaggaggag gaatcttcta 360
gacgccgtca tttgctgctc tccgccgctg gtgattccgg tactcatcac gctcttgatg 420
ccctctccca agaagatgac tggacagggt tatcagaaga accggtgcag cagcaagacc 480
agacagatgc ggcgggggaat aacggcggag gagggagtgg ttattgggaa gcaggtcaag 540
caaagatgaa gaagccacaa caaaggagga gaaagaaacc gatggtggcg tcagtggaaa 600
ccgatgacga cggcaatgaa ggcgaggatg atgacgggat ggataacggt aacggaggta 660
gtggtgggat ggggacggag agacagaggg agcatccgtt tatcgtaacg gagccagggg 720
aagtggcacg tggcaaaaag aacggtttgg attatctgtt ccatttgtac gaacaatgcc 780
gtgagttcct tcttcaggtc attcagacga tagctaaaga ccgtggcgag aaatgcccca 840
ccaaggtgac gtaccaagtg tttagatacg cgaagaaatc tggggcgagt tacataaaca 900
aacccaaaat gcgacactac gtccactgtt atgctctcca ctgtctagac gaagacgctt 960
cgaacgctct tcgaaggtct ttcaaagaac gcggtgagaa cgttggctcg tggcgtcagg 1020
cttgttacaa gccacttgtg aacatcgctt gtcgtcatgg ctgggatatt gacgccgtct 1080
ttaacgcaca ccctcgcctc tctatttggt atgtccccac taagctacgt cagctttgcc 1140
atttggagcg gaacaatgcg gttgctgcgg ctacggcttt agttggcggt attagctgt 1199

<210> 187
<211> 1183
<212> DNA
<213> Barbarea vulgaris

<400> 187
gtggcttatt ccgatggaac ccaacgagag caacggttca agcactacct ccggttcctc 60
ctccaccaca gcaacagccg gcaacaacac agacggcggc ttttgggatg cgacttggtg 120
gtttggaggg actgttcggt gcttacggga tacgttttta cacggcggcg aagatagcgg 180
agttaggttt tacggcgagc acgcttgtgg gtatgaggga cgaggagctt gaggaaatga 240
tgaatagcct ctctcatatc tttcggtggg agcttctcgt aggtgagcgg tacggtatca 300
aagctgccgt tagagctgaa cggagacgat tgcaagaaga ggaggaggaa gaatcttcta 360
gacgacgtca tttgctactc tccgccgctg gtgattccgg cactcatcac gctcttgatg 420
ctctctccca agaagatgat tggacaggct tatcagagga gccggtgcag cagcaagatc 480
accagactga tgcggcgggg aataacggcg gtaattggga agcaggtaaa ggaaagatga 540
agaagcaaca gcagagaagg agaaagaaac cgatgatgac gtcagtggaa acagacgatg 600
acatcaacga aggtgaggat gaagacggga tggataacgg taacggagga ggaggtggtg 660
gtgggttggg gacggagaga cagagagagc atccgtttat tgtaacggag ccaggggaag 720
tggcacgtgg caaaaagaac ggtttagatt acctgttcca tttgtacgaa caatgccgtg 780
agttccttct tcaggtccag acaattgcta aagaccgtgg cgagaaatgc gtgacgaacc 840
aagtgttcag gtacgcgaag aaatcgggag cgagttacat aaacaagccc aaaatgcgac 900
gctgcgtccg ctgttgcgct cttcactgcc tagacgagga tgcctcgagc gctctccgac 960
gagcgttcaa ggaacgcggt gggaacgtag gctcgtggcg tcaggcttgt tgcaagccac 1020
ttgtgaacat cgcttgtcgt catggctggg acatagatgc cgtctttaac gctcatcctc 1080
gcctctctat ttggtatgtc cctaccaagc tgcgtcagct ctgccatttg gaacggaaca 1140
atgcggttgc tgcagctacg gttttagttg gcggtattag ctg 1183

<210> 188
<211> 1239
<212> DNA

<213> Petunia hybrida

<400> 188

```
atggacccag aggctttctc agcaagtttg ttcaagtggg acccacgagg tgcaatgcca     60
ccaccaaacc ggttgttgga agcggtggca ccaccacaac caccacctcc tcctcttcca    120
cctccgcagc ctctaccacc ggcttattcc attagaacaa gagagctagg gggcctagag    180
gaaatgttcc aagcttatgg gataagatat tacactgctg ctaagataac tgagttaggt    240
tttacggtga atacactttt ggacatgaaa gatgatgaac ttgatgatat gatgaatagc    300
ctttcacaaa ttttcagatg ggaactgctt gttggagaaa ggtatggtat caaagctgct    360
attagagctg aacggcggag gcttgaggag gaagaagggc ggcgccggca cattctttct    420
gatggtggaa ctaatgttct tgatgctctc tcacaagaag ggttatctga ggaaccagtg    480
cagcagcaag agagagaagc agcgggaagc ggcggaggag ggacggcatg ggaagtggtg    540
gcgccaggcg gtggcagaat gagacaaagg aggaggaaga aggtggtggt ggggagggag    600
agaagggggt catcaatgga ggaagatgaa gacacggagg agggacaaga agataatgaa    660
gattataaca ttaataatga gggtggtgga ggaattagcg agagacaaag ggaacatccc    720
ttcatagtaa ctgagcctgg ggaggtggcg cgtggcaaaa agaatggctt agattacttg    780
ttccatctct atgaacaatg cagggatttc ttgatccaag ttcagaatat tgccaaggaa    840
cgtggtgaaa aatgccctac taaggtaaca aatcaggtgt tcaggttcgc aaagaaggca    900
ggagcaagtt acataaacaa gccaaaaatg cgacactacg tgcactgcta tgcacttcat    960
tgccttgatg aggatgcttc aaatgctcta agaagagcat tcaaggagag aggagagaat   1020
gttggggcat ggagacaggc atgttacaaa cccctggtag ccatagctgc tcgacaaggc   1080
tgggatatcg acgccatttt taatggacat cctcgactat ccatttggta tgtgcccacc   1140
aagctccgcc agctttgcca ttctgaacga agcaatgccg ctgcagctgc ttccacctca   1200
gtttctggtg gtggtgttga tcatctgcct catttttag                          1239
```

<210> 189
<211> 1191
<212> DNA
<213> Antirrhinum majus subsp. majus floricaula

<400> 189

```
atggatcctg atgcattctt gttcaaatgg gaccacagaa ccgccctccc tcaaccaaac     60
aggctcctcg acgccgtggc ccaccgcct cctccgccgc ctcaggcgcc gtcatactcc    120
atgaggccaa gagaactcgg cggcttagaa gaattattcc aagcttatgg catcagatac    180
tacactgccg ctaaaatcgc tgaacttgga ttcactgtga acacgctttt ggacatgagg    240
gacgaggagc tagacgagat gatgaacagc ctttgtcaga ttttcaggtg ggacctactt    300
gtcggagaga ggtatgggat taaggcggcg gtgagagcgg aacgacgtcg tatcgacgag    360
gaggaagtga ggcggaggca tctcttgttg ggtgatacta cgcatgctct tgatgctctt    420
tctcaagaag ggttgtcgga ggagccggtg cagcaagaaa aggaagcaat gggaagcggc    480
ggaggcggtg taggaggcgt gtgggaaatg atgggggcgg gtggtcgaaa agcaccgcag    540
cggcgtagga agaattacaa agggaggtct agaatggctt cgatggagga ggatgatgat    600
gatgatgacg acgaaaccga aggggcggaa gacgacgaaa atatcgtaag cgagcggcag    660
agggagcatc cgtttatcgt gacggagccc ggagaggtgg cgcgtgggaa aaagaatggt    720
cttgattatt tgtttcattt gtacgagcaa tgccgcgact tcttgatcca agttcaaact    780
attgctaagg agagaggtga aaaatgtccc actaaggtga cgaaccaagt gttcaggtac    840
gcaaagaagg ctggcgctaa ctacatcaac aaaccaaaaa tgcgccacta cgtgcactgc    900
tacgccctgc actgccttga tgaggccgcg tccaatgcac ttcgtcgggc attcaaggag    960
cgtggtgaga acgtcggtgc atggcgtcag gcatgctaca agcccttggt ggccattgca   1020
gcaagacaag gatgggatat cgataccata ttcaacgctc atccccgtct ctcgatctgg   1080
tatgtcccca ccaagcttcg tcagctctgc catgccgaga ggagcagtgc ggcagttgct   1140
gccaccagct ccatcaccgg aggtgggccg gcagatcact tgccgtttta g            1191
```

<210> 190
<211> 1242
<212> DNA
<213> Nicotiana tabacum

<400> 190

```
atggacccag aggctttctc agcgagtttg ttcaaatggg accctagagg tgcaatgcca     60
ccgccaaccc ggctgttgga agccgcggtg gcgcctcctc ctccaccacc agttctgcca    120
ccgccgcagc ctctatcggc ggcctattcc attaggacaa gggagttagg agggctagag    180
gagttgtttc aagcttacgg tatacgttat tacactgctg ctaaaatagc ggagctaggt    240
tttacggtga atactctatt ggacatgaaa gatgaggaac ttgatgatat gatgaatagc    300
```

ctttcacaga ttttcagatg ggaactcctc gtcggagaaa ggtacggtat caaagctgca 360
atcagggcgg aacggcggag gcttgaggag gaagaactac ggcggcgcag ccaccttctg 420
tctgatggtg gaactaatgc ccttgacgct ctctcacaag aagggttgtc tgaggaacca 480
gtgcagcagc aagagagaga agcagttgga agcggcggag ggggaacgac atgggaagtg 540
gtggcggcag ttggcggtgg aagaatgaaa caaagaagga ggaagaaggt ggtgtcgacg 600
gggagggaga gaaggggaag agcgtcggcg gaggaggatg aagaaacgga ggaaggtcaa 660
gaagatgagt ggaatattaa cgacgccggg ggaggaataa gcgagaggca aagggagcat 720
ccttttatcg tgacggagcc aggtgaggtg gcgcgtggga aaaagaacgg cttggattac 780
ttgttccacc tctacgagca atgccgggat ttcttgattc aagttcagaa tattgccaag 840
gaacgtggtg aaaaatgtcc cactaaggta acaaatcagg tgttcaggta cgcgaagaag 900
gcaggggcaa gctacataaa taagccaaaa atgcgacact acgtgcattg ctacgcactt 960
cattgccttg atgaggaggc ctccaatgcg ctaagaagag ctttcaagga gcgaggagag 1020
aatgttgggg catggagaca agcatgttac aagcccctgg tagccatagc tgctcgacaa 1080
ggctgggata tcgacaccat ctttaatgca catcctcgac tcgccatttg gtatgtcccc 1140
accaggctcc gccagctttg ccattctgaa cgaagcaacg ctgctgctgc tgcttctagc 1200
tcggtttctg gtggtgttgg tgatcacctg ccgcatttct aa 1242

<210> 191
<211> 1251
<212> DNA
<213> Nicotiana tabacum

<400> 191
atggacccag aggctttctc agcgagtttg ttcaagtggg accctagagg tgcaatgcca 60
ccgccaaccc ggctgttgga agcagcggtg gcgcctcctc ctcctccgcc agctcttcca 120
ccgccgcagc ctctgtcggc ggcttattcc attaagacaa gggagttagg aggactagag 180
gagttatttc aagcttacgg tataagatat tacactgctg ctaaaatagc ggagttaggt 240
tttacggtga acactctatt ggacatgaaa gatgaggaac ttgatgatat gatgaatagc 300
ctttcacaga ttttcagatg ggaactactc gtcggagaaa ggtacggtat caaagctgca 360
atcagggcgg aacggcggag gcttgaggag gaagaactgc ggcggcgtgg ccaccttctg 420
tctgatggtg gaactaatgc ccttgacgct ctctcacaag aagggttgtc tgaggaacca 480
gtgcagcagc aagagagaga agcagtggga agtggcggag ggggaacgac atgggaagtg 540
gtggcggcag ctggcggtgg gagaatgaaa caaaggagga ggaagaaggt ggtggcggcg 600
gggagggaga aaaggggagg agcgtcggcg gaggaggatg aagaaacgga ggaaggtcaa 660
gaagatgact ggaacattaa cgacgccagt ggaggaataa gcgagaggca aagggagcat 720
ccttttatcg tgacggagcc aggtgaggtg gcgcgtggga aaaagaacgg cttggattac 780
ttgttccacc tctatgagca atgccgggat ttcttgatcc aagttcagaa tattgccaag 840
gaacgtggtg aaaaatgccc cactaaggta acaaatcagg tgttcaggta cgcgaagaag 900
gcaggggcaa gctacataaa caagccaaaa atgcgacact acgtgcattg ctacgcactt 960
cattgccttg acgaggaagc ctccaatgcg ctaagaagag ctttcaagga gcgaggagag 1020
aacgtcgggg cgtggagaca ggcatgttac aaaccccttg tggccatagc tgctcgacaa 1080
ggctgggata tcgacaccat ctttaatgca catcctcgac tcgccatttg gtatgttccc 1140
accaagctcc gccagctttg ccactctgaa cggagcaatg ctgctgctgc tgctgcttct 1200
agctcggttt ctggtggtgg tggtggtggt gatcacctcc ctcatttcta a 1251

<210> 192
<211> 1179
<212> DNA
<213> Triticum aestivum

<400> 192
atggatccca acgacgcctt cttggccgcg cacccgttca ggtgggacct cggcccgccg 60
gctccggcag ccgtgcctcc tccccctccc ccgcctccgc ctcctcctgc gctacctccg 120
gcgaacgcgc cgagggagct ggaggacctc gtggtcgggt atggcgtgcg cgcgtccacg 180
gtggcgcgga tctcggagct cgggttcacg gccagcacgc tcctcgtcat gacggagcgc 240
gagctcgacg acatgacggc cgcgctcgcg ggactattcc gctgggacct gctcatcggc 300
gagcggttcg gccttcgtgc cgcgctgcgc gccgagcgcg gccgcctcat gtcaccgggc 360
tgccgccacc acggatacca gtccgggagc accatcgacg gcgcctcaca ggaagtgctg 420
tcgaacgagc gcgatggggc ggctagcggc ggcatcggcg aagaggacgc catgaggatg 480
atggcgtcgg gcaagaagca gaagaatggg tccgcaggga ggaaggccaa gaaggccagg 540
aggaagaagg tgaacgacct gcggctggac atgcaggggg acgagcacga ggaaggcggg 600
ggcggccggt cggagtcgac ggagtcgtca gccggcggag gcgtcggcgg ggagcggcag 660
cgggagcacc cgttcgtggt gacggagccc ggcgaggtgg cgagggccaa gaagaacggg 720

```
ctggactacc tgttccatct ctacgagcag tgccgcctct tcctgctcca ggtgcagtcc    780
atggccaagc tgcatggcca gaagtctcca accaaggtga cgaaccaggt gttcaggtac    840
gcgagcaagg tgggggcgag ctacatcaac aagcccaaga tgcggcacta cgtgcactgc    900
tacgcgctgc actgcctgga cgaggacgcc tccgacgcgc tgcgccgggc gtacaaggcg    960
cgcggcgaga acgtcggggc gtggcggcag gcctgctacg cgccgctggt ggacatcgcg   1020
gcgcgccacg gcttcgacat cgacgccgtc ttcgccgcgc acccgcggct cgccatctgg   1080
tacgtgccca ccaggctccg ccagctctgc caccaggccc ggagcgccca cgacaccgcc   1140
gccgcgcacg ccggcgccat gccgccgccc atgttctag                          1179
```

<210> 193
<211> 1179
<212> DNA
<213> Triticum aestivum

<400> 193

```
atggatccca acgacgcctt cttggccgcg cacccgttta ggtgggacct cggcccaccg     60
gctccggcag ccgtgcctcc tcctcctccc ccgcctccgc ttcctcctgc gctgcctccg    120
gcgaacgcgc cgagggagct ggaggacctc gtggtcgggt atggcgtgcg cgcgtccacg    180
gtggcgcgga tctcggagct cgggttcacg gctagcacgc tcctggtcat gaccgagagc    240
gagctcgacg acatgacggc cgcgctcgcg ggttgttcc gctgggacct gctcatcggc    300
gagcggttcg gccttcgcgc cgcgctgcgc gccgaacgtg gccgcctcat gtcaccaggc    360
tgccgccacc acggatacca gtccggcagc accatcgacg gcgcctcaca ggaagtgttg    420
tcgaacgagc gcgatggggc ggctagcggc ggcatcggcg aagacgacgc catgaggatg    480
atggcgtctg gcaagaagca gaagaatggg tccgcagcga ggaaggccaa gaaggcgagg    540
aggaacaagg tgaaggagct gcgactggac atgcaggggg acgagcacga ggacggcggg    600
ggcggccggt cggagtcgac ggagtcgtca gccggaggcg tcggcgggga gcggcagcgg    660
gagcacccgt tcgtggtgac ggagcccggc gaggtggcga gggcgaagaa gaacgggctg    720
gactacctgt tccatctcta cgagcagcgc cgcctcttcc tgctccaggt gcagtccatg    780
gccaagctgc atggccagaa gtctccaacc aaggtgacga accaggtgtt caggtacgcg    840
agcaaggtgg gggcgagcta catcaacaag cccaagatgc ggcactacgt gcactgctac    900
gcgctgcact gcctggacga ggacgcctcc gacgcgctgc gccgggcgta caaggcgcgc    960
ggcgagaacg tcggcgcctg gaggcaggcg tgctacgcgc cgctggtgga catcgcggcg   1020
cgccacggct tcgacatcga cgccgtcttc gccgcgcacc cgcggctcgc catctggtac   1080
gtgcccacca ggctccgcca gctctgtcac caggcgcgca gcgcccacga cgccgccgcc   1140
gccgcacacg ccggctccat gccgccgcca atgttctag                          1179
```

<210> 194
<211> 1203
<212> DNA
<213> Lolium temulentum

<400> 194

```
atggatcccc acgacgcctt cctcgccgcg cacccgttcc ggtgggacct cggcccgccg     60
gctccggcgg ccgtgccccc tcctcctcca ctgcccatgc ctcaaactcc cgcgctgcct    120
ccggcgaact cgccgaggga gctggaggat ctcgtggccg ggtacggcgt gcgcggggcc    180
acggttgcgc gaatctccga gctcggcttc acggccagca cgctcctggt catgacggac    240
cgcgagctgg acgacatgac ggccgcactc gccggcctgt tccgctggga cctgctcatc    300
ggcgagcggt tcggcctgcg cgccgcgctg cgagcagagc gcggccgcct gatggcactg    360
catgggggcc gacaccacgg tcaccagtcc ggcagcacca tcgacggcgc ctcccaagaa    420
gtgttgtcca acgaacggga tggggcggcg agcggcgagg acgacgccgg caggatgatg    480
ttatcgggca agaagctgaa gaatggatcg gtggcgagaa aggccaagaa agcaaggagg    540
aagaaggtgg acgggctccg gctggaccac atgcaggagg acgagcgcga ggacggcggc    600
ggccgctcgg agtcaacgga gtcgtcggct ggcggaggcg gcggcgttgg aggggagcgg    660
cagcgggagc acccgttcgt ggtgacggag cccggggagg tggcgagggc caagaagaac    720
gggctggact acctgttcca tctctacgag cagtgccgcc tcttcctgct ccaggtgcag    780
tccatggcca agctgcatgg ccacaagtct ccaaccaagg tgacgaacca ggtgttcagg    840
tacgcgagca aggtgggggc gagctacatc aacaagccca agatgcgcca ctacgtgcac    900
tgctacgcgc tgcactgcct cgaccaggag gcctccgacg cgctgcgccg cgcgtacaag    960
gcccgcggcg agaacgtcgg cgcctggagg caggcatgct acgcgccgct cgtcgacatc   1020
gccgccggcc acggcttcga cgtcgacgcc gtcttcgccg cgcacccgcg actcgccatc   1080
tggtacgtgc ccaccaggct ccgccagctc tgccaccagg caaggagcgc gcacgaagcc   1140
gccgccgcca acgccaacgc caacggggcc atgccgccgc cgccgccgcc gcccatgttc   1200
tag                                                                 1203
```

<210> 195
<211> 1170
<212> DNA
<213> Oryza sativa

<400> 195
atggatccca acgatgcctt ctcggccgcg cacccgttcc ggtgggacct cggcccgccg 60
gcgccggcgc ccgtgccacc accgccgcca ccaccgccgc cgccgccgcc ggctaacgtg 120
cccagggagc tggaggagct ggtggcaggg tacggcgtgc ggatgtcgac ggtggcgcgg 180
atctcggagc tcgggttcac ggcgagcacg ctcctggcca tgacggagcg cgagctcgac 240
gacatgatgg ccgcgctcgc cgggctgttc cgctgggacc tgctcctcgg cgagcggttc 300
ggcctccgcg ccgcgctgcg agccgagcgc ggccgcctga tgtcgctcgg cggccgccac 360
catgggcacc agtccgggag caccgtggac ggcgcctccc aggaagtgtt gtccgacgag 420
catgacatgg cggggagcgg cggcatgggc gacgacgaca acggcaggag gatggtgacc 480
ggcaagaagc aggcgaagaa gggatccgcg gcgaggaagg gcaagaaggc gaggaggaag 540
aaggtggacg acctaaggct ggacatgcag gaggacgaga tggactgctg cgacgaggac 600
ggcggcggcg ggtcggagtc gacggagtcg tcggccggcg gcggcggcgg ggagcggcag 660
agggagcatc ctttcgtggt gacggagccc ggcgaggtgg cgagggccaa gaagaacggg 720
ctggactacc tgttccatct gtacgagcag tgccgcctct tcctgctgca ggtgcaatcc 780
atggctaagc tgcatggaca caagtcccca accaaggtga cgaaccaggt gttccggtac 840
gcgaagaagg tcggggcgag ctacatcaac aagcccaaga tgcggcacta cgtgcactgc 900
tacgcgctgc actgcctgga cgaggaggcg tcggacgcgc tgcggcgcgc ctacaaggcc 960
cgcggcgaga acgtgggggc gtggaggcag gcctgctacg cgccgctcgt cgacatctcc 1020
gcgcgccacg gattcgacat cgacgccgtc ttcgccgcgc acccgcgcct cgccatctgg 1080
tacgtgccca ccagactccg ccagctctgc caccaggcgc ggagcagcca cgccgccgcc 1140
gccgccgcgc tcccgccgcc cttgttctaa 1170

<210> 196
<211> 1182
<212> DNA
<213> Zea mays

<400> 196
gatcccaacg acgccttctc ggcggcgcac ccgttccggt gggacctggg cccgccggcc 60
cccgccgcgc ccgcgcctcc gcccccaccg ccgcccgcgc cgcagctgct gccccacgcg 120
ccgctgctga gcgcgccgag ggagctggag gacctggtgg ccggctacgg cgtgcgcccg 180
tccacggtgg cgcggatctc ggagctcggg ttcacggcca gcacgctcct cggcatgacg 240
gagcgcgagc tcgacgacat gatggccgcg ctcgcggggc tgttccgctg ggacgtgctc 300
ctcggcgagc gcttcggcct ccgcgccgcg ctgcgggccg agcgcgggcg tgtcatgtcc 360
ctcggcggcc gcttccacac cgggagcaca ttggacgccg cgtcacaaga agtgctgtcc 420
gacgagcgcg acgccgcggc cagcggcggc ttagcggaag gcgaggccgg caggaggatg 480
gtgacgaccg gcaagaagaa gggcaagaaa ggggttggcg cgaggaaggg caagaaggcg 540
aggaggaaga aggagctgag gccgttggac gtgctggacg acgagaacga cggagacgag 600
gacggcggcg gcggcgggtc agactcgacg gagtcttccg ctggcggctc cggcggcggg 660
gagaggcagc gggagcaccc cttcgtggtc acagagcccg gcgaggtggc cagggccaag 720
aagaacgggc ttgactacct cttccatctg tacgagcagt gccgcgtctt cctgctgcag 780
gtgcagtccc ttgctaagct gggcggccac aagtccccta caaaggtgac caaccaggtg 840
ttccggtacg ccaagaagtg cggcgcgagc tacatcaaca agcccaagat gcggcactac 900
gtgcactgct acgcgctgca ctgcctggac gaggatgcct ccaacgcgct gcgccgggcg 960
tacaaggccc gtggcgagaa cgtcggtgcc tggaggcagg cctgctacgc gccgctcgtc 1020
gagatcgccg cgcgccacgg cttcgacatc gacgccgtct tcgccgcgca cccgcgcctc 1080
accatctggt acgtgcccac caggttgcgc cagctctgcc accaggcacg gggagccac 1140
gcccacgccg ccgccggcct ccccccgccc ccgatgttct ag 1182

<210> 197
<211> 1176
<212> DNA
<213> Zea mays

<400> 197
atggatccca acgacgcctt ctcggcggcg cacccgttcc ggtgggacct cggcccgccg 60
gcgcacgccg cgcccgcgcc cgcgcctccg cctccgccgc tagcaccgct gctgctgccg 120

cctcacgcgc cgcgggagct ggaggacctg gtggccggct acggcgtgcg cccgtccacg 180
gtggcgcgga tctcggagct cgggttcacg gcgagcacgc tcctcggcat gacggagcgc 240
gagctggacg acatgatggc cgcgctcgcg ggctgttcc gctgggacgt gctcctcggc 300
gagcgcttcg gcctccgcgc cgcgctgcgc gccgagcgcg gccgcgtcat gtccctcggc 360
gcccgctgct tccacgccgg gagcaccttg gatgccgcgt cacaagaagc gctgtccgac 420
gagcgcgacg ccgcggccag cggcggcggc atggcagaag gcgaggccgg caggaggatg 480
gtgacgacga ccgccggcaa gaagggcaag aaaggggtcg ttggcacgag gaagggcaag 540
aaggcgagga ggaagaagga gctgaggccg ctgaacgtgc tggacgacga gaacgacggg 600
gacgagtacg gcggcgggtc ggagtcgacc gagtcgtccg cgggaggctc cggggagagg 660
cagcgggagc acccgttcgt ggtcaccgag cccggcgagg tggcgagggc caagaagaac 720
gggctcgact acctcttcca cctgtacgag cagtgccgcg tcttcctgct ccaggtgcag 780
tccatcgcta agctgggcgg ccacaaatcc cctaccaagg tgaccaacca ggtgttccgg 840
tacgcgaaca agtgcggggc gagctacatc aacaagccca agatgcggca ctacgtgcac 900
tgctacgcgc tgcactgcct ggacgaggag gcctccaacg cgctgcgccg ggcgtacaag 960
tcccgcggcg agaacgtggg cgcctggagg caggcctgct acgcgccgct cgtcgagatc 1020
gccgcgcgcc acggcttcga cattgacgcc gtcttcgccg cgcacccgcg cctcgccgtc 1080
tggtacgtgc ccaccaggct gcgccagctc tgccaccagg cgcgggggag ccacgcccac 1140
gctgccgccg gactcccgcc gcccccgatg ttctag 1176

<210> 198
<211> 1371
<212> DNA
<213> Ophrys tenthredinifera

<400> 198
atggtgctgg ccacatcgca gcaacaccac cagcataacc ctcacgaagt ccagcagcac 60
ctgcagccgc attcgacggc aacagagtcg tcgcgggagc tagaggaggt gttcgagggg 120
tacggagttc ggtactcgac gattgctcgg attggggatc tgggcttcac agcgagcacg 180
ctggcaggta tgagggagga ggaggtggac gatatgatgg ccgcactgtc gcatctcttc 240
cggtgggatc ttcttgtcgg cgaacgatat gggatcaaag cggcaattag ggcagagcga 300
cgccgtcttg aagcgctcat tttttctcat gtctccggcg cagcccgcct aagccatcat 360
caacatcaaa tgggatacct cttttcgtct gccaccacag gctaccactt aatgcctgat 420
gatccacgca agaggcacct tctcctctcc cccgatcacc acagcgctct cgacgcactt 480
tcccaagaag gactctctga ggagccagtg cagctggaga gggaggcggc tggcagcggt 540
ggcgaagtgg taggcaggag agatggaaag gggaagaacc aacaacggca aacctcggca 600
aagaagaagg atgcctcctc tacgaagagc aagaaaaaga agaagaaagg gatcgaagaa 660
ggagacgatg aagaagagga ggtcgaagtg tgggggcgcg gggcaagcat tgagaatgat 720
gaggatgacg acggggatga gtcgcaatca gagcaaagca gcgctgcaga gcggcagagg 780
gagcacccgt tcatcgtgac ggagccaggt gaggtggcgc gagctaagaa gaacgggctc 840
gattacctct tcaatcttta cgaacaatgc catgaatttc tgaaccaggt ccagtccgtg 900
gcgaaggagc gcggggacaa gtgcccaact aaggtgacga acctggtatt ccgatatgcg 960
aagaagaaag tgggagcaag ctacatcaat aagccgaaga tgaggcacta cgtgcactgc 1020
tacgcgctcc acgtgctaga cgaggatgcg tccaactccc tgaggcgggc gttcaaggaa 1080
cgcggggaga acgttggcgc ctggcgactt gcctgctaca agcccttggt ggccatctcc 1140
gcctcccaca gcttcgacat agacgccgtt ttcaacgcgc atccccgcct ctccatctgg 1200
tacgtcccca ctaagctacg ccagctctgc cacctcgccc gcagttccac ctctcagttc 1260
ccgctggccg ttcccagaac tacaggcagt tcgaaccaac gcgtctcatc caccgtccac 1320
gttgttgaag actcagctgc ggcacactcc ttccgtccgc ccatgttcta a 1371

<210> 199
<211> 1239
<212> DNA
<213> Lycopersicon esculentum

<400> 199
atggacccag atgctttctc ggcgagtttg ttcaagtggg acccaagagg tgcaatgcca 60
ccaccaagcc ggttattaga accggtggcg ccaccacaac ctcctccatc tctaccacca 120
ccaccacctc ctcagccgct cccaacatca tcctactcca tacggagtac gagggagctc 180
ggaggactag aggagttatt tcaagcgtac ggcatacgct actacaccgc cgctaagata 240
gcggagttag ggttcactgt gaacacgtta ttggacatga aagatgaaga acttgatgat 300
atgatgaata gcctctcgca gatatttcgg tgggacctac tcgtcggaga gaggtacggt 360
atcaaagcgg cgattagagc tgaatggcgg aggctggagg aggaggaagc acggcgccgc 420
ggacacattt tgtccgacgg tggaacgaat gtccttgacg ctctatcaca agaaggatta 480

```
tcggaggaac cagtgcagca gcagcacgag agagaagcgg caggaagtgg tggtggaggt    540
acatgggaag tggctgccgg tggtggtggt aggatgaaac aaaggaggag gaagaaggcg    600
gggagggaga gaagagggga agaggatgag gaaacggagg aattaggaga agaagatgaa    660
gaaaatatga accaaggagg tggaggtgga ggaataagcg agagacaaag ggagcatccg    720
tttatcgtga cggagcctgg tgaagtagca cgtggcaaaa agaacggctt ggattatctg    780
ttccatctct acgaacaatg ccgtgatttc ttgatccaag ttcagactat tgctaaggaa    840
cgaggtgaaa aatgccctac gaaggtgacg aatcaggtgt tcaggtacgc gaagaaggca    900
ggggcaagct acataaacaa gcccaaaatg agacattatg tgcattgcta tgcacttcac    960
tgccttgatg aggatgcttc caatgctctg agaagagctt tcaaggagcg gggagagaat   1020
gttggggcat ggagacaggc gtgttacaag ccattggtgg ctatagcggc tcgacaaggc   1080
tgggatatcg atgcaatctt caatgcacat cctcgactag ccatttggta tgtccccacc   1140
aagctccgac agctgtgcca ttctgaaaga agcaacgcag ctgcagctgc ttctagctcc   1200
gtctctggtg gtgttgctga tcacctgcca catttctaa                          1239
```

<210> 200
<211> 1104
<212> DNA
<213> Carica papaya

<400> 200

```
atggatccag acggcttttc ttccagcttg ttcaagtggg acccaacgag gggaatagtg     60
caggcgccag tcaggttgct ggaggcggta gctgcggcgc ctacgcaggc ggcgtacgga    120
gtgaggccga gggagctggg tggtctagag gagctttttc aagattacgg catcaggtac    180
ttcaccgctg cgaagatcgc cgagctgggt ttcacggcta gcacgctggt ggatatgaag    240
gatgaggaac tggacgagat gatgaacagc ttgagccaga tttttaggtg ggagcttctg    300
gtgggagaga ggtatgggat taaggctgct gttcgcgctg aaaggaggcg gcttgacgac    360
gacgattcca gaagaagaca gaccctctct actgacacta cccacgctct cgatgctctc    420
tcccaggaag ggttatcaga ggagccggtg cagcaggaga aggaggcggc ggggagcggg    480
ggaggtacga tatgggaggt tgggccgggg aagaaaaagc agcggcggag aaaggtggtg    540
ggtgaggagg agcaggagga ggaaaacggt ggtggaagcg agagacagcg cgagcaccct    600
ttcatcgtga cagagcctgg ggaggtggca cgtggcaaaa aaaatggcct tgattatctc    660
ttccacttgt acgagcagtg tcgtgacttc ttgatccaag tccagaacat cgccaaggag    720
cgaggagaaa agtgtcccac gaaggtgacg aaccaggtgt ttagatatgc aaagaaagct    780
ggggcgagtt atataaacaa gccaaaaatg cgacactatg tgcactgcta tgctttacac    840
tgtcttgacg agaaggaatc aaatgcgttg aggacagcat ttaaggagag aggagaaaat    900
gtagggtcgt ggagacaggc gtgttataag cctcttgtcg ccattgcagc acgccaaggt    960
tgggacattg atgccatttt caatgcacat cctcgtcttg ccatttggta tgtccccaac   1020
aagcttcgcc aactttgcca tgccgagcgc aataatactg ccattgcttc tacctccgcg   1080
gctgctcatc atcttccatt ctaa                                          1104
```